# EUROPEAN PATENT APPLICATION

(11) **EP 4 516 792 A1**
(43) Date of publication of application: **05.03.2025**
(21) Application number: 23796476.2
(22) Date of filing: 27.04.2023
(51) Int. Cl.: C07D 471/04, A61K 31/55, A61K 31/553, A61P 1/04, A61P 1/16, A61P 3/10, A61P 7/10, A61P 9/00, A61P 9/04, A61P 9/10, A61P 9/12, A61P 11/00, A61P 11/06, A61P 11/14, A61P 13/12, A61P 17/00, A61P 17/06, A61P 19/02, A61P 21/02, A61P 25/00

(54) **BENZOTHIOPHENE COMPOUND**

(30) Priority: 28.04.2022 JP 2022075222
(71) Applicant: Kyoto Pharmaceutical Industries, Ltd., Kyoto-shi, Kyoto 604-8444 (JP)
(72) Inventor: OGURA, Yoshihiro, Tokyo 103-8426 (JP); FUJIMOTO, Teppei, Tokyo 103-8426 (JP); INUI, Masaharu, Tokyo 103-8426 (JP); TAKANO, Rieko, Tokyo 103-8426 (JP); OFUKU, Masafumi, Tokyo 103-8426 (JP); KAWAI, Junya, Tokyo 103-8426 (JP); MIYAZAKI, Masaki, Tokyo 103-8426 (JP); YOSHITOMI, Tomomi, Tokyo 103-8426 (JP); EDO, Naoko, Tokyo 103-8426 (JP); OTAKE, Kazuya, Kyoto-shi, Kyoto 604-8444 (JP); TAKASHIMA, Shunsuke, Kyoto-shi, Kyoto 604-8444 (JP); ANDO, Masafumi, Kyoto-shi, Kyoto 604-8444 (JP)
(74) Representative: Engelhard, Markus
(86) International application number: PCT/JP2023/016628
(87) International publication number: WO 2023/210740

(57) **Abstract**

The present invention aims to provide a medicament capable of treating and/or preventing diseases associated with oxidative stress by inhibiting the protein-protein interaction between Keap1 and Nrf2 and activating Nrf2. The present invention relates to a compound represented by the following formula (1): 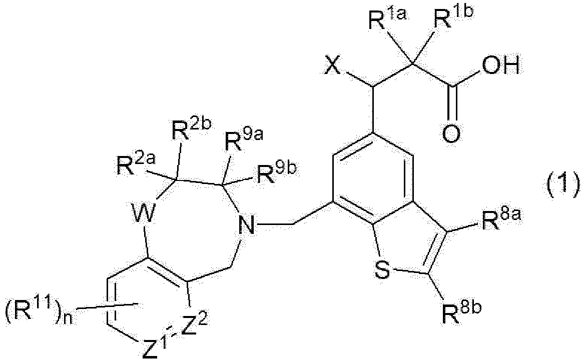 wherein each symbol is as described in the DESCRIPTION,
or a pharmaceutically acceptable salt thereof. In addition, the present invention also relates to a medicament containing the compound, for the prophylaxis and/or treatment of diseases involving oxidative stress selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, age-related macular degeneration, retinitis pigmentosa and glaucoma.

## Description

### [Technical Field]

The present invention relates to a benzothiophene compound or a pharmaceutically acceptable salt thereof, which is useful for the treatment and/or prophylaxis of diseases involving oxidative stress, particularly, chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, or age-related macular degeneration, by inhibiting Kelch-like ECH-associated protein 1 (Keap1) and activating NF-E2-related factor 2 (Nrf2).

### [Background Art]

When reactive oxygen species generated during the energy metabolism are detected, the body's defense system, including antioxidant enzymes and detoxification metabolic enzymes, is activated. Nrf2 controls the activation of this defense system.

It is known that activation of Nrf2 induces its target genes, such as NAD(P)H quinone oxidoreductase-1 (NQO1), heme oxygenase-1 (HO-1), gamma-glutamate cysteine ligase catalytic subunit (GCLC) and the like (Non Patent Literature 1). NQO1 is a phase II enzyme of the xenobiotic metabolic system, and is important for detoxification. HO-1 and GCLC are known as typical antioxidant enzymes. When the amount of these enzymes increases or these enzymes are activated, cells become resistant to toxins, oxidative stress, inflammation, and the like, and therefore, compounds that activate Nrf2 are considered to be therapeutic agents for various diseases (Non Patent Literature 2).

Since Nrf2 is ubiquitinated by Keap1 and degraded in the proteasome system in the steady state, compounds that inhibit Keap1 activate Nrf2. Compounds that activate Nrf2 by modifying the cysteine residues of Keap1 have been known; however, low specificity thereof due to its activation mechanism is of concern. On the other hand, compounds that inhibit the protein-protein interaction (PPI) between Keap1 and Nrf2 are expected to more specifically activate Nrf2, and have been attracting increasing attention in recent years as prophylactic and/or therapeutic drugs for various diseases caused by oxidative stress (Non Patent Literature 3).

To date, compounds that inhibit Keap1 and activate Nrf2, for example, compounds described in Patent Literatures 1 to 11, have been reported but all of them are structurally different from the compound of the present invention.

### [Citation List]

### [Patent Literature]

[Patent Literature 1]
   WO 2015/092713
[Patent Literature 2]
   WO 2016/202253
[Patent Literature 3]
   WO 2016/203400
[Patent Literature 4]
   WO 2016/203401
[Patent Literature 5]
   WO 2018/109643
[Patent Literature 6]
   WO 2018/109647
[Patent Literature 7]
   WO 2018/109648
[Patent Literature 8]
   WO 2019/224667
[Patent Literature 9]
   WO 2020/165776
[Patent Literature 10]
   WO 2018/181345
[Patent Literature 11]
   WO 2020/241853

### [Non Patent Literature]

[Non Patent Literature 1]
   Int. J. Biochem. Cell. Biol., 2012, 44, 1315-1320
[Non Patent Literature 2]
   Nat. Rev. Drug. Discov., 2019, 18, 295-317
[Non Patent Literature 3]
   Eur. J. Med. Chem., 2020, 202, 112532

### [Summary of Invention]

### [Technical Problem]

The present invention aims to provide a medicament capable of treating and/or preventing diseases involving oxidative stress, particularly, chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, or age-related macular degeneration, by inhibiting protein-protein interactions between Keap1 and Nrf2 and activating Nrf2.

### [Solution to Problem]

The present inventors have conducted intensive studies in an attempt to solve the above-mentioned problems and found that a compound represented by the following formula (1): 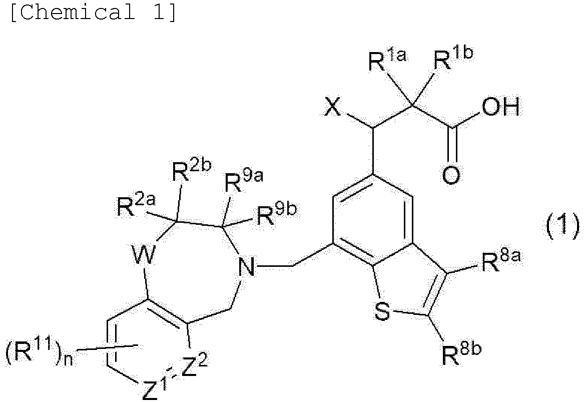 wherein
R^{1a} and R^{1b} are each independently a hydrogen atom or a C₁₋₆ alkyl group,
R^{2a} and R^{2b} are each independently a hydrogen atom, a C₁₋₆ alkyl group optionally substituted by 1 to 3 substituents selected from substituent group a, or a C₃₋₆ cycloalkyl group optionally substituted by 1 to 3 substituents selected from substituent group a, or R^{2a} and R^{2b} are bonded together to form, together with the carbon atom to which R^{2a} and R^{2b} are bonded, a C₃₋₈ cycloalkane optionally substituted by 1 to 3 substituents selected from substituent group b, or a 3- to 8-membered saturated oxygen-containing heterocycle,
Z¹ is -CH-, -CR³- or a nitrogen atom,
R³ is a halogen atom, a hydroxy group, an amino group optionally substituted by one or two C₁₋₆ alkyl groups, a C₁₋₆ alkyl group optionally substituted by 1 to 3 substituents selected from substituent group a, or a C₁₋₆ alkoxy group optionally substituted by 1 to 3 substituents selected from substituent group a,
Z² is -CH-, -CR⁴- or a nitrogen atom,
R⁴ is a halogen atom, a hydroxy group, an amino group optionally substituted by one or two C₁₋₆ alkyl groups, a C₁₋₆ alkyl group optionally substituted by 1 to 3 substituents selected from substituent group a, or a C₁₋₆ alkoxy group optionally substituted by 1 to 3 substituents selected from substituent group a,
R¹¹ in the number of n are each independently a halogen atom, a hydroxy group, an amino group optionally substituted by one or two C₁₋₆ alkyl groups, a C₁₋₆ alkyl group optionally substituted by 1 to 3 substituents selected from substituent group a, or a C₁₋₆ alkoxy group optionally substituted by 1 to 3 substituents selected from substituent group a,
n is an integer of 0 to 2,
R^{8a} and R^{8b} are each independently a hydrogen atom, a halogen atom, a cyano group, a C₁₋₆ alkyl group or a C₁₋₆ alkoxy group, R^{9a} and R^{9b} are each independently a hydrogen atom or a C₁₋₆ alkyl group,
W is -CH₂-, -CHR¹⁰- or an oxygen atom,
R¹⁰ is a C₁₋₆ alkyl group, and
X is a group represented by the following formula (A1) or (A2): 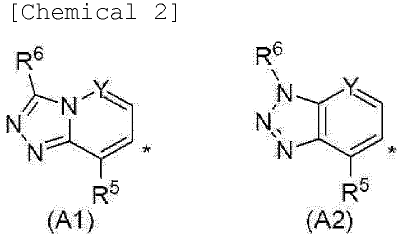 wherein
   * is the bonding position to the carbon atom to which X is bonded,
   R⁵ is a hydrogen atom, a halogen atom, or a C₁₋₆ alkyl group optionally substituted by 1 to 3 substituents selected from substituent group a,
   R⁶ is a hydrogen atom, or a C₁₋₆ alkyl group optionally substituted by 1 to 3 substituents selected from substituent group a,
   Y is -CH-, -CR⁷- or a nitrogen atom, and
   R⁷ is a hydroxy group, a halogen atom, a cyano group, a C₁₋₆ alkyl group optionally substituted by 1 to 3 substituents selected from substituent group a, a C₁₋₆ alkoxy group optionally substituted by 1 to 3 substituents selected from substituent group a, or a C₃₋₆ cycloalkyl group,
   provided that when Z¹ is -CH- or -CR³-, then Z² is a nitrogen atom, when Z² is -CH- or -CR⁴-, then Z¹ is a nitrogen atom, and both Z¹ and Z² cannot simultaneously be -CH-, -CR³- or -CR⁴-,
   substituent group a:
      a hydroxy group,
      a halogen atom,
      a cyano group,
      a C₁₋₆ alkyl group,
      a C₁₋₆ alkoxy group,
      a C₁₋₆ alkyl group substituted by substituent group b,
      a C₁₋₆ alkoxy group substituted by substituent group b,
      an amino group optionally substituted by one or two C₁₋₆ alkyl groups,
      a C₁₋₆ alkylsulfonyl group
      substituent group b:
         a halogen atom,
         a cyano group,
         a C₁₋₆ alkyl group,
         a C₁₋₆ alkoxy group
         (hereinafter sometimes abbreviated as "compound (1)") or a pharmaceutically acceptable salt thereof has a superior Nrf2 activation action by inhibiting Keap1, and completed the present invention.

That is, the present invention provides the following.
[1] A compound represented by the following formula (1): 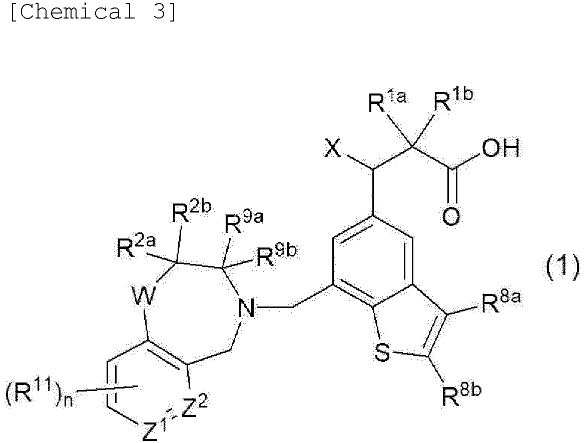 wherein
   R^{1a} and R^{1b} are each independently a hydrogen atom or a C₁₋₆ alkyl group,
   R^{2a} and R^{2b} are each independently a hydrogen atom, a C₁₋₆ alkyl group optionally substituted by 1 to 3 substituents selected from substituent group a, or a C₃₋₆ cycloalkyl group optionally substituted by 1 to 3 substituents selected from substituent group a, or R^{2a} and R^{2b} are bonded together to form, together with the carbon atom to which R^{2a} and R^{2b} are bonded, a C₃₋₈ cycloalkane optionally substituted by 1 to 3 substituents selected from substituent group b, or a 3- to 8-membered saturated oxygen-containing heterocycle,
   Z¹ is -CH-, -CR³- or a nitrogen atom,
   R³ is a halogen atom, a hydroxy group, an amino group optionally substituted by one or two C₁₋₆ alkyl groups, a C₁₋₆ alkyl group optionally substituted by 1 to 3 substituents selected from substituent group a, or a C₁₋₆ alkoxy group optionally substituted by 1 to 3 substituents selected from substituent group a,
   Z² is -CH-, -CR⁴- or a nitrogen atom,
   R⁴ is a halogen atom, a hydroxy group, an amino group optionally substituted by one or two C₁₋₆ alkyl groups, a C₁₋₆ alkyl group optionally substituted by 1 to 3 substituents selected from substituent group a, or a C₁₋₆ alkoxy group optionally substituted by 1 to 3 substituents selected from substituent group a,
   R¹¹ in the number of n are each independently a halogen atom, a hydroxy group, an amino group optionally substituted by one or two C₁₋₆ alkyl groups, a C₁₋₆ alkyl group optionally substituted by 1 to 3 substituents selected from substituent group a, or a C₁₋₆ alkoxy group optionally substituted by 1 to 3 substituents selected from substituent group a,
   n is an integer of 0 to 2,
   R^{8a} and R^{8b} are each independently a hydrogen atom, a halogen atom, a cyano group, a C₁₋₆ alkyl group or a C₁₋₆ alkoxy group, R^{9a} and R^{9b} are each independently a hydrogen atom or a C₁₋₆ alkyl group,
   W is -CH₂-, -CHR¹⁰- or an oxygen atom,
   R¹⁰ is a C₁₋₆ alkyl group, and
   X is a group represented by the following formula (A1) or (A2): 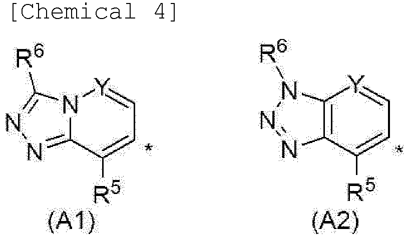 wherein
      * is the bonding position to the carbon atom to which X is bonded,
      R⁵ is a hydrogen atom, a halogen atom, or a C₁₋₆ alkyl group optionally substituted by 1 to 3 substituents selected from substituent group a,
      R⁶ is a hydrogen atom, or a C₁₋₆ alkyl group optionally substituted by 1 to 3 substituents selected from substituent group a,
      Y is -CH-, -CR ⁷- or a nitrogen atom, and
      R⁷ is a hydroxy group, a halogen atom, a cyano group, a C₁₋₆ alkyl group optionally substituted by 1 to 3 substituents selected from substituent group a, a C₁₋₆ alkoxy group optionally substituted by 1 to 3 substituents selected from substituent group a, or a C₃₋₆ cycloalkyl group,
      provided that when Z¹ is -CH- or -CR³-, then Z² is a nitrogen atom, when Z² is -CH- or -CR⁴-, then Z¹ is a nitrogen atom, and both Z¹ and Z² cannot simultaneously be -CH-, -CR³- or -CR⁴-,
      substituent group a:
         a hydroxy group,
         a halogen atom,
         a cyano group,
         a C₁₋₆ alkyl group,
         a C₁₋₆ alkoxy group,
         a C₁₋₆ alkyl group substituted by substituent group b,
         a C₁₋₆ alkoxy group substituted by substituent group b,
         an amino group optionally substituted by one or two C₁₋₆ alkyl groups,
         a C₁₋₆ alkylsulfonyl group
         substituent group b:
            a halogen atom,
            a cyano group,
            a C₁₋₆ alkyl group,
            a C₁₋₆ alkoxy group,
            or a pharmaceutically acceptable salt thereof.
[2] The compound of the above-mentioned [1], wherein R^{1a} and R^{1b} are each independently a hydrogen atom or a methyl group, or a pharmaceutically acceptable salt thereof.
[3] The compound of the above-mentioned [1] or [2], wherein R^{2a} and R^{2b} are each independently a hydrogen atom, a C₁₋₆ alkyl group, a C₁₋₆ haloalkyl group or a C₃₋₆ cycloalkyl group, or R^{2a} and R^{2b} are bonded together to form, together with the carbon atom to which R^{2a} and R^{2b} are bonded, a C₃₋₆ cycloalkane, or a pharmaceutically acceptable salt thereof.
[4] The compound of the above-mentioned [1] or [2], wherein R^{2a} and R^{2b} are each independently a hydrogen atom, a methyl group, an ethyl group, a trifluoromethyl group or a difluoroethyl group, or
   R^{2a} and R^{2b} are bonded together to form, together with the carbon atom to which R^{2a} and R^{2b} are bonded, a cyclopropane, or a pharmaceutically acceptable salt thereof.
[5] The compound of any of the above-mentioned [1] to [4], wherein Z¹ is -CH- or -CR³-, and
   Z² is a nitrogen atom, or a pharmaceutically acceptable salt thereof.
[6] The compound of any of the above-mentioned [1] to [4], wherein Z¹ is -CR³-, and
   Z² is a nitrogen atom, or a pharmaceutically acceptable salt thereof.
[7] The compound of the above-mentioned [5] or [6], wherein R³ is a halogen atom, a hydroxy group, an amino group, a C₁₋₆ alkylamino group or a C₁₋₆ alkyl group, or a pharmaceutically acceptable salt thereof.
[8] The compound of the above-mentioned [5] or [6], wherein R³ is a hydroxy group, or a pharmaceutically acceptable salt thereof.
[9] The compound of any of the above-mentioned [1] to [8], wherein n is 1, and
   R¹¹ is a hydroxy group, or a pharmaceutically acceptable salt thereof.
[10] The compound of any of the above-mentioned [1] to [8], wherein n is 0, or a pharmaceutically acceptable salt thereof.
[11] The compound of any of the above-mentioned [1] to [10], wherein R^{8a} and R^{8b} are both hydrogen atoms, or a pharmaceutically acceptable salt thereof.
[12] The compound of any of the above-mentioned [1] to [11], wherein R^{9a} and R^{9b} are both hydrogen atoms, or a pharmaceutically acceptable salt thereof.
[13] The compound of any of the above-mentioned [1] to [12], wherein W is an oxygen atom, or a pharmaceutically acceptable salt thereof.
[14] The compound of any of the above-mentioned [1] to [13], wherein X is a group represented by the following formula (A2): 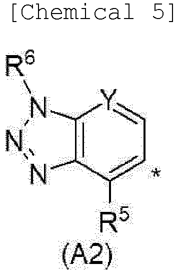
   wherein each symbol is as defined above,
   or a pharmaceutically acceptable salt thereof.
[15] The compound of the above-mentioned [14], wherein R⁵ is a halogen atom or a C₁₋₆ alkyl group, or a pharmaceutically acceptable salt thereof.
[16] The compound of the above-mentioned [14], wherein R⁵ is a methyl group, or a pharmaceutically acceptable salt thereof.
[17] The compound of any of the above-mentioned [14] to [16], wherein R⁶ is a methyl group, an ethyl group, a dimethylaminoethyl group, a 2,2,2-trifluoroethyl group, a 2-hydroxy-2-methylpropyl group or a 3-methanesulfonylpropyl group, or a pharmaceutically acceptable salt thereof.
[18] The compound of any of the above-mentioned [14] to [16], wherein R⁶ is a methyl group, or a pharmaceutically acceptable salt thereof.
[19] The compound of any of the above-mentioned [1] to [18], wherein R⁷ is a chlorine atom, a cyano group, a methyl group, a cyclopropyl group, a trifluoromethyl group, a methoxy group, a difluoromethoxy group or a trifluoromethoxy group, or a pharmaceutically acceptable salt thereof.
[20] Any compound selected from the following group or a pharmaceutically acceptable salt thereof:
   (3S)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid,
   (3R)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid,
   (3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}propanoic acid,
   (3R)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}propanoic acid,
   (3R)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
   (3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
   (3S)-3-(7-{[(2S)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
   (3R)-3-(7-{[(2S)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
   (3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid,
   (3R)-3-(7-{[(2R)-2-cyclopropyl-7-hydroxy-2,3-dihydropyrido[2,3-f][1,4]oxazepin-4(5H)-yl]methyl}-1-benzothiophen-5-yl)-3-(3,7-dimethyl-3H-[1,2,3]triazolo[4,5-b]pyridin-6-yl)propanoic acid,
   (3S)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoic acid,
   (3R)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoic acid,
   (3S)-3-(7-{[(2R)-2-cyclopropyl-7-hydroxy-2,3-dihydropyrido[2,3-f][1,4]oxazepin-4(5H)-yl]methyl}-1-benzothiophen-5-yl)-3-(1,4-dimethyl-1H-benzotriazol-5-yl)-2,2-dimethylpropanoic acid, and
   (3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}-2,2-dimethylpropanoic acid.
[21] Any compound selected from the following group or a pharmaceutically acceptable salt thereof:
   (3R)-3-(7-{[(2R)-2-cyclopropyl-7-hydroxy-2,3-dihydropyrido[2,3-f][1,4]oxazepin-4(5H)-yl]methyl}-1-benzothiophen-5-yl)-3-(1,4-dimethyl-1H-benzotriazol-5-yl)propanoic acid,
   (3S)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoic acid, and
   (3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}-2,2-dimethylpropanoic acid.
[22] (3R)-3-(7-{[(2R)-2-cyclopropyl-7-hydroxy-2,3-dihydropyrido[2,3-f][1,4]oxazepin-4(5H)-yl]methyl}-1-benzothiophen-5-yl)-3-(1,4-dimethyl-1H-benzotriazol-5-yl)propanoic acid or a pharmaceutically acceptable salt thereof.
[23] (3S)-3-(1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)-2,2-dimethylpropanoic acid or a pharmaceutically acceptable salt thereof.
[24] (3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoic acid or a pharmaceutically acceptable salt thereof.
[25] A medicament comprising the compound of any of the above-mentioned [1] to [24] or a pharmaceutically acceptable salt thereof as an active ingredient (hereinafter sometimes abbreviated as "the medicament of the present invention").
[26] The medicament of the above-mentioned [25], for activating Nrf2.
[27] The medicament of the above-mentioned [25], for inhibiting a protein-protein interaction between Keap1 and Nrf2.
[28] The medicament of the above-mentioned [25], for the prophylaxis and/or treatment of an oxidative stress-related disease.
[29] The medicament of the above-mentioned [28], wherein the oxidative stress-related disease is selected from the group consisting of renal diseases, liver diseases, respiratory diseases, dermatic diseases, cardiovascular diseases, central nervous system diseases, autoimmune diseases and ophthalmic diseases.
[30] The medicament of the above-mentioned [25], for the prophylaxis and/or treatment of a disease selected from the group consisting of a renal disease selected from the group consisting of chronic kidney disease, acute nephritis, chronic nephritis, acute renal failure, chronic renal failure, nephrotic syndrome, IgA nephropathy, diabetic nephropathy, gouty kidney, nephrosclerosis, hydronephrosis and tubulointerstitial nephritis; a liver disease selected from the group consisting of alcoholic fatty liver, non-alcoholic steatohepatitis, hepatic fibrosis and cirrhosis; a respiratory disease selected from the group consisting of bronchitis, pneumonia, pleurisy, chronic obstructive pulmonary diseases, acute lung disorder, diffuse panbronchiolitis, interstitial pneumonia and asthma; a dermatic disease selected from the group consisting of UV and radiation skin disorder, radiation mucosal disorder, epidermolysis blister syndrome, psoriasis, atopic dermatitis and scleroderma; a cardiovascular disease selected from the group consisting of cardiac failure, myocardial infarction, arteriosclerosis and pulmonary arterial hypertension; a central nervous system disease selected from the group consisting of Alzheimer's disease, Parkinson's disease, Huntington's disease, amyotrophic lateral sclerosis, cerebral infarction, polyglutamine disease and autism; a mitochondrial disease selected from the group consisting of Friedreich's ataxia and mitochondrial myopathy; an autoimmune disease selected from the group consisting of multiple sclerosis, rheumatoid arthritis, systemic lupus erythematosus, Sjogren syndrome, type 1 diabetes, ulcerative colitis and Crohn's disease; and an ophthalmic disease selected from the group consisting of allergic conjunctival diseases, viral conjunctivitis, pterygium, cornea infectious disease, dry eye, corneal disorders, uveitis, Behcet's disease, diabetic retinopathy, retinal detachment, retinal vein occlusion, central serous chorioretinopathy, age-related macular degeneration, diabetic macular edema, macular disease, retinitis pigmentosa, glaucoma and cataract.
[31] The medicament of the above-mentioned [25], for the prophylaxis and/or treatment of a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, and age-related macular degeneration
[32] The compound of any of the above-mentioned [1] to [24] or a salt thereof, for use in the prophylaxis and/or treatment of a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, and age-related macular degeneration.
[33] The medicament of the above-mentioned [25], for use in the prophylaxis and/or treatment of a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, and age-related macular degeneration.
[34] Use of the compound of any of the above-mentioned [1] to [24] or a pharmaceutically acceptable salt thereof in producing a prophylactic and/or therapeutic agent for a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, and age-related macular degeneration.
[35] A method for the prophylaxis and/or treatment of a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, and age-related macular degeneration in a mammal, comprising administering a pharmaceutically effective amount of the compound of any of the above-mentioned [1] to [24] or a pharmaceutically acceptable salt thereof to the mammal.
[36] A method for activating Nrf2 in a mammal, comprising administering a pharmaceutically effective amount of the compound of any of the above-mentioned [1] to [24] or a pharmaceutically acceptable salt thereof to the mammal.
[37] A method for inhibiting protein-protein interaction between Keap1 and Nrf2 in a mammal, comprising administering a pharmaceutically effective amount of the compound of any of the above-mentioned [1] to [24] or a pharmaceutically acceptable salt thereof to the mammal.
[38] An Nrf2 activator comprising the compound of any of the above-mentioned [1] to [24] or a pharmaceutically acceptable salt thereof as an active ingredient.
[39] An inhibitor of protein-protein interaction between Keap1 and Nrf2, comprising the compound of any of the above-mentioned [1] to [24] or a pharmaceutically acceptable salt thereof as an active ingredient.
[40] The compound of any of the above-mentioned [1] to [24] or a salt thereof, for use in the prophylaxis and/or treatment of a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, age-related macular degeneration, retinitis pigmentosa, and glaucoma.
[41] The medicament of the above-mentioned [25], for use in the prophylaxis and/or treatment of a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, age-related macular degeneration, retinitis pigmentosa, and glaucoma.
[42] The medicament of any of the above-mentioned [25] to [31], [33] and [41], which is administered in combination with other medicament.
[43] The medicament of the above-mentioned [42], wherein said other medicament is a prophylactic agent and/or a therapeutic agent for a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, age-related macular degeneration, retinitis pigmentosa, and glaucoma.
[44] The medicament of the above-mentioned [42] or [43], wherein the medicament of any of the above-mentioned [25] to [31], [33] and [41] and other medicament are separately contained as active ingredients of different preparations, and administered simultaneously or at different times.
[45] The medicament of the above-mentioned [42] or [43], wherein the compound of any of the above-mentioned [1] to [24] or a pharmaceutically acceptable salt thereof and other medicament are contained in a single preparation.
[46] A pharmaceutical composition comprising the compound of any of the above-mentioned [1] to [24] or a pharmaceutically acceptable salt thereof, and a pharmaceutically acceptable carrier (hereinafter sometimes abbreviated as "the pharmaceutical composition of the present invention").
[47] A method for producing the compound of any of the above-mentioned [1] to [24] or a pharmaceutically acceptable salt thereof.
[48] A prodrug of the compound of any of the above-mentioned [1] to [24] or a pharmaceutically acceptable salt thereof.

### [Advantageous Effects of Invention]

The compound (1) of the present invention or a pharmaceutically acceptable salt thereof shows an action to effectively activate Nrf2 by inhibiting the protein-protein interaction between Keap1 and Nrf2. That is, a medicament containing the compound (1) of the present invention or a pharmaceutically acceptable salt thereof as an active ingredient can be used for the prophylaxis and/or treatment of diseases whose symptoms are improved by activating Nrf2 when administered to mammals. Examples of the diseases whose symptoms are improved by activation of Nrf2 include oxidative stress-related diseases, specifically, for example, a disease selected from the group consisting of a renal disease selected from the group consisting of chronic kidney disease, acute nephritis, chronic nephritis, acute renal failure, chronic renal failure, nephrotic syndrome, IgA nephropathy, diabetic nephropathy, gouty kidney, nephrosclerosis, hydronephrosis and tubulointerstitial nephritis; a liver disease selected from the group consisting of alcoholic fatty liver, non-alcoholic steatohepatitis, hepatic fibrosis and cirrhosis; a respiratory disease selected from the group consisting of bronchitis, pneumonia, pleurisy, chronic obstructive pulmonary diseases, acute lung disorder, diffuse panbronchiolitis, interstitial pneumonia and asthma; a dermatic disease selected from the group consisting of UV and radiation skin disorder, radiation mucosal disorder, epidermolysis blister syndrome, psoriasis, atopic dermatitis and scleroderma; a cardiovascular disease selected from the group consisting of cardiac failure, myocardial infarction, arteriosclerosis and pulmonary arterial hypertension; a central nervous system disease selected from the group consisting of Alzheimer's disease, Parkinson's disease, Huntington's disease, amyotrophic lateral sclerosis, cerebral infarction, polyglutamine disease and autism; a mitochondrial disease selected from the group consisting of Friedreich's ataxia and mitochondrial myopathy; an autoimmune disease selected from the group consisting of multiple sclerosis, rheumatoid arthritis, systemic lupus erythematosus, Sjogren syndrome, type 1 diabetes, ulcerative colitis and Crohn's disease; and an ophthalmic disease selected from the group consisting of allergic conjunctival diseases, viral conjunctivitis, pterygium, cornea infectious disease, dry eye, corneal disorders, uveitis, Behcet's disease, diabetic retinopathy, retinal detachment, retinal vein occlusion, central serous chorioretinopathy, age-related macular degeneration, diabetic macular edema, macular disease, retinitis pigmentosa, glaucoma and cataract, and the like. Among these, a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, age-related macular degeneration, retinitis pigmentosa, and glaucoma can be preferably mentioned.

### [Description of Embodiments]

The definitions of the terms and symbols used in the present specification are explained below. Unless otherwise defined, all technical and scientific terms used herein have the same meaning as generally understood by those skilled in the art to which the present invention belongs.

In the present specification, the "halogen atom" means a fluorine atom, a chlorine atom, a bromine atom or an iodine atom.

In the present specification, the "C₁₋₆ alkyl group" means a linear or branched alkyl group having 1 to 6 carbon atoms. Examples of the C₁₋₆ alkyl group include methyl group, ethyl group, n-propyl group, isopropyl group, n-butyl group, sec-butyl group, tert-butyl group, n-pentyl group, isopentyl group, 2-methylbutyl group, neopentyl group, 1-ethylpropyl group, n-hexyl group, 4-methylpentyl group, 3-methylpentyl group, 2-methylpentyl group, 1-methylpentyl group, 3,3-dimethylbutyl group, 2,2-dimethylbutyl group, 1,1-dimethylbutyl group, 1,2-dimethylbutyl group, 1,3-dimethylbutyl group, 2,3-dimethylbutyl group, 2-ethylbutyl group and the like.

In the present specification, the "C₁₋₆ haloalkyl group" means a group in which one or more hydrogen atoms in the aforementioned "C₁₋₆ alkyl group" are substituted by a halogen. Examples of the C₁₋₆ haloalkyl group include fluoromethyl, difluoromethyl, trifluoromethyl, 2-chloroethyl, 2-bromoethyl, 2-iodoethyl, 2-fluoroethyl, difluoroethyl (e.g., 1,1-difluoroethyl, 2,2-difluoroethyl), 2,2,2-trifluoroethyl, pentafluoroethyl, 2,2,3,3-tetrafluoropropyl, 3,3,3-trifluoropropyl, 4,4,4-trifluorobutyl, 5,5,5-trifluoropentyl, 6,6,6-trifluorohexyl and the like.

In the present specification, the "C₁₋₆ alkoxy group" means a group in which the aforementioned "C₁₋₆ alkyl group" is bonded to an oxygen atom. Examples of the C₁₋₆ alkoxy group include methoxy group, ethoxy group, n-propoxy group, isopropoxy group, n-butoxy group, sec-butoxy group, tert-butoxy group, n-pentoxy group, isopentoxy group, 2-methylbutoxy group, n-hexyloxy group and the like.

In the present specification, the "C₁₋₆ haloalkoxy group" means a group in which one or more hydrogen atoms in the aforementioned "C₁₋₆ alkoxy group" are substituted by a halogen. Examples of the C₁₋₆ haloalkoxy group include fluoromethoxy, difluoromethoxy, trifluoromethoxy, 2-chloroethoxy, 2-bromoethoxy, 2-iodoethoxy, 2-fluoroethoxy, 2,2-difluoroethoxy, 2,2,2-trifluoroethoxy, pentafluoroethoxy, 2,2,3,3-tetrafluoropropoxy, 3,3,3-trifluoropropoxy, 4,4,4-trifluorobutoxy, 5,5,5-trifluoropentyloxy, 6,6,6-trifluorohexyloxy and the like.

In the present specification, the "C₃₋₆ cycloalkyl group" means a 3- to 6-membered monocyclic saturated hydrocarbocyclic group and, for example, a cyclopropyl group, cyclobutyl group, cyclopentyl group, and cyclohexyl group can be mentioned.

In the present specification, the "C₁₋₆ alkylsulfonyl group" means a group in which the aforementioned "C₁₋₆ alkyl group" is bonded to the sulfur atom of the sulfonyl group. Examples of the C₁₋₆ alkylsulfonyl group include methylsulfonyl group, ethylsulfonyl group, n-propylsulfonyl group, isopropylsulfonyl group, n-butylsulfonyl group, sec-butylsulfonyl group, tert-butylsulfonyl group, n-pentylsulfonyl group and the like.

In the present specification, the "amino group optionally substituted by 1 or 2 C₁₋₆ alkyl groups" means an unsubstituted amino group, or a group in which one or two hydrogen atoms of an amino group are each independently substituted by the aforementioned "C₁₋₆ alkyl group", that is, a C₁₋₆ alkylamino group or a di C₁₋₆ alkylamino group. As an amino group optionally substituted by 1 or 2 C₁₋₆ alkyl groups, amino group, methylamino group, dimethylamino group, ethylamino group, diethylamino group, ethyl(methyl)amino group, n-propylamino group, di(n-propyl)amino group, isopropylamino group, n-butylamino group, di(n-butyl)amino group, sec-butylamino group, tert-butylamino group, n-pentylamino group, n-hexylamino group and the like can be mentioned.

In the present specification, the "C₃₋₈ cycloalkane" means a 3- to 8-membered monocyclic saturated hydrocarbocycle and, for example, cyclopropane, cyclobutane, cyclopentane, cyclohexane, cycloheptane and cyclooctane can be mentioned. The "C₃₋₈ cycloalkane" is preferably cyclopropane or cyclobutane.

In the present specification, the "3- to 8-membered saturated oxygen-containing heterocycle" means a 3- to 8-membered monocyclic saturated oxygen-containing heterocycle and, for example, oxirane, oxetane, tetrahydrofuran, tetrahydropyran, oxepane, oxocane and the like can be mentioned. The "3- to 8-membered saturated oxygen-containing heterocycle" is preferably oxetane or tetrahydropyran.

In the present specification, the "optionally substituted" means that it is unsubstituted or substituted by a specific number of specific substituents at any substitutable position (any hydrogen atom is replaced with a substituent). The "substituent" may be a substituent selected from the group consisting of "substituent group a" and "substituent group b" below. When multiple substituents are present, each substituent may be the same or different.

substituent group a:
   a hydroxy group,
   a halogen atom,
   a cyano group,
   a C₁₋₆ alkyl group optionally substituted by 1 to 3 substituents selected from substituent group b,
   a C₁₋₆ alkoxy group optionally substituted by 1 to 3 substituents selected from substituent group b,
   a C₁₋₆ alkylsulfonyl group optionally substituted by 1 to 3 substituents selected from substituent group b,
   an amino group optionally substituted by 1 or 2 C₁₋₆ alkyl groups.
substituent group b:
   a halogen atom,
   a cyano group,
   a C₁₋₆ alkyl group,
   a C₁₋₆ alkoxy group

However, when an optional substituent of the "optionally substituted C₁₋₆ alkyl group" or the "optionally substituted C₁₋₆ alkoxy group" is selected from the aforementioned substituent group a or substituent group b, the list of the aforementioned substituent group a or substituent group b does not include the "C₁₋₆ alkyl group".

In the present specification, the "pharmaceutically acceptable salt" means a salt that can be used as a medicament, and includes both pharmaceutically acceptable acid addition salts and pharmaceutically acceptable base addition salts.

In the present specification, the "pharmaceutically acceptable carrier" means a pharmaceutically acceptable material (e.g., excipient, diluent, additive, solvent, etc.) involved in transporting compound (1) of the present invention or a composition containing the same from one organ to another organ.

In the present specification, "treatment" and its derivatives mean, in a patient who has developed a disease, illness, disorder, etc. (hereinafter referred to as "disease, and the like"), the remission, alleviation, or delayed aggravation of the clinical symptoms of said disease, and the like.

In the present specification, "prophylaxis" and its derivatives mean to inhibit, deter, control, slow down, or stop the onset of clinical symptoms of a disease, and the like in mammals that are likely to develop the disease, and the like but have not yet done so, or are concerned about the recurrence of the disease, and the like after treatment of the disease, and the like.

In the present specification, the "oxidative stress" means a state in which the production of reactive oxygen species is excessive due to external factors (e.g., ultraviolet rays, radiation, air pollution, tobacco, drugs, intake of oxidized substances, etc.) and the balance of antioxidant defense mechanisms is upset. In addition, "oxidative stress-related diseases" means diseases in which such oxidative stress is involved in the onset or worsening of symptoms. Such "oxidative stress-related diseases" include, for example, a disease selected from the group consisting of a renal disease selected from the group consisting of chronic kidney disease, acute nephritis, chronic nephritis, acute renal failure, chronic renal failure, nephrotic syndrome, IgA nephropathy, diabetic nephropathy, gouty kidney, nephrosclerosis, hydronephrosis and tubulointerstitial nephritis; a liver disease selected from the group consisting of alcoholic fatty liver, non-alcoholic steatohepatitis, hepatic fibrosis and cirrhosis; a respiratory disease selected from the group consisting of bronchitis, pneumonia, pleurisy, chronic obstructive pulmonary diseases, acute lung disorder, diffuse panbronchiolitis, interstitial pneumonia and asthma; a dermatic disease selected from the group consisting of UV and radiation skin disorder, radiation mucosal disorder, epidermolysis blister syndrome, psoriasis, atopic dermatitis and scleroderma; a cardiovascular disease selected from the group consisting of cardiac failure, myocardial infarction, arteriosclerosis and pulmonary arterial hypertension; a central nervous system disease selected from the group consisting of Alzheimer's disease, Parkinson's disease, Huntington's disease, amyotrophic lateral sclerosis, cerebral infarction, polyglutamine disease and autism; a mitochondrial disease selected from the group consisting of Friedreich's ataxia and mitochondrial myopathy; an autoimmune disease selected from the group consisting of multiple sclerosis, rheumatoid arthritis, systemic lupus erythematosus, Sjogren syndrome, type 1 diabetes, ulcerative colitis and Crohn's disease; and an ophthalmic disease selected from the group consisting of allergic conjunctival diseases, viral conjunctivitis, pterygium, cornea infectious disease, dry eye, corneal disorders, uveitis, Behcet's disease, diabetic retinopathy, retinal detachment, retinal vein occlusion, central serous chorioretinopathy, age-related macular degeneration, diabetic macular edema, macular disease, retinitis pigmentosa, glaucoma and cataract and the like. The "oxidative stress-related disease" in the present invention is, in particular, a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, age-related macular degeneration, retinitis pigmentosa and glaucoma.

Generally, Keap1 and Nrf2 form a complex and the function of Nrf2 is inhibited by ubiquitination by E3 ubiquitin ligase. In the present specification, the "inhibitor of protein-protein interaction between Keap1 and Nrf2" means a substance that inhibits the formation of the complex and releases Nrf2.

In the present specification, the "activating Nrf2" or "Nrf2 activator" means a substance that inhibits the protein-protein interaction between Keap1 and Nrf2, thereby preventing the formation of a complex between Keap1 and Nrf2, and allows the liberated Nrf2 to transfer into the nucleus and promote the expression of antioxidant genes, or that induces high expression of antioxidant genes.

In the present specification, the "pharmaceutically effective amount" means the dose of the compound (1) of the present invention or a pharmaceutically acceptable salt thereof to be orally or parenterally (topically, rectally, intravenously, intramuscularly, subcutaneously, etc.) administered to a mammal.

In the present specification, the "mammal" is not particularly limited, and human and mammals other than human (e.g., mouse, rat, hamster, guinea pig, rabbit, cat, dog, swine, bovine, horse, sheep, monkey, etc.) can be mentioned.

### (Compound of the present invention (compound (1)))

Each group in the aforementioned formula (1) of compound (1) is explained in the following.

R^{1a} and R^{1b} are each independently a hydrogen atom or a C₁₋₆ alkyl group.

Preferably, R^{1a} and R^{1b} are each independently a hydrogen atom or a methyl group.

R^{2a} and R^{2b} are each independently a hydrogen atom, a C₁₋₆ alkyl group optionally substituted by 1 to 3 substituents selected from the aforementioned substituent group a, or a C₃₋₆ cycloalkyl group, or
R^{2a} and R^{2b} are bonded together to form, together with the carbon atom to which R^{2a} and R^{2b} are bonded, a C₃₋₈ cycloalkane which is optionally substituted by 1 to 3 substituents selected from the aforementioned substituent group b, or a 3- to 8-membered saturated oxygen-containing heterocycle.

R^{2a} and R^{2b} are preferably each independently a hydrogen atom, a C₁₋₆ alkyl group, a C₁₋₆ haloalkyl group or a C₃₋₆ cycloalkyl group, or
R^{2a} and R^{2b} are bonded together to form, together with the carbon atom to which R^{2a} and R^{2b} are bonded, a C₃₋₆ cycloalkane, more preferably, R^{2a} and R^{2b} are each independently a hydrogen atom, a methyl group, an ethyl group, a trifluoromethyl group or a difluoroethyl group, or
R^{2a} and R^{2b} are bonded together to form, together with the carbon atom to which R^{2a} and R^{2b} are bonded, a cyclopropane.

Z¹ is -CH-, -CR³- (R³ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom.

Z¹ is preferably -CH- or -CR³- (R³ is a halogen atom, a hydroxy group, an amino group, a C₁₋₆ alkylamino group or a C₁₋₆ alkyl group), more preferably, -CR³- (R³ is a hydroxy group).

Z² is -CH-, -CR⁴- (R⁴ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom.

Z² is preferably a nitrogen atom.

Provided that when Z¹ is -CH- or -CR³-, then Z² is a nitrogen atom, when Z² is -CH- or -CR⁴-, then Z¹ is a nitrogen atom, and both Z¹ and Z² cannot simultaneously be -CH-, -CR³- or -CR⁴- .

R¹¹ in the number of n are each independently a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a.

R¹¹ is preferably a hydroxy group.

n is an integer of 0 to 2.

n is preferably 0 or 1, more preferably 0.

R^{8a} and R^{8b} are each independently a hydrogen atom, a halogen atom, a cyano group, a C₁₋₆ alkyl group or a C₁₋₆ alkoxy group.

R^{8a} and R^{8b} are preferably both hydrogen atoms.

R^{9a} and R^{9b} are each independently a hydrogen atom or a C₁₋₆ alkyl group.

R^{9a} and R^{9b} are preferably both hydrogen atoms.

W is -CH₂-, -CHR¹⁰- (R¹⁰ is a C₁₋₆ alkyl group) or an oxygen atom.

W is preferably an oxygen atom.

X is a group represented by the following formula (A1) or (A2): 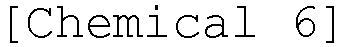 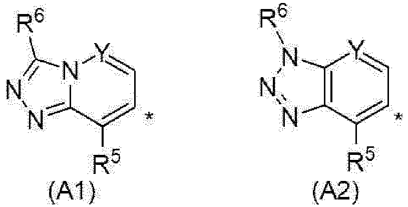 wherein
* is the bonding position to the carbon atom to which X is bonded,
R⁵ is a hydrogen atom, a halogen atom, or a C₁₋₆ alkyl group optionally substituted by 1 to 3 substituents selected from the aforementioned substituent group a,
R⁶ is a hydrogen atom, or a C₁₋₆ alkyl group optionally substituted by 1 to 3 substituents selected from the aforementioned substituent group a,
Y is -CH-, -CR⁷- or a nitrogen atom, and
R⁷ is a hydroxy group, a halogen atom, a cyano group, a C₁₋₆ alkyl group optionally substituted by 1 to 3 substituents selected from the aforementioned substituent group a, a C₁₋₆ alkoxy group optionally substituted by 1 to 3 substituents selected from the aforementioned substituent group a, or a C₃₋₆ cycloalkyl group.

X is preferably a group represented by the aforementioned formula (A1) or (A2), wherein
* is the bonding position to the carbon atom to which X is bonded,
R⁵ is a halogen atom or a C₁₋₆ alkyl group,
R⁶ is a methyl group, an ethyl group, a dimethylaminoethyl group, a 2,2,2-trifluoroethyl group, a 2-hydroxy-2-methylpropyl group or a 3-methanesulfonylpropyl group,
Y is -CH-, -CR⁷- or a nitrogen atom, and
R⁷ is a chlorine atom, a cyano group, a methyl group, a cyclopropyl group, a trifluoromethyl group, a methoxy group, a difluoromethoxy group or a trifluoromethoxy group.

X is more preferably a group represented by the aforementioned formula (A1) or (A2), wherein
* is the bonding position to the carbon atom to which X is bonded,
R⁵ is a methyl group,
R⁶ is a methyl group,
Y is -CH-, -CR⁷- or a nitrogen atom, and
R⁷ is a chlorine atom, a cyano group, a methyl group, a cyclopropyl group, a trifluoromethyl group, a methoxy group, a difluoromethoxy group or a trifluoromethoxy group.

X is further preferably a group represented by the aforementioned formula (A2), wherein
* is the bonding position to the carbon atom to which X is bonded,
R⁵ is a halogen atom or a C₁₋₆ alkyl group,
R⁶ is a methyl group, an ethyl group, a dimethylaminoethyl group, a 2,2,2-trifluoroethyl group, a 2-hydroxy-2-methylpropyl group or a 3-methanesulfonylpropyl group,
Y is -CH-, -CR⁷- or a nitrogen atom, and
R⁷ is a chlorine atom, a cyano group, a methyl group, a cyclopropyl group, a trifluoromethyl group, a methoxy group, a difluoromethoxy group or a trifluoromethoxy group.

X is particularly preferably a group represented by the aforementioned formula (A2), wherein
* is the bonding position to the carbon atom to which X is bonded,
R⁵ is a methyl group,
R⁶ is a methyl group,
Y is -CH-, -CR⁷- or a nitrogen atom, and
R⁷ is a chlorine atom, a cyano group, a methyl group, a cyclopropyl group, a trifluoromethyl group, a methoxy group, a difluoromethoxy group or a trifluoromethoxy group.

As compound (1), the following compounds are preferred.

### [Compound (1A)]

Compound (1) wherein
R^{1a} and R^{1b} are each independently a hydrogen atom or a methyl group;
R^{2a} and R^{2b} are each independently a hydrogen atom, a C₁₋₆ alkyl group optionally substituted by 1 to 3 substituents selected from the aforementioned substituent group a, or a C₃₋₆ cycloalkyl group, or
R^{2a} and R^{2b} are bonded together to form, together with the carbon atom to which R^{2a} and R^{2b} are bonded, a C₃₋₈ cycloalkane which is optionally substituted by 1 to 3 substituents selected from the aforementioned substituent group b, or a 3- to 8-membered saturated oxygen-containing heterocycle;
Z¹ is -CH-, -CR³- (R³ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
Z² is -CH-, -CR⁴- (R⁴ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
R¹¹ in the number of n are each independently a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a;
n is an integer of 0 to 2;
R^{8a} and R^{8b} are each independently a hydrogen atom, a halogen atom, a cyano group, a C₁₋₆ alkyl group or a C₁₋₆ alkoxy group; R^{9a} and R^{9b} are each independently a hydrogen atom or a C₁₋₆ alkyl group;
W is -CH₂-, -CHR¹⁰- (R¹⁰ is a C₁₋₆ alkyl group) or an oxygen atom; and
X is a group represented by the following formula (A1) or (A2): 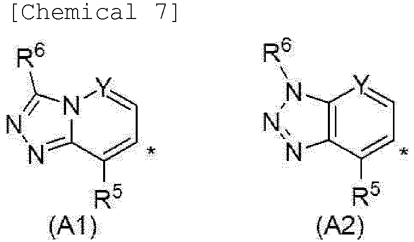 wherein
   * is the bonding position to the carbon atom to which X is bonded,
   R⁵ is a hydrogen atom, a halogen atom, or a C₁₋₆ alkyl group optionally substituted by 1 to 3 substituents selected from the aforementioned substituent group a (preferably, a halogen atom or a C₁₋₆ alkyl group, more preferably, a methyl group),
   R⁶ is a hydrogen atom, or a C₁₋₆ alkyl group optionally substituted by 1 to 3 substituents selected from the aforementioned substituent group a (preferably, a methyl group, an ethyl group, a dimethylaminoethyl group, a 2,2,2-trifluoroethyl group, a 2-hydroxy-2-methylpropyl group or a 3-methanesulfonylpropyl group, more preferably, a methyl group), Y is -CH-, -CR ⁷- or a nitrogen atom, and
   R⁷ is a hydroxy group, a halogen atom, a cyano group, a C₁₋₆ alkyl group optionally substituted by 1 to 3 substituents selected from the aforementioned substituent group a, a C₁₋₆ alkoxy group optionally substituted by 1 to 3 substituents selected from the aforementioned substituent group a, or a C₃₋₆ cycloalkyl group (preferably, a chlorine atom, a cyano group, a methyl group, a cyclopropyl group, a trifluoromethyl group, a methoxy group, a difluoromethoxy group or a trifluoromethoxy group),
      provided that when Z¹ is -CH- or -CR³-, then Z² is a nitrogen atom, when Z² is -CH- or -CR⁴·-, then Z¹ is a nitrogen atom, and both Z¹ and Z² cannot simultaneously be -CH-, -CR³- or -CR⁴-,
   or a pharmaceutically acceptable salt thereof.

### [compound (1B)]

Compound (1) wherein
R^{1a} and R^{1b} are each independently a hydrogen atom or a C₁₋₆ alkyl group;
R^{2a} and R^{2b} are each independently a hydrogen atom, a C₁₋₆ alkyl group, a C₁₋₆ haloalkyl group or a C₃₋₆ cycloalkyl group, or R^{2a} and R^{2b} are bonded together to form, together with the carbon atom to which R^{2a} and R^{2b} are bonded, a C₃₋₆ cycloalkane (preferably, R^{2a} and R^{2b} are each independently a hydrogen atom, a methyl group, an ethyl group, a trifluoromethyl group or a difluoroethyl group, or
R^{2a} and R^{2b} are bonded together to form, together with the carbon atom to which R^{2a} and R^{2b} are bonded, a cyclopropane);
Z¹ is -CH-, -CR³- (R³ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
Z² is -CH-, -CR⁴- (R⁴ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
R¹¹ in the number of n are each independently a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a;
n is an integer of 0 to 2;
R^{8a} and R^{8b} are each independently a hydrogen atom, a halogen atom, a cyano group, a C₁₋₆ alkyl group or a C₁₋₆ alkoxy group; R^{9a} and R^{9b} are each independently a hydrogen atom or a C₁₋₆ alkyl group;
W is -CH₂-, -CHR¹⁰- (R¹⁰ is a C₁₋₆ alkyl group) or an oxygen atom; and
X is a group represented by the following formula (A1) or (A2): 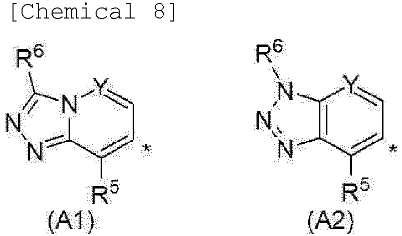 wherein
   * is the bonding position to the carbon atom to which X is bonded,
   R⁵ is a hydrogen atom, a halogen atom, or a C₁₋₆ alkyl group optionally substituted by 1 to 3 substituents selected from the aforementioned substituent group a (preferably, a halogen atom or a C₁₋₆ alkyl group, more preferably, a methyl group),
   R⁶ is a hydrogen atom, or a C₁₋₆ alkyl group optionally substituted by 1 to 3 substituents selected from the aforementioned substituent group a (preferably, a methyl group, an ethyl group, a dimethylaminoethyl group, a 2,2,2-trifluoroethyl group, a 2-hydroxy-2-methylpropyl group or a 3-methanesulfonylpropyl group, more preferably, a methyl group), Y is -CH-, -CR⁷- or a nitrogen atom, and
   R⁷ is a hydroxy group, a halogen atom, a cyano group, a C₁₋₆ alkyl group optionally substituted by 1 to 3 substituents selected from the aforementioned substituent group a, a C₁₋₆ alkoxy group optionally substituted by 1 to 3 substituents selected from the aforementioned substituent group a, or a C₃₋₆ cycloalkyl group (preferably, a chlorine atom, a cyano group, a methyl group, a cyclopropyl group, a trifluoromethyl group, a methoxy group, a difluoromethoxy group or a trifluoromethoxy group)),
      provided that when Z¹ is -CH- or -CR³-, then Z² is a nitrogen atom, when Z² is -CH- or -CR⁴·-, then Z¹ is a nitrogen atom, and both Z¹ and Z² cannot simultaneously be -CH-, -CR³- or -CR⁴-,
   or a pharmaceutically acceptable salt thereof.

### [Compound (1C)]

Compound (1) wherein
R^{1a} and R^{1b} are each independently a hydrogen atom or a C₁₋₆ alkyl group;
R^{2a} and R^{2b} are each independently a hydrogen atom, a C₁₋₆ alkyl group optionally substituted by 1 to 3 substituents selected from the aforementioned substituent group a, or a C₃₋₆ cycloalkyl group, or
R^{2a} and R^{2b} are bonded together to form, together with the carbon atom to which R^{2a} and R^{2b} are bonded, a C₃₋₈ cycloalkane which is optionally substituted by 1 to 3 substituents selected from the aforementioned substituent group b, or a 3- to 8-membered saturated oxygen-containing heterocycle;
Z¹ is -CH- or -CR³- (R³ is a halogen atom, a hydroxy group, an amino group, a C₁₋₆ alkylamino group or a C₁₋₆ alkyl group) (preferably, Z¹ is -CR³- (R³ is a hydroxy group));
Z² is -CH-, -CR⁴- (R⁴ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
R¹¹ in the number of n are each independently a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a;
n is an integer of 0 to 2;
R^{8a} and R^{8b} are each independently a hydrogen atom, a halogen atom, a cyano group, a C₁₋₆ alkyl group or a C₁₋₆ alkoxy group; R^{9a} and R^{9b} are each independently a hydrogen atom or a C₁₋₆ alkyl group;
W is -CH₂-, -CHR¹⁰- (R¹⁰ is a C₁₋₆ alkyl group) or an oxygen atom; and
X is a group represented by the following formula (A1) or (A2): 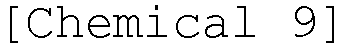 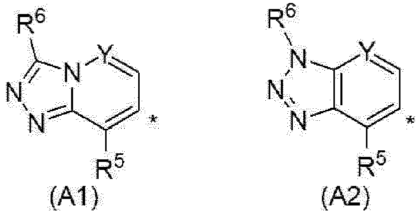 wherein
   * is the bonding position to the carbon atom to which X is bonded,
   R⁵ is a hydrogen atom, a halogen atom, or a C₁₋₆ alkyl group optionally substituted by 1 to 3 substituents selected from the aforementioned substituent group a (preferably, a halogen atom or a C₁₋₆ alkyl group, more preferably, a methyl group),
   R⁶ is a hydrogen atom, or a C₁₋₆ alkyl group optionally substituted by 1 to 3 substituents selected from the aforementioned substituent group a (preferably, a methyl group, an ethyl group, a dimethylaminoethyl group, a 2,2,2-trifluoroethyl group, a 2-hydroxy-2-methylpropyl group or a 3-methanesulfonylpropyl group, more preferably, a methyl group), Y is -CH-, -CR⁷- or a nitrogen atom, and
   R⁷ is a hydroxy group, a halogen atom, a cyano group, a C₁₋₆ alkyl group optionally substituted by 1 to 3 substituents selected from the aforementioned substituent group a, a C₁₋₆ alkoxy group optionally substituted by 1 to 3 substituents selected from the aforementioned substituent group a, or a C₃₋₆ cycloalkyl group (preferably, chlorine atom, a cyano group, a methyl group, a cyclopropyl group, a trifluoromethyl group, a methoxy group, a difluoromethoxy group or a trifluoromethoxy group),
      provided that when Z¹ is -CH- or -CR³-, then Z² is a nitrogen atom, when Z² is -CH- or -CR⁴·-, then Z¹ is a nitrogen atom, and both Z¹ and Z² cannot simultaneously be -CH-, -CR³- or -CR⁴-,
   or a pharmaceutically acceptable salt thereof.

### [Compound (1D)]

Compound (1) wherein
R^{1a} and R^{1b} are each independently a hydrogen atom or a C₁₋₆ alkyl group;
R^{2a} and R^{2b} are each independently a hydrogen atom, a C₁₋₆ alkyl group optionally substituted by 1 to 3 substituents selected from the aforementioned substituent group a, or a C₃₋₆ cycloalkyl group, or
R^{2a} and R^{2b} are bonded together to form, together with the carbon atom to which R^{5a} and R^{5b} are bonded, a C₃₋₈ cycloalkane which is optionally substituted by 1 to 3 substituents selected from the aforementioned substituent group b, or a 3- to 8-membered saturated oxygen-containing heterocycle,
Z¹ is -CH- or -CR³- (R³ is a halogen atom, a hydroxy group, an amino group, a C₁₋₆ alkylamino group or a C₁₋₆ alkyl group) (preferably, Z¹ is -CR³- (R³ is a hydroxy group));
Z² is a nitrogen atom;
R¹¹ in the number of n are each independently a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a;
n is an integer of 0 to 2;
R^{8a} and R^{8b} are each independently a hydrogen atom, a halogen atom, a cyano group, a C₁₋₆ alkyl group or a C₁₋₆ alkoxy group; R^{9a} and R^{9b} are each independently a hydrogen atom or a C₁₋₆ alkyl group;
W is -CH₂-, -CHR¹⁰- (R¹⁰ is a C₁₋₆ alkyl group) or an oxygen atom; and
X is a group represented by the following formula (A1) or (A2): 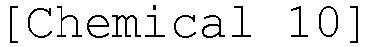 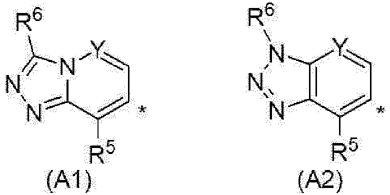 wherein
   * is the bonding position to the carbon atom to which X is bonded,
   R⁵ is a hydrogen atom, a halogen atom, or a C₁₋₆ alkyl group optionally substituted by 1 to 3 substituents selected from the aforementioned substituent group a (preferably, a halogen atom or a C₁₋₆ alkyl group, more preferably, a methyl group),
   R⁶ is a hydrogen atom, or a C₁₋₆ alkyl group optionally substituted by 1 to 3 substituents selected from the aforementioned substituent group a (preferably, a methyl group, an ethyl group, a dimethylaminoethyl group, a 2,2,2-trifluoroethyl group, a 2-hydroxy-2-methylpropyl group or a 3-methanesulfonylpropyl group, more preferably, a methyl group), Y is -CH-, -CR⁷- or a nitrogen atom, and
   R⁷ is a hydroxy group, a halogen atom, a cyano group, a C₁₋₆ alkyl group optionally substituted by 1 to 3 substituents selected from the aforementioned substituent group a, a C₁₋₆ alkoxy group optionally substituted by 1 to 3 substituents selected from the aforementioned substituent group a, or a C₃₋₆ cycloalkyl group (preferably, a chlorine atom, a cyano group, a methyl group, a cyclopropyl group, a trifluoromethyl group, a methoxy group, a difluoromethoxy group or a trifluoromethoxy group),
   or a pharmaceutically acceptable salt thereof.

### [Compound (1E)]

Compound (1) wherein
R^{1a} and R^{1b} are each independently a hydrogen atom or a C₁₋₆ alkyl group;
R^{2a} and R^{2b} are each independently a hydrogen atom, a C₁₋₆ alkyl group optionally substituted by 1 to 3 substituents selected from the aforementioned substituent group a, or a C₃₋₆ cycloalkyl group, or
R^{2a} and R^{2b} are bonded together to form, together with the carbon atom to which R^{2a} and R^{2b} are bonded, a C₃₋₈ cycloalkane which is optionally substituted by 1 to 3 substituents selected from the aforementioned substituent group b, or a 3- to 8-membered saturated oxygen-containing heterocycle;
Z¹ is -CH-, -CR³- (R³ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
Z² is -CH-, -CR⁴- (R⁴ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
R¹¹ in the number of n are each a hydroxy group;
n is an integer of 0 to 2 (preferably, 1, more preferably, 0); R^{8a} and R^{8b} are each independently a hydrogen atom, a halogen atom, a cyano group, a C₁₋₆ alkyl group or a C₁₋₆ alkoxy group; R^{9a} and R^{9b} are each independently a hydrogen atom or a C₁₋₆ alkyl group;
W is -CH₂-, -CHR¹⁰- (R¹⁰ is a C₁₋₆ alkyl group) or an oxygen atom; and
X is a group represented by the following formula (A1) or (A2): 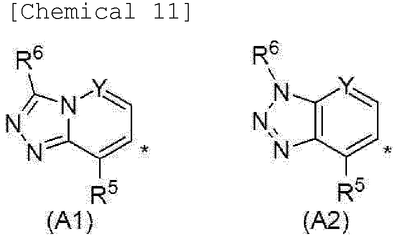 wherein
   * is the bonding position to the carbon atom to which X is bonded,
   R⁵ is a hydrogen atom, a halogen atom, or a C₁₋₆ alkyl group optionally substituted by 1 to 3 substituents selected from the aforementioned substituent group a (preferably, a halogen atom or a C₁₋₆ alkyl group, more preferably, a methyl group),
   R⁶ is a hydrogen atom, or a C₁₋₆ alkyl group optionally substituted by 1 to 3 substituents selected from the aforementioned substituent group a (preferably, a methyl group, an ethyl group, a dimethylaminoethyl group, a 2,2,2-trifluoroethyl group, a 2-hydroxy-2-methylpropyl group or a 3-methanesulfonylpropyl group, more preferably, a methyl group), Y is -CH-, -CR⁷- or a nitrogen atom, and
   R⁷ is a hydroxy group, a halogen atom, a cyano group, a C₁₋₆ alkyl group optionally substituted by 1 to 3 substituents selected from the aforementioned substituent group a, a C₁₋₆ alkoxy group optionally substituted by 1 to 3 substituents selected from the aforementioned substituent group a, or a C₃₋₆ cycloalkyl group (preferably, a chlorine atom, a cyano group, a methyl group, a cyclopropyl group, a trifluoromethyl group, a methoxy group, a difluoromethoxy group or a trifluoromethoxy group),
      provided that when Z¹ is -CH- or -CR³-, then Z² is a nitrogen atom, when Z² is -CH- or -CR⁴·-, then Z¹ is a nitrogen atom, and both Z¹ and Z² cannot simultaneously be -CH-, -CR³- or -CR⁴-,
   or a pharmaceutically acceptable salt thereof.

### [Compound (1F)]

Compound (1) wherein
R^{1a} and R^{1b} are each independently a hydrogen atom or a C₁₋₆ alkyl group;
R^{2a} and R^{2b} are each independently a hydrogen atom, a C₁₋₆ alkyl group optionally substituted by 1 to 3 substituents selected from the aforementioned substituent group a, or a C₃₋₆ cycloalkyl group, or
R^{2a} and R^{2b} are bonded together to form, together with the carbon atom to which R^{2a} and R^{2b} are bonded, a C₃₋₈ cycloalkane which is optionally substituted by 1 to 3 substituents selected from the aforementioned substituent group b, or a 3- to 8-membered saturated oxygen-containing heterocycle;
Z¹ is -CH-, -CR³- (R³ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
Z² is -CH-, -CR⁴- (R⁴ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
R¹¹ in the number of n are each independently a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a;
n is an integer of 0 to 2;
R^{8a} and R^{8b} are both hydrogen atoms;
R^{9a} and R^{9b} are each independently a hydrogen atom or a C₁₋₆ alkyl group (preferably, both hydrogen atoms);
W is -CH₂-, -CHR¹⁰- (R¹⁰ is a C₁₋₆ alkyl group) or an oxygen atom; and
X is a group represented by the following formula (A1) or (A2): 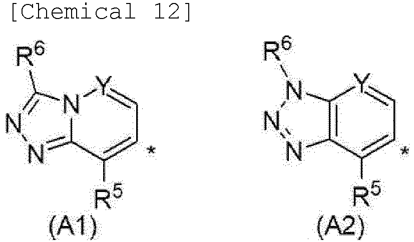 wherein
   * is the bonding position to the carbon atom to which X is bonded,
   R⁵ is a hydrogen atom, a halogen atom, or a C₁₋₆ alkyl group optionally substituted by 1 to 3 substituents selected from the aforementioned substituent group a (preferably, a halogen atom or a C₁₋₆ alkyl group, more preferably, a methyl group),
   R⁶ is a hydrogen atom, or a C₁₋₆ alkyl group optionally substituted by 1 to 3 substituents selected from the aforementioned substituent group a (preferably, a methyl group, an ethyl group, a dimethylaminoethyl group, a 2,2,2-trifluoroethyl group, a 2-hydroxy-2-methylpropyl group or a 3-methanesulfonylpropyl group, more preferably, a methyl group), Y is -CH-, -CR⁷- or a nitrogen atom, and
   R⁷ is a hydroxy group, a halogen atom, a cyano group, a C₁₋₆ alkyl group optionally substituted by 1 to 3 substituents selected from the aforementioned substituent group a, a C₁₋₆ alkoxy group optionally substituted by 1 to 3 substituents selected from the aforementioned substituent group a, or a C₃₋₆ cycloalkyl group (preferably, a chlorine atom, a cyano group, a methyl group, a cyclopropyl group, a trifluoromethyl group, a methoxy group, a difluoromethoxy group or a trifluoromethoxy group),
      provided that when Z¹ is -CH- or -CR³-, then Z² is a nitrogen atom, when Z² is -CH- or -CR⁴·-, then Z¹ is a nitrogen atom, and both Z¹ and Z² cannot simultaneously be -CH-, -CR³- or -CR⁴-,
   or a pharmaceutically acceptable salt thereof.

### [Compound (1G)]

Compound (1) wherein
R^{1a} and R^{1b} are each independently a hydrogen atom or a C₁₋₆ alkyl group;
R^{2a} and R^{2b} are each independently a hydrogen atom, a C₁₋₆ alkyl group optionally substituted by 1 to 3 substituents selected from the aforementioned substituent group a, or a C₃₋₆ cycloalkyl group, or
R^{2a} and R^{2b} are bonded together to form, together with the carbon atom to which R^{2a} and R^{2b} are bonded, a C₃₋₈ cycloalkane which is optionally substituted by 1 to 3 substituents selected from the aforementioned substituent group b, or a 3- to 8-membered saturated oxygen-containing heterocycle;
Z¹ is -CH-, -CR³- (R³ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
Z² is -CH-, -CR⁴- (R⁴ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
R¹¹ in the number of n are each independently a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a;
n is an integer of 0 to 2;
R^{8a} and R^{8b} are each independently a hydrogen atom, a halogen atom, a cyano group, a C₁₋₆ alkyl group or a C₁₋₆ alkoxy group; R^{9a} and R^{9b} are each independently a hydrogen atom or a C₁₋₆ alkyl group;
W is an oxygen atom; and
X is a group represented by the following formula (A1) or (A2): 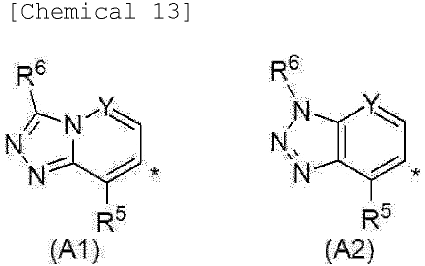 wherein
   * is the bonding position to the carbon atom to which X is bonded,
   R⁵ is a hydrogen atom, a halogen atom, or a C₁₋₆ alkyl group optionally substituted by 1 to 3 substituents selected from the aforementioned substituent group a (preferably, a halogen atom or a C₁₋₆ alkyl group, more preferably, a methyl group),
   R⁶ is a hydrogen atom, or a C₁₋₆ alkyl group optionally substituted by 1 to 3 substituents selected from the aforementioned substituent group a (preferably, a methyl group, an ethyl group, a dimethylaminoethyl group, a 2,2,2-trifluoroethyl group, a 2-hydroxy-2-methylpropyl group or a 3-methanesulfonylpropyl group, more preferably, a methyl group), Y is -CH-, -CR⁷- or a nitrogen atom, and
   R⁷ is a hydroxy group, a halogen atom, a cyano group, a C₁₋₆ alkyl group optionally substituted by 1 to 3 substituents selected from the aforementioned substituent group a, a C₁₋₆ alkoxy group optionally substituted by 1 to 3 substituents selected from the aforementioned substituent group a, or a C₃₋₆ cycloalkyl group (preferably, a chlorine atom, a cyano group, a methyl group, a cyclopropyl group, a trifluoromethyl group, a methoxy group, a difluoromethoxy group or a trifluoromethoxy group),
      provided that when Z¹ is -CH- or -CR³-, then Z² is a nitrogen atom, when Z² is -CH- or -CR⁴·-, then Z¹ is a nitrogen atom, and both Z¹ and Z² cannot simultaneously be -CH-, -CR³- or -CR⁴-,
   or a pharmaceutically acceptable salt thereof.

### [Compound (1H)]

Compound (1) wherein
R^{1a} and R^{1b} are each independently a hydrogen atom or a C₁₋₆ alkyl group;
R^{2a} and R^{2b} are each independently a hydrogen atom, a C₁₋₆ alkyl group optionally substituted by 1 to 3 substituents selected from the aforementioned substituent group a, or a C₃₋₆ cycloalkyl group, or
R^{2a} and R^{2b} are bonded together to form, together with the carbon atom to which R^{2a} and R^{2b} are bonded, a C₃₋₈ cycloalkane which is optionally substituted by 1 to 3 substituents selected from the aforementioned substituent group b, or a 3- to 8-membered saturated oxygen-containing heterocycle;
Z¹ is -CH-, -CR³- (R³ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
Z² is -CH-, -CR⁴- (R⁴ is a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a) or a nitrogen atom;
R¹¹ in the number of n are each independently a halogen atom, a hydroxy group, an amino group optionally substituted by one or two C₁₋₆ alkyl groups, a C₁₋₆ alkyl group optionally substituted by 1 to 3 substituents selected from the aforementioned substituent group a, or a C₁₋₆ alkoxy group optionally substituted by 1 to 3 substituents selected from the aforementioned substituent group a;
n is an integer of 0 to 2;
R^{8a} and R^{8b} are each independently a hydrogen atom, a halogen atom, a cyano group, a C₁₋₆ alkyl group or a C₁₋₆ alkoxy group; R^{9a} and R^{9b} are each independently a hydrogen atom or a C₁₋₆ alkyl group;
W is -CH₂-, -CHR¹⁰- (R¹⁰ is a C₁₋₆ alkyl group) or an oxygen atom;
X is a group represented by the following formula (A2): 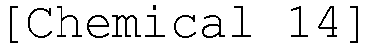 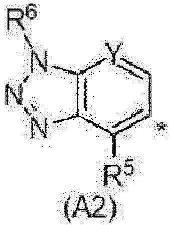 wherein
   * is the bonding position to the carbon atom to which X is bonded,
   R⁵ is a hydrogen atom, a halogen atom, or a C₁₋₆ alkyl group optionally substituted by 1 to 3 substituents selected from the aforementioned substituent group a (preferably, a halogen atom or a C₁₋₆ alkyl group, more preferably, a methyl group),
   R⁶ is a hydrogen atom, or a C₁₋₆ alkyl group optionally substituted by 1 to 3 substituents selected from the aforementioned substituent group a (preferably, a methyl group, an ethyl group, a dimethylaminoethyl group, a 2,2,2-trifluoroethyl group, a 2-hydroxy-2-methylpropyl group or a 3-methanesulfonylpropyl group, more preferably, a methyl group), Y is -CH-, -CR⁷- or a nitrogen atom, and
   R⁷ is a hydroxy group, a halogen atom, a cyano group, a C₁₋₆ alkyl group optionally substituted by 1 to 3 substituents selected from the aforementioned substituent group a, a C₁₋₆ alkoxy group optionally substituted by 1 to 3 substituents selected from the aforementioned substituent group a, or a C₃₋₆ cycloalkyl group (preferably, a chlorine atom, a cyano group, a methyl group, a cyclopropyl group, a trifluoromethyl group, a methoxy group, a difluoromethoxy group or a trifluoromethoxy group),
      provided that when Z¹ is -CH- or -CR³-, then Z² is a nitrogen atom, when Z² is -CH- or -CR⁴-, then Z¹ is a nitrogen atom, and both Z¹ and Z² cannot simultaneously be -CH-, -CR³- or -CR⁴-,
   or a pharmaceutically acceptable salt thereof.

### [Compound (1J)]

Compound (1) wherein
R^{1a} and R^{1b} are each independently a hydrogen atom or a methyl group;
R^{2a} and R^{2b} are each independently a hydrogen atom, a C₁₋₆ alkyl group, a C₁₋₆ haloalkyl group or a C₃₋₆ cycloalkyl group, or R^{2a} and R^{2b} are bonded together to form, together with the carbon atom to which R^{2a} and R^{2b} are bonded, a C₃₋₆ cycloalkane; Z¹ is -CH- or -CR³- (R³ is a halogen atom, a hydroxy group, an amino group, a C₁₋₆ alkylamino group or a C₁₋₆ alkyl group);
Z² is a nitrogen atom;
R¹¹ in the number of n are each a hydroxy group;
n is 0 or 1;
R^{8a} and R^{8b} are both hydrogen atoms;
R^{9a} and R^{9b} are both hydrogen atoms;
W is an oxygen atom; and
X is a group represented by the following formula (A1) or (A2): 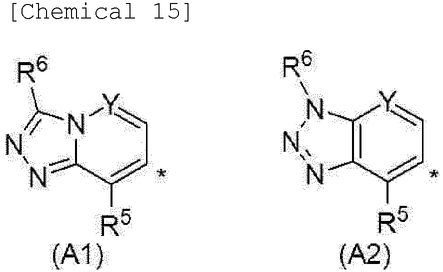 wherein
   * is the bonding position to the carbon atom to which X is bonded,
   R⁵ is a halogen atom or a C₁₋₆ alkyl group (preferably, a methyl group)
   R⁶ is a methyl group, an ethyl group, a dimethylaminoethyl group, a 2,2,2-trifluoroethyl group, a 2-hydroxy-2-methylpropyl group or a 3-methanesulfonylpropyl group (preferably, a methyl group),
   Y is -CH-, -CR⁷- or a nitrogen atom, and
   R⁷ is a chlorine atom, a cyano group, a methyl group, a cyclopropyl group, a trifluoromethyl group, a methoxy group, a difluoromethoxy group or a trifluoromethoxy group,
   or a pharmaceutically acceptable salt thereof.

### [Compound (1K)]

Compound (1) wherein
R^{1a} and R^{1b} are each independently a hydrogen atom or a methyl group;
R^{2a} and R^{2b} are each independently a hydrogen atom, a C₁₋₆ alkyl group, a C₁₋₆ haloalkyl group or a C₃₋₆ cycloalkyl group, or R^{2a} and R^{2b} are each independently a hydrogen atom, a methyl group, an ethyl group, a trifluoromethyl group or a difluoroethyl group, or
R^{2a} and R^{2b} are bonded together to form, together with the carbon atom to which R^{2a} and R^{2b} are bonded, a cyclopropane;
Z¹ is -CR³-(R³ is a hydroxy group);
Z² is a nitrogen atom;
n is 0;
R^{8a} and R^{8b} are both hydrogen atoms;
R^{9a} and R^{9b} are both hydrogen atoms;
W is an oxygen atom; and
X is a group represented by the following (A2): 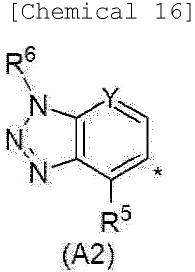 wherein
   * is the bonding position to the carbon atom to which X is bonded,
   R⁵ is a halogen atom or a C₁₋₆ alkyl group (preferably, a methyl group)
   R⁶ is a methyl group, an ethyl group, a dimethylaminoethyl group, a 2,2,2-trifluoroethyl group, a 2-hydroxy-2-methylpropyl group or a 3-methanesulfonylpropyl group (preferably, a methyl group),
   Y is -CH-, -CR⁷- or a nitrogen atom, and
   R⁷ is a chlorine atom, a cyano group, a methyl group, a cyclopropyl group, a trifluoromethyl group, a methoxy group, a difluoromethoxy group or a trifluoromethoxy group),
   or a pharmaceutically acceptable salt thereof.

### [Compound (1L)]

Compound (1) wherein
R^{1a} and R^{1b} are each independently a hydrogen atom or a methyl group;
R^{2a} and R^{2b} are each independently a hydrogen atom, a C₁₋₆ alkyl group, a C₁₋₆ haloalkyl group or a C₃₋₆ cycloalkyl group, or R^{2a} and R^{2b} are each independently a hydrogen atom, a methyl group, an ethyl group, a trifluoromethyl group or a difluoroethyl group, or
R^{2a} and R^{2b} are bonded together to form, together with the carbon atom to which R^{2a} and R^{2b} are bonded, a cyclopropane;
Z¹ is -CR³- (R³ is a hydroxy group);
Z² is a nitrogen atom;
n is 0;
R^{8a} and R^{8b} are both hydrogen atoms;
R^{9a} and R^{9b} are both hydrogen atoms;
W is an oxygen atom; and
X is a group represented by the following (A2): 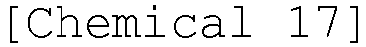 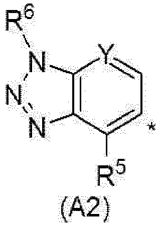 wherein
   * is the bonding position to the carbon atom to which X is bonded,
   R⁵ is a methyl group
   R⁶ is a methyl group,
   Y is -CH-, -CR⁷- or a nitrogen atom, and
   R⁷ is a chlorine atom, a cyano group, a methyl group, a cyclopropyl group, a trifluoromethyl group, a methoxy group, a difluoromethoxy group or a trifluoromethoxy group,
   or a pharmaceutically acceptable salt thereof.

Specific examples of preferred compound (1) are compounds of the below-mentioned Examples 1 to 93 or pharmaceutically acceptable salts thereof, more preferably any compound (1) selected from the following group or a pharmaceutically acceptable salt thereof:
(3S)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid,
(3R)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid,
(3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}propanoic acid,
(3R)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}propanoic acid,
(3R)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
(3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
(3S)-3-(7-{[(2S)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
(3R)-3-(7-{[(2S)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
(3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid,
(3R)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid,
(3S)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoic acid,
(3R)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoic acid,
(3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)-2,2-dimethylpropanoic acid, and
(3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}-2,2-dimethylpropanoic acid.

Among them, any compound (1) selected from the following group or a pharmaceutically acceptable salt thereof:
(3R)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
(3S)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoic acid, and
(3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}-2,2-dimethylpropanoic acid
are particularly preferred.

Since the compound (1) of the present invention has a basic group such as a nitrogen-containing heterocyclic group in the molecule, it can generally form a pharmaceutically acceptable acid addition salt. Examples of such acid addition salts include hydrohalides such as hydrofluoride, hydrochloride, hydrobromide, hydroiodide and the like; inorganic acid salts such as nitrate, perchlorate, sulfate, phosphate and the like; lower alkane sulfonates such as methanesulfonate, trifluoromethanesulfonate, ethanesulfonate and the like; arylsulfonates such as benzenesulfonate, p-toluenesulfonate and the like; organic acid salts such as acetate, malate, fumarate, succinate, citrate, tartrate, oxalate, maleate, mucicate, adipate and the like; amino acid salts such as ornithinate, glutamate, aspartate and the like; and the like. Among them, hydrohalides, arylsulfonates, and organic acid salts are preferred.

The acid addition salt of the compound (1) of the present invention includes an acid addition salt that can be formed by combining an acid added to the compound of the present invention with the compound (1) of the present invention in any ratio. For example, the hydrochloride includes salts that can be formed such as monohydrochloride, dihydrochloride, trihydrochloride, and the like, the fumarate includes salts that can be formed such as monofumarate, 1/2 fumarate, and the like, and the succinate includes salts that can be formed such as monosuccinate, 2/3 succinate, 1/3 succinate, and the like.

Since the compound (1) of the present invention has a carboxy group in the molecule, it can generally form a pharmaceutically acceptable base addition salt. Examples of such base addition salt include alkali metal salts such as sodium salt, potassium salt, lithium salt and the like; alkaline earth metal salts such as calcium salt, magnesium salt and the like; inorganic salts such as ammonium salt and the like; and organic amine salts such as dibenzylamine salt, morpholine salt, alkyl phenylglycinate salt, ethylenediamine salt, N-methylglucamine salt, diethylamine salt, triethylamine salt, cyclohexylamine salt, dicyclohexylamine salt, N,N'-dibenzylethylenediamine salt, diethanolamine salt, N-benzyl-N-(2-phenylethoxy)amine salt, piperazine salt, tetramethylammonium salt, tris(hydroxymethyl)aminomethane salt and the like.

When the compound (1) of the present invention has an asymmetric carbon atom in the molecule, it may exist as a plurality of stereoisomers (i.e., diastereoisomers, optical isomers) based on the asymmetric carbon atom. The present invention encompasses any one of these stereoisomers and a mixture containing the plurality of stereoisomers in any ratio. In addition, isomers due to conformation or tautomerism may be generated. Such isomers and mixtures thereof are also encompassed in the compound (1) of the present invention.

In the names of the compounds of the present invention, when the compound has a carbon atom that serves as an asymmetric center in the compound structure, the absolute configuration thereof is indicated by R and S (along with the position number).

Even when optical isomers have been separated, if the configuration of the carbon atom that serves as an asymmetric center in the compound structure has not been determined, it is indicated by R* or S*.

Furthermore, by using R* and S* simultaneously, the relative configuration may be indicated even when the absolute configuration has not been determined.

The compound (1) of the present invention may be labeled or substituted by isotopes (e.g., ²H, ³H, ¹³C, ¹⁴C, ¹⁵N, ¹⁸F, ³²P, ³⁵S, ¹²⁵I, etc.), and compounds (1) labeled or substituted by isotopes are useful as therapeutic or prophylactic agents, research reagents (e.g., assay reagents), and diagnostic agents (e.g., in vivo imaging agents). The compound of the present invention containing all proportions of radioactive or nonradioactive isotopes are within the scope of the present invention.

The compound (1) of the present invention or a pharmaceutically acceptable salt thereof may be crystal, in a single crystal form or in a mixture of several crystal forms.

The compound (1) of the present invention may also exist as a non-solvate or solvate. The solvate is not particularly limited as long as it is pharmaceutically acceptable, and hydrate, ethanol solvate and the like are specifically preferred.

The compound (1) of the present invention may be a prodrug.

Prodrugs of compound (1) of the present invention are compounds that are converted to compound (1) in vivo by reactions with enzymes, gastric acid and the like. Prodrugs of compound (1) may have a structure that are easily hydrolyzed or metabolized after administration to a patient.

As prodrugs of compound (1), for example, when compound (1) has an amino group, compounds in which the amino group is acylated, alkylated, or phosphorylated (e.g., eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated, tert-butylated, and the like); when compound (1) has an hydroxy group, compounds in which the hydroxy group of compound (1) is acylated, alkylated, phosphorylated, or borylated (e.g., compounds in which the hydroxy group of compound (1) is acetylated, palmitoylated, propanoylated, pivaloylated, succinylated, fumarylated, alanylated, dimethylaminomethylcarbonylated, etc.); compounds in which the carboxy group of compound (1) is esterified, amidated (e.g., compounds in which the carboxy group of compound (1) is ethyl esterified, phenyl esterified, carboxymethyl esterified, dimethylaminomethyl esterified, pivaloyloxymethyl esterified, 1-{(ethoxycarbonyl)oxy}ethyl esterified, phthalidyl esterified, (5-methyl-2-oxo-1,3-dioxolen-4-yl)methyl esterified, 1-{[(cyclohexyloxy)carbonyl]oxy}ethyl esterified, methylamidated compounds, etc.).

Prodrugs of compound (1) can be made from compound (1) by known methods. Prodrugs of compound (1) also include those that change to compound (1) under physiological conditions, as described in "Development of Pharmaceuticals", Vol. 7, Molecular Design, pp. 163-198, published by Hirokawa Shoten 1990. Furthermore, prodrugs of compound (1) may be both hydrate and non-hydrate.

### (Production methods of the compound (1) of the present invention)

Representative methods for producing compound (1) of the present invention or a pharmaceutically acceptable salt thereof are described below.

The compound (1) of the present invention can be produced by various production methods, and the production methods shown below and Reference Examples and Examples described below are only examples, and the present invention should not be interpreted as being limited to these.

Each raw material compound may form a salt as long as it does not inhibit the reaction, and examples of such salts include the same as the pharmaceutically acceptable salts of compound (1) described above.

When no specific production method is described, the raw material compounds can be easily obtained from commercial sources and used, or can be produced according to a method known per se or a method equivalent thereto. In addition, the production intermediates generated in the following production methods may be isolated and purified by a method such as column chromatography (including normal phase and reverse phase) using silica gel or alumina, recrystallization, reprecipitation, distillation, and the like, or may be used directly in the next reaction without isolation and purification.

In the present specification, all patent literature, non-patent literature, or references expressly cited herein may all be cited herein as a part of the present specification.

The compound (1), a pharmaceutically acceptable salt thereof, and a production intermediate therefor can be produced utilizing the characteristics based on the kind of the basic skeleton or substituent, and applying various known production methods. Examples of the known method include the methods described in "ORGANIC FUNCTIONAL GROUP PREPARATIONS", 2nd edition, ACADEMIC PRESS, INC., 1989, "Comprehensive Organic Transformations", 2nd edition, VCH Publishers Inc., 1999, and the like.

In such cases, depending on the type of functional group present in the compound, it may be effective in terms of manufacturing technology to protect the functional group with an appropriate protecting group at the starting material or intermediate stage, or to replace the functional group with a group that can be easily converted to the functional group concerned.

Examples of the functional group include amino group, hydroxy group, formyl group, carbonyl group, carboxy group, and the like, and examples of the protecting group thereof include the protecting groups described in P.G. Wuts, "Protective Groups in Organic Synthesis", 5th edition, Wiley, 2014.

The protecting group or the group that can be easily converted into the functional group may be appropriately selected depending on the reaction conditions of the production method for producing the compound.

According to such a method, a desired compound can be obtained by introducing the group and performing a reaction, and then removing the protecting group as necessary or converting same into a desired group.

A prodrug of the compound can be produced by, similar to the above-mentioned protecting groups, introducing a particular group in the stage of a starting material or intermediate, or by a reaction using the obtained compound. The reaction for producing a prodrug can be performed by applying a known method by those of ordinary skill in the art such as general esterification, amidation, dehydration, hydrogenation and the like.

The compound (1) of the present invention can be produced, for example, by the following Method A or Method B. The production intermediates used in Method A or Method B can be produced, for example, by the following Method C to Method J.

In the reactions in each step of the following Method A to Method J, the reaction temperature varies depending on the solvent, starting material, reagents, and the like, and the reaction time varies depending on the solvent, starting material, reagents, reaction temperature, and the like. Furthermore, the amounts of the solvent, starting material, reagents, and the like used can be appropriately determined depending on the progress of the reaction.

The conversion of functional groups on heterocycles in the production intermediates used in each step of the following Method A to Method J can be performed by a method known per se (specifically, the reaction conditions for converting a halogen atom to a C₁₋₆ alkyl group or a C₃₋₆ cycloalkyl group are described in, for example, Zou, G.; Reddy, Y. K.; Falck, J. R. Tetrahedron Lett. 2001, 42, 7213, Molander, G. A.; Yun, C. -S. Tetrahedron 2002, 58, 1465, Tsuji, J. Palladium Reagents and Catalysts; John Wiley & Sons, Inc.: England 2004, Metal-Catalyzed Cross-Coupling Reactions; de Meijere, A.; Diederich, F.; Wiley-VCH: Weinheim, 2004 and the like) or a method analogous thereto, or the method described in Examples below or a method analogous thereto.

### (Method A)

In this production method, compound (2A) and compound (3) are condensed to obtain compound (4), and then the protecting group of compound (4) is removed to produce compound (1). 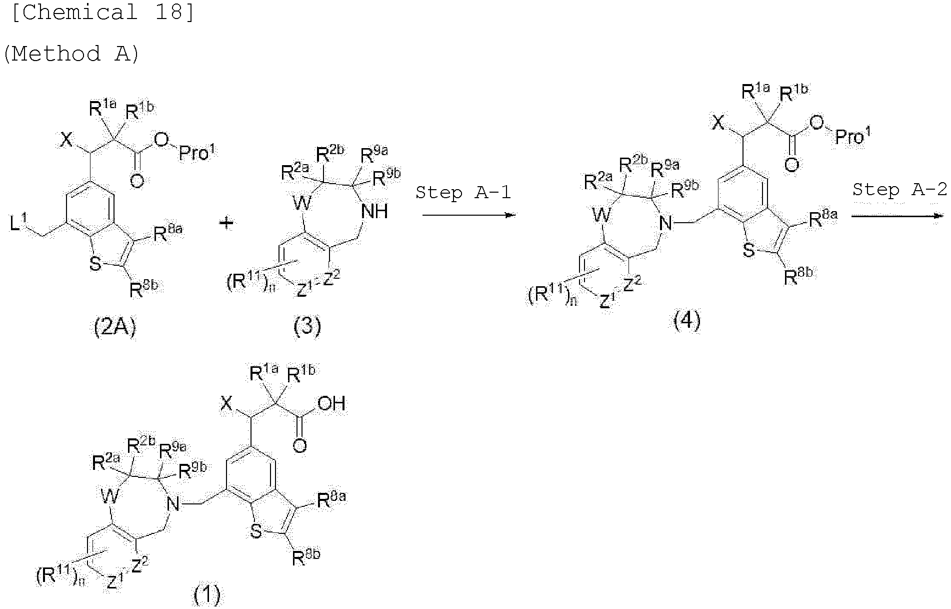 wherein L¹ is a leaving group (preferably a halogen atom), Pro¹ is a protecting group (preferably a C₁₋₆ alkyl group such as methyl group, ethyl group, tert-butyl group and the like, and other symbols are as defined above.

### (Step A-1)

In this step, compound (2A) and compound (3) are condensed, in the presence or absence of a base, in a solvent to produce compound (4).

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; alcohols such as methanol, ethanol, tert-butanol, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; mixed solvents containing the organic solvents mentioned above and water in any ratio; and the like.

The base to be used is not particularly limited as long as it is one that is used as a base in a normal reaction. Preferred examples of the base include organic bases such as triethylamine, N,N-diisopropylethylamine, N-methylmorpholine, lutidine, pyridine, and the like; alkali metal carbonates such as sodium carbonate, potassium carbonate, and the like; alkaline earth metal carbonates such as magnesium carbonate, and the like; alkali metal hydrogen carbonates such as potassium hydrogen carbonate, and the like; alkaline earth metal hydrogen carbonates such as calcium hydrogen carbonate, and the like; alkali metal hydroxides such as sodium hydroxide, and the like; alkaline earth metal hydroxides such as magnesium hydroxide, and the like; alkali metal phosphates such as tripotassium phosphate, and the like.

While the reaction temperature varies depending on the raw material compounds, reagents, and the like, it is generally -10°C to 150°C, preferably 0°C to 100°C.

While the reaction time varies depending on the raw material compounds, reagents, and the like, it is generally 5 min to 72 hr, preferably 10 min to 48 hr.

### (Step A-2)

In this step, the protecting group (Pro¹) of compound (4) is removed to produce compound (1).

This step is performed according to a known method appropriately selected from, for example, P. G. Wuts, "Protective Groups in Organic Synthesis", 5th Edition, Wiley, 2014, and the like, depending on the type of Pro¹. Here, a method of removing Pro¹ by using a base in a solvent (Step A-2-1), and a method of removing Pro¹ by using an acid in a solvent (Step A-2-2) are described; however, this step is not limited thereto.

### (Step A-2-1)

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; ethers such as diethyl ether, tetrahydrofuran, 1,4-dioxane, 1,2-dimethoxyethane, and the like; alcohols such as methanol, ethanol, tert-butanol, and the like; esters such as ethyl acetate, propyl acetate, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; mixed solvents containing the organic solvents mentioned above and water in any ratio; and the like.

The base to be used is not particularly limited as long as it is one that is used as a base in a normal reaction. Examples of the base include organic bases such as triethylamine, and the like; alkali metal carbonates such as sodium carbonate, potassium carbonate, and the like; alkaline earth metal carbonates such as magnesium carbonate, and the like; alkali metal hydrogen carbonates such as potassium hydrogen carbonate, and the like; alkaline earth metal hydrogen carbonates such as calcium hydrogen carbonate, and the like; alkali metal hydroxides such as lithium hydroxide, sodium hydroxide, potassium hydroxide and the like; alkaline earth metal hydroxides such as magnesium hydroxide, and the like; alkali metal phosphates such as tripotassium phosphate, and the like.

While the reaction temperature varies depending on the raw material compounds, reagents, and the like, it is generally -10°C to 150°C, preferably 10°C to 90°C.

While the reaction time varies depending on the raw material compounds, reagents, and the like, it is generally 1 min to 48 hr, preferably 10 min to 24 hr.

### (Step A-2-2)

The solvent to be used is not particularly limited as long as it does not inhibit the reaction and dissolves the starting material to some extent, and preferred examples include aromatic hydrocarbons such as benzene, toluene, xylene, and the like; halogenated hydrocarbons such as dichloromethane, chloroform, and the like; ethers such as diethyl ether, tetrahydrofuran, 1,4-dioxane, 1,2-dimethoxyethane, and the like; alcohols such as methanol, ethanol, and the like; esters such as ethyl acetate, propyl acetate, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; mixed solvents containing the organic solvents mentioned above and water in any ratio; and the like.

The acid to be used is not particularly limited as long as it is an acid that is used in normal reactions. Examples thereof include inorganic acids such as hydrochloric acid, sulfuric acid and the like; Lewis acids such as boron trifluoride, boron trichloride, boron tribromide, iodotrimethylsilane and the like; and organic acids such as trifluoroacetic acid and the like.

While the reaction temperature varies depending on the raw material compounds, reagents, and the like, it is generally -100°C to 150°C, preferably -78°C to 100°C.

While the reaction time varies depending on the raw material compounds, reagents, and the like, it is generally 5 min to 24 hr, preferably 10 min to 6 hr.

### (Method B)

In this production method, compound (2B) and compound (3) are subjected to a reductive amination reaction to obtain compound (4), and then the protecting group of compound (4) is removed to produce compound (1). 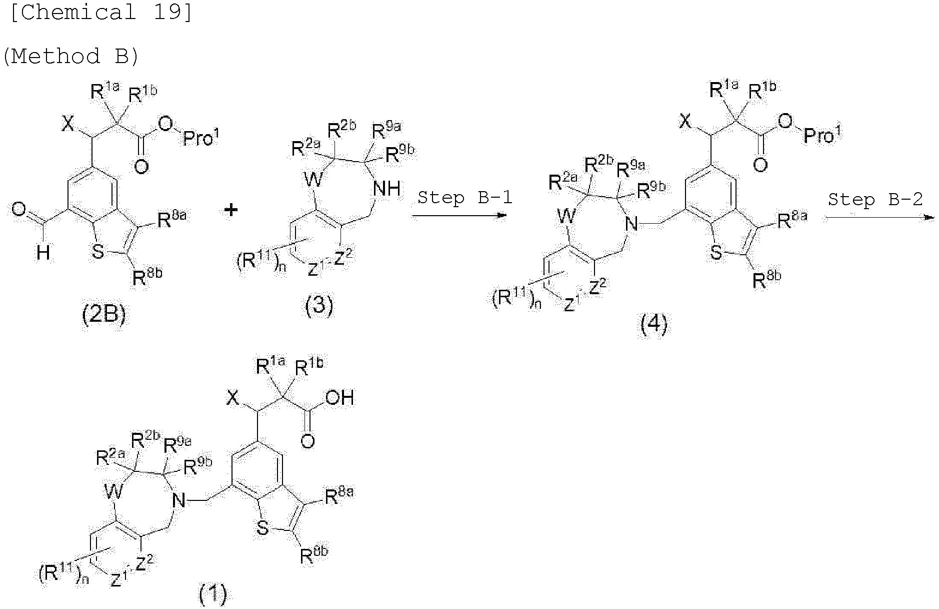 wherein each symbol is as defined above.

### (Step B-1)

In this step, compound (2B) and compound (3) are subjected to a reductive amination reaction using a reducing agent in the presence or absence of an acid and in the presence or absence of an additive in a solvent to produce compound (4).

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; and the like.

The reducing agent to be used is not particularly limited and, for example, reducing agents such as sodium borohydride, sodium cyanoborohydride, sodium triacetoxyborohydride, picoline borane, pyridineborane and the like can be mentioned.

The acid that can be used is not particularly limited as long as it is an acid that is used in normal reactions. Examples thereof include Lewis acids such as boron trifluoride, boron trichloride, boron tribromide, iodotrimethylsilane and the like; and organic acids such as acetic acid, trifluoroacetic acid and the like.

The additive that can be used is not particularly limited and, for example, inorganic salts such as sodium sulfate, magnesium sulfate and the like, and the like can be mentioned.

While the reaction temperature varies depending on the raw material compounds, reagents, and the like, it is generally -100°C to 150°C, preferably 0°C to 50°C.

While the reaction time varies depending on the raw material compounds, reagents, and the like, it is generally 5 min to 120 hr, preferably 10 min to 96 hr.

### (Step B-2)

In this step, a protecting group (Pro¹) is removed from compound (4) to produce compound (1).

In this step, the reaction can be performed under conditions similar to those in the aforementioned step A-2.

### (Method C)

In this production method, compound (2A-1) and compound (2B-1) (compounds of the aforementioned formulas (2A) and (2B) wherein R^{1a} and R^{1b} are hydrogen atoms), which are intermediate compounds used in the aforementioned method A or method B, are produced. 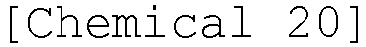 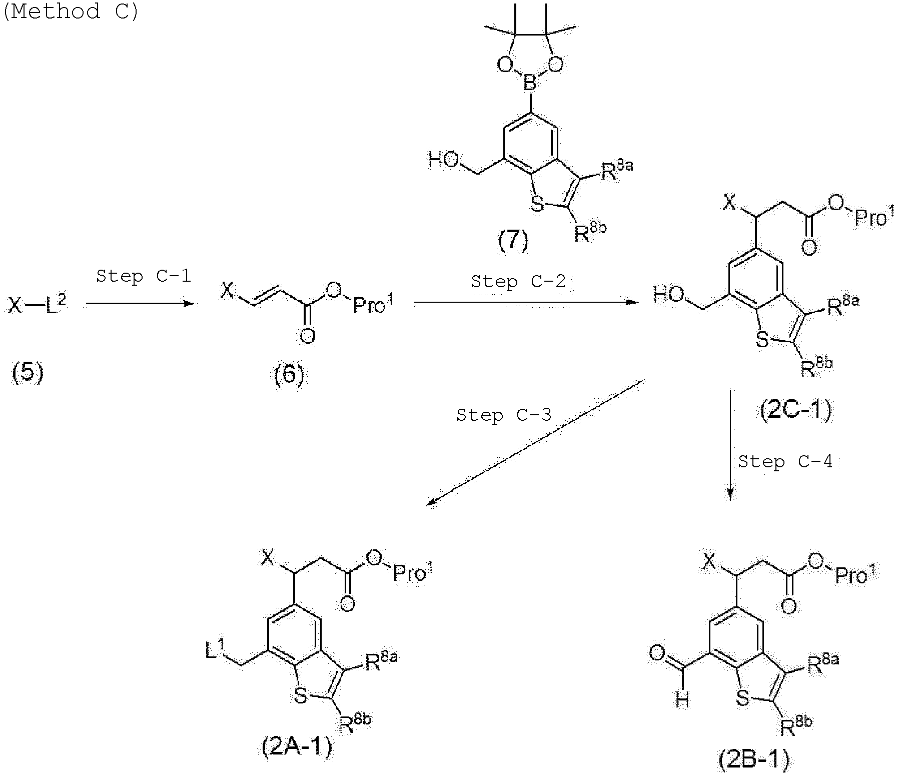 wherein L² is a leaving group (preferably a halogen atom), and other symbols are as defined above.

### (Step C-1)

In this step, compound (5) and an acrylic ester compound are reacted using a metal catalyst in a solvent in the presence or absence of a base and in the presence or absence of an additive to convert leaving group (L²) to an acrylic ester group (-CH=CH-CO₂Pro¹) to produce compound (6).

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; alcohols such as methanol, ethanol, tert-butanol and the like; nitriles such as acetonitrile and the like; amides such as formamide, N,N-dimethylformamide and the like; sulfoxides such as dimethyl sulfoxide and the like; mixed solvents containing a plurality of the organic solvents mentioned above in any ratio; mixed solvents containing the aforementioned organic solvent and water in any ratio and the like.

The base to be used is not particularly limited as long as it is one that is used as a base in a normal reaction. Preferred examples include organic bases such as triethylamine, N,N-diisopropylethylamine, N-methylmorpholine, lutidine, pyridine and the like; alkali metal carbonates such as sodium carbonate, potassium carbonate and the like; alkaline earth metal carbonates such as magnesium carbonate and the like; alkali metal hydrogen carbonates such as potassium hydrogen carbonate and the like; alkaline earth metal hydrogen carbonates such as calcium hydrogen carbonate and the like; alkali metal hydroxides such as sodium hydroxide and the like; alkaline earth metal hydroxides such as magnesium hydroxide and the like; alkali metal phosphates such as tripotassium phosphate and the like; metal alkoxides such as sodium tert-butoxide, potassium tert-butoxide and the like, and the like.

The additives that can be used are not particularly limited to those used as known methods. Preferred examples include metal oxides such as silver oxide and alumina; phosphines such as triphenylphosphine, tri-tert-butylphosphine, tricyclohexylphosphine, tri(o-toluyl)phosphine, diphenylphosphinoferrocene, 2-dicyclohexylphosphino-2',6'-dimethoxy-1,1'-biphenyl (S-PHOS), 2-dicyclohexylphosphino-2'-2',4',6'-triisopropyl-1,1'-biphenyl (X-PHOS), 2,2'-bis(diphenylphosphino)-1,1'-binaphthyl (BINAP) and the like; phosphine oxides such as triphenylphosphine oxide and the like; metal salts such as lithium chloride, potassium fluoride, cesium fluoride, and the like; ammonium salts such as tetrabutylammonium bromide, and the like. These may be used in any combination in any ratio.

The metal catalyst to be used is not particularly limited as long as it is used in a known method. Preferred examples include palladium catalysts such as tetrakis(triphenylphosphine)palladium, bis(tri-tert-butylphosphine)palladium, 2 palladium acetate, 2 palladium chloride diphenylphosphinoferrocene complex, 2 palladium chloride benzonitrile complex, 2 palladium chloride acetonitrile complex, bis(dibenzylideneacetone)palladium, tris(dibenzylideneacetone)dipalladium, bis[1,2-bis(diphenylphosphino)ethane]palladium, 3-chloropyridine[1,3-bis(2,6-diisopropylphenyl)imidazo-2-ylidene]palladium, palladium-activated carbon and the like.

The acrylic ester compounds to be used are not particularly limited, but include, for example, methyl acrylate, ethyl acrylate, tert-butyl acrylate and the like.

While the reaction temperature varies depending on the raw material compounds, reagents, and the like, it is generally 0°C to 150°C, preferably 20°C to 120°C.

While the reaction time varies depending on the raw material compounds, reagents, and the like, it is generally 5 min to 24 hr, preferably 10 min to 12 hr.

### (Step C-2)

In this step, compound (6) and compound (7) obtained by the below-mentioned method G are reacted using a metal catalyst in a solvent in the presence or absence of a base and in the presence or absence of an additive to produce compound (2C-1).

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; alcohols such as methanol, ethanol, tert-butanol and the like; nitriles such as acetonitrile and the like; amides such as formamide, N,N-dimethylformamide and the like; sulfoxides such as dimethyl sulfoxide and the like; mixed solvents containing a plurality of the organic solvents mentioned above in any ratio; mixed solvents containing the aforementioned organic solvent and water in any ratio and the like.

The base that can be used is not particularly limited as long as it is one that is used as a base in a normal reaction. Preferred examples include organic bases such as triethylamine, N,N-diisopropylethylamine, N-methylmorpholine, lutidine, pyridine and the like; alkali metal carbonates such as sodium carbonate, potassium carbonate and the like; alkaline earth metal carbonates such as magnesium carbonate and the like; alkali metal hydrogen carbonates such as potassium hydrogen carbonate and the like; alkaline earth metal hydrogen carbonates such as calcium hydrogen carbonate and the like; alkali metal hydroxides such as sodium hydroxide, potassium hydroxide and the like; alkaline earth metal hydroxides such as magnesium hydroxide and the like; alkali metal phosphates such as tripotassium phosphate and the like; metal alkoxides such as sodium tert-butoxide, potassium tert-butoxide and the like, and the like.

The additives that may be used are not particularly limited to those used as known methods. Preferred examples include phosphines such as 2,3-bis(diphenylphosphino)butane and the like, and different types of phosphines may be used in any combination in any ratio.

The metal catalysts to be used are not particularly limited to those used in known methods. Preferred examples include rhodium catalysts such as bis(norbornadiene)rhodium(I) tetrafluoroborate, chloro(1,5-cyclooctadiene)rhodium(I) dimer and the like, and the like.

While the reaction temperature varies depending on the raw material compounds, reagents, and the like, it is generally 0°C to 150°C, preferably 20°C to 100°C.

While the reaction time varies depending on the raw material compounds, reagents, and the like, it is generally 5 min to 24 hr, preferably 10 min to 12 hr.

### (Step C-3)

In this step, the hydroxy group of compound (2C-1) is converted to a leaving group (L¹) by reacting with an acid chloride or an acid anhydride in a solvent, in the presence or absence of a base, to produce compound (2A-1).

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; nitriles such as acetonitrile and the like; amides such as N,N-dimethylformamide and the like; sulfoxides such as dimethyl sulfoxide and the like; mixed solvents containing a plurality of the organic solvents mentioned above in any ratio.

The acid chlorides or acid anhydrides to be used are not particularly limited, but preferably include, for example, sulfurous chlorides such as thionyl chloride, and the like, substituted or unsubstituted alkylsulfonic anhydrides or arylsulfonic anhydrides such as trifluoromethanesulfonic anhydride, and the like,; substituted or unsubstituted alkylsulfonyl chlorides or arylsulfonyl chloride such as methanesulfonyl chloride, p-toluenesulfonyl chloride, and the like; substituted or unsubstituted alkyl chlorophosphate or aryl chlorophosphate, and the like.

The base that can be used is not particularly limited as long as it is one that is used as a base in a normal reaction. Preferred examples include organic bases such as triethylamine, N,N-diisopropylethylamine, N-methylmorpholine, lutidine, pyridine and the like; alkali metal carbonates such as sodium carbonate, potassium carbonate and the like; alkaline earth metal carbonates such as magnesium carbonate and the like; alkali metal hydrogen carbonates such as potassium hydrogen carbonate and the like; alkaline earth metal hydrogen carbonates such as calcium hydrogen carbonate and the like; alkali metal hydroxides such as sodium hydroxide and the like; alkaline earth metal hydroxides such as magnesium hydroxide and the like; alkali metal phosphates such as tripotassium phosphate and the like, and the like.

While the reaction temperature varies depending on the raw material compounds, reagents, and the like, it is generally -100°C to 150°C, preferably -80°C to 40°C.

While the reaction time varies depending on the raw material compounds, reagents, and the like, it is generally 5 min to 24 hr, preferably 10 min to 6 hr.

### (Step C-4)

In this step, the hydroxy group of compound (2C-1) is converted to a formyl group by using an appropriate oxidizing agent in a solvent to produce compound (2B-1).

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; nitriles such as acetonitrile and the like; amides such as formamide, N,N-dimethylformamide and the like; sulfoxides such as dimethyl sulfoxide and the like; mixed solvents containing a plurality of the organic solvents mentioned above in any ratio and the like.

The oxidizing agent to be used is not particularly limited. For example, a known method described in the fourth edition of Experimental Chemistry Course (21. Organic Synthesis III Aldehydes, Ketones, and Quinones), Kazuhiro Maruyama et al, Chemical Society of Japan, Maruzen Corporation, 1990, and the like can be appropriately selected. This step is performed according thereto. Representative examples of oxidation reactions include, for example, oxidation reaction using a chromic acid such as chromic anhydride, chromium oxide(VI)-pyridine complex (Collins reagent), pyridinium chlorochromate (PCC), pyridinium dichromate (PDC); oxidation reaction using activated manganese dioxide; oxidation using dicyclohexylcarbodiimide (DCC), acetic anhydride, phosphorus pentoxide, sulfur trioxide-pyridine complexes, or oxalyl chloride in combination with dimethyl sulfoxide (DMSO); oxidation reaction using a hypervalent iodine compound (Dess-Martin reagent), and the like.

While the reaction temperature varies depending on the raw material compounds, reagents, and the like, it is generally -100°C to 150°C, preferably 0°C to 50°C.

While the reaction time varies depending on the raw material compounds, reagents, and the like, it is generally 5 min to 24 hr, preferably 10 min to 6 hr.

### (Method D)

In this production method, compound (2A) and compound (2B) which are intermediate compounds used in the aforementioned Method A or Method B are produced. 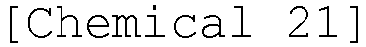 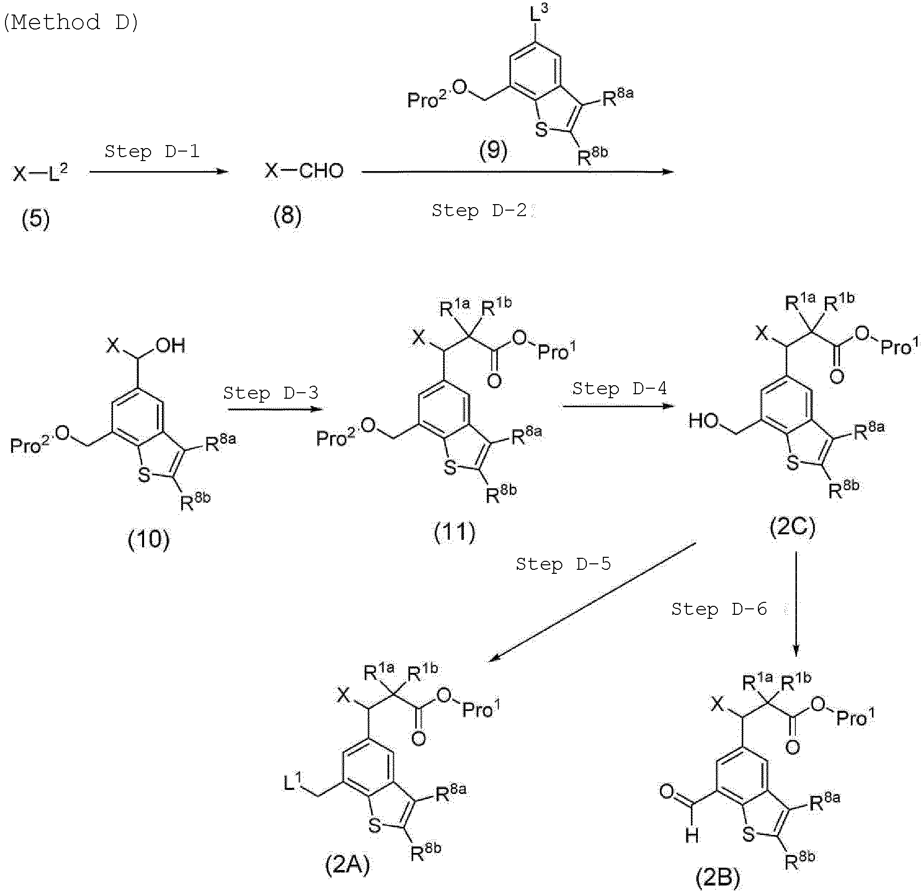 wherein L³ is a leaving group (preferably a halogen atom), Pro² is a protecting group (preferably a p-methoxybenzyl group.), and other symbols are as defined above.

### (Step D-1)

In this step, compound (5) is reacted with an organometallic compound and a formylating agent in a solvent to convert leaving group (L²) to a formyl group to produce compound (8). Here, the case where L² is a bromine atom is described, but the present invention is not limited thereto.

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; ethers such as diethyl ether, tetrahydrofuran, 1,4-dioxane, 1,2-dimethoxyethane and the like; sulfoxides such as dimethyl sulfoxide and the like; mixed solvents containing a plurality of the organic solvents mentioned above in any ratio and the like.

Examples of the organometallic compound to be used include organic lithium compounds such as n-butyllithium, tert-butyllithium and the like; mixtures of organolithium compounds with organomagnesium compounds such as methylmagnesium bromide, ethylmagnesium bromide, and the like in any ratio, and the like.

The formylating agent to be used is not particularly limited and, for example, N,N-di-substituted formamides such as N,N-dimethylformamide and the like; orthoformates such as trimethyl orthoformate and the like; N-ethoxymethyleneaniline and the like can be mentioned.

While the reaction temperature varies depending on the raw material compounds, reagents, and the like, it is generally -100°C to 100°C, preferably -80°C to 50°C.

While the reaction time varies depending on the raw material compounds, reagents, and the like, it is generally 5 min to 12 hr, preferably 10 min to 6 hr.

### (Step D-2)

In this step, leaving group (L³) of compound (9) is converted to a metal in a solvent in the presence of a base, and the compound is reacted with compound (8) obtained in step D-1 to produce compound (10). Here, the case where L³ is a bromine atom is described, but the present invention is not limited thereto.

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; ethers such as diethyl ether, tetrahydrofuran, 1,4-dioxane, 1,2-dimethoxyethane and the like; sulfoxides such as dimethyl sulfoxide and the like; mixed solvents containing a plurality of the organic solvents mentioned above in any ratio and the like.

The base that can be used is not particularly limited as long as it is one that is used as a base in a normal reaction. For example, n-butyllithium, sec-butyllithium, tert-butyllithium and the like can be mentioned.

While the reaction temperature varies depending on the raw material compounds, reagents, and the like, it is generally -100°C to 100°C, preferably -80°C to 50°C.

While the reaction time varies depending on the raw material compounds, reagents, and the like, it is generally 5 min to 24 hr, preferably 10 min to 6 hr.

### (Step D-3)

In this step, a hydroxy group of compound (10) is converted to a leaving group (Step D-3-1), and then converted to an acetate ester group (-C(R^{1a}) (R^{1b}) -CO₂Pro¹) (Step D-3-2) to produce compound (11).

### (Step D-3-1)

The leaving group is not particularly limited and, for example, (2,2,2-trichloroacetimidoyl)oxy group, chlorine atom, trifluoromethanesulfonyloxy group, methanesulfonyloxy group, tosyloxy group and the like can be mentioned. In the following, a method for converting a hydroxy group of compound (10) to a (2,2,2-trichloroacetimidoyl)oxy group by using trichloroacetonitrile in a solvent in the presence of a base is described, but the method is not limited thereto.

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; alcohols such as methanol, ethanol, tert-butanol, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; mixed solvents containing the organic solvents mentioned above and water in any ratio; and the like.

The base to be used is not particularly limited as long as it is one that is used as a base in a normal reaction. Preferred examples of the base include organic bases such as triethylamine, N,N-diisopropylethylamine, N-methylmorpholine, lutidine, pyridine, and the like; alkali metal carbonates such as sodium carbonate, potassium carbonate, and the like; alkaline earth metal carbonates such as magnesium carbonate, and the like; alkali metal hydrogen carbonates such as potassium hydrogen carbonate, and the like; alkaline earth metal hydrogen carbonates such as calcium hydrogen carbonate, and the like; alkali metal hydroxides such as sodium hydroxide, and the like; alkaline earth metal hydroxides such as magnesium hydroxide, and the like; alkali metal phosphates such as tripotassium phosphate; metal alkoxides such as sodium tert-butoxide, potassium tert-butoxide, and the like; and the like.

While the reaction temperature varies depending on the raw material compounds, reagents, and the like, it is generally -20°C to 150°C, preferably 0°C to 100°C.

While the reaction time varies depending on the raw material compounds, reagents, and the like, it is generally 5 min to 48 hr, preferably 10 min to 24 hr.

### (Step D-3-2)

In this step, the compound obtained in the aforementioned step D-3-1 is reacted with keteneacetals in a solvent in the presence of a catalyst to convert same to an acetate ester group (-C(R¹³)(R¹⁶)-CO₂Pro¹) to produce compound (11).

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; alcohols such as methanol, ethanol, tert-butanol, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; mixed solvents containing the organic solvents and water in any ratio; and the like.

Examples of the catalyst to be used include sulfonic anhydrides such as trifluoromethanesulfonic anhydride and the like, and the like.

Examples of the keteneacetals to be used include methyltrimethylsilyl dimethylketene acetal, 1-(tert-butyldimethylsilyloxy)-1-methoxyethene and the like.

While the reaction temperature varies depending on the raw material compounds, reagents, and the like, it is generally -20°C to 150°C, preferably 0°C to 100°C.

While the reaction time varies depending on the raw material compounds, reagents, and the like, it is generally 5 min to 48 hr, preferably 10 min to 24 hr.

### (Step D-4)

In this step, protecting group (Pro²) is removed from compound (11) in a solvent to convert same to compound (2C). In the following, as regards the case where Pro² is a p-methoxybenzyl group, a deprotection method using an oxidizing agent (Step D-4-1) and a deprotection method using an acid (Step D-4-2) are described, but the method is not limited thereto.

### (Step D-4-1) (Deprotection method using oxidizing agent)

In this step, p-methoxybenzyl group is removed from compound (11) in a solvent by using an appropriate oxidizing agent.

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; alcohols such as methanol, ethanol, tert-butanol, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; mixed solvents containing the organic solvents mentioned above and water in any ratio; and the like.

The oxidizing agent to be used is not particularly limited as long as it is used as an oxidizing agent in general reactions. For example, 2,3-dichloro-5,6-dicyano-p-benzoquinone and the like can be mentioned.

While the reaction temperature varies depending on the raw material compounds, reagents, and the like, it is generally -20°C to 150°C, preferably 0°C to 100°C.

While the reaction time varies depending on the raw material compounds, reagents, and the like, it is generally 5 min to 24 hr, preferably 10 min to 12 hr.

### (Step D-4-2) (Deprotection method using acid)

In this step, p-methoxybenzyl group is removed from compound (11) by using an appropriate acid in a solvent or without solvent, in the presence or absence of anisole.

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; alcohols such as methanol, ethanol, tert-butanol, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; mixed solvents containing the organic solvents mentioned above and water in any ratio; and the like.

The acid to be used is not particularly limited as long as it is an acid that is used in normal reactions. Examples thereof include inorganic acids such as hydrochloric acid, sulfuric acid and the like; Lewis acids such as boron trifluoride, boron trichloride, boron tribromide, iodotrimethylsilane and the like; and organic acids such as trifluoroacetic acid and the like.

While the reaction temperature varies depending on the raw material compounds, reagents, and the like, it is generally -100°C to 150°C, preferably -78°C to 100°C.

While the reaction time varies depending on the raw material compounds, reagents, and the like, it is generally 5 min to 24 hr, preferably 10 min to 6 hr.

### (Step D-5)

In this step, acid chloride or acid anhydride is reacted with compound (2C) in a solvent in the presence or absence of a base to convert the hydroxy group of compound (2C) to a leaving group (L¹) to produce compound (2A), and the reaction can be performed under the conditions similar to those in the aforementioned step C-3.

### (Step D-6)

In this step, the hydroxy group of compound (2C) is converted to a formyl group in a solvent by using an appropriate oxidizing agent to produce compound (2B), and the reaction can be performed under the conditions similar to those in the aforementioned step C-4.

### (Method E)

In this production method, compound (3) (compound (3A) wherein W is an oxygen atom), which is an intermediate compound used in the aforementioned method A and method B, is produced. 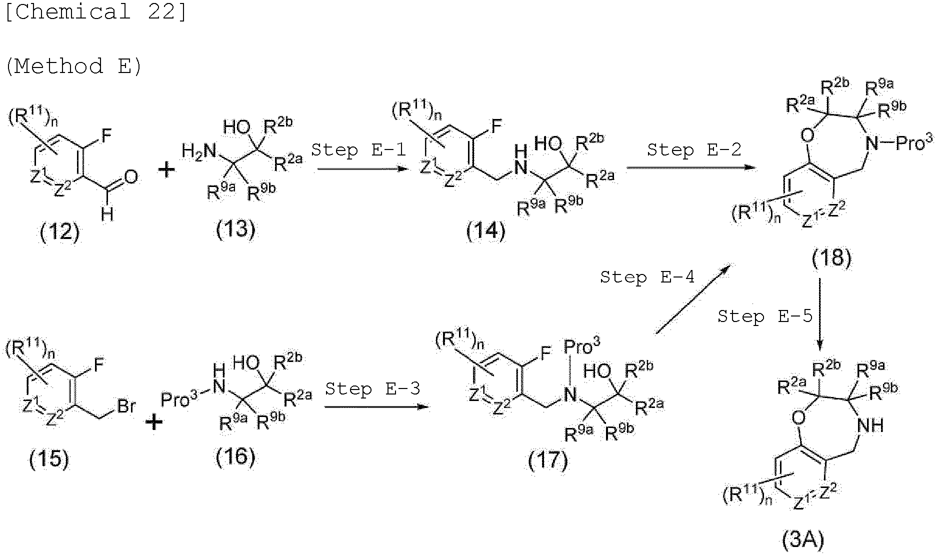 wherein Pro³ is a protecting group (preferably a tert-butoxycarbonyl group or a p-methoxybenzyl group), and other symbols are as defined above.

### (Step E-1)

In this step, compound (14) is produced by reductive amination of compound (12) and compound (13), which can be performed according to the aforementioned step B-1, but the method is not limited thereto.

### (Step E-2)

In this step, compound (14) is converted to compound (18) and a step of protecting an amino group with a protecting group (Pro³) (Step E-2a), and a step of intramolecular cyclization to construct a 7-membered ring (Step E-2b) are included. Either Step E-2a or Step E-2b may be performed first, and a person skilled in the art may select the order thereof as appropriate.

### (Step E-2a)

In this step, an amino group of compound (14) is protected by a protecting group (Pro³), and the step is performed according to a known method appropriately selected from, for example, P. G. Wuts, "Protective Groups in Organic Synthesis", 5th Edition, Wiley, 2014, and the like, depending on the Pro³ to be used. In the following, a protection method (Step E-2a-1) when Pro³ is a tert-butoxycarbonyl group and a protection method (Step E-2a-2) when Pro³ is protected by a p-methoxybenzyl group are described; however, this step is not limited thereto.

### (Step E-2a-1)

In this step, a tert-butoxycarbonyl group (protecting group) is introduced by using a protecting reagent in a solvent in the presence of an appropriate base.

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; alcohols such as methanol, ethanol, tert-butanol and the like; nitriles such as acetonitrile and the like; amides such as formamide, N,N-dimethylformamide and the like; sulfoxides such as dimethyl sulfoxide and the like; mixed solvents containing a plurality of the organic solvents mentioned above in any ratio; mixed solvents containing the aforementioned organic solvent and water in any ratio and the like.

The base to be used is not particularly limited as long as it is one that is used as a base in a normal reaction. Preferred examples of the base include organic bases such as triethylamine and the like; alkali metal alkoxides such as potassium tert-butoxide, sodium tert-butoxide and the like; alkali metal carbonates such as sodium carbonate, potassium carbonate and the like; alkaline earth metal carbonates such as magnesium carbonate and the like; alkali metal hydrogen carbonates such as potassium hydrogen carbonate and the like; alkaline earth metal hydrogen carbonates such as calcium hydrogen carbonate and the like; alkali metal hydroxides such as sodium hydroxide, potassium hydroxide and the like; alkaline earth metal hydroxides such as magnesium hydroxide and the like; alkali metal phosphates such as tripotassium phosphate and the like, and the like.

Examples of the protecting reagent to be used include di-tert-butyl dicarbonate and the like.

While the reaction temperature varies depending on the raw material compounds, reagents, and the like, it is generally -100°C to 150°C, preferably 0°C to 50°C.

While the reaction time varies depending on the reaction temperature, raw material compounds, reaction reagents or the kind of the solvent to be used, it is generally 5 min to 24 hr, preferably 10 min to 6 hr.

### (Step E-2a-2)

In this step, a p-methoxybenzyl group (protecting group) is introduced. In the following, a method of introducing p-methoxybenzyl group by reductive amination (Step E-2a-2-1), and a method of introducing p-methoxybenzyl group by alkylation (Step E-2a-2-2) are described, but the method is not limited thereto.

### (Step E-2a-2-1)

In this step, a p-methoxybenzyl group is introduced by reductive amination using p-methoxybenzaldehyde and an appropriate reducing agent in a solvent in the presence or absence of an acid.

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; and the like.

The reducing agent to be used is not particularly limited and, for example, sodium borohydride, sodium cyanoborohydride, sodium triacetoxyborohydride, picoline borane, pyridineborane and the like can be mentioned.

The acid that can be used is not particularly limited as long as it is an acid that is used in normal reactions. Examples thereof include Lewis acids such as boron trifluoride, boron trichloride, boron tribromide, iodotrimethylsilane and the like; organic acids such as acetic acid, trifluoroacetic acid and the like; inorganic acids such as hydrochloric acid and the like; and the like.

While the reaction temperature varies depending on the raw material compounds, reagents, and the like, it is generally -100°C to 150°C, preferably 0°C to 50°C.

While the reaction time varies depending on the raw material compounds, reagents, and the like, it is generally 5 min to 24 hr, preferably 10 min to 18 hr.

### (Step E-2a-2-2)

In this step, a p-methoxybenzyl group is introduced using an alkylating agent such as p-methoxybenzyl chloride, p-methoxybenzylbromide and the like in a solvent in the presence of an appropriate base.

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; and the like.

The base to be used is not particularly limited as long as it is one that is used as a base in a normal reaction. Preferred examples of the base include organic bases such as triethylamine and the like; alkali metal alkoxides such as potassium tert-butoxide, sodium tert-butoxide and the like; alkali metal carbonates such as sodium carbonate, potassium carbonate and the like; alkaline earth metal carbonates such as magnesium carbonate and the like; alkali metal hydrogen carbonates such as potassium hydrogen carbonate and the like; alkaline earth metal hydrogen carbonates such as calcium hydrogen carbonate and the like; alkali metal hydroxides such as sodium hydroxide, potassium hydroxide and the like; alkaline earth metal hydroxides such as magnesium hydroxide and the like; alkali metal phosphates such as tripotassium phosphate and the like, and the like.

While the reaction temperature varies depending on the raw material compounds, reagents, and the like, it is generally -100°C to 200°C, preferably 0°C to 150°C.

While the reaction time varies depending on the raw material compounds, reagents, and the like, it is generally 5 min to 48 hr, preferably 10 min to 24 hr.

### (Step E-2b)

In this step, a 7-membered ring is constructed by intramolecular cyclization using an appropriate base in a solvent.

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; and the like.

The base to be used is not particularly limited as long as it is one that is used as a base in a normal reaction. Preferred examples of the base include organic bases such as triethylamine and the like; alkali metal alkoxides such as potassium tert-butoxide, sodium tert-butoxide and the like; alkali metal carbonates such as sodium carbonate, potassium carbonate and the like; alkaline earth metal carbonates such as magnesium carbonate and the like; alkali metal hydrogen carbonates such as potassium hydrogen carbonate and the like; alkaline earth metal hydrogen carbonates such as calcium hydrogen carbonate and the like; alkali metal hydroxides such as sodium hydroxide, potassium hydroxide and the like; alkaline earth metal hydroxides such as magnesium hydroxide and the like; alkali metal phosphates such as tripotassium phosphate and the like, and the like.

While the reaction temperature varies depending on the raw material compounds, reagents, and the like, it is generally -100°C to 150°C, preferably 0°C to 50°C.

While the reaction time varies depending on the raw material compounds, reagents, and the like, it is generally 5 min to 24 hr, preferably 10 min to 6 hr.

### (Step E-3)

In this step, a nitrogen atom of compound (16) is alkylated using compound (15) in a solvent in the presence of an appropriate base.

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; and the like.

The base to be used is not particularly limited as long as it is one that is used as a base in a normal reaction. Preferred examples of the base include organic bases such as triethylamine and the like; alkali metal alkoxides such as potassium tert-butoxide, sodium tert-butoxide and the like; alkali metal carbonates such as sodium carbonate, potassium carbonate and the like; alkaline earth metal carbonates such as magnesium carbonate and the like; alkali metal hydrogen carbonates such as potassium hydrogen carbonate and the like; alkaline earth metal hydrogen carbonates such as calcium hydrogen carbonate and the like; alkali metal hydroxides such as sodium hydroxide, potassium hydroxide and the like; alkaline earth metal hydroxides such as magnesium hydroxide and the like; alkali metal phosphates such as tripotassium phosphate and the like, and the like.

While the reaction temperature varies depending on the raw material compounds, reagents, and the like, it is generally -100°C to 150°C, preferably 0°C to 100°C.

While the reaction time varies depending on the raw material compounds, reagents, and the like, it is generally 5 min to 48 hr, preferably 10 min to 24 hr.

### (Step E-4)

In this step, compound (17) is converted to compound (18) by intramolecular cyclization according to the aforementioned step E-2b, but the method is not limited thereto.

### (Step E-5)

In this step, the protecting group (Pro³) is removed from compound (18). A known method described in, for example, P. G. Wuts, "Protective Groups in Organic Synthesis", 5th Edition, Wiley, 2014, and the like is appropriately selected according to the protecting group to be used, and the step is performed according to the method. Here, a method of removing the protecting group by using an acid in a solvent (Step E-5-1), and a method of removing the protecting group by using a catalyst in a solvent under a hydrogen atmosphere (Step E-5-2) are described, but the method is not limited thereto.

### (Step E-5-1)

In this step, the protecting group is removed using an appropriate acid in a solvent in the presence or absence of anisole.

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; alcohols such as methanol, ethanol and the like; nitriles such as acetonitrile and the like; amides such as formamide, N,N-dimethylformamide and the like; sulfoxides such as dimethyl sulfoxide and the like; mixed solvents containing a plurality of the organic solvents mentioned above in any ratio; mixed solvents containing the aforementioned organic solvent and water in any ratio and the like.

The acid to be used is not particularly limited as long as it is an acid that is used in normal reactions for example, inorganic acids such as hydrochloric acid, sulfuric acid and the like; Lewis acids such as boron trifluoride, boron trichloride, boron tribromide, iodotrimethylsilane and the like; organic acids such as trifluoroacetic acid and the like, and the like can be mentioned.

While the reaction temperature varies depending on the raw material compounds, reagents, and the like, it is generally -100°C to 150°C, preferably at -78°C to 100°C.

While the reaction time varies depending on the raw material compounds, reagents, and the like, it is generally 5 min to 24 hr, preferably 10 min to 6 hr.

### (Step E-5-2)

In this step, the protecting group is removed using a catalyst under a hydrogen atmosphere in a solvent in the presence or absence of an additive.

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; alcohols such as methanol, ethanol and the like; nitriles such as acetonitrile and the like; amides such as formamide, N,N-dimethylformamide and the like; sulfoxides such as dimethyl sulfoxide and the like; mixed solvents containing a plurality of the organic solvents mentioned above in any ratio; mixed solvents containing the aforementioned organic solvent and water in any ratio and the like.

The additive that may be used is not particularly limited as long as it is used in a known method, such as hydrochloric acid and the like.

The catalyst to be used is not particularly limited as long as it is used in a known method. Preferred examples include metal catalysts such as palladium-activated carbon, tris(triphenylphosphine)rhodium chloride, palladium hydroxide and the like.

While the reaction temperature varies depending on the raw material compounds, reagents, and the like, it is generally -100°C to 150°C, preferably 0°C to 100°C.

While the reaction time varies depending on the raw material compounds, reagents, and the like, it is generally 1 min to 24 hr, preferably 5 min to 10 hr.

### (Method F)

In this production method, compound (3) (compound (3B)), which is an intermediate compound used in the aforementioned method A and method B, is produced. 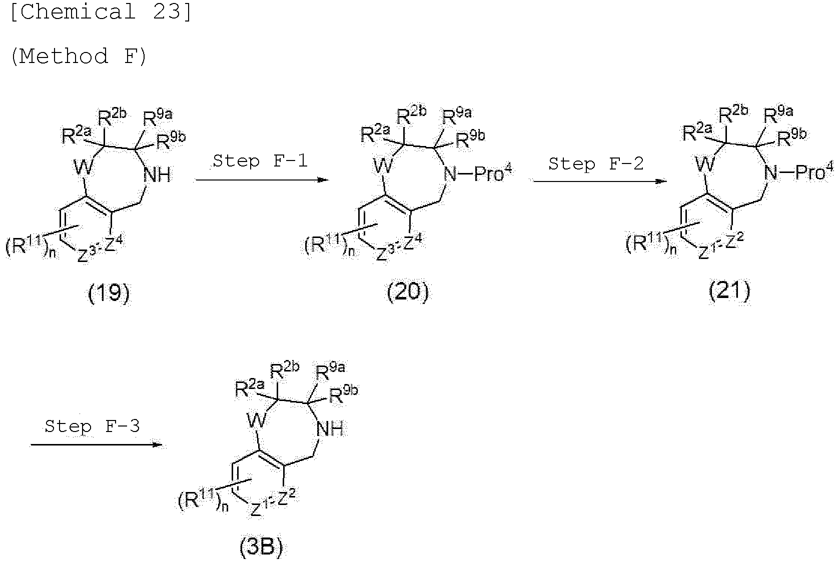 wherein W is -CH₂- or -CHR¹⁰- (R¹⁰ is as defined above), Z³ and Z⁴ are precursor groups of Z¹ and Z², respectively (a group having no substituent or a leaving group bound thereto), Pro⁴ is an amino-protecting group selected by a known method (e.g., P. G. Wuts, "Protective Groups in Organic Synthesis", 5th Edition, Wiley, 2014), which is not particularly limited as long as it is stably present during the reaction and does not inhibit the reaction, but is preferably a tert-butoxycarbonyl group, and other symbols are as defined above.

### (Step F-1)

In this step, an amino group of compound (19) is protected with a protecting group (Pro⁴). The step is performed according to the aforementioned step E-2a, but the method is not limited thereto.

### (Step F-2)

In this step, compound (21) is produced according to a known functional group conversion method, for example, the methods described in "ORGANIC FUNCTIONAL GROUP PREPARATIONS", 2nd edition, ACADEMIC PRESS, INC., 1989, "Comprehensive Organic Transformations", 2nd edition, VCH Publishers Inc., 1999 and the like, and the like. Those of ordinary skill in the art can appropriately convert the compound by combining known methods.

### (Step F-3)

In this step, the protecting group (Pro⁴) is removed. The step is performed according to the aforementioned step E-2c, but the method is not limited thereto.

### (Method G)

In this production method, compound (7) which is an intermediate compound used in the aforementioned method C, is produced from compound (22). 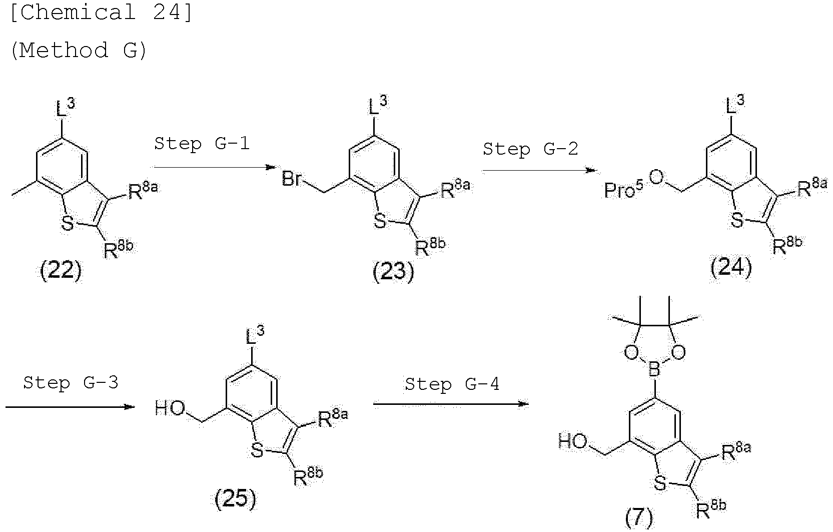 wherein Pro⁵ is a hydroxy-protecting group selected by a known method (e.g., P. G. Wuts, "Protective Groups in Organic Synthesis", 5th Edition, Wiley, 2014), which is not particularly limited as long as it is stably present during the reaction and does not inhibit the reaction, but is preferably an acetyl group, and other symbols are as defined above.

### (Step G-1)

In this step, compound (22) is brominated using a brominating agent in a solvent in the presence of a catalyst to produce compound (23).

The solvent to be used is not particularly limited as long as it does not inhibit the reaction and dissolves the starting materials to some extent, and preferred examples include aromatic hydrocarbons such as benzene and the like; halogenated hydrocarbons such as dichloromethane, chloroform, carbon tetrachloride and the like; esters such as ethyl acetate, propyl acetate and the like; ethers such as diethyl ether, tetrahydrofuran, 1,4-dioxane, 1,2-dimethoxyethane and the like; nitriles such as acetonitrile and the like; amides such as formamide, N,N-dimethylformamide and the like; sulfoxides such as dimethyl sulfoxide and the like; mixed solvents containing a plurality of the organic solvents mentioned above in any ratio and the like.

Examples of the catalyst to be used include radical initiators such as azobisisobutyronitrile, benzoyl peroxide and the like.

The brominating agent to be used is not particularly limited and, for example, N-bromosuccinimide, N-bromoacetamide, and the like can be mentioned.

While the reaction temperature varies depending on the raw material compounds, reagents, and the like, it is generally 0°C to 200°C, preferably 20°C to 150°C.

While the reaction time varies depending on the raw material compounds, reagents, and the like, it is generally 5 min to 24 hr, preferably 10 min to 12 hr.

### (Step G-2)

In this step, a bromine atom of compound (23) is converted to a hydroxy group protected with a protecting group (Pro⁵) to produce compound (24). Here, a method using metal carboxylates in a solvent is described, but the method is not limited thereto.

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; and the like.

Examples of the metal carboxylates to be used include potassium acetate, sodium acetate, potassium benzoate, sodium benzoate and the like.

While the reaction temperature varies depending on the raw material compounds, reagents, and the like, it is generally 0°C to 150°C, preferably 20°C to 100°C.

While the reaction time varies depending on the raw material compounds, reagents, and the like, it is generally 5 min to 48 hr, preferably 10 min to 24 hr.

### (Step G-3)

In this step, the protecting group (Pro⁵) of compound (24) is removed, which can be performed according to the aforementioned step A-2.

### (Step G-4)

In this step, the leaving group (L³) of compound (25) is converted to a borate ester by using an appropriate metal catalyst and a boron compound under an inert gas (nitrogen or argon) atmosphere in a solvent in the presence or absence of a base and in the presence or absence of an additive to produce compound (7).

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; nitriles such as acetonitrile and the like; amides such as formamide, N,N-dimethylformamide and the like; sulfoxides such as dimethyl sulfoxide and the like; mixed solvents containing a plurality of the organic solvents mentioned above in any ratio and the like.

The boron compound to be used is not particularly limited as long as it is generally used in borate ester synthesis. For example, it is preferably bis(pinacolato)diboron, pinacolborane or the like.

The base that can be used is not particularly limited as long as it is one that is used as a base in a normal reaction. Preferred examples include organic bases such as triethylamine, N,N-diisopropylethylamine and the like; alkali metal carbonates such as sodium carbonate, potassium carbonate and the like; alkaline earth metal carbonates such as magnesium carbonate and the like; alkali metal hydrogen carbonates such as sodium hydrogen carbonate and the like; alkaline earth metal hydrogen carbonates such as calcium hydrogen carbonate and the like; alkali metal hydroxides such as sodium hydroxide, cesium hydroxide and the like; alkaline earth metal hydroxides such as magnesium hydroxide and the like; alkali metal acetates such as sodium acetate, potassium acetate and the like; alkali metal phosphates such as tripotassium phosphate and the like; metal alkoxides such as sodium tert-butoxide, potassium tert-butoxide and the like, and the like.

The additives that may be used are not particularly limited to those used as known methods. Preferred examples include metal oxides such as silver oxide, alumina and the like; phosphines such as triphenylphosphine, tri-tert-butylphosphine, tricyclohexylphosphine, tri(o-toluyl)phosphine, diphenylphosphinoferrocene, 2-dicyclohexylphosphino-2',6'-dimethoxy-1,1'-biphenyl (S-PHOS), 2-dicyclohexylphosphino-2',4',6'-triisopropyl-1,1'-biphenyl (X-PHOS), 2,2'-bis(diphenylphosphino)-1,1'-binaphthyl (BINAP) and the like; phosphine oxides such as triphenylphosphine oxide and the like; metal salts such as lithium chloride, potassium fluoride, cesium fluoride and the like; ammonium salts such as tetrabutylammonium bromide and the like, and the like. These may be used in any combination in any ratio.

The metal catalyst to be used is not particularly limited as long as it is used in a known method. Preferred examples include palladium catalysts such as tetrakis(triphenylphosphine)palladium, bis(tri-tert-butylphosphine)palladium, 2 palladium acetate, 2 palladium chloride diphenylphosphinoferrocene complex, 2 palladium chloride benzonitrile complex, 2 palladium chloride acetonitrile complex, bis(dibenzylideneacetone)palladium, tris(dibenzylideneacetone)dipalladium, bis[1,2-bis(diphenylphosphino)ethane]palladium, [1,1'-bis(diphenylphosphino)ferrocene]dichloropalladium(II) dichloromethane adduct, 3-chloropyridine[1,3-bis(2,6-diisopropylphenyl)imidazo-2-ylidene] palladium, palladium-activated carbon and the like, and the like.

While the reaction temperature varies depending on the raw material compounds, reagents, and the like, it is generally -10°C to 200°C, preferably 0°C to 150°C.

While the reaction time varies depending on the raw material compounds, reagents, and the like, it is generally 5 min to 48 hr, preferably 10 min to 12 hr.

### (Method H)

In this production method, compound (5A1) which is compound (5) (an intermediate compound used in the aforementioned method C and method D) wherein X is a group represented by the aforementioned formula (A1) and L² is a bromine atom is produced. 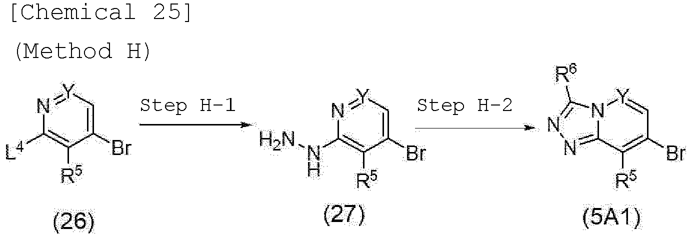 wherein L⁴ is a leaving group (preferably a halogen atom), and other symbols are as defined above.

### (Step H-1)

In this step, the leaving group (L⁴) of compound (26) is converted to a hydrazino group by using hydrazine monohydrate in a solvent or without a solvent to produce compound (27).

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; alcohols such as methanol, ethanol, tert-butanol and the like; nitriles such as acetonitrile and the like; amides such as formamide, N,N-dimethylformamide and the like; sulfoxides such as dimethyl sulfoxide and the like; organic bases such as pyridine and the like; mixed solvents containing a plurality of the organic solvents mentioned above in any ratio; mixed solvents containing the aforementioned organic solvent and water in any ratio and the like.

While the reaction temperature varies depending on the raw material compounds, reagents, and the like, it is generally -20°C to 200°C, preferably 0°C to 150°C.

While the reaction time varies depending on the raw material compounds, reagents, and the like, it is generally 5 min to 48 hr, preferably 10 min to 24 hr.

### (Step H-2)

In this step, compound (5A1) is produced from compound (27) by using appropriate carboxylic acid anhydrides, carboxylic acids, aliphatic nitro compounds or ortho esters corresponding to R⁶ in a solvent in the presence or absence of an acid.

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; and the like.

The acid to be used is not particularly limited as long as it is an acid that is used in normal reactions. Preferred examples include inorganic acids such as hydrochloric acid, sulfuric acid, ammonium chloride and the like; Lewis acids such as boron trifluoride, boron trichloride, boron tribromide, iodotrimethylsilane and the like; organic acids such as acetic acid, trifluoroacetic acid and the like, and the like.

While the reaction temperature varies depending on the raw material compounds, reagents, and the like, it is generally 0°C to 250°C, preferably 20°C to 200°C.

While the reaction time varies depending on the raw material compounds, reagents, and the like, it is generally 5 min to 48 hr, preferably 10 min to 24 hr.

### (Method J)

In this production method, compound (5A2) which is compound (5) (an intermediate compound used in the aforementioned method C and method D) wherein X is a group represented by the aforementioned formula (A2) and L² is a bromine atom is produced. 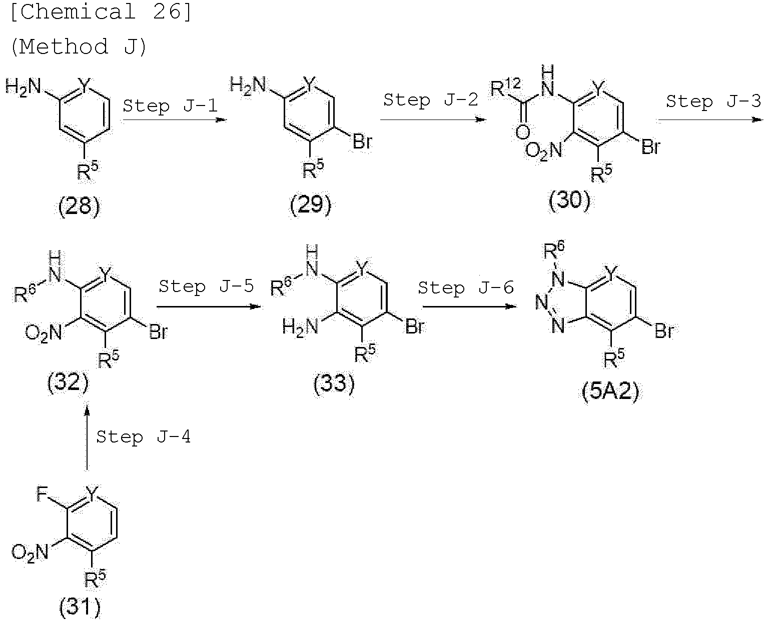 wherein R¹² is a hydrogen atom or an alkyl group, and other symbols are as defined above.

### (Step J-1)

In this step, compound (28) is brominated using an appropriate brominating agent in a solvent to produce compound (29).

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; nitriles such as acetonitrile and the like; amides such as formamide, N,N-dimethylformamide and the like; sulfoxides such as dimethyl sulfoxide and the like; organic acids such as acetic acid and the like; mixed solvents containing a plurality of the organic solvents mentioned above in any ratio and the like.

The brominating agent to be used is not particularly limited and, for example, N-bromosuccinimide, bromine, and the like can be mentioned.

While the reaction temperature varies depending on the raw material compounds, reagents, and the like, it is generally -100°C to 150°C, preferably -20°C to 50°C.

While the reaction time varies depending on the raw material compounds, reagents, and the like, it is generally 5 min to 48 hr, preferably 10 min to 24 hr.

### (Step J-2)

In this step, compound (29) is converted to compound (30), and a step of amidating an amino group (Step J-2a) and a step of nitrating (Step J-2b) are included. Either Step J-2a or Step J-2b may be performed first, and those of ordinary skill in the art can easily select the order thereof as appropriate.

### (Step J-2a) (amidation step)

Here, a method in which formic acid (R¹² corresponds to a hydrogen atom) is used for compound (29) (or compound (29) in a nitrated form) in a solvent in the presence or absence of an appropriate acid anhydride (Step J-2a-1), and a method in which compound (29) (or compound (29) in a nitrated form) and alkylcarboxylic acid (R¹² corresponds to an alkyl group, R¹²CO₂H) are used with an appropriate condensing agent in the presence or absence of a base (Step J-2a-2) are described, but the method is not limited thereto.

### (Step J-2a-1) (method using formic acid)

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; and the like.

The acid anhydride that can be used is not particularly limited as long as it is used as an acid anhydride in a normal reaction, and examples thereof include carboxylic acid anhydrides such as acetic anhydride and the like.

While the reaction temperature varies depending on the raw material compounds, reagents, and the like, it is generally -100°C to 150°C, preferably 0°C to 50°C.

While the reaction time varies depending on the raw material compounds, reagents, and the like, it is generally 5 min to 48 hr, preferably 10 min to 24 hr.

### (Step J-2a-2) (method using carboxylic acid and condensing agent)

The solvent to be used is not particularly limited as long as it does not inhibit the reaction and dissolves the starting materials to some extent, and preferred examples include aromatic hydrocarbons such as benzene, toluene, xylene, and the like; halogenated hydrocarbons such as dichloromethane, chloroform, and the like; esters such as ethyl acetate, propyl acetate, and the like; ethers such as diethyl ether, tetrahydrofuran, 1,4-dioxane, 1,2-dimethoxyethane, and the like; alcohols such as methanol, ethanol, tert-butanol, and the like; nitriles such as acetonitrile, and the like; amides such as formamide, N,N-dimethylformamide, and the like; sulfoxides such as dimethyl sulfoxide, and the like; mixed solvents containing a plurality of the organic solvents mentioned above in any ratio; mixed solvents containing the organic solvents mentioned above and water in any ratio; and the like.

The base to be used is not particularly limited as long as it is one that is used as a base in a normal reaction. Preferred examples of the base include organic bases such as triethylamine, N,N-diisopropylethylamine, N-methylmorpholine, lutidine, pyridine, and the like; alkali metal carbonates such as sodium carbonate, potassium carbonate, and the like; alkaline earth metal carbonates such as magnesium carbonate, and the like; alkali metal hydrogen carbonates such as potassium hydrogen carbonate, and the like; alkaline earth metal hydrogen carbonates such as calcium hydrogen carbonate, and the like; alkali metal hydroxides such as sodium hydroxide, and the like; alkaline earth metal hydroxides such as magnesium hydroxide, and the like; alkali metal phosphates such as tripotassium phosphate, and the like.

The condensing agent to be used is not particularly limited to those used as condensing agents to form amide bonds (e.g., methods described in Shoichi Kusumoto et al. Experimental Science Course IV; Chemical Society of Japan; Maruzen, 1990, and Nobuo Izumiya et al. Fundamentals and Experiments of Peptide Synthesis; Maruzen, 1985. etc.). Preferably, for example, 1-[bis(dimethylamino)methylene]-1H-1,2,3-triazolo[4,5-b]pyridinium 3-oxide hexafluorophosphate (HATU), O-benzotriazole-N,N,N' ,N'-tetramethyluronium hexafluorophosphate (HBTU), 2-(1H-benzotriazol-1-yl)-1,1,3,3-tetramethyluronium tetrafluoroborate (TBTU), 1-(3-dimethylaminopropyl)-3-ethylcarbodiimide hydrochloride (EDCI), 4-(2-{[(cyclohexylimino)methylene]amino}ethyl-4-methylmorpholinium p-toluenesulfonate (CMC), dicyclohexylcarbodiimide (DCC), 1,1'-carbonyl bis(1H-imidazole) (CDI), (1H-benzotriazol-1-yloxy) (tripyrrolidin-1-yl)phosphonium hexafluorophosphate (PyBOP), bromo(tripyrrolidin-1-yl)phosphonium hexafluorophosphate (PyBrOP), 4-(4,6-dimethoxy-1,3,5-triazin-2-yl)-4-methylmorpholinium chloride (DMT-MM), 2-chloro-4,6-dimethoxy-1,3,5-triazine (DMT), and the like can be mentioned. Where necessary, additives such as 1-hydroxybenzotriazole (HOBT) and N,N-dimethylaminopyridine, and the like may be added.

While the reaction temperature varies depending on the raw material compounds, reagents, and the like, it is generally -10°C to 150°C, preferably 0°C to 100°C.

While the reaction time varies depending on the raw material compounds, reagents, and the like, it is generally 5 min to 48 hr, preferably 10 min to 24 hr.

### (Step J-2b) (nitration step)

In this step, compound (29) (or an amidated form of compound (29)) is nitrated using an appropriate nitrating agent in a solvent in the presence or absence of an appropriate acid.

The solvent to be used is not limited as long as it does not inhibit the reaction and dissolves the starting material to some extent, but it is preferable to use an acid that also serves as a solvent, for example, mineral acids such as sulfuric acid; mixed solvents containing plural solvents in any ratio.

The nitrating agent to be used is not limited as long as it is used as a nitrating agent in normal reactions. For example, mineral acids such as nitric acid and the like, and the like can be mentioned.

While the reaction temperature varies depending on the raw material compounds, reagents, and the like, it is generally -100°C to 150°C, preferably -20°C to 50°C.

While the reaction time varies depending on the raw material compounds, reagents, and the like, it is generally 5 min to 24 hr, preferably 10 min to 6 hr.

### (Step J-3)

In this step, compound (30) is reduced with an appropriate reducing agent in a solvent to convert to compound (32).

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; nitriles such as acetonitrile and the like; amides such as formamide, N,N-dimethylformamide and the like; sulfoxides such as dimethyl sulfoxide and the like; mixed solvents containing a plurality of the organic solvents mentioned above in any ratio.

The reducing agents to be used are not limited to those used as reducing agents in normal reactions. For example, metal hydrides such as lithium aluminum hydride, diisobutylaluminum hydride, sodium bis(2-methoxyethoxy)aluminum dihydride; borone hydrides such as borane; silicon hydrides such as triethylsilane, and the like can be mentioned.

While the reaction temperature varies depending on the raw material compounds, reagents, and the like, it is generally -100°C to 150°C, preferably 0°C to 80°C.

While the reaction time varies depending on the raw material compounds, reagents, and the like, it is generally 5 min to 24 hr, preferably 10 min to 6 hr.

### (Step J-4)

In this step, the fluorine atom of compound (31) is converted to the NHR⁶ group (Step J-4-1), and then the bromine atom is introduced (Step J-4-2) (or fluorine atom is converted to NHR⁶ group after introduction of the bromine atom) to produce compound (32). In Step J-4, either Step J-4-1 or Step J-4-2 may be performed first, and a person skilled in the art may select the order thereof as appropriate.

### (Step J-4-1)

In this step, the fluorine atom is converted to an NHR⁶ group by reacting with a primary amine (R⁶NH₂ (wherein R⁶ is as defined above)) in a solvent in the presence or absence of a base.

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; nitriles such as acetonitrile and the like; amides such as formamide, N,N-dimethylformamide and the like; alcohols such as methanol, ethanol and the like; sulfoxides such as dimethyl sulfoxide and the like; mixed solvents containing a plurality of the organic solvents mentioned above in any ratio; mixed solvents containing the aforementioned organic solvent and water in any ratio and the like.

The base to be used is not particularly limited as long as it is one that is used as a base in a normal reaction. Preferred examples include organic bases such as triethylamine, N,N-diisopropylethylamine, N-methylmorpholine, lutidine, pyridine and the like; alkali metal carbonates such as sodium carbonate, potassium carbonate and the like; alkaline earth metal carbonates such as magnesium carbonate and the like; alkali metal hydrogen carbonates such as potassium hydrogen carbonate and the like; alkaline earth metal hydrogen carbonates such as calcium hydrogen carbonate and the like; alkali metal hydroxides such as sodium hydroxide and the like; alkaline earth metal hydroxides such as magnesium hydroxide and the like; alkali metal phosphates such as tripotassium phosphate and the like; metal alkoxides such as sodium tert-butoxide, potassium tert-butoxide and the like, and the like.

While the reaction temperature varies depending on the raw material compounds, reagents, and the like, it is generally -10°C to 150°C, preferably 0°C to 100°C.

While the reaction time varies depending on the raw material compounds, reagents, and the like, it is generally 5 min to 48 hr, preferably 10 min to 12 hr.

### (Step J-4-2)

This step involves the introduction of a bromine atom in a solvent. In this step, the reaction can be carried out under the same conditions as in the aforementioned step J-1.

### (Step J-5)

This step is the step for producing compound (33) by converting a nitro group of compound (32) to an amino group in a solvent.

The methods for converting the nitro group of compound (32) to an amino group include the methods described below, but are not limited to, those using an appropriate metal and an appropriate acid (Step J-5-1), hydrogen in the presence of an appropriate metal catalyst (Step J-5-2), and a metal hydride (Step J-5-3).

### (Step J-5-1)

This step is a step of converting a nitro group of compound (32) to an amino group using a metal in the presence of an acid in a solvent.

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; alcohols such as methanol, ethanol, tert-butanol and the like; nitriles such as acetonitrile and the like; amides such as formamide, N,N-dimethylformamide and the like; sulfoxides such as dimethyl sulfoxide and the like; mixed solvents containing a plurality of the organic solvents mentioned above in any ratio; mixed solvents containing the aforementioned organic solvent and water in any ratio and the like.

The acid to be used is not particularly limited as long as it is an acid that is used in normal reactions. Preferred examples include inorganic acids such as hydrochloric acid, sulfuric acid, ammonium chloride and the like; Lewis acids such as boron trifluoride, boron trichloride, boron tribromide, iodotrimethylsilane and the like; organic acids such as acetic acid, trifluoroacetic acid and the like, and the like.

The metal to be used is not particularly limited and, for example, iron, zinc, tin and the like can be mentioned.

While the reaction temperature varies depending on the raw material compounds, reagents, and the like, it is generally -10°C to 150°C, preferably 0°C to 100°C.

While the reaction time varies depending on the raw material compounds, reagents, and the like, it is generally 5 min to 48 hr, preferably 10 min to 24 hr.

### (Step J-5-2)

In this step, the nitro group of compound (32) is converted to an amino group using hydrogen in the presence of a suitable metal catalyst in a solvent.

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; alcohols such as methanol, ethanol, tert-butanol and the like; nitriles such as acetonitrile and the like; amides such as formamide, N,N-dimethylformamide and the like; sulfoxides such as dimethyl sulfoxide and the like; mixed solvents containing a plurality of the organic solvents mentioned above in any ratio; mixed solvents containing the aforementioned organic solvent and water in any ratio and the like.

The metal catalysts used are not particularly limited, but preferably include, for example, palladium-activated carbon, platinum-activated carbon, nickel, osmium-activated carbon, and the like.

Examples of the hydrogen source to be used include hydrogen gas, ammonium formate, hydrazine and the like.

While the reaction temperature varies depending on the raw material compounds, reagents, and the like, it is generally -10°C to 150°C, preferably 0°C to 100°C.

While the reaction time varies depending on the raw material compounds, reagents, and the like, it is generally 5 min to 24 hr, preferably 10 min to 12 hr.

### (Step J-5-3)

In this step, the nitro group of compound (32) is converted to an amino group by using an appropriate metal hydride in a solvent.

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; ethers such as diethyl ether, tetrahydrofuran, 1,4-dioxane, 1,2-dimethoxyethane and the like; mixed solvents containing a plurality of the organic solvents mentioned above in any ratio and the like.

The metal hydride to be used is not particularly limited, and preferred examples include lithium aluminum hydride and the like.

While the reaction temperature varies depending on the raw material compounds, reagents, and the like, it is generally -10°C to 150°C, preferably 0°C to 100°C.

While the reaction time varies depending on the raw material compounds, reagents, and the like, it is generally 5 min to 24 hr, preferably 10 min to 12 hr.

### (Step J-6)

This step is to produce compound (5A2) by constructing a triazole ring from compound (33). In the following, the method using nitrites or nitrite esters in the presence of an acid in a solvent is described, but is not limited to this method.

That is, this step is the step of constructing a triazole ring by reacting compound (33) with nitrites or nitrite esters in the presence of an acid in a solvent.

The solvent to be used is not particularly limited as long as it does not inhibit the reaction and dissolves the starting materials to some extent, and preferred examples include aromatic hydrocarbons such as benzene, toluene, xylene and the like; halogenated hydrocarbons such as dichloromethane, chloroform and the like; esters such as ethyl acetate, propyl acetate and the like; ethers such as diethyl ether, tetrahydrofuran, 1,4-dioxane, 1,2-dimethoxyethane and the like; alcohols such as methanol, ethanol, tert-butanol and the like; nitriles such as acetonitrile and the like; amides such as formamide, N,N-dimethylformamide and the like; sulfoxides such as dimethyl sulfoxide and the like; mixed solvents containing a plurality of the organic solvents mentioned above in any ratio; mixed solvents containing the aforementioned organic solvent and water in any ratio and the like.

The acid to be used is not particularly limited as long as it is an acid that is used in normal reactions. Preferred examples include inorganic acids such as hydrochloric acid, sulfuric acid, tetrafluoroboric acid and the like; Lewis acids such as boron trifluoride, boron trichloride, boron tribromide, iodotrimethylsilane and the like; organic acids such as acetic acid, trifluoroacetic acid and the like, and the like.

The nitrites to be used are not particularly limited, but include, for example, alkali metal salts such as sodium nitrite, and the like.

The nitrite esters to be used are not particularly limited, but include, for example, isobutyl nitrite, tert-butyl nitrite, and the like.

While the reaction temperature varies depending on the raw material compounds, reagents, and the like, it is generally -20°C to 100°C, preferably -10°C to 60°C.

While the reaction time varies depending on the raw material compounds, reagents, and the like, it is generally 5 min to 24 hr, preferably 10 min to 6 hr.

The compound (1) or a pharmaceutically acceptable salt thereof obtained by the above-mentioned production method can be isolated and purified by a conventional separation means such as recrystallization, distillation, chromatography, and the like.

When the compound (1) or a pharmaceutically acceptable salt thereof exists as an optical isomer based on an asymmetric carbon, it can be separated into individual optical isomers by conventional optical resolution means (e.g., fractionated crystal method, resolution using a chiral column). Alternatively, the optical isomers can be synthesized using optically pure starting materials. Furthermore, optical isomers can also be synthesized by stereoselectively carrying out each reaction using chiral auxiliary groups or asymmetric catalysts.

### (Medicament (or pharmaceutical composition) of the present invention)

The medicament of the present invention is a drug for preventing and/or treating oxidative stress-related diseases, which contains compound (1) or a pharmaceutically acceptable salt thereof as an active ingredient.

The medicament of the present invention may be either a medicament consisting of only compound (1) or a pharmaceutically acceptable salt thereof, or a pharmaceutical composition containing compound (1) or a pharmaceutically acceptable salt thereof and a pharmaceutically acceptable carrier, and the like. The medicament of the present invention can be administered in a pharmaceutically effective amount to a subject (e.g., a mammal such as human, mouse, rat, hamster, guinea pig, rabbit, cat, dog, pig, cow, horse, sheep, or monkey).

Examples of the pharmaceutically acceptable carrier include excipient (e.g., starch, lactose, sugar, calcium carbonate, calcium phosphate etc.), binder (e.g., starch, gum arabic, carboxymethylcellulose, hydroxypropylcellulose, crystalline cellulose etc.), lubricant (e.g., magnesium stearate, talc etc.), disintegrant (e.g., carboxymethylcellulose, talc etc.), solvent (e.g., water for injection, physiological brine, Ringer's solution, alcohol, propylene glycol, polyethylene glycol, sesame oil, corn oil, olive oil, cotton seed oil etc.), solubilizing agent (e.g., polyethylene glycol, propylene glycol, D-mannitol, trehalose, benzyl benzoate, ethanol, tris-aminomethane, cholesterol, triethanolamine, sodium carbonate, sodium citrate, sodium salicylate, sodium acetate etc.), suspending agent (e.g., surfactants such as stearyl triethanolamine, sodium lauryl sulfate, lauryl aminopropionate, lecithin, benzalkonium chloride, benzethonium chloride, glycerol monostearate and the like; hydrophilic polymers such as poly(vinyl alcohol), polyvinylpyrrolidone, sodium carboxymethylcellulose, methylcellulose, hydroxymethylcellulose, hydroxyethylcellulose, hydroxypropylcellulose and the like; polysorbates, polyoxyethylene hydrogenated castor oil etc.), isotonic agent (e.g., sodium chloride, glycerol, D-mannitol, D-sorbitol, glucose etc.), buffering agent (e.g., buffer such as phosphate, acetate, carbonate, citrate and the like), soothing agent (e.g., benzyl alcohol etc.), antiseptic (e.g., p-hydroxybenzoate esters, chlorobutanol, benzyl alcohol, phenethyl alcohol, dehydroacetic acid, sorbic acid etc.), antioxidant (e.g., sulfite, ascorbate etc.), colorant (e.g., water-soluble food tar dyes (e.g., food colors such as Food Color Red Nos. 2 and 3, Food Color yellow Nos. 4 and 5, Food Color Blue Nos. 1 and 2 and the like), water insoluble lake pigment (e.g., aluminum salt of the aforementioned water-soluble food tar color), natural dye (e.g., β-carotene, chlorophyll, red iron oxide) etc.), sweetening agent (e.g., sodium saccharin, dipotassium glycyrrhizinate, stevia etc.), corrigent (e.g., fennel oil, cassia oil, ethyl vanillin, orange oil etc.), aromatic and the like.

The medicament (pharmaceutical composition) of the present invention can be formulated by mixing the above-mentioned components and then subjecting the mixture to a method known per se, into a preparation for oral administration such as tablets, pills, fine granules, granules, capsules, dry syrup, elixir, and the like; or a preparation for parenteral administration such as injections (e.g., subcutaneous injections, intravenous injections, intramuscular injections, intraperitoneal injections, drips, and the like), topical preparations (e.g., transdermal preparations, ointments, lotions, patches), suppositories (e.g., rectal suppositories, vaginal suppositories), pellets, nasal preparations, pulmonary preparations (inhalants), eye drops, implants, microcapsules, liposome preparations, and the like. Tablets or pills may be coated with sugar coating, or with a gastric or enteric coating agent as necessary. Preparations for parenteral administration can be sterilized, for example, by filtration through a bacteria-retaining filter, by blending with a bactericide, or by radiation irradiation. Furthermore, a sterile solid composition may be dissolved or suspended in sterile water or a solvent for injection before use, and the resulting composition may be used as a preparation for parenteral administration.

The content of the compound (1) of the present invention or a pharmaceutically acceptable salt thereof in the medicament (pharmaceutical composition) of the present invention varies depending on the form of the preparation. It is generally in the range of about 0.001 to 100% by weight, preferably about 0.01 to 50% by weight, and more preferably about 0.01 to 20% by weight, based on the total weight of the preparation.

The dosage and frequency of administration of the compound (1) of the present invention or a pharmaceutically acceptable salt thereof are appropriately determined for each individual case, taking into consideration the symptoms, age or sex of the subject, and the like. The dosage is generally 0.001 mg/kg to 100 mg/kg per dose for adults when administered orally, and generally 0.0001 mg/kg to 10 mg/kg per dose for adults when administered intravenously. The frequency of administration is generally once to six times a day, or once a day to once every seven days.

The compound (1) of the present invention or a pharmaceutically acceptable salt thereof is effective for prophylactic and/or therapeutic use for a disease selected from the group consisting of a renal disease selected from the group consisting of chronic kidney disease, acute nephritis, chronic nephritis, acute renal failure, chronic renal failure, nephrotic syndrome, IgA nephropathy, diabetic nephropathy, gouty kidney, nephrosclerosis, hydronephrosis and tubulointerstitial nephritis; a liver disease selected from the group consisting of alcoholic fatty liver, non-alcoholic steatohepatitis, hepatic fibrosis and cirrhosis; a respiratory disease selected from the group consisting of bronchitis, pneumonia, pleurisy, chronic obstructive pulmonary diseases, acute lung disorder, diffuse panbronchiolitis, interstitial pneumonia and asthma; a dermatic disease selected from the group consisting of UV and radiation skin disorder, radiation mucosal disorder, epidermolysis blister syndrome, psoriasis, atopic dermatitis and scleroderma; a cardiovascular disease selected from the group consisting of cardiac failure, myocardial infarction, arteriosclerosis and pulmonary arterial hypertension; a central nervous system disease selected from the group consisting of Alzheimer's disease, Parkinson's disease, Huntington's disease, amyotrophic lateral sclerosis, cerebral infarction, polyglutamine disease and autism; a mitochondrial disease selected from the group consisting of Friedreich's ataxia and mitochondrial myopathy; an autoimmune disease selected from the group consisting of multiple sclerosis, rheumatoid arthritis, systemic lupus erythematosus, Sjogren syndrome, type 1 diabetes, ulcerative colitis and Crohn's disease; and an ophthalmic disease selected from the group consisting of allergic conjunctival diseases, viral conjunctivitis, pterygium, cornea infectious disease, dry eye, corneal disorders, uveitis, Behcet's disease, diabetic retinopathy, retinal detachment, retinal vein occlusion, central serous chorioretinopathy, age-related macular degeneration, diabetic macular edema, macular disease, retinitis pigmentosa, glaucoma and cataract, and the like, and among them,
particularly effective for prophylactic and/or therapeutic use for an oxidative stress-related disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, age-related macular degeneration, retinitis pigmentosa and glaucoma.

The compound (1) of the present invention or a pharmaceutically acceptable salt thereof can be used in combination with other drugs (concomitant drugs) as long as the efficacy thereof is not impaired. The concomitant drug is not particularly limited and, for example, one or more known drugs that have been conventionally used for the treatment of oxidative stress-related diseases, which are exemplified above and selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, age-related macular degeneration, retinitis pigmentosa and glaucoma can be preferably used.

Other drugs (concomitant drugs) which are suitable for use in combination with the compound (1) of the present invention or a pharmaceutically acceptable salt thereof include, but are not limited to, for example, ACE inhibitor (e.g., enalapril etc.), angiotensin II receptor antagonists (e.g., olmesartan etc.), β blockers (e.g., carvedilol etc.), aldosteron antagonists (e.g., spironolactone etc.), bronchodilators (e.g., anticholinergics, theophylline etc.), steroids (e.g., predonisolone etc.), prostacyclins (e.g., veraprost etc.), endothelin receptor antagonist (e.g., bosentan etc.), phosphodiesterase-5 inhibitors (e.g., sildenafil etc.), soluble guanylate cyclase (sGC) stimulants (e.g., belisiguat etc.), L-dopa, dopamine agonists (e.g., pramipexole etc.), catechol-O-methyl transferase (COMT) inhibitors (e.g., entacapone etc.), monoamine oxidase B (MAO-B) inhibitors (e.g., selegiline etc.), interferon β, copaxone and the like.

When a concomitant drug is used, the administration period is not limited, and it may be administered to the administration subject simultaneously, or may be administered at a different time than the administration of the medicament of the present invention (i.e., may be administered with a time lag). In the administration with a time lag, the medicament of the present invention may be administered first and the concomitant drug may be administered later, or the concomitant drug may be administered first and the medicament of the present invention may be administered later. The administration method of each may be the same or different. In addition, the compound (1) of the present invention or a pharmaceutically acceptable salt thereof and the concomitant drug may be administered in combination as a single preparation (combination drug). As a pharmaceutically acceptable carrier that may be used in the production of a combination drug, those similar to the ones used in the pharmaceutical composition of the present invention described above can be mentioned.

The dosage of the concomitant drug can be appropriately selected based on the dosage generally used in clinical practice. The mixing ratio of the compound of the present invention or a pharmaceutically acceptable salt thereof to the concomitant drug can be appropriately selected depending on the subject of administration (age, body weight, general health condition, sex, degree of disease, etc. of the subject), the administration route, the type of disease, the type of concomitant drug, and the like.

The mass ratio of compound (1) or a pharmaceutically acceptable salt thereof to a concomitant drug is not particularly limited.

Also, concomitant drugs that complement and/or enhance the therapeutic effect of compound (1) or pharmaceutically acceptable salts thereof include those that have not been found to date and will be found in the future based on the mechanism described above (i.e., the Nrf2 activation mechanism by inhibiting the protein-protein interaction between Keap1 and Nrf2).

The medicament or pharmaceutical composition of the present invention may be provided in the form of a kit together with instructions on how to administer the medicament or pharmaceutical composition. The medicament contained in the kit is supplied in a container manufactured from a material that will effectively sustain the activity of the components of the medicament or pharmaceutical composition for a long period of time, will not adsorb on the inner side of the container, and will not alter the components. For example, a sealed glass ampoule may contain buffer and the like sealed in the presence of a neutral, non-reactive gas such as nitrogen gas. The kit may also be accompanied by instructions for use. The instructions for use of the kit may be printed on paper or other media, or may be stored on an electromagnetically readable medium such as a CD-ROM, DVD-ROM, and the like, and supplied to the user.

### Experimental Example 1: FP assay (in vitro)

The inhibitory activity of test compounds on the binding between Nrf2 and Keap1 was determined by fluorescence polarization. A solution of 50 mM Tris-HCl pH.8.0 (NACALAI, REF: 06938-15), and 5 mM DTT (SIGMA-ALDRICH, REF: 646563-10X.5 mL) was used as a buffer solution. 8 µL of a buffer solution containing 40 nM GST-fused human Keap1 (amino acid residues: 325-642) protein (Protein tech, REF: Ag0779) was taken in a black 384-well plate (final concentration 20 nM), and 4 µL of the test compound solution prepared to each concentration in buffer solution was added thereto. A well containing a portion of the buffer solution without Keap1 was placed as a negative control. A well with no compound was used as a positive control. 40 nM of FITC-labeled Nrf2 peptide (FITC-X-LQLDEETGEFLPIQ (X=ε-Acp), Peptide Institute Inc.) was added thereto (10 nM final concentration). The wells were incubated at room temperature for 2 hr, and then fluorescence polarization was measured with a plate reader SPECTRAMAX Paradigm (Molecular devices) at excitation wavelength of 485 nm and fluorescence wavelength of 535 nm. The fluorescence polarization of the negative control was set to 100% inhibition and that of the positive control to 0% inhibition, and the inhibitory rate upon addition of the test compound was calculated using the following formula. inhibitory rate (%)=(fluorescence polarization of negative control - fluorescence polarization when test compound is added)/(fluorescence polarization of positive control - fluorescence polarization of negative control) x 100

The results of each test compound in this study are shown in Table 1 below in the range of two concentrations (µM) between 50% inhibitory rate (A: inhibitory rate 50% concentration (IC₅₀) ≤ 0.01, B: 0.01 <inhibitory rate 50% concentration (IC₅₀) ≤ 0.03, C: 0.03 <inhibitory rate 50% concentration (IC₅₀) ≤ 0.3, D: 0.3 <inhibitory rate 50% concentration (IC₅₀) ≤ 3).

**[Table 1]**

| Ex. No. | IC₅₀ | Ex. No. | IC₅₀ | Ex. No. | IC₅₀ |
|---|---|---|---|---|---|
| 1 | B | 32 | B | 63 | C |
| 2 | C | 33 | B | 64 | C |
| 3 | B | 34 | B | 65 | C |
| 4 | B | 35 | B | 66 | C |
| 5 | B | 36 | B | 67 | B |
| 6 | B | 37 | B | 68 | B |
| 7 | C | 38 | A | 69 | B |
| 8 | C | 39 | D | 70 | A |
| 9 | C | 40 | D | 71 | B |
| 10 | B | 41 | A | 72 | C |
| 11 | A | 42 | A | 73 | B |
| 12 | D | 13 | B | 71 | B |
| 13 | B | 11 | A | 75 | B |
| 14 | A | 15 | B | 76 | B |
| 15 | B | 46 | A | 77 | A |
| 16 | C | 47 | D | 78 | A |
| 17 | C | 18 | B | 79 | B |
| 18 | C | 49 | D | 80 | B |
| 19 | C | 50 | B | 81 | B |
| 20 | B | 51 | B | 82 | B |
| 21 | B | 52 | C | 83 | A |
| 22 | A | 53 | B | 84 | A |
| 23 | C | 54 | B | 85 | A |
| 21 | B | 55 | A | 86 | A |
| 25 | B | 56 | A | 87 | A |
| 26 | B | 57 | B | 88 | |
| 27 | B | 58 | D | 89 | C |
| 28 | R | 59 | D | 90 | C |
| 29 | B | 60 | | 91 | C |
| 30 | B | 61 | | 92 | C |
| 31 | A | 62 | B | 93 | A |

### Experimental Example 2: NAD(P)H: quinone oxidoreductase-1 (NQO1) enzyme induction assay (in vitro)

The NQO1 assay was performed based on a previous report (Methods in Enzymology 2004; 382: 243-258). Hepa1c1c7 cells (mouse hepatocyte cell line, ATCC, cat. no. CRL-2026) were cultured in D-MEM medium containing 10% FBS and 1% penicillin/streptomycin (5% CO₂, 37°C), and seeded into 96-well plates (Corning REF. 353072) at 1 × 104 cells/well. A portion of the wells were prepared with medium only for background measurement without seeding cells. The next day, 400 nM of the test compound (0.004% of the final concentration in DMSO) dissolved in DMSO was added thereto, and the wells were incubated for about 48 hr. A well without adding the test compound was prepared for base activity assay in part.

Cell lysate (solution containing 0.8% digitonin, 2 mM EDTA), reaction solution (solution containing 25 mM Tris-HCl, 0.07% albumin, 0.01% Tween-20, 2 U/mL glucose-6-phosphate dehydrogenase, 5 µM flavin adenine dinucleotide, 1 µM glucose-6-phosphate, 30 µM nicotinamide adenine dinucleotide phosphate, 0.03% 3-(4,5-dimethyl-2-thiazolyl)-2,5-diphenyltetrazolium bromide (MTT), and 50 µM menadione), stop solution (solution containing 0.3 mM dicoumarol and 5 mM potassium dihydrogen phosphate, pH 7.4) were prepared. After the medium was removed, 50 µL of the cell lysate was added and incubated at 37°C for 10 minutes, followed by shaking at room temperature for 10 minutes. 200 µL of reaction solution was added thereto, and allowed to stand for 5 minutes at room temperature. 50 µL of the stop solution was added thereto, and absorbance was measured at 610 nm on a SpectraMax PLUS384 (Molecular devices) or POWERSCAN HT (DS Pharma Biomedical). The test results were analyzed in Excel, and the NQO1 activity upon addition of 400 nM of test compound was calculated as a ratio from the base without the compound. NQO1 activity ratio when test compound is added=(absorbance when test compound is added - absorbance of medium only)/(absorbance without addition of compound - absorbance of medium only)

The results of each test compound in this test are shown in the following Table 2 (A: 5 <NQO1 activity ratio, B: 3 <NQO1 activity ratio ≤ 5, C: 1.5 <NQO1 activity ratio ≤ 3).

**[Table 2]**

| Ex. No. | activity ratio | Ex. No. | activity ratio | Ex. No. | activity ratio |
|---|---|---|---|---|---|
| 1 | A | 33 | A | 64 | A |
| 2 | A | 34 | B | 65 | A |
| 3 | A | | A | 66 | A |
| 4 | A | 36 | A | | A |
| 5 | A | 37 | A | 67 68 | A |
| | 6 A | 38 | A | 69 | A |
| 7 | A | 39 | C | 70 | A |
| 8 | B | 40 | C | 71 | A |
| 9 | A | 41 | A | 72 | A |
| 10 | A | 42 | A | 73 | A |
| 11 | A | 43 | A | 74 | A |
| 13 | A | 41 | A | 75 | A |
| 14 | A | 45 | A | 76 | A |
| 15 | A | 46 | B | 77 | A |
| 16 | A | 47 | | 78 | B |
| 17 | A | 48 | A | 79 | B |
| 18 | A | 49 | B | 80 | A |
| 19 | B | 50 | A | 81 | A |
| 20 | B | 51 | A | 82 | B |
| 21 | A | 52 | B | 83 | A |
| 22 | B | 53 | B | 84 | A |
| 23 | B | 54 | B | 85 | A |
| 24 | B | 55 | A | 86 | A |
| 25 | A | 56 | A | 87 | A |
| 26 | B | 57 | A | 88 | A |
| 27 | A | 58 | C | 89 | A |
| 28 | A | 59 | C | 90 | A |
| 29 | A | 60 | A | 91 | A |
| 30 | A | 61 | A | 92 | A |
| 31 | A | 62 | A | 93 | A |
| 32 | A | 63 | B | | |

These results suggest that the compound (1) of the present invention or a pharmaceutically acceptable salt thereof has the action of activating Nrf2 by inhibiting the protein-protein interaction between Keap1 and Nrf2.

### Experimental Example 3: NQO1 activity assay in rat kidney (single administration)

The test compound solution for administration was prepared by dissolving or suspending the test compound in 0.5 w/v% methylcellulose solution.

The test compound was administered orally (3 mg/kg, 2 mL/kg) to SD rats (male, Japan SLC). The solvent group was used as a control group. The test compound group was anesthetized with isoflurane 24 and 48 hr after administration, and the control group was anesthetized with isoflurane 24 hr after administration, and the rats were euthanized by abdominal aortotomy with blood release. The right kidney was immediately removed, and the renal cortex and medullary portion of the kidney were cut out for measurement of NQO1 activity.

Homogenization buffer (10 mM Tris-HCl, 250 mM sucrose, pH 7.4) and assay buffer (25 mM Tris-HCl, 0.7 mg/mL BSA, pH 7.4) were prepared.

1 mL of homogenization buffer was added to the collected kidney samples, and the organs were homogenized uniformly in ice using a Polytron homogenizer (PHYSCOTRON, Microtec Co. Ltd.) or TissueLyser II (QIAGEN). Homogenized samples were centrifuged (MX-305, TOMY) at 9000 x g for 20 minutes at 4°C. The supernatant was then scalped with homogenization buffer to approximately 2 mL and ultracentrifuged (centrifuge; OptimaL-100XP, rotor; 50.4 Ti, Beckman Coulter) at 100,000 x g for 60 min at 4°C. The protein concentration of the supernatant was measured with the Protein Assay BCA kit (#06385-00, Nacalai Tesque), and samples were diluted to 5 µg protein/30 µL with homogenization buffer. 2,6-Dichlorophenolindophenol (DCPIP) reaction buffer (assay buffer with 200 µM NADPH and 160 µM DCPIP, containing 1/1000 volume of DMSO or 20 µM dicoumarol DMSO solution) was prepared immediately before the measurement. The DCPIP dilution series for the calibration curve was prepared as shown in Table 3 below.

**[Table 3]**

| DCPIP concentration | DMSO-containing DCPIP reaction buffer | assay buffer | homogenization buffer |
|---|---|---|---|
| 160 µM | 800 µL | 0 µL | 120 µL |
| 120 µM | 600 µL | 200 µL | 120 µL |
| 80 µM | 400 µL | 400 µL | 120 µL |
| 40 µM | 200 µL | 600 µL | 120 µL |
| 0 µM | 0 µL | 800 µL | 120 µL |

The 230 µL of each concentration of calibration solution was added to a 96-well plate (CORNING, 9017). To 30 µL of the sample solution was added 200 µL of DMSO-containing DCPIP reaction buffer or dicoumarol-containing DCPIP reaction buffer. The absorbance at 600 nm was immediately measured on a SpectraMax PLUS384 (Molecular devices) at 1 minute intervals for 10 minutes. The range (0 to N min) in which the decrease in absorbance showed linearity from the start to the end of the measurement was checked, and the amounts of DCPIP (nmol) at the start of the measurement and after N min were calculated using a calibration curve to obtain the amount of DCPIP decrease (nmol/N min) for N min. A regression line was used to create the calibration curve.

The difference between the DCPIP decrease in the wells with DMSO-containing DCPIP reaction buffer and the DCPIP decrease in the wells with dicoumarol-containing DCPIP reaction buffer was divided by the amount of protein (mg) and time (min) to obtain NQO1 activity (nmol/mg/min). In Table 4 below, the NQO1 activity of the test compound-administered groups is expressed as a percentage of the control group.

**[Table 4]**

| Example | 24 hr later | 48 hr later |
|---|---|---|
| Example 5 | 9.4 | 10.9 |
| Example 11 | 9.3 | 11.0 |
| Example 13 | 6.5 | 11.7 |

These results indicate that these compounds induce potent Nrf2 activation in renal tissues when administered orally.

### [Example]

The present invention is described in detail by the following examples, which are merely an implementation and do not limit the present invention, and may be varied to the extent not departing from the scope of the present invention.

In the following examples, "room temperature" usually indicates about 10°C to about 35°C. % indicates mol/mol% for yield, volume % for solvents used in chromatography, and weight % for others.

Nuclear magnetic resonance spectra (¹H-NMR, resonance frequency 400 MHz or 500 MHz) are listed as δ values (ppm) with tetramethylsilane as the standard or with the chemical shift value of the deuterated solvent used as the reference value.

Other abbreviations used in the text have the following meanings.
s: singlet
d: doublet
dd: doublet of doublets
dt: doublet of triplets
td: triplet of doublets
ddd: doublet of doublet of doublets
t: triplet
q: quartet
m: multiplet
br: broad
J: coupling constant
Hz: Hertz
CDCl₃: deuterochloroform
DMSO-D₆: deuterodimethyl sulfoxide
CD₃OD: deuterated methanol
tBu: tert-butyl
XPhos: 2-dicyclohexylphosphino-2',4',6'-triisopropyl-1,1'-biphenyl
¹H-NMR: proton nuclear magnetic resonance
HPLC: high performance liquid chromatography
APCI: atmospheric chemical ionization method
ESI: electrospray ionization method

The reagents, solvents, devices, and the like used in the following Examples are commercially available unless otherwise specified. Furthermore, the raw material compounds used are known compounds and commercially available, or compounds synthesized and identified according to a method known per se or a method analogous thereto, unless otherwise specified.

### (Example 1)

### (3S)-3-(1,4-Dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (Example 2)

### (3R)-3-(1,4-Dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid

### (1a) 5-Bromo-7-methyl-1-benzothiophene-2-carboxylic acid

Diisopropylamine (86.7 g) was dissolved in tetrahydrofuran (800 mL), n-butyllithium (1.6 M n-hexane solution) (500 mL) was added dropwise over 25 min at -78°C, and the mixture was stirred for 30 min. Then, a solution of 4-bromo-1-fluoro-2-methylbenzene (108 g) in tetrahydrofuran (300 mL) was added dropwise over 35 min, and the mixture was stirred for 30 min at -78°C. To the reaction mixture was added N,N-dimethylformamide (53.3 mL) at -78°C, and the mixture was stirred for 10 min and then stirred for 1 hr while heating to room temperature. To the reaction mixture was added 2 M hydrochloric acid (800 mL), and the mixture was extracted with ethyl acetate. The organic layer was washed successively with water and saturated brine, dried over anhydrous sodium sulfate, and the solvent was evaporated under reduced pressure. The obtained residue was dissolved in N,N-dimethylformamide (830 mL), potassium carbonate (86.5 g) and ethyl thioglycolate (54.7 mL) were added, and the mixture was stirred at 90°C for 1 hr. The reaction mixture was cooled to room temperature, water was added, and the mixture was extracted with ethyl acetate. The organic layer was washed successively with water and saturated brine, dried over anhydrous sodium sulfate, and the solvent was evaporated under reduced pressure. The obtained residue was dissolved in ethanol (820 mL), 5 M aqueous sodium hydroxide solution (209 mL) was added, and the mixture was stirred at room temperature for 1 hr. 6 M Hydrochloric acid (228 mL) was added to the reaction mixture at 0°C, and the mixture was stirred at 0°C for 30 min. The solid was collected by filtration and washed with water. The obtained solid was dissolved in a mixed solvent of ethyl acetate (1 L) and tetrahydrofuran (1 L), washed with saturated brine, dried over anhydrous sodium sulfate, and the solvent was evaporated under reduced pressure to give a residue. In addition, the filtrate was extracted with ethyl acetate. The organic layer was washed with saturated brine, dried over anhydrous sodium sulfate, and the solvent was evaporated under reduced pressure. The residue obtained earlier was combined, n-hexane was added, and the solid was collected by filtration to give the title compound (120.0 g, yield: 78%) as a solid.

### (1b) 5-Bromo-7-methyl-1-benzothiophene

5-Bromo-7-methyl-1-benzothiophene-2-carboxylic acid (105 g) of Example 1a was dissolved in 1-methyl-2-pyrrolidinone (390 mL), 1,8-diazabicyclo[5.4.0]-7-undecene (289 mL) was added, and the mixture was stirred at 190°C for 20.5 hr. 3 M Hydrochloric acid (720 mL) was added to the reaction mixture at 0°C, and the mixture was extracted with ethyl acetate. The organic layer was washed successively with water and saturated brine, dried over anhydrous sodium sulfate, and the solvent was evaporated under reduced pressure. The obtained residue was purified by silica gel column chromatography [elution solvent: n-hexane/ethyl acetate=100/0-95/5(V/V)] to give the title compound (49.8 g, yield: 57%) as an oil.

### (1c) (5-Bromo-1-benzothiophen-7-yl)methylacetate

5-Bromo-7-methyl-1-benzothiophene (114.2 g) of Example 1b was dissolved in carbon tetrachloride (1.0 L), and N-bromosuccinimide (98.5 g) and 2,2'-azodiisobutyronitrile (8.26 g) were added and the mixture was heated under reflux for 12 hr. The reaction mixture was cooled to room temperature and insoluble material was filtered off. The filtrate was washed with water, the organic layer was dried over anhydrous sodium sulfate, and the solvent was evaporated under reduced pressure. The obtained residue was dissolved in N,N-dimethylformamide (535 mL), potassium acetate (247 g) was added and the mixture was stirred at room temperature for 1 hr. To the reaction mixture was added water, and the mixture was extracted with ethyl acetate. The organic layer was washed successively with water and saturated brine, dried over anhydrous sodium sulfate, and the solvent was evaporated under reduced pressure. The obtained residue was purified by column chromatography [elution solvent: n-hexane/ethyl acetate=100/0-90/10(V/V)] to give the title compound (94.4 g, yield: 66%) as a solid.

### (1d) (5-Bromo-1-benzothiophen-7-yl)methanol

(5-Bromo-1-benzothiophen-7-yl)methyl acetate (50.2 g) of Example 1c was dissolved in a mixed solvent of methanol (290 mL) and tetrahydrofuran (290 mL), 2 M aqueous sodium hydroxide solution (264 mL) was added and the mixture was stirred at room temperature for 1 hr. To the reaction mixture was added water, and the mixture was extracted with ethyl acetate. The organic layer was washed with saturated brine, dried over anhydrous sodium sulfate, and the solvent was evaporated under reduced pressure. To the obtained residue was added n-hexane and the mixture was subjected to ultrasonic treatment. The solid was collected by filtration to give the title compound (39.6 g, yield: 92%) as a solid.

### (1e) [5-(4,4,5,5-Tetramethyl-1,3,2-dioxaborolan-2-yl)-1-benzothiophen-7-yl]methanol

(5-Bromo-1-benzothiophen-7-yl)methanol (2.00 g) of Example 1d was dissolved in 1,4-dioxane (25 mL), bis(pinacolato)diboron (2.51 g), [1,1'-bis(diphenylphosphino)ferrocene]dichloropalladium(II) dichloromethane adduct (672 mg) and potassium acetate (2.42 g) were added, and the mixture was stirred under a nitrogen atmosphere at 90°C for 1 hr. The reaction mixture was cooled to room temperature, water was added, and the mixture was extracted with ethyl acetate. The organic layer was washed with saturated brine, dried over anhydrous sodium sulfate, and the solvent was evaporated under reduced pressure. The obtained residue was purified by silica gel column chromatography [elution solvent: n-hexane/ethyl acetate=100/0-70/30(V/V)] to give the title compound (2.75 g, yield: quantitative) as an oil.

### (1f) Ethyl 3-(1,4-dimethyl-1H-benzotriazol-5-yl)-3-[7-(hydroxymethyl)-1-benzothiophen-5-yl]propanoate

[5-(4,4,5,5-Tetramethyl-1,3,2-dioxaborolan-2-yl)-1-benzothiophen-7-yl]methanol (500 mg) of Example 1e was dissolved in a mixed solvent of 1,4-dioxane (11.2 mL) and water (5.6 mL), triethylamine(0.48 mL) and ethyl (2E)-3-(1,4-dimethyl-1H-benzotriazol-5-yl)prop-2-enoate (WO 2015/092713) (423 mg) were added, and the mixture was stirred at 90°C for 5 min. Then, chloro(1,5-cyclooctadiene)rhodium(I) dimer(42 mg) was added, 90°C for 15 min was stirred. The reaction mixture was cooled to room temperature, water was added, and the mixture was extracted with ethyl acetate. The organic layer was washed with saturated brine, dried over anhydrous sodium sulfate, and the solvent was evaporated under reduced pressure. The obtained residue was purified by column chromatography [elution solvent: n-hexane/ethyl acetate=80/20-30/70(V/V)] to give the title compound (276 mg, yield: 39%) as a solid.

### (1g) Ethyl 3-[7-(chloromethyl)-1-benzothiophen-5-yl]-3-(1,4-dimethyl-1H-benzotriazol-5-yl)propanoate

To a solution of ethyl 3-(1,4-dimethyl-1H-benzotriazol-5-yl)-3-[7-(hydroxymethyl)-1-benzothiophen-5-yl]propanoate (380 mg) of Example 1f in dichloromethane (5 mL) was added thionyl chloride (0.135 mL), and the mixture was stirred at room temperature for 40 min. The reaction mixture was concentrated under reduced pressure, and the residue was azeotropically distilled with toluene to give the title compound (397 mg, yield: quantitative) as a highly viscous oil.

### (1h) (2R)-2-Ethyl-2,3,4,5-tetrahydropyrido[2,3-f][1,4]oxazepin-7-ol hydrochloride

tert-Butyl (2R)-2-ethyl-7-hydroxy-3,5-dihydro-2H-pyrido[2,3-f][1,4]oxazepine-4-carboxylate (WO 2020/16577) (1.57 g) was dissolved in dichloromethane (15 mL), 4 M hydrogen chloride-1,4-dioxane solution (10 mL) was added, and the mixture was stirred at room temperature for 12 hr. The solvent was evaporated under reduced pressure, n-hexane was added to the obtained residue, and the mixture was subjected to an ultrasonic treatment, and the resulting solid was collected by filtration. The mixture was vacuum dried at 60°C for 3 hr to give the title compound (1.23 g, yield: quantitative) as a solid.

### (1i) Ethyl 3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoate

To a solution of ethyl 3-[7-(chloromethyl)-1-benzothiophen-5-yl]-3-(1,4-dimethyl-1H-benzotriazol-5-yl)propanoate (2.20 g) of Example 1g in acetonitrile (200 mL) were added N,N-diisopropylethylamine (0.740 mL) and (2R)-2-ethyl-2,3,4,5-tetrahydropyrido[2,3-f][1,4]oxazepin-7-ol hydrochloride (1.00 g), and the mixture was stirred at 85°C for 48 hr and cooled to room temperature. The reaction mixture was poured into water and extracted with ethyl acetate. The organic layer was washed with saturated brine and dried over anhydrous sodium sulfate. After filtration, the solvent was evaporated under reduced pressure and the obtained residue was purified by silica gel column chromatography [elution solvent: n-hexane/ethyl acetate/methanol=50/50/0-0/95/5(V/V/V)] to give the title compound (1.40 g, yield: 55%) as an oil.

### (1j)=(1)

### (3S)-3-(1,4-Dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (2a)=(2)

### (3R)-3-(1,4-Dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

To a mixture of ethyl (3R)-3-(1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (1.40 g) of Example 1i, ethanol (20 mL), and tetrahydrofuran (20 mL) was added 1 M aqueous sodium hydroxide solution (4.8 mL) and the mixture was stirred at room temperature for 12 hr. To the reaction mixture was added 1 M hydrochloric acid (4.8 mL), and the mixture was stirred and extracted with ethyl acetate. The organic layer was washed with saturated brine and dried over anhydrous sodium sulfate. After filtration, the solvent was evaporated under reduced pressure and the obtained residue was purified by silica gel column chromatography [elution solvent: dichloromethane/methanol=100/0-98/2(V/V)] to give two compounds. (3R)-3-(1,4-Dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid (0.300 g, yield: 23%) eluted earlier was obtained as a solid.

In addition, (3S)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid (0.250 g, yield: 19%) eluted later was obtained as a solid.

### (Example 3)

### (3S)-3-(1,4-Dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}propanoic acid

### (3a) 6-Bromo-2-(bromomethyl)-3-fluoropyridine

To a suspension of 6-bromo-3-fluoro-2-methylpyridine (71.3 g) in carbon tetrachloride (1.5 L) were added N-bromosuccinimide (201 g) and 2,2'-azodiisobutyronitrile (18.6 g), and the mixture was heated under reflux for 4 days. The reaction mixture was cooled to room temperature and insoluble material was filtered off. The obtained filtrate was washed with water, the organic layer was dried over anhydrous sodium sulfate, and the solvent was evaporated under reduced pressure. To a solution of the obtained residue in tetrahydrofuran (1.5 L) were added N,N-diisopropylethylamine (65.3 mL) and diethyl phosphite (48.4 mL) at 0°C and the mixture was stirred at room temperature for 4 hr. The reaction mixture was concentrated under reduced pressure, water was added to the obtained residue, and the mixture was extracted with ethyl acetate. The organic layer was washed successively with water and saturated brine, and dried over anhydrous sodium sulfate. Under reduced pressure, the solvent was evaporated and the obtained residue was purified by silica gel column chromatography [elution solvent: n-hexane/ethyl acetate/chloroform=95/5/20-75/25/20(V/V/V)]. To the obtained residue was added n-hexane, and an ultrasonic treatment was performed. The solid was collected by filtration to give the title compound (80.8 g, yield: 80%) as a solid.

### (3b) 1-{[Bis(4-methoxybenzyl)amino]methyl}cyclopropanol

Ethyl glycinate hydrochloride (64.1 g) was dissolved in acetonitrile (920 mL), potassium carbonate (191 g), 4-methoxybenzyl chloride (125 mL) and sodium iodide (6.88 g) were added, and the mixture was stirred at 50°C for 4 hr. The insoluble material in the reaction mixture was filtered off, and washed with ethyl acetate. The obtained filtrate was concentrated under reduced pressure to give a crude product (158.8 g) as an oil. The obtained crude product (91.7 g) was dissolved in tetrahydrofuran (370 mL), under a nitrogen atmosphere, tetraisopropyl orthotitanate (19.0 mL) was added, then ethylmagnesium bromide (1.0 M tetrahydrofuran solution) (800 mL) was added over 50 min at 0°C, and the mixture was stirred at room temperature for 35 min. To the reaction mixture was added saturated aqueous ammonium chloride solution, and the reaction mixture was filtered through celite. Insoluble material was removed, and washed successively with saturated aqueous ammonium chloride solution, water and ethyl acetate. To the obtained filtrate was added saturated aqueous sodium hydrogen carbonate solution, and the mixture was extracted with ethyl acetate. The organic layer was washed successively with water and saturated brine, and dried over anhydrous sodium sulfate. Under reduced pressure, the solvent was evaporated and the obtained residue was purified by silica gel column chromatography [elution solvent: n-hexane/ethyl acetate=100/0-80/20(V/V)] to give the title compound (70.2 g, yield: 80%) as an oil.

### (3c) 1-{[(4-Methoxybenzyl)amino]methyl}cyclopropanol

To a suspension of 20% palladium hydroxide-activated carbon (382 mg) in a small amount of ethyl acetate were added a solution of 1-{[bis(4-methoxybenzyl)amino]methyl}cyclopropanol (7.64 g) of Example 3b in methanol (200 mL) and acetic acid (40 mL), and the mixture was subjected to catalytic hydrogenation at 50°C, 0.4 MPa for 35 min. To the reaction mixture was added celite, and insoluble material was filtered off and washed with ethyl acetate. The obtained filtrate was concentrated under reduced pressure, toluene was added and the mixture was azeotropically distilled. The obtained residue was purified by NH silica gel column chromatography [elution solvent: n-hexane/ethyl acetate=100/0-70/30 (V/V), chloroform/methanol=100/0-97/3(V/V)] to give the title compound (2.47 g, yield: 51%) as an oil.

### (3d) 1-({[(6-Bromo-3-fluoropyridin-2-yl)methyl](4-methoxybenzyl)amino}methyl)cyclopropanol

6-Bromo-2-(bromomethyl)-3-fluoropyridine (2.64 g) of Example 3a and 1-{[(4-methoxybenzyl)amino]methyl}cyclopropanol (2.07 g) of Example 3c were dissolved in acetonitrile (50 mL), potassium carbonate (1.80 g) was added, and the mixture was stirred at 50°C for 1 hr. The insoluble material in the reaction mixture was filtered off and the filtrate was concentrated under reduced pressure. The obtained residue was purified by silica gel column chromatography [elution solvent: n-hexane/ethyl acetate=100/0-70/30 (V/V)] to give the title compound (2.59 g, yield: 66%) as an oil.

### (3e) 7'-Bromo-4'-(4-methoxybenzyl)-4',5'-dihydro-3'H-spiro[cyclopropane-1,2'-pyrido[2,3-f] [1,4]oxazepine]

1-({[(6-Bromo-3-fluoropyridin-2-yl)methyl](4-methoxybenzyl)amino}methyl)cyclopropanol (2.31 g) of Example 3d was dissolved in a mixed solution of tetrahydrofuran (42 mL) and N,N-dimethylformamide (42 mL), and the mixture was stirred at 60°C for 80 min in total while adding 60% sodium hydride (257 mg) at 20 min later, 50 min later, and 60 min later. The reaction mixture was cooled to room temperature, water was added, and the mixture was extracted with ethyl acetate. The organic layer was washed successively with water and saturated brine, dried over anhydrous sodium sulfate, and the solvent was evaporated under reduced pressure. The obtained residue was purified by silica gel column chromatography [elution solvent: n-hexane/ethyl acetate=100/0-65/35(V/V)] give the title compound (1.88 g, yield: 80%) as an oil.

### (3f) 4'-(4-Methoxybenzyl)-4',5'-dihydro-3'H-spiro[cyclopropane-1,2'-pyrido[2,3-f] [1,4]oxazepin]-7'-ol

7'-Bromo-4'-(4-methoxybenzyl)-4',5'-dihydro-3'H-spiro[cyclopropane-1,2'-pyrido[2,3-f][1,4]oxazepine] (750 mg) of Example 3e was dissolved in 1,4-dioxane (10 mL), bis(dibenzylideneacetone)palladium(0) (57 mg), 5-(di-tert-butylphosphino)-1',3',5'-triphenyl-1'H-1,4'-bipyrazole (101 mg) and cesium hydroxide monohydrate (1.01 g) were added, and the mixture was stirred under a nitrogen atmosphere at 100°C for 3.5 hr. The reaction mixture was cooled to room temperature, 1 M hydrochloric acid and saturated aqueous sodium hydrogen carbonate solution were added, and the mixture was extracted with ethyl acetate. The organic layer was dried over anhydrous sodium sulfate and the solvent was evaporated under reduced pressure. The obtained residue was purified by silica gel column chromatography [elution solvent:
chloroform/methanol=100/0-92/8(V/V)] to give the title compound (475 mg, yield: 75%) as a solid.

### (3g) 4',5'-Dihydro-3'H-spiro[cyclopropane-1,2'-pyrido[2,3-f][1,4]oxazepin]-7'-ol hydrochloride

4'-(4-Methoxybenzyl)-4',5'-dihydro-3'H-spiro[cyclopropane-1,2'-pyrido[2,3-f][1,4]oxazepin]-7'-ol (443 mg) of Example 3f was dissolved in trifluoroacetic acid (14 mL) and microwave irradiation was performed at 180°C for 10 min. The reaction mixture was cooled to room temperature, and the residue was azeotropically distilled with toluene. The obtained residue was dissolved in ethyl acetate (10 mL), 1 M hydrogen chloride-ethyl acetate (4.0 mL) was added and the mixture was stirred at room temperature for 5 min. To the reaction mixture was added tert-butyl methyl ether, and the solid was collected by filtration to give the title compound (354 mg, yield: quantitative) as a solid.

### (3h) ethyl 3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoate

Ethyl 3-(1,4-dimethyl-1H-benzotriazol-5-yl)-3-[7-(hydroxymethyl)-1-benzothiophen-5-yl]propanoate (807 mg) of Example 1f was dissolved in dichloromethane (7.6 mL), dimethyl sulfoxide (0.70 mL), N,N-diisopropylethylamine (1.0 mL) and sulfur trioxide-pyridine complex (1.05 g) were added and the mixture was stirred at room temperature for 20 min. To the reaction mixture was added water, and the mixture was extracted with ethyl acetate. The organic layer was washed with saturated brine and dried over anhydrous sodium sulfate. The solvent was evaporated under reduced pressure and the obtained residue was dissolved in dichloromethane (7.6 mL). 4',5'-Dihydro-3'H-spiro[cyclopropane-1,2'-pyrido[2,3-f][1,4]oxazepin]-7'-ol hydrochloride (586 mg) of Example 3g, N,N-diisopropylethylamine (0.41 mL) and acetic acid (0.023 mL) were added and the mixture was stirred at room temperature for 30 min. Sodium triacetoxyborohydride (835 mg) was added thereto, and the mixture was stirred at room temperature for 14.5 hr. Successively, sodium triacetoxyborohydride (209 mg) was added thereto, and the mixture was stirred at room temperature for 50 min. Furthermore, sodium triacetoxyborohydride (209 mg) was added thereto, and the mixture was stirred at room temperature for 15 min. To the reaction mixture was added anhydrous magnesium sulfate (284 mg), and the mixture was stirred at 40°C for 20 min. Sodium triacetoxyborohydride (209 mg) was added and the mixture was stirred at 40°C for 30 min. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=40/60-0/100(V/V), chloroform/methanol=100/0-95/5(V/V)] to give the title compound (706 mg, yield: 57%) as a solid.

### (3i) Ethyl (3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoate

Ethyl 3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoate (700 mg) of Example 3h was subjected to chiral HPLC [column: CHIRALPAK IG (20 mm I.D. × 250 mm), mobile phase: n-hexane:ethanol=50:50] to give 340 mg of ethyl (3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoate as the first peak (yield: 49%).
retention time: 9.773 min [column: CHIRALPAK IG (4.6 mm I.D. × 250 mm), mobile phase: n-hexane:ethanol=50:50, flow rate: 1.0 mL/min, temperature: 40°C, wavelength: 254 nm]

### (3j)=(3)

### (3S)-3-(1,4-Dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}propanoic acid

To a mixture of ethyl (3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoate (340 mg) of Example 3i, methanol (4 mL) and tetrahydrofuran (4 mL) was added 1 M aqueous sodium hydroxide solution (1.7 mL), and the mixture was stirred at 60°C for 30 min. The reaction mixture was cooled to room temperature, and Dowex 50w-X8 was added to adjust the reaction mixture to pH 6-7. Under reduced pressure, the solvent was evaporated and the obtained residue was purified by silica gel column chromatography [elution solvent:
chloroform/methanol=50/1-9/1(V/V)]. Ethyl acetate and hexane were added for solidification to give the title compound (242 mg, yield: 75%) as a solid.

### (Example 4)

### (3R)-3-(1,4-Dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}propanoic acid

### (4a) Ethyl (3R)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoate

Ethyl 3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoate (700 mg) of Example 3h was subjected to chiral HPLC [column: CHIRALPAK IG (20 mm I.D. × 250 mm), mobile phase: n-hexane:ethanol=50:50] to give 341 mg of ethyl (3R)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoate as the second peak (yield: 49%).
retention time: 12.270 min [column: CHIRALPAK IG (4.6 mm I.D. × 250 mm), mobile phase: n-hexane:ethanol=50:50, flow rate: 1.0 mL/min, temperature: 40°C, wavelength: 254 nm]

### (4b)=(4)

### (3R)-3-(1,4-Dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}propanoic acid

To a mixture of ethyl (3R)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoate (340 mg) of Example 4a, methanol (4 mL) and tetrahydrofuran (4 mL) was added 1 M aqueous sodium hydroxide solution (1.7 mL), and the mixture was stirred at 60°C for 30 min. The reaction mixture was cooled to room temperature, and Dowex 50w-X8 was added to adjust the reaction mixture to pH 6-7. The solvent was evaporated under reduced pressure, and the obtained residue was purified by silica gel column chromatography [elution solvent:
chloroform/methanol=50/1-9/1(V/V)]. Ethyl acetate and hexane were added for solidification to give the title compound (289 mg, yield: 89%) as a solid.

### (Example 5)

### (3R)-3-(7-{[(2R)-2-Cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

### (Example 6)

### (3S)-3-(7-{[(2R)-2-Cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

### (5a) 2-{[(6-Bromo-3-fluoropyridin-2-yl)methyl](4-methoxybenzyl)amino}-1-cyclopropylethanol

To a solution of 2-amino-1-cyclopropylethanol (WO 2018/196662) (2.43 g) in dichloromethane (48 mL) were added 4-methoxybenzaldehyde (1.46 mL), anhydrous magnesium sulfate (2.89 g) and acetic acid (one drop), and the mixture was stirred at room temperature for 30 min. Sodium triacetoxyborohydride (5.09 g) was added thereto, and the mixture was stirred at room temperature for 1 hr. To the reaction mixture was added saturated aqueous sodium hydrogen carbonate solution, and the mixture was extracted with chloroform. The organic layer was dried over anhydrous sodium sulfate. The solvent was evaporated under reduced pressure. To a solution of the obtained residue and 6-bromo-2-(bromomethyl)-3-fluoropyridine (5.09 g) of Example 3a in dimethylformamide (60 mL) was added potassium carbonate (3.32 g) and the mixture was stirred at room temperature for 1.5 hr. To the reaction mixture was added water, and the mixture was extracted with ethyl acetate. The organic layer was washed successively with water and saturated brine, and dried over anhydrous sodium sulfate. Under reduced pressure, the solvent was evaporated and the obtained residue was purified by silica gel column chromatography [elution solvent: n-hexane/ethyl acetate=90/10-60/40(V/V)] to give the title compound (4.65 g, yield: 95%) as an oil.

### (5b) 7-Bromo-2-cyclopropyl-4-(4-methoxybenzyl)-2,3,4,5-tetrahydropyrido[2,3-f][1,4]oxazepine

2-{[(6-Bromo-3-fluoropyridin-2-yl)methyl](4-methoxybenzyl)amino}-1-cyclopropylethanol (5.50 g) of Example 5a was divided equally into two lots, to each solution thereof in N,N-dimethylformamide (96 mL) was added 60% sodium hydride (808 mg) at room temperature and the mixture was stirred at room temperature for 50 min. To each reaction mixture were added a saturated aqueous ammonium chloride solution and water, and the mixture was extracted with ethyl acetate. The organic layer was washed successively with water and saturated brine, and dried over anhydrous sodium sulfate. The solvent was evaporated under reduced pressure and the obtained residues were combined and purified by silica gel column chromatography [elution solvent: n-hexane/ethyl acetate=100/0-85/15(V/V)]to give the title compound (3.57 g, yield: 68%) as an oil.

### (5c) 2-Cyclopropyl-4-(4-methoxybenzyl)-2,3,4,5-tetrahydropyrido[2,3-f] [1,4]oxazepin-7-ol

To a solution of 7-bromo-2-cyclopropyl-4-(4-methoxybenzyl)-2,3,4,5-tetrahydropyrido[2,3-f] [1,4]oxazepine (3.57 g) of Example 5b in 1,4-dioxane (46 mL) were added bis(dibenzylideneacetone)palladium(0) (264 mg), 5-(di-tert-butylphosphino)-1',3',5'-triphenyl-1'H-1,4'-bipyrazole (465 mg) and cesium hydroxide monohydrate (4.62 g), and the mixture was stirred at 100°C for 3 hr. The reaction mixture was cooled to room temperature, water and 2 M hydrochloric acid were added, and the mixture was extracted with ethyl acetate. The organic layer was dried over anhydrous sodium sulfate. The solvent was evaporated under reduced pressure and the obtained residue was purified by silica gel column chromatography [elution solvent: chloroform/methanol=100/0-97/3(V/V)] to give the title compound (2.36 g, yield: 70%) as a solid.

### (5d) (2R)-2-Cyclopropyl-4-(4-methoxybenzyl)-2,3,4,5-tetrahydropyrido[2,3-f][1,4]oxazepin-7-ol

2-Cyclopropyl-4-(4-methoxybenzyl)-2,3,4,5-tetrahydropyrido[2,3-f][1,4]oxazepin-7-ol (930 mg) of Example 5c was subjected to chiral HPLC [column: CHIRALART Cellulose-SZ (20 mm I.D. × 250 mm), mobile phase: n-hexane:
ethanol:chloroform=60:20:20] to give 388 mg of (2R)-2-cyclopropyl-4-(4-methoxybenzyl)-2,3,4,5-tetrahydropyrido[2,3-f][1,4]oxazepin-7-ol as the second peak (yield: 42%). retention time: 8.6367 min [column: CHIRALART Cellulose-SZ (4.6 mm I.D. × 250 mm), mobile phase: n-hexane:ethanol=50:50, flow rate: 1.0 mL/min, temperature: 40°C, wavelength: 280 nm]

### (5e) (2R)-2-Cyclopropyl-2,3,4,5-tetrahydropyrido[2,3-f][1,4]oxazepin-7-ol hydrochloride

To a solution of (2R)-2-cyclopropyl-4-(4-methoxybenzyl)-2,3,4,5-tetrahydropyrido[2,3-f][1,4]oxazepin-7-ol (480 mg) of Example 5d in trifluoroacetic acid (8 mL) was added anisole (1.6 mL), and the mixture was stirred at 85°C for 4 hr. The reaction mixture was cooled to room temperature. The solvent was evaporated under reduced pressure, and the mixture was azeotropically distilled with toluene (20 mL). Ethyl acetate (10 mL) and 4 M hydrogen chloride-1,4-dioxane solution (4 mL) were added thereto, and the mixture was stirred for 10 min. The solvent was evaporated under reduced pressure, ethyl acetate was added to the obtained residue, and the mixture was subjected to an ultrasonic treatment. The solid was collected by filtration and vacuum dried to give the title compound (440 mg, yield: quantitative, purity: 85%) as a solid.

### (5f) Ethyl 3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoate

To a solution of ethyl 3-(1,4-dimethyl-1H-benzotriazol-5-yl)-3-[7-(hydroxymethyl)-1-benzothiophen-5-yl]propanoate (530 mg) of Example 1f in dichloromethane (6 mL) was added thionyl chloride (0.190 mL), and the mixture was stirred at room temperature for 1 hr. The solvent was evaporated under reduced pressure, and the residue was azeotropically distilled with toluene. The obtained residue was dissolved in acetonitrile (5 mL), and added dropwise to a solution of (2R)-2-cyclopropyl-2,3,4,5-tetrahydropyrido[2,3-f][1,4]oxazepin-7-ol hydrochloride (300 mg) of Example 5e and N,N-diisopropylethylamine (1.5 mL) in acetonitrile (15 mL), and the mixture was stirred at 90°C for 10 hr and cooled to room temperature. The solvent was evaporated under reduced pressure, and the residue was extracted with ethyl acetate and saturated aqueous sodium hydrogen carbonate solution. The organic layer was washed with saturated brine and dried over anhydrous sodium sulfate. Under reduced pressure, the solvent was evaporated and the obtained residue was purified by silica gel column chromatography [elution solvent: chloroform/methanol=90/1-50/1(V/V)] to give the title compound (417 mg, yield: 65%) as a solid.

### (5g)=(5)

### (3R)-3-(7-{[(2R)-2-Cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

### (6a)=(6)

### (3S)-3-(7-{[(2R)-2-Cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

To a mixture of ethyl 3-(7-{[(2R)-2-cyclopropyl-7-hydroxy-2,3-dihydropyrido[2,3-f][1,4]oxazepin-4(5H)-yl]methyl}-1-benzothiophen-5-yl)-3-(1,4-dimethyl-1H-benzotriazol-5-yl)propanoate (400 mg) of Example 5f, methanol (4 mL) and tetrahydrofuran (4 mL) was added 1 M aqueous sodium hydroxide solution (1.7 mL), and the mixture was stirred at 60°C for 30 min. The reaction mixture was cooled to room temperature, and Dowex 50w-X8 was added to adjust the reaction mixture to pH 5-6. The solvent was evaporated under reduced pressure and the obtained residue was purified by silica gel column chromatography [elution solvent: chloroform/methanol=50/1-9/1(V/V)]. Ethyl acetate and n-hexane were added to the compound eluted earlier and the mixture was subjected to an ultrasonic treatment. The solid was collected by filtration and vacuum dried to give (3R)-3-(7-{[(2R)-2-cyclopropyl-7-hydroxy-2,3-dihydropyrido[2,3-f][1,4]oxazepin-4(5H)-yl]methyl}-1-benzothiophen-5-yl)-3-(1,4-dimethyl-1H-benzotriazol-5-yl)propanoic acid (128 mg, yield: 34%) as a solid.

In the same manner, moreover, the compound eluted later, (3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid (128 mg, yield: 34%), was obtained as a solid.

### (Example 7)

### (3S)-3-(7-{[(2S)-2-Cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

### (Example 8)

### (3R)-3-(7-{[(2S)-2-Cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

### (7a) (2S)-2-Cyclopropyl-4-(4-methoxybenzyl)-2,3,4,5-tetrahydropyrido[2,3-f][1,4]oxazepin-7-ol

2-Cyclopropyl-4-(4-methoxybenzyl)-2,3,4,5-tetrahydropyrido[2,3-f][1,4]oxazepin-7-ol (930 mg) of Example 5c was subjected to chiral HPLC [column: CHIRALART Cellulose-SZ (20 mm I.D. × 250 mm), mobile phase: n-hexane:ethanol:chloroform=60:20:20] to give 380 mg of (2S)-2-cyclopropyl-4-(4-methoxybenzyl)-2,3,4,5-tetrahydropyrido[2,3-f][1,4]oxazepin-7-ol as the first peak (yield: 41%).
retention time: 7.1100 min [column: CHIRALART Cellulose-SZ (4.6 mm I.D. × 250 mm), mobile phase: n-hexane: ethanol=50:50, flow rate: 1.0 mL/min, temperature: 40°C, wavelength: 280 nm]

### (7b) (2S)-2-Cyclopropyl-2,3,4,5-tetrahydropyrido[2,3-f][1,4]oxazepin-7-ol hydrochloride

To a solution of (2S)-2-cyclopropyl-4-(4-methoxybenzyl)-2,3,4,5-tetrahydropyrido[2,3-f][1,4]oxazepin-7-ol (480 mg) of Example 7a in trifluoroacetic acid (8 mL) was added anisole (1.6 mL), and the mixture was stirred at 85°C for 4 hr and cooled to room temperature. The solvent was evaporated under reduced pressure, and the residue was azeotropically distilled with toluene (20 mL). Ethyl acetate (10 mL) and 4 M hydrogen chloride-1,4-dioxane solution (4 mL) were added, and the mixture was stirred for 10 min. The solvent was evaporated under reduced pressure, ethyl acetate was added to the obtained residue, and the mixture was subjected to an ultrasonic treatment. The solid was collected by filtration and vacuum dried to give the title compound (393 mg, yield: 94%, purity: 85%) as a solid.

### (7c) Ethyl 3-(7-{[(2S)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoate

To a solution of ethyl 3-(1,4-dimethyl-1H-benzotriazol-5-yl)-3-[7-(hydroxymethyl)-1-benzothiophen-5-yl]propanoate (411 mg) of Example 1f in dichloromethane (6 mL) was added thionyl chloride (0.150 mL) and the mixture was stirred at room temperature for 1 hr. The solvent was evaporated under reduced pressure, and the obtained residue was azeotropically distilled with toluene. The obtained residue was dissolved in acetonitrile (5 mL), and was added dropwise to a separately prepared solution of (2S)-2-cyclopropyl-2,3,4,5-tetrahydropyrido[2,3-f][1,4]oxazepin-7-ol hydrochloride (300 mg) of Example 7b and N,N-diisopropylethylamine (1.3 mL) in acetonitrile (15 mL), and the mixture was stirred at 90°C for 10 hr and cooled to room temperature. The solvent was evaporated under reduced pressure, and the residue was extracted with ethyl acetate and saturated aqueous sodium hydrogen carbonate solution. The organic layer was washed with saturated brine and dried over anhydrous sodium sulfate. The residue obtained by concentration under reduced pressure was purified by silica gel column chromatography [elution solvent: chloroform/methanol=90/1-50/1(V/V)] to give the title compound (383 mg, yield: 70%) as an amorphous solid.

### (7d)=(7)

### (3S)-3-(7-{[(2S)-2-Cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

### (8a)=(8)

### (3R)-3-(7-{[(2S)-2-Cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

To a mixture of ethyl 3-(7-{[(2S)-2-cyclopropyl-7-hydroxy-2,3-dihydropyrido[2,3-f][1,4]oxazepin-4(5H)-yl]methyl}-1-benzothiophen-5-yl)-3-(1,4-dimethyl-1H-benzotriazol-5-yl)propanoate (370 mg) of Example 7c, methanol (4 mL) and tetrahydrofuran (4 mL) was added 1 M aqueous sodium hydroxide solution (1.5 mL), and the mixture was stirred at 60°C for 30 min and cooled to room temperature. Dowex 50w-X8 was added to adjust the reaction mixture to pH 5-6, and the mixture was concentrated under reduced pressure. The obtained residue was purified by silica gel column chromatography [elution solvent: chloroform/methanol=50/1-9/1(V/V)]. Ethyl acetate and n-hexane were added to the compound eluted earlier and the mixture was subjected to an ultrasonic treatment. The solid was collected by filtration and vacuum dried to give (3S)-3-(7-{[(2S)-2-cyclopropyl-7-hydroxy-2,3-dihydropyrido[2,3-f][1,4]oxazepin-4(5H)-yl]methyl}-1-benzothiophen-5-yl)-3-(1,4-dimethyl-1H-benzotriazol-5-yl)propanoic acid (122 mg, yield: 39%) as a solid.

In the same manner, moreover, the compound eluted later, (3R)-3-(7-{[(2S)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid (136 mg, yield: 39%), was obtained as a solid.

### (Example 9)

### (3S)-3-(7-{[(2R)-2-Cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid

### (9a) Ethyl 3-(3,7-dimethyl-3H-[1,2,3]triazolo[4,5-b]pyridin-6-yl)-3-[7-(hydroxymethyl)-1-benzothiophen-5-yl]propanoate

Using ethyl (2E)-3-(3,7-dimethyl-3H-[1,2,3]triazolo[4,5-b]pyridin-6-yl)prop-2-enoate (WO 2015/092713) (2.5 g), and [5-(4,4,5,5-tetramethyl-1,3,2-dioxaborolan-2-yl)-1-benzothiophen-7-yl]methanol (4.4 g) of Example 1e, and by a method similar to that of Example 1f, the title compound (2.4 g, yield: 58%) was obtained as a solid.

### (9b) Ethyl 3-(3,7-dimethyl-3H-[1,2,3]triazolo[4,5-b]pyridin-6-yl)-3-(7-formyl-1-benzothiophen-5-yl)propanoate

To a solution of ethyl 3-(3,7-dimethyl-3H-[1,2,3]triazolo[4,5-b]pyridin-6-yl)-3-[7-(hydroxymethyl)-1-benzothiophen-5-yl]propanoate (700 mg) of Example 9a in chloroform (40 mL) was added manganese(IV) oxide (2.2 g), and the mixture was stirred at 60°C for 5 hr. Thereafter, manganese(IV) oxide (740 mg) was further added, and the mixture was stirred at 90°C for 2 hr and cooled to room temperature. Insoluble material was filtered off, the filtrate was concentrated under reduced pressure and the obtained residue was purified by silica gel column chromatography [elution solvent: n-hexane/ethyl acetate=50/50(V/V)] to give the title compound (642 mg, yield: 92%) as a solid.

### (9c) Ethyl (3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoate

To a solution of (2R)-2-cyclopropyl-2,3,4,5-tetrahydropyrido[2,3-f][1,4]oxazepin-7-ol hydrochloride (130 mg) of Example 5e in dichloromethane (3 mL) was added N,N-diisopropylethylamine (0.293 mL) and the mixture was stirred at room temperature for 10 min. The solvent was evaporated under reduced pressure. A solution of ethyl 3-(3,7-dimethyl-3H-[1,2,3]triazolo[4,5-b]pyridin-6-yl)-3-(7-formyl-1-benzothiophen-5-yl)propanoate (173 mg) of Example 9b in dichloromethane (10 mL) and sodium triacetoxyborohydride (339 mg) were added thereto, and the mixture was stirred at room temperature for 2.5 hr. To the reaction mixture was added sodium triacetoxyborohydride (338 mg) and the mixture was stirred at room temperature for 30 min. To the reaction mixture was added acetic acid (0.040 mL) and the mixture was stirred at room temperature for 3 hr. To the reaction mixture was added acetic acid (0.040 mL) and the mixture was stirred at room temperature for 30 min. The reaction mixture was washed successively with saturated aqueous sodium hydrogen carbonate solution and saturated brine, and dried over anhydrous sodium sulfate. Under reduced pressure, the solvent was evaporated and the obtained residue was purified by silica gel column chromatography [elution solvent: ethyl acetate/methanol=100/0-95/5(V/V)] and subjected to chiral HPLC [column: CHIRALPAK OD-H (20 mm I.D. × 250 mm), mobile phase: n-hexane:ethanol=50:50] to give 56.0 mg of ethyl (3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoate as the first peak (yield: 22%). retention time: 6.183 min [column: CHIRALPAK OD-H (4.6 mm I.D. × 250 mm), mobile phase: n-hexane:ethanol=50:50, flow rate: 1.0 mL/min, temperature: 40°C, wavelength: 210 nm]

### (9d)=(9)

### (3S)-3-(7-{[(2R)-2-Cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid

To a mixture of ethyl (3S)-3-(7-{[(2R)-2-cyclopropyl-7-hydroxy-2,3-dihydropyrido[2,3-f][1,4]oxazepin-4(5H)-yl]methyl}-1-benzothiophen-5-yl)-3-(3,7-dimethyl-3H-[1,2,3]triazolo[4,5-b]pyridin-6-yl)propanoate (56.0 mg) of Example 9c, ethanol (5 mL) and water (1 mL) was added lithium hydroxide monohydrate (12.6 mg) and the mixture was stirred at room temperature for 18 hr. To the reaction solution was added 1 M hydrochloric acid (0.3 mL) and the mixture was stirred and concentrated under reduced pressure. The obtained residue was purified by silica gel column chromatography [elution solvent: ethyl acetate/methanol=100/0-90/10(V/V)] and further slurry purified from hexane/ethyl acetate to give the title compound (33.6 mg) as a solid.

### (Example 10)

### (3R)-3-(7-{[(2R)-2-Cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid

### (10a) Ethyl (3R)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoate

Ethyl (3R)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoate (69.0 mg) of Example 9c was obtained as the second peak of chiral HPLC (yield: 27%).
retention time: 8.277 min [column: CHIRALPAK OD-H (4.6 mm I.D. × 250 mm), mobile phase: n-hexane:ethanol=50:50, flow rate: 1.0 mL/min, temperature: 40°C, wavelength: 210 nm]

### (10b)=(10)

### (3R)-3-(7-{[(2R)-2-Cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid

Using ethyl (3R)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoate (69.0 mg) of Example 10a, and by a method similar to that of Example 9d, the title compound (36.3 mg) was obtained as a solid.

### (Example 11)

### (3S)-3-(1,4-Dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoic acid

### (11a) 5-Bromo-7-{[(4-methoxybenzyl)oxy]methyl}-1-benzothiophene

To a solution of (5-bromo-1-benzothiophen-7-yl)methanol (5.00 g) of Example 1d in N,N-dimethylformamide (42 mL) was added sodium hydride (55%, 987 mg) at 0°C and the mixture was stirred at room temperature for 30 min. To the reaction mixture was added 4-methoxybenzyl chloride (3.36 mL) and the mixture was stirred at room temperature for 3 hr. To the reaction mixture was added saturated aqueous ammonium chloride solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=100/0-90/10(V/V)] to give the title compound (7.11 g, yield: 95%) as an oil.

### (11b) (1,4-Dimethyl-1H-benzotriazol-5-yl) (7-{ [(4-methoxybenzyl)oxy]methyl}-1-benzothiophen-5-yl)methanol

To a solution of 5-bromo-7-{[(4-methoxybenzyl)oxy]methyl}-1-benzothiophene(3.24 g) of Example 11a in tetrahydrofuran (40 mL) was added n-butyllithium (1.6 M n-hexane solution) (5.72 mL) at -78°C, and the mixture was stirred for 10 min. Then, a solution of 1,4-dimethyl-1H-benzotriazole-5-carbaldehyde (WO 2016/202253) (1.42 g) in tetrahydrofuran (40 mL) was added thereto, and the mixture was stirred at -78°C for 15 min and at 0°C for 2 hr. To the reaction mixture was added saturated aqueous ammonium chloride solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=80/0-60/40-55/45(V/V)] to give the title compound (1.56 g, yield: 42%) as an oil.

### (11c) Methyl 3-(1,4-dimethyl-1H-benzotriazol-5-yl)-3-(7-{[(4-methoxybenzyl)oxy]methyl}-1-benzothiophen-5-yl)-2,2-dimethylpropanoate

To a solution of (1,4-dimethyl-1H-benzotriazol-5-yl)(7-{[(4-methoxybenzyl)oxy]methyl}-1-benzothiophen-5-yl)methanol (3.20 g) of Example 11b and trichloroacetonitrile (0.844 mL) in acetonitrile (100 mL) was added 1,8-diazabicyclo[5.4.0]-7-undecene (0.052 mL) and the mixture was stirred at room temperature for 2 hr. Then, methyltrimethylsilyl dimethylketene acetal (3.53 mL) and bis(trifluoromethanesulfonyl)imide (196 mg) were added and the mixture was stirred at room temperature for 5 hr and at 50°C for 30 min. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=80/20-50/50(V/V)] to give the title compound (2.97 g, yield: 79%) as an oil.

### (11d) Methyl 3-(1,4-dimethyl-1H-benzotriazol-5-yl)-3-[7-(hydroxymethyl)-1-benzothiophen-5-yl]-2,2-dimethylpropanoate

To a solution of methyl 3-(1,4-dimethyl-1H-benzotriazol-5-yl)-3-(7-{[(4-methoxybenzyl)oxy]methyl}-1-benzothiophen-5-yl)-2,2-dimethylpropanoate (2.97 g) of Example 11c in dichloromethane (55 mL) and water (5.5 mL) was added 2,3-dichloro-5,6-dicyano-1,4-benzoquinone (2.73 g) at 0°C, and the mixture was stirred at 0°C for 2 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-30/70(V/V)] to give the title compound (1.60 g, yield: 69%) as an oil.

### (11e) Methyl (3S)-3-(1,4-dimethyl-1H-benzotriazol-5-yl)-3-[7-(hydroxymethyl)-1-benzothiophen-5-yl]-2,2-dimethylpropanoate

Methyl 3-(1,4-dimethyl-1H-benzotriazol-5-yl)-3-[7-(hydroxymethyl)-1-benzothiophen-5-yl]-2,2-dimethylpropanoate (550 mg) of Example 11d was subjected to chiral HPLC [column: CHIRALPAK IH (20 mm I.D. × 250 mm), mobile phase: n-hexane/ethanol=85/15(V/V)] to give 237 mg of methyl (3S)-3-(1,4-dimethyl-1H-benzotriazol-5-yl)-3-[7-(hydroxymethyl)-1-benzothiophen-5-yl]-2,2-dimethylpropanoate as the first peak (yield: 43%).
retention time: 13.210 min [column: CHIRALPAK IH (4.6 mm I.D. × 250 mm), mobile phase: n-hexane/ethanol=85/15(V/V), flow rate: 1.0 mL/min, temperature: 40°C, wavelength: 210 nm]

### (11f) Methyl (3S)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoate

To a solution of methyl (3S)-3-(1,4-dimethyl-1H-benzotriazol-5-yl)-3-[7-(hydroxymethyl)-1-benzothiophen-5-yl]-2,2-dimethylpropanoate (39.0 mg) of Example 11e in dichloromethane (2.0 mL) was added thionyl chloride (0.0141 mL) and the mixture was stirred at room temperature for 2 hr. The solvent was evaporated under reduced pressure, and the residue was azeotropically distilled with toluene. The residue was dissolved in acetonitrile (1.0 mL), and added to another solution of N,N-diisopropylethylamine (0.160 mL) and (2R)-2-ethyl-2,3,4,5-tetrahydropyrido[2,3-f][1,4]oxazepin-7-ol hydrochloride (25.5 mg) of Example 1h in acetonitrile (1.0 mL), and the mixture was stirred at 80°C for 12 hr and cooled to room temperature. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V), ethyl acetate/methanol=100/0-95/5(V/V)] to give the title compound (55.0 mg, yield: 100%) as an oil.

### (11g)=(11)

### (3S)-3-(1,4-Dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoic acid

To a solution of methyl (3S)-3-(1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)-2,2-dimethylpropanoate (55.0 mg) of Example 11f in dimethyl sulfoxide (1.0 mL) was added 1 M aqueous potassium hydroxide solution (0.459 mL), and the mixture was stirred at 70°C for 12 hr. The reaction mixture was neutralized with 1 M hydrochloric acid, saturated aqueous ammonium chloride solution was added, and the mixture was extracted with ethyl acetate. The organic layer was washed with saturated brine and dried over anhydrous sodium sulfate. The solvent was evaporated under reduced pressure, and the residue was purified by silica gel column chromatography [elution solvent: n-hexane/ethyl acetate=30/70-0/100(V/V), ethyl acetate/methanol=100/0-95/5(V/V)]. Ethyl acetate and n-hexane were added to the obtained solid, and an ultrasonic treatment was performed. The solid was collected by filtration and vacuum dried to give the title compound (53.0 mg, yield: 98.7%) as a solid.

### (Example 12)

### (3R)-3-(1,4-Dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoic acid

### (12a) Methyl (3R)-3-(1,4-dimethyl-1H-benzotriazol-5-yl)-3-[7-(hydroxymethyl)-1-benzothiophen-5-yl]-2,2-dimethylpropanoate

Methyl 3-(1,4-dimethyl-1H-benzotriazol-5-yl)-3-[7-(hydroxymethyl)-1-benzothiophen-5-yl]-2,2-dimethylpropanoate (148 mg) of Example 11d was subjected to chiral HPLC [column: CHIRALPAK IH (20 mm I.D. × 250 mm), mobile phase: n-hexane/ethanol=85/15(V/V)] to give 75 mg of methyl (3R)-3-(1,4-dimethyl-1H-benzotriazol-5-yl)-3-[7-(hydroxymethyl)-1-benzothiophen-5-yl]-2,2-dimethylpropanoate as the second peak (yield: 51%).
retention time: 15.753 min [column: CHIRALPAK IH (4.6 mm I.D. × 250 mm), mobile phase: n-hexane/ethanol=85/15(V/V), flow rate: 1.0 mL/min, temperature: 40°C, wavelength: 254 nm]

### (12b) Methyl (3R)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoate

To a solution of methyl (3R)-3-(1,4-dimethyl-1H-benzotriazol-5-yl)-3-[7-(hydroxymethyl)-1-benzothiophen-5-yl]-2,2-dimethylpropanoate (39.0 mg) of Example 12a in dichloromethane (2.0 mL) was added thionyl chloride (0.0141 mL) and the mixture was stirred at room temperature for 2 hr. The solvent was evaporated under reduced pressure, and the residue was azeotropically distilled with toluene. The residue was dissolved in acetonitrile (1.0 mL), and added to another solution of N,N-diisopropylethylamine (0.160 mL) and (2R)-2-ethyl-2,3,4,5-tetrahydropyrido[2,3-f][1,4]oxazepin-7-ol hydrochloride (25.5 mg) of Example 1h in acetonitrile (1.0 mL), and the mixture was stirred at 80°C for 12 hr and cooled to room temperature. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V), ethyl acetate/methanol=100/0-95/5(V/V)] to give the title compound (55.0 mg, yield: 100%) as an oil.

### (12c)=(12)

### (3R)-3-(1,4-Dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)-2,2-dimethylpropanoic acid

To a solution of methyl (3R)-3-(1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)-2,2-dimethylpropanoate (55.0 mg) of Example 12b in dimethyl sulfoxide (1.0 mL) was added 1 M aqueous potassium hydroxide solution (0.459 mL), and the mixture was stirred at 70°C for 12 hr. The reaction mixture was neutralized with 1 M hydrochloric acid, saturated aqueous ammonium chloride solution was added, and the mixture was extracted with ethyl acetate. The organic layer was washed with saturated brine and dried over anhydrous sodium sulfate. The solvent was evaporated under reduced pressure, and the residue was purified by silica gel column chromatography [elution solvent: n-hexane/ethyl acetate=30/70-0/100(V/V), ethyl acetate/methanol=100/0-95/5(V/V)]. Ethyl acetate and n-hexane were added to the obtained solid, and an ultrasonic treatment was performed. The solid was collected by filtration and vacuum dried to give the title compound (51.0 mg, yield: 94.9%) as a solid.

### (Example 13)

### (3S)-3-(1,4-Dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}-2,2-dimethylpropanoic acid

### (13a) Methyl (3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoate

To a solution of methyl (3S)-3-(1,4-dimethyl-1H-benzotriazol-5-yl)-3-[7-(hydroxymethyl)-1-benzothiophen-5-yl]-2,2-dimethylpropanoate (366 mg) of Example 11e in dichloromethane (10 mL) was added thionyl chloride (0.126 mL) and the mixture was stirred at room temperature for 1 hr. The solvent was evaporated under reduced pressure, and the residue was azeotropically distilled with toluene. The obtained residue was dissolved in acetonitrile (10 mL), N,N-diisopropylethylamine (0.740 mL) and 4',5'-dihydro-3'H-spiro[cyclopropane-1,2'-pyrido[2,3-f][1,4]oxazepin]-7'-ol hydrochloride (217 mg) of Example 3g were added, and the mixture was stirred at 85°C for 15 hr and cooled to room temperature. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent:
dichloromethane/methanol=100/0-90/10(V/V)] to give the title compound (489 mg, yield: 95%) as an oil.

### (13b)=(13)

### (3S)-3-(1,4-Dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}-2,2-dimethylpropanoic acid

To a solution of methyl (3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoate (489 mg) of Example 13a in dimethyl sulfoxide (8 mL) was added 1 M aqueous potassium hydroxide solution (4.09 mL), and the mixture was stirred at 70°C for 24 hr and neutralized with 1 M hydrochloric acid. Saturated aqueous ammonium chloride solution was added thereto, and the mixture was extracted with ethyl acetate. The organic layer was washed with saturated brine, dried over anhydrous sodium sulfate, and concentrated under reduced pressure. The residue was purified by silica gel column chromatography [elution solvent: n-hexane/ethyl acetate=40/60-30/70(V/V)]. To the obtained solid was added diisopropyl ether, and an ultrasonic treatment was performed. The solid was collected by filtration and washed with diisopropyl ether. Vacuum drying at 60°C for 3 hr gave the title compound (379 mg, yield: 79%) as a solid.

### (Example 14)

### (3S)-3-(7-{[(2R)-2-Cyclopropyl-7-hydroxy-2,3-dihydropyrido[2,3-f][1,4]oxazepin-4(5H)-yl]methyl}-1-benzothiophen-5-yl)-3-(1,4-dimethyl-1H-benzotriazol-5-yl)-2,2-dimethylpropanoic acid

### (14a) (2R)-2-Cyclopropyl-2,3,4,5-tetrahydropyrido[2,3-f] [1,4]oxazepin-7-ol

To a solution of (2R)-2-cyclopropyl-4-(4-methoxybenzyl)-2,3,4,5-tetrahydropyrido[2,3-f][1,4]oxazepin-7-ol (1.1 g) of Example 5d in trifluoroacetic acid (10 mL) was added anisole (5.5 mL), and the mixture was stirred at 100°C for 20 hr. The reaction mixture was cooled to room temperature, the solvent was evaporated under reduced pressure, toluene (20 mL) was added and azeotropic distillation was performed. The obtained residue was purified by silica gel column chromatography [elution solvent: chloroform/methanol=90/1-20/1-9:1(V/V)] to give the title compound (537 mg, purity 70%, 54%) as an amorphous solid.

### (14b) Methyl (3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)-2,2-dimethylpropanoate

To a solution of methyl (3S)-3-(1,4-dimethyl-1H-benzotriazol-5-yl)-3-[7-(hydroxymethyl)-1-benzothiophen-5-yl]-2,2-dimethylpropanoate (35 mg) of Example 11e in dichloromethane (2 mL) was added thionyl chloride (0.012 mL) and the mixture was stirred at room temperature for 2 hr. The solvent was evaporated under reduced pressure, and the residue was azeotropically distilled with toluene. The residue was dissolved in acetonitrile (2 mL), N,N-diisopropylethylamine (0.142 mL) and (2R)-2-cyclopropyl-2,3,4,5-tetrahydropyrido[2,3-f][1,4]oxazepin-7-ol (34 mg) of Example 14a were added, and the mixture was stirred at 85°C for 12 hr. (2R)-2-cyclopropyl-2,3,4,5-tetrahydropyrido[2,3-f][1,4]oxazepin-7-ol (34 mg) was further added thereto, and the mixture was stirred at 85°C for 5 hr. Water was added to the reaction mixture, and the mixture was extracted with ethyl acetate. The organic layer was washed with water and saturated brine, and dried over anhydrous sodium sulfate. Under reduced pressure, the solvent was evaporated and the obtained residue was purified by silica gel column chromatography [elution solvent:
dichloromethane/methanol=100/0-90/10(V/V)] to give the title compound (35 mg, yield: 69%) as a yellow oil.

### (14c)=(14)

### (3S)-3-(7-{[(2R)-2-Cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)-2,2-dimethylpropanoic acid

To a solution of methyl (3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)-2,2-dimethylpropanoate (35 mg) of Example 14b in dimethyl sulfoxide (1 mL) was added 1M aqueous potassium hydroxide solution (0.286 mL), and the mixture was stirred at 70°C for 12 hr and neutralized with 1M hydrochloric acid. Saturated aqueous ammonium chloride solution was added thereto, and the mixture was extracted with ethyl acetate. The organic layer was washed with saturated brine and dried over anhydrous sodium sulfate. The solvent was evaporated under reduced pressure, and the residue was purified by silica gel column chromatography [elution solvent: chloroform/methanol=100/0-90/10(V/V)] to give the title compound (11 mg, yield: 32%) as a pale-yellow solid.

### (Example 15)

### (3S)-3-(1,4-Dimethyl-1H-benzotriazol-5-yl)-3-(7-{[(2R)-7-hydroxy-2-methyl-2,3-dihydropyrido[2,3-f] [1,4]oxazepin-4(5H)-yl]methyl}-1-benzothiophen-5-yl)propanoic acid

### (Example 16)

### (3R)-3-(1,4-Dimethyl-1H-benzotriazol-5-yl)-3-(7-{[(2R)-7-hydroxy-2-methyl-2,3-dihydropyrido[2,3-f] [1,4]oxazepin-4(5H)-yl]methyl}-1-benzothiophen-5-yl)propanoic acid

### (15a) (2R)-1-{[(6-Bromo-3-fluoropyridin-2-yl)methyl]amino}propan-2-ol

To a solution of 6-bromo-3-fluoro-2-methylpyridine (1.90 g) in carbon tetrachloride (50 mL) were added N-bromosuccinimide (1.96 g) and 75% benzoyl peroxide (242 mg), and the mixture was heated under reflux for 4 hr. The reaction mixture was cooled to room temperature, water was added, and the mixture was extracted with chloroform. The organic layer was dried over anhydrous sodium sulfate. Under reduced pressure, the solvent was evaporated and the obtained residue was purified by silica gel column chromatography [elution solvent: n-hexane/ethyl acetate=90/10-70/30(V/V)]. The obtained residue was dissolved in dichloromethane (30 mL), (2R)-1-aminopropan-2-ol (0.79 mL) and triethylamine (2.77 mL) were added and the mixture was stirred at room temperature for 15 hr. Under reduced pressure, the solvent in the reaction mixture was evaporated and the obtained residue was purified by silica gel column chromatography [elution solvent:
chloroform/methanol=99/1-95/5(V/V)] to give the title compound (670 mg, yield: 25%) as an oil.

### (15b) (2R)-1-{[(6-Bromo-3-fluoropyridin-2-yl)methyl](4-methoxybenzyl)amino}propan-2-ol

To a solution of (2R)-1-{[(6-bromo-3-fluoropyridin-2-yl)methyl]amino}propan-2-ol (660 mg) of Example 15a in dichloromethane (10 mL) was added anisaldehyde (0.61 mL) and the mixture was stirred at room temperature for 0.5 hr. Then, sodium triacetoxyborohydride (1.06 g) was added thereto, and the mixture was stirred at room temperature for 4 hr. To the reaction mixture was added saturated aqueous sodium hydrogen carbonate solution, and the mixture was extracted with chloroform. The organic layer was dried over anhydrous sodium sulfate. Under reduced pressure, the solvent was evaporated and the obtained residue was purified by silica gel column chromatography [elution solvent: n-hexane/ethyl acetate=90/10-70/30(V/V)] to give the title compound (940 mg, yield: 98%) as an oil.

### (15c) (2R)-7-Bromo-4-(4-methoxybenzyl)-2-methyl-2,3,4,5-tetrahydropyrido[2,3-f][1,4]oxazepine

To a solution of (2R)-1-{[(6-bromo-3-fluoropyridin-2-yl)methyl](4-methoxybenzyl)amino}propan-2-ol (820 mg) of Example 15b in N,N-dimethylformamide (30 mL) was added 60% sodium hydride (94 mg) at 0°C and the mixture was stirred at room temperature for 16.5 hr. To the reaction mixture was added water, and the mixture was extracted with ethyl acetate. The organic layer was washed with water and saturated brine, and dried over anhydrous sodium sulfate. Under reduced pressure, the solvent was evaporated and the obtained residue was purified by silica gel column chromatography [elution solvent: n-hexane/ethyl acetate=90/10-0/100(V/V)] to give the title compound (210 mg, yield: 27%) as an oil.

### (15d) (2R)-4-(4-Methoxybenzyl)-2-methyl-2,3,4,5-tetrahydropyrido[2,3-f][1,4]oxazepin-7-ol

To a solution of (2R)-7-bromo-4-(4-methoxybenzyl)-2-methyl-2,3,4,5-tetrahydropyrido[2,3-f][1,4]oxazepine (330 mg) of Example 15c in 1,4-dioxane (5 mL) were added bis(dibenzylideneacetone)palladium(0) (26 mg), 5-(di-tert-butylphosphino)-1',3',5'-triphenyl-1'H-1,4'-bipyrazole (46 mg) and cesium hydroxide monohydrate (457 mg), and the mixture was stirred at 100°C for 4 hr. The reaction mixture was cooled to room temperature, 1 M hydrochloric acid and saturated aqueous sodium hydrogen carbonate solution were added, and the mixture was extracted with chloroform. The organic layer was dried over anhydrous sodium sulfate. Under reduced pressure, the solvent was evaporated and the obtained residue was purified by silica gel column chromatography [elution solvent: chloroform/methanol=100/0-95/5(V/V)] to give the title compound (210 mg, yield: 77%) as an oil.

### (15e) (2R)-2-Methyl-2,3,4,5-tetrahydropyrido[2,3-f][1,4]oxazepin-7-ol hydrochloride

A solution of (2R)-4-(4-methoxybenzyl)-2-methyl-2,3,4,5-tetrahydropyrido[2,3-f][1,4]oxazepin-7-ol (210 mg) of Example 15d in trifluoroacetic acid (5 mL) was heated under reflux for 60.5 hr. The reaction mixture was cooled to room temperature, and azeotropically distilled with toluene. The obtained residue was dissolved in ethyl acetate (5 mL), 1 M hydrogen chloride-ethyl acetate solution (2 mL) was added and the mixture was stirred at room temperature for 10 min. To the reaction mixture was added tert-butyl methyl ether (5 mL), and the solid was collected by filtration to give the title compound (180 mg, yield: quantitative) as a solid.

### (15f) Ethyl 3-(1,4-dimethyl-1H-benzotriazol-5-yl)-3-(7-{[(2R)-7-hydroxy-2-methyl-2,3-dihydropyrido[2,3-f][1,4]oxazepin-4(5H)-yl]methyl}-1-benzothiophen-5-yl)propanoate

Ethyl 3-(1,4-dimethyl-1H-benzotriazol-5-yl)-3-[7-(hydroxymethyl)-1-benzothiophen-5-yl]propanoate (200 mg) of Example 1f was dissolved in dichloromethane (5 mL), dimethyl sulfoxide (0.17 mL), N,N-diisopropylethylamine (0.21 mL) and sulfur trioxide-pyridine complex (194 mg) were added and the mixture was stirred at room temperature for 30 min. Dimethyl sulfoxide (0.085 mL), N,N-diisopropylethylamine (0.10 mL) and sulfur trioxide-pyridine complex (97 mg) were added, and the mixture was stirred at room temperature for 30 min. To the reaction mixture was added water, and the mixture was extracted with ethyl acetate. The organic layer was washed successively with water and saturated brine, and dried over anhydrous sodium sulfate. The solvent was evaporated under reduced pressure and the obtained residue was dissolved in dichloromethane (5 mL), (2R)-2-methyl-2,3,4,5-tetrahydropyrido[2,3-f][1,4]oxazepin-7-ol hydrochloride (180 mg) of Example 15e, N,N-diisopropylethylamine (0.17 mL) and acetic acid (0.17 mL) were added and the mixture was stirred at room temperature for 20 min. Then, sodium triacetoxyborohydride (207 mg) was added thereto, and the mixture was stirred at room temperature for 1.5 hr. Sodium triacetoxyborohydride (207 mg) was added, and the mixture was stirred at room temperature for 30 min and at 40°C for 30 min. To the reaction mixture was added anhydrous sodium sulfate (300 mg), and the mixture was stirred at 40°C for 30 min. To the reaction mixture was added saturated aqueous sodium hydrogen carbonate solution, and the mixture was extracted with chloroform. The organic layer was dried over anhydrous sodium sulfate. The solvent was evaporated under reduced pressure and the obtained residue was purified by DIOL silica gel column chromatography [elution solvent: n-hexane/ethyl acetate=50/50-0/100(V/V)] to give the title compound (230 mg, yield: 82%) as a solid.

### (15g)=(15)

### (3S)-3-(1,4-Dimethyl-1H-benzotriazol-5-yl)-3-(7-{[(2R)-7-hydroxy-2-methyl-2,3-dihydropyrido[2,3-f] [1,4]oxazepin-4(5H)-yl]methyl}-1-benzothiophen-5-yl)propanoic acid

### (16a)=(16)

### (3R)-3-(1,4-Dimethyl-1H-benzotriazol-5-yl)-3-(7-{ [(2R)-7-hydroxy-2-methyl-2,3-dihydropyrido[2,3-f] [1,4]oxazepin-4(5H)-yl]methyl}-1-benzothiophen-5-yl)propanoic acid

To a mixture of ethyl 3-(1,4-dimethyl-1H-benzotriazol-5-yl)-3-(7-{[(2R)-7-hydroxy-2-methyl-2,3-dihydropyrido[2,3-f] [1,4]oxazepin-4(5H)-yl]methyl}-1-benzothiophen-5-yl)propanoate (230 mg) of Example 15f, tetrahydrofuran (3.6 mL) and methanol (1.2 mL) was added 1 M aqueous lithium hydroxide solution (0.12 mL), and the mixture was stirred at 40°C for 30 min and at 60°C for 30 min. 1 M aqueous lithium hydroxide solution (0.12 mL) was added thereto, and the mixture was stirred at 60°C for 1.5 hr. To the reaction mixture was added saturated aqueous ammonium chloride solution, and the mixture was extracted with a mixed solvent of ethyl acetate and tetrahydrofuran. The obtained aqueous layer was extracted with chloroform, and the organic layer was dried over anhydrous sodium sulfate. Under reduced pressure, the solvent was evaporated and the obtained residue was purified by silica gel column chromatography [elution solvent:
chloroform/methanol=99/1-95/5(V/V)]. Ethyl acetate and n-hexane were added to the compound eluted earlier, and an ultrasonic treatment was performed. The solid was collected by filtration to give (3R)-3-(1,4-dimethyl-1H-benzotriazol-5-yl)-3-(7-{[(2R)-7-hydroxy-2-methyl-2,3-dihydropyrido[2,3-f] [1,4]oxazepin-4(5H)-yl]methyl}-1-benzothiophen-5-yl)propanoic acid (68.8 mg, yield: 31%) as a solid.

In the same manner, moreover, the compound eluted later, (3S)-3-(1,4-dimethyl-1H-benzotriazol-5-yl)-3-(7-{[(2R)-7-hydroxy-2-methyl-2,3-dihydropyrido[2,3-f] [1,4]oxazepin-4(5H)-yl]methyl}-1-benzothiophen-5-yl)propanoic acid (61.3 mg, yield: 28%), was obtained as a solid.

### (Example 17)

### (3S)-3-(7-{[(2R)-2-Ethyl-7-hydroxy-2,3-dihydropyrido[2,3-f][1,4]oxazepin-4(5H)-yl]methyl}-1-benzothiophen-5-yl)-3-(1,4,7-trimethyl-1H-benzotriazol-5-yl)propanoic acid

### (Example 18)

### (3R)-3-(7-{[(2R)-2-Ethyl-7-hydroxy-2,3-dihydropyrido[2,3-f][1,4]oxazepin-4(5H)-yl]methyl}-1-benzothiophen-5-yl)-3-(1,4,7-trimethyl-1H-benzotriazol-5-yl)propanoic acid

### (17a) (2R)-1-Aminobutan-2-ol

To a solution of (2R)-2-ethyloxirane (25 mL) in ethanol (100 mL) was added 28% aqueous ammonia solution (100 mL) and the mixture was stirred at room temperature for 24 hr. The solvent was evaporated under reduced pressure, and the residue was azeotropically distilled with toluene. A mixture (21.6 g) containing the title compound was obtained as an oil.

### (17b) (2R)-1-{[(6-Bromo-3-fluoropyridin-2-yl)methyl](4-methoxybenzyl)amino}butan-2-ol

To a solution of (2R)-1-aminobutan-2-ol (1.16 g) of Example 17a and 4-methoxybenzaldehyde (0.79 mL) in dichloromethane (26 mL) were added anhydrous magnesium sulfate (1.56 g) and one drop of acetic acid, and the mixture was stirred at room temperature for 45 min. Then, sodium triacetoxyborohydride (2.76 g) was added thereto, and the mixture was stirred at room temperature for 80 min. To the reaction mixture was added saturated aqueous sodium hydrogen carbonate solution, and the mixture was extracted with chloroform. The organic layer was dried over anhydrous sodium sulfate. The solvent was evaporated under reduced pressure. To a solution of the obtained residue and 6-bromo-2-(bromomethyl)-3-fluoropyridine (2.53 g) of Example 3a in acetonitrile (33 mL) was added potassium carbonate (1.17 g), and the mixture was stirred at 50°C for 3 hr. The reaction mixture was cooled to room temperature, insoluble material was filtered off, and washed with ethyl acetate. The filtrate was concentrated under reduced pressure and the obtained residue was purified by silica gel column chromatography [elution solvent: n-hexane/ethyl acetate=100/0-60/40(V/V)] to give the title compound (2.22 g, yield: 86%) as an oil.

### (17c) (2R)-7-Bromo-2-ethyl-4-(4-methoxybenzyl)-2,3,4,5-tetrahydropyrido[2,3-f][1,4]oxazepine

To a suspension of 60% sodium hydride (640 mg) in N,N-dimethylformamide (38 mL) was added a solution of (2R)-1-{[(6-bromo-3-fluoropyridin-2-yl)methyl](4-methoxybenzyl)amino}butan-2-ol (2.12 g) of Example 17b in tetrahydrofuran (38 mL) at 60°C, and the mixture was stirred at 60°C for 2 hr. The reaction mixture was cooled to room temperature, water was added, and the mixture was extracted with ethyl acetate. The organic layer was washed successively with water and saturated brine, and dried over anhydrous sodium sulfate. Under reduced pressure, the solvent was evaporated and the obtained residue was purified by silica gel column chromatography [elution solvent: n-hexane/ethyl acetate=100/0-60/40(V/V)] to give the title compound (1.84 g, yield: 87%) as an oil.

### (17d) (2R)-2-Ethyl-4-(4-methoxybenzyl)-2,3,4,5-tetrahydropyrido[2,3-f][1,4]oxazepin-7-ol

To a solution of (2R)-7-bromo-2-ethyl-4-(4-methoxybenzyl)-2,3,4,5-tetrahydropyrido[2,3-f] [1,4]oxazepine (1.84 g) of Example 17c in 1,4-dioxane (24 mL) were added bis(dibenzylideneacetone)palladium(0) (140 mg), 5-(di-tert-butylphosphino)-1',3',5'-triphenyl-1'H-1,4'-bipyrazole(247 mg) and cesium hydroxide monohydrate (2.45 g), and the mixture was stirred at 100°C for 4 hr. The reaction mixture was cooled to room temperature, saturated aqueous ammonium chloride solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: chloroform/methanol=100/0-95/5(V/V)] to give the title compound (1.07 g, yield: 70%) as a solid.

### (17e) (2R)-2-Ethyl-2,3,4,5-tetrahydropyrido[2,3-f][1,4]oxazepin-7-ol

To a solution of (2R)-2-ethyl-4-(4-methoxybenzyl)-2,3,4,5-tetrahydropyrido[2,3-f][1,4]oxazepin-7-ol (512 mg) of Example 17d in trifluoroacetic acid (8.2 mL) was added anisole (1.77 mL), and the mixture was stirred at 100°C for 95 min under microwave irradiation. The reaction mixture was cooled to room temperature, azeotropically distilled with toluene, and the obtained residue was purified by NH silica gel column chromatography [elution solvent: chloroform/methanol=100/0-90/10(V/V)] to give the title compound (274 mg, yield: 86%) as a solid.

### (17f) 4-Bromo-N,3,6-trimethyl-2-nitroaniline

2,5-Dimethylaniline (25.1 g) was dissolved in N,N-dimethylformamide (207 mL), N-bromosuccinimide (38.6 g) was added in portions over 17 min at 0°C and the mixture was stirred at 0°C for 15 min. To the reaction mixture was added water, and the mixture was extracted with ethyl acetate. The organic layer was washed successively with water and saturated brine, dried over anhydrous sodium sulfate, and the solvent was evaporated under reduced pressure. The obtained residue was dissolved in dichloromethane (690 mL), triethylamine (43.3 mL) was added, trifluoroacetic anhydride (35.0 mL) was added dropwise over 11 min at 0°C, and the mixture was stirred at room temperature for 25 min. To the reaction mixture was added saturated aqueous sodium hydrogen carbonate solution, and the mixture was extracted with chloroform. The organic layer was washed with 1 M hydrochloric acid and saturated brine, dried over anhydrous sodium sulfate, and the solvent was evaporated under reduced pressure. The obtained residue was dissolved in concentrated sulfuric acid (150 mL), and then 69% concentrated nitric acid (13.6 mL) was added dropwise over 5 min while cooling in an ice-salt bath, and the mixture was stirred at room temperature for 45 min. The reaction mixture was added to ice water, and the precipitate was collected by filtration and washed with water. The obtained solid was dissolved in chloroform, saturated aqueous sodium hydrogen carbonate solution was added, and the mixture was extracted with a mixed solvent of chloroform and methanol. The organic layer was dried over anhydrous sodium sulfate and the solvent was evaporated under reduced pressure. To the obtained residue were added tert-butyl methyl ether and n-hexane and, after an ultrasonic treatment, the solid was collected by filtration. 60% Sodium hydride (7.40 g) was suspended in N,N-dimethylformamide (310 mL), the solid obtained in the earlier reaction was added in portions over 10 min at 0°C, and the mixture was stirred at room temperature for 18 min. Methyl iodide (11.5 mL) was added dropwise over 4 min at 0°C, and the mixture was stirred at room temperature for 50 min, at 50°C for 5 min, and at room temperature for 80 min. The reaction mixture was added to the saturated aqueous ammonium chloride solution at 0°C, and the mixture was extracted with ethyl acetate. The organic layer was washed with water and saturated brine, dried over anhydrous sodium sulfate, and the solvent was evaporated under reduced pressure. The obtained residue was dissolved in ethanol (510 mL), 5 M aqueous sodium hydroxide solution (154 mL) was added, and the mixture was heated under reflux for 20 min. The reaction mixture was cooled to room temperature and concentrated under reduced pressure. Water was added, and the mixture was extracted with a mixed solvent of chloroform and methanol. The organic layer was dried over anhydrous sodium sulfate and the solvent was evaporated under reduced pressure. To the obtained residue was added n-hexane, and an ultrasonic treatment was performed. The solid was collected by filtration to give the title compound (24.1 g, yield: 45%) as a solid.

### (17g) 4-Bromo-N¹,3,6-trimethylbenzene-1,2-diamine

4-Bromo-N,3,6-trimethyl-2-nitroaniline (24.1 g) of Example 17f was dissolved in acetic acid (280 mL), zinc powder (30.4 g) was added in portions over 9 min, and the mixture was stirred at room temperature for 14 min. To the reaction mixture was added ethyl acetate, and the mixture was filtered through Celite, and the insoluble material was removed. To the filtrate were added saturated brine and water, and the mixture was extracted with ethyl acetate. The organic layer was washed with saturated aqueous sodium hydrogen carbonate solution and saturated brine, dried over anhydrous sodium sulfate, and the solvent was evaporated under reduced pressure. The obtained residue was purified by silica gel column chromatography [elution solvent: n-hexane/ethyl acetate/chloroform=90/10/5-50/50/5 (V/V/V)] to give the title compound (16.5 g, yield: 77%) as an oil.

### (17h) 5-Bromo-1,4,7-trimethyl-1H-benzotriazole

4-Bromo-N¹,3,6-trimethylbenzene-1,2-diamine (16.5 g) of Example 17g was dissolved in dichloromethane (144 mL), cooled in an ice-salt bath, tert-butyl nitrite (10.4 mL) was added dropwise over 5 min, 42% tetrafluoroboric acid (23.0 mL) was added dropwise over 7 min, and the mixture was stirred at room temperature for 28 min. The reaction mixture was added to a saturated aqueous sodium hydrogen carbonate solution, and the mixture was extracted with chloroform. The organic layer was dried over anhydrous sodium sulfate and the solvent was evaporated under reduced pressure. To the obtained residue were added ethyl acetate and n-hexane, and an ultrasonic treatment was performed. The solid was collected by filtration to give the title compound (13.5 g, yield: 78%) as a solid.

### (17i) Ethyl (2E)-3-(1,4,7-trimethyl-1H-benzotriazol-5-yl)prop-2-enoate

5-Bromo-1,4,7-trimethyl-1H-benzotriazole (13.5 g) of Example 17h was dissolved in N,N-dimethylformamide (187 mL), ethyl acrylate (61.2 mL), N,N-diisopropylethylamine (48.9 mL), tris(dibenzylideneacetone)dipalladium(0) (5.15 g) and tri(o-tolyl)phosphine (6.85 g) were added, and the mixture was stirred under a nitrogen atmosphere at 100°C for 11 hr. The reaction mixture was cooled to room temperature, water was added, and the mixture was extracted with a mixed solvent of ethyl acetate, methanol and tetrahydrofuran. The organic layer was washed successively with water and saturated brine, dried over anhydrous sodium sulfate, and the solvent was evaporated under reduced pressure. The obtained residue was purified by silica gel column chromatography [elution solvent: n-hexane/ethyl acetate/chloroform=90/10/10-40/60/20 (V/V/V)] to give the title compound (6.10 g, yield: 84%) as a solid.

### (17j) Ethyl 3-[7-(hydroxymethyl)-1-benzothiophen-5-yl]-3-(1,4,7-trimethyl-1H-benzotriazol-5-yl)propanoate

Using ethyl (2E)-3-(1,4,7-trimethyl-1H-benzotriazol-5-yl)prop-2-enoate (6.10 g) of Example 17i and [5-(4,4,5,5-tetramethyl-1,3,2-dioxaborolan-2-yl)-1-benzothiophen-7-yl]methanol (11.6 g) of Example 1e, and by a method similar to that of Example 1f, the title compound (6.89 g, yield: 69%) was obtained as a solid.

### (17k) Ethyl 3-(7-{[(2R)-2-ethyl-7-hydroxy-2,3-dihydropyrido[2,3-f][1,4]oxazepin-4(5H)-yl]methyl}-1-benzothiophen-5-yl)-3-(1,4,7-trimethyl-1H-benzotriazol-5-yl)propanoate

To a solution of ethyl 3-[7-(hydroxymethyl)-1-benzothiophen-5-yl]-3-(1,4,7-trimethyl-1H-benzotriazol-5-yl)propanoate (275 mg) of Example 17j in dichloromethane (2.6 mL) were added dimethyl sulfoxide (0.23 mL), N,N-diisopropylethylamine (0.35 mL) and sulfur trioxide-pyridine complex (345 mg) at 0°C and the mixture was stirred at room temperature for 25 min. To the reaction mixture were added 2 M hydrochloric acid and water, and the mixture was extracted with ethyl acetate. The organic layer was washed successively with water and saturated brine, and dried over anhydrous sodium sulfate. The solvent was evaporated under reduced pressure and the obtained residue was dissolved in dichloromethane (4.2 mL). (2R)-2-ethyl-2,3,4,5-tetrahydropyrido[2,3-f][1,4]oxazepin-7-ol (137 mg) of Example 17e and dimethyl sulfoxide (1 mL) were added, and the mixture was stirred at 40°C for 5 min. Anhydrous magnesium sulfate (156 mg) and one drop of acetic acid were added thereto, and the mixture was stirred at 40°C for 30 min. The reaction mixture was cooled to room temperature, sodium triacetoxyborohydride (276 mg) was added, and the mixture was stirred at 35°C for 13 hr. To the reaction mixture was added saturated aqueous sodium hydrogen carbonate solution, and the mixture was extracted with ethyl acetate. The organic layer was washed with water and saturated brine, and dried over anhydrous sodium sulfate. Under reduced pressure, the solvent was evaporated and the obtained residue was purified by silica gel column chromatography [elution solvent: n-hexane/ethyl acetate=20/80-0/100(V/V), chloroform/methanol=100/0-96/4(V/V)] to give the title compound (251 mg, yield: 65%) as a solid.

### (171)=(17)

### (3S)-3-(7-{[(2R)-2-Ethyl-7-hydroxy-2,3-dihydropyrido[2,3-f][1,4]oxazepin-4(5H)-yl]methyl}-1-benzothiophen-5-yl)-3-(1,4,7-trimethyl-1H-benzotriazol-5-yl)propanoic acid

### (18a)=(18)

### (3R)-3-(7-{[(2R)-2-Ethyl-7-hydroxy-2,3-dihydropyrido[2,3-f][1,4]oxazepin-4(5H)-yl]methyl}-1-benzothiophen-5-yl)-3-(1,4,7-trimethyl-1H-benzotriazol-5-yl)propanoic acid

To a mixture of ethyl 3-(7-{[(2R)-2-ethyl-7-hydroxy-2,3-dihydropyrido[2,3-f] [1,4]oxazepin-4(5H)-yl]methyl}-1-benzothiophen-5-yl)-3-(1,4,7-trimethyl-1H-benzotriazol-5-yl)propanoate (251 mg) of Example 17k, tetrahydrofuran (3.9 mL) and methanol (1.3 mL) was added 1 M aqueous lithium hydroxide solution (1.3 mL), and the mixture was stirred at 40°C for 2 hr. To the reaction mixture was added saturated aqueous ammonium chloride solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: chloroform/methanol=100/0-95/5(V/V)]. Ethyl acetate and n-hexane were added to the compound eluted earlier, and an ultrasonic treatment was performed. The solid was collected by filtration to give (3R)-3-(7-{[(2R)-2-ethyl-7-hydroxy-2,3-dihydropyrido[2,3-f] [1,4]oxazepin-4(5H)-yl]methyl}-1-benzothiophen-5-yl)-3-(1,4,7-trimethyl-1H-benzotriazol-5-yl)propanoic acid (98 mg, yield: 41%) as a solid.

In the same manner, moreover, the compound eluted later, (3S)-3-(7-{[(2R)-2-ethyl-7-hydroxy-2,3-dihydropyrido[2,3-f][1,4]oxazepin-4(5H)-yl]methyl}-1-benzothiophen-5-yl)-3-(1,4,7-trimethyl-1H-benzotriazol-5-yl)propanoic acid (60 mg, yield: 25%) was obtained as a solid.

### (Example 19)

### (3R)-3-(7-{[(2R)-2-Ethyl-7-hydroxy-2,3-dihydropyrido[2,3-f] [1,4]oxazepin-4(5H)-yl]methyl}-1-benzothiophen-5-yl)-3-[1-(2-hydroxy-2-methylpropyl)-4-methyl-1H-benzotriazol-5-yl]propanoic acid

### (Example 20)

### (3S)-3-(7-{[(2R)-2-Ethyl-7-hydroxy-2,3-dihydropyrido[2,3-f] [1,4]oxazepin-4(5H)-yl]methyl}-1-benzothiophen-5-yl)-3-[1-(2-hydroxy-2-methylpropyl)-4-methyl-1H-benzotriazol-5-yl]propanoic acid

### (19a) 2-Methyl-1-(3-methyl-2-nitroanilino)propan-2-ol

To a solution of 1-fluoro-3-methyl-2-nitrobenzene (1.00 g) and potassium carbonate (1.35 g) in ethanol (13 mL) was added 1-amino-2-methyl-2-propanol (1.83 mL), and the mixture was stirred at 70°C for 24 hr. The reaction mixture was cooled to room temperature, and concentrated under reduced pressure. Saturated aqueous sodium hydrogen carbonate solution was added to the residue, the mixture was extracted with ethyl acetate, and the organic layer was dried over anhydrous sodium sulfate. After filtration, the filtrate was concentrated under reduced pressure, and the obtained residue was purified by silica gel chromatography [elution solvent: n-hexane/ethyl acetate=95/5-80/20(V/V)] to give the title compound (750 mg, yield: 52%) as a solid.

### (19b) 1-(4-Bromo-3-methyl-2-nitroanilino)-2-methylpropan-2-ol

To a solution of 2-methyl-1-(3-methyl-2-nitroanilino)propan-2-ol (750 mg) of Example 19a in N,N-dimethylformamide (3.3 mL) was added N-bromosuccinimide (607 mg) at 0°C, and the mixture was stirred at room temperature for 24 hr. To the reaction mixture was added saturated aqueous sodium hydrogen carbonate solution, and the mixture was extracted with ethyl acetate. The organic layer was washed with 10% brine, and dried over anhydrous sodium sulfate. After filtration, the filtrate was concentrated under reduced pressure, and the obtained residue was purified by silica gel column chromatography [elution solvent: n-hexane/ethyl acetate=95/5-80/20(V/V)] to give the title compound (1.01 g, yield: 100%) as a solid.

### (19c) 1-(2-Amino-4-bromo-3-methylanilino)-2-methylpropan-2-ol

Using 1-(4-bromo-3-methyl-2-nitroanilino)-2-methylpropan-2-ol (1.01 g) of Example 19b, and by a method similar to that of the below-mentioned Example 41e, the title compound (810 mg, yield: 89%) was obtained as a solid.

### (19d) 1-(5-Bromo-4-methyl-1H-benzotriazol-1-yl)-2-methylpropan-2-ol

Using 1-(2-amino-4-bromo-3-methylanilino)-2-methylpropan-2-ol (810 mg) of Example 19c, and by a method similar to that of the below-mentioned Example 41f, the title compound (589 mg, yield: 70%) was obtained as a solid.

### (19e) Ethyl (2E)-3-[1-(2-hydroxy-2-methylpropyl)-4-methyl-1H-benzotriazol-5-yl]prop-2-enoate

Using 1-(5-bromo-4-methyl-1H-benzotriazol-1-yl)-2-methylpropan-2-ol (284 mg) of Example 19d, and by a method similar to that of the below-mentioned Example 41g, the title compound (297 mg, yield: 98%) was obtained as a solid.

### (19f) Ethyl 3-[7-(hydroxymethyl)-1-benzothiophen-5-yl]-3-[1-(2-hydroxy-2-methylpropyl)-4-methyl-1H-benzotriazol-5-yl]propanoate

Using ethyl (2E)-3-[1-(2-hydroxy-2-methylpropyl)-4-methyl-1H-benzotriazol-5-yl]prop-2-enoate (297 mg) of Example 19e and [5-(4,4,5,5-tetramethyl-1,3,2-dioxaborolan-2-yl)-1-benzothiophen-7-yl]methanol (426 mg) of Example 1e, and by a method similar to that of Example 1f, the title compound (148 mg, yield: 32%) was obtained as an oil.

### (19g) Ethyl 3-(7-formyl-1-benzothiophen-5-yl)-3-[1-(2-hydroxy-2-methylpropyl)-4-methyl-1H-benzotriazol-5-yl]propanoate

To a solution of ethyl 3-[7-(hydroxymethyl)-1-benzothiophen-5-yl]-3-[1-(2-hydroxy-2-methylpropyl)-4-methyl-1H-benzotriazol-5-yl]propanoate (62.0 mg) of Example 19f in dichloromethane (2.0 mL) was added manganese(IV) oxide (136 mg), and the mixture was stirred at 40°C for 6 hr. The reaction mixture was cooled to room temperature, was filtered through celite, and insoluble material was removed. Under reduced pressure, the solvent was evaporated and the obtained residue was purified by silica gel column chromatography [elution solvent: n-hexane/ethyl acetate=60/40-40/60(V/V)] to give the title compound (56.0 mg, yield: 91%) as an oil.

### (19h) Ethyl 3-(7-{[(2R)-2-ethyl-7-hydroxy-2,3-dihydropyrido[2,3-f] [1,4]oxazepin-4(5H)-yl]methyl}-1-benzothiophen-5-yl)-3-[1-(2-hydroxy-2-methylpropyl)-4-methyl-1H-benzotriazol-5-yl]propanoate

To a solution of ethyl 3-(7-formyl-1-benzothiophen-5-yl)-3-[1-(2-hydroxy-2-methylpropyl)-4-methyl-1H-benzotriazol-5-yl]propanoate (56.0 mg) of Example 19g, (2R)-2-ethyl-2,3,4,5-tetrahydropyrido[2,3-f][1,4]oxazepin-7-ol hydrochloride (41.6 mg) of Example 1h, N,N-diisopropylethylamine (0.0314 mL) and acetic acid (0.0620 mL) in dichloromethane (2.0 mL) was added sodium triacetoxyborohydride (95.6 mg) at 0°C and the mixture was stirred at room temperature for 14 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/100/0-0/90/10(V/V/V)] to give the title compound (67.0 mg, yield: 87%) as a solid.

### (19i)=(19)

### (3R)-3-(7-{[(2R)-2-Ethyl-7-hydroxy-2,3-dihydropyrido[2,3-f] [1,4]oxazepin-4(5H)-yl]methyl}-1-benzothiophen-5-yl)-3-[1-(2-hydroxy-2-methylpropyl)-4-methyl-1H-benzotriazol-5-yl]propanoic acid

### (20a) =(20)

### (3S)-3-(7-{[(2R)-2-Ethyl-7-hydroxy-2,3-dihydropyrido[2,3-f] [1,4]oxazepin-4(5H)-yl]methyl}-1-benzothiophen-5-yl)-3-[1-(2-hydroxy-2-methylpropyl)-4-methyl-1H-benzotriazol-5-yl]propanoic acid

To a mixture of ethyl 3-(7-{[(2R)-2-ethyl-7-hydroxy-2,3-dihydropyrido[2,3-f] [1,4]oxazepin-4(5H)-yl]methyl}-1-benzothiophen-5-yl)-3-[1-(2-hydroxy-2-methylpropyl)-4-methyl-1H-benzotriazol-5-yl]propanoate (67.0 mg) of Example 19h, methanol (1.0 mL), and tetrahydrofuran (1.0 mL) was added 1 M aqueous sodium hydroxide solution (0.520 mL) and the mixture was stirred at room temperature for 24 hr. To the reaction mixture was added 1 M hydrochloric acid (0.520 mL) and the mixture was stirred, and concentrated under reduced pressure. Saturated aqueous ammonium chloride solution and saturated brine were added, and the mixture was extracted with chloroform/2-propanol=3/1(V/V). The organic layer was dried over anhydrous sodium sulfate. After filtration, the solvent was evaporated under reduced pressure and the obtained residue was purified by silica gel column chromatography [elution solvent: n-hexane/ethyl acetate/methanol=30/70/0-0/100/0-0/80/20(V/V/V)]. Ethyl acetate and n-hexane were added to the compound eluted earlier and the mixture was subjected to an ultrasonic treatment. The solid was collected by filtration and vacuum dried to give (3R)-3-(7-{[(2R)-2-ethyl-7-hydroxy-2,3-dihydropyrido[2,3-f] [1,4]oxazepin-4(5H)-yl]methyl}-1-benzothiophen-5-yl)-3-[1-(2-hydroxy-2-methylpropyl)-4-methyl-1H-benzotriazol-5-yl]propanoic acid (25.0 mg, yield: 39%) as a solid. In the same manner, moreover, the compound eluted later, (3S)-3-(7-{[(2R)-2-ethyl-7-hydroxy-2,3-dihydropyrido[2,3-f] [1,4]oxazepin-4(5H)-yl]methyl}-1-benzothiophen-5-yl)-3-[1-(2-hydroxy-2-methylpropyl)-4-methyl-1H-benzotriazol-5-yl]propanoic acid (24.0 mg, yield: 38%), was obtained as a solid.

### (Example 21)

### (3R)-3-(7-{[(2R)-2-Ethyl-7-hydroxy-2,3-dihydropyrido[2,3-f] [1,4]oxazepin-4(5H)-yl]methyl}-1-benzothiophen-5-yl)-3-(7-methoxy-1,4-dimethyl-1H-benzotriazol-5-yl)propanoic acid

### (Example 22)

### (3S)-3-(7-{[(2R)-2-Ethyl-7-hydroxy-2,3-dihydropyrido[2,3-f] [1,4]oxazepin-4(5H)-yl]methyl}-1-benzothiophen-5-yl)-3-(7-methoxy-1,4-dimethyl-1H-benzotriazol-5-yl)propanoic acid

### (21a) Ethyl 3-[7-(hydroxymethyl)-1-benzothiophen-5-yl]-3-(7-methoxy-1,4-dimethyl-1H-benzotriazol-5-yl)propanoate

Ethyl (2E)-3-(7-methoxy-1,4-dimethyl-1H-benzotriazol-5-yl)prop-2-enoate (WO 2015/092713) (220 mg) was dissolved in a mixed solvent of 1,4-dioxane (4.0 mL) and water (1.3 mL), heated to 90°C, [5-(4,4,5,5-tetramethyl-1,3,2-dioxaborolan-2-yl)-1-benzothiophen-7-yl]methanol (348 mg) of Example 1e, triethylamine (0.499 mL), and chloro(1,5-cyclooctadiene)rhodium(I) dimer (121 mg) were added every 1 hr in 3 portions, and the mixture was further stirred with heating for 2 hr. The reaction mixture was cooled to room temperature, saturated aqueous sodium hydrogen carbonate solution was added, and the mixture was extracted with ethyl acetate. The organic layer was washed with saturated brine and dried over anhydrous sodium sulfate. After filtration, the filtrate was concentrated under reduced pressure, and the obtained residue was purified by silica gel column chromatography [elution solvent: n-hexane/ethyl acetate=70/30-40/60(V/V)] to give the title compound (164 mg, yield: 47%) as an oil.

### (21b) Ethyl 3-(7-formyl-1-benzothiophen-5-yl)-3-(7-methoxy-1,4-dimethyl-1H-benzotriazol-5-yl)propanoate

To a solution of ethyl 3-[7-(hydroxymethyl)-1-benzothiophen-5-yl]-3-(7-methoxy-1,4-dimethyl-1H-benzotriazol-5-yl)propanoate (60.0 mg) of Example 21a in dichloromethane (2.0 mL) was added manganese(IV) oxide (140 mg), and the mixture was stirred at 40°C for 6 hr. The reaction mixture was cooled to room temperature, the mixture was filtered through celite and insoluble material was removed. Under reduced pressure, the solvent was evaporated and the obtained residue was purified by silica gel column chromatography [elution solvent: n-hexane/ethyl acetate=80/20-60/40(V/V)] to give the title compound (52.0 mg, yield: 87%) as an oil.

### (21c) Ethyl 3-(7-{[(2R)-2-ethyl-7-hydroxy-2,3-dihydropyrido[2,3-f] [1,4]oxazepin-4(5H)-yl]methyl}-1-benzothiophen-5-yl)-3-(7-methoxy-1,4-dimethyl-1H-benzotriazol-5-yl)propanoate

To a solution of ethyl 3-(7-formyl-1-benzothiophen-5-yl)-3-(7-methoxy-1,4-dimethyl-1H-benzotriazol-5-yl)propanoate (52.0 mg) of Example 21b, (2R)-2-ethyl-2,3,4,5-tetrahydropyrido[2,3-f][1,4]oxazepin-7-ol hydrochloride (41.1 mg) of Example 1h, N,N-diisopropylethylamine (0.0311 mL) and acetic acid (0.0612 mL) in dichloromethane (2.0 mL) was added sodium triacetoxyborohydride (94.5 mg) at 0°C, and the mixture was stirred at room temperature for 14 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/100/0-0/90/10(V/V/V)] to give the title compound (61.0 mg, yield: 83%) as a solid.

### (21d)=(21)

### (3R)-3-(7-{[(2R)-2-Ethyl-7-hydroxy-2,3-dihydropyrido[2,3-f] [1,4]oxazepin-4(5H)-yl]methyl}-1-benzothiophen-5-yl)-3-(7-methoxy-1,4-dimethyl-1H-benzotriazol-5-yl)propanoic acid

### (22a)=(22)

### (3S)-3-(7-{[(2R)-2-Ethyl-7-hydroxy-2,3-dihydropyrido[2,3-f] [1,4]oxazepin-4(5H)-yl]methyl}-1-benzothiophen-5-yl)-3-(7-methoxy-1,4-dimethyl-1H-benzotriazol-5-yl)propanoic acid

To a mixture of ethyl 3-(7-{[(2R)-2-ethyl-7-hydroxy-2,3-dihydropyrido[2,3-f] [1,4]oxazepin-4(5H)-yl]methyl}-1-benzothiophen-5-yl)-3-(7-methoxy-1,4-dimethyl-1H-benzotriazol-5-yl)propanoate (61.0 mg) of Example 21c, methanol (1.0 mL), and tetrahydrofuran (1.0 mL) was added 1 M aqueous sodium hydroxide solution (0.495 mL) and the mixture was stirred at room temperature for 24 hr. To the reaction mixture was added 1 M hydrochloric acid (0.495 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the solvent was evaporated under reduced pressure and the obtained residue was purified by silica gel column chromatography [elution solvent: n-hexane/ethyl acetate/methanol=30/70/0-0/100/0-0/80/20(V/V/V)]. Ethyl acetate and n-hexane were added to the compound eluted earlier and the mixture was subjected to an ultrasonic treatment. The solid was collected by filtration and vacuum dried to give (3R)-3-(7-{[(2R)-2-ethyl-7-hydroxy-2,3-dihydropyrido[2,3-f] [1,4]oxazepin-4(5H)-yl]methyl}-1-benzothiophen-5-yl)-3-(7-methoxy-1,4-dimethyl-1H-benzotriazol-5-yl)propanoic acid (26.0 mg, yield: 45%) as a solid.

In the same manner, moreover, the compound eluted later, (3S)-3-(7-{[(2R)-2-ethyl-7-hydroxy-2,3-dihydropyrido[2,3-f] [1,4]oxazepin-4(5H)-yl]methyl}-1-benzothiophen-5-yl)-3-(7-methoxy-1,4-dimethyl-1H-benzotriazol-5-yl)propanoic acid (24.0 mg, yield: 41%) was obtained as a solid.

### (Example 23)

### (3R)-3-(3,7-Dimethyl-3H-[1,2,3]triazolo[4,5-b]pyridin-6-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid

### (23a) Ethyl (3R)-3-(3,7-dimethyl-3H-[1,2,3]triazolo[4,5-b]pyridin-6-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate

To a solution of ethyl 3-(3,7-dimethyl-3H-[1,2,3]triazolo[4,5-b]pyridin-6-yl)-3-(7-formyl-1-benzothiophen-5-yl)propanoate (81.0 mg) of Example 9b, (2R)-2-ethyl-2,3,4,5-tetrahydropyrido[2,3-f][1,4]oxazepin-7-ol hydrochloride (68.6 mg) of Example 1h, N,N-diisopropylethylamine (0.0518 mL) and acetic acid (0.102 mL) in dichloromethane (2.0 mL) was added sodium triacetoxyborohydride (158 mg) at 0°C and the mixture was stirred at room temperature for 22 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/100/0-0/95/5(V/V/V)]. As the compound eluted earlier, ethyl (3R)-3-(3,7-dimethyl-3H-[1,2,3]triazolo[4,5-b]pyridin-6-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (47.0 mg, yield: 40%) was obtained as an oil.

### (23b)=(23)

### (3R)-3-(3,7-dimethyl-3H-[1,2,3]triazolo[4,5-b]pyridin-6-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

To a mixture of ethyl (3R)-3-(3,7-dimethyl-3H-[1,2,3]triazolo[4,5-b]pyridin-6-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoate (47.0 mg) of Example 23a, methanol (1.0 mL), and tetrahydrofuran (1.0 mL) was added 1 M aqueous sodium hydroxide solution (0.401 mL) and the mixture was stirred at room temperature for 18 hr. To the reaction mixture was added 1 M hydrochloric acid (0.401 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the solvent was evaporated under reduced pressure and the obtained residue was purified by silica gel column chromatography [elution solvent: n-hexane/ethyl acetate/dichloromethane/methanol=30/70/0/0-0/100/0/0-0/0/100/0-0/0/90/10(V/V/V/V)]. Ethyl acetate and n-hexane were added to the obtained solid, and an ultrasonic treatment was performed. The solid was collected by filtration and vacuum dried to give the title compound (38.0 mg, yield: 85%) as a solid.

### (Example 24)

### (3S)-3-(3,7-Dimethyl-3H-[1,2,3]triazolo[4,5-b]pyridin-6-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (24a) Ethyl (3S)-3-(3,7-dimethyl-3H-[1,2,3]triazolo[4,5-b]pyridin-6-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate

To a solution of ethyl 3-(3,7-dimethyl-3H-[1,2,3]triazolo[4,5-b]pyridin-6-yl)-3-(7-formyl-1-benzothiophen-5-yl)propanoate (81.0 mg) of Example 9b, (2R)-2-ethyl-2,3,4,5-tetrahydropyrido[2,3-f][1,4]oxazepin-7-ol hydrochloride (68.6 mg) of Example 1h, N,N-diisopropylethylamine (0.0518 mL) and acetic acid (0.102 mL) in dichloromethane (2.0 mL) was added sodium triacetoxyborohydride (158 mg) at 0°C, and the mixture was stirred at room temperature for 22 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/100/0-0/95/5(V/V/V)]. As the compound eluted later, ethyl (3S)-3-(3,7-dimethyl-3H-[1,2,3]triazolo[4,5-b]pyridin-6-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoate (45.0 mg, yield: 39%) was obtained as a solid.

### (24b)=(24)

### (3S)-3-(3,7-Dimethyl-3H-[1,2,3]triazolo[4,5-b]pyridin-6-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

To a mixture of ethyl (3S)-3-(3,7-dimethyl-3H-[1,2,3]triazolo[4,5-b]pyridin-6-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoate (45.0 mg) of Example 24a, methanol (1.0 mL), and tetrahydrofuran (1.0 mL) was added 1 M aqueous sodium hydroxide solution (0.383 mL), and the mixture was stirred at room temperature for 18 hr. To the reaction mixture was added 1 M hydrochloric acid (0.383 mL), and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/dichloromethane/methanol=30/70/0/0-0/100/0/0-0/0/100/0-0/0/90/10(V/V/V/V)]. Ethyl acetate and n-hexane were added to the obtained solid, and an ultrasonic treatment was performed. The solid was collected by filtration and vacuum dried to give the title compound (38.0 mg, yield: 89%) as a solid.

### (Example 25)

### (3R)-3-{1-[2-(Dimethylamino)ethyl]-4-methyl-1H-benzotriazol-5-yl}-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid

### (25a) N,N-Dimethyl-N'-(3-methyl-2-nitrophenyl)ethane-1,2-diamine

To a solution of 1-fluoro-3-methyl-2-nitrobenzene (1.00 g), potassium carbonate (2.70 g) in ethanol (13 mL) was added N,N-dimethylethylenediamine (3.51 mL), and the mixture was stirred at 70°C for 24 hr. The reaction mixture was cooled to room temperature and concentrated under reduced pressure. Saturated aqueous sodium hydrogen carbonate solution was added to the residue, and the mixture was extracted with ethyl acetate. The organic layer was dried over anhydrous sodium sulfate. After filtration, the filtrate was concentrated under reduced pressure, and the obtained residue was purified by NH silica gel column chromatography [elution solvent: n-hexane/ethyl acetate=95/5-80/20(V/V)] to give the title compound (1.30 g, yield: 90%) as an oil.

### (25b) N'-(4-Bromo-3-methyl-2-nitrophenyl)-N,N-dimethylethane-1,2-diamine

To a solution of N,N-dimethyl-N'-(3-methyl-2-nitrophenyl)ethane-1,2-diamine (1.28 g) of Example 25a in N,N-dimethylformamide (5.7 mL) was added N-bromosuccinimide (1.04 g) at 0°C, and the mixture was stirred at room temperature for 24 hr. To the reaction mixture was added saturated aqueous sodium hydrogen carbonate solution, and the mixture was extracted with ethyl acetate. The organic layer was washed with 10% brine, and dried over anhydrous sodium sulfate. After filtration, the filtrate was concentrated under reduced pressure, and the obtained residue was purified by NH silica gel column chromatography [elution solvent: n-hexane/ethyl acetate=90/10-70/30(V/V)] to give the title compound (983 mg, yield: 57%) as a solid.

### (25c) 4-Bromo-N¹-[2-(dimethylamino)ethyl]-3-methylbenzene-1,2-diamine

Using N'-(4-bromo-3-methyl-2-nitrophenyl)-N,N-dimethylethane-1,2-diamine (983 mg) of Example 25b, and by a method similar to that of the below-mentioned Example 41e, the title compound (509 mg, yield: 58%) was obtained as a solid.

### (25d) 2-(5-Bromo-4-methyl-1H-benzotriazol-1-yl)-N,N-dimethylethanamine

Using 4-bromo-N¹-[2-(dimethylamino)ethyl]-3-methylbenzene-1,2-diamine (506 mg) of Example 25c, and by a method similar to that of the below-mentioned Example 41f, the title compound (416 mg, yield: 79%) was obtained as an oil.

### (25e) Ethyl (2E)-3-{1-[2-(dimethylamino)ethyl]-4-methyl-1H-benzotriazol-5-yl}prop-2-enoate

Using 2-(5-bromo-4-methyl-1H-benzotriazol-1-yl)-N,N-dimethylethanamine (403 mg) of Example 25d, and by a method similar to that of below-mentioned Example 41g, the title compound (329 mg, yield: 77%) was obtained as a solid.

### (25f) Ethyl 3-{1-[2-(dimethylamino)ethyl]-4-methyl-1H-benzotriazol-5-yl}-3-[7-(hydroxymethyl)-1-benzothiophen-5-yl]propanoate

Using ethyl (2E)-3-{1-[2-(dimethylamino)ethyl]-4-methyl-1H-benzotriazol-5-yl}prop-2-enoate (151 mg) of Example 25e and [5-(4,4,5,5-tetramethyl-1,3,2-dioxaborolan-2-yl)-1-benzothiophen-7-yl]methanol (290 mg) of Example 1e, and by a method similar to that of Example 1f, the title compound (96.0 mg, yield: 41%) was obtained as an oil.

### (25g) Ethyl 3-{1-[2-(dimethylamino)ethyl]-4-methyl-1H-benzotriazol-5-yl}-3-(7-formyl-1-benzothiophen-5-yl)propanoate

To a solution of ethyl 3-{1-[2-(dimethylamino)ethyl]-4-methyl-1H-benzotriazol-5-yl}-3-[7-(hydroxymethyl)-1-benzothiophen-5-yl]propanoate (104 mg) of Example 25f in dichloromethane (2.0 mL) was added manganese(IV) oxide (228 mg), and the mixture was stirred at 40°C for 6 hr. The reaction mixture was cooled to room temperature, filtered through celite, and insoluble material was removed. Under reduced pressure, the solvent was evaporated and the obtained residue was purified by silica gel column chromatography [elution solvent: n-hexane/ethyl acetate/methanol=20/80/0-0/100/0-0/90/10(V/V/V)] to give the title compound (88.0 mg, yield: 85%) as an oil.

### (25h) Ethyl 3-{1-[2-(dimethylamino)ethyl]-4-methyl-1H-benzotriazol-5-yl}-3-(7-{[(2R)-2-ethyl-7-hydroxy-2,3-dihydropyrido[2,3-f] [1,4]oxazepin-4(5H)-yl]methyl}-1-benzothiophen-5-yl)propanoate

To a solution of ethyl 3-{1-[2-(dimethylamino)ethyl]-4-methyl-1H-benzotriazol-5-yl}-3-(7-formyl-1-benzothiophen-5-yl)propanoate (88.0 mg) of Example 25g, (2R)-2-ethyl-2,3,4,5-tetrahydropyrido[2,3-f][1,4]oxazepin-7-ol hydrochloride (65.5 mg) of Example 1h, N,N-diisopropylethylamine (0.0495 mL) and acetic acid (0.0976 mL) in dichloromethane (2.0 mL) was added sodium triacetoxyborohydride (151 mg) at 0°C, and the mixture was stirred at room temperature for 14 hr. To the reaction mixture was added saturated aqueous sodium hydrogen carbonate solution, and the mixture was extracted with ethyl acetate. The organic layer was washed with saturated brine and dried over anhydrous sodium sulfate. The solvent was evaporated under reduced pressure and the obtained residue was purified by NH silica gel column chromatography [elution solvent: n-hexane/ethyl acetate/methanol=50/50/0-0/100/0-0/90/10(V/V/V)] to give the title compound (98.0 mg, yield: 81%) as an oil.

### (25i) Ethyl (3R)-3-{1-[2-(dimethylamino)ethyl]-4-methyl-1H-benzotriazol-5-yl}-3-(7-{[(2R)-2-ethyl-7-hydroxy-2,3-dihydropyrido[2,3-f][1,4]oxazepin-4(5H)-yl]methyl}-1-benzothiophen-5-yl)propanoate

Ethyl 3-{1-[2-(dimethylamino)ethyl]-4-methyl-1H-benzotriazol-5-yl}-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (98 mg) of Example 25h was subjected to chiral HPLC [column: CHIRALCEL OZ-H (20 mm I.D. × 250 mm), mobile phase: n-hexane/ethanol=40/60(V/V)] to give 48 mg of ethyl (3R)-3-{1-[2-(dimethylamino)ethyl]-4-methyl-1H-benzotriazol-5-yl}-3-(7-{[(2R)-2-ethyl-7-hydroxy-2,3-dihydropyrido[2,3-f] [1,4]oxazepin-4(5H)-yl]methyl}-1-benzothiophen-5-yl)propanoate as the second peak (yield: 49%). retention time: 13.1100 min [column: CHIRALCEL OZ-H (4.6 mm I.D. × 250 mm), mobile phase: n-hexane/ethanol=40/60(V/V), flow rate: 1.0 mL/min, temperature: 40°C, wavelength: 230 nm]

### (25j)=(25)

### (3R)-3-{1-[2-(Dimethylamino)ethyl]-4-methyl-1H-benzotriazol-5-yl}-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid

To a mixture of ethyl (3R)-3-{1-[2-(dimethylamino)ethyl]-4-methyl-1H-benzotriazol-5-yl}-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (48.0 mg) of Example 25i, methanol (1.0 mL), and tetrahydrofuran (1.0 mL) was added 1 M aqueous sodium hydroxide solution (0.373 mL), and the mixture was stirred at room temperature for 18 hr. To the reaction mixture was added 1 M hydrochloric acid (0.373 mL), and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the solvent was evaporated under reduced pressure and the obtained residue was purified by silica gel column chromatography [elution solvent: n-hexane/ethyl acetate/dichloromethane/methanol=30/70/0/0-0/100/0/0-0/0/100/0-0/0/80/20(V/V/V/V)]. Ethyl acetate and n-hexane were added to the obtained solid, and an ultrasonic treatment was performed. The solid was collected by filtration and vacuum dried to give the title compound (34.0 mg, yield: 74%) as a solid.

### (Example 26)

### (3S)-3-{1-[2-(Dimethylamino)ethyl]-4-methyl-1H-benzotriazol-5-yl}-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid

### (26a) Ethyl (3S)-3-{1-[2-(dimethylamino)ethyl]-4-methyl-1H-benzotriazol-5-yl}-3-(7-{[(2R)-2-ethyl-7-hydroxy-2,3-dihydropyrido[2,3-f][1,4]oxazepin-4(5H)-yl]methyl}-1-benzothiophen-5-yl)propanoate

Ethyl 3-{1-[2-(dimethylamino)ethyl]-4-methyl-1H-benzotriazol-5-yl}-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (98 mg) of Example 25h was subjected to chiral HPLC [column: CHIRALCEL OZ-H (20 mm I.D. × 250 mm), mobile phase: n-hexane/ethanol=40/60(V/V)] to give 48 mg of ethyl (3S)-3-{1-[2-(dimethylamino)ethyl]-4-methyl-1H-benzotriazol-5-yl}-3-(7-{[(2R)-2-ethyl-7-hydroxy-2,3-dihydropyrido[2,3-f] [1,4]oxazepin-4(5H)-yl]methyl}-1-benzothiophen-5-yl)propanoate as the first peak (yield: 49%). retention time: 10.5967 min [column: CHIRALCEL OZ-H (4.6 mm I.D. × 250 mm), mobile phase: n-hexane/ethanol=40/60(V/V), flow rate: 1.0 mL/min, temperature: 40°C, wavelength: 230 nm]

### (26b)=(26)

### (3S)-3-{1-[2-(Dimethylamino)ethyl]-4-methyl-1H-benzotriazol-5-yl}-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

To a mixture of ethyl (3S)-3-{1-[2-(dimethylamino)ethyl]-4-methyl-1H-benzotriazol-5-yl}-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (48.0 mg) of Example 26a, methanol (1.0 mL), and tetrahydrofuran (1.0 mL) was added 1 M aqueous sodium hydroxide solution (0.373 mL), and the mixture was stirred at room temperature for 18 hr. To the reaction mixture was added 1 M hydrochloric acid (0.373 mL), and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ ethyl acetate/ dichloromethane/ methanol=30/70/0/0-0/100/0/0-0/0/100/0-0/0/60/40(V/V/V/V)]. Ethyl acetate and n-hexane were added to the obtained solid, and an ultrasonic treatment was performed. The solid was collected by filtration and vacuum dried to give the title compound (34.0 mg, yield: 74%) as a solid.

### (Example 27)

### (3R)-3-(7-{[(2R)-2-Ethyl-7-hydroxy-2,3-dihydropyrido[2,3-f] [1,4]oxazepin-4(5H)-yl]methyl}-1-benzothiophen-5-yl)-3-[4-methyl-1-(2,2,2-trifluoroethyl)-1H-benzotriazol-5-yl]propanoic acid

### (Example 28)

### (3S)-3-(7-{[(2R)-2-Ethyl-7-hydroxy-2,3-dihydropyrido[2,3-f] [1,4]oxazepin-4(5H)-yl]methyl}-1-benzothiophen-5-yl)-3-[4-methyl-1-(2,2,2-trifluoroethyl)-1H-benzotriazol-5-yl]propanoic acid

### (27a) N-(4-Bromo-3-methyl-2-nitrophenyl)-2,2,2-trifluoroacetamide

To a solution of 4-bromo-3-methyl-2-nitroaniline (1.00 g), triethylamine (0.660 mL) in dichloromethane (22 mL) was added trifluoroacetic anhydride (0.662 mL), and the mixture was stirred at room temperature for 24 hr. To the reaction mixture was added saturated aqueous sodium hydrogen carbonate solution, and the mixture was extracted with ethyl acetate. The organic layer was washed with saturated brine and dried over anhydrous sodium sulfate. After filtration, the filtrate was concentrated under reduced pressure, and the obtained residue was purified by silica gel column chromatography [elution solvent: n-hexane/ethyl acetate=95/5-90/10(V/V)] to give the title compound (1.02 g, yield: 72%) as a solid.

### (27b) 4-Bromo-3-methyl-2-nitro-N-(2,2,2-trifluoroethyl)aniline

To a solution of N-(4-bromo-3-methyl-2-nitrophenyl)-2,2,2-trifluoroacetamide (960 mg) of Example 27a in tetrahydrofuran (29 mL) was added borane-tetrahydrofuran (0.9 M tetrahydrofuran solution) (22.6 mL) at 0°C, and the mixture was stirred at room temperature for 24 hr. The reaction mixture was cooled to 0°C, methanol was added, and the mixture was stirred with heating at 60°C for 2 hr. The reaction mixture was cooled to room temperature, saturated aqueous sodium hydrogen carbonate solution was added, and the mixture was extracted with ethyl acetate. The organic layer was washed with saturated brine and dried over anhydrous sodium sulfate. After filtration, the filtrate was concentrated under reduced pressure, and the obtained residue was purified by silica gel column chromatography [elution solvent: n-hexane/ethyl acetate=95/5-80/20(V/V)] to give the title compound (89.0 mg, yield: 10%) as a solid.

In addition, 4-bromo-3-methyl-N¹-(2,2,2-trifluoroethyl)benzene-1,2-diamine (306 mg, yield: 37%) of Example 27c was also obtained as a solid.

### (27c) 4-Bromo-3-methyl-N¹-(2,2,2-trifluoroethyl)benzene-1,2-diamine

Using 4-bromo-3-methyl-2-nitro-N-(2,2,2-trifluoroethyl)aniline (96.0 mg) of Example 27b, and by a method similar to that of the below-mentioned Example 41e, the title compound (64 mg, yield: 74%) was obtained as a solid.

### (27d) 5-Bromo-4-methyl-1-(2,2,2-trifluoroethyl)-1H-benzotriazole

Using 4-bromo-3-methyl-N¹-(2,2,2-trifluoroethyl)benzene-1,2-diamine (392 mg) of Example 27c, and by a method similar to that of the below-mentioned Example 41f, the title compound (204 mg, yield: 50%) was obtained as a solid.

### (27e) Ethyl (2E)-3-[4-methyl-1-(2,2,2-trifluoroethyl)-1H-benzotriazol-5-yl]prop-2-enoate

Using 5-bromo-4-methyl-1-(2,2,2-trifluoroethyl)-1H-benzotriazole (204 mg) of Example 27d, and by a method similar to that of the below-mentioned Example 41g, the title compound (199 mg, yield: 92%) was obtained as a solid.

### (27f) Ethyl 3-[7-(hydroxymethyl)-1-benzothiophen-5-yl]-3-[4-methyl-1-(2,2,2-trifluoroethyl)-1H-benzotriazol-5-yl]propanoate

Using ethyl (2E)-3-[4-methyl-1-(2,2,2-trifluoroethyl)-1H-benzotriazol-5-yl]prop-2-enoate (195 mg) of Example 27e and [5-(4,4,5,5-tetramethyl-1,3,2-dioxaborolan-2-yl)-1-benzothiophen-7-yl]methanol (271 mg) of Example 1e, and by a method similar to that of Example 1f, the title compound (296 mg, yield: 85%) was obtained as an oil.

### (27g) Ethyl 3-(7-formyl-1-benzothiophen-5-yl)-3-[4-methyl-1-(2,2,2-trifluoroethyl)-1H-benzotriazol-5-yl]propanoate

To a solution of ethyl 3-[7-(hydroxymethyl)-1-benzothiophen-5-yl]-3-[4-methyl-1-(2,2,2-trifluoroethyl)-1H-benzotriazol-5-yl]propanoate (180 mg) of Example 27f in dichloromethane (3.2 mL) was added manganese(IV) oxide (328 mg), and the mixture was stirred at 40°C for 6 hr. The reaction mixture was cooled to room temperature and filtered through celite, and insoluble material was removed. Under reduced pressure, the solvent was evaporated and the obtained residue was purified by silica gel column chromatography [elution solvent: n-hexane/ethyl acetate=80/20-60/40(V/V)] to give the title compound (152 mg, yield: 100%) as an oil.

### (27h) Ethyl 3-(7-{[(2R)-2-ethyl-7-hydroxy-2,3-dihydropyrido[2,3-f] [1,4]oxazepin-4(5H)-yl]methyl}-1-benzothiophen-5-yl)-3-[4-methyl-1-(2,2,2-trifluoroethyl)-1H-benzotriazol-5-yl]propanoate

To a solution of ethyl 3-(7-formyl-1-benzothiophen-5-yl)-3-[4-methyl-1-(2,2,2-trifluoroethyl)-1H-benzotriazol-5-yl]propanoate (93.0 mg) of Example 27g, (2R)-2-ethyl-2,3,4,5-tetrahydropyrido[2,3-f][1,4]oxazepin-7-ol hydrochloride (67.7 mg) of Example 1h and N,N-diisopropylethylamine (0.0511 mL) in dichloromethane (2.0 mL) was added acetic acid (0.101 mL), and the mixture was stirred at room temperature for 4 hr. Then, sodium triacetoxyborohydride (155 mg) was added thereto at 0°C and the mixture was stirred at room temperature for 12 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V)] to give the title compound (93.0 mg, yield: 73%) as an oil.

### (27i)=(27)

### (3R)-3-(7-{[(2R)-2-Ethyl-7-hydroxy-2,3-dihydropyrido[2,3-f][1,4]oxazepin-4(5H)-yl]methyl}-1-benzothiophen-5-yl)-3-[4-methyl-1-(2,2,2-trifluoroethyl)-1H-benzotriazol-5-yl]propanoic acid

### (28a)=(28)

### (3S)-3-(7-{[(2R)-2-Ethyl-7-hydroxy-2,3-dihydropyrido[2,3-f][1,4]oxazepin-4(5H)-yl]methyl}-1-benzothiophen-5-yl)-3-[4-methyl-1-(2,2,2-trifluoroethyl)-1H-benzotriazol-5-yl]propanoic acid

To a mixture of ethyl 3-(7-{[(2R)-2-ethyl-7-hydroxy-2,3-dihydropyrido[2,3-f][1,4]oxazepin-4(5H)-yl]methyl}-1-benzothiophen-5-yl)-3-[4-methyl-1-(2,2,2-trifluoroethyl)-1H-benzotriazol-5-yl]propanoate (93.0 mg) of Example 27h, methanol (1.0 mL), and tetrahydrofuran (1.0 mL) was added 1 M aqueous sodium hydroxide solution (0.711 mL), and the mixture was stirred at room temperature for 24 hr. To the reaction mixture was added 1 M hydrochloric acid (0.711 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/methanol=30/70/0-0/100/0-0/80/20(V/V/V)]. Ethyl acetate and n-hexane were added to the compound eluted earlier, and the mixture was subjected to an ultrasonic treatment. The solid was collected by filtration and vacuum dried to give (3R)-3-(7-{[(2R)-2-ethyl-7-hydroxy-2,3-dihydropyrido[2,3-f][1,4]oxazepin-4(5H)-yl]methyl}-1-benzothiophen-5-yl)-3-[4-methyl-1-(2,2,2-trifluoroethyl)-1H-benzotriazol-5-yl]propanoic acid (42.0 mg, yield: 47%) as a solid.

In the same manner, moreover, the compound eluted later, (3S)-3-(7-{[(2R)-2-ethyl-7-hydroxy-2,3-dihydropyrido[2,3-f][1,4]oxazepin-4(5H)-yl]methyl}-1-benzothiophen-5-yl)-3-[4-methyl-1-(2,2,2-trifluoroethyl)-1H-benzotriazol-5-yl]propanoic acid (43.0 mg, yield: 48%) was obtained as a solid.

### (Example 29)

### (3R)-3-[1,4-Dimethyl-7-(trifluoromethyl)-1H-benzotriazol-5-yl]-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (Example 30)

### (3S)-3-[1,4-Dimethyl-7-(trifluoromethyl)-1H-benzotriazol-5-yl]-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (29a) N-[4-Bromo-5-methyl-2-(trifluoromethyl)phenyl]formamide

Acetic anhydride (5.11 mL) and formic acid (5.10 mL) were mixed and stirred with heating at 60°C for 2 hr, and cooled to room temperature. The mixture was added to a solution of 4-bromo-5-methyl-2-(trifluoromethyl)aniline (WO 2015/180685) (2.29 g) in dichloromethane (45 mL), which had been cooled to 0°C, and the mixture was stirred at room temperature for 6 hr. To the reaction mixture was added saturated aqueous sodium hydrogen carbonate solution, and the mixture was extracted with ethyl acetate. The organic layer was washed with saturated brine and dried over anhydrous sodium sulfate. After filtration, the filtrate was concentrated under reduced pressure, and the obtained residue was purified by silica gel column chromatography [elution solvent: n-hexane/ethyl acetate=95/5-80/20(V/V)] to give the title compound (2.36 g, yield: 93%) as a solid.

### (29b) N-[4-Bromo-3-methyl-2-nitro-6-(trifluoromethyl)phenyl]formamide

To a mixture of N-[4-bromo-5-methyl-2-(trifluoromethyl)phenyl]formamide (2.79 g) of Example 29a and concentrated sulfuric acid (9.9 mL) was added 69% nitric acid (1.19 mL) at 0°C, and the mixture was stirred at 0°C for 3 hr. To the reaction mixture was added ice water, and the mixture was extracted with ethyl acetate. The organic layer was washed with saturated brine and dried over anhydrous sodium sulfate. After filtration, the filtrate was concentrated under reduced pressure, and the obtained residue was purified by silica gel column chromatography [elution solvent: n-hexane/ethyl acetate=95/5-80/20(V/V)] to give the title compound (2.70 g, yield: 84%) as a solid.

### (29c) 4-Bromo-N,3-dimethyl-2-nitro-6-(trifluoromethyl)aniline

Using N-[4-bromo-3-methyl-2-nitro-6-(trifluoromethyl)phenyl]formamide (2.70 g) of Example 29b, and by a method similar to that of the below-mentioned Example 41d, the title compound (1.55 g, yield: 60%) was obtained as a solid.

### (29d) 4-Bromo-N¹,3-dimethyl-6-(trifluoromethyl)benzene-1,2-diamine

Using 4-bromo-N,3-dimethyl-2-nitro-6-(trifluoromethyl)aniline (1.55 g) of Example 29c, and by a method similar to that of the below-mentioned Example 41e, the title compound (1.07 g, yield: 76%) was obtained as an oil.

### (29e) 5-Bromo-1,4-dimethyl-7-(trifluoromethyl)-1H-benzotriazole

Using 4-bromo-N¹,3-dimethyl-6-(trifluoromethyl)benzene-1,2-diamine (1.07 g) of Example 29d, and by a method similar to that of the below-mentioned Example 41f, the title compound (933 mg, yield: 84%) was obtained as a solid.

### (29f) Ethyl (2E)-3-[1,4-dimethyl-7-(trifluoromethyl)-1H-benzotriazol-5-yl]prop-2-enoate

Using 5-bromo-1,4-dimethyl-7-(trifluoromethyl)-1H-benzotriazole (933 mg) of Example 29e, and by a method similar to that of the below-mentioned Example 41g, the title compound (912 mg, yield: 92%) was obtained as a solid.

### (29g) Ethyl 3-[1,4-dimethyl-7-(trifluoromethyl)-1H-benzotriazol-5-yl]-3-[7-(hydroxymethyl)-1-benzothiophen-5-yl]propanoate

Using ethyl (2E)-3-[1,4-dimethyl-7-(trifluoromethyl)-1H-benzotriazol-5-yl]prop-2-enoate (313 mg) of Example 29f and [5-(4,4,5,5-tetramethyl-1,3,2-dioxaborolan-2-yl)-1-benzothiophen-7-yl]methanol (435 mg) of Example 1e, and by a method similar to that of Example 1f, the title compound (408 mg, yield: 86%) was obtained as an oil.

### (29h) Ethyl 3-[1,4-dimethyl-7-(trifluoromethyl)-1H-benzotriazol-5-yl]-3-(7-formyl-1-benzothiophen-5-yl)propanoate

To a solution of ethyl 3-[1,4-dimethyl-7-(trifluoromethyl)-1H-benzotriazol-5-yl]-3-[7-(hydroxymethyl)-1-benzothiophen-5-yl]propanoate (158 mg) of Example 29g in dichloromethane (3.3 mL) was added manganese(IV) oxide (338 mg), and the mixture was stirred at 40°C for 8 hr. The reaction mixture was cooled to room temperature, filtered through celite, and insoluble material was removed. Under reduced pressure, the solvent was evaporated and the obtained residue was purified by silica gel column chromatography [elution solvent: n-hexane/ethyl acetate=90/10-60/40(V/V)] to give the title compound (123 mg, yield: 78%) as an oil.

### (29i) Ethyl 3-[1,4-dimethyl-7-(trifluoromethyl)-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoate

To a solution of ethyl 3-[1,4-dimethyl-7-(trifluoromethyl)-1H-benzotriazol-5-yl]-3-(7-formyl-1-benzothiophen-5-yl)propanoate (89.0 mg) of Example 29h, (2R)-2-ethyl-2,3,4,5-tetrahydropyrido[2,3-f][1,4]oxazepin-7-ol hydrochloride (64.8 mg) of Example 1h and N,N-diisopropylethylamine (0.0489 mL) in dichloromethane (2.0 mL) was added acetic acid (0.0964 mL), and the mixture was stirred at room temperature for 4 hr. Then, sodium triacetoxyborohydride (149 mg) was added thereto at 0°C and the mixture was stirred at room temperature for 12 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V)] to give the title compound (91.0 mg, yield: 74%) as an oil.

### (29j) = (29)

### (3R)-3-[1,4-Dimethyl-7-(trifluoromethyl)-1H-benzotriazol-5-yl]-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (30a) = (30)

### (3S)-3-[1,4-Dimethyl-7-(trifluoromethyl)-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid

To a mixture of ethyl 3-[1,4-dimethyl-7-(trifluoromethyl)-1H-benzotriazol-5-yl]-3-(7-{[(2R)-2-ethyl-7-hydroxy-2,3-dihydropyrido[2,3-f][1,4]oxazepin-4(5H)-yl]methyl}-1-benzothiophen-5-yl)propanoate (91.0 mg) of Example 29i, methanol (1.0 mL), and tetrahydrofuran (1.0 mL) was added 1 M aqueous sodium hydroxide solution (0.696 mL) and the mixture was stirred at room temperature for 18 hr. To the reaction mixture was added 1 M hydrochloric acid (0.696 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/methanol=30/70/0-0/100/0-0/80/20(V/V/V)]. Ethyl acetate and n-hexane were added to the compound eluted earlier and the mixture was subjected to an ultrasonic treatment. The solid was collected by filtration and vacuum dried to give (3R)-3-[1,4-dimethyl-7-(trifluoromethyl)-1H-benzotriazol-5-yl]-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid (33.0 mg, yield: 38%) as a solid. In the same manner, moreover, the compound eluted later, (3S)-3-[1,4-dimethyl-7-(trifluoromethyl)-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid (36.0 mg, yield: 41%), was obtained as a solid.

### (Example 31)

### (3R)-3-(7-Chloro-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (Example 32)

### (3S)-3-(7-Chloro-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (31a) 4-Bromo-6-chloro-N,3-dimethyl-2-nitroaniline

4-Bromo-N,3-dimethyl-2-nitroaniline (WO 2020/165776) (5.04 g) was dissolved in N,N-dimethylformamide, N-chlorosuccinimide (2.88 g) was added, and the mixture was stirred at 80°C for 2 hr after being purged with nitrogen and cooled to room temperature. The reaction mixture was diluted with ethyl acetate, successively washed three times with water and once with saturated brine, and dried over anhydrous sodium sulfate. After filtration, the solvent was evaporated under reduced pressure to give an unpurified title compound (5.72 g, yield: quantitative) as a solid.

### (31b) 4-Bromo-6-chloro-N¹,3-dimethylbenzene-1,2-diamine

Using 4-bromo-6-chloro-N,3-dimethyl-2-nitroaniline (5.84 g) of Example 31a, and by a method similar to that of the below-mentioned Example 41e, the title compound (2.69 g, yield: 52%) was obtained as an oil.

### (31c) 5-Bromo-7-chloro-1,4-dimethyl-1H-benzotriazole

4-Bromo-6-chloro-N¹,3-dimethylbenzene-1,2-diamine (2.69 g) of Example 31b was suspended in 5 M hydrochloric acid (50 mL), and a solution of sodium nitrite (1.49 g) in water (6 mL) was added dropwise over 20 min at 0°C under a nitrogen atmosphere. After stirring at room temperature for 1.5 hr, the reaction mixture was cooled to 0°C and neutralized by adding 5 M aqueous sodium hydroxide solution (50 mL) dropwise. The reaction mixture was extracted with dichloromethane, and the organic layer was washed with saturated saline and dried over anhydrous sodium sulfate. After filtration, the filtrate was concentrated under reduced pressure to give an unpurified title compound (2.76 g, yield: 98%) as a solid.

### (31d) Ethyl (2E)-3-(7-chloro-1,4-dimethyl-1H-benzotriazol-5-yl)prop-2-enoate

A mixture of 5-bromo-7-chloro-1,4-dimethyl-1H-benzotriazole (2.76 g) of Example 31c, palladium(II) acetate (238 mg), tri(o-tolyl)phosphine (645 mg), N,N-dimethylformamide (20 mL) was purged with nitrogen, and N,N-diisopropylethylamine (9.1 mL) and then ethyl acrylate (2.3 mL) were added, and the mixture was stirred with heating at 90°C for 2 hr. To the reaction mixture were added tris(dibenzylideneacetone)dipalladium(0) (485 mg), tri(o-tolyl)phosphine (645 mg), and ethyl acrylate (2.3 mL), and the mixture was stirred with heating at 90°C for 3 hr. The reaction mixture was cooled to room temperature, and ethyl acetate and saturated aqueous sodium hydrogen carbonate solution were added and stirred, and insoluble material was filtered through a cotton plug. The organic layer of the filtrate was separated by a partitioning operation, washed twice with water and once with saturated saline, and then dried over anhydrous sodium sulfate. After filtration, the filtrate was concentrated under reduced pressure, and the obtained residue was purified by silica gel column chromatography [elution solvent: n-hexane/dichloromethane/ethyl acetate=90/0/10-75/0/25-0/100/0-0/90/10(V/V/V)]. The obtained solid was slurry washed with n-hexane/ethyl acetate to give the title compound (2.23 g, yield: 75%) as a solid.

### (31e) Ethyl 3-(7-chloro-1,4-dimethyl-1H-benzotriazol-5-yl)-3-[7-(hydroxymethyl)-1-benzothiophen-5-yl]propanoate

Ethyl (2E)-3-(7-chloro-1,4-dimethyl-1H-benzotriazol-5-yl)prop-2-enoate (2.00 g) of Example 31d, and [5-(4,4,5,5-tetramethyl-1,3,2-dioxaborolan-2-yl)-1-benzothiophen-7-yl]methanol (2.41 g) of Example 1e were suspended in a mixed solvent of 1,4-dioxane (48 mL) and water (8.4 mL), and purged with nitrogen. Hydroxy(1,5-cyclooctadiene)rhodium(I) dimer (293 mg) was added, then 1 M aqueous potassium hydroxide solution (3.57 mL) was added thereto, and the mixture was stirred with heating at 50°C for 2 hr. The reaction mixture was cooled to room temperature, water was added, and the mixture was extracted three times with dichloromethane. The combined organic layer was washed with saturated brine and dried over anhydrous sodium sulfate. After filtration, the filtrate was concentrated under reduced pressure and the obtained residue was purified by silica gel column chromatography [elution solvent: n-hexane/ethyl acetate=4/1-1/1(V/V)] to give the title compound (1.61 g, yield: 51%) as a solid.

### (31f) Ethyl 3-(7-chloro-1,4-dimethyl-1H-benzotriazol-5-yl)-3-(7-formyl-1-benzothiophen-5-yl)propanoate

To a solution of ethyl 3-(7-chloro-1,4-dimethyl-1H-benzotriazol-5-yl)-3-[7-(hydroxymethyl)-1-benzothiophen-5-yl]propanoate (147 mg) of Example 31e in dichloromethane (3.3 mL) was added manganese(IV) oxide (339 mg), and the mixture was stirred at 40°C for 12 hr. The reaction mixture was cooled to room temperature, filtered through celite, and insoluble material was removed. Under reduced pressure, the solvent was evaporated and the obtained residue was purified by silica gel column chromatography [elution solvent: n-hexane/ethyl acetate=90/10-70/30(V/V)] to give the title compound (100 mg, yield: 68%) as an oil.

### (31g) Ethyl 3-(7-chloro-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoate

To a solution of ethyl 3-(7-chloro-1,4-dimethyl-1H-benzotriazol-5-yl)-3-(7-formyl-1-benzothiophen-5-yl)propanoate (100 mg) of Example 31f, (2R)-2-ethyl-2,3,4,5-tetrahydropyrido[2,3-f][1,4]oxazepin-7-ol hydrochloride (78.3 mg) of Example 1h and N,N-diisopropylethylamine (0.0591 mL) in dichloromethane (2.0 mL) was added acetic acid (0.117 mL) and the mixture was stirred at room temperature for 4 hr. Then, sodium triacetoxyborohydride (300 mg) was added thereto at 0°C and the mixture was stirred at room temperature for 20 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V)] to give the title compound (100 mg, yield: 71%) as an oil.

### (31h)=(31)

### (3R)-3-(7-Chloro-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (32a)=(32)

### (3S)-3-(7-Chloro-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

To a mixture of ethyl 3-(7-chloro-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (100 mg) of Example 31g, methanol (1.0 mL), and tetrahydrofuran (1.0 mL) was added 1 M aqueous sodium hydroxide solution (0.806 mL), and the mixture was stirred at room temperature for 12 hr. To the reaction mixture was added 1 M hydrochloric acid (0.806 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/methanol=30/70/0-0/100/0-0/70/30(V/V/V)]. Ethyl acetate and n-hexane were added to the compound eluted earlier and the mixture was subjected to an ultrasonic treatment. The solid was collected by filtration and vacuum dried to give (3R)-3-(7-chloro-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid (43.0 mg, yield: 45%) as a solid.

In the same manner, moreover, the compound eluted later, (3S)-3-(7-chloro-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid (34.0 mg, yield: 36%), was obtained as a solid.

### (Example 33)

### (3R)-3-(7-Cyano-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (Example 34)

### (3S)-3-(7-Cyano-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (33a) Ethyl (2E)-3-(7-cyano-1,4-dimethyl-1H-benzotriazol-5-yl)prop-2-enoate

Ethyl (2E)-3-(7-chloro-1,4-dimethyl-1H-benzotriazol-5-yl)prop-2-enoate (226 mg) of Example 31d, potassium hexacyanoferrate(II) trihydrate (341 mg), potassium acetate (20.4 mg), and tBuXPhos (CAS Registry number: 564483-19-8) (35.4 mg) were dissolved in a mixed solvent of dioxane (2.0 mL) and water (2.0 mL), tBuXPhos Pd G1 (CAS Registry number: 1142811-12-8) (2.3 mg) was added, and the mixture was stirred with heating under a nitrogen atmosphere at 90°C for 8 hr. The reaction mixture was cooled to room temperature, and concentrated under reduced pressure. Saturated aqueous sodium hydrogen carbonate solution was added, and the mixture was extracted with ethyl acetate. The organic layer was washed with saturated brine and dried over anhydrous sodium sulfate. After filtration, the filtrate was concentrated under reduced pressure, and the obtained residue was purified by silica gel column chromatography [elution solvent: n-hexane/ethyl acetate=95/5-80/20(V/V)] to give the title compound (92.0 mg, yield: 42%) as a solid.

### (33b) Ethyl 3-(7-cyano-1,4-dimethyl-1H-benzotriazol-5-yl)-3-[7-(hydroxymethyl)-1-benzothiophen-5-yl]propanoate

Using ethyl (2E)-3-(7-cyano-1,4-dimethyl-1H-benzotriazol-5-yl)prop-2-enoate (100 mg) of Example 33a and [5-(4,4,5,5-tetramethyl-1,3,2-dioxaborolan-2-yl)-1-benzothiophen-7-yl]methanol (215 mg) of Example 1e, and by a method similar to that of Example 1f, the title compound (109 mg, yield: 68%) was obtained as an oil.

### (33c) Ethyl 3-(7-cyano-1,4-dimethyl-1H-benzotriazol-5-yl)-3-(7-formyl-1-benzothiophen-5-yl)propanoate

To a solution of ethyl 3-(7-cyano-1,4-dimethyl-1H-benzotriazol-5-yl)-3-[7-(hydroxymethyl)-1-benzothiophen-5-yl]propanoate (161 mg) of Example 33b in dichloromethane (3.7 mL) was added manganese(IV) oxide (378 mg), and the mixture was stirred at 40°C for 10 hr. The reaction mixture was cooled to room temperature and filtered through celite, and insoluble material was removed. Under reduced pressure, the solvent was evaporated and the obtained residue was purified by silica gel column chromatography [elution solvent: n-hexane/ethyl acetate=90/10-70/30(V/V)] to give the title compound (110 mg, yield: 69%) as an oil.

### (33d) Ethyl 3-(7-cyano-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoate

To a solution of ethyl 3-(7-cyano-1,4-dimethyl-1H-benzotriazol-5-yl)-3-(7-formyl-1-benzothiophen-5-yl)propanoate (110 mg) of Example 33c, (2R)-2-ethyl-2,3,4,5-tetrahydropyrido[2,3-f][1,4]oxazepin-7-ol hydrochloride (88.0 mg) of Example 1h and N,N-diisopropylethylamine (0.0665 mL) in dichloromethane (2.0 mL) was added acetic acid (0.131 mL), and the mixture was stirred at room temperature for 4 hr. Then, sodium triacetoxyborohydride (337 mg) was added thereto at 0°C and the mixture was stirred at room temperature for 14 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/100/0-0/95/5(V/V/V)] to give the title compound (112 mg, yield: 72%) as an oil.

### (33e)=(33)

### (3R)-3-(7-Cyano-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (34a)=(34)

### (3S)-3-(7-Cyano-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

To a mixture of ethyl 3-(7-cyano-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (112 mg) of Example 33d, methanol (1.0 mL), and tetrahydrofuran (1.0 mL) was added 1 M aqueous sodium hydroxide solution (0.917 mL) and the mixture was stirred at room temperature for 18 hr. To the reaction mixture was added 1 M hydrochloric acid (0.917 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/methanol=30/70/0-0/100/0-0/90/10(V/V/V)]. Ethyl acetate and n-hexane were added to the compound eluted earlier and the mixture was subjected to an ultrasonic treatment. The solid was collected by filtration and vacuum dried to give (3R)-3-(7-cyano-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid (50.0 mg, yield: 47%) as a solid.

In the same manner, moreover, the compound eluted later, (3S)-3-(7-cyano-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid (49.0 mg, yield: 46%), was obtained as a solid.

### (Example 35)

### (3R)-3-(7-Cyclopropyl-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (35a) Ethyl (2E)-3-(7-cyclopropyl-1,4-dimethyl-1H-benzotriazol-5-yl)prop-2-enoate

Ethyl (2E)-3-(7-chloro-1,4-dimethyl-1H-benzotriazol-5-yl)prop-2-enoate (27.9 mg) of Example 31d, potassium cyclopropyltrifluoroborate (17.7 mg), potassium carbonate (41.4 mg), and XPhos (9.8 mg) were dissolved in a mixed solvent of cyclopentylmethylether (30 mL) and water (0.20 mL), palladium(II) acetate (2.3 mg) was added, and the mixture was stirred with heating under a nitrogen atmosphere at 100°C for 4 hr. The reaction mixture was cooled to room temperature, saturated aqueous sodium hydrogen carbonate solution was added, and the mixture was extracted with ethyl acetate. The organic layer was washed with saturated brine and dried over anhydrous sodium sulfate. After filtration, the filtrate was concentrated under reduced pressure, and the obtained residue was purified by silica gel column chromatography [elution solvent: n-hexane/ethyl acetate=90/10-70/30(V/V)] to give the title compound (28.0 mg, yield: 98%) as a solid.

### (35b) Ethyl 3-(7-cyclopropyl-1,4-dimethyl-1H-benzotriazol-5-yl)-3-[7-(hydroxymethyl)-1-benzothiophen-5-yl]propanoate

Using ethyl (2E)-3-(7-cyclopropyl-1,4-dimethyl-1H-benzotriazol-5-yl)prop-2-enoate (152 mg) of Example 35a and [5-(4,4,5,5-tetramethyl-1,3,2-dioxaborolan-2-yl)-1-benzothiophen-7-yl]methanol (232 mg) of Example 1e, and by a method similar to that of Example 1f, the title compound (182 mg, yield: 76%) was obtained as an oil.

### (35c) Ethyl 3-(7-cyclopropyl-1,4-dimethyl-1H-benzotriazol-5-yl)-3-(7-formyl-1-benzothiophen-5-yl)propanoate

To a solution of ethyl 3-(7-cyclopropyl-1,4-dimethyl-1H-benzotriazol-5-yl)-3-[7-(hydroxymethyl)-1-benzothiophen-5-yl]propanoate (140 mg) of Example 35b in dichloromethane (3.1 mL) was added manganese(IV) oxide (319 mg), and the mixture was stirred at 40°C for 6 hr. The reaction mixture was cooled to room temperature, the mixture was filtered through celite and insoluble material was removed. Under reduced pressure, the solvent was evaporated and the obtained residue was purified by silica gel column chromatography [elution solvent: n-hexane/ethyl acetate=70/30-50/50(V/V)] to give the title compound (119 mg, yield: 85%) as an oil.

### (35d) Ethyl 3-(7-cyclopropyl-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoate

To a solution of ethyl 3-(7-cyclopropyl-1,4-dimethyl-1H-benzotriazol-5-yl)-3-(7-formyl-1-benzothiophen-5-yl)propanoate (88.0 mg) of Example 35c, (2R)-2-ethyl-2,3,4,5-tetrahydropyrido[2,3-f][1,4]oxazepin-7-ol hydrochloride (68.0 mg) of Example 1h and N,N-diisopropylethylamine (0.0514 mL) in dichloromethane (2.0 mL) was added acetic acid (0.101 mL), and the mixture was stirred at room temperature for 4 hr. Then, sodium triacetoxyborohydride (260 mg) was added thereto at 0°C and the mixture was stirred at room temperature for 14 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/100/0-0/95/5(V/V/V)] to give the title compound (83.0 mg, yield: 68%) as an oil.

### (35e) 3-(7-Cyclopropyl-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

To a mixture of ethyl 3-(7-cyclopropyl-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (83.0 mg) of Example 35d, methanol (1.0 mL), and tetrahydrofuran (1.0 mL) was added 1 M aqueous sodium hydroxide solution (0.663 mL), and the mixture was stirred at room temperature for 18 hr. To the reaction mixture was added 1 M hydrochloric acid (0.663 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/methanol=30/70/0-0/100/0-0/90/10(V/V/V)] to give the title compound (74.0 mg, yield: 93%) as a solid.

### (35f) Methyl 3-(7-cyclopropyl-1,4-dimethyl-1H-benzotriazol-5-yl)-3-(7-{[(2R)-2-ethyl-7-methoxy-2,3-dihydropyrido[2,3-f] [1,4]oxazepin-4(5H)-yl]methyl}-1-benzothiophen-5-yl)propanoate

To a mixture of 3-(7-cyclopropyl-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid (65.0 mg) of Example 35e, methanol (1.0 mL), and tetrahydrofuran (1.0 mL) was added trimethylsilyl diazomethane (0.60 M n-hexane solution) (0.906 mL), and the mixture was stirred at room temperature for 12 hr. The reaction mixture was concentrated under reduced pressure and the obtained residue was purified by silica gel column chromatography [elution solvent: n-hexane/ethyl acetate/methanol=50/50/0-0/100/0-0/95/5(V/V/V)] to give the title compound (24.0 mg, yield: 35%) as an oil.

### (35g) Methyl 3-(7-cyclopropyl-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate

To a solution of methyl 3-(7-cyclopropyl-1,4-dimethyl-1H-benzotriazol-5-yl)-3-(7-{[(2R)-2-ethyl-7-methoxy-2,3-dihydropyrido[2,3-f] [1,4]oxazepin-4(5H)-yl]methyl}-1-benzothiophen-5-yl)propanoate (24.0 mg) of Example 35f and sodium iodide (57.5 mg) in acetonitrile (2.0 mL) was added chlorotrimethylsilane (0.0145 mL), and the mixture was stirred at room temperature for 16 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=20/80/0-0/100/0-0/90/10(V/V/V)] to give the title compound (23.0 mg, yield: 98%) as an oil.

### (35h) Methyl (3R)-3-(7-cyclopropyl-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate

Methyl 3-(7-cyclopropyl-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (25 mg) of Example 35g was subjected to chiral HPLC [column: CHIRALPAK IG (20 mm I.D. × 250 mm), mobile phase: n-hexane/ethanol=50/50(V/V)] to give 10 mg of methyl (3R)-3-(7-cyclopropyl-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoate as the second peak (yield: 40%).
retention time: 11.3433 min [column: CHIRALPAK IG (4.6 mm I.D. × 250 mm), mobile phase: n-hexane/ethanol=50/50(V/V), flow rate: 1.0 mL/min, temperature: 40°C, wavelength: 230 nm]

### (35i)=(35)

### (3R)-3-(7-Cyclopropyl-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

To a mixture of methyl (3R)-3-(7-cyclopropyl-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (10.0 mg) of Example 35h, methanol (1.0 mL), and tetrahydrofuran (1.0 mL) was added 1 M aqueous sodium hydroxide solution (0.327 mL), and the mixture was stirred at room temperature for 18 hr. To the reaction mixture was added 1 M hydrochloric acid (0.327 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the solvent was evaporated under reduced pressure and the obtained residue was purified by silica gel column chromatography [elution solvent: n-hexane/ethyl acetate/dichloromethane/methanol=30/70/0/0-0/100/0/0-0/0/100/0-0/0/90/10(V/V/V/V)]. Ethyl acetate and n-hexane were added to the obtained solid, and an ultrasonic treatment was performed. The solid was collected by filtration and vacuum dried to give the title compound (7.0 mg, yield: 72%) as a solid.

### (Example 36)

### (3S)-3-(7-Cyclopropyl-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (36a) Methyl (3S)-3-(7-cyclopropyl-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate

Methyl 3-(7-cyclopropyl-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (25 mg) of Example 35g was subjected to chiral HPLC [column: CHIRALPAK IG (20 mm I.D. × 250 mm), mobile phase: n-hexane/ethanol=50/50(V/V)] to give 11 mg of methyl (3S)-3-(7-cyclopropyl-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoate as the first peak (yield: 44%).
retention time: 10.5100 min [column: CHIRALPAK IG (4.6 mm I.D. × 250 mm), mobile phase: n-hexane/ethanol=50/50(V/V), flow rate: 1.0 mL/min, temperature: 40°C, wavelength: 230 nm]

### (36b)=(36)

### (3S)-3-(7-Cyclopropyl-1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

To a mixture of methyl (3S)-3-(7-cyclopropyl-1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (11.0 mg) of Example 36a, methanol (1.0 mL), and tetrahydrofuran (1.0 mL) was added 1 M aqueous sodium hydroxide solution (0.360 mL), and the mixture was stirred at room temperature for 18 hr. To the reaction mixture was added 1 M hydrochloric acid (0.360 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/dichloromethane/methanol=30/70/0/0-0/100/0/0-0/0/100/0-0/0/90/10(V/V/V/V)]. Ethyl acetate and n-hexane were added to the solid, and an ultrasonic treatment was performed. The solid was collected by filtration and vacuum dried to give the title compound (10.0 mg, yield: 93%) as a solid.

### (Example 37)

### (3R)-3-(1,4-Dimethyl-1H-benzotriazol-5-yl)-3-(7-{ [(2S)-7-hydroxy-2-(trifluoromethyl)-2,3-dihydropyrido[2,3-f][1,4]oxazepin-4(5H)-yl]methyl}-1-benzothiophen-5-yl)propanoic acid

### (Example 38)

### (3S)-3-(1,4-Dimethyl-1H-benzotriazol-5-yl)-3-(7-{[(2S)-7-hydroxy-2-(trifluoromethyl)-2,3-dihydropyrido[2,3-f][1,4]oxazepin-4(5H)-yl]methyl}-1-benzothiophen-5-yl)propanoic acid

### (37a) 3-{[(6-Chloro-3-fluoropyridin-2-yl)methyl]amino}-1,1,1-trifluoropropan-2-ol

To a solution of 3-amino-1,1,1-trifluoropropan-2-ol (1.56 g) and 6-chloro-3-fluoropyridine-2-carbaldehyde (2.98 g) in tetrahydrofuran (50 mL) was added sodium triacetoxyborohydride (7.87 g), and the mixture was stirred at room temperature for 1 hr. To the reaction mixture was added saturated aqueous sodium hydrogen carbonate solution, and the mixture was extracted with ethyl acetate. The organic layer was washed with saturated brine and dried over anhydrous sodium sulfate. The solvent was evaporated under reduced pressure, and the obtained residue was washed with diethyl ether to give the title compound (2.14 g, yield: 66%) as a solid. Diethyl ether used for washing was evaporated under reduced pressure, and the obtained residue was purified by silica gel column chromatography [elution solvent: n-hexane/ethyl acetate=80/20-50/50(V/V)] to give the title compound (361 mg, yield: 11%) as a solid.

### (37b) tert-Butyl 7-chloro-2-(trifluoromethyl)-2,3-dihydropyrido[2,3-f][1,4]oxazepine-4(5H)-carboxylate

To a solution of 3-{[(6-chloro-3-fluoropyridin-2-yl)methyl]amino}-1,1,1-trifluoropropan-2-ol (356 mg) of Example 37a in dimethyl sulfoxide (1.5 mL) and tetrahydrofuran (1.5 mL) was added potassium tert-butoxide (230 mg), and the mixture was stirred at room temperature for 17 hr. Potassium tert-butoxide (246 mg) was added thereto, and the mixture was stirred at room temperature for 3 hr. Triethylamine (272 µL) and di-tert-butyl dicarbonate (373 mg) were added thereto, and the mixture was stirred at room temperature for 19 hr. The reaction mixture was diluted with ethyl acetate, washed successively with water and saturated brine, and dried over anhydrous sodium sulfate. Under reduced pressure, the solvent was evaporated and the obtained residue was purified by silica gel column chromatography [elution solvent: n-hexane/ethyl acetate=100/0-90/10(V/V)] to give the title compound (80.6 mg, yield: 18%) as an oil.

### (37c) tert-Butyl (2S)-7-hydroxy-2-(trifluoromethyl)-2,3-dihydropyrido[2,3-f][1,4]oxazepine-4(5H)-carboxylate

To a solution of tert-butyl 7-chloro-2-(trifluoromethyl)-2,3-dihydropyrido[2,3-f][1,4]oxazepine-4(5H)-carboxylate (947 mg) of Example 37b in 1,4-dioxane (15 mL) were added 5-(di-tert-butylphosphino)-1',3',5'-triphenyl-1'H-1,4'-bipyrazole (281 mg), tris(dibenzylideneacetone)dipalladium(0) (245 mg), and cesium hydroxide monohydrate (1.60 g), and the mixture was stirred at 90°C for 3 hr. To the reaction mixture was added saturated aqueous ammonium chloride solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-20/80(V/V)]. This was further subjected to chiral HPLC [column: CHIRALPAK IG (20 mm I.D. × 250 mm), mobile phase: n-hexane/ethanol=90/10(V/V)] to give the title compound (165 mg, yield: 18%) as a solid as the second peak.
retention time: 11.980 min [column: CHIRALPAK IG (4.6 mm I.D. × 250 mm), mobile phase: n-hexane/ethanol=90/10(V/V), flow rate: 1.0 mL/min, temperature: 40°C, wavelength: 280 nm]

### (37d) (2S)-2-(Trifluoromethyl)-2,3,4,5-tetrahydropyrido[2,3-f][1,4]oxazepin-7-ol hydrochloride

To a solution of tert-butyl (2S)-7-hydroxy-2-(trifluoromethyl)-2,3-dihydropyrido[2,3-f] [1,4]oxazepine-4(5H)-carboxylate (165 mg) of Example 37c in 1,4-dioxane (3 mL) was added 4 M hydrogen chloride-1,4-dioxane solution (3 mL), and the mixture was stirred at room temperature for 4 hr. The solvent was evaporated under reduced pressure to give the title compound (134 mg, yield: quantitative) as a solid.

### (37e) Ethyl 3-(1,4-dimethyl-1H-benzotriazol-5-yl)-3-(7-{[(2S)-7-hydroxy-2-(trifluoromethyl)-2,3-dihydropyrido[2,3-f] [1,4]oxazepin-4(5H)-yl]methyl}-1-benzothiophen-5-yl)propanoate

To a solution of ethyl 3-(1,4-dimethyl-1H-benzotriazol-5-yl)-3-[7-(hydroxymethyl)-1-benzothiophen-5-yl]propanoate (73.7 mg) of Example 1f in dichloromethane (3.0 mL) was added thionyl chloride (0.0219 mL), the mixture was stirred at room temperature for 20 min, and the solvent was evaporated under reduced pressure. The obtained residue was dissolved in acetonitrile (2.0 mL), added to a separately-prepared solution of N,N-diisopropylethylamine (0.259 mL) and (2S)-2-(trifluoromethyl)-2,3,4,5-tetrahydropyrido[2,3-f][1,4]oxazepin-7-ol hydrochloride (40.6 mg) of Example 37d in acetonitrile (3.0 mL), and the mixture was stirred at 60°C for 70 hr and cooled to room temperature. Under reduced pressure, the solvent was evaporated and the obtained residue was purified by silica gel column chromatography [elution solvent: n-hexane/ethyl acetate=80/20-0/100(V/V)] to give the title compound (39.0 mg, yield: 42%) as an oil.

### (37f)=(37)

### (3R)-3-(1,4-Dimethyl-1H-benzotriazol-5-yl)-3-(7-{ [(2S)-7-hydroxy-2-(trifluoromethyl)-2,3-dihydropyrido[2,3-f] [1,4]oxazepin-4(5H)-yl]methyl}-1-benzothiophen-5-yl)propanoic acid

### (38a)=(38)

### (3S)-3-(1,4-Dimethyl-1H-benzotriazol-5-yl)-3-(7-{[(2S)-7-hydroxy-2-(trifluoromethyl)-2,3-dihydropyrido[2,3-f] [1,4]oxazepin-4(5H)-yl]methyl}-1-benzothiophen-5-yl)propanoic acid

To a mixture of ethyl 3-(1,4-dimethyl-1H-benzotriazol-5-yl)-3-(7-{[(2S)-7-hydroxy-2-(trifluoromethyl)-2,3-dihydropyrido[2,3-f] [1,4]oxazepin-4(5H)-yl]methyl}-1-benzothiophen-5-yl)propanoate (39.0 mg) of Example 37e, ethanol (4.0 mL), and water (1.0 mL) was added lithium hydroxide monohydrate (7.9 mg), and the mixture was stirred at 50°C for 30 hr. To the reaction mixture was added 1 M hydrochloric acid (0.365 mL) and the mixture was stirred and the solvent was evaporated under reduced pressure. The obtained residue was purified by silica gel column chromatography [elution solvent: n-hexane/ethyl acetate/methanol=20/80/0-0/100/0-0/90/10(V/V/V)]. (3R)-3-(1,4-Dimethyl-1H-benzotriazol-5-yl)-3-(7-{[(2S)-7-hydroxy-2-(trifluoromethyl)-2,3-dihydropyrido[2,3-f] [1,4]oxazepin-4(5H)-yl]methyl}-1-benzothiophen-5-yl)propanoic acid (2.5 mg, yield: 7%) eluted earlier was obtained as a solid.

In addition, (3S)-3-(1,4-dimethyl-1H-benzotriazol-5-yl)-3-(7-{[(2S)-7-hydroxy-2-(trifluoromethyl)-2,3-dihydropyrido[2,3-f] [1,4]oxazepin-4(5H)-yl]methyl}-1-benzothiophen-5-yl)propanoic acid (3.3 mg, yield: 9%) eluted later was obtained as a solid.

### (Example 39)

### (3R)-3-(1,4-Dimethyl-1H-benzotriazol-5-yl)-3-(7-{[(2R)-7-hydroxy-2-(trifluoromethyl)-2,3-dihydropyrido[2,3-f] [1,4]oxazepin-4(5H)-yl]methyl}-1-benzothiophen-5-yl)propanoic acid

### (Example 40)

### (3S)-3-(1,4-Dimethyl-1H-benzotriazol-5-yl)-3-(7-{[(2R)-7-hydroxy-2-(trifluoromethyl)-2,3-dihydropyrido[2,3-f][1,4]oxazepin-4(5H)-yl]methyl}-1-benzothiophen-5-yl)propanoic acid

### (39a) tert-Butyl (2R)-7-hydroxy-2-(trifluoromethyl)-2,3-dihydropyrido[2,3-f][1,4]oxazepine-4(5H)-carboxylate

To a solution of tert-butyl 7-chloro-2-(trifluoromethyl)-2,3-dihydropyrido[2,3-f][1,4]oxazepine-4(5H)-carboxylate (947 mg) of Example 37b in 1,4-dioxane (15 mL) were added 5-(di-tert-butylphosphino)-1',3',5'-triphenyl-1'H-1,4'-bipyrazole (281 mg), tris(dibenzylideneacetone)dipalladium(0) (245 mg) and cesium hydroxide monohydrate (1.60 g), and the mixture was stirred at 90°C for 3 hr. To the reaction mixture was added saturated aqueous ammonium chloride solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-20/80(V/V)]. This was further subjected to chiral HPLC [column: CHIRALPAK IG (20 mm I.D. × 250 mm), mobile phase: n-hexane/ethanol=90/10(V/V)] to give the title compound (178 mg, yield: 20%) as a solid as the first peak.
retention time: 9.673 min [column: CHIRALPAK IG (4.6 mm I.D. × 250 mm), mobile phase: n-hexane/ethanol=90/10(V/V), flow rate: 1.0 mL/min, temperature: 40°C, wavelength: 280 nm]

### (39b) (2R)-2-(Trifluoromethyl)-2,3,4,5-tetrahydropyrido[2,3-f][1,4]oxazepin-7-ol hydrochloride

To a solution of tert-butyl (2R)-7-hydroxy-2-(trifluoromethyl)-2,3-dihydropyrido[2,3-f] [1,4]oxazepine-4(5H)-carboxylate (178 mg) of Example 39a in 1,4-dioxane (3 mL) was added 4 M hydrogen chloride-1,4-dioxane solution (3 mL), and the mixture was stirred at room temperature for 4 hr. The reaction mixture was concentrated under reduced pressure to give the title compound (144 mg, yield: quantitative) as a solid.

### (39c) Ethyl 3-(1,4-dimethyl-1H-benzotriazol-5-yl)-3-(7-{[(2R)-7-hydroxy-2-(trifluoromethyl)-2,3-dihydropyrido[2,3-f] [1,4]oxazepin-4(5H)-yl]methyl}-1-benzothiophen-5-yl)propanoate

To a solution of ethyl 3-(1,4-dimethyl-1H-benzotriazol-5-yl)-3-[7-(hydroxymethyl)-1-benzothiophen-5-yl]propanoate (82.4 mg) of Example 1f in dichloromethane (3.0 mL) was added thionyl chloride (0.0238 mL), the mixture was stirred at room temperature for 20 min, and the solvent was evaporated under reduced pressure. The obtained residue was dissolved in acetonitrile (2.0 mL), added to a separately-prepared solution of N,N-diisopropylethylamine (0.282 mL), and (2R)-2-(trifluoromethyl)-2,3,4,5-tetrahydropyrido[2,3-f][1,4]oxazepin-7-ol hydrochloride (44.2 mg) of Example 39b in acetonitrile (3.0 mL), and the mixture was stirred at 60°C for 16 hr. To the reaction mixture was added acetonitrile (5.0 mL), and the mixture was stirred at 60°C for 70 hr and cooled to room temperature. Under reduced pressure, the solvent was evaporated and the obtained residue was purified by silica gel column chromatography [elution solvent: n-hexane/ethyl acetate=80/20-0/100(V/V)] to give the title compound (68.3 mg, yield: 67%) as an oil.

### (39d) = (39)

### (3R)-3-(1,4-Dimethyl-1H-benzotriazol-5-yl)-3-(7-{[(2R)-7-hydroxy-2-(trifluoromethyl)-2,3-dihydropyrido[2,3-f][1,4]oxazepin-4(5H)-yl]methyl}-1-benzothiophen-5-yl)propanoic acid

### (40a)=(40)

### (3S)-3-(1,4-Dimethyl-1H-benzotriazol-5-yl)-3-(7-{[(2R)-7-hydroxy-2-(trifluoromethyl)-2,3-dihydropyrido[2,3-f][1,4]oxazepin-4(5H)-yl]methyl}-1-benzothiophen-5-yl)propanoic acid

To a mixture of ethyl 3-(1,4-dimethyl-1H-benzotriazol-5-yl)-3-(7-{[(2R)-7-hydroxy-2-(trifluoromethyl)-2,3-dihydropyrido[2,3-f] [1,4]oxazepin-4(5H)-yl]methyl}-1-benzothiophen-5-yl)propanoate (68.3 mg) of Example 39c, ethanol (4.0 mL), and water (1.0 mL) was added lithium hydroxide monohydrate (15.3 mg), and the mixture was stirred at 50°C for 6 hr and at 60°C for 24 hr. To the reaction mixture was added 1 M hydrochloric acid (0.365 mL) and the mixture was stirred. The solvent was evaporated under reduced pressure and the obtained residue was purified by silica gel column chromatography [elution solvent: n-hexane/ethyl acetate/methanol=20/80/0-0/100/0-0/90/10(V/V/V)]. (3R)-3-(1,4-Dimethyl-1H-benzotriazol-5-yl)-3-(7-{[(2R)-7-hydroxy-2-(trifluoromethyl)-2,3-dihydropyrido[2,3-f][1,4]oxazepin-4(5H)-yl]methyl}-1-benzothiophen-5-yl)propanoic acid (2.4 mg, yield: 4%) eluted earlier was obtained as a solid. In addition, (3S)-3-(1,4-dimethyl-1H-benzotriazol-5-yl)-3-(7-{[(2R)-7-hydroxy-2-(trifluoromethyl)-2,3-dihydropyrido[2,3-f][1,4]oxazepin-4(5H)-yl]methyl}-1-benzothiophen-5-yl)propanoic acid (0.81 mg, yield: 1%) eluted later was obtained as a solid.

### (Example 41)

### (3R)-3-[1,4-Dimethyl-7-(trifluoromethoxy)-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid

### (Example 42)

### (3S)-3-[1,4-Dimethyl-7-(trifluoromethoxy)-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid

### (41a) 4-Bromo-5-methyl-2-(trifluoromethoxy)aniline

To a solution of 5-methyl-2-(trifluoromethoxy)aniline (1.91 g) in N,N-dimethylformamide (10 mL) was added N-bromosuccinimide (1.81 g) at 0°C, and the mixture was stirred at room temperature for 24 hr. To the reaction mixture was added saturated aqueous sodium hydrogen carbonate solution, and the mixture was extracted with ethyl acetate. The organic layer was washed with 10% brine, and dried over anhydrous sodium sulfate. After filtration, the filtrate was concentrated under reduced pressure, and the obtained residue was purified by silica gel column chromatography [elution solvent: n-hexane/ethyl acetate=100/0-90/10(V/V)] to give the title compound (2.61 g, yield: 97%) as an oil.

### (41b) N-[4-Bromo-5-methyl-2-(trifluoromethoxy)phenyl]formamide

Acetic anhydride (2.74 mL) and formic acid (2.73 mL) were mixed, stirred with heating at 60°C for 2 hr, and cooled to room temperature. This mixture was added to a solution of 4-bromo-5-methyl-2-(trifluoromethoxy)aniline (2.61 g) of Example 41a in dichloromethane (48 mL) cooled to 0°C, and the mixture was stirred at room temperature for 18 hr. To the reaction mixture was added saturated aqueous sodium hydrogen carbonate solution, and the mixture was extracted with ethyl acetate. The organic layer was washed with saturated brine and dried over anhydrous sodium sulfate. After filtration, the filtrate was concentrated under reduced pressure to give the title compound (crude product, 2.73 g, yield: 95%) as a solid.

### (41c) N-[4-Bromo-3-methyl-2-nitro-6-(trifluoromethoxy)phenyl]formamide

To a mixture of N-[4-bromo-5-methyl-2-(trifluoromethoxy)phenyl]formamide (2.66 g) of Example 41b and concentrated sulfuric acid (8.9 mL) was added 69% nitric acid (1.07 mL) at 0°C, and the mixture was stirred at 0°C for 2 hr. To the reaction mixture was added ice water, and the mixture was extracted with ethyl acetate. The organic layer was washed with saturated brine and dried over anhydrous sodium sulfate. After filtration, the filtrate was concentrated under reduced pressure to give the title compound (crude product, 2.97 g, yield: 97%) as a solid.

### (41d) 4-Bromo-N,3-dimethyl-2-nitro-6-(trifluoromethoxy)aniline

To a solution of N-[4-bromo-3-methyl-2-nitro-6-(trifluoromethoxy)phenyl]formamide (2.48 g) of Example 41c in tetrahydrofuran (36 mL) was added borane-tetrahydrofuran (0.9 M tetrahydrofuran solution) (23.8 mL) at 0°C, and the mixture was stirred at room temperature for 2 hr. The reaction mixture was cooled to 0°C, methanol was added, and the mixture was stirred with heating at 60°C for 2 hr. The reaction mixture was cooled to room temperature, saturated aqueous sodium hydrogen carbonate solution was added, and the mixture was extracted with ethyl acetate. The organic layer was washed with saturated brine and dried over anhydrous sodium sulfate. After filtration, the filtrate was concentrated under reduced pressure, and the obtained residue was purified by silica gel column chromatography [elution solvent: n-hexane/ethyl acetate=100/0-95/5(V/V)] to give the title compound (2.35 g, yield: 99%) as a solid.

### (41e) 4-Bromo-N¹,3-dimethyl-6-(trifluoromethoxy)benzene-1,2-diamine

4-Bromo-N,3-dimethyl-2-nitro-6-(trifluoromethoxy)aniline (2.35 g) of Example 41d and iron powder (1.99 g) were mixed with ethanol (14 mL) and water (7.1 mL), ammonium chloride (3.86 g) was added, and the mixture was stirred with heating at 80°C for 4 hr. The reaction mixture was cooled to room temperature, saturated aqueous sodium hydrogen carbonate solution and celite were added, and the mixture was stirred at 4 hr. The mixture was filtered through celite, insoluble material was removed, and the filtrate was extracted with ethyl acetate. The organic layer was washed with saturated brine and dried over anhydrous sodium sulfate. After filtration, the filtrate was concentrated under reduced pressure, and the obtained residue was purified by silica gel column chromatography [elution solvent: n-hexane/ethyl acetate=100/0-95/5(V/V)] to give the title compound (1.27 g, yield: 60%) as an oil.

### (41f) 5-Bromo-1,4-dimethyl-7-(trifluoromethoxy)-1H-benzotriazole

42% tetrafluoroboric acid (1.27 mL) and tert-butyl nitrite (0.755 mL) were dissolved in acetonitrile (8.5 mL) and cooled to 0°C. A solution of 4-bromo-N¹,3-dimethyl-6-(trifluoromethoxy)benzene-1,2-diamine (1.27 g) of Example 41e and 42% tetrafluoroboric acid (1.27 mL) dissolved in acetonitrile (8.5 mL) were added dropwise thereto over 20 min, and the mixture was stirred at 0°C for 2 hr and at room temperature for 2 hr. The reaction mixture was neutralized by adding 5 M aqueous sodium hydroxide solution and extracted with ethyl acetate. The organic layer was washed with saturated brine and dried over anhydrous sodium sulfate. After filtration, the filtrate was concentrated under reduced pressure, and the obtained residue was purified by silica gel column chromatography [elution solvent: n-hexane/ethyl acetate=95/5-10/90(V/V)] to give the title compound (10.9 g, yield: 83%) as a solid.

### (41g) Ethyl (2E)-3-[1,4-dimethyl-7-(trifluoromethoxy)-1H-benzotriazol-5-yl]prop-2-enoate

5-Bromo-1,4-dimethyl-7-(trifluoromethoxy)-1H-benzotriazole (310 mg) of Example 41f, ethyl acrylate (0.326 mL), and triethylamine (0.832 mL) were dissolved in N,N-dimethylformamide (2.0 mL), bis(tri-tert-butylphosphine)palladium(0) (25.5 mg) was added, and the mixture was stirred with heating under a nitrogen atmosphere at 90°C for 4 hr. The reaction mixture was cooled to room temperature, saturated aqueous sodium hydrogen carbonate solution was added, and the mixture was extracted with ethyl acetate. The organic layer was washed with saturated brine and dried over anhydrous sodium sulfate. After filtration, the filtrate was concentrated under reduced pressure, and the obtained residue was purified by silica gel column chromatography [elution solvent: n-hexane/ethyl acetate=100/0-90/10(V/V)] to give the title compound (313 mg, yield: 95%) as an oil.

### (41h) Ethyl 3-[1,4-dimethyl-7-(trifluoromethoxy)-1H-benzotriazol-5-yl]-3-[7-(hydroxymethyl)-1-benzothiophen-5-yl]propanoate

Bis(norbornadiene)rhodium(I) tetrafluoroborate (35.2 mg), (2S,3S)-bis(diphenylphosphino)butane (20.1 mg), (2R,3R)-bis(diphenylphosphino)butane (20.1 mg) were dissolved in a mixed solvent of 1,4-dioxane (7.5 mL) and water (1.9 mL) and stirred at room temperature for 15 min. Then, ethyl (2E)-3-[1,4-dimethyl-7-(trifluoromethoxy)-1H-benzotriazol-5-yl]prop-2-enoate (310 mg) of Example 41g, [5-(4,4,5,5-tetramethyl-1,3,2-dioxaborolan-2-yl)-1-benzothiophen-7-yl]methanol (410 mg) of Example 1e, 1 M aqueous potassium hydroxide solution (0.941 mL) were added thereto, and the mixture was stirred with heating at 50°C for 4 hr. The reaction mixture was cooled to room temperature, saturated aqueous ammonium chloride solution was added, and the mixture was extracted with ethyl acetate. The organic layer was washed with saturated brine and dried over anhydrous sodium sulfate. After filtration, the filtrate was concentrated under reduced pressure, and the obtained residue was purified by silica gel column chromatography [elution solvent: n-hexane/ethyl acetate=90/10-50/50(V/V)] to give the title compound (369 mg, yield: 79%) as an oil.

### (411) Ethyl 3-[1,4-dimethyl-7-(trifluoromethoxy)-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoate

To a solution of ethyl 3-[1,4-dimethyl-7-(trifluoromethoxy)-1H-benzotriazol-5-yl]-3-[7-(hydroxymethyl)-1-benzothiophen-5-yl]propanoate (85.0 mg) of Example 41h in dichloromethane (2.0 mL) was added thionyl chloride (0.0263 mL) and the mixture was stirred at room temperature for 1 hr. The solvent was evaporated under reduced pressure, and the residue was azeotropically distilled with toluene. The obtained residue was dissolved in acetonitrile (1.0 mL) and added to a separately-prepared solution of N,N-diisopropylethylamine (0.240 mL) and (2R)-2-ethyl-2,3,4,5-tetrahydropyrido[2,3-f][1,4]oxazepin-7-ol hydrochloride (39.7 mg) of Example 1h in acetonitrile (1.0 mL), and the mixture was stirred at 80°C for 8 hr and cooled to room temperature. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V), ethyl acetate/methanol=100/0-90/10(V/V)] to give the title compound (85.0 mg, yield: 74%) as an oil.

### (41j)=(41)

### (3R)-3-[1,4-Dimethyl-7-(trifluoromethoxy)-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid

### (42a)=(42)

### (3S)-3-[1,4-Dimethyl-7-(trifluoromethoxy)-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid

To a mixture of ethyl 3-[1,4-dimethyl-7-(trifluoromethoxy)-1H-benzotriazol-5-yl]-3-(7-{[(2R)-2-ethyl-7-hydroxy-2,3-dihydropyrido[2,3-f][1,4]oxazepin-4(5H)-yl]methyl}-1-benzothiophen-5-yl)propanoate (85.0 mg) of Example 41i, methanol (1.0 mL), and tetrahydrofuran (1.0 mL) was added 1 M aqueous sodium hydroxide solution (0.635 mL), and the mixture was stirred at room temperature for 18 hr. To the reaction mixture was added 1 M hydrochloric acid (0.635 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the solvent was evaporated under reduced pressure and the obtained residue was purified by silica gel column chromatography [elution solvent: n-hexane/ethyl acetate/methanol=30/70/0-0/100/0-0/80/20(V/V/V)]. Ethyl acetate and n-hexane were added to the compound eluted earlier and the mixture was subjected to an ultrasonic treatment. The solid was collected by filtration and vacuum dried to give (3R)-3-[1,4-dimethyl-7-(trifluoromethoxy)-1H-benzotriazol-5-yl]-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid (34.0 mg, yield: 42%) as a solid.

In the same manner, moreover, the compound eluted later, (3S)-3-[1,4-dimethyl-7-(trifluoromethoxy)-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid (33.0 mg, yield: 41%) was obtained as a solid.

### (Example 43)

### (3R)-3-[7-(Difluoromethoxy)-1,4-dimethyl-1H-benzotriazol-5-yl]-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (Example 44)

### (3S)-3-[7-(Difluoromethoxy)-1,4-dimethyl-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid

### (43a) 4-Bromo-2-(difluoromethoxy)-5-methylaniline

To a solution of 2-(difluoromethoxy)-5-methylaniline (1.73 g) in N,N-dimethylformamide (10 mL) was added N-bromosuccinimide (1.81 g) at 0°C, and the mixture was stirred at room temperature for 24 hr. To the reaction mixture was added saturated aqueous sodium hydrogen carbonate solution, and the mixture was extracted with ethyl acetate. The organic layer was washed with 10% brine, and dried over anhydrous sodium sulfate. After filtration, the filtrate was concentrated under reduced pressure, and the obtained residue was purified by silica gel column chromatography [elution solvent: n-hexane/ethyl acetate=95/5-80/20(V/V)] to give the title compound (2.30 g, yield: 91%) as an oil.

### (43b) N-[4-Bromo-2-(difluoromethoxy)-5-methylphenyl]formamide

Using 4-bromo-2-(difluoromethoxy)-5-methylaniline (2.30 g) of Example 43a, and by a method similar to that of Example 41b, the title compound (crude product, 2.37 g, yield: 93%) was obtained as a solid.

### (43c) N-[4-Bromo-6-(difluoromethoxy)-3-methyl-2-nitrophenyl]formamide

Using N-[4-bromo-2-(difluoromethoxy)-5-methylphenyl]formamide (2.31 g) of Example 43b, and by a method similar to that of Example 41c, the title compound (crude product, 2.42 g, yield: 97%) was obtained as a solid.

### (43d) 4-Bromo-6-(difluoromethoxy)-N,3-dimethyl-2-nitroaniline

Using N-[4-bromo-6-(difluoromethoxy)-3-methyl-2-nitrophenyl]formamide (2.48 g) of Example 43c, and by a method similar to that of Example 41d, the title compound (1.76 g, yield: 74%) was obtained as a solid.

### (43e) 4-Bromo-6-(difluoromethoxy)-N¹,3-dimethylbenzene-1,2-diamine

Using 4-bromo-6-(difluoromethoxy)-N,3-dimethyl-2-nitroaniline (1.76 g) of Example 43d, and by a method similar to that of Example 41e, the title compound (980 mg, yield: 62%) as an oil.

### (43f) 5-Bromo-7-(difluoromethoxy)-1,4-dimethyl-1H-benzotriazole

Using 4-bromo-6-(difluoromethoxy)-N¹,3-dimethylbenzene-1,2-diamine (980 mg) of Example 43e, and by a method similar to that of Example 41f, the title compound (845 mg, yield: 83%) was obtained as a solid.

### (43g) Ethyl (2E)-3-[7-(difluoromethoxy)-1,4-dimethyl-1H-benzotriazol-5-yl]prop-2-enoate

Using 5-bromo-7-(difluoromethoxy)-1,4-dimethyl-1H-benzotriazole (292 mg) of Example 43f, and by a method similar to that of Example 41g, the title compound (310 mg, yield: quantitative) was obtained as an oil.

### (43h) Ethyl 3-[7-(difluoromethoxy)-1,4-dimethyl-1H-benzotriazol-5-yl]-3-[7-(hydroxymethyl)-1-benzothiophen-5-yl]propanoate

Using ethyl (2E)-3-[7-(difluoromethoxy)-1,4-dimethyl-1H-benzotriazol-5-yl]prop-2-enoate (310 mg) of Example 43g, and [5-(4,4,5,5-tetramethyl-1,3,2-dioxaborolan-2-yl)-1-benzothiophen-7-yl]methanol (432 mg) of Example 1e, and by a method similar to that of Example 1f, the title compound (308 mg, yield: 65%) was obtained as an oil.

### (43i) Ethyl 3-[7-(difluoromethoxy)-1,4-dimethyl-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoate

To a solution of ethyl 3-[7-(difluoromethoxy)-1,4-dimethyl-1H-benzotriazol-5-yl]-3-[7-(hydroxymethyl)-1-benzothiophen-5-yl]propanoate (82.0 mg) of Example 43h in dichloromethane (2.0 mL) was added thionyl chloride (0.0263 mL) and the mixture was stirred at room temperature for 1 hr. The solvent was evaporated under reduced pressure, and the residue was azeotropically distilled with toluene. The obtained residue was dissolved in acetonitrile (1.0 mL), added to a separately-prepared solution of N,N-diisopropylethylamine (0.240 mL) and (2R)-2-ethyl-2,3,4,5-tetrahydropyrido[2,3-f][1,4]oxazepin-7-ol hydrochloride (39.8 mg) of Example 1h in acetonitrile (1.0 mL), and the mixture was stirred at 80°C for 8 hr and cooled to room temperature. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/100/0-0/90/10(V/V/V)] to give the title compound (100 mg, yield: 89%) as an oil.

### (43j) = (43)

### (3R)-3-[7-(Difluoromethoxy)-1,4-dimethyl-1H-benzotriazol-5-yl]-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (44a)=(44)

### (3S)-3-[7-(Difluoromethoxy)-1,4-dimethyl-1H-benzotriazol-5-yl]-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

To a mixture of ethyl 3-[7-(difluoromethoxy)-1,4-dimethyl-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (100 mg) of Example 43i, methanol (1.0 mL), and tetrahydrofuran (1.0 mL) was added 1 M aqueous sodium hydroxide solution (0.767 mL), and the mixture was stirred at room temperature for 18 hr. To the reaction mixture was added 1 M hydrochloric acid (0.767 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/methanol=30/70/0-0/100/0-0/80/20(V/V/V)]. Ethyl acetate and n-hexane were added to the compound eluted earlier and the mixture was subjected to an ultrasonic treatment. The solid was collected by filtration and vacuum dried to give (3R)-3-[7-(difluoromethoxy)-1,4-dimethyl-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid (39.0 mg, yield: 41%) as a solid.

In the same manner, moreover, the compound eluted later, (3S)-3-[7-(difluoromethoxy)-1,4-dimethyl-1H-benzotriazol-5-yl]-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid (39.0 mg, yield: 41%) was obtained as a solid.

### (Example 45)

### (3R)-3-(7-{[(2S)-2-(1,1-Difluoroethyl)-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

### (45a) 1-Amino-3,3-difluorobutan-2-ol

To a suspension of 10% palladium -activated carbon (436 mg) in a small amount of ethyl acetate were added a solution of 3,3-difluoro-1-nitrobutan-2-ol (WO 2013/078468) (2.18 g) in methanol (100 mL) and water (5 mL), and the mixture was stirred at room temperature under a hydrogen atmosphere at 0.4 MPa for 13 hr. To the reaction mixture was added celite, insoluble material was filtered off, washed with ethyl acetate and the obtained filtrate was concentrated under reduced pressure to give the title compound (1.68 g, yield: 95%) as an oil.

### (45b) 3,3-Difluoro-1-{[(3-fluoropyridin-2-yl)methyl]amino}butan-2-ol

To a solution of 1-amino-3,3-difluorobutan-2-ol (1.68 g) of Example 45a in dichloromethane (60 mL) were added 3-fluoropyridine-2-carbaldehyde (1.52 g) and anhydrous sodium sulfate (1.68 g), and the mixture was stirred at room temperature for 10 min. Then, sodium triacetoxyborohydride (3.88 g) was added thereto, and the mixture was stirred at room temperature for 1 hr. To the reaction mixture was added saturated aqueous sodium hydrogen carbonate solution, and the mixture was extracted with chloroform. The organic layer was dried over anhydrous sodium sulfate. The solvent was evaporated under reduced pressure and the obtained residue was purified by NH silica gel column chromatography [elution solvent: n-hexane/ethyl acetate=80/20-0/100(V/V)] to give the title compound (1.84 g, yield: 64%) as an oil.

### (45c) tert-Butyl 2-(1,1-difluoroethyl)-2,3-dihydropyrido[2,3-f][1,4]oxazepine-4(5H)-carboxylate

To a solution of 3,3-difluoro-1-{[(3-fluoropyridin-2-yl)methyl]amino}butan-2-ol (2.02 g) of Example 45b in dimethyl sulfoxide (35 mL) was added potassium tert-butoxide (1.93 g), and the mixture was stirred at 100°C for 15 min. The reaction mixture was cooled to room temperature, di-tert-butyl dicarbonate (2.38 mL) was added and the mixture was stirred at room temperature for 1 hr. To the reaction mixture was added water, and the mixture was extracted with ethyl acetate. The organic layer was washed successively with water and saturated brine, and dried over anhydrous sodium sulfate. Under reduced pressure, the solvent was evaporated and the obtained residue was purified by silica gel column chromatography [elution solvent: chloroform/methanol=99/1(V/V)] to give the title compound (1.29 g, yield: 48%) as an oil.

### (45d) tert-Butyl (2S)-2-(1,1-difluoroethyl)-2,3-dihydropyrido[2,3-f][1,4]oxazepine-4(5H)-carboxylate

tert-Butyl 2-(1,1-difluoroethyl)-2,3-dihydropyrido[2,3-f] [1,4]oxazepine-4(5H)-carboxylate (1.2 g) of Example 45c was subjected to chiral HPLC [column: CHIRALPAK IG (20 mm I.D. × 250 mm), mobile phase: n-hexane/ethanol=70/30(V/V)] to give 507 mg of tert-butyl (2S)-2-(1,1-difluoroethyl)-2,3-dihydropyrido[2,3-f][1,4]oxazepine-4(5H)-carboxylate as the second peak (yield: 50%).
retention time: 6.9867 min [column: CHIRALPAK IG (4.6 mm I.D. × 250 mm), mobile phase: n-hexane/ethanol=80/20(V/V), flow rate: 1.0 mL/min, temperature: 40°C, wavelength: 254 nm]

### (45e) (2S)-2-(1,1-Difluoroethyl)-2,3,4,5-tetrahydropyrido[2,3-f][1,4]oxazepine dihydrochloride

To a solution of tert-butyl (2S)-2-(1,1-difluoroethyl)-2,3-dihydropyrido[2,3-f][1,4]oxazepine-4(5H)-carboxylate (505 mg) of Example 45d in 1,4-dioxane (5 mL) was added 4M hydrogen chloride-1,4-dioxane solution (5 mL), and the mixture was stirred at room temperature for 2 hr. The solvent was evaporated under reduced pressure, to the obtained residue were added ethyl acetate and n-hexane, and an ultrasonic treatment was performed. The solid was collected by filtration and vacuum dried to give the title compound (427 mg, yield: 93%) as a solid.

### (45f) 1-[(2S)-2-(1,1-Difluoroethyl)-2,3-dihydropyrido[2,3-f][1,4]oxazepin-4(5H)-yl]-2,2,2-trifluoroethanone

To a solution of (2S)-2-(1,1-difluoroethyl)-2,3,4,5-tetrahydropyrido[2,3-f][1,4]oxazepine dihydrochloride (351 mg) of Example 45e in dichloromethane (10 mL) were added triethylamine (0.685 mL) and trifluoroacetic anhydride (0.260 mL), and the mixture was stirred at room temperature for 50 min. Trifluoroacetic anhydride (0.0867 mL) was added thereto, and the mixture was stirred at room temperature for 20 min. To the reaction mixture was added water, and the mixture was extracted with dichloromethane. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=70/30-50/50(V/V)] to give the title compound (341 mg, yield: 90%) as an oil.

### (45g) 1-[(2S)-2-(1,1-Difluoroethyl)-6-oxido-2,3-dihydropyrido[2,3-f][1,4]oxazepin-4(5H)-yl]-2,2,2-trifluoroethanone

To a solution of 1-[(2S)-2-(1,1-difluoroethyl)-2,3-dihydropyrido[2,3-f] [1,4]oxazepin-4(5H)-yl]-2,2,2-trifluoroethanone (341 mg) of Example 45f in dichloromethane (10 mL) was added metachloroperbenzoic acid (600 mg), and the mixture was stirred at room temperature for 2 hr. Under reduced pressure, the solvent was evaporated and the obtained residue was purified by silica gel column chromatography [elution solvent: ethyl acetate/methanol=100/0-95/5(V/V)] to give the title compound (366 mg, yield: quantitative) as a solid.

### (45h) 1-[(2S)-7-Chloro-2-(1,1-difluoroethyl)-2,3-dihydropyrido[2,3-f] [1,4]oxazepin-4(5H)-yl]-2,2,2-trifluoroethanone

To 1-[(2S)-2-(1,1-difluoroethyl)-6-oxido-2,3-dihydropyrido[2,3-f] [1,4]oxazepin-4(5H)-yl]-2,2,2-trifluoroethanone (366 mg) of Example 45g was added phosphoryl chloride (16.0 g), and the mixture was stirred at 60°C for 3 hr. The solvent was evaporated under reduced pressure, to the obtained residue was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=100/0-70/30(V/V)] to give the title compound (59.4 mg, yield: 15%) as a solid.

### (45i) tert-Butyl (2S)-7-chloro-2-(1,1-difluoroethyl)-2,3-dihydropyrido[2,3-f][1,4]oxazepine-4(5H)-carboxylate

To a mixture of 1-[(2S)-7-chloro-2-(1,1-difluoroethyl)-2,3-dihydropyrido[2,3-f][1,4]oxazepin-4(5H)-yl]-2,2,2-trifluoroethanone (59.4 mg) of Example 45h, methanol (4 mL) and water (1 mL) was added potassium carbonate (81.7 mg), and the mixture was stirred at room temperature for 40 min. The solvent was evaporated under reduced pressure, the obtained residue was dissolved in 1,4-dioxane (4 mL) and water (1 mL), di-tert-butyl dicarbonate (131 mg) was added and the mixture was stirred at room temperature for 40 min. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=100/0-90/10(V/V)] to give the title compound (45.7 mg, yield: 76%) as an oil.

### (45j) tert-Butyl (2S)-2-(1,1-difluoroethyl)-7-hydroxy-2,3-dihydropyrido[2,3-f][1,4]oxazepine-4(5H)-carboxylate

To a solution of tert-butyl (2S)-7-chloro-2-(1,1-difluoroethyl)-2,3-dihydropyrido[2,3-f][1,4]oxazepine-4(5H)-carboxylate (45.7 mg) of Example 45i in 1,4-dioxane (2 mL) were added 5-(di-tert-butylphosphino)-1',3',5'-triphenyl-1'H-1,4'-bipyrazole (15.4 mg), tris(dibenzylideneacetone)dipalladium(0) (14.7 mg) and cesium hydroxide monohydrate (78.4 mg), and the mixture was stirred at 100°C for 5 hr. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=20/80-0/100(V/V)] to give the title compound (13.5 mg, yield: 32%) as a solid.

### (45k) (2S)-2-(1,1-Difluoroethyl)-2,3,4,5-tetrahydropyrido[2,3-f][1,4]oxazepin-7-ol hydrochloride

To a solution of tert-butyl (2S)-2-(1,1-difluoroethyl)-7-hydroxy-2,3-dihydropyrido[2,3-f][1,4]oxazepine-4(5H)-carboxylate (13.5 mg) of Example 45j in 1,4-dioxane (2 mL) was added 4 M hydrogen chloride-1,4-dioxane solution (1 mL), and the mixture was stirred at room temperature for 4 hr. The solvent was evaporated under reduced pressure to give the title compound (10.9 mg, yield: quantitative) as a solid.

### (451) Ethyl (3R)-3-(1,4-dimethyl-1H-benzotriazol-5-yl)-3-[7-(hydroxymethyl)-1-benzothiophen-5-yl]propanoate

Ethyl 3-(1,4-dimethyl-1H-benzotriazol-5-yl)-3-[7-(hydroxymethyl)-1-benzothiophen-5-yl]propanoate (120 g) of Example 1f was subjected to chiral SFC [column: CHIRALPAK AD (50 mm I.D.× 250 mm), mobile phase: carbon dioxide/ethanol=60/40(V/V)] to give the title compound (52.5 g) as the second peak (yield: 44%).
retention time: 2.455 min

### (45m) Ethyl (3R)-3-(7-{[(2S)-2-(1,1-difluoroethyl)-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoate

To a solution of ethyl (3R)-3-(1,4-dimethyl-1H-benzotriazol-5-yl)-3-[7-(hydroxymethyl)-1-benzothiophen-5-yl]propanoate (20.4 mg) of Example 451 in dichloromethane (3.0 mL) was added thionyl chloride (0.0059 mL) and the mixture was stirred at room temperature for 40 min, and the solvent was evaporated under reduced pressure. The obtained residue was dissolved in acetonitrile (2.0 mL), added to a separately prepared solution of N,N-diisopropylethylamine (0.0707 mL) and (2S)-2-(1,1-difluoroethyl)-2,3,4,5-tetrahydropyrido[2,3-f][1,4]oxazepin-7-ol hydrochloride (10.9 mg) of Example 45k in acetonitrile (2.0 mL), and the mixture was stirred at 90°C for 8 hr and cooled to room temperature. Under reduced pressure, the solvent was evaporated and the obtained residue was purified by silica gel column chromatography [elution solvent: ethyl acetate/methanol=100/0-90/10(V/V)] to give the title compound (17.0 mg, yield: 67%) as an oil.

### (45n)=(45)

### (3R)-3-(7-{[(2S)-2-(1,1-Difluoroethyl)-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

To a solution of ethyl (3R)-3-(7-{[(2S)-2-(1,1-difluoroethyl)-7-hydroxy-2,3-dihydropyrido[2,3-f][1,4]oxazepin-4(5H)-yl]methyl}-1-benzothiophen-5-yl)-3-(1,4-dimethyl-1H-benzotriazol-5-yl)propanoate (17.0 mg) of Example 45m in ethanol (3 mL) was added 1 M aqueous sodium hydroxide solution (0.0820 mL), and the mixture was stirred at 50°C for 18 hr. To the reaction mixture was added 1 M hydrochloric acid (0.0820 mL) and the mixture was stirred. The solvent was evaporated under reduced pressure and the obtained residue was purified by silica gel column chromatography [elution solvent: ethyl acetate/methanol=100/0-90/10(V/V)]. To the eluted compound were added ethyl acetate and n-hexane, and an ultrasonic treatment was performed. The solid was collected by filtration and vacuum dried to give the title compound (9.7 mg, yield: 60%) as a solid.

### (Example 46)

### (3R)-3-(3,7-Dimethyl-3H-[1,2,3]triazolo[4,5-b]pyridin-6-yl)-3-(7-{[(2S)-7-hydroxy-2-(trifluoromethyl)-2,3-dihydropyrido[2,3-f] [1,4]oxazepin-4(5H)-yl]methyl}-1-benzothiophen-5-yl)propanoic acid

### (46a) Ethyl (3R)-3-(3,7-dimethyl-3H-[1,2,3]triazolo[4,5-b]pyridin-6-yl)-3-[7-(hydroxymethyl)-1-benzothiophen-5-yl]propanoate

Bis(norbornadiene)rhodium(I) tetrafluoroborate (300 mg) and (2S,3S)-(-)-bis(diphenylphosphino)butane (346 mg) were dissolved in a mixed solvent of 1,4-dioxane (5 mL) and water (1 mL), and the mixture was stirred for 5 min. This was added to a separately-prepared mixed solution of [5-(4,4,5,5-tetramethyl-1,3,2-dioxaborolan-2-yl)-1-benzothiophen-7-yl]methanol (7.1 g) of Example 1e and ethyl (2E)-3-(3,7-dimethyl-3H-[1,2,3]triazolo[4,5-b]pyridin-6-yl)prop-2-enoate (WO 2015/092713) (4.0 g) in 1,4-dioxane (60 mL) and water (10 mL) at room temperature, and 1 M aqueous potassium hydroxide solution (16 mL) was added. The temperature was raised to 50°C, and the mixture was stirred with heating for 2 hr. The reaction mixture was cooled to room temperature and extracted with aqueous ammonium chloride solution and ethyl acetate. The organic layer was washed with saturated brine and dried over anhydrous sodium sulfate. After filtration, the filtrate was concentrated under reduced pressure, and the obtained residue was purified by silica gel column chromatography [elution solvent: hexane/ethyl acetate=1/1(V/V)] to give the title compound (mixture of both enantiomers, 6.1 g, yield: 91%) as a solid. This was subjected to chiral HPLC [column: CHIRALCEL OZ-H (20 mm I.D. × 250 mm), mobile phase: n-hexane/ethanol=70/30(V/V)] to give 4.0 g of ethyl (3S)-3-(3,7-dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate as the first peak (yield: 66%).
retention time: 10.10 min [column: CHIRALCEL OZ-H (4.6 mm I.D. × 250 mm), mobile phase: n-hexane/ethanol=70/30(V/V), flow rate: 1.0 mL/min, temperature: 40°C, wavelength: 254 nm]

### (46b) Ethyl (3R)-3-(3,7-dimethyl-3H-[1,2,3]triazolo[4,5-b]pyridin-6-yl)-3-(7-{[(2S)-7-hydroxy-2-(trifluoromethyl)-2,3-dihydropyrido[2,3-f][1,4]oxazepin-4(5H)-yl]methyl}-1-benzothiophen-5-yl)propanoate

To a solution of ethyl (3R)-3-(3,7-dimethyl-3H-[1,2,3]triazolo[4,5-b]pyridin-6-yl)-3-[7-(hydroxymethyl)-1-benzothiophen-5-yl]propanoate (70.2 mg) of Example 46a in dichloromethane (3.0 mL) was added thionyl chloride (0.0207 mL), and the mixture was stirred at room temperature for 1 hr, and the solvent was evaporated under reduced pressure. The obtained residue was dissolved in acetonitrile (3.0 mL), added to a separately-prepared solution of N,N-diisopropylethylamine (0.245 mL) and (2S)-2-(trifluoromethyl)-2,3,4,5-tetrahydropyrido[2,3-f][1,4]oxazepin-7-ol hydrochloride (38.4 mg) of Example 39b in acetonitrile (2.0 mL), and the mixture was stirred at 90°C for 9 hr and cooled to room temperature. Under reduced pressure, the solvent was evaporated and the obtained residue was purified by silica gel column chromatography [elution solvent: hexane/ethyl acetate=50/50-20/80(V/V)] to give the title compound (44.8 mg, yield: 50%) as an oil.

### (46c)=(46)

### (3R)-3-(3,7-Dimethyl-3H-[1,2,3]triazolo[4,5-b]pyridin-6-yl)-3-(7-{[(2S)-7-hydroxy-2-(trifluoromethyl)-2,3-dihydropyrido[2,3-f] [1,4]oxazepin-4(5H)-yl]methyl}-1-benzothiophen-5-yl)propanoic acid

To a solution of ethyl (3R)-3-(3,7-dimethyl-3H-[1,2,3]triazolo[4,5-b]pyridin-6-yl)-3-(7-{[(2S)-7-hydroxy-2-(trifluoromethyl)-2,3-dihydropyrido[2,3-f][1,4]oxazepin-4(5H)-yl]methyl}-1-benzothiophen-5-yl)propanoate (44.8 mg) of Example 46b in ethanol (5 mL) was added 1 M aqueous sodium hydroxide solution (0.357 mL), and the mixture was stirred at 60°C for 3.5 hr. To the reaction mixture was added 1 M hydrochloric acid (0.357 mL) and the mixture was stirred. The solvent was evaporated under reduced pressure and the obtained residue was purified by silica gel column chromatography [elution solvent: ethyl acetate/methanol=100/0-90/10(V/V)]. Ethyl acetate and n-hexane were added to the eluted compound, and an ultrasonic treatment was performed. The solid was collected by filtration and vacuum dried to give the title compound (26.0 mg, yield: 61%) as a solid.

### (Example 47)

### (3R)-3-(1,4-Dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoic acid

### (47a) Methyl (3R)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoate

To a solution of methyl (3R)-3-(1,4-dimethyl-1H-benzotriazol-5-yl)-3-[7-(hydroxymethyl)-1-benzothiophen-5-yl]-2,2-dimethylpropanoate (60.0 mg) of Example 12a in dichloromethane (3 mL) was added thionyl chloride (0.021 mL), and the mixture was stirred at room temperature for 2 hr. The solvent was evaporated under reduced pressure, and the residue was azeotropically distilled with toluene. The obtained residue was dissolved in acetonitrile (3 mL), N,N-diisopropylethylamine (0.243 mL) and 4',5'-dihydro-3'H-spiro[cyclopropane-1,2'-pyrido[2,3-f][1,4]oxazepin]-7'-ol hydrochloride (39.0 mg) of Example 3g were added, and the mixture was stirred at 85°C for 8 hr and cooled to room temperature. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: dichloromethane/methanol=100/0-90/10(V/V)] to give the title compound (75 mg, yield: 89%) as an oil.

### (47b)=(47)

### (3R)-3-(1,4-Dimethyl-1H-benzotriazol-5-yl)-3-{7-[(7'-hydroxy-3'H-spiro[cyclopropane-1,2'-pyrido[2,3-f][1,4]oxazepin]-4' (5'H)-yl)methyl]-1-benzothiophen-5-yl}-2,2-dimethylpropanoic acid

To a solution of methyl (3R)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoate (75.0 mg) of Example 47a in dimethyl sulfoxide (1 mL) was added 1 M aqueous potassium hydroxide solution (0.627 mL), and the mixture was stirred at 70°C for 13 hr and cooled to room temperature. The reaction mixture was neutralized by adding 1 M hydrochloric acid, saturated aqueous ammonium chloride solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent:
chloroform/methanol=100/0-90/10(V/V)] to give the title compound (69 mg, yield: 94%) as a solid.

### (Example 48)

### (3S)-3-(3,7-Dimethyl-3H-[1,2,3]triazolo[4,5-b]pyridin-6-yl)-3-(7-{[(2R)-2-ethyl-7-hydroxy-2,3-dihydropyrido[2,3-f] [1,4]oxazepin-4(5H)-yl]methyl}-1-benzothiophen-5-yl)-2,2-dimethylpropanoic acid

### (48a) 3,7-Dimethyl-3H-[1,2,3]triazolo[4,5-b]pyridine-6-carbaldehyde

To a solution of ethylmagnesium bromide (0.950 M tetrahydrofuran solution) (11.6 mL) in toluene (88 mL) was added n-butyllithium (1.56 M n-hexane solution) (15.5 mL) at - 45°C, and the mixture was stirred at -45°C for 1 hr. A solution of 6-bromo-3,7-dimethyl-triazolo[4,5-b]pyridine (5.00 g) in tetrahydrofuran (22 mL) was added thereto at -45°C, and the mixture was stirred at -45°C for 1 hr. The temperature was raised to -15°C and N,N-dimethylformamide (10.2 mL) was added thereto. The mixture was stirred at -15°C for 30 min and at room temperature for 1 hr, and tetrahydrofuran (80 mL) was added. To the reaction mixture was added saturated aqueous ammonium chloride solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=60/40-40/60(V/V)] to give the title compound (710 mg, yield: 18%) as a solid.

### (48b) (3,7-Dimethyl-3H-[1,2,3]triazolo[4,5-b]pyridin-6-yl) (7-{[(4-methoxybenzyl)oxy]methyl}-1-benzothiophen-5-yl)methanol

To a solution of 5-bromo-7-{[(4-methoxybenzyl)oxy]methyl}-1-benzothiophene (1.61 g) of Example 11a in tetrahydrofuran (20 mL) was added n-butyllithium (1.56 M n-hexane solution) (2.84 mL) at -78°C, and the mixture was stirred at -78°C for 10 min. A separately prepared solution of 3,7-dimethyl-3H-[1,2,3]triazolo[4,5-b]pyridine-6-carbaldehyde (710 mg) of Example 48a in tetrahydrofuran (20 mL) was added thereto at -78°C, and the mixture was stirred at -78°C for 15 min and at 0°C for 2 hr. To the reaction mixture was added saturated aqueous ammonium chloride solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=60/40-45/55(V/V)] to give the title compound (425 mg, yield: 23%) as a solid.

### (48c) Methyl 3-(3,7-dimethyl-3H-[1,2,3]triazolo[4,5-b]pyridin-6-yl)-3-(7-{[(4-methoxybenzyl)oxy]methyl}-1-benzothiophen-5-yl)-2,2-dimethylpropanoate

To a solution of (3,7-dimethyl-3H-[1,2,3]triazolo[4,5-b]pyridin-6-yl) (7-{ [(4-methoxybenzyl)oxy]methyl}-1-benzothiophen-5-yl)methanol (425 mg) of Example 48b and trichloroacetonitrile (0.112 mL) in acetonitrile (20 mL) was added 1,8-diazabicyclo[5.4.0]-7-undecene (0.007 mL), and the mixture was stirred at room temperature for 2 hr. Methyltrimethylsilyl dimethylketene acetal (0.468 mL) and bis(trifluoromethanesulfonyl)imide (26 mg) were added thereto, and the mixture was stirred at room temperature for 15 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=70/30-50/50(V/V)] to give the title compound (367 mg, yield: 73%) as an oil.

### (48d) Methyl 3-(3,7-dimethyl-3H-[1,2,3]triazolo[4,5-b]pyridin-6-yl)-3-[7-(hydroxymethyl)-1-benzothiophen-5-yl]-2,2-dimethylpropanoate

To a solution of methyl 3-(3,7-dimethyl-3H-[1,2,3]triazolo[4,5-b]pyridin-6-yl)-3-(7-{[(4-methoxybenzyl)oxy]methyl}-1-benzothiophen-5-yl)-2,2-dimethylpropanoate (367 mg) of Example 48c in dichloromethane (6.74 mL) and water (0.674 mL) was added 2,3-dichloro-5,6-dicyano-1,4-benzoquinone (337 mg) at 0°C, and the mixture was stirred at 0°C for 2 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=40/60-20/80(V/V)] to give the title compound (230 mg, yield: 80%) as an oil.

### (48e) Methyl (3S)-3-(3,7-dimethyl-3H-[1,2,3]triazolo[4,5-b]pyridin-6-yl)-3-[7-(hydroxymethyl)-1-benzothiophen-5-yl]-2,2-dimethylpropanoate

Methyl 3-(3,7-dimethyl-3H-[1,2,3]triazolo[4,5-b]pyridin-6-yl)-3-[7-(hydroxymethyl)-1-benzothiophen-5-yl]-2,2-dimethylpropanoate (550 mg) of Example 48d was subjected to chiral HPLC [column: CHIRALPAK IH (20 mm I.D. × 250 mm), mobile phase: n-hexane/ethanol=70/30(V/V)] to give 112 mg of methyl (3S)-3-(3,7-dimethyl-3H-[1,2,3]triazolo[4,5-b]pyridin-6-yl)-3-[7-(hydroxymethyl)-1-benzothiophen-5-yl]-2,2-dimethylpropanoate as the first peak (yield: 49%).
retention time: 6.017 min [column: CHIRALPAK IH (4.6 mm I.D. × 250 mm), mobile phase: n-hexane/ethanol=70/30(V/V), flow rate: 1.0 mL/min, temperature: 40°C, wavelength: 254 nm]

### (48f) Methyl (3S)-3-(3,7-dimethyl-3H-[1,2,3]triazolo[4,5-b]pyridin-6-yl)-3-(7-{[(2R)-2-ethyl-7-hydroxy-2,3-dihydropyrido[2,3-f] [1,4]oxazepin-4(5H)-yl]methyl}-1-benzothiophen-5-yl)-2,2-dimethylpropanoate

To a solution of methyl (3S)-3-(3,7-dimethyl-3H-[1,2,3]triazolo[4,5-b]pyridin-6-yl)-3-[7-(hydroxymethyl)-1-benzothiophen-5-yl]-2,2-dimethylpropanoate (29.0 mg) of Example 48e in dichloromethane (1 mL) was added thionyl chloride (0.010 mL), and the mixture was stirred at room temperature for 1 hr. The reaction mixture was concentrated under reduced pressure, and the residue was azeotropically distilled with toluene. The obtained residue was dissolved in acetonitrile (2 mL), N,N-diisopropylethylamine (0.059 mL) and (2R)-2-ethyl-2,3,4,5-tetrahydropyrido[2,3-f][1,4]oxazepin-7-ol (15.0 mg) of Example 17e were added, and the mixture was stirred at 85°C for 8 hr and cooled to room temperature. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: dichloromethane/methanol=100/0-85/15(V/V)] to give the title compound (37 mg, yield: 90%) as an oil.

### (48g)=(48)

### (3S)-3-(3,7-Dimethyl-3H-[1,2,3]triazolo[4,5-b]pyridin-6-yl)-3-(7-{[(2R)-2-ethyl-7-hydroxy-2,3-dihydropyrido[2,3-f] [1,4]oxazepin-4(5H)-yl]methyl}-1-benzothiophen-5-yl)-2,2-dimethylpropanoic acid

To a solution of methyl (3S)-3-(3,7-dimethyl-3H-[1,2,3]triazolo[4,5-b]pyridin-6-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoate (37.0 mg) of Example 48f in dimethyl sulfoxide (1 mL) was added 1 M aqueous potassium hydroxide solution (0.308 mL), and the mixture was stirred at 70°C for 6 hr and cooled to room temperature. The reaction mixture was neutralized by adding 1 M hydrochloric acid, saturated aqueous ammonium chloride solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent:
chloroform/methanol=100/0-90/10(V/V)] to give the title compound (34 mg, yield: 94%) as a solid.

### (Example 49)

### (3R)-3-(3,7-Dimethyl-3H-[1,2,3]triazolo[4,5-b]pyridin-6-yl)-3-(7-{[(2R)-2-ethyl-7-hydroxy-2,3-dihydropyrido[2,3-f] [1,4]oxazepin-4(5H)-yl]methyl}-1-benzothiophen-5-yl)-2,2-dimethylpropanoic acid

### (49a) Methyl (3R)-3-(3,7-dimethyl-3H-[1,2,3]triazolo[4,5-b]pyridin-6-yl)-3-[7-(hydroxymethyl)-1-benzothiophen-5-yl]-2,2-dimethylpropanoate

Methyl 3-(3,7-dimethyl-3H-[1,2,3]triazolo[4,5-b]pyridin-6-yl)-3-[7-(hydroxymethyl)-1-benzothiophen-5-yl]-2,2-dimethylpropanoate (550 mg) of Example 48d was subjected to chiral HPLC [column: CHIRALPAK IH (20 mm I.D. × 250 mm), mobile phase: n-hexane/ethanol=70/30(V/V)] to give 114 mg of methyl (3R)-3-(3,7-dimethyl-3H-[1,2,3]triazolo[4,5-b]pyridin-6-yl)-3-[7-(hydroxymethyl)-1-benzothiophen-5-yl]-2,2-dimethylpropanoate as the second peak (yield: 49%).
retention time: 6.753 min [column: CHIRALPAK IH (4.6 mm I.D. × 250 mm), mobile phase: n-hexane/ethanol=70/30(V/V), flow rate: 1.0 mL/min, temperature: 40°C, wavelength: 254 nm]

### (49b) Methyl (3R)-3-(3,7-dimethyl-3H-[1,2,3]triazolo[4,5-b]pyridin-6-yl)-3-(7-{[(2R)-2-ethyl-7-hydroxy-2,3-dihydropyrido[2,3-f] [1,4]oxazepin-4(5H)-yl]methyl}-1-benzothiophen-5-yl)-2,2-dimethylpropanoate

To a solution of methyl (3R)-3-(3,7-dimethyl-3H-[1,2,3]triazolo[4,5-b]pyridin-6-yl)-3-[7-(hydroxymethyl)-1-benzothiophen-5-yl]-2,2-dimethylpropanoate (28.0 mg) of Example 49a in dichloromethane (1 mL) was added thionyl chloride (0.010 mL) and the mixture was stirred at room temperature for 1 hr. The solvent was evaporated under reduced pressure, and the residue was azeotropically distilled with toluene. The obtained residue was dissolved in acetonitrile (2 mL), N,N-diisopropylethylamine (0.057 mL) and (2R)-2-ethyl-2,3,4,5-tetrahydropyrido[2,3-f][1,4]oxazepin-7-ol (14 mg) of Example 17e were added, and the mixture was stirred at 85°C for 10 hr and cooled to room temperature. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: dichloromethane/methanol=100/0-85/15(V/V)] to give the title compound (34 mg, yield: 86%) as an oil.

### (49c)=(49)

### (3R)-3-(3,7-Dimethyl-3H-[1,2,3]triazolo[4,5-b]pyridin-6-yl)-3-(7-{[(2R)-2-ethyl-7-hydroxy-2,3-dihydropyrido[2,3-f] [1,4]oxazepin-4(5H)-yl]methyl}-1-benzothiophen-5-yl)-2,2-dimethylpropanoic acid

To a solution of methyl (3R)-3-(3,7-dimethyl-3H-[1,2,3]triazolo[4,5-b]pyridin-6-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoate (34.0 mg) of Example 49b in dimethyl sulfoxide (1 mL) was added 1 M aqueous potassium hydroxide solution (0.283 mL), and the mixture was stirred at 70°C for 9 hr and cooled to room temperature. The reaction mixture was neutralized by adding 1 M hydrochloric acid, saturated aqueous ammonium chloride solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent:
chloroform/methanol=100/0-90/10(V/V)] to give the title compound (28 mg, yield: 84%) as a solid.

### (Example 50)

### (3S)-3-(3,7-Dimethyl-3H-[1,2,3]triazolo[4,5-b]pyridin-6-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoic acid

### (50a) Methyl (3S)-3-(3,7-dimethyl-3H-[1,2,3]triazolo[4,5-b]pyridin-6-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoate

To a solution of methyl (3S)-3-(3,7-dimethyl-3H-[1,2,3]triazolo[4,5-b]pyridin-6-yl)-3-[7-(hydroxymethyl)-1-benzothiophen-5-yl]-2,2-dimethylpropanoate (31.0 mg) of Example 48e in dichloromethane (1 mL) was added thionyl chloride (0.011 mL), and the mixture was stirred at room temperature for 2 hr. The solvent was evaporated under reduced pressure, and the residue was azeotropically distilled with toluene. The obtained residue was dissolved in acetonitrile (2 mL), N,N-diisopropylethylamine (0.125 mL) and 4',5'-dihydro-3'H-spiro[cyclopropane-1,2'-pyrido[2,3-f] [1,4]oxazepin]-7'-ol hydrochloride (19 mg) of Example 3g were added, and the mixture was stirred at 85°C for 10 hr and cooled to room temperature. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent:
dichloromethane/methanol=100/0-90/10(V/V)] to give the title compound (36 mg, yield: 82%) as an oil.

### (50b)=(50)

### (3S)-3-(3,7-Dimethyl-3H-[1,2,3]triazolo[4,5-b]pyridin-6-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoic acid

To a solution of methyl (3S)-3-(3,7-dimethyl-3H-[1,2,3]triazolo[4,5-b]pyridin-6-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoate (36 mg) of Example 50a in dimethyl sulfoxide (1 mL) was added 1 M aqueous potassium hydroxide solution (0.301 mL), and the mixture was stirred at 70°C for 23 hr and cooled to room temperature. The reaction mixture was neutralized by adding 1 M hydrochloric acid, saturated aqueous ammonium chloride solution was added, and the mixture was extracted with ethyl acetate. The organic layer was washed with saturated brine and dried over anhydrous sodium sulfate. The solvent was evaporated under reduced pressure and the obtained residue was purified by silica gel column chromatography [elution solvent: chloroform/methanol=100/0-90/10(V/V)] to give the title compound (30 mg, yield: 85%) as a solid.

### (Example 51)

### (3S)-3-(7-{[(2R)-2-Cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)-2,2-dimethylpropanoic acid

### (51a) Methyl (3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)-2,2-dimethylpropanoate

To a solution of methyl (3S)-3-(3,7-dimethyl-3H-[1,2,3]triazolo[4,5-b]pyridin-6-yl)-3-[7-(hydroxymethyl)-1-benzothiophen-5-yl]-2,2-dimethylpropanoate (38.0 mg) of Example 48e in dichloromethane (1 mL) was added thionyl chloride (0.014 mL), and the mixture was stirred at room temperature for 2 hr. The solvent was evaporated under reduced pressure, and the residue was azeotropically distilled with toluene. The obtained residue was dissolved in acetonitrile (2 mL), N,N-diisopropylethylamine (0.077 mL) and (2R)-2-cyclopropyl-2,3,4,5-tetrahydropyrido[2,3-f][1,4]oxazepin-7-ol (21 mg) of Example 14a were added, and the mixture was stirred at 85°C for 8 hr and cooled to room temperature. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: dichloromethane/methanol=100/0-90/10(V/V)] to give the title compound (49 mg, yield: 89%) as an oil.

### (51b) Methyl (3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)-2,2-dimethylpropanoate

Methyl (3S)-3-(7-{[(2R)-2-cyclopropyl-7-hydroxy-2,3-dihydropyrido[2,3-f][1,4]oxazepin-4(5H)-yl]methyl}-1-benzothiophen-5-yl)-3-(3,7-dimethyl-3H-[1,2,3]triazolo[4,5-b]pyridin-6-yl)-2,2-dimethylpropanoate (49.0 mg) of Example 51a was subjected to chiral HPLC [column: CHIRALPAK IG (20 mm I.D. × 250 mm), mobile phase: n-hexane/ethanol=50/50(V/V)] to give 34.0 mg of methyl (3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)-2,2-dimethylpropanoate as the first peak (yield: 69%).
retention time: 9.247 min [column: CHIRALPAK IG (4.6 mm I.D. × 250 mm), mobile phase: n-hexane/ethanol=50/50(V/V), flow rate: 1.0 mL/min, temperature: 40°C, wavelength: 254 nm]

### (51c)=(51)

### (3S)-3-(7-{[(2R)-2-Cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)-2,2-dimethylpropanoic acid

To a solution of methyl (3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)-2,2-dimethylpropanoate (34.0 mg) of Example 51b in dimethyl sulfoxide (1 mL) was added 1 M aqueous potassium hydroxide solution (0.277 mL), and the mixture was stirred at 70°C for 8 hr and cooled to room temperature. The reaction mixture was neutralized by adding 1 M hydrochloric acid, saturated aqueous ammonium chloride solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: chloroform/methanol=100/0-90/10(V/V)] to give the title compound (23 mg, yield: 69%) as a solid.

### (Example 52)

### (3R*)-3-(3,8-Dimethyl[1,2,4]triazolo[4,3-a]pyridin-7-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (Example 53)

### (3S*)-3-(3,8-Dimethyl[1,2,4]triazolo[4,3-a]pyridin-7-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (52a) 4-Bromo-2-hydrazinyl-3-methylpyridine

To a solution of 4-bromo-2-fluoro-3-methylpyridine (5.00 g) in pyridine (16.6 mL) was added hydrazine monohydrate (8.31 mL), and the mixture was stirred at 60°C for 22 hr. The reaction mixture was cooled to room temperature, azeotropically distilled with toluene and methanol. To the obtained residue was added methanol, and an ultrasonic treatment was performed. The solid was collected by filtration to give the title compound (5.43 g, yield: quantitative) as a solid.

### (52b) 7-Bromo-3,8-dimethyl[1,2,4]triazolo[4,3-a]pyridine

To 4-bromo-2-hydrazinyl-3-methylpyridine (1.40 g) obtained in Example 52a was added acetic anhydride (6.55 mL), and the mixture was stirred at room temperature for 30 min and heated under reflux for 75 min. The reaction mixture was cooled to room temperature, saturated aqueous sodium hydrogen carbonate solution was added, and the mixture was extracted with ethyl acetate. The organic layer was washed with saturated brine and dried over anhydrous sodium sulfate. The solvent was evaporated under reduced pressure, tert-butyl methyl ether was added to the obtained residue, and an ultrasonic treatment was performed. The solid was collected by filtration to give the title compound (1.00 g, yield: 64%) as a solid.

### (52c) Ethyl (2E)-3-(3,8-dimethyl[1,2,4]triazolo[4,3-a]pyridin-7-yl)prop-2-enoate

To a suspension of 7-bromo-3,8-dimethyl[1,2,4]triazolo[4,3-a]pyridine (1.00 g) of Example 52b in N,N-dimethylformamide (9 mL) were added ethyl acrylate (4.81 mL), N,N-diisopropylethylamine (3.86 mL), tris(dibenzylideneacetone)dipalladium(0) (405 mg) and tri(o-tolyl)phosphine (539 mg), and the mixture was stirred at 110°C for 12 hr. The reaction mixture was cooled to room temperature, water was added, and the mixture was saturated with sodium chloride, and extracted with ethyl acetate. The organic layer was dried over anhydrous sodium sulfate. Under reduced pressure, the solvent was evaporated and the obtained residue was purified by silica gel column chromatography [elution solvent: chloroform/methanol=100/0-90/10(V/V)] to give the title compound (1.00 g, yield: 92%) as a solid.

### (52d) Ethyl 3-(3,8-dimethyl[1,2,4]triazolo[4,3-a]pyridin-7-yl)-3-[7-(hydroxymethyl)-1-benzothiophen-5-yl]propanoate

To a mixture of ethyl (2E)-3-(3,8-dimethyl[1,2,4]triazolo[4,3-a]pyridin-7-yl)prop-2-enoate (600 mg) of Example 52c and [5-(4,4,5,5-tetramethyl-1,3,2-dioxaborolan-2-yl)-1-benzothiophen-7-yl]methanol (801 mg) of Example 1e, 1,4-dioxane (16 mL) and water (8 mL) was added triethylamine (0.68 mL), and the mixture was stirred at 90°C for 10 min. Then, chloro(1,5-cyclooctadiene)rhodium(I) dimer (60 mg) was added thereto, and the mixture was stirred at 90°C for 15 min. The reaction mixture was cooled to room temperature, water was added, and the mixture was extracted with chloroform. The organic layer was dried over anhydrous sodium sulfate. Under reduced pressure, the solvent was evaporated and the obtained residue was purified by silica gel column chromatography [elution solvent:
chloroform/methanol=100/0-90/10(V/V)] to give the title compound (670 mg, yield: 67%) as a solid.

### (52e) Ethyl 3-(3,8-dimethyl[1,2,4]triazolo[4,3-a]pyridin-7-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoate

To a solution of ethyl 3-(3,8-dimethyl[1,2,4]triazolo[4,3-a]pyridin-7-yl)-3-[7-(hydroxymethyl)-1-benzothiophen-5-yl]propanoate (160 mg) of Example 52d in dichloromethane (1.3 mL) were added dimethyl sulfoxide (0.14 mL), N,N-diisopropylethylamine (0.20 mL) and sulfur trioxide-pyridine complex (207 mg), and the mixture was stirred at room temperature for 20 min. To the reaction mixture was added water, and the mixture was extracted with chloroform. The organic layer was dried over anhydrous sodium sulfate. The solvent was evaporated under reduced pressure. To a solution of the obtained residue in dichloromethane (2.0 mL) were added (2R)-2-ethyl-2,3,4,5-tetrahydropyrido[2,3-f][1,4]oxazepin-7-ol hydrochloride (180 mg) of Example 1h, N,N-diisopropylethylamine (0.11 mL) and acetic acid (0.11 mL) and the mixture was stirred at room temperature for 40 min. Then, sodium triacetoxyborohydride (166 mg) was added thereto, and the mixture was stirred at room temperature for 2 hr. To the reaction mixture was added sodium triacetoxyborohydride (23 mg), and the mixture was stirred at room temperature for 1 hr, sodium triacetoxyborohydride (23 mg) was added again, and the mixture was stirred at room temperature for 30 min. To the reaction mixture was added saturated aqueous sodium hydrogen carbonate solution, and the mixture was extracted with chloroform. The organic layer was dried over anhydrous sodium sulfate. The solvent was evaporated under reduced pressure and the obtained residue was purified by silica gel column chromatography [elution solvent: acetone, chloroform/methanol=100/0-90/10(V/V)] to give the title compound (121 mg, yield: 53%) as a solid.

### (52f)=(52)

### (3R*)-3-(3,8-Dimethyl(1,2,4]triazolo(4,3-a]pyridin-7-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

### (53a)=(53)

### (3S*)-3-(3,8-Dimethyl[1,2,4]triazolo[4,3-a]pyridin-7-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid

To a mixture of ethyl 3-(3,8-dimethyl[1,2,4]triazolo[4,3-a]pyridin-7-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoate (121 mg) of Example 52e, tetrahydrofuran (1.9 mL) and methanol (0.6 mL) was added 1 M aqueous lithium hydroxide solution (0.62 mL), and the mixture was stirred at 40°C for 80 min. To the reaction mixture was added saturated aqueous ammonium chloride solution, and the mixture was extracted with a mixed solvent of chloroform and methanol. The organic layer was washed with saturated brine and dried over anhydrous sodium sulfate. The solvent was evaporated under reduced pressure and the obtained residue was purified by DIOL silica gel column chromatography [elution solvent: n-hexane/chloroform=50/50-0/100 (V/V), chloroform/methanol=100/0-90/10 (V/V)]. Ethyl acetate and n-hexane were added to the compound eluted earlier, and an ultrasonic treatment was performed. The solid was collected by filtration to give (3S*)-3-(3,8-dimethyl[1,2,4]triazolo[4,3-a]pyridin-7-yl)-3-(7-{[(2R)-2-ethyl-7-hydroxy-2,3-dihydropyrido[2,3-f] [1,4]oxazepin-4(5H)-yl]methyl}-1-benzothiophen-5-yl)propanoic acid (30.6 mg, yield: 27%) as a solid.

In the same manner, moreover, the compound eluted later, (3R*)-3-(3,8-dimethyl[1,2,4]triazolo[4,3-a]pyridin-7-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid (35.9 mg, yield: 31%) was obtained as a solid.

### (Example 54)

### 3-(3,8-Dimethyl[1,2,4]triazolo[4,3-a]pyridin-7-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoic acid

### (54a) Ethyl 3-(3,8-dimethyl[1,2,4]triazolo[4,3-a]pyridin-7-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoate

To a solution of ethyl 3-(3,8-dimethyl[1,2,4]triazolo[4,3-a]pyridin-7-yl)-3-[7-(hydroxymethyl)-1-benzothiophen-5-yl]propanoate (160 mg) of Example 52d in dichloromethane (1.3 mL) were added dimethyl sulfoxide (0.14 mL), N,N-diisopropylethylamine (0.20 mL) and sulfur trioxide-pyridine complex (207 mg), and the mixture was stirred at room temperature for 30 min. To the reaction mixture was added water, and the mixture was extracted with chloroform. The organic layer was dried over anhydrous sodium sulfate. The solvent was evaporated under reduced pressure and the obtained residue was dissolved in dichloromethane (1.3 mL). 4',5'-Dihydro-3'H-spiro[cyclopropane-1,2'-pyrido[2,3-f][1,4]oxazepin]-7'-ol hydrochloride (124 mg) of Example 3g, N,N-diisopropylethylamine (0.081 mL) and acetic acid (0.081 mL) were added thereto, and the mixture was stirred at room temperature for 30 min and at 35°C for 20 min. Then, sodium triacetoxyborohydride (166 mg) was added thereto, and the mixture was stirred at 35°C for 2 hr. To the reaction mixture was added saturated aqueous sodium hydrogen carbonate solution, and the mixture was extracted with chloroform. The organic layer was dried over anhydrous sodium sulfate. Under reduced pressure, the solvent was evaporated and the obtained residue was purified by silica gel column chromatography [elution solvent: acetone/ethyl acetate=50/50-100/0(V/V), chloroform/methanol=100/0-90/10(V/V)] to give the title compound (88 mg, yield: 39%) as a solid.

### (54b)=(54)

### 3-(3,8-Dimethyl[1,2,4]triazolo[4,3-a]pyridin-7-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoic acid

To a mixture of ethyl 3-(3,8-dimethyl[1,2,4]triazolo[4,3-a]pyridin-7-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoate (88 mg) of Example 54a, tetrahydrofuran (1.35 mL) and methanol (0.45 mL) was added 1 M aqueous lithium hydroxide solution (0.45 mL) and the mixture was stirred at room temperature for 13 hr. To the reaction mixture was added saturated aqueous ammonium chloride solution, and the mixture was extracted with a mixed solvent of chloroform and methanol. The organic layer was dried over anhydrous sodium sulfate. The solvent was evaporated under reduced pressure and the obtained residue was purified by DIOL silica gel column chromatography [elution solvent: chloroform/methanol=100/0-95/5(V/V)]. To the obtained residue were added ethyl acetate and n-hexane, and an ultrasonic treatment was performed. The solid was collected by filtration to give the title compound (51 mg, yield: 61%) as a solid.

### (Example 55)

### (3R)-3-(7-{[(2R)-7-Amino-2-ethyl-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

### (Example 56)

### (3S)-3-(7-{[(2R)-7-Amino-2-ethyl-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

### (55a) 6-Bromo-2-[(E)-{[(2S)-2-hydroxybutyl]imino}methyl]pyridine-3-ol

To a solution of 6-bromo-3-hydroxypyridine-2-carbaldehyde (29 g) and (2S)-1-aminobutan-2-ol (29 g) in tetrahydrofuran (500 mL) was added sodium sulfate (121 g), and the mixture was stirred at 20°C for 3 hr. The reaction mixture was filtered, and the filtrate was concentrated under reduced pressure to give an unpurified title compound (36 g) as an oil.

### (55b) tert-Butyl [(6-bromo-3-hydroxypyridin-2-yl)methyl][(2S)-2-hydroxybutyl]carbamate

To a mixture of 6-bromo-2-[(E)-{[(2S)-2-hydroxybutyl]imino}methyl]pyridine-3-ol (34.0 g) of Example 55a, dichloromethane (500 mL), and methanol (100 mL) was added sodium borohydride (18.97 g), and the mixture was stirred at 20°C for 16 hr. To the reaction mixture were added di-tert-butyl dicarbonate (68.0 g) and triethylamine (49.44 g), and the mixture was further stirred for 16 hr. To the reaction mixture was added water (600 mL), and the mixture was extracted twice with dichloromethane. The combined organic layer was dried over anhydrous sodium sulfate, filtered, the solvent was evaporated under reduced pressure, and the obtained residue was purified by silica gel column chromatography [elution solvent: petroleum ether/ethyl acetate=1/1(V/V)] to give the title compound (38 g, yield: 70%) as an oil.

### (55c) tert-Butyl (2R)-7-bromo-2-ethyl-2,3-dihydropyrido[2,3-f][1,4]oxazepine-4(5H)-carboxylate

To a solution of tert-butyl [(6-bromo-3-hydroxypyridin-2-yl)methyl][(2S)-2-hydroxybutyl]carbamate (35 g) of Example 55b in tetrahydrofuran (300 mL) was added a solution of diisopropyl azodicarboxylate (26 g) in tetrahydrofuran (20 mL), then a solution of triphenylphosphine (30 g) in tetrahydrofuran (80 mL) was added at 0°C, and the mixture was stirred at 0 - 20°C for 16 hr. The reaction mixture was concentrated under reduced pressure and the obtained residue was purified by silica gel column chromatography [elution solvent: petroleum ether/ethyl acetate=5/1-3/1(V/V)], then preparative HPLC [column: Phenomenex Luna C18 (50 mm I.D.× 250 mm), mobile phase: 0.225% aqueous formic acid solution/acetonitrile=50/50-25/75(V/V)] to give the title compound (15.62 g, yield: 47%) as a solid.

### (55d) tert-Butyl (2R)-7-amino-2-ethyl-2,3-dihydropyrido[2,3-f][1,4]oxazepine-4(5H)-carboxylate

tert-Butyl (2R)-7-bromo-2-ethyl-2,3-dihydropyrido[2,3-f] [1,4]oxazepine-4(5H)-carboxylate (1.0 g) of Example 55c, 2-(dicyclohexylphosphino)biphenyl (0.05 g) and bis(dibenzylideneacetone)palladium (0.08 g) were dissolved in tetrahydrofuran (30 mL), lithium bis(trimethylsilyl)amide (1 M tetrahydrofuran solution) (2.8 mL) was added and the mixture was stirred at room temperature for 3 hr. The reaction mixture was poured into water, and the mixture was extracted with ethyl acetate. The organic layer was washed with saturated brine and dried over anhydrous sodium sulfate. The solvent was evaporated under reduced pressure and the obtained residue was purified by NH silica gel column chromatography [elution solvent: n-hexane/ethyl acetate=50/50-0/100(V/V)] and further purified by NH silica gel column chromatography [elution solvent: n-hexane/ethyl acetate=70/30-0/100(V/V)] to give the title compound (300 mg, yield: 37%) as an oil.

### (55e) Ethyl 3-(7-{[(2R)-7-amino-2-ethyl-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoate

To a solution of tert-butyl (2R)-7-amino-2-ethyl-2,3-dihydropyrido[2,3-f][1,4]oxazepine-4(5H)-carboxylate (22 mg) of Example 55d in dichloromethane (1 mL) was added trifluoroacetic acid (0.5 mL), and the mixture was stirred at room temperature for 1 hr. The solvent was evaporated under reduced pressure, the obtained residue was dissolved in acetonitrile (3 mL), added to a separately-prepared solution of N,N-diisopropylethylamine (0.13 mL) and ethyl 3-[7-(chloromethyl)-1-benzothiophen-5-yl]-3-(1,4-dimethyl-1H-benzotriazol-5-yl)propanoate (30 mg) of Example 1g in acetonitrile (3 mL), and the mixture was stirred at 85°C for 48 hr and cooled to room temperature. The reaction mixture was poured into water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V)] and further purified by NH silica gel column chromatography [elution solvent: n-hexane/ethyl acetate=50/50-0/100(V/V)] to give the title compound (14 mg, yield: 32%) as an oil.

### (55f)=(55)

### (3R)-3-(7-{[(2R)-7-Amino-2-ethyl-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

### (56a)=(56)

### (3S)-3-(7-{[(2R)-7-Amino-2-ethyl-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid

To a mixture of ethyl 3-(7-{[(2R)-7-amino-2-ethyl-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoate (14 mg) of Example 55e, ethanol (1 mL), and tetrahydrofuran (1 mL) was added 1 M aqueous sodium hydroxide solution (0.12 mL), and the mixture was stirred at room temperature for 12 hr. To the reaction mixture was added 1 M hydrochloric acid (0.12 mL), and the mixture was stirred and extracted with ethyl acetate. The organic layer was washed with saturated brine and dried over anhydrous sodium sulfate. Under reduced pressure, the solvent was evaporated and the obtained residue was purified by silica gel column chromatography [elution solvent: dichloromethane/methanol=10/1(V/V)] to give two compounds. Ethyl acetate and n-hexane were added to the compound eluted earlier and the mixture was subjected to an ultrasonic treatment. The solid was collected by filtration and vacuum dried to give (3R)-3-(7-{[(2R)-7-amino-2-ethyl-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid (3.0 mg, yield: 23%) as a solid.

In the same manner, moreover, the compound eluted later, (3S)-3-(7-{[(2R)-7-amino-2-ethyl-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid (2.0 mg, yield: 19%) was obtained as a solid.

### (Example 57)

### 3-(1,4-Dimethyl-1H-benzotriazol-5-yl)-3-(7-{[(2R)-2-ethyl-7-fluoro-2,3-dihydropyrido[2,3-f] [1,4]oxazepin-4(5H)-yl]methyl}-1-benzothiophen-5-yl)propanoic acid

### (57a) (2R)-2-Ethyl-7-fluoro-2,3,4,5-tetrahydropyrido[2,3-f][1,4]oxazepine

To a solution of tert-butyl (2R)-7-amino-2-ethyl-2,3-dihydropyrido[2,3-f][1,4]oxazepine-4(5H)-carboxylate (71 mg) of Example 55e in hydrogen fluoride pyridine (1.5 mL) was added dropwise a separately-prepared solution of sodium nitrite (50 mg) in water (1 mL) at -5°C, and the mixture was stirred at room temperature for 1 hr. The reaction mixture was poured into saturated aqueous sodium hydrogen carbonate solution, and the mixture was extracted with dichloromethane. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V)] to give the title compound (20 mg, yield: 42%) as a solid.

### (57b) Ethyl 3-(1,4-dimethyl-1H-benzotriazol-5-yl)-3-(7-formyl-1-benzothiophen-5-yl)propanoate

To a solution of ethyl 3-(1,4-dimethyl-1H-benzotriazol-5-yl)-3-[7-(hydroxymethyl)-1-benzothiophen-5-yl]propanoate (250 mg) of Example 1f in chloroform (50 mL) was added manganese(IV) oxide (530 mg), and the mixture was stirred at 85°C for 4 hr. The reaction mixture was filtered through celite, the filtrate was concentrated under reduced pressure and the obtained residue was purified by silica gel column chromatography [elution solvent: n-hexane/ethyl acetate=70/30-60/40(V/V)] to give the title compound (240 mg, yield: 96%) as a solid.

### (57c) Ethyl 3-(1,4-dimethyl-1H-benzotriazol-5-yl)-3-(7-{[(2R)-2-ethyl-7-fluoro-2,3-dihydropyrido[2,3-f] [1,4]oxazepin-4(5H)-yl]methyl}-1-benzothiophen-5-yl)propanoate

Ethyl 3-(1,4-dimethyl-1H-benzotriazol-5-yl)-3-(7-formyl-1-benzothiophen-5-yl)propanoate (46 mg) of Example 57b, and (2R)-2-ethyl-7-fluoro-2,3,4,5-tetrahydropyrido[2,3-f][1,4]oxazepine (20 mg) of Example 57a were dissolved in dichloromethane (3 mL), acetic acid (0.010 mL) and sodium triacetoxyborohydride (45 mg) were added and the mixture was stirred at room temperature for 12 hr. Under reduced pressure, the solvent was evaporated and the obtained residue was purified by silica gel column chromatography [elution solvent: n-hexane/ethyl acetate=50/50-30/70(V/V)] to give the title compound (35 mg, yield: 58%) as a solid.

### (57d)=(57)

### 3-(1,4-Dimethyl-1H-benzotriazol-5-yl)-3-(7-{[(2R)-2-ethyl-7-fluoro-2,3-dihydropyrido[2,3-f][1,4]oxazepin-4(5H)-yl]methyl}-1-benzothiophen-5-yl)propanoic acid

To a mixture of ethyl 3-(1,4-dimethyl-1H-benzotriazol-5-yl)-3-(7-{[(2R)-2-ethyl-7-fluoro-2,3-dihydropyrido[2,3-f] [1,4]oxazepin-4(5H)-yl]methyl}-1-benzothiophen-5-yl)propanoate (35 mg) of Example 57c, ethanol (1 mL), and tetrahydrofuran (1 mL) was added 1 M aqueous sodium hydroxide solution (0.30 mL), and the mixture was stirred at room temperature for 12 hr. To the reaction mixture was added 1 M hydrochloric acid (0.30 mL), and the mixture was stirred and extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/95/5(V/V/V)] to give the title compound (30 mg, yield: 90%) as a solid.

### (Example 58)

### (3R*)-3-(1,4-Dimethyl-1H-benzotriazol-5-yl)-3-(7-([(3'R)-7'-hydroxy-3'-methyl-3'H-spiro[cyclopropane-1,2'-pyrido[2,3-f][1,4]oxazepin]-4' (5'H)-yl]methyl}-1-benzothiophen-5-yl)propanoic acid

### (Example 59)

### (3S*)-3-(1,4-Dimethyl-1H-benzotriazol-5-yl)-3-(7-([(3'R)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoic acid

### (58a) 1-{(1R)-1-[Bis(4-methoxybenzyl)amino]ethyl}cyclopropanol

To a suspension of ethyl D-alaninate hydrochloride (2.13 g) in acetonitrile (22 mL) were added potassium carbonate (4.64 g), 4-methoxybenzyl chloride (3.05 mL) and sodium iodide (168 mg), and the mixture was stirred at 50°C for 5.5 hr. The reaction mixture was cooled to room temperature, and insoluble material was filtered off. The filtrate was concentrated under reduced pressure and the obtained residue was suspended in tetrahydrofuran (16 mL), tetraisopropyl orthotitanate (0.80 mL) was added, ethylmagnesium bromide (1 M tetrahydrofuran solution) (33.6 mL) was added at 0°C and the mixture was stirred at room temperature for 1 hr. To the reaction mixture was added ethylmagnesium bromide (1 M tetrahydrofuran solution) (16.8 mL), and the mixture was stirred at room temperature for 30 min. To the reaction mixture was added saturated aqueous ammonium chloride solution and the mixture was stirred at room temperature for 10 min. Saturated aqueous sodium hydrogen carbonate solution was added, and the mixture was extracted with ethyl acetate. The organic layer was washed with water and saturated brine, and dried over anhydrous sodium sulfate. Under reduced pressure, the solvent was evaporated and the obtained residue was purified by silica gel column chromatography [elution solvent: n-hexane/ethyl acetate=100/0-70/30(V/V)] to give the title compound (3.35 g, yield: 78%) as an oil.

### (58b) 1-{(1R)-1-[(4-Methoxybenzyl)amino]ethyl}cyclopropanol

20% Palladium hydroxide-activated carbon (160 mg) was suspended in ethyl acetate, a solution of 1-{(1R)-1-[bis(4-methoxybenzyl)amino]ethyl}cyclopropanol (3.35 g) of Example 58a in methanol (80 mL) and acetic acid (8 mL) were added, and the mixture was stirred at room temperature under a hydrogen atmosphere at normal pressure for 1 hr. To the reaction mixture was added celite, insoluble material was filtered off, and washed with ethyl acetate. The filtrate was concentrated under reduced pressure and the obtained residue was azeotropically distilled with toluene and purified by NH silica gel column chromatography [elution solvent: n-hexane/ethyl acetate=100/0-0/100(V/V), chloroform/methanol=100/0-95/5(V/V)] to give the title compound (1.77 g, yield: 91%) as an oil.

### (58c) 1-[(1R)-1-{[(6-Bromo-3-fluoropyridin-2-yl)methyl](4-methoxybenzyl)amino}ethyl]cyclopropanol

To a solution of 6-bromo-2-(bromomethyl)-3-fluoropyridine (1.97 g) of Example 3a and 1-{(1R)-1-[(4-methoxybenzyl)amino]ethyl}cyclopropanol (970 mg) of Example 58b in acetonitrile (22 mL) was added potassium carbonate (908 mg), and the mixture was stirred at 50°C for 2 hr. The insoluble material in the reaction mixture was filtered off, the filtrate was concentrated under reduced pressure and the obtained residue was purified by silica gel column chromatography [elution solvent: n-hexane/ethyl acetate=100/0-70/30(V/V)] to give the title compound (1.61 g, yield: 82%) as an oil.

### (58d) (3'R)-7'-Bromo-4'-(4-methoxybenzyl)-3'-methyl-4',5'-dihydro-3'H-spiro[cyclopropane-1,2'-pyrido[2,3-f][1,4]oxazepine]

To a suspension of 60% sodium hydride (472 mg) in N,N-dimethylformamide (25 mL) was added dropwise over 15 min at 60°C a solution of 1-[(1R)-1-{[(6-bromo-3-fluoropyridin-2-yl)methyl](4-methoxybenzyl)amino}ethyl]cyclopropanol (1.61 g) of Example 58c in tetrahydrofuran (25 mL), and the mixture was stirred at 60°C for 40 min. The reaction mixture was cooled to room temperature, water was added, and the mixture was extracted with ethyl acetate. The organic layer was washed successively with water and saturated brine, and dried over anhydrous sodium sulfate. Under reduced pressure, the solvent was evaporated and the obtained residue was purified by silica gel column chromatography [elution solvent: n-hexane/ethyl acetate=100/0-80/20(V/V)] to give the title compound (1.40 g, yield: 96%) as an oil.

### (58e) (3'R)-4'-(4-Methoxybenzyl)-3'-methyl-4',5'-dihydro-3'H-spiro[cyclopropane-1,2'-pyrido[2,3-f][1,4]oxazepin]-7'-ol

To a solution of (3'R)-7'-bromo-4'-(4-methoxybenzyl)-3'-methyl-4',5'-dihydro-3'H-spiro[cyclopropane-1,2'-pyrido[2,3-f][1,4]oxazepine] (1.40 g) of Example 58d in 1,4-dioxane (17 mL) were added bis(dibenzylideneacetone)palladium(0) (99 mg), 5-(di-tert-butylphosphino)-1',3',5'-triphenyl-1'H-1,4'-bipyrazole (174 mg) and cesium hydroxide monohydrate (1.73 g), and the mixture was stirred at 100°C for 5 hr. The reaction mixture was cooled to room temperature, 1 M hydrochloric acid and saturated aqueous sodium hydrogen carbonate solution were added, and the mixture was extracted with ethyl acetate. The organic layer was dried over anhydrous sodium sulfate. Under reduced pressure, the solvent was evaporated and the obtained residue was purified by silica gel column chromatography [elution solvent: chloroform/methanol=100/0-92/8(V/V)] to give the title compound (930 mg, yield: 83%) as an oil.

### (58f) (3'R)-3'-Methyl-4',5'-dihydro-3'H-spiro[cyclopropane-1,2'-pyrido[2,3-f][1,4]oxazepin]-7'-ol hydrochloride

To a solution of (3'R)-4'-(4-methoxybenzyl)-3'-methyl-4',5'-dihydro-3'H-spiro[cyclopropane-1,2'-pyrido[2,3-f][1,4]oxazepin]-7'-ol (430 mg) of Example 58e in trifluoroacetic acid (0.5 mL) was added anisole (1.42 mL), and the mixture was heated under reflux for 16 hr. The reaction mixture was cooled to room temperature, and the residue was azeotropically distilled with toluene. The obtained residue was dissolved in ethyl acetate (9.4 mL), 1 M hydrogen chloride-ethyl acetate (3.9 mL) was added and the mixture was stirred at room temperature for 5 min. To the reaction mixture was added tert-butyl methyl ether, and the solid was collected by filtration to give the title compound (320 mg, yield: quantitative) as a solid.

### (58g) Ethyl 3-(1,4-dimethyl-1H-benzotriazol-5-yl)-3-(7-{[(3'R)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoate

To a solution of ethyl 3-(1,4-dimethyl-1H-benzotriazol-5-yl)-3-[7-(hydroxymethyl)-1-benzothiophen-5-yl]propanoate (200 mg) of Example 1f in dichloromethane (1.6 mL) were added dimethyl sulfoxide (0.17 mL), N,N-diisopropylethylamine (0.26 mL) and sulfur trioxide-pyridine complex (259 mg) and the mixture was stirred at room temperature for 20 min. To the reaction mixture was added water, and the mixture was extracted with chloroform. The organic layer was dried over anhydrous sodium sulfate. The solvent was evaporated under reduced pressure, the obtained residue was dissolved in dichloromethane (1.6 mL). (3'R)-3'-methyl-4',5'-dihydro-3'H-spiro[cyclopropane-1,2'-pyrido[2,3-f][1,4]oxazepin]-7'-ol hydrochloride (178 mg) of Example 58f, N,N-diisopropylethylamine (0.13 mL), anhydrous magnesium sulfate (118 mg) and acetic acid (0.006 mL) were added thereto, and the mixture was stirred at 35°C for 40 min. Then, sodium triacetoxyborohydride (207 mg) was added thereto, and the mixture was stirred at 35°C for 2 hr. To the reaction mixture was added saturated aqueous sodium hydrogen carbonate solution, and the mixture was extracted with chloroform. The organic layer was dried over anhydrous sodium sulfate. Under reduced pressure, the solvent was evaporated and the obtained residue was purified by silica gel column chromatography [elution solvent: n-hexane/ethyl acetate=30/70-0/100(V/V), chloroform/methanol=100/0-90/10(V/V)] to give the title compound (178 mg, yield: 61%) as an oil.

### (58h)=(58)

### (3R*)-3-(1,4-Dimethyl-1H-benzotriazol-5-yl)-3-(7-(3'R)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoic acid

### (59a)=(59)

### (3S*)-3-(1,4-Dimethyl-1H-benzotriazol-5-yl)-3-(7-{[(3'R)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoic acid

To a mixture of ethyl 3-(1,4-dimethyl-1H-benzotriazol-5-yl)-3-(7-{[(3'R)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoate (178 mg) of Example 58g, tetrahydrofuran (2.7 mL) and methanol (0.9 mL) was added 1 M aqueous lithium hydroxide solution (0.89 mL), and the mixture was stirred at 40°C for 1.5 hr. To the reaction mixture was added saturated aqueous ammonium chloride solution, and the mixture was extracted with a mixed solvent of chloroform and methanol. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=30/70-0/100(V/V), chloroform/methanol=100/0-95/5(V/V)]. Ethyl acetate and n-hexane were added to the compound eluted earlier, and an ultrasonic treatment was performed. The solid was collected by filtration to give (3S*)-3-(1,4-dimethyl-1H-benzotriazol-5-yl)-3-(7-{[(3'R)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoic acid (47.5 mg, yield: 28%) as a solid.

In the same manner, moreover, the compound eluted later, (3R')-3-(1,4-dimethyl-1H-benzotriazol-5-yl)-3-(7-{[(3'R)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoic acid (50.1 mg, yield: 30%) as a solid.

### (Example 60)

### (3R')-3-(1,4-Dimethyl-1H-benzotriazol-5-yl)-3-(7-{[(3'S)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoic acid

### (Example 61)

### (3S*)-3-(1,4-Dimethyl-1H-benzotriazol-5-yl)-3-(7-([(3'S)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoic acid

### (60a) Methyl N,N-bis(4-methoxybenzyl)-L-alaninate

To a suspension of methyl L-alaninate hydrochloride (5.10 g) in acetonitrile (90 mL) were added potassium carbonate (15.2 g), 4-methoxybenzyl chloride (9.95 mL) and sodium iodide (548 mg), and the mixture was stirred at 50°C for 3.5 hr. The reaction mixture was cooled to room temperature, insoluble material was filtered off, and washed with ethyl acetate. The filtrate was concentrated under reduced pressure to give the title compound (yield: quantitative) as an oil.

### (60b) 1-{(1S)-1-[Bis(4-methoxybenzyl)amino]ethyl}cyclopropanol

To a suspension of methyl N,N-bis(4-methoxybenzyl)-L-alaninate (6.61 g) of Example 60a in tetrahydrofuran (25 mL) was added tetraisopropyl orthotitanate (1.3 mL), ethylmagnesium bromide (1 M tetrahydrofuran solution) (55 mL) was added dropwise over 20 min at 0°C, and the mixture was stirred at room temperature for 14 hr. To the reaction mixture were added saturated aqueous ammonium chloride solution and ethyl acetate and the mixture was stirred at room temperature for 30 min, and saturated aqueous sodium hydrogen carbonate solution was added. Insoluble material was filtered off through celite, and the filtrate was extracted with ethyl acetate. The organic layer was washed successively with saturated aqueous sodium hydrogen carbonate solution and saturated brine, and dried over anhydrous sodium sulfate. Under reduced pressure, the solvent was evaporated and the obtained residue was purified by silica gel column chromatography [elution solvent: n-hexane/ethyl acetate=95/5-70/30(V/V)] to give the title compound (5.24 g, yield: 78%) as an oil.

### (60c) 1-{(1S)-1-[(4-Methoxybenzyl)amino]ethyl}cyclopropanol

20% Palladium hydroxide-activated carbon (257 mg) was suspended in a small amount of ethyl acetate, a solution of 1-{(1S)-1-[bis(4-methoxybenzyl)amino]ethyl}cyclopropanol (5.14 g) of Example 60b in methanol (130 mL) and acetic acid (26 mL) were added, and the mixture was stirred at room temperature under a hydrogen atmosphere at normal pressure for 30 min. To the reaction mixture was added celite, insoluble material was filtered off, and the filtrate was concentrated under reduced pressure. The obtained residue was azeotropically distilled with toluene and purified by NH silica gel column chromatography [elution solvent: n-hexane/ethyl acetate=100/0-0/100(V/V), chloroform/methanol=100/0-95/5(V/V)] to give the title compound (2.86 g, yield: 93%) as an oil.

### (60d) 1-[(1S)-1-{[(6-Bromo-3-fluoropyridin-2-yl)methyl](4-methoxybenzyl)amino}ethyl]cyclopropanol

To a solution of 6-bromo-2-(bromomethyl)-3-fluoropyridine (1.96 g) of Example 3a, and 1-{(1S)-1-[(4-methoxybenzyl)amino]ethyl}cyclopropanol (1.06 g) of Example 60c in acetonitrile (24 mL) was added potassium carbonate (861 mg), and the mixture was stirred at 50°C for 17.5 hr. The reaction mixture was cooled to room temperature, and insoluble material was filtered off. The filtrate was concentrated under reduced pressure and the obtained residue was purified by silica gel column chromatography [elution solvent: n-hexane/ethyl acetate=95/5-65/35(V/V)] to give the title compound (1.71 g, yield: 87%) as an oil.

### (60e) (3'S)-7'-Bromo-4'-(4-methoxybenzyl)-3'-methyl-4',5'-dihydro-3'H-spiro[cyclopropane-1,2'-pyrido[2,3-f][1,4]oxazepine]

To a suspension of 60% sodium hydride (501 mg) in N,N-dimethylformamide (30 mL) was added a solution of 1-[(1S)-1-{[(6-bromo-3-fluoropyridin-2-yl)methyl](4-methoxybenzyl)amino}ethyl]cyclopropanol (1.71 g) of Example 60d in tetrahydrofuran (30 mL) over 30 min at 60°C, and the mixture was stirred at 60°C for 1.5 hr. The reaction mixture was cooled to room temperature, water was added, and the mixture was extracted with ethyl acetate. The organic layer was washed successively with water and saturated brine, and dried over anhydrous sodium sulfate. Under reduced pressure, the solvent was evaporated and the obtained residue was purified by silica gel column chromatography [elution solvent: n-hexane/ethyl acetate=95/5-65/35(V/V)] to give the title compound (1.48 g, yield: 85%) as an oil.

### (60f) (3'S)-4'-(4-Methoxybenzyl)-3'-methyl-4',5'-dihydro-3'H-spiro[cyclopropane-1,2'-pyrido[2,3-f][1,4]oxazepin]-7'-ol

To a solution of (3'S)-7'-bromo-4'-(4-methoxybenzyl)-3'-methyl-4',5'-dihydro-3'H-spiro[cyclopropane-1,2'-pyrido[2,3-f][1,4]oxazepine] (1.48 g) of Example 60e in 1,4-dioxane (18 mL) were added bis(dibenzylideneacetone)palladium(0) (102 mg), 5-(di-tert-butylphosphino)-1',3',5'-triphenyl-1'H-1,4'-bipyrazole (181 mg) and cesium hydroxide monohydrate (1.80 g), and the mixture was stirred at 100°C for 4.5 hr. The reaction mixture was cooled to room temperature, 2 M hydrochloric acid was added, and the mixture was basified with saturated aqueous sodium hydrogen carbonate solution and extracted with chloroform. The organic layer was dried over anhydrous sodium sulfate. Under reduced pressure, the solvent was evaporated and the obtained residue was purified by silica gel column chromatography [elution solvent: chloroform/methanol=100/0-97/3(V/V)] to give the title compound (1.05 g, yield: 90%) as an oil.

### (60g) (3'S)-3'-Methyl-4',5'-dihydro-3'H-spiro[cyclopropane-1,2'-pyrido[2,3-f][1,4]oxazepin]-7'-ol hydrochloride

To a solution of (3'S)-4'-(4-methoxybenzyl)-3'-methyl-4',5'-dihydro-3'H-spiro[cyclopropane-1,2'-pyrido[2,3-f][1,4]oxazepin]-7'-ol (1.05 g) of Example 60f in trifluoroacetic acid (30 mL) was added anisole (3.5 mL), and the mixture was heated under reflux for 15 hr. The reaction mixture was cooled to room temperature, and the residue was azeotropically distilled with toluene. The obtained residue was dissolved in ethyl acetate (30 mL), 1 M hydrogen chloride-ethyl acetate (9.7 mL) was added and the mixture was stirred at room temperature for 5 min. To the reaction mixture was added tert-butyl methyl ether, and the solid was collected by filtration to give the title compound (793 mg, yield: quantitative) as a solid.

### (60h) Ethyl 3-(1,4-dimethyl-1H-benzotriazol-5-yl)-3-(7-{[(3'S)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoate

To a solution of ethyl 3-(1,4-dimethyl-1H-benzotriazol-5-yl)-3-[7-(hydroxymethyl)-1-benzothiophen-5-yl]propanoate (200 mg) of Example 1f in dichloromethane (1.6 mL) were added dimethyl sulfoxide (0.17 mL), N,N-diisopropylethylamine (0.26 mL) and sulfur trioxide-pyridine complex (259 mg), and the mixture was stirred at room temperature for 30 min. To the reaction mixture was added water, and the mixture was extracted with chloroform. The organic layer was dried over anhydrous sodium sulfate. The solvent was evaporated under reduced pressure and the obtained residue was dissolved in dichloromethane (1.6 mL). (3'S)-3'-methyl-4',5'-dihydro-3'H-spiro[cyclopropane-1,2'-pyrido[2,3-f][1,4]oxazepin]-7'-ol hydrochloride (142 mg) of Example 60g, N,N-diisopropylethylamine (0.10 mL), anhydrous magnesium sulfate (118 mg), and acetic acid (0.006 mL) were added thereto, and the mixture was stirred at 35°C for 30 min. Then, sodium triacetoxyborohydride (166 mg) was added thereto, and the mixture was stirred at 35°C for 19 hr. The reaction mixture was cooled to room temperature, saturated aqueous sodium hydrogen carbonate solution was added, and the mixture was extracted with chloroform. The organic layer was dried over anhydrous sodium sulfate. Under reduced pressure, the solvent was evaporated and the obtained residue was purified by silica gel column chromatography [elution solvent:
chloroform/methanol=100/0-97/3(V/V)] to give the title compound (165 mg, yield: 57%) as an oil.

### (60i)=(60)

### (3R')-3-(1,4-Dimethyl-1H-benzotriazol-5-yl)-3-(7-{[(3'S)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoic acid

### (61a)=(61)

### (3S*)-3-(1,4-Dimethyl-1H-benzotriazol-5-yl)-3-(7-{[(3'S)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoic acid

To a mixture of ethyl 3-(1,4-dimethyl-1H-benzotriazol-5-yl)-3-(7-{[(3'S)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoate (165 mg) of Example 60h, tetrahydrofuran (2.5 mL) and methanol (0.83 mL) was added 1 M aqueous lithium hydroxide solution (0.83 mL), and the mixture was stirred at 40°C for 1 hr. The reaction mixture was cooled to room temperature, saturated aqueous ammonium chloride solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: chloroform/methanol=100/0-95/5(V/V)] to give a low polar compound (51 mg) and a highly-polar compound (35 mg). Similarly, to a mixture of ethyl 3-(1,4-dimethyl-1H-benzotriazol-5-yl)-3-(7-{[(3'S)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoate (145 mg) of Example 60h, tetrahydrofuran (2.2 mL) and methanol (0.73 mL) was added 1 M aqueous lithium hydroxide solution (0.73 mL), and the mixture was stirred at 40°C for 22 hr. The reaction mixture was cooled to room temperature, saturated aqueous ammonium chloride solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: chloroform/methanol=100/0-95/5(V/V)] to give a low polar compound (49 mg) and highly-polar compound (48 mg). The obtained low polar compounds were combined, ethyl acetate and n-hexane were added, and an ultrasonic treatment was performed. The solid was collected by filtration to give (3S*)-3-(1,4-dimethyl-1H-benzotriazol-5-yl)-3-(7-{[(3'S)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoic acid (84.1 mg, yield: 28%) as a solid.

In the same manner, a highly-polar compound, (3R*)-3-(1,4-dimethyl-1H-benzotriazol-5-yl)-3-(7-{[(3'S)-7'-hydroxy-3'-methyl-3'H-spiro[cyclopropane-1,2'-pyrido[2,3-f][1,4]oxazepin]-4'(5'H)-yl]methyl}-1-benzothiophen-5-yl)propanoic acid (71.9 mg, yield: 24%), was also obtained as a solid.

### (Example 62)

### 3-(7-{[(2R)-2-Ethyl-7-hydroxy-2,3-dihydropyrido[2,3-f] [1,4]oxazepin-4(5H)-yl]methyl}-1-benzothiophen-5-yl)-3-{4-methyl-1-[3-(methylsulfonyl)propyl]-1H-benzotriazol-5-yl}propanoic acid

### (62a) 3-Methyl-N-[3-(methylsulfonyl)propyl]-2-nitroaniline

To a solution of 1-fluoro-3-methyl-2-nitrobenzene (1.00 g), potassium carbonate (2.70 g) in ethanol (13 mL) was added 3-methylsulfonylpropane-1-amine hydrochloride (1.41 g), and the mixture was stirred at 70°C for 48 hr. The reaction mixture was cooled to room temperature and concentrated under reduced pressure. Saturated aqueous sodium hydrogen carbonate solution was added to the residue and the mixture was extracted with ethyl acetate, and the organic layer was dried over anhydrous sodium sulfate. After filtration, the filtrate was concentrated under reduced pressure, and the obtained residue was purified by silica gel column chromatography [elution solvent: n-hexane/ethyl acetate=70/30-50/50(V/V)] to give the title compound (410 mg, yield: 23%) as a solid.

### (62b) 4-Bromo-3-methyl-N-[3-(methylsulfonyl)propyl]-2-nitroaniline

To a solution of 3-methyl-N-[3-(methylsulfonyl)propyl]-2-nitroaniline (410 mg) of Example 62a in N,N-dimethylformamide (2.0 mL) was added N-bromosuccinimide (273 mg) at 0°C, and the mixture was stirred at room temperature for 18 hr. To the reaction mixture was added saturated aqueous sodium hydrogen carbonate solution, and the mixture was extracted with ethyl acetate. The organic layer was washed with 10% brine, and dried over anhydrous sodium sulfate. After filtration, the filtrate was concentrated under reduced pressure, and the obtained residue was purified by silica gel column chromatography [elution solvent: n-hexane/ethyl acetate=70/30-20/80(V/V)] to give the title compound (528 mg, yield: quantitative) as a solid.

### (62c) 4-Bromo-3-methyl-N¹-[3-(methylsulfonyl)propyl]benzene-1,2-diamine

Using 4-bromo-3-methyl-N-[3-(methylsulfonyl)propyl]-2-nitroaniline (528 mg) of Example 62b, and by a method similar to that of Example 41e, the title compound (404 mg, yield: 84%) was obtained as a solid.

### (62d) 5-Bromo-4-methyl-1-[3-(methylsulfonyl)propyl]-1H-benzotriazole

Using 4-bromo-3-methyl-N¹-[3-(methylsulfonyl)propyl]benzene-1,2-diamine (404 mg) of Example 62c, and by a method similar to that of Example 41f, the title compound (210 mg, yield: 50%) was obtained as a solid.

### (62e) Ethyl (2E)-3-{4-methyl-1-[3-(methylsulfonyl)propyl]-1H-benzotriazol-5-yl}prop-2-enoate

Using 5-bromo-4-methyl-1-[3-(methylsulfonyl)propyl]-1H-benzotriazole (210 mg) of Example 62d, and by a method similar to that of Example 41g, the title compound (221 mg, yield: quantitative) was obtained as a solid.

### (62f) Ethyl 3-[7-(hydroxymethyl)-1-benzothiophen-5-yl]-3-{4-methyl-1-[3-(methylsulfonyl)propyl]-1H-benzotriazol-5-yl}propanoate

Using ethyl (2E)-3-{4-methyl-1-[3-(methylsulfonyl)propyl]-1H-benzotriazol-5-yl}prop-2-enoate (221 mg) of Example 62e and [5-(4,4,5,5-tetramethyl-1,3,2-dioxaborolan-2-yl)-1-benzothiophen-7-yl]methanol (274 mg) of Example 1e, and by a method similar to that of Example 1f, the title compound (106 mg, yield: 33%) was obtained as an oil.

### (62g) Ethyl 3-(7-formyl-1-benzothiophen-5-yl)-3-{4-methyl-1-[3-(methylsulfonyl)propyl]-1H-benzotriazol-5-yl}propanoate

To a solution of ethyl 3-[7-(hydroxymethyl)-1-benzothiophen-5-yl]-3-{4-methyl-1-[3-(methylsulfonyl)propyl]-1H-benzotriazol-5-yl}propanoate (111 mg) of Example 62f in dichloromethane (2.2 mL) was added manganese(IV) oxide (220 mg), and the mixture was stirred at 40°C for 8 hr. The reaction mixture was cooled to room temperature, and insoluble material was removed by filtration through celite. Under reduced pressure, the solvent was evaporated and the obtained residue was purified by silica gel column chromatography [elution solvent: n-hexane/ethyl acetate=40/60-20/80(V/V)] to give the title compound (86.0 mg, yield: 78%) as an oil.

### (62h) Ethyl 3-(7-{[(2R)-2-ethyl-7-hydroxy-2,3-dihydropyrido[2,3-f] [1,4]oxazepin-4(5H)-yl]methyl}-1-benzothiophen-5-yl)-3-{4-methyl-1-[3-(methylsulfonyl)propyl]-1H-benzotriazol-5-yl}propanoate

To a solution of ethyl 3-(7-formyl-1-benzothiophen-5-yl)-3-{4-methyl-1-[3-(methylsulfonyl)propyl]-1H-benzotriazol-5-yl}propanoate (86.0 mg) of Example 62g, (2R)-2-ethyl-2,3,4,5-tetrahydropyrido[2,3-f][1,4]oxazepin-7-ol hydrochloride (57.9 mg) of Example 1h and N,N-diisopropylethylamine (0.0437 mL) in dichloromethane (2.0 mL) was added acetic acid (0.0863 mL), and the mixture was stirred at room temperature for 4 hr. Then, sodium triacetoxyborohydride (222 mg) was added thereto at 0°C and the mixture was stirred at room temperature for 12 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/100/0-0/95/5(V/V/V)] to give the title compound (88.0 mg, yield: 76%) as an oil.

### (62i) 3-(7-{[(2R)-2-Ethyl-7-hydroxy-2,3-dihydropyrido[2,3-f] [1,4]oxazepin-4(5H)-yl]methyl}-1-benzothiophen-5-yl)-3-{4-methyl-1-[3-(methylsulfonyl)propyl]-1H-benzotriazol-5-yl}propanoic acid

To a mixture of ethyl 3-(7-{[(2R)-2-ethyl-7-hydroxy-2,3-dihydropyrido[2,3-f] [1,4]oxazepin-4(5H)-yl]methyl}-1-benzothiophen-5-yl)-3-{4-methyl-1-[3-(methylsulfonyl)propyl]-1H-benzotriazol-5-yl}propanoate (88.0 mg) of Example 62h, methanol (1.0 mL), and tetrahydrofuran (1.0 mL) was added 1 M aqueous sodium hydroxide solution (0.636 mL), and the mixture was stirred at room temperature for 12 hr. To the reaction mixture was added 1 M hydrochloric acid (0.636 mL) and the mixture was stirred and concentrated under reduced pressure. Saturated aqueous ammonium chloride solution, and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/methanol=30/70/0-0/100/0-0/90/10(V/V/V)]. Ethyl acetate and n-hexane were added to the obtained solid, and an ultrasonic treatment was performed. The solid was collected by filtration and vacuum dried to give the title compound (76.0 mg, yield: 90%) as a solid.

### (Example 63)

### 3-(1,4-Dimethyl-1H-benzotriazol-5-yl)-3-(7-{[(6R)-6-ethyl-5,6,7,9-tetrahydro-8H-pyrido[2,3-c]azepin-8-yl]methyl}-1-benzothiophen-5-yl)propanoic acid

### (63a) tert-Butyl (6R)-6-ethyl-5,6,7,9-tetrahydro-8H-pyrido[2,3-c]azepine-8-carboxylate

To a solution of potassium tert-butoxide (15 g) in N,N-dimethylformamide (200 mL) was added dropwise a solution of 2-[(2-{[(tert-butoxycarbonyl)amino]methyl}pyridin-3-yl)methyl]butyl methanesulfonate (WO 2018/109649) (20.24 g) in N,N-dimethylformamide (200 mL) at 0°C, and the mixture was stirred at 20°C for 12 hr. The reaction mixture was diluted with water (2 L), and extracted three times with ethyl acetate. The combined organic layer was washed twice with saturated brine and dried over anhydrous sodium sulfate. After filtration, the filtrate was concentrated under reduced pressure and the obtained residue was purified by silica gel column chromatography [elution solvent: petroleum ether/ethyl acetate=30/1-5/1(V/V)] and then purified by preparative HPLC [column: Phenomenex Luna C18 (50 mm I.D.× 250 mm), mobile phase: 0.225% aqueous formic acid solution/acetonitrile=80/20-50/50(V/V)] to give the title compound (racemate 12 g). This was subjected to chiral SFC [column: CHIRALPAK IG (30 mm I.D.× 250 mm), mobile phase: carbon dioxide/methanol=65/35(V/V)]. The compound obtained as the first peak was purified by preparative HPLC [column: Phenomenex Luna C18 (70 mm I.D.× 250 mm), mobile phase: 0.225% aqueous formic acid solution/acetonitrile=80/20-50/50(V/V)] to give tert-butyl (6R)-6-ethyl-5,6,7,9-tetrahydro-8H-pyrido[2,3-c]azepine-8-carboxylate (5.25 g, yield: 35%) as a highly viscous oil. retention time: 0.948 min [column: CHIRALPAK IG-3 (4.6 mm I.D.× 50 mm), mobile phase: carbon dioxide/methanol (0.05% diethylamine)=95/5-60/40(V/V), flow rate: 3 mL/min, temperature: 35°C, wavelength: 220 nm]

### (63b) (6R)-6-Ethyl-6,7,8,9-tetrahydro-5H-pyrido[2,3-c]azepine

To a solution of tert-butyl (6R)-6-ethyl-5,6,7,9-tetrahydro-8H-pyrido[2,3-c]azepine-8-carboxylate (553 mg) of Example 63a in dichloromethane (8.0 mL) was added trifluoroacetic acid (2.00 mL), and the mixture was stirred at room temperature for 4 hr. The reaction mixture was diluted with toluene and the solvent was evaporated under reduced pressure. The obtained residue was purified by NH silica gel column chromatography [elution solvent: n-hexane/ethyl acetate=50/50/0-0/100(V/V)] to give the title compound (353 mg, yield: 100%) as an oil.

### (63c) Ethyl 3-(1,4-dimethyl-1H-benzotriazol-5-yl)-3-(7-{[(6R)-6-ethyl-5,6,7,9-tetrahydro-8H-pyrido[2,3-c]azepin-8-yl]methyl}-1-benzothiophen-5-yl)propanoate

To a solution of ethyl 3-(1,4-dimethyl-1H-benzotriazol-5-yl)-3-(7-formyl-1-benzothiophen-5-yl)propanoate (40.7 mg) of Example 57b, and (6R)-6-ethyl-6,7,8,9-tetrahydro-5H-pyrido[2,3-c]azepine (26.4 mg) of Example 63b in dichloromethane (2.0 mL) was added acetic acid (0.515 mL), and the mixture was stirred at room temperature for 3 hr. Then, sodium triacetoxyborohydride (132 mg) was added thereto at 0°C and the mixture was stirred at room temperature for 12 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was successively purified by silica gel column chromatography [elution solvent: n-hexane/ethyl acetate=60/40-30/70(V/V)] and NH silica gel column chromatography [elution solvent: n-hexane/ethyl acetate=70/30-40/60(V/V)] to give the title compound (19.0 mg, yield: 34%) as an oil.

### (63d)=(63)

### 3-(1,4-Dimethyl-1H-benzotriazol-5-yl)-3-(7-{[(6R)-6-ethyl-5,6,7,9-tetrahydro-8H-pyrido[2,3-c]azepin-8-yl]methyl}-1-benzothiophen-5-yl)propanoic acid

To a mixture of ethyl 3-(1,4-dimethyl-1H-benzotriazol-5-yl)-3-(7-{[(6R)-6-ethyl-5,6,7,9-tetrahydro-8H-pyrido[2,3-c]azepin-8-yl]methyl}-1-benzothiophen-5-yl)propanoate (19.0 mg) of Example 63c, methanol (1.0 mL), and tetrahydrofuran (1.0 mL) was added 1 M aqueous sodium hydroxide solution (0.335 mL) and the mixture was stirred at room temperature for 24 hr. To the reaction mixture was added 1 M hydrochloric acid (0.335 mL), and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/methanol=30/70/0-0/100/0-0/80/20(V/V/V)]. Ethyl acetate and n-hexane were added to the obtained solid, and an ultrasonic treatment was performed. The solid was collected by filtration and vacuum dried to give the title compound (17.0 mg, yield: 94%) as a solid.

### (Example 64)

### 3-(1,4-Dimethyl-1H-benzotriazol-5-yl)-3-(7-{[(6S)-6-ethyl-5,6,7,9-tetrahydro-8H-pyrido[2,3-c]azepin-8-yl]methyl}-1-benzothiophen-5-yl)propanoic acid

### (64a) tert-Butyl (6S)-6-ethyl-5,6,7,9-tetrahydro-8H-pyrido[2,3-c]azepine-8-carboxylate

The compound obtained as the second peak in Example 63a was also treated in the same manner to obtain tert-butyl (6S)-6-ethyl-5,6,7,9-tetrahydro-8H-pyrido[2,3-c]azepine-8-carboxylate (4.71 g, yield: 30%) as a highly viscous oil. retention time: 1.338 min

### (64b) (6S)-6-Ethyl-6,7,8,9-tetrahydro-5H-pyrido[2,3-c]azepine

To a solution of tert-butyl (6S)-6-ethyl-5,6,7,9-tetrahydro-8H-pyrido[2,3-c]azepine-8-carboxylate (553 mg) of Example 64a in dichloromethane (8.0 mL) was added trifluoroacetic acid (2.00 mL), and the mixture was stirred at room temperature for 4 hr. The reaction mixture was diluted with toluene, and the solvent was evaporated under reduced pressure. The obtained residue was purified by NH silica gel column chromatography [elution solvent: n-hexane/ethyl acetate=50/50-0/100(V/V)] to give the title compound (318 mg, yield: 90%) as an oil.

### (64c) Ethyl 3-(1,4-dimethyl-1H-benzotriazol-5-yl)-3-(7-{[(6S)-6-ethyl-5,6,7,9-tetrahydro-8H-pyrido[2,3-c]azepin-8-yl]methyl}-1-benzothiophen-5-yl)propanoate

To a solution of ethyl 3-(1,4-dimethyl-1H-benzotriazol-5-yl)-3-(7-formyl-1-benzothiophen-5-yl)propanoate (40.7 mg) of Example 57b and (6S)-6-ethyl-6,7,8,9-tetrahydro-5H-pyrido[2,3-c]azepine (26.4 mg) of Example 64b in dichloromethane (2.0 mL) was added acetic acid (0.515 mL), and the mixture was stirred at room temperature for 3 hr. Then, sodium triacetoxyborohydride (132 mg) was added thereto at 0°C, and the mixture was stirred at room temperature for 12 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=60/40-30/70(V/V)] to give the title compound (11.0 mg, yield: 19%) as an oil.

### (64d)=(64)

### 3-(1,4-Dimethyl-1H-benzotriazol-5-yl)-3-(7-{[(6S)-6-ethyl-5,6,7,9-tetrahydro-8H-pyrido[2,3-c]azepin-8-yl]methyl}-1-benzothiophen-5-yl)propanoic acid

To a mixture of ethyl 3-(1,4-dimethyl-1H-benzotriazol-5-yl)-3-(7-{[(6S)-6-ethyl-5,6,7, 9-tetrahydro-8H-pyrido[2, 3-c]azepin-8-yl]methyl}-1-benzothiophen-5-yl)propanoate (11.0 mg) of Example 64c, methanol (1.0 mL), and tetrahydrofuran (1.0 mL) was added 1 M aqueous sodium hydroxide solution (0.387 mL), and the mixture was stirred at room temperature for 24 hr. To the reaction mixture was added 1 M hydrochloric acid (0.387 mL), and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/methanol=30/70/0-0/100/0-0/80/20(V/V/V)]. Ethyl acetate and n-hexane were added to the obtained solid, and an ultrasonic treatment was performed. The solid was collected by filtration and vacuum dried to give the title compound (10.0 mg, yield: 96%) as a solid.

### (Example 65)

### (3R*)-3-(1,4-Dimethyl-1H-benzotriazol-5-yl)-3-(7-{[(2R)-2-ethyl-8-hydroxy-2,3-dihydropyrido[2,3-f][1,4]oxazepin-4(5H)-yl]methyl}-1-benzothiophen-5-yl)propanoic acid

### (Example 66)

### (3S*)-3-(1,4-Dimethyl-1H-benzotriazol-5-yl)-3-(7-{[(2R)-2-ethyl-8-hydroxy-2,3-dihydropyrido[2,3-f][1,4]oxazepin-4(5H)-yl]methyl}-1-benzothiophen-5-yl)propanoic acid

### (65a) (2R)-1-[(4-Methoxybenzyl)amino]butan-2-ol

To a solution of (2R)-1-aminobutan-2-ol (1.16 g) of Example 17a and 4-methoxybenzaldehyde (0.79 mL) in dichloromethane (26 mL) were added anhydrous magnesium sulfate (1.56 g) and one drop of acetic acid, and the mixture was stirred at room temperature for 45 min. Sodium triacetoxyborohydride (2.76 g) was added thereto, and the mixture was stirred at room temperature for 80 min. To the reaction mixture was added saturated aqueous sodium hydrogen carbonate solution, and the mixture was extracted with chloroform. The organic layer was dried over anhydrous sodium sulfate. The solvent was evaporated under reduced pressure to give a mixture containing the title compound (1.48 g) as an oil.

### (65b) (2R)-1-{[(5-Bromo-3-fluoropyridin-2-yl)methyl](4-methoxybenzyl)amino}butan-2-ol

To a solution of (5-bromo-3-fluoropyridin-2-yl)methanol (WO 2014/008223) (2.92 g) in dichloromethane (55 mL) were added dimethyl sulfoxide (5.0 mL) and N,N-diisopropylethylamine (7.4 mL), sulfur trioxide-pyridine complex (7.52 g) was added at 0°C, and the mixture was stirred at room temperature for 30 min. To the reaction mixture was added water, and the mixture was extracted with chloroform, and the organic layer was dried over anhydrous sodium sulfate. The solvent was evaporated under reduced pressure to give a residue (3.76 g). To a solution of the obtained residue (1.88 g) and (2R)-1-[(4-methoxybenzyl)amino]butan-2-ol (1.78 g) of Example 65a in dichloromethane (25 mL) were added anhydrous magnesium sulfate (1.71 g) and acetic acid (0.081 mL), and the mixture was stirred at room temperature for 30 min. Sodium triacetoxyborohydride (3.01 g) was added thereto, and the mixture was stirred at room temperature for 1 hr. To the reaction mixture was added saturated aqueous sodium hydrogen carbonate solution, and the mixture was extracted with chloroform, and the organic layer was dried over anhydrous sodium sulfate. The solvent was evaporated under reduced pressure and the obtained residue was purified by NH silica gel column chromatography [elution solvent: n-hexane/ethyl acetate=100/0-85/15(V/V)] to give the title compound (2.46 g, yield: 81%) as an oil.

### (65c) (2R)-8-Bromo-2-ethyl-4-(4-methoxybenzyl)-2,3,4,5-tetrahydropyrido[2,3-f][1,4]oxazepine

To a suspension of 60% sodium hydride (685 mg) in N,N-dimethylformamide (40 mL) was added dropwise over 8 min a solution of (2R)-1-{[(5-bromo-3-fluoropyridin-2-yl)methyl](4-methoxybenzyl)amino}butan-2-ol (2.46 g) of Example 65b in tetrahydrofuran (40 mL) at 60°C, and the mixture was stirred at 60°C for 1.5 hr. The reaction mixture was cooled to room temperature, 60% sodium hydride (685 mg) was added, and the mixture was stirred at 60°C for 12 hr. The reaction mixture was cooled to room temperature, water was added, and the mixture was extracted with ethyl acetate. The organic layer was washed with water and saturated brine, and dried over anhydrous sodium sulfate. Under reduced pressure, the solvent was evaporated and the obtained residue was purified by silica gel column chromatography [elution solvent: n-hexane/ethyl acetate=90/10-60/40(V/V)] to give the title compound (1.76 g, yield: 82%) as an oil.

### (65d) (2R)-2-Ethyl-2,3,4,5-tetrahydropyrido[2,3-f][1,4]oxazepin-8-ol hydrochloride

To a solution of (2R)-8-bromo-2-ethyl-4-(4-methoxybenzyl)-2,3,4,5-tetrahydropyrido[2,3-f] [1,4]oxazepine (1.00 g) of Example 65c in 1,4-dioxane (8.8 mL) were added bis(pinacolato)diboron (808 mg), potassium acetate (780 mg) and [1,1'-bis(diphenylphosphino)ferrocene]dichloropalladium(II) dichloromethane adduct (216 mg), and the mixture was stirred at 90°C for 2.5 hr. The reaction mixture was cooled to room temperature, water was added, and the mixture was extracted with ethyl acetate. The organic layer was washed with saturated brine and dried over anhydrous sodium sulfate. The solvent was evaporated under reduced pressure and the obtained residue was dissolved in tetrahydrofuran (26 mL). 0.5 M aqueous sodium hydroxide solution (26 mL) was added thereto, 30% aqueous hydrogen peroxide solution (2.6 mL) was added dropwise, and the mixture was stirred at room temperature for 0.5 hr. To the reaction mixture were added 1 M hydrochloric acid (5.8 mL) and saturated aqueous ammonium chloride solution, and the mixture was extracted with ethyl acetate. The organic layer was washed with saturated brine, dried over anhydrous sodium sulfate, and concentrated under reduced pressure. The obtained residue was purified by silica gel column chromatography [elution solvent: chloroform/methanol=100/0-92/8(V/V)]. The obtained residue was dissolved in trifluoroacetic acid (18 mL), anisole (2.9 mL) was added, and the mixture was heated under reflux for 21 hr. The reaction mixture was cooled to room temperature, and the residue was azeotropically distilled with toluene. The obtained residue was dissolved in ethyl acetate (18 mL), 1 M hydrogen chloride-ethyl acetate (8.0 mL) was added and the mixture was stirred at room temperature for 5 min. To the reaction mixture was added ethyl acetate, and the solid was collected by filtration to give the title compound (539 mg, yield: 88%) as a solid.

### (65e) Ethyl 3-(1,4-dimethyl-1H-benzotriazol-5-yl)-3-(7-{[(2R)-2-ethyl-8-hydroxy-2,3-dihydropyrido[2,3-f][1,4]oxazepin-4(5H)-yl]methyl}-1-benzothiophen-5-yl)propanoate

To a solution of ethyl 3-(1,4-dimethyl-1H-benzotriazol-5-yl)-3-[7-(hydroxymethyl)-1-benzothiophen-5-yl]propanoate (240 mg) of Example 1f in dichloromethane (2.3 mL) were added dimethyl sulfoxide (0.21 mL), N,N-diisopropylethylamine (0.31 mL) and sulfur trioxide-pyridine complex (311 mg), and the mixture was stirred at room temperature for 20 min. To the reaction mixture was added water, and the mixture was extracted with ethyl acetate. The organic layer was washed with saturated brine and dried over anhydrous sodium sulfate. The solvent was evaporated under reduced pressure and the obtained residue was dissolved in dichloromethane (2.3 mL). (2R)-2-ethyl-2,3,4,5-tetrahydropyrido[2,3-f][1,4]oxazepin-8-ol hydrochloride (162 mg) of Example 65d, N,N-diisopropylethylamine (0.12 mL), anhydrous magnesium sulfate (141 mg) and acetic acid (0.007 mL) were added thereto, and the mixture was stirred at 35°C for 30 min. Sodium triacetoxyborohydride (248 mg) was added thereto, and the mixture was stirred at 35°C for 1.5 hr. The reaction mixture was cooled to room temperature, saturated aqueous sodium hydrogen carbonate solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V)] to give the title compound (225 mg, yield: 66%) as a solid.

### (65f)=(65)

### (3R*)-3-(1,4-Dimethyl-1H-benzotriazol-5-yl)-3-(7-{[(2R)-2-ethyl-8-hydroxy-2,3-dihydropyrido[2,3-f][1,4]oxazepin-4(5H)-yl]methyl}-1-benzothiophen-5-yl)propanoic acid

### (66a)=(66)

### (3S')-3-(1,4-Dimethyl-1H-benzotriazol-5-yl)-3-(7-{[(2R)-2-ethyl-8-hydroxy-2,3-dihydropyrido[2,3-f][1,4]oxazepin-4(5H)-yl]methyl}-1-benzothiophen-5-yl)propanoic acid

To a mixture of ethyl 3-(1,4-dimethyl-1H-benzotriazol-5-yl)-3-(7-{[(2R)-2-ethyl-8-hydroxy-2,3-dihydropyrido[2,3-f] [1,4]oxazepin-4(5H)-yl]methyl}-1-benzothiophen-5-yl)propanoate (225 mg) of Example 65e, tetrahydrofuran (3.6 mL) and methanol (1.2 mL) was added 1 M aqueous lithium hydroxide solution (1.2 mL), and the mixture was stirred at 40°C for 1.5 hr. The reaction mixture was cooled to room temperature, 1 M hydrochloric acid and saturated aqueous ammonium chloride solution were added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: chloroform/methanol=99/1-97/3(V/V)]. To the compound eluted earlier were added ethyl acetate and n-hexane, and an ultrasonic treatment was performed. The solid was collected by filtration to give (3S*)-3-(1,4-dimethyl-1H-benzotriazol-5-yl)-3-(7-{[(2R)-2-ethyl-8-hydroxy-2,3-dihydropyrido[2,3-f][1,4]oxazepin-4(5H)-yl]methyl}-1-benzothiophen-5-yl)propanoic acid (80.5 mg, yield: 38%) as a solid.

In the same manner, moreover, the compound eluted later, (3R*)-3-(1,4-dimethyl-1H-benzotriazol-5-yl)-3-(7-{[(2R)-2-ethyl-8-hydroxy-2,3-dihydropyrido[2,3-f][1,4]oxazepin-4(5H)-yl]methyl}-1-benzothiophen-5-yl)propanoic acid (71.0 mg, yield: 33%) was obtained as a solid.

### (Example 67)

### (3R)-3-(7-{[(2R)-7-Amino-2-ethyl-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid

### (67a) tert-Butyl (2R)-7-[(tert-butoxycarbonyl)amino]-2-ethyl-2,3-dihydropyrido[2,3-f][1,4]oxazepine-4(5H)-carboxylate

A solution of tert-butyl (2R)-7-bromo-2-ethyl-2,3-dihydropyrido[2,3-f][1,4]oxazepine-4(5H)-carboxylate (2.58 g) of Example 55d, tert-butyl carbamate (1.69 g), cesium carbonate (4.71 g), palladium acetate (0.324 g) and 4,5-bis(diphenylphosphino)-9,9-dimethylxanthene (0.836 g) in 1,4-dioxane (100 mL) was stirred at 100°C for 6 hr. After cooling to room temperature, the reaction mixture was poured into water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=95/5-85/15(V/V)] to give the title compound (0.96 g, yield: 34%) as a solid.

### (67b) Ethyl 3-[7-(chloromethyl)-1-benzothiophen-5-yl]-3-(3,7-dimethyl-3H-[1,2,3]triazolo[4,5-b]pyridin-6-yl)propanoate

To a solution of ethyl 3-(3,7-dimethyl-3H-[1,2,3]triazolo[4,5-b]pyridin-6-yl)-3-[7-(hydroxymethyl)-1-benzothiophen-5-yl]propanoate (420 mg) of Example 9a in dichloromethane (5 mL) was added thionyl chloride (0.187 mL), and the mixture was stirred at room temperature for 1 hr. The solvent was evaporated under reduced pressure, and the residue was azeotropically distilled with toluene. The solvent was evaporated under reduced pressure to give the title compound (438 mg, yield: 99%) as an oil.

### (67c) Ethyl (3R)-3-(7-{[(2R)-7-amino-2-ethyl-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoate

tert-Butyl (2R)-7-[(tert-butoxycarbonyl)amino]-2-ethyl-2,3-dihydropyrido[2,3-f][1,4]oxazepine-4(5H)-carboxylate (260 mg) of Example 67a was dissolved in dichloromethane (2 mL), trifluoroacetic acid (2 mL) was added, and the mixture was stirred at room temperature for 1 hr. The solvent was evaporated under reduced pressure and the obtained residue was dissolved in acetonitrile (5 mL) solution. Ethyl 3-[7-(chloromethyl)-1-benzothiophen-5-yl]-3-(3,7-dimethyl-3H-[1,2,3]triazolo[4,5-b]pyridin-6-yl)propanoate (430 mg) of Example 67b was dissolved in acetonitrile (5 mL), N,N-diisopropylethylamine (1.4 mL) was added, and the mixture was stirred at 85°C for 15 hr and cooled to room temperature. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=25/75/0-20/80/0-0/95/5(V/V/V)] to give two compounds. Ethyl acetate and n-hexane were added to the compound eluted earlier and the mixture was subjected to an ultrasonic treatment. The solid was collected by filtration and vacuum dried to give the title compound (97 mg, yield: 21%) as a solid.

### (67d)=(67)

### (3R)-3-(7-{[(2R)-7-Amino-2-ethyl-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid

To a mixture of ethyl (3R)-3-(7-{[(2R)-7-amino-2-ethyl-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoate (97 mg) of Example 67c, ethanol (1 mL), and tetrahydrofuran (1 mL) was added 1 M aqueous sodium hydroxide solution (0.83 mL), and the mixture was stirred at room temperature for 12 hr. To the reaction mixture was added 1 M hydrochloric acid (0.83 mL), and the mixture was stirred and extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=45/45/10-38/38/25(V/V/V)] to give the title compound (89 mg, yield: 96%) as a solid.

### (Example 68)

### (3S)-3-(7-{[(2R)-7-Amino-2-ethyl-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid

### (68a) Ethyl (3S)-3-(7-{[(2R)-7-amino-2-ethyl-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoate

tert-Butyl (2R)-7-[(tert-butoxycarbonyl)amino]-2-ethyl-2,3-dihydropyrido[2,3-f][1,4]oxazepine-4(5H)-carboxylate (260 mg) of Example 67a was dissolved in dichloromethane (2 mL), trifluoroacetic acid (2 mL) was added, and the mixture was stirred at room temperature for 1 hr. The solvent was evaporated under reduced pressure and the obtained residue was dissolved in acetonitrile (5 mL) solution. Ethyl 3-[7-(chloromethyl)-1-benzothiophen-5-yl]-3-(3,7-dimethyl-3H-[1,2,3]triazolo[4,5-b]pyridin-6-yl)propanoate (430 mg) of Example 67b was dissolved in acetonitrile (5 mL), N,N-diisopropylethylamine (1.4 mL) was added, and the mixture was stirred at 85°C for 15 hr and cooled to room temperature. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=25/75/0-20/80/0-0/95/5(V/V/V)] to give two compounds. Ethyl acetate and n-hexane were added to the compound eluted later, and an ultrasonic treatment was performed. The solid was collected by filtration and vacuum dried to give the title compound (78 mg, yield: 17%) as a solid.

### (68b)=(68)

### (3S)-3-(7-{[(2R)-7-Amino-2-ethyl-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid

To a mixture of ethyl (3S)-3-(7-{[(2R)-7-amino-2-ethyl-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoate (78 mg) of Example 68a, ethanol (1 mL), and tetrahydrofuran (1 mL) was added 1 M aqueous sodium hydroxide solution (0.67 mL), and the mixture was stirred at room temperature for 12 hr. To the reaction mixture was added 1 M hydrochloric acid (0.67 mL), and the mixture was stirred and extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=45/45/10-38/38/25(V/V/V)] to give the title compound (59 mg, yield: 79%) as a solid.

### (Example 69)

### (3R)-3-(1,4-Dimethyl-1H-benzotriazol-5-yl)-3-(7-{[(6S)-6-ethyl-5,6,7,9-tetrahydro-8H-pyrido[2,3-c]azepin-8-yl]methyl}-1-benzothiophen-5-yl)propanoic acid

### (69a) (6S)-6-Ethyl-6,7,8,9-tetrahydro-5H-pyrido[2,3-c]azepine dihydrochloride

To a solution of tert-butyl (6S)-6-ethyl-5,6,7,9-tetrahydro-8H-pyrido[2,3-c]azepine-8-carboxylate (1.00 g) of Example 64a in 1,4-dioxane (3.6 mL) was added 4 M hydrogen chloride-1,4-dioxane solution (9.05 mL) at 0°C, and the mixture was stirred at room temperature for 2 hr. To the reaction mixture was added diethyl ether, and the resulting solid was collected by filtration to give the title compound (902 mg, yield: quantitative) as a solid.

### (69b) Ethyl (3R)-3-(1,4-dimethyl-1H-benzotriazol-5-yl)-3-(7-{[(6S)-6-ethyl-5,6,7,9-tetrahydro-8H-pyrido[2,3-c]azepin-8-yl]methyl}-1-benzothiophen-5-yl)propanoate

To a solution of ethyl (3R)-3-(1,4-dimethyl-1H-benzotriazol-5-yl)-3-[7-(hydroxymethyl)-1-benzothiophen-5-yl]propanoate (50.0 mg) of Example 451 in dichloromethane (2.0 mL) was added thionyl chloride (0.0177 mL), and the mixture was stirred at room temperature for 1 hr. The solvent was evaporated under reduced pressure, and the residue was azeotropically distilled with toluene. The obtained residue was dissolved in acetonitrile (1.0 mL), added to a separately-prepared solution of N,N-diisopropylethylamine (0.170 mL) and (6S)-6-ethyl-6,7,8,9-tetrahydro-5H-pyrido[2,3-c]azepine dihydrochloride (30.4 mg) of Example 69a in acetonitrile (1.0 mL), and the mixture was stirred at 80°C for 4 hr and cooled to room temperature. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=60/40-30/70(V/V)] to give the title compound (69.0 mg, yield: quantitative) as an oil.

### (69c)=(69)

### (3R)-3-(1,4-Dimethyl-1H-benzotriazol-5-yl)-3-(7-{[(6S)-6-ethyl-5,6,7,9-tetrahydro-8H-pyrido[2,3-c]azepin-8-yl]methyl}-1-benzothiophen-5-yl)propanoic acid

To a mixture of ethyl (3R)-3-(1,4-dimethyl-1H-benzotriazol-5-yl)-3-(7-{[(6S)-6-ethyl-5,6,7,9-tetrahydro-8H-pyrido[2,3-c]azepin-8-yl]methyl}-1-benzothiophen-5-yl)propanoate (69.0 mg) of Example 69b, methanol (1.0 mL), and tetrahydrofuran (1.0 mL) was added 1 M aqueous sodium hydroxide solution (0.608 mL), and the mixture was stirred at room temperature for 6 hr. To the reaction mixture was added 1 M hydrochloric acid (0.608 mL) and the mixture was stirred and concentrated under reduced pressure. Saturated aqueous ammonium chloride solution and saturated brine were added, and the mixture was extracted with chloroform/2-propanol=3/1(V/V). The organic layer was dried over anhydrous sodium sulfate. After filtration, the solvent was evaporated under reduced pressure and the obtained residue was purified by silica gel column chromatography [elution solvent: n-hexane/ethyl acetate/dichloromethane/methanol=30/70/0/0-0/100/0/0-0/0/100/0-0/0/90/10(V/V/V/V)]. Ethyl acetate and n-hexane were added to the obtained solid, and an ultrasonic treatment was performed. The solid was collected by filtration and vacuum dried to give the title compound (55.0 mg, yield: 84%) as a solid.

### (Example 70)

### (3S)-3-(1,4-Dimethyl-1H-benzotriazol-5-yl)-3-(7-{[(6S)-6-ethyl-5,6,7,9-tetrahydro-8H-pyrido[2,3-c]azepin-8-yl]methyl}-1-benzothiophen-5-yl)propanoic acid

### (70a) Ethyl (3S)-3-(1,4-dimethyl-1H-benzotriazol-5-yl)-3-[7-(hydroxymethyl)-1-benzothiophen-5-yl]propanoate

Ethyl 3-(1,4-dimethyl-1H-benzotriazol-5-yl)-3-[7-(hydroxymethyl)-1-benzothiophen-5-yl]propanoate (120 g) of Example 1f was subjected to chiral SFC [column: CHIRALPAK AD (50 mm I.D.× 250 mm), mobile phase: carbon dioxide/ethanol=60/40(V/V)] to give the title compound (51.0 g) as the first peak (yield: 43%).
retention time: 2.078 min

### (70b) Ethyl (3S)-3-(1,4-dimethyl-1H-benzotriazol-5-yl)-3-(7-{[(6S)-6-ethyl-5,6,7,9-tetrahydro-8H-pyrido[2,3-c]azepin-8-yl]methyl}-1-benzothiophen-5-yl)propanoate

To a solution of ethyl (3S)-3-(1,4-dimethyl-1H-benzotriazol-5-yl)-3-[7-(hydroxymethyl)-1-benzothiophen-5-yl]propanoate (50.0 mg) of Example 70a in dichloromethane (2.0 mL) was added thionyl chloride (0.0177 mL), and the mixture was stirred at room temperature for 1 hr. The solvent was evaporated under reduced pressure, and the residue was azeotropically distilled with toluene. The obtained residue was dissolved in acetonitrile (1.0 mL), added to a separately-prepared solution of N,N-diisopropylethylamine (0.170 mL) and (6S)-6-ethyl-6,7,8,9-tetrahydro-5H-pyrido[2,3-c]azepine dihydrochloride (30.4 mg) of Example 69a in acetonitrile (1.0 mL), and the mixture was stirred at 80°C for 4 hr and cooled to room temperature. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=60/40-30/70(V/V)] to give the title compound (69.0 mg, yield: quantitative) as an oil.

### (70c)=(70)

### (3S)-3-(1,4-Dimethyl-1H-benzotriazol-5-yl)-3-(7-{[(6S)-6-ethyl-5,6,7,9-tetrahydro-8H-pyrido[2,3-c]azepin-8-yl]methyl}-1-benzothiophen-5-yl)propanoic acid

To a mixture of ethyl (3S)-3-(1,4-dimethyl-1H-benzotriazol-5-yl)-3-(7-{[(6S)-6-ethyl-5,6,7,9-tetrahydro-8H-pyrido[2,3-c]azepin-8-yl]methyl}-1-benzothiophen-5-yl)propanoate (69.0 mg) of Example 70b, methanol (1.0 mL), and tetrahydrofuran (1.0 mL) was added 1 M aqueous sodium hydroxide solution (0.608 mL), and the mixture was stirred at room temperature for 6 hr. To the reaction mixture was added 1 M hydrochloric acid (0.608 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the solvent was evaporated under reduced pressure and the obtained residue was purified by silica gel column chromatography [elution solvent: n-hexane/ethyl acetate/dichloromethane/methanol=30/70/0/0-0/100/0/0-0/0/100/0-0/0/90/10(V/V/V/V)]. Ethyl acetate and n-hexane were added to the obtained solid, and an ultrasonic treatment was performed. The solid was collected by filtration and vacuum dried to give the title compound (56.0 mg, yield: 85%) as a solid.

### (Example 71)

### 3-{7-[(7'-Amino-3'H-spiro[cyclopropane-1,2'-pyrido[2,3-f][1,4]oxazepin]-4'(5'H)-yl)methyl]-1-benzothiophen-5-yl}-3-(3,7-dimethyl-3H-[1,2,3]triazolo[4,5-b]pyridin-6-yl)propanoic acid

### (71a) 4'-(4-Methoxybenzyl)-4',5'-dihydro-3'H-spiro[cyclopropane-1,2'-pyrido[2,3-f][1,4]oxazepin]-7'-amine

To a solution of 7'-bromo-4'-(4-methoxybenzyl)-4',5'-dihydro-3'H-spiro[cyclopropane-1,2'-pyrido[2,3-f][1,4]oxazepine] (3.00 g) of Example 3e in toluene (38 mL) were added benzophenone imine (1.55 mL), (±)-2,2'-bis(diphenylphosphino)-1,1'-binaphthyl (959 mg), sodium tert-butoxide (1.48 g) and tris(dibenzylideneacetone)dipalladium(0) (703 mg), and the mixture was stirred at 80°C for 40 min. The reaction mixture was cooled to room temperature, the solvent was evaporated under reduced pressure and the obtained residue was dissolved in tetrahydrofuran (38 mL). 2 M Hydrochloric acid (38 mL) was added thereto, and the mixture was stirred at room temperature for 15 min. To the reaction mixture was added saturated aqueous sodium hydrogen carbonate solution, and the mixture was extracted with ethyl acetate. The organic layer was washed with saturated brine and dried over anhydrous sodium sulfate. The solvent was evaporated under reduced pressure and the obtained residue was purified by NH silica gel column chromatography [elution solvent: n-hexane/ethyl acetate/chloroform=90/10/15-0/100/15(V/V/V)] to give the title compound (2.09 g, yield: 87%) as an oil.

### (71b) 2,2,2-Trifluoro-N-[4'-(4-methoxybenzyl)-4',5'-dihydro-3'H-spiro[cyclopropane-1,2'-pyrido[2,3-f][1,4]oxazepin]-7'-yl]acetamide

To a suspension of 4'-(4-methoxybenzyl)-4',5'-dihydro-3'H-spiro[cyclopropane-1,2'-pyrido[2,3-f][1,4]oxazepin]-7'-amine (2.09 g) of Example 71a in tetrahydrofuran (45 mL) was added triethylamine (1.86 mL) at 0°C, trifluoroacetic anhydride (1.03 mL) was added dropwise, and the mixture was stirred at room temperature for 20 min. To the reaction mixture was added water, and the mixture was extracted with ethyl acetate. The organic layer was washed with water, saturated aqueous sodium hydrogen carbonate solution and saturated brine, and dried over anhydrous sodium sulfate. The solvent was evaporated under reduced pressure to give the title compound (2.85 g, yield: quantitative) as an oil.

### (71c) N-(4',5'-Dihydro-3'H-spiro[cyclopropane-1,2'-pyrido[2,3-f][1,4]oxazepin]-7'-yl)-2,2,2-trifluoroacetamide trifluoroacetate (1:1)

To a solution of 2,2,2-trifluoro-N-[4'-(4-methoxybenzyl)-4',5'-dihydro-3'H-spiro[cyclopropane-1,2'-pyrido[2,3-f][1,4]oxazepin]-7'-yl]acetamide (2.85 g) of Example 71b in trifluoroacetic acid (67 mL) was added anisole (7.26 mL), and the mixture was heated under reflux for 15.5 hr. The reaction mixture was cooled to room temperature, and the residue was azeotropically distilled with toluene and the obtained residue was purified by silica gel column chromatography [elution solvent: chloroform/methanol=100/0-90/10(V/V)] to give the title compound (2.01 g, yield: 75%) as a solid.

### (71d) Ethyl 3-(3,7-dimethyl-3H-[1,2,3]triazolo[4,5-b]pyridin-6-yl)-3-(7-{[7'-(2,2,2-trifluoroacetamido)-3'H-spiro[cyclopropane-1,2'-pyrido[2,3-f][1,4]oxazepin]-4'(5'H)-yl]methyl}-1-benzothiophen-5-yl)propanoate

To a solution of ethyl 3-(3,7-dimethyl-3H-[1,2,3]triazolo[4,5-b]pyridin-6-yl)-3-[7-(hydroxymethyl)-1-benzothiophen-5-yl]propanoate (170 mg) of Example 9a in dichloromethane (2.1 mL) were added dimethyl sulfoxide (0.15 mL), N,N-diisopropylethylamine (0.22 mL) and sulfur trioxide-pyridine complex (219 mg), and the mixture was stirred at room temperature for 20 min. To the reaction mixture was added water, and the mixture was extracted with ethyl acetate. The organic layer was washed with saturated brine and dried over anhydrous sodium sulfate. The solvent was evaporated under reduced pressure and the obtained residue was dissolved in dichloromethane (2.1 mL). N-(4',5'-dihydro-3'H-spiro[cyclopropane-1,2'-pyrido[2,3-f][1,4]oxazepin]-7'-yl)-2,2,2-trifluoroacetamide trifluoroacetate (1:1) (199 mg) of Example 71c, N,N-diisopropylethylamine (0.17 mL), acetic acid (0.17 mL) and anhydrous magnesium sulfate (100 mg) were added, and the mixture was stirred at 35°C for 26 min. Then, sodium triacetoxyborohydride (175 mg) was added thereto, and the mixture was stirred at 35°C for 1 hr. The reaction mixture was cooled to room temperature, saturated aqueous sodium hydrogen carbonate solution was added, and the mixture was extracted with chloroform. The organic layer was dried over anhydrous sodium sulfate. Under reduced pressure, the solvent was evaporated and the obtained residue was purified by silica gel column chromatography [elution solvent: n-hexane/ethyl acetate=90/10-20/80(V/V)] to give the title compound (189 mg, yield: 67%) as a solid.

### (71e)=(71)

### 3-{7-[(7'-Amino-3'H-spiro[cyclopropane-1,2'-pyrido[2,3-f][1,4]oxazepin]-4'(5'H)-yl)methyl]-1-benzothiophen-5-yl}-3-(3,7-dimethyl-3H-[1,2,3]triazolo[4,5-b]pyridin-6-yl)propanoic acid

To a mixture of ethyl 3-(3,7-dimethyl-3H-[1,2,3]triazolo[4,5-b]pyridin-6-yl)-3-(7-{[7'-(2,2,2-trifluoroacetamido)-3'H-spiro[cyclopropane-1,2'-pyrido[2,3-f][1,4]oxazepin]-4'(5'H)-yl]methyl}-1-benzothiophen-5-yl)propanoate (189 mg) of Example 71d, tetrahydrofuran (2.5 mL) and methanol (0.83 mL) was added 1 M aqueous lithium hydroxide solution (0.83 mL), and the mixture was stirred at 40°C for 40 min. 1 M Aqueous lithium hydroxide solution (0.42 mL) was added thereto, and the mixture was stirred for 1 hr. The reaction mixture was cooled to room temperature, saturated aqueous ammonium chloride solution was added, and the mixture was extracted with chloroform. The organic layer was dried over anhydrous sodium sulfate. Under reduced pressure, the solvent was evaporated and the obtained residue was purified by silica gel column chromatography [elution solvent: chloroform/methanol=100/0-93/7(V/V)]. To the obtained residue were added ethyl acetate and n-hexane, and an ultrasonic treatment was performed. The solid was collected by filtration to give the title compound (117 mg, yield: 76%) as a solid.

### (Example 72)

### (3R)-3-(1,4-Dimethyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoic acid

### (72a) 1-[(6S)-6-Ethyl-5,6,7,9-tetrahydro-8H-pyrido[2,3-c]azepin-8-yl]-2,2,2-trifluoroethanone

To a solution of (6S)-6-ethyl-6,7,8,9-tetrahydro-5H-pyrido[2,3-c]azepine dihydrochloride (730 mg) of Example 69a in dichloromethane (5.9 mL) was added triethylamine (2.0 mL), and the mixture was stirred at room temperature for 30 min. After cooling to 0°C, trifluoroacetic anhydride (0.61 mL) was added thereto, and the mixture was stirred at room temperature for 1 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=70/30-50/50(V/V)] to give the title compound (800 mg, yield: quantitative) as an oil.

### (72b) 1-[(6S)-6-Ethyl-1-oxido-5,6,7,9-tetrahydro-8H-pyrido[2,3-c]azepin-8-yl]-2,2,2-trifluoroethanone

To a solution of 1-[(6S)-6-ethyl-5,6,7,9-tetrahydro-8H-pyrido[2,3-c]azepin-8-yl]-2,2,2-trifluoroethanone (800 mg) of Example 72a in dichloromethane (100 mL) was added 3-chloroperbenzoic acid (1.01 g), and the mixture was stirred at room temperature for 12 hr. To the reaction mixture was added saturated aqueous sodium hydrogen carbonate solution, and the mixture was extracted with dichloromethane. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=20/80/0-0/80/20(V/V/V)] to give the title compound (829 mg, yield: 98%) as an oil.

### (72c) 1-[(6S)-2-Chloro-6-ethyl-5,6,7,9-tetrahydro-8H-pyrido[2,3-c]azepin-8-yl]-2,2,2-trifluoroethanone

To a solution of 1-[(6S)-6-ethyl-1-oxido-5,6,7,9-tetrahydro-8H-pyrido[2,3-c]azepin-8-yl]-2,2,2-trifluoroethanone (829 mg) of Example 72b in chloroform (10 mL) was added phosphoryl chloride (4.52 g), and the mixture was stirred at 65°C for 14 hr. The reaction mixture was cooled to room temperature, saturated aqueous sodium hydrogen carbonate solution was added, and the mixture was extracted with dichloromethane. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=20/80/0-0/80/20(V/V/V)] to give the title compound (160 mg, yield: 18%) as an oil.

### (72d) tert-Butyl (6S)-2-chloro-6-ethyl-5,6,7,9-tetrahydro-8H-pyrido[2,3-c]azepine-8-carboxylate

To a solution of 1-[(6S)-2-chloro-6-ethyl-5,6,7,9-tetrahydro-8H-pyrido[2,3-c]azepin-8-yl]-2,2,2-trifluoroethanone (170 mg) of Example 72c in methanol (4.0 mL) were added potassium carbonate (0.23 g) and water (1.0 mL), and the mixture was stirred at room temperature for 60 hr. The solvent was evaporated under reduced pressure and the obtained residue was dissolved in 1,4-dioxane (4.0 mL) and water (1.0 mL). tert-Butyl dicarbonate (0.36 g) was added thereto, and the mixture was stirred at room temperature for 4 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=90/10-80/20(V/V)] to give the title compound (160 mg, yield: 93%) as an oil.

### (72e) tert-Butyl (6S)-6-ethyl-2-hydroxy-5,6,7,9-tetrahydro-8H-pyrido[2,3-c]azepine-8-carboxylate

A solution of tert-butyl (6S)-2-chloro-6-ethyl-5,6,7,9-tetrahydro-8H-pyrido[2,3-c]azepine-8-carboxylate (160 mg) of Example 72d, tris(dibenzylideneacetone)dipalladium (47 mg), 5-(di-tert-butylphosphino)-1',3',5'-triphenyl-1'H-1,4'-bipyrazole (100 mg), and cesium hydroxide monohydrate (310 mg) in 1,4-dioxane (2.0 mL) was stirred at 100°C for 2 hr. To the reaction mixture was added saturated aqueous ammonium chloride solution, and the mixture was extracted with chloroform/2-propanol=3/1(V/V). The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/95/5(V/V/V)] to give the title compound (130 mg, yield: 86%) as a solid.

### (72f) (6S)-6-Ethyl-6,7,8,9-tetrahydro-5H-pyrido[2,3-c]azepin-2-ol hydrochloride

To a solution of tert-butyl (6S)-6-ethyl-2-hydroxy-5,6,7,9-tetrahydro-8H-pyrido[2,3-c]azepine-8-carboxylate (270 mg) of Example 72e in dichloromethane (0.92 mL) was added 4 M hydrogen chloride-1,4-dioxane solution (2.31 mL) at 0°C, and the mixture was stirred at room temperature for 2 hr. The solvent was evaporated under reduced pressure, diethyl ether was added to the obtained residue, and an ultrasonic treatment was performed. The solid was collected by filtration to give the title compound (210 mg, yield: 99%) as a solid.

### (72g) Ethyl (3R)-3-(1,4-dimethyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate

To a solution of ethyl (3R)-3-(1,4-dimethyl-1H-benzotriazol-5-yl)-3-[7-(hydroxymethyl)-1-benzothiophen-5-yl]propanoate (50.0 mg) of Example 451 in dichloromethane (2.0 mL) was added thionyl chloride (0.0177 mL), and the mixture was stirred at room temperature for 1 hr. The solvent was evaporated under reduced pressure, and the residue was azeotropically distilled with toluene. The obtained residue was dissolved in acetonitrile (1.0 mL), added to a separately-prepared solution of N,N-diisopropylethylamine (0.170 mL) and (6S)-6-ethyl-6,7,8,9-tetrahydro-5H-pyrido[2,3-c]azepin-2-ol hydrochloride (27.9 mg) of Example 72f in acetonitrile (1.0 mL), and the mixture was stirred at 85°C for 30 hr and cooled to room temperature. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/100/0-0/90/10(V/V/V)] to give the title compound (48.0 mg, yield: 67%) as an oil.

### (72h)=(72)

### (3R)-3-(1,4-Dimethyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoic acid

To a mixture of ethyl (3R)-3-(1,4-dimethyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate (48.0 mg) of Example 72g, methanol (1.0 mL), and tetrahydrofuran (1.0 mL) was added 1 M aqueous sodium hydroxide solution (0.411 mL), and the mixture was stirred at 50°C for 2 hr and cooled to room temperature. To the reaction mixture was added 1 M hydrochloric acid (0.411 mL) and the mixture was stirred and concentrated under reduced pressure. Saturated aqueous ammonium chloride solution, saturated aqueous sodium chloride solution and saturated brine were added thereto, and the mixture was extracted with chloroform/2-propanol=3/1(V/V). The organic layer was dried over anhydrous sodium sulfate. After filtration, the solvent was evaporated under reduced pressure and the obtained residue was purified by silica gel column chromatography [elution solvent: n-hexane/ethyl acetate/dichloromethane/methanol=30/70/0/0-0/100/0/0-0/0/100/0-0/0/90/10(V/V/V/V)]. Ethyl acetate and n-hexane were added to the obtained solid, and an ultrasonic treatment was performed. The solid was collected by filtration and vacuum dried to give the title compound (31.0 mg, yield: 68%) as a solid.

### (Example 73)

### (3S)-3-(1,4-Dimethyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoic acid

### (73a) Ethyl (3S)-3-(1,4-dimethyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate

To a solution of ethyl (3S)-3-(1,4-dimethyl-1H-benzotriazol-5-yl)-3-[7-(hydroxymethyl)-1-benzothiophen-5-yl]propanoate (50.0 mg) of Example 70a in dichloromethane (2.0 mL) was added thionyl chloride (0.0177 mL), and the mixture was stirred at room temperature for 1 hr. The solvent was evaporated under reduced pressure, and the residue was azeotropically distilled with toluene. The obtained residue was dissolved in acetonitrile (1.0 mL), added to a separately-prepared solution of N,N-diisopropylethylamine (0.170 mL) and (6S)-6-ethyl-6,7,8,9-tetrahydro-5H-pyrido[2,3-c]azepin-2-ol hydrochloride (27.9 mg) of Example 72f in acetonitrile (1.0 mL), and the mixture was stirred at 85°C for 30 hr and cooled to room temperature. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/100/0-0/90/10(V/V/V)] to give the title compound (52.0 mg, yield: 73%) as an oil.

### (73b)=(73)

### (3S)-3-(1,4-Dimethyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoic acid

To a mixture of ethyl (3S)-3-(1,4-dimethyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate (52.0 mg) of Example 73a, methanol (1.0 mL), and tetrahydrofuran (1.0 mL) was added 1 M aqueous sodium hydroxide solution (0.445 mL), and the mixture was stirred at 50°C for 2 hr and cooled to room temperature. To the reaction mixture was added 1 M hydrochloric acid (0.445 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/dichloromethane/methanol=30/70/0/0-0/100/0/0-0/0/100/0-0/0/90/10(V/V/V/V)]. Ethyl acetate and n-hexane were added to the obtained solid, and an ultrasonic treatment was performed. The solid was collected by filtration and vacuum dried to give the title compound (28.0 mg, yield: 57%) as a solid.

### (Example 74)

### 3-(1,4-Dimethyl-1H-benzotriazol-5-yl)-3-(7-{[(2R)-2-ethyl-7-fluoro-8-hydroxy-2,3-dihydropyrido[2,3-f] [1,4]oxazepin-4(5H)-yl]methyl}-1-benzothiophen-5-yl)propanoic acid

### (74a) (2R)-2-Ethyl-4-(4-methoxybenzyl)-2,3,4,5-tetrahydropyrido[2,3-f] [1,4]oxazepin-7-amine

To a solution of (2R)-7-bromo-2-ethyl-4-(4-methoxybenzyl)-2,3,4,5-tetrahydropyrido[2,3-f] [1,4]oxazepine (2.12 g) of Example 17c and benzophenone imine (1.04 mL) in toluene (26 mL) were added (±)-2,2'-bis(diphenylphosphino)-1,1'-binaphthyl (648 mg), sodium tert-butoxide (1.00 g) and tris(dibenzylideneacetone)dipalladium(0) (474 mg), and the mixture was stirred at 80°C for 45 min. The reaction mixture was cooled to room temperature, 2 M hydrochloric acid (25 mL) was added and the mixture was stirred at room temperature for 1 hr. The aqueous layer was neutralized with saturated aqueous sodium hydrogen carbonate solution and extracted with ethyl acetate. The organic layer was washed successively with water and saturated brine, and dried over anhydrous sodium sulfate. The solvent was evaporated under reduced pressure and the obtained residue was purified by NH silica gel column chromatography [elution solvent: n-hexane/ethyl acetate=100/0-60/40(V/V)] to give the title compound (1.46 g, yield: 90%) as a solid.

### (74b) tert-Butyl [(2R)-2-ethyl-4-(4-methoxybenzyl)-2,3,4,5-tetrahydropyrido[2,3-f][1,4]oxazepin-7-yl]carbamate

To a solution of (2R)-2-ethyl-4-(4-methoxybenzyl)-2,3,4,5-tetrahydropyrido[2,3-f][1,4]oxazepin-7-amine (1.46 g) of Example 74a in dichloromethane (23 mL) were added di-tert-butyl dicarbonate (1.18 mL) and 4-dimethylaminopyridine (854 mg), and the mixture was stirred at room temperature for 2 hr. To the reaction mixture was added water, and the mixture was extracted with chloroform. The organic layer was dried over anhydrous sodium sulfate. The solvent was evaporated under reduced pressure and the obtained residue was dissolved in methanol (23 mL). Potassium carbonate (1.29 g) was added thereto, and the mixture was stirred at room temperature for 15 hr and at 60°C for 2 hr. Potassium carbonate (1.29 g) was added thereto, and the mixture was stirred at 60°C for 1 hr. The reaction mixture was cooled to room temperature, water was added, and the mixture was extracted with ethyl acetate. The organic layer was washed successively with water and saturated brine, and dried over anhydrous sodium sulfate. Under reduced pressure, the solvent was evaporated and the obtained residue was purified by silica gel column chromatography [elution solvent: n-hexane/ethyl acetate=100/0-50/50(V/V)] to give the title compound (1.21 g, yield: 63%) as a solid.

### (74c) (2R)-8-Bromo-2-ethyl-4-(4-methoxybenzyl)-2,3,4,5-tetrahydropyrido[2,3-f][1,4]oxazepin-7-amine

To a solution of tert-butyl [(2R)-2-ethyl-4-(4-methoxybenzyl)-2,3,4,5-tetrahydropyrido[2,3-f] [1,4]oxazepin-7-yl]carbamate (928 mg) of Example 74b in dichloromethane (9 mL) was added trifluoroacetic acid (9 mL), and the mixture was stirred at room temperature for 70 min. The reaction mixture was added to saturated aqueous sodium hydrogen carbonate solution, and the mixture was extracted with chloroform. The organic layer was dried over anhydrous sodium sulfate. The solvent was evaporated under reduced pressure and the obtained residue was dissolved in acetonitrile (9 mL). N-bromosuccinimide (399 mg) was added thereto at 0°C, and the mixture was stirred at 0°C for 13 min. N-bromosuccinimide (80 mg) was added thereto, and the mixture was stirred at 0°C for 38 min. To the reaction mixture was added water, and the mixture was extracted with a mixed solution of chloroform and methanol. The organic layer was dried over anhydrous sodium sulfate. Under reduced pressure, the solvent was evaporated and the obtained residue was purified by silica gel column chromatography [elution solvent: n-hexane/ethyl acetate=90/10-50/50(V/V)] to give the title compound (299 mg, yield: 34%) as a solid.

### (74d) (2R)-8-Bromo-2-ethyl-7-fluoro-4-(4-methoxybenzyl)-2,3,4,5-tetrahydropyrido[2,3-f][1,4]oxazepine

To a solution of (2R)-8-bromo-2-ethyl-4-(4-methoxybenzyl)-2,3,4,5-tetrahydropyrido[2,3-f][1,4]oxazepin-7-amine (274 mg) of Example 74c in hydrogen fluoride pyridine (3.3 mL) was added sodium nitrite (72 mg) at 0°C, and the mixture was stirred at 0°C for 30 min. Sodium nitrite (48 mg) was added thereto, and the mixture was stirred at 0°C for 20 min. The reaction mixture was added to ice water, sodium bicarbonate was added, and the mixture was extracted with chloroform. The organic layer was dried over anhydrous sodium sulfate. Under reduced pressure, the solvent was evaporated and the obtained residue was purified by silica gel column chromatography [elution solvent: n-hexane/ethyl acetate=90/10-70/30(V/V)] to give the title compound (275 mg, yield: 100%) as an oil.

### (74e) (2R)-2-Ethyl-7-fluoro-2,3,4,5-tetrahydropyrido[2,3-f][1,4]oxazepin-8-ol hydrochloride

To a solution of (2R)-8-bromo-2-ethyl-7-fluoro-4-(4-methoxybenzyl)-2,3,4,5-tetrahydropyrido[2,3-f][1,4]oxazepine (275 mg) of Example 74d in 1,4-dioxane (2.3 mL) were added bis(pinacolato)diboron (212 mg), potassium acetate (205 mg) and [1,1'-bis(diphenylphosphino)ferrocene]dichloropalladium(II) dichloromethane adduct (57 mg), and the mixture was stirred at 90°C for 18 hr. The reaction mixture was cooled to room temperature, water and ethyl acetate were added, the mixture was filtered through celite and insoluble material was removed. The filtrate was extracted with ethyl acetate, and the organic layer was dried over anhydrous sodium sulfate. The solvent was evaporated under reduced pressure and the obtained residue was dissolved in tetrahydrofuran (7.0 mL), 0.5 M aqueous sodium hydroxide solution (7.0 mL) was added at 0°C, 30% aqueous hydrogen peroxide solution (0.71 mL) was added and the mixture was stirred at room temperature for 40 min. To the reaction mixture was added saturated aqueous ammonium chloride solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent:
chloroform/methanol=100/0-99/1(V/V)]. The obtained residue was dissolved in trifluoroacetic acid (3.5 mL), anisole (0.75 mL) was added, and the mixture was heated under reflux for 20 hr. The reaction mixture was cooled to room temperature, and stirred at 120°C for 80 min and at 140°C for 70 min under microwave irradiation. The reaction mixture was cooled to room temperature, and the residue was azeotropically distilled with toluene. The obtained residue was dissolved in ethyl acetate (4.7 mL), 1 M hydrogen chloride-ethyl acetate (2.1 mL) was added and the mixture was stirred at room temperature for 20 min. The solid was collected by filtration to give the title compound (119 mg, yield: 69%) as a solid.

### (74f) Ethyl 3-(1,4-dimethyl-1H-benzotriazol-5-yl)-3-(7-{[(2R)-2-ethyl-7-fluoro-8-hydroxy-2,3-dihydropyrido[2,3-f][1,4]oxazepin-4(5H)-yl]methyl}-1-benzothiophen-5-yl)propanoate

To a solution of ethyl 3-(1,4-dimethyl-1H-benzotriazol-5-yl)-3-[7-(hydroxymethyl)-1-benzothiophen-5-yl]propanoate (240 mg) of Example 1f in dichloromethane (2.8 mL) were added dimethyl sulfoxide (0.20 mL), N,N-diisopropylethylamine (0.29 mL) and sulfur trioxide-pyridine complex (294 mg), and the mixture was stirred at room temperature for 18 min. To the reaction mixture was added water, and the mixture was extracted with ethyl acetate. The organic layer was washed successively with water and saturated brine, and dried over anhydrous sodium sulfate. The solvent was evaporated under reduced pressure and the obtained residue was dissolved in dichloromethane (1.6 mL). (2R)-2-ethyl-7-fluoro-2,3,4,5-tetrahydropyrido[2,3-f][1,4]oxazepin-8-ol hydrochloride (119 mg) of Example 74e, N,N-diisopropylethylamine (0.16 mL), acetic acid (0.16 mL) and anhydrous magnesium sulfate (115 mg) were added thereto, and the mixture was stirred at 35°C for 40 min. Sodium triacetoxyborohydride (203 mg) was added thereto, and the mixture was stirred at 35°C for 14 hr. The reaction mixture was cooled to room temperature, saturated aqueous sodium hydrogen carbonate solution was added, and the mixture was extracted with chloroform. The organic layer was dried over anhydrous sodium sulfate. Under reduced pressure, the solvent was evaporated and the obtained residue was purified by silica gel column chromatography [elution solvent: n-hexane/ethyl acetate=90/10-60/40(V/V)] to give the title compound (144 mg, yield: 50%) as an oil.

### (74g)=(74)

### 3-(1,4-Dimethyl-1H-benzotriazol-5-yl)-3-(7-{[(2R)-2-ethyl-7-fluoro-8-hydroxy-2,3-dihydropyrido[2,3-f] [1,4]oxazepin-4(5H)-yl]methyl}-1-benzothiophen-5-yl)propanoic acid

To a mixture of ethyl 3-(1,4-dimethyl-1H-benzotriazol-5-yl)-3-(7-{[(2R)-2-ethyl-7-fluoro-8-hydroxy-2,3-dihydropyrido[2,3-f] [1,4]oxazepin-4(5H)-yl]methyl}-1-benzothiophen-5-yl)propanoate (144 mg) of Example 74f, tetrahydrofuran (2.2 mL) and methanol (0.72 mL) was added 1 M aqueous lithium hydroxide solution (0.72 mL), and the mixture was stirred at 40°C for 3 hr. The reaction mixture was cooled to room temperature, saturated aqueous ammonium chloride solution was added, and the mixture was extracted with chloroform. The organic layer was dried over anhydrous sodium sulfate. Under reduced pressure, the solvent was evaporated and the obtained residue was purified by silica gel column chromatography [elution solvent: chloroform/methanol=100/0-90/10(V/V)]. To the obtained residue were added ethyl acetate and n-hexane, and an ultrasonic treatment was performed. The solid was collected by filtration to give the title compound (95 mg, yield: 69%) as a solid.

### (Example 75)

### (3R)-3-(3,7-Dimethyl-3H-[1,2,3]triazolo[4,5-b]pyridin-6-yl)-3-(7-{[(6S)-6-ethyl-5,6,7,9-tetrahydro-8H-pyrido[2,3-c]azepin-8-yl]methyl}-1-benzothiophen-5-yl)propanoic acid

### (75a) Ethyl 3-(3,7-dimethyl-3H-[1,2,3]triazolo[4,5-b]pyridin-6-yl)-3-(7-{[(6S)-6-ethyl-5,6,7,9-tetrahydro-8H-pyrido[2,3-c]azepin-8-yl]methyl}-1-benzothiophen-5-yl)propanoate

To a solution of ethyl 3-(3,7-dimethyl-3H-[1,2,3]triazolo[4,5-b]pyridin-6-yl)-3-[7-(hydroxymethyl)-1-benzothiophen-5-yl]propanoate (100 mg) of Example 9a in dichloromethane (2.0 mL) was added thionyl chloride (0.0372 mL) and the mixture was stirred at room temperature for 1 hr. The solvent was evaporated under reduced pressure, and the residue was azeotropically distilled with toluene. The obtained residue was dissolved in acetonitrile (1.0 mL), added to a separately-prepared solution of N,N-diisopropylethylamine (0.339 mL) and (6S)-6-ethyl-6,7,8,9-tetrahydro-5H-pyrido[2,3-c]azepine dihydrochloride (60.7 mg) of Example 69a in acetonitrile (1.0 mL), and the mixture was stirred at 80°C for 3 hr and cooled to room temperature. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-80/20(V/V)] to give the title compound (135 mg, yield: 97%) as an oil.

### (75b) Ethyl (3R)-3-(3,7-dimethyl-3H-[1,2,3]triazolo[4,5-b]pyridin-6-yl)-3-(7-{[(6S)-6-ethyl-5,6,7,9-tetrahydro-8H-pyrido[2,3-c]azepin-8-yl]methyl}-1-benzothiophen-5-yl)propanoate

Ethyl 3-(3,7-dimethyl-3H-[1,2,3]triazolo[4,5-b]pyridin-6-yl)-3-(7-{[(6S)-6-ethyl-5,6,7, 9-tetrahydro-8H-pyrido[2, 3-c]azepin-8-yl]methyl}-1-benzothiophen-5-yl)propanoate (130 mg) of Example 75a was subjected to chiral HPLC [column: CHIRALCEL OZ-H (20 mm I.D. × 250 mm), mobile phase: n-hexane/ethanol=70/30(V/V)] to give 59 mg of ethyl (3R)-3-(3,7-dimethyl-3H-[1,2,3]triazolo[4,5-b]pyridin-6-yl)-3-(7-{[(6S)-6-ethyl-5,6,7,9-tetrahydro-8H-pyrido[2,3-c]azepin-8-yl]methyl}-1-benzothiophen-5-yl)propanoate as the second peak (yield: 45%). retention time: 15.890 min [column: CHIRALCEL OZ-H (4.6 mm I.D. × 250 mm), mobile phase: n-hexane/ethanol=70/30(V/V), flow rate: 1.0 mL/min, temperature: 40°C, wavelength: 230 nm]

### (75c)=(75)

### (3R)-3-(3,7-Dimethyl-3H-[1,2,3]triazolo[4,5-b]pyridin-6-yl)-3-(7-{[(6S)-6-ethyl-5,6,7,9-tetrahydro-8H-pyrido[2,3-c]azepin-8-yl]methyl}-1-benzothiophen-5-yl)propanoic acid

To a mixture of ethyl (3R)-3-(3,7-dimethyl-3H-[1,2,3]triazolo[4,5-b]pyridin-6-yl)-3-(7-{[(6S)-6-ethyl-5,6,7,9-tetrahydro-8H-pyrido[2,3-c]azepin-8-yl]methyl}-1-benzothiophen-5-yl)propanoate (59.0 mg) of Example 75b, methanol (1.0 mL), and tetrahydrofuran (1.0 mL) was added 1 M aqueous sodium hydroxide solution (0.519 mL), and the mixture was stirred at room temperature for 24 hr. To the reaction mixture was added 1 M hydrochloric acid (0.519 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ ethyl acetate/ dichloromethane/ methanol=30/70/0/0-0/100/0/0-0/0/100/0-0/0/90/10(V/V/V/V)]. Ethyl acetate and n-hexane were added to the obtained solid, and an ultrasonic treatment was performed. The solid was collected by filtration and vacuum dried to give the title compound (47.0 mg, yield: 84%) as a solid.

### (Example 76)

### (3S)-3-(3,7-Dimethyl-3H-[1,2,3]triazolo[4,5-b]pyridin-6-yl)-3-(7-{[(6S)-6-ethyl-5,6,7,9-tetrahydro-8H-pyrido[2,3-c]azepin-8-yl]methyl}-1-benzothiophen-5-yl)propanoic acid

### (76a) Ethyl (3S)-3-(3,7-dimethyl-3H-[1,2,3]triazolo[4,5-b]pyridin-6-yl)-3-(7-{[(6S)-6-ethyl-5,6,7,9-tetrahydro-8H-pyrido[2,3-c]azepin-8-yl]methyl}-1-benzothiophen-5-yl)propanoate

Ethyl 3-(3,7-dimethyl-3H-[1,2,3]triazolo[4,5-b]pyridin-6-yl)-3-(7-{[(6S)-6-ethyl-5,6,7, 9-tetrahydro-8H-pyrido[2, 3-c]azepin-8-yl]methyl}-1-benzothiophen-5-yl)propanoate (130 mg) of Example 75a was subjected to chiral HPLC [column: CHIRALCEL OZ-H (20 mm I.D. × 250 mm), mobile phase: n-hexane/ethanol=70/30(V/V)] to give 64 mg of ethyl (3S)-3-(3,7-dimethyl-3H-[1,2,3]triazolo[4,5-b]pyridin-6-yl)-3-(7-{[(6S)-6-ethyl-5,6,7,9-tetrahydro-8H-pyrido[2,3-c]azepin-8-yl]methyl}-1-benzothiophen-5-yl)propanoate as the first peak (yield: 49%). retention time: 13.047 min [column: CHIRALCEL OZ-H (4.6 mm I.D. × 250 mm), mobile phase: n-hexane/ethanol=70/30(V/V), flow rate: 1.0 mL/min, temperature: 40°C, wavelength: 230 nm]

### (76b)=(76)

### (3S)-3-(3,7-Dimethyl-3H-[1,2,3]triazolo[4,5-b]pyridin-6-yl)-3-(7-{[(6S)-6-ethyl-5,6,7,9-tetrahydro-8H-pyrido[2,3-c]azepin-8-yl]methyl}-1-benzothiophen-5-yl)propanoic acid

To a mixture of ethyl (3S)-3-(3,7-dimethyl-3H-[1,2,3]triazolo[4,5-b]pyridin-6-yl)-3-(7-{[(6S)-6-ethyl-5,6,7,9-tetrahydro-8H-pyrido[2,3-c]azepin-8-yl]methyl}-1-benzothiophen-5-yl)propanoate (65.0 mg) of Example 76a, methanol (1.0 mL), and tetrahydrofuran (1.0 mL) was added 1 M aqueous sodium hydroxide solution (0.571 mL), and the mixture was stirred at room temperature for 24 hr. To the reaction mixture was added 1 M hydrochloric acid (0.571 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ ethyl acetate/ dichloromethane/ methanol=30/70/0/0-0/100/0/0-0/0/100/0-0/0/95/5(V/V/V/V)]. Ethyl acetate and n-hexane were added to the obtained solid, and an ultrasonic treatment was performed. The solid was collected by filtration and vacuum dried to give the title compound (53.0 mg, yield: 86%) as a solid.

### (Example 77)

### (3R)-3-(1,4-Dimethyl-1H-benzotriazol-5-yl)-3-(7-{[(2R)-2-ethyl-7-(methylamino)-2,3-dihydropyrido[2,3-f] [1,4]oxazepin-4(5H)-yl]methyl}-1-benzothiophen-5-yl)propanoic acid

### (Example 78)

### (3S)-3-(1,4-Dimethyl-1H-benzotriazol-5-yl)-3-(7-{[(2R)-2-ethyl-7-(methylamino)-2,3-dihydropyrido[2,3-f] [1,4]oxazepin-4(5H)-yl]methyl}-1-benzothiophen-5-yl)propanoic acid

### (77a) tert-Butyl (2R)-7-[(tert-butoxycarbonyl) (methyl)amino]-2-ethyl-2,3-dihydropyrido[2,3-f][1,4]oxazepine-4(5H)-carboxylate

To a solution of tert-butyl (2R)-7-[(tert-butoxycarbonyl)amino]-2-ethyl-2,3-dihydropyrido[2,3-f] [1,4]oxazepine-4(5H)-carboxylate (195 mg) of Example 67a in tetrahydrofuran (5 mL) were added sodium hydride (36 mg) and methyl iodide (0.0617 mL) at 0°C, and the mixture was stirred for 1 hr. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V)] to give the title compound (193 mg, yield: 96%) as an oil.

### (77b) (2R)-2-Ethyl-N-methyl-2,3,4,5-tetrahydropyrido[2,3-f][1,4]oxazepin-7-amine

To a solution of tert-butyl (2R)-7-[(tert-butoxycarbonyl) (methyl)amino]-2-ethyl-2,3-dihydropyrido[2,3-f] [1,4]oxazepine-4(5H)-carboxylate (190 mg) of Example 77a in dichloromethane (3 mL) was added trifluoroacetic acid (3 mL), and the mixture was stirred at room temperature for 1 hr and concentrated under reduced pressure. The obtained residue was dried under reduced pressure to give the title compound (100 mg, yield: quantitative) as an oil.

### (77c) Ethyl 3-(1,4-dimethyl-1H-benzotriazol-5-yl)-3-(7-{[(2R)-2-ethyl-7-(methylamino)-2,3-dihydropyrido[2,3-f][1,4]oxazepin-4(5H)-yl]methyl}-1-benzothiophen-5-yl)propanoate

To a solution of ethyl 3-[7-(chloromethyl)-1-benzothiophen-5-yl]-3-(1,4-dimethyl-1H-benzotriazol-5-yl)propanoate (100 mg) of Example 1g in acetonitrile (3 mL) were added (2R)-2-ethyl-N-methyl-2,3,4,5-tetrahydropyrido[2,3-f][1,4]oxazepin-7-amine (55 mg) of Example 77b and N,N-diisopropylethylamine (0.40 mL), and the mixture was stirred at 85°C for 48 hr and cooled to room temperature. The reaction mixture was poured into water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-20/80(V/V)] to give the title compound (85 mg, yield: 61%) as an oil.

### (77d)=(77)

### (3R)-3-(1,4-Dimethyl-1H-benzotriazol-5-yl)-3-(7-{[(2R)-2-ethyl-7-(methylamino)-2,3-dihydropyrido[2,3-f] [1,4]oxazepin-4(5H)-yl]methyl}-1-benzothiophen-5-yl)propanoic acid

### (78a)=(78)

### (3S)-3-(1,4-Dimethyl-1H-benzotriazol-5-yl)-3-(7-{[(2R)-2-ethyl-7-(methylamino)-2,3-dihydropyrido[2,3-f] [1,4]oxazepin-4(5H)-yl]methyl}-1-benzothiophen-5-yl)propanoic acid

To a mixture of ethyl 3-(1,4-dimethyl-1H-benzotriazol-5-yl)-3-(7-{[(2R)-2-ethyl-7-(methylamino)-2,3-dihydropyrido[2,3-f] [1,4]oxazepin-4(5H)-yl]methyl}-1-benzothiophen-5-yl)propanoate (85 mg) of Example 77c, ethanol (1 mL), and tetrahydrofuran (2 mL) was added 1 M aqueous sodium hydroxide solution (2 mL), and the mixture was stirred at room temperature for 12 hr. To the reaction mixture was added 1 M hydrochloric acid (2 mL) and the mixture was stirred and extracted with ethyl acetate. The organic layer was washed with saturated brine and dried over anhydrous sodium sulfate. Under reduced pressure, the solvent was evaporated and the obtained residue was purified by silica gel column chromatography [elution solvent:
dichloromethane/methanol=15/1(V/V)] to give two compounds. Ethyl acetate and n-hexane were added to the compound eluted earlier and the mixture was subjected to an ultrasonic treatment. The solid was collected by filtration and vacuum dried to give (3R)-3-(1,4-dimethyl-1H-benzotriazol-5-yl)-3-(7-{[(2R)-2-ethyl-7-(methylamino)-2,3-dihydropyrido[2,3-f][1,4]oxazepin-4(5H)-yl]methyl}-1-benzothiophen-5-yl)propanoic acid (30 mg, yield: 37%) as a solid.

In the same manner, moreover, the compound eluted later, (3S)-3-(1,4-dimethyl-1H-benzotriazol-5-yl)-3-(7-{[(2R)-2-ethyl-7-(methylamino)-2,3-dihydropyrido[2,3-f] [1,4]oxazepin-4(5H)-yl]methyl}-1-benzothiophen-5-yl)propanoic acid (40 mg, yield: 49%) was obtained as a solid.

### (Example 79)

### (3S)-3-(1,4-Dimethyl-1H-benzotriazol-5-yl)-3-{7-[(8'-hydroxy-3'H-spiro[cyclopropane-1,2'-pyrido[3,4-f][1,4]oxazepin]-4'(5'H)-yl)methyl]-1-benzothiophen-5-yl}propanoic acid

### (79a) 5-(Bromomethyl)-2-chloro-4-fluoropyridine

To a solution of 2-chloro-4-fluoro-5-methylpyridine (770 mg) in carbon tetrachloride (26 mL) were added N-bromosuccinimide (1.03 g) and 2,2'-azobisisobutyronitrile (87 mg), and the mixture was heated under reflux for 6 hr. To the reaction mixture were added N-bromosuccinimide (1.79 g) and 2,2'-azobisisobutyronitrile (87 mg) and the mixture was heated under reflux for 16 hr. The reaction mixture was cooled to room temperature, water was added, and the mixture was extracted with chloroform. The organic layer was dried over anhydrous sodium sulfate. The solvent was evaporated under reduced pressure and the obtained residue was dissolved in tetrahydrofuran (25 mL), N,N-diisopropylethylamine (0.92 mL) and diethyl phosphite (0.68 mL) were added at 0°C and the mixture was stirred at room temperature for 3.5 hr. To the reaction mixture was added water, and the mixture was extracted with chloroform. The organic layer was dried over anhydrous sodium sulfate. Under reduced pressure, the solvent was evaporated and the obtained residue was purified by silica gel column chromatography [elution solvent: n-hexane/ethyl acetate=95/5-85/15(V/V)] to give the title compound (1.10 g, yield: 93%) as an oil.

### (79b) 1-({[(6-Chloro-4-fluoropyridin-3-yl)methyl](4-methoxybenzyl)amino}methyl)cyclopropanol

To a solution of 5-(bromomethyl)-2-chloro-4-fluoropyridine(1.10 g) of Example 79a in acetonitrile (15 mL) were added 1-{[(4-methoxybenzyl)amino]methyl}cyclopropanol (1.52 g) of Example 3c and potassium carbonate (1.02 g), and the mixture was stirred at 50°C for 1.5 hr. The reaction mixture was cooled to room temperature, and insoluble material was filtered off. The filtrate was concentrated under reduced pressure and the obtained residue was purified by silica gel column chromatography [elution solvent: n-hexane/ethyl acetate=90/10-75/25(V/V)] to give the title compound (1.31 g, yield: 76%) as an oil.

### (79c) 8'-Chloro-4'-(4-methoxybenzyl)-4',5'-dihydro-3'H-spiro[cyclopropane-1,2'-pyrido[3,4-f][1,4]oxazepine]

To a suspension of 60% sodium hydride (431 mg) in N,N-dimethylformamide (25 mL) was added a solution of 1-({[(6-chloro-4-fluoropyridin-3-yl)methyl] (4-methoxybenzyl)amino}methyl)cyclopropanol (1.26 g) of Example 79b in tetrahydrofuran (25 mL), and the mixture was stirred at 60°C for 1.5 hr. The reaction mixture was cooled to room temperature, water was added, and the mixture was extracted with ethyl acetate. The organic layer was washed with water, and dried over anhydrous sodium sulfate. Under reduced pressure, the solvent was evaporated and the obtained residue was purified by silica gel column chromatography [elution solvent: n-hexane/ethyl acetate=80/20-50/50(V/V)] to give the title compound (1.15 g, yield: 93%) as an oil.

### (79d) 4'-(4-Methoxybenzyl)-4',5'-dihydro-3'H-spiro[cyclopropane-1,2'-pyrido[3,4-f] [1,4]oxazepin]-8'-ol

To a solution of 8'-chloro-4'-(4-methoxybenzyl)-4',5'-dihydro-3'H-spiro[cyclopropane-1,2'-pyrido[3,4-f][1,4]oxazepine] (540 mg) of Example 79c in 1,4-dioxane (9.0 mL) were added cesium hydroxide monohydrate (823 mg), bis(dibenzylideneacetone)palladium(0) (47 mg) and 5-(di-tert-butylphosphino)-1',3',5'-triphenyl-1'H-1,4'-bipyrazole (83 mg), and the mixture was stirred at 100°C for 15 hr. The reaction mixture was cooled to room temperature, saturated aqueous sodium hydrogen carbonate solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: chloroform/methanol=100/0-92/8(V/V)] to give the title compound (238 mg, yield: 48%) as an oil.

### (79e) 4',5'-Dihydro-3'H-spiro[cyclopropane-1,2'-pyrido[3,4-f][1,4]oxazepin]-8'-ol hydrochloride

To a solution of 4'-(4-methoxybenzyl)-4',5'-dihydro-3'H-spiro[cyclopropane-1,2'-pyrido[3,4-f][1,4]oxazepin]-8'-ol (235 mg) of Example 79d in trifluoroacetic acid (7.5 mL) was added anisole (0.81 mL), and the mixture was stirred at 120°C for 2 hr under microwave irradiation. The reaction mixture was cooled to room temperature, and the residue was azeotropically distilled with toluene. The obtained residue was dissolved in ethyl acetate (7.5 mL), 1 M hydrogen chloride-ethyl acetate (2.3 mL) was added and the mixture was stirred at room temperature for 15 min. The solid was collected by filtration to give the title compound (157 mg, yield: 91%) as a solid.

### (79f) Ethyl (3S)-3-(1,4-dimethyl-1H-benzotriazol-5-yl)-3-{7-[(8'-hydroxy-3'H-spiro[cyclopropane-1,2'-pyrido[3,4-f][1,4]oxazepin]-4'(5'H)-yl)methyl]-1-benzothiophen-5-yl}propanoate

To a solution of ethyl (3S)-3-(1,4-dimethyl-1H-benzotriazol-5-yl)-3-[7-(hydroxymethyl)-1-benzothiophen-5-yl]propanoate (151 mg) of Example 70a in dichloromethane (1.5 mL) were added dimethyl sulfoxide (0.14 mL), N,N-diisopropylethylamine (0.19 mL) and sulfur trioxide-pyridine complex (177 mg), and the mixture was stirred at room temperature for 40 min. To the reaction mixture was added water, and the mixture was extracted with chloroform. The organic layer was washed with saturated brine and dried over anhydrous sodium sulfate. The solvent was evaporated under reduced pressure and the obtained residue was dissolved in dichloromethane (1.5 mL). 4',5'-Dihydro-3'H-spiro[cyclopropane-1,2'-pyrido[3,4-f][1,4]oxazepin]-8'-ol hydrochloride (78 mg) of Example 79e, N,N-diisopropylethylamine (0.060 mL), acetic acid (0.10 mL) and anhydrous magnesium sulfate (99 mg) were added and the mixture was stirred at room temperature for 20 min. Then, sodium triacetoxyborohydride (145 mg) was added thereto, and the mixture was stirred at room temperature for 30 min and at 35°C for 92 hr. To the reaction mixture was added sodium triacetoxyborohydride (145 mg), and the mixture was stirred at 35°C for 45 min. The reaction mixture was cooled to room temperature, saturated aqueous sodium hydrogen carbonate solution was added, and the mixture was extracted with ethyl acetate. The organic layer was washed with saturated brine, and the organic layer was dried over anhydrous sodium sulfate. Under reduced pressure, the solvent was evaporated and the obtained residue was purified by silica gel column chromatography [elution solvent: n-hexane/ethyl acetate=50/50-0/100(V/V), chloroform/methanol=0/100-95/5(V/V)] to give the title compound (111 mg, yield: 56%) as an oil.

### (79g) = (79)

### (3S)-3-(1,4-Dimethyl-1H-benzotriazol-5-yl)-3-{7-[(8'-hydroxy-3'H-spiro[cyclopropane-1,2'-pyrido[3,4-f][1,4]oxazepin]-4' (5'H)-yl)methyl]-1-benzothiophen-5-yl}propanoic acid

To a mixture of ethyl (3S)-3-(1,4-dimethyl-1H-benzotriazol-5-yl)-3-{7-[(8'-hydroxy-3'H-spiro[cyclopropane-1,2'-pyrido[3,4-f][1,4]oxazepin]-4'(5'H)-yl)methyl]-1-benzothiophen-5-yl}propanoate (108 mg) of Example 79f, tetrahydrofuran (1.2 mL) and methanol (0.60 mL) was added 1 M aqueous lithium hydroxide solution (0.56 mL), and the mixture was stirred at 40°C for 1.5 hr. The reaction mixture was cooled to room temperature, saturated aqueous ammonium chloride solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V), chloroform/methanol=0/100-95/5(V/V)]. Ethyl acetate and n-hexane were added to the obtained residue, and an ultrasonic treatment was performed. The solid was collected by filtration to give the title compound (57.6 mg, yield: 56%) as a solid.

### (Example 80)

### (3R)-3-(3,7-Dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoic acid

### (80a) Ethyl 3-(3,7-dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate

To a solution of ethyl 3-(3,7-dimethyl-3H-[1,2,3]triazolo[4,5-b]pyridin-6-yl)-3-[7-(hydroxymethyl)-1-benzothiophen-5-yl]propanoate (129 mg) of Example 9a in dichloromethane (2.0 mL) was added thionyl chloride (0.0481 mL), and the mixture was stirred at room temperature for 1 hr. The solvent was evaporated under reduced pressure, and the residue was azeotropically distilled with toluene. The obtained residue was dissolved in acetonitrile (1.0 mL), added to a separately-prepared solution of N,N-diisopropylethylamine (0.457 mL) and (6S)-6-ethyl-6,7,8,9-tetrahydro-5H-pyrido[2,3-c]azepin-2-ol hydrochloride (60.0 mg) of Example 72f in acetonitrile (1.0 mL), and the mixture was stirred at 85°C for 30 hr and cooled to room temperature. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ ethyl acetate/ methanol=50/50/0-0/100/0-0/90/10(V/V/V)] to give the title compound (119 mg, yield: 78%) as an oil.

### (80b) Ethyl (3R)-3-(3,7-dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate

Ethyl 3-(3,7-dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate (121 mg) of Example 80a was subjected to chiral HPLC [column: CHIRALCEL OD-H (20 mm I.D. × 250 mm), mobile phase: n-hexane/ethanol=60/40(V/V)] to give 61 mg of ethyl (3R)-3-(3,7-dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate as the second peak (yield: 50%).
retention time: 8.5533 min [column: CHIRALCEL OD-H (4.6 mm I.D. × 250 mm), mobile phase: n-hexane/ethanol=60/40(V/V), flow rate: 1.0 mL/min, temperature: 40°C, wavelength: 254 nm]

### (80c)=(80)

### (3R)-3-(3,7-Dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoic acid

To a mixture of ethyl (3R)-3-(3,7-dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate (61.0 mg) of Example 80b, methanol (1.0 mL), and tetrahydrofuran (1.0 mL) was added 1 M aqueous sodium hydroxide solution (0.522 mL), and the mixture was stirred at room temperature for 18 hr. To the reaction mixture was added 1 M hydrochloric acid (0.522 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/dichloromethane/methanol=30/70/0/0-0/100/0/0-0/0/100/0-0/0/90/10(V/V/V/V)]. Ethyl acetate and n-hexane were added to the obtained solid, and an ultrasonic treatment was performed. The solid was collected by filtration and vacuum dried to give the title compound (37.0 mg, yield: 64%) as a solid.

### (Example 81)

### (3S)-3-(3,7-Dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoic acid

### (81a) Ethyl (3S)-3-(3,7-dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate

Ethyl 3-(3,7-dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate (121 mg) of Example 80a was subjected to chiral HPLC [column: CHIRALCEL OD-H (20 mm I.D. × 250 mm), mobile phase: n-hexane/ethanol=60/40(V/V)] to give 53 mg of ethyl (3S)-3-(3,7-dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate as the first peak (yield: 44%).
retention time: 6.2467 min [column: CHIRALCEL OD-H (4.6 mm I.D. × 250 mm), mobile phase: n-hexane/ethanol=60/40(V/V), flow rate: 1.0 mL/min, temperature: 40°C, wavelength: 254 nm]

### (81b)=(81)

### (3S)-3-(3,7-Dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoic acid

To a mixture of ethyl (3S)-3-(3,7-dimethyl-3H-[1,2,3]triazolo[4,5-b]pyridin-6-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate (53.0 mg) of Example 81a, methanol (1.0 mL), and tetrahydrofuran (1.0 mL) was added 1 M aqueous sodium hydroxide solution (0.453 mL), and the mixture was stirred at room temperature for 18 hr. To the reaction mixture was added 1 M hydrochloric acid (0.453 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/dichloromethane/methanol=30/70/0/0-0/100/0/0-0/0/100/0-0/0/90/10(V/V/V/V)]. Ethyl acetate and n-hexane were added to the obtained solid, and an ultrasonic treatment was performed. The solid was collected by filtration and vacuum dried to give the title compound (41.0 mg, yield: 81%) as a solid.

### (Example 82)

### (3S)-3-(1,4-Dimethyl-1H-benzotriazol-5-yl)-3-{7-[(6'-hydroxy-3'H-spiro[cyclopropane-1,2'-pyrido[3,4-f][1,4]oxazepin]-4' (5'H)-yl)methyl]-1-benzothiophen-5-yl}propanoic acid

### (82a) 3-(Bromomethyl)-2-chloro-4-fluoropyridine

To a solution of 2-chloro-4-fluoro-3-methylpyridine (1.10 g) in carbon tetrachloride (33 mL) were added N-bromosuccinimide (1.30 g) and 2,2'-azobisisobutyronitrile (109 mg), and the mixture was heated under reflux for 14 hr. The reaction mixture was cooled to room temperature, water was added, and the mixture was extracted with chloroform. The organic layer was dried over anhydrous sodium sulfate. Under reduced pressure, the solvent was evaporated and the obtained residue was purified by silica gel column chromatography [elution solvent: n-hexane/ethyl acetate=100/0-80/20(V/V)] to give the title compound (667 mg, yield: 45%) as an oil.

### (82b) 1-({[(2-Chloro-4-fluoropyridin-3-yl)methyl](4-methoxybenzyl)amino}methyl)cyclopropanol

To a solution of 3-(bromomethyl)-2-chloro-4-fluoropyridine (667 mg) of Example 82a in acetonitrile (9.1 mL) were added 1-{[(4-methoxybenzyl)amino]methyl}cyclopropanol (921 mg) of Example 3c and potassium carbonate (616 mg), and the mixture was stirred at 50°C for 2 hr. The reaction mixture was cooled to room temperature, insoluble material was filtered off, and washed with ethyl acetate. The filtrate was concentrated under reduced pressure and the obtained residue was purified by silica gel column chromatography [elution solvent: n-hexane/ethyl acetate=85/15-60/40(V/V)] to give the title compound (779 mg, yield: 75%) as an oil.

### (82c) 6'-Chloro-4'-(4-methoxybenzyl)-4',5'-dihydro-3'H-spiro[cyclopropane-1,2'-pyrido[3,4-f][1,4]oxazepine]

To a suspension of 60% sodium hydride (187 mg) in N,N-dimethylformamide (15 mL) was added dropwise a solution of 1-({[(2-chloro-4-fluoropyridin-3-yl)methyl](4-methoxybenzyl)amino}methyl)cyclopropanol (729 mg) of Example 82b in tetrahydrofuran (15 mL) at 60°C, and the mixture was stirred at 60°C for 3.5 hr. The reaction mixture was cooled to room temperature, 60% sodium hydride (94 mg) was added, and the mixture was stirred at 60°C for 13 hr. The reaction mixture was cooled to room temperature, water was added, and the mixture was extracted with ethyl acetate. The organic layer was washed successively with water and saturated brine, and dried over anhydrous sodium sulfate. Under reduced pressure, the solvent was evaporated and the obtained residue was purified by silica gel column chromatography [elution solvent: n-hexane/ethyl acetate=90/10-60/40(V/V)] to give the title compound (632 mg, yield: 92%) as a solid.

### (82d) 4'-(4-Methoxybenzyl)-4',5'-dihydro-3'H-spiro[cyclopropane-1,2'-pyrido[3,4-f] [1,4]oxazepine]-6'-ol

To a solution of 6'-chloro-4'-(4-methoxybenzyl)-4',5'-dihydro-3'H-spiro[cyclopropane-1,2'-pyrido[3,4-f][1,4]oxazepine] (425 mg) of Example 82c in 1,4-dioxane (6.4 mL) were added cesium hydroxide monohydrate (645 mg), bis(dibenzylideneacetone)palladium(0) (74 mg) and 5-(di-tert-butylphosphino)-1',3',5'-triphenyl-1'H-1,4'-bipyrazole (130 mg), and the mixture was stirred at 100°C for 4 hr. The reaction mixture was cooled to room temperature, water and 5% aqueous citric acid solution were added, and the mixture was extracted with ethyl acetate. The organic layer was washed with saturated brine and dried over anhydrous sodium sulfate. The solvent was evaporated under reduced pressure to give the title compound (80 mg, yield: 20%).

### (82e) 4',5'-Dihydro-3'H-spiro[cyclopropane-1,2'-pyrido[3,4-f][1,4]oxazepin]-6'-ol hydrochloride

To a solution of 4'-(4-methoxybenzyl)-4',5'-dihydro-3'H-spiro[cyclopropane-1,2'-pyrido[3,4-f][1,4]oxazepin]-6'-ol (141 mg) of Example 82d in trifluoroacetic acid (4.5 mL) was added anisole (0.49 mL), and the mixture was stirred at 120°C for 190 min under microwave irradiation. The reaction mixture was cooled to room temperature, and the residue was azeotropically distilled with toluene. The obtained residue was dissolved in ethyl acetate (4.5 mL), 1 M hydrogen chloride-ethyl acetate (1.4 mL) was added and the mixture was stirred at room temperature for 10 min. The solid was collected by filtration to give a mixture containing the title compound (96.6 mg) as a solid.

### (82f) Ethyl (3S)-3-(1,4-dimethyl-1H-benzotriazol-5-yl)-3-{7-[(6'-hydroxy-3'H-spiro[cyclopropane-1,2'-pyrido[3,4-f][1,4]oxazepin]-4'(5'H)-yl)methyl]-1-benzothiophen-5-yl}propanoate

To a solution of ethyl (3S)-3-(1,4-dimethyl-1H-benzotriazol-5-yl)-3-[7-(hydroxymethyl)-1-benzothiophen-5-yl]propanoate (160 mg) of Example 70a in dichloromethane (4.5 mL) were added dimethyl sulfoxide (0.14 mL), N,N-diisopropylethylamine (0.21 mL) and sulfur trioxide-pyridine complex (207 mg), and the mixture was stirred at room temperature for 45 min. To the reaction mixture was added water, and the mixture was extracted with chloroform. The organic layer was washed with saturated brine and dried over anhydrous sodium sulfate. The obtained residue and 4',5'-dihydro-3'H-spiro[cyclopropane-1,2'-pyrido[3,4-f][1,4]oxazepin]-6'-ol hydrochloride (96.6 mg) of Example 82e were dissolved in a mixed solvent of dichloromethane (1.3 mL) and dimethyl sulfoxide (1.3 mL). N,N-diisopropylethylamine (0.074 mL), acetic acid (0.005 mL) and anhydrous magnesium sulfate (94 mg) were added thereto, and the mixture was stirred at room temperature for 30 min. Then, sodium triacetoxyborohydride (166 mg) was added thereto, and the mixture was stirred at room temperature for 37 hr. To the reaction mixture was added sodium triacetoxyborohydride (166 mg), and the mixture was stirred at 35°C for 45 min. Sodium triacetoxyborohydride (166 mg) was added thereto, and the mixture was stirred at 35°C for 1.5 hr. The reaction mixture was cooled to room temperature, saturated aqueous sodium hydrogen carbonate solution was added, and the mixture was extracted with ethyl acetate. The organic layer was washed with saturated brine, and dried over anhydrous magnesium sulfate. Under reduced pressure, the solvent was evaporated and the obtained residue was purified by silica gel column chromatography [elution solvent: n-hexane/ethyl acetate=50/50-0/100(V/V), chloroform/methanol=100/0-95/5(V/V)] to give the title compound (121 mg, yield: 39%) as an oil.

### (82g) = (82)

### (3S)-3-(1,4-Dimethyl-1H-benzotriazol-5-yl)-3-{7-[(6'-hydroxy-3'H-spiro[cyclopropane-1,2'-pyrido[3,4-f][1,4]oxazepin]-4' (5'H)-yl)methyl]-1-benzothiophen-5-yl}propanoic acid

To a mixture of ethyl (3S)-3-(1,4-dimethyl-1H-benzotriazol-5-yl)-3-{7-[(6'-hydroxy-3'H-spiro[cyclopropane-1,2'-pyrido[3,4-f][1,4]oxazepin]-4'(5'H)-yl)methyl]-1-benzothiophen-5-yl}propanoate (118 mg) of Example 82f, tetrahydrofuran (1.0 mL) and methanol (0.50 mL) was added 1 M aqueous sodium hydroxide solution (0.45 mL), and the mixture was stirred at 40°C for 75 min. The reaction mixture was cooled to room temperature, saturated aqueous ammonium chloride solution was added, and the mixture was extracted with ethyl acetate. The organic layer was washed with saturated brine and dried over anhydrous magnesium sulfate. The solvent was evaporated under reduced pressure and the obtained residue was purified by silica gel column chromatography [elution solvent: chloroform/methanol=100/0-92/8(V/V)]. Ethyl acetate and n-hexane were added to the obtained residue, and an ultrasonic treatment was performed. The solid was collected by filtration to give the title compound (51.7 mg, yield: 62%) as a solid.

### (Example 83)

### (3R)-3-(7-{[(6S)-2-Amino-6-ethyl-5,6,7,9-tetrahydro-8H-pyrido[2,3-c]azepin-8-yl]methyl}-1-benzothiophen-5-yl)-3-(1,4-dimethyl-1H-benzotriazol-5-yl)propanoic acid

### (83a) tert-Butyl (6S)-6-ethyl-5,6,7,9-tetrahydro-8H-pyrido[2,3-c]azepine-8-carboxylate 1-oxide

To a solution of tert-butyl (6S)-6-ethyl-5,6,7,9-tetrahydro-8H-pyrido[2,3-c]azepine-8-carboxylate (533 mg) of Example 64a in dichloromethane (19 mL) was added 3-chloroperbenzoic acid (about 70%) (951 mg) at 0°C, and the mixture was stirred at room temperature for 18 hr. To the reaction mixture were added saturated aqueous sodium hydrogen carbonate solution and 5% aqueous sodium thiosulfate solution, and the mixture was extracted with dichloromethane. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=20/80/0-0/100/0-0/90/10(V/V/V)] to give the title compound (527 mg, yield: 94%) as an oil.

### (83b) tert-Butyl (6S)-2-(tert-butylamino)-6-ethyl-5,6,7,9-tetrahydro-8H-pyrido[2,3-c]azepine-8-carboxylate

To a solution of tert-butyl (6S)-6-ethyl-5,6,7,9-tetrahydro-8H-pyrido[2,3-c]azepine-8-carboxylate 1-oxide (431 mg) of Example 83a and tert-butylamine (0.790 mL) in benzotrifluoride (7.4 mL) was added trifluoromethanesulfonic anhydride (0.496 mL) at 0°C, and the mixture was stirred at 0°C for 30 min. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=100/0-90/10(V/V)] to give the title compound (144 mg, yield: 28%) as an oil.

### (83c) (6S)-6-Ethyl-6,7,8,9-tetrahydro-5H-pyrido[2,3-c]azepin-2-amine

tert-Butyl (6S)-2-(tert-butylamino)-6-ethyl-5,6,7,9-tetrahydro-8H-pyrido[2,3-c]azepine-8-carboxylate (170 mg) of Example 83b was dissolved in trifluoroacetic acid (4.9 mL), and the mixture was stirred at 70°C for 4 hr and cooled to room temperature. The reaction mixture was evaporated to remove the solvent under reduced pressure, and the obtained residue was purified by NH silica gel column chromatography [elution solvent: n-hexane/ethyl acetate/methanol=30/70/0-0/100/0-0/90/10 (V/V/V) ] to give the title compound (86.0 mg, yield: 92%) as a solid.

### (83d) Ethyl (3R)-3-(7-{[(6S)-2-amino-6-ethyl-5,6,7,9-tetrahydro-8H-pyrido[2,3-c]azepin-8-yl]methyl}-1-benzothiophen-5-yl)-3-(1,4-dimethyl-1H-benzotriazol-5-yl)propanoate

To a solution of ethyl (3R)-3-(1,4-dimethyl-1H-benzotriazol-5-yl)-3-[7-(hydroxymethyl)-1-benzothiophen-5-yl]propanoate (49.1 mg) of Example 45l in dichloromethane (2.0 mL) was added thionyl chloride (0.0183 mL), and the mixture was stirred at room temperature for 1 hr. The solvent was evaporated under reduced pressure, and the residue was azeotropically distilled with toluene. The obtained residue was dissolved in acetonitrile (1.0 mL), added to a separately-prepared solution of N,N-diisopropylethylamine (0.174 mL) and (6S)-6-ethyl-6,7,8,9-tetrahydro-5H-pyrido[2,3-c]azepin-2-amine (19.1 mg) of Example 83c in acetonitrile (1.0 mL), and the mixture was stirred at 80°C for 6 hr and cooled to room temperature. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=30/70-0/100(V/V)] to give the title compound (58.0 mg, yield: quantitative) as an oil.

### (83e)=(83)

### (3R)-3-(7-{[(6S)-2-Amino-6-ethyl-5,6,7,9-tetrahydro-8H-pyrido[2,3-c]azepin-8-yl]methyl}-1-benzothiophen-5-yl)-3-(1,4-dimethyl-1H-benzotriazol-5-yl)propanoic acid

To a mixture of ethyl (3R)-3-(7-{[(6S)-2-amino-6-ethyl-5,6,7,9-tetrahydro-8H-pyrido[2,3-c]azepin-8-yl]methyl}-1-benzothiophen-5-yl)-3-(1,4-dimethyl-1H-benzotriazol-5-yl)propanoate (58.0 mg) of Example 83d, methanol (1.0 mL), and tetrahydrofuran (1.0 mL) was added 1 M aqueous sodium hydroxide solution (0.697 mL), and the mixture was stirred at room temperature for 24 hr. To the reaction mixture was added 1 M hydrochloric acid (0.697 mL) and the mixture was stirred and concentrated under reduced pressure. Saturated aqueous ammonium chloride solution and saturated brine were added thereto, and the mixture was extracted with chloroform/2-propanol=3/1(V/V). The organic layer was dried over anhydrous sodium sulfate. After filtration, the filtrate was concentrated under reduced pressure and the obtained residue was purified by silica gel column chromatography [elution solvent: n-hexane/ethyl acetate/dichloromethane/methanol=30/70/0/0-0/100/0/0-0/0/100/0-0/0/85/15(V/V/V/V)]. Ethyl acetate and n-hexane were added to the obtained solid, and an ultrasonic treatment was performed. The solid was collected by filtration and vacuum dried to give the title compound (55.0 mg, yield: quantitative) as a solid.

### (Example 84)

### (3S)-3-(4-Chloro-1-methyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoic acid

### (84a) Ethyl (3S)-3-(4-chloro-1-methyl-1H-benzotriazol-5-yl)-3-[7-(hydroxymethyl)-1-benzothiophen-5-yl]propanoate

Ethyl (2E)-3-(4-chloro-1-methyl-1H-benzotriazol-5-yl)prop-2-enoate (WO 2015/092713) (11.0 g), and chloro(1,5-cyclooctadiene)rhodium (I) (3.1 g) were added to 1,4-dioxane (200 mL), purged with nitrogen, and dissolved at 60°C. Thereto were added [5-(4,4,5,5-tetramethyl-1,3,2-dioxaborolan-2-yl)-1-benzothiophen-7-yl]methanol (16.0 g) of Example 1e, and then 1 M aqueous potassium hydroxide solution (41.4 mL), and the mixture was stirred with heating at 60°C for 5 hr. The reaction mixture was cooled to room temperature, the solvent was evaporated under reduced pressure and the obtained residue was purified by silica gel column chromatography [elution solvent: petroleum ether/ethyl acetate=10/1-1/1(V/V)] to give the title compound (racemate). This was subjected to chiral SFC [column: CHIRALPAK AD (30 mm I.D.× 250 mm), mobile phase: carbon dioxide/methanol (0.1% aqueous ammonia)=60/40(V/V)] to give 2.8 g of the title compound as the first peak (yield: 16%).
retention time: 1.823 min [column: CHIRALPAK AD-3 (4.6 mm I.D. × 50 mm), mobile phase: carbon dioxide/methanol (0.05% diethylamine)=95/5-60/40(V/V), flow rate: 3 mL/min, temperature: 35°C, wavelength: 220 nm]

### (84b) Ethyl (3S)-3-[7-(chloromethyl)-1-benzothiophen-5-yl]-3-(4-chloro-1-methyl-1H-benzotriazol-5-yl)propanoate

To a solution of ethyl (3S)-3-(4-chloro-1-methyl-1H-benzotriazol-5-yl)-3-[7-(hydroxymethyl)-1-benzothiophen-5-yl]propanoate (200 mg) of Example 84a in dichloromethane (5.0 mL) was added thionyl chloride (0.053 mL), and the mixture was stirred at room temperature for 1 hr. The solvent was evaporated under reduced pressure, and the residue was azeotropically distilled with toluene. The obtained residue was dried under reduced pressure to give the title compound (200 mg, yield: 96%) as an oil.

### (84c) Ethyl (3S)-3-(4-chloro-1-methyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate

To a solution of ethyl (3S)-3-[7-(chloromethyl)-1-benzothiophen-5-yl]-3-(4-chloro-1-methyl-1H-benzotriazol-5-yl)propanoate (100 mg) of Example 84b in acetonitrile (5 mL) were added N,N-diisopropylethylamine (0.38 mL) and (6S)-6-ethyl-6,7,8,9-tetrahydro-5H-pyrido[2,3-c]azepin-2-ol hydrochloride (54 mg) of Example 72f, and the mixture was stirred at 85°C for 15 hr and cooled to room temperature. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=20/80/0-0/100/0-0/90/10(V/V/V)] to give the title compound (68 mg, yield: 50%) as an oil.

### (84d)=(84)

### (3S)-3-(4-Chloro-1-methyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoic acid

To a mixture of ethyl (3S)-3-(4-chloro-1-methyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate (68 mg) of Example 84c, ethanol (3 mL), and tetrahydrofuran (3 mL) was added 1 M aqueous sodium hydroxide solution (3 mL), and the mixture was stirred at room temperature for 12 hr. To the reaction mixture was added 1 M hydrochloric acid (3 mL) and the mixture was stirred and extracted with ethyl acetate. The organic layer was washed with saturated brine and dried over anhydrous sodium sulfate. Under reduced pressure, the solvent was evaporated and the obtained residue was purified by silica gel column chromatography [elution solvent: dichloromethane/methanol=10/0-9/1(V/V)] to give the title compound (53 mg, yield: 82%) as a solid.

### (Example 85)

### (3S)-3-(7-Chloro-1,4-dimethyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoic acid

### (85a) Ethyl (3S)-3-(7-chloro-1,4-dimethyl-1H-benzotriazol-5-yl)-3-[7-(hydroxymethyl)-1-benzothiophen-5-yl]propanoate

Ethyl 3-(7-chloro-1,4-dimethyl-1H-benzotriazol-5-yl)-3-[7-(hydroxymethyl)-1-benzothiophen-5-yl]propanoate (1.61 g) of Example 31e was subjected to chiral HPLC [column: CHIRALCEL OJ-H (20 mm I.D. × 250 mm), mobile phase: n-hexane/2-propanol=50/50(V/V)] to give the title compound (593 mg) as the first peak (yield: 37%).
retention time: 7.797 min [column: CHIRALCEL OJ-H (4.6 mm I.D. × 250 mm), mobile phase: n-hexane/2-propanol=50/50(V/V), flow rate: 1.0 mL/min, temperature: 40°C, wavelength: 210 nm]

### (85b) Ethyl (3S)-3-(7-chloro-1,4-dimethyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate

To a solution of ethyl (3S)-3-(7-chloro-1,4-dimethyl-1H-benzotriazol-5-yl)-3-[7-(hydroxymethyl)-1-benzothiophen-5-yl]propanoate (100 mg) of Example 85a in dichloromethane (5 mL) was added thionyl chloride (0.049 mL), and the mixture was stirred at room temperature for 1 hr. The solvent was evaporated under reduced pressure, and the residue was azeotropically distilled with toluene. The obtained residue was dissolved in acetonitrile (5 mL), N,N-diisopropylethylamine (0.39 mL) and (6S)-6-ethyl-6,7,8,9-tetrahydro-5H-pyrido[2,3-clazepin-2-ol hydrochloride (52 mg) of Example 72f were added, and the mixture was stirred at 85°C for 15 hr and cooled to room temperature. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate/methanol=50/50/0-0/100/0-0/95/5(V/V/V)] to give the title compound (100 mg, yield: 72%) as an oil.

### (85c)=(85)

### (3S)-3-(7-Chloro-1,4-dimethyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoic acid

To a mixture of ethyl (3S)-3-(7-chloro-1,4-dimethyl-1H-benzotriazol-5-yl)-3-(7-{[(6S)-6-ethyl-2-hydroxy-5,6,7,9-tetrahydro-8H-pyrido[2,3-c]azepin-8-yl]methyl}-1-benzothiophen-5-yl)propanoate (100 mg) of Example 85b, ethanol (5 mL), and tetrahydrofuran (2 mL) was added 1 M aqueous sodium hydroxide solution (5 mL), and the mixture was stirred at room temperature for 12 hr. To the reaction mixture was added 1 M hydrochloric acid (5 mL) and the mixture was stirred and extracted with ethyl acetate. The organic layer was washed with saturated brine and dried over anhydrous sodium sulfate. Under reduced pressure, the solvent was evaporated and the obtained residue was purified by silica gel column chromatography [elution solvent: dichloromethane/methanol=10/1-95/5(V/V)] to give the title compound (50 mg, yield: 52%) as a solid.

### (Example 86)

### (3S)-3-(7-{[(6S)-6-Ethyl-2-hydroxy-5,6,7,9-tetrahydro-8H-pyrido[2,3-c]azepin-8-yl]methyl}-1-benzothiophen-5-yl)-3-(1,4,7-trimethyl-1H-benzotriazol-5-yl)propanoic acid

### (86a) Ethyl (3S)-3-[7-(hydroxymethyl)-1-benzothiophen-5-yl]-3-(1,4,7-trimethyl-1H-benzotriazol-5-yl)propanoate

Ethyl 3-[7-(hydroxymethyl)-1-benzothiophen-5-yl]-3-(1,4,7-trimethyl-1H-benzotriazol-5-yl)propanoate (270 mg) of Example 17j was subjected to chiral HPLC [column: CHIRALART Cellulose-SZ (20 mm I.D. × 250 mm), mobile phase: n-hexane:ethanol=80:20] to give the title compound (120 mg) as the first peak (yield: 44%).
retention time: 15.6 min [column: CHIRALART Cellulose-SZ (4.6 mm I.D. × 250 mm), mobile phase: n-hexane:ethanol=80:20, flow rate: 1.0 mL/min, temperature: 40°C, wavelength: 254 nm]

### (86b) Ethyl (3S)-3-(7-{[(6S)-6-ethyl-2-hydroxy-5,6,7,9-tetrahydro-8H-pyrido[2,3-c]azepin-8-yl]methyl}-1-benzothiophen-5-yl)-3-(1,4,7-trimethyl-1H-benzotriazol-5-yl)propanoate

To a solution of ethyl (3S)-3-[7-(hydroxymethyl)-1-benzothiophen-5-yl]-3-(1,4,7-trimethyl-1H-benzotriazol-5-yl)propanoate (100 mg) of Example 86a in dichloromethane (5 mL) was added thionyl chloride (0.055 mL), and the mixture was stirred at room temperature for 1 hr. The solvent was evaporated under reduced pressure, and the residue was azeotropically distilled with toluene. The obtained residue was dissolved in acetonitrile (5 mL), N,N-diisopropylethylamine (0.36 mL) and (6S)-6-ethyl-6,7,8,9-tetrahydro-5H-pyrido[2,3-c]azepin-2-ol hydrochloride (48 mg) of Example 72f were added and the mixture was stirred at 85°C for 15 hr and cooled to room temperature. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: ethyl acetate/methanol=100/0-90/10(V/V)] and further purified by NH silica gel column chromatography [elution solvent: ethyl acetate/methanol=100/0-97/3(V/V)] to give the title compound (68 mg, yield: 54%) as a solid.

### (86c)=(86)

### (3S)-3-(7-{[(6S)-6-Ethyl-2-hydroxy-5,6,7,9-tetrahydro-8H-pyrido[2,3-c]azepin-8-yl]methyl}-1-benzothiophen-5-yl)-3-(1,4,7-trimethyl-1H-benzotriazol-5-yl)propanoic acid

To a mixture of ethyl (3S)-3-(7-{[(6S)-6-ethyl-2-hydroxy-5,6,7,9-tetrahydro-8H-pyrido[2,3-c]azepin-8-yl]methyl}-1-benzothiophen-5-yl)-3-(1,4,7-trimethyl-1H-benzotriazol-5-yl)propanoate (68 mg) of Example 86b, ethanol (3 mL), and tetrahydrofuran (3 mL) was added 1 M aqueous sodium hydroxide solution (3 mL), and the mixture was stirred at room temperature for 12 hr. To the reaction mixture was added 1 M hydrochloric acid (3 mL) and the mixture was stirred and extracted with ethyl acetate. The organic layer was washed with saturated brine and dried over anhydrous sodium sulfate. Under reduced pressure, the solvent was evaporated and the obtained residue was purified by silica gel column chromatography [elution solvent: chloroform/methanol=10/1-9/1(V/V)] to give the title compound (55 mg, yield: 85%) as a solid.

### (Example 87)

### (3R)-3-(7-{[(6S)-6-Ethyl-2-hydroxy-5,6,7,9-tetrahydro-8H-pyrido[2,3-c]azepin-8-yl]methyl}-1-benzothiophen-5-yl)-3-(1,4,7-trimethyl-1H-benzotriazol-5-yl)propanoic acid

### (87a) Ethyl (3R)-3-[7-(hydroxymethyl)-1-benzothiophen-5-yl]-3-(1,4,7-trimethyl-1H-benzotriazol-5-yl)propanoate

Ethyl 3-[7-(hydroxymethyl)-1-benzothiophen-5-yl]-3-(1,4,7-trimethyl-1H-benzotriazol-5-yl)propanoate (270 mg) of Example 17j was subjected to chiral HPLC [column: CHIRALART Cellulose-SZ (20 mm I.D. × 250 mm), mobile phase: n-hexane:ethanol=80:20] to give the title compound (126 mg) as the second peak (yield: 47%).
retention time: 17.4 min [column: CHIRALART Cellulose-SZ (4.6 mm I.D. × 250 mm), mobile phase: n-hexane:ethanol=80:20, flow rate: 1.0 mL/min, temperature: 40°C, wavelength: 254 nm]

### (87b) Ethyl (3R)-3-(7-{[(6S)-6-ethyl-2-hydroxy-5,6,7,9-tetrahydro-8H-pyrido[2,3-c]azepin-8-yl]methyl}-1-benzothiophen-5-yl)-3-(1,4,7-trimethyl-1H-benzotriazol-5-yl)propanoate

To a solution of ethyl (3R)-3-[7-(hydroxymethyl)-1-benzothiophen-5-yl]-3-(1,4,7-trimethyl-1H-benzotriazol-5-yl)propanoate (100 mg) of Example 87a in dichloromethane (5 mL) was added thionyl chloride (0.055 mL), and the mixture was stirred at room temperature for 1 hr. The solvent was evaporated under reduced pressure, and the residue was azeotropically distilled with toluene. The obtained residue was dissolved in acetonitrile (5 mL), N,N-diisopropylethylamine (0.36 mL) and (6S)-6-ethyl-6,7,8,9-tetrahydro-5H-pyrido[2,3-c]azepin-2-ol hydrochloride (48 mg) of Example 72f were added and the mixture was stirred at 85°C for 15 hr and cooled to room temperature. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: ethyl acetate/methanol=100/0-90/10(V/V)] to give the title compound (70 mg, yield: 56%) as a solid.

### (87c)=(87)

### (3R)-3-(7-{[(6S)-6-Ethyl-2-hydroxy-5,6,7,9-tetrahydro-8H-pyrido[2,3-c]azepin-8-yl]methyl}-1-benzothiophen-5-yl)-3-(1,4,7-trimethyl-1H-benzotriazol-5-yl)propanoic acid

To a mixture of ethyl (3R)-3-(7-{[(6S)-6-ethyl-2-hydroxy-5,6,7,9-tetrahydro-8H-pyrido[2,3-c]azepin-8-yl]methyl}-1-benzothiophen-5-yl)-3-(1,4,7-trimethyl-1H-benzotriazol-5-yl)propanoate (70 mg) of Example 87b, ethanol (3 mL), and tetrahydrofuran (3 mL) was added 1 M aqueous sodium hydroxide solution (3 mL), and the mixture was stirred at room temperature for 12 hr. To the reaction mixture was added 1 M hydrochloric acid (3 mL) and the mixture was stirred and extracted with ethyl acetate. The organic layer was washed with saturated brine and dried over anhydrous sodium sulfate. Under reduced pressure, the solvent was evaporated and the obtained residue was purified by silica gel column chromatography [elution solvent: chloroform/methanol=10/1-85/15(V/V)] to give the title compound (38 mg, yield: 57%) as a solid.

### (Example 88)

### (3R)-3-(7-{[(6S')-6-cyclopropyl-2-hydroxy-5,6,7,9-tetrahydro-8H-pyrido[2,3-c]azepin-8-yl]methyl}-1-benzothiophen-5-yl)-3-(1,4-dimethyl-1H-benzotriazol-5-yl)propanoic acid

### (Example 89)

### (3R)-3-(7-{[(6R') -6-Cyclopropyl-2-hydroxy-5,6,7,9-tetrahydro-8H-pyrido[2,3-c]azepin-8-yl]methyl}-1-benzothiophen-5-yl)-3-(1,4-dimethyl-1H-benzotriazol-5-yl)propanoic acid

### (88a) Methyl 3-(6-chloro-2-cyanopyridin-3-yl)-2-cyclopropylpropanoate

To a solution of 6-chloro-3-methylpyridine-2-carbonitrile (WO 2009/155156) (9.70 g) in carbon tetrachloride (400 mL) were added N-bromosuccinimide (36.1 g) and 2,2'-azobisisobutyronitrile (2.09 g), and the mixture was heated under reflux for 21 hr. The reaction mixture was cooled to room temperature, and insoluble material was filtered off. To the filtrate was added water, and the mixture was extracted with chloroform. The organic layer was dried over anhydrous sodium sulfate. The solvent was evaporated under reduced pressure and the obtained residue was dissolved in tetrahydrofuran (200 mL). N,N-diisopropylethylamine (22.2 mL) and diethyl phosphite(12.3 mL) were added at 0°C and the mixture was stirred at room temperature for 1 hr. To the reaction mixture was added water, and the mixture was extracted with ethyl acetate. The organic layer was washed successively with water and saturated brine, and dried over anhydrous sodium sulfate. Under reduced pressure, the solvent was evaporated and the obtained residue was purified by silica gel column chromatography [elution solvent: n-hexane/ethyl acetate/chloroform=95/5/20-70/30/20(V/V/V)] to give a crude product (14.5 g) containing 3-(bromomethyl)-6-chloropyridine-2-carbonitrile as a solid. Separately, to a solution of N,N-diisopropylamine (13.2 mL) in tetrahydrofuran (75 mL) was added dropwise n-butyllithium (1.6 M n-hexane solution) (58.7 mL) at 0°C, a solution of methyl cyclopropylacetate (9.33 g) in tetrahydrofuran (55 mL) was added dropwise at -78°C, and the mixture was stirred at -78°C for 25 min. To the reaction mixture was added dropwise a solution of a crude product (14.5 g) containing 3-(bromomethyl)-6-chloropyridine-2-carbonitrile in tetrahydrofuran (55 mL), and the mixture was stirred at - 78°C for 1 hr. To the reaction mixture were added saturated aqueous ammonium solution, acetic acid and 2 M hydrochloric acid, and the mixture was extracted with ethyl acetate. The organic layer was washed successively with water and saturated brine, and dried over anhydrous sodium sulfate. The solvent was evaporated under reduced pressure and the obtained residue was dissolved in N,N-dimethylformamide (120 mL). Potassium acetate (6.14 g) was added thereto, and the mixture was stirred at room temperature for 11.5 hr. To the reaction mixture were added water and saturated aqueous ammonium chloride solution, and the mixture was extracted with ethyl acetate. The organic layer was washed successively with water and saturated brine, and dried over anhydrous sodium sulfate. Under reduced pressure, the solvent was evaporated and the obtained residue was purified by silica gel column chromatography [elution solvent: n-hexane/ethyl acetate=90/10-60/40(V/V)] to give the title compound (11.3 g, yield: 68%) as an oil.

### (88b) Methyl 3-(2-{[(tert-butoxycarbonyl)amino]methyl}-6-chloropyridin-3-yl)-2-cyclopropylpropanoate

To a suspension of platinum(IV) oxide n hydrate (670 mg) in chloroform (80 mL) was added a solution of methyl 3-(6-chloro-2-cyanopyridin-3-yl)-2-cyclopropylpropanoate (6.72 g) of Example 88a in methanol (260 mL), and the mixture was stirred at room temperature under a hydrogen atmosphere at normal pressure for 120 hr. To the reaction mixture was added celite, insoluble material was filtered off, and the filtrate was concentrated under reduced pressure. To the obtained residue was added saturated aqueous sodium hydrogen carbonate solution, and the mixture was extracted with ethyl acetate. The organic layer was washed with saturated brine and dried over anhydrous sodium sulfate. The solvent was evaporated under reduced pressure and the obtained residue was dissolved in dichloromethane (87 mL). Di-tert-butyl dicarbonate (5.97 mL) was added thereto, and the mixture was stirred at room temperature for 12 hr. Under reduced pressure, the solvent was evaporated and the obtained residue was purified by silica gel column chromatography [elution solvent: n-hexane/ethyl acetate=90/10-75/25(V/V)] to give the title compound (4.32 g, yield: 41%) as an oil.

### (88c) tert-Butyl {[6-chloro-3-(2-cyclopropyl-3-hydroxypropyl)pyridin-2-yl]methyl}carbamate

To a solution of methyl 3-(2-{[(tert-butoxycarbonyl)amino]methyl}-6-chloropyridin-3-yl)-2-cyclopropylpropanoate (4.18 g) of Example 88b in dichloromethane (60 mL) was added diisobutylaluminum hydride (1 M toluene solution) (34.0 mL) at 0°C, and the mixture was stirred at 0°C for 1 hr. Diisobutylaluminum hydride (1 M toluene solution) (34.0 mL) was added thereto, and the mixture was stirred at 0°C for 50 min. To the reaction mixture was added saturated aqueous potassium sodium tartrate solution and the mixture was stirred at room temperature for 1.5 hr and extracted with ethyl acetate. The organic layer was dried over anhydrous sodium sulfate. Under reduced pressure, the solvent was evaporated and the obtained residue was purified by silica gel column chromatography [elution solvent: n-hexane/ethyl acetate=70/30-40/60(V/V)] to give the title compound (2.65 g, yield: 77%) as an oil.

### (88d) 3-(2-{[(tert-Butoxycarbonyl)amino]methyl}-6-chloropyridin-3-yl)-2-cyclopropylpropyl methanesulfonate

To a solution of tert-butyl {[6-chloro-3-(2-cyclopropyl-3-hydroxypropyl)pyridin-2-yl]methyl}carbamate (2.65 g) of Example 88c in dichloromethane (30 mL) were added triethylamine (2.2 mL) and methanesulfonyl chloride (0.91 mL) at 0°C, and the mixture was stirred at 0°C for 30 min. To the reaction mixture was added water, and the mixture was extracted with chloroform. The organic layer was dried over anhydrous sodium sulfate. Under reduced pressure, the solvent was evaporated and the obtained residue was purified by silica gel column chromatography [elution solvent: n-hexane/ethyl acetate=80/20-50/50(V/V)] to give the title compound (2.73 g, yield: 84%) as an oil.

### (88e) tert-Butyl 2-chloro-6-cyclopropyl-5,6,7,9-tetrahydro-8H-pyrido[2,3-c]azepine-8-carboxylate

To a suspension of 60% sodium hydride (391 mg) in N,N-dimethylformamide (33 mL) was added at 0°C a solution of 3-(2-{[(tert-butoxycarbonyl)amino]methyl}-6-chloropyridin-3-yl)-2-cyclopropylpropyl methanesulfonate (2.73 g) of Example 88d in N,N-dimethylformamide (33 mL), and the mixture was stirred at room temperature for 30 min. To the reaction mixture was added water, and the mixture was extracted with ethyl acetate. The organic layer was washed successively with water and saturated brine, and dried over anhydrous sodium sulfate. Under reduced pressure, the solvent was evaporated and the obtained residue was purified by silica gel column chromatography [elution solvent: n-hexane/ethyl acetate=90/10-75/25(V/V)] to give the title compound (1.69 g, yield: 80%) as an oil.

### (88f) tert-Butyl 6-cyclopropyl-2-hydroxy-5,6,7,9-tetrahydro-8H-pyrido[2,3-c]azepine-8-carboxylate

To a solution of tert-butyl 2-chloro-6-cyclopropyl-5,6,7,9-tetrahydro-8H-pyrido[2,3-c]azepine-8-carboxylate (1.69 g) of Example 88e in 1,4-dioxane (37 mL) were added cesium hydroxide monohydrate (2.64 g), bis(dibenzylideneacetone)palladium(0) (150 mg) and 5-(di-tert-butylphosphino)-1',3',5'-triphenyl-1'H-1,4'-bipyrazole (265 mg), and the mixture was stirred at 100°C for 3 hr. The reaction mixture was cooled to room temperature, saturated aqueous sodium hydrogen carbonate solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V), chloroform/methanol=100/0-95/5(V/V)] to give the title compound (1.16 g, yield: 73%) as an oil.

### (88g) 6-Cyclopropyl-6,7,8,9-tetrahydro-5H-pyrido[2,3-c]azepin-2-ol hydrochloride

To a solution of tert-butyl 6-cyclopropyl-2-hydroxy-5,6,7,9-tetrahydro-8H-pyrido[2,3-c]azepine-8-carboxylate (294 mg) of Example 88f in dichloromethane (0.20 mL) was added 4 M hydrogen chloride-1,4-dioxane (1.2 mL) at 0°C, and the mixture was stirred at room temperature for 2.5 hr. To the reaction mixture was added tert-butyl methyl ether, and the solid was collected by filtration to give the title compound (250 mg, yield: quantitative) as a solid.

### (88h) Ethyl (3R)-3-{7-[(6-cyclopropyl-2-hydroxy-5,6,7,9-tetrahydro-8H-pyrido[2,3-c]azepin-8-yl)methyl]-1-benzothiophen-5-yl}-3-(1,4-dimethyl-1H-benzotriazol-5-yl)propanoate

To a solution of ethyl (3R)-3-(1,4-dimethyl-1H-benzotriazol-5-yl)-3-[7-(hydroxymethyl)-1-benzothiophen-5-yl]propanoate (328 mg) of Example 45l in dichloromethane (2.4 mL) were added dimethyl sulfoxide (0.28 mL), N,N-diisopropylethylamine (0.42 mL) and sulfur trioxide-pyridine complex (424 mg), and the mixture was stirred at room temperature for 40 min. To the reaction mixture was added water, and the mixture was extracted with chloroform. The organic layer was dried over anhydrous sodium sulfate. The solvent was evaporated under reduced pressure and the obtained residue was dissolved in dichloromethane (2.4 mL). 6-Cyclopropyl-6,7,8,9-tetrahydro-5H-pyrido[2,3-c]azepin-2-ol hydrochloride (212 mg) of Example 88g, N,N-diisopropylethylamine (0.15 mL) and 4 drops of acetic acid were added and the mixture was stirred at room temperature for 45 min. Sodium triacetoxyborohydride (424 mg) and anhydrous magnesium sulfate (193 mg) were added thereto, and the mixture was stirred at 35°C for 14.5 hr. The reaction mixture was cooled to room temperature, saturated aqueous ammonium chloride solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=50/50-0/100(V/V), chloroform/methanol=100/0-95/5(V/V)] to give the title compound (374 mg, yield: 78%) as an oil.

### (881)=(88)

### (3R)-3-(7-f((6S*)-6-Cyclopropyl-2-hydroxy-5,6,7,9-tetrahydro-8H-pyrido[2,3-c]azepin-8-yl]methyl}-1-benzothiophen-5-yl)-3-(1,4-dimethyl-1H-benzotriazol-5-yl)propanoic acid

### (89a)=(89)

### (3R)-3-(7-{[(6R*)-6-Cyclopropyl-2-hydroxy-5,6,7,9-tetrahydro-8H-pyrido[2,3-c]azepin-8-yl]methyl}-1-benzothiophen-5-yl)-3-(1,4-dimethyl-1H-benzotriazol-5-yl)propanoic acid

To a mixture of ethyl (3R)-3-{7-[(6-cyclopropyl-2-hydroxy-5,6,7,9-tetrahydro-8H-pyrido[2,3-c]azepin-8-yl)methyl]-1-benzothiophen-5-yl}-3-(1,4-dimethyl-1H-benzotriazol-5-yl)propanoate (465 mg) of Example 88h, tetrahydrofuran (4.6 mL) and methanol (2.3 mL) was added 1 M aqueous lithium hydroxide solution (2.3 mL), and the mixture was stirred at 40°C for 1.5 hr. The reaction mixture was cooled to room temperature, saturated aqueous ammonium chloride solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: chloroform/methanol=100/0-95/5(V/V)]. Ethyl acetate and n-hexane were added to the compound eluted earlier, and an ultrasonic treatment was performed. The solid was collected by filtration to give (3R)-3-(7-{[(6R*)-6-cyclopropyl-2-hydroxy-5,6,7,9-tetrahydro-8H-pyrido[2,3-c]azepin-8-yl]methyl}-1-benzothiophen-5-yl)-3-(1,4-dimethyl-1H-benzotriazol-5-yl)propanoic acid (128 mg, yield: 29%) as a solid.

In the same manner, moreover, the compound eluted later, (3R)-3-(7-{[(6S*)-6-cyclopropyl-2-hydroxy-5,6,7,9-tetrahydro-8H-pyrido[2,3-c]azepin-8-yl]methyl}-1-benzothiophen-5-yl)-3-(1,4-dimethyl-1H-benzotriazol-5-yl)propanoic acid (116 mg, yield: 26%) was obtained as a solid.

### (Example 90)

### (3R)-3-(7-{[(6R*)-6-Cyclopropyl-2-hydroxy-5,6,7,9-tetrahydro-8H-pyrido[2,3-c]azepin-8-yl]methyl}-1-benzothiophen-5-yl)-3-(3,7-dimethyl-3H-[1,2,3]triazolo[4,5-b]pyridin-6-yl)propanoic acid

### (Example 91)

### (3R)-3-(7-{[(6S*)-6-Cyclopropyl-2-hydroxy-5,6,7,9-tetrahydro-8H-pyrido[2,3-c]azepin-8-yl]methyl}-1-benzothiophen-5-yl)-3-(3,7-dimethyl-3H-[1,2,3]triazolo[4,5-b]pyridin-6-yl)propanoic acid

### (90a) Ethyl (3R)-3-{7-[(6-cyclopropyl-2-hydroxy-5,6,7,9-tetrahydro-8H-pyrido[2,3-c]azepin-8-yl)methyl]-1-benzothiophen-5-yl}-3-(3,7-dimethyl-3H-[1,2,3]triazolo[4,5-b]pyridin-6-yl)propanoate

To a solution of ethyl (3R)-3-(3,7-dimethyl-3H-[1,2,3]triazolo[4,5-b]pyridin-6-yl)-3-[7-(hydroxymethyl)-1-benzothiophen-5-yl]propanoate (282 mg) of Example 46a in dichloromethane (2.8 mL) were added dimethyl sulfoxide (0.24 mL), N,N-diisopropylethylamine (0.36 mL) and sulfur trioxide-pyridine complex (328 mg), and the mixture was stirred at room temperature for 10 min. To the reaction mixture was added water, and the mixture was extracted with chloroform. The organic layer was dried over anhydrous sodium sulfate. The solvent was evaporated under reduced pressure and the obtained residue was dissolved in dichloromethane (2.3 mL). 6-Cyclopropyl-6,7,8,9-tetrahydro-5H-pyrido[2,3-c]azepin-2-ol hydrochloride (138 mg) of Example 88h, N,N-diisopropylethylamine (0.10 mL), acetic acid (0.0065 mL) and anhydrous magnesium sulfate (138 mg) were added, and the mixture was stirred at 35°C for 45 min. Then, sodium triacetoxyborohydride (244 mg) was added thereto, and the mixture was stirred at 35°C for 13 hr. Sodium triacetoxyborohydride (122 mg) and one drop of acetic acid were added thereto, and the mixture was stirred at 35°C for 40 min. The reaction mixture was cooled to room temperature, saturated aqueous sodium hydrogen carbonate solution was added, and the mixture was extracted with chloroform. The organic layer was dried over anhydrous sodium sulfate. Under reduced pressure, the solvent was evaporated and the obtained residue was purified by silica gel column chromatography [elution solvent: ethyl acetate/methanol=100/0-50/50(V/V)] to give the title compound (253 mg, yield: 74%) as a solid.

### (90b)=(90)

### (3R)-3-(7-{[(6R')-6-Cyclopropyl-2-hydroxy-5,6,7,9-tetrahydro-8H-pyrido[2,3-c]azepin-8-yl]methyl}-1-benzothiophen-5-yl)-3-(3,7-dimethyl-3H-[1,2,3]triazolo[4,5-b]pyridin-6-yl)propanoic acid

### (91a)=(91)

### (3R)-3-(7-{[(6S*)-Cyclopropyl-2-hydroxy-5,6,7,9-tetrahydro-8H-pyrido[2,3-c]azepin-8-yl]methyl}-1-benzothiophen-5-yl)-3-(3,7-dimethyl-3H-[1,2,3]triazolo[4,5-b]pyridin-6-yl)propanoic acid

To a mixture of ethyl (3R)-3-{7-[(6-cyclopropyl-2-hydroxy-5,6,7,9-tetrahydro-8H-pyrido[2,3-c]azepin-8-yl)methyl]-1-benzothiophen-5-yl}-3-(3,7-dimethyl-3H-[1,2,3]triazolo[4,5-b]pyridin-6-yl)propanoate (253 mg) of Example 90a, tetrahydrofuran (3.9 mL) and methanol (1.3 mL) was added 1 M aqueous lithium hydroxide solution (1.3 mL), and the mixture was stirred at 40°C for 1.5 hr. The reaction mixture was cooled to room temperature, saturated aqueous ammonium chloride solution was added, and the mixture was extracted with chloroform. The organic layer was dried over anhydrous sodium sulfate. Under reduced pressure, the solvent was evaporated and the obtained residue was purified by silica gel column chromatography [elution solvent: chloroform/methanol=100/0-90/10(V/V)]. To the compound eluted earlier were added ethyl acetate and n-hexane, and an ultrasonic treatment was performed. The solid was collected by filtration to give (3R)-3-(7-{[(6S*)-cyclopropyl-2-hydroxy-5,6,7,9-tetrahydro-8H-pyrido[2,3-c]azepin-8-yl]methyl}-1-benzothiophen-5-yl)-3-(3,7-dimethyl-3H-[1,2,3]triazolo[4,5-b]pyridin-6-yl)propanoic acid (85 mg, yield: 35%) as a solid.

In the same manner, moreover, the compound eluted later, (3R)-3-(7-{[(6R*)-cyclopropyl-2-hydroxy-5,6,7,9-tetrahydro-8H-pyrido[2,3-c]azepin-8-yl]methyl}-1-benzothiophen-5-yl)-3-(3,7-dimethyl-3H-[1,2,3]triazolo[4,5-b]pyridin-6-yl)propanoic acid (83 mg, yield: 35%), was obtained as a solid.

### (Example 92)

### 3-(1-Ethyl-4-methyl-1H-benzotriazol-5-yl)-3-(7-{[(2R)-2-ethyl-7-methyl-2,3-dihydropyrido[2,3-f] [1,4]oxazepin-4(5H)-yl]methyl}-1-benzothiophen-5-yl)propanoic acid

### (92a) tert-Butyl [(2S)-2-hydroxybutyl][(3-hydroxy-6-methylpyridin-2-yl)methyl]carbamate

To a solution of 3-hydroxy-6-methylpyridine-2-carbaldehyde (3.32 g) in dichloromethane (155 mL) were added (2S)-1-aminobutan-2-ol (4.25 g) and sodium triacetoxyborohydride (12 g), and the mixture was stirred at room temperature for 30 min. To the reaction mixture was added saturated aqueous sodium hydrogen carbonate solution, and the mixture was extracted with chloroform. The organic layer was dried over anhydrous sodium sulfate. The solvent was evaporated under reduced pressure and the obtained residue was dissolved in tetrahydrofuran (150 mL). 2 M Aqueous sodium carbonate solution (45 mL) was added thereto, di-tert-butyl dicarbonate (10 mL) was added dropwise thereto at 0°C, and the mixture was stirred at room temperature for 1 hr. Thereto was added di-tert-butyl dicarbonate (3.0 mL) and the mixture was stirred at room temperature for 30 min. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=95/5-30/70(V/V)] to give the title compound (4.70 g, yield: 49%) as an oil.

### (92b) tert-Butyl (2R)-2-ethyl-7-methyl-2,3-dihydropyrido[2,3-f] [1,4]oxazepine-4(5H)-carboxylate

To a solution of tert-butyl [(2S)-2-hydroxybutyl][(3-hydroxy-6-methylpyridin-2-yl)methyl]carbamate (1.50 g) of Example 92a in tetrahydrofuran (50 mL) were added tri-n-butyl phosphine (1.5 mL) and 1,1'-(azodicarbonyl)dipiperidine (1.46 g), and the mixture was stirred at room temperature for 10 min. Under reduced pressure, the solvent was evaporated and the obtained residue was purified by silica gel column chromatography [elution solvent: n-hexane/ethyl acetate=90/10-20/80(V/V)] to give the title compound (463 mg, yield: 33%) as an oil.

### (92c) (2R)-2-Ethyl-7-methyl-2,3,4,5-tetrahydropyrido[2,3-f] [1,4]oxazepine hydrochloride

A solution of tert-butyl (2R)-2-ethyl-7-methyl-2,3-dihydropyrido[2,3-f][1,4]oxazepine-4(5H)-carboxylate (463 mg) of Example 92b in 4 M hydrogen chloride-1,4-dioxane (2.0 mL) was stirred at room temperature for 11.5 hr. To the reaction mixture was added tert-butyl methyl ether, and insoluble material was collected by filtration to give the title compound (383 mg, yield: quantitative) as a solid.

### (92d) Ethyl 3-(1-ethyl-4-methyl-1H-benzotriazol-5-yl)-3-[7-(hydroxymethyl)-1-benzothiophen-5-yl]propanoate

To a mixture of [5-(4,4,5,5-tetramethyl-1,3,2-dioxaborolan-2-yl)-1-benzothiophen-7-yl]methanol (182 mg) of Example 1e, ethyl (2E)-3-(1-ethyl-4-methyl-1H-benzotriazol-5-yl)prop-2-enoate (WO 2015/092713) (203 mg), 1,4-dioxane (1.4 mL) and water (0.70 mL) was added triethylamine (0.20 mL), and the mixture was stirred at 90°C for 5 min. Then, chloro(1,5-cyclooctadiene)rhodium(I) dimer (17 mg) was added thereto, and the mixture was stirred at 90°C for 20 min. The reaction mixture was cooled to room temperature, water was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=80/20-50/50(V/V)] to give the title compound (130 mg, yield: 44%) as a solid.

### (92e) Ethyl 3-(1-ethyl-4-methyl-1H-benzotriazol-5-yl)-3-(7-{[(2R)-2-ethyl-7-methyl-2,3-dihydropyrido[2,3-f][1,4]oxazepin-4(5H)-yl]methyl}-1-benzothiophen-5-yl)propanoate

To a solution of ethyl 3-(1-ethyl-4-methyl-1H-benzotriazol-5-yl)-3-[7-(hydroxymethyl)-1-benzothiophen-5-yl]propanoate (120 mg) of Example 92d in dichloromethane (1.4 mL) were added N,N-diisopropylethylamine (0.15 mL), dimethyl sulfoxide (0.10 mL) and sulfur trioxide-pyridine complex (110 mg) and the mixture was stirred at room temperature for 40 min. To the reaction mixture were added N,N-diisopropylethylamine (0.08 mL), dimethyl sulfoxide (0.05 mL) and sulfur trioxide-pyridine complex (55 mg) and the mixture was stirred at room temperature for 30 min. To the reaction mixture were added N,N-diisopropylethylamine (0.08 mL), dimethyl sulfoxide (0.05 mL) and sulfur trioxide-pyridine complex (55 mg) and the mixture was stirred at room temperature for 40 min. To the reaction mixture was added water, and the mixture was extracted with ethyl acetate. The organic layer was washed with water and saturated brine, and dried over anhydrous sodium sulfate. The solvent was evaporated under reduced pressure and the obtained residue was dissolved in dichloromethane (1.4 mL). (2R)-2-ethyl-7-methyl-2,3,4,5-tetrahydropyrido[2,3-f] [1,4]oxazepine hydrochloride (77 mg) of Example 92c, N,N-diisopropylethylamine (0.059 mL) and acetic acid (0.003 mL) were added thereto, and the mixture was stirred at room temperature for 25 min. Sodium triacetoxyborohydride (90 mg) was added thereto, and the mixture was stirred at room temperature for 2.5 hr. To the reaction mixture were added (2R)-2-ethyl-7-methyl-2,3,4,5-tetrahydropyrido[2,3-f] [1,4]oxazepine hydrochloride (39 mg) of Example 92c, N,N-diisopropylethylamine (0.030 mL) and acetic acid (0.0015 mL), and the mixture was stirred at room temperature for 25 min. Sodium triacetoxyborohydride (45 mg) was added thereto, and the mixture was stirred at room temperature for 40.5 hr. To the reaction mixture were added (2R)-2-ethyl-7-methyl-2,3,4,5-tetrahydropyrido[2,3-f][1,4]oxazepine hydrochloride (39 mg) of Example 92c, N,N-diisopropylethylamine (0.030 mL) and acetic acid (0.0015 mL), and the mixture was stirred at room temperature for 25 min. Sodium triacetoxyborohydride (45 mg) was added thereto, and the mixture was stirred at room temperature for 1 hr. To the reaction mixture was added saturated aqueous sodium hydrogen carbonate solution, and the mixture was extracted with ethyl acetate. The organic layer was washed successively with water and saturated aqueous sodium hydrogen carbonate solution, and dried over anhydrous sodium sulfate. Under reduced pressure, the solvent was evaporated and the obtained residue was purified by silica gel column chromatography [elution solvent: n-hexane/ethyl acetate=70/30-35/65(V/V)] to give the title compound (89 mg, yield: 53%) as a solid.

### (92f)=(92) 3-(1-Ethyl-4-methyl-1H-benzotriazol-5-yl)-3-(7-{[(2R)-2-ethyl-7-methyl-2,3-dihydropyrido[2,3-f] [1,4]oxazepin-4(5H)-yl]methyl}-1-benzothiophen-5-yl)propanoic acid

To a mixture of ethyl 3-(1-ethyl-4-methyl-1H-benzotriazol-5-yl)-3-(7-{[(2R)-2-ethyl-7-methyl-2,3-dihydropyrido[2,3-f] [1,4]oxazepin-4(5H)-yl]methyl}-1-benzothiophen-5-yl)propanoate (89 mg) of Example 92e, tetrahydrofuran (1.35 mL) and methanol (0.45 mL) was added 1 M aqueous lithium hydroxide solution (0.45 mL), and the mixture was stirred at 40°C for 1 hr. The reaction mixture was cooled to room temperature, saturated aqueous ammonium chloride solution was added, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: chloroform/methanol=99/1-95/5(V/V)]. The obtained residue was purified by silica gel column chromatography [elution solvent: n-hexane/ethyl acetate=50/50(V/V), chloroform/methanol=99/1-90/10(V/V)]. Ethyl acetate and n-hexane were added to the obtained residue, and an ultrasonic treatment was performed. The solid was collected by filtration to give the title compound (63.3 mg, yield: 75%) as a solid.

### (Example 93)

### 3-(1,4-Dimethyl-1H-benzotriazol-5-yl)-3-(7-{[(2R)-2-ethyl-7-(trifluoromethyl)-2,3-dihydropyrido[2,3-f] [1,4]oxazepin-4(5H)-yl]methyl}-1-benzothiophen-5-yl)propanoic acid

### (93a) 3-(Benzyloxy)-2-bromo-6-(trifluoromethyl)pyridine

To a solution of benzyl alcohol (1.2 g) in dimethyl sulfoxide (80 mL) was added sodium hydride 55% (0.49 g) at 0°C, and the mixture was stirred at room temperature for 1 hr. To the reaction mixture was added a separately-prepared solution of 2-bromo-3-fluoro-6-(trifluoromethyl)pyridine (2.5 g) in dimethyl sulfoxide (80 mL) and the mixture was stirred at room temperature for 12 hr. The reaction mixture was poured into aqueous ammonium chloride solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=90/10-80/20(V/V)] to give the title compound (1.5 g, yield: 44%) as an oil.

### (93b) 3-(Benzyloxy)-2-ethenyl-6-(trifluoromethyl)pyridine

A mixture of 3-(benzyloxy)-2-bromo-6-(trifluoromethyl)pyridine (1.5 g) of Example 93a, bis(di-tert-butyl (4-dimethylaminophenyl)phosphine)dichloropalladium (II) (0.16 g), 4,4,5,5-tetramethyl-2-vinyl-1,3,2-dioxaborolane (1.0 g), cesium carbonate (4.4 g), 1,4-dioxane (10 mL) and water (1 mL) was stirred at 100°C for 3 hr. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=97/3-90/10(V/V)] to give the title compound (1.15 g, yield: 91%) as an oil.

### (93c) 3-(Benzyloxy)-6-(trifluoromethyl)pyridine-2-carbaldehyde

To a mixture of 3-(benzyloxy)-2-ethenyl-6-(trifluoromethyl)pyridine(1.15 g) of Example 93b, 2,6-lutidine (1.35 g), osmium tetraoxide (2.5% tert-butanol solution) (2.58 mL), 1,4-dioxane (31.7 mL) and water (10.3 mL) was added sodium periodate (3.52 g), and the mixture was stirred at room temperature for 4 hr. To the reaction mixture was added saturated aqueous sodium hydrogen carbonate solution, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=90/10-80/20(V/V)] to give the title compound (1.0 g, yield: 86%) as a solid.

### (93d) tert-Butyl {[3-(benzyloxy)-6-(trifluoromethyl)pyridin-2-yl]methyl} [(2S)-2-hydroxybutyl]carbamate

To a solution of 3-(benzyloxy)-6-(trifluoromethyl)pyridine-2-carbaldehyde (0.5 g) of Example 93c and (2R)-1-aminobutan-2-ol (0.8 g, purity 44%) of Example 17a in dichloromethane (100 mL) was added sodium triacetoxyborohydride (0.8 g), and the mixture was stirred at room temperature for 3 hr. The solvent was evaporated and the obtained residue was dissolved in tetrahydrofuran (30 mL). Di-tert-butyl dicarbonate (1.5 g), sodium hydrogen carbonate (1 g) and water (15 mL) were added thereto, and the mixture was stirred at room temperature for 12 hr. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=90/10-80/20(V/V)] to give the title compound (0.86 g, yield: quantitative) as an oil.

### (93e) tert-Butyl [(2S)-2-hydroxybutyl]{[3-hydroxy-6-(trifluoromethyl)pyridin-2-yl]methyl}carbamate

To a solution of tert-butyl {[3-(benzyloxy)-6-(trifluoromethyl)pyridin-2-yl]methyl} [(2S)-2-hydroxybutyl]carbamate (0.45 g) of Example 93d in ethanol (20 mL) was added 5% palladium carbon (0.50 g), and the mixture was stirred under a hydrogen atmosphere at room temperature for 4 hr. The reaction mixture was filtered through celite, the filtrate was concentrated under reduced pressure, and the obtained residue was purified by silica gel column chromatography [elution solvent: n-hexane/ethyl acetate=90/10-80/20(V/V)] to give the title compound (0.21 g, yield: 59%) as an oil.

### (93f) tert-Butyl (2R)-2-ethyl-7-(trifluoromethyl)-2,3-dihydropyrido[2,3-f][1,4]oxazepine-4(5H)-carboxylate

A solution of tert-butyl [(2S)-2-hydroxybutyl]{[3-hydroxy-6-(trifluoromethyl)pyridin-2-yl]methyl}carbamate (0.21 mg) of Example 93e, triphenyl phosphine (0.460 g) and di-tert-butyl azodicarboxylate (0.404 g) in toluene (29 mL) was stirred at room temperature for 1 hr. To the reaction mixture was added water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=90/10-80/20(V/V)] to give the title compound (0.20 g, yield: quantitative) as a solid.

### (93g) (2R)-2-Ethyl-7-(trifluoromethyl)-2,3,4,5-tetrahydropyrido[2,3-f] [1,4]oxazepine hydrochloride

tert-Butyl (2R)-2-ethyl-7-(trifluoromethyl)-2,3-dihydropyrido[2,3-f][1,4]oxazepine-4(5H)-carboxylate (90 mg) of Example 93f was dissolved in dichloromethane (1 mL), 4 M hydrogen chloride-1,4-dioxane solution (1 mL) was added, and the mixture was stirred at room temperature for 1 hr. The solvent was evaporated under reduced pressure to give the title compound (73 mg, yield: 99%) as a solid.

### (93h) Ethyl 3-(1,4-dimethyl-1H-benzotriazol-5-yl)-3-(7-{[(2R)-2-ethyl-7-(trifluoromethyl)-2,3-dihydropyrido[2,3-f] [1,4]oxazepin-4(5H)-yl]methyl}-1-benzothiophen-5-yl)propanoate

To a solution of (2R)-2-ethyl-7-(trifluoromethyl)-2,3,4,5-tetrahydropyrido[2,3-f][1,4]oxazepine hydrochloride (73 mg) of Example 93g in acetonitrile (3 mL) were added a separately prepared solution of ethyl 3-[7-(chloromethyl)-1-benzothiophen-5-yl]-3-(1,4-dimethyl-1H-benzotriazol-5-yl)propanoate (104 mg) of Example 1g in acetonitrile (3 mL) and N,N-diisopropylethylamine (0.42 mL), and the mixture was stirred at 85°C for 22 hr and cooled to room temperature. The reaction mixture was poured into water, and the mixture was extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=90/10-80/20(V/V)] to give the title compound (96 mg, yield: 62%) as an oil.

### (93i)=(93)

### 3-(1,4-Dimethyl-1H-benzotriazol-5-yl)-3-(7-{[(2R)-2-ethyl-7-(trifluoromethyl)-2,3-dihydropyrido[2,3-f] [1,4]oxazepin-4(5H)-yl]methyl}-1-benzothiophen-5-yl)propanoic acid

To a mixture of ethyl 3-(1,4-dimethyl-1H-benzotriazol-5-yl)-3-(7-{[(2R)-2-ethyl-7-(trifluoromethyl)-2,3-dihydropyrido[2,3-f] [1,4]oxazepin-4(5H)-yl]methyl}-1-benzothiophen-5-yl)propanoate (96 mg) of Example 93h, ethanol (2 mL), and tetrahydrofuran (5 mL) was added 1 M aqueous sodium hydroxide solution (2 mL), and the mixture was stirred at room temperature for 4 hr. To the reaction mixture was added 1 M hydrochloric acid (2 mL) and the mixture was stirred and extracted with ethyl acetate. The organic layer was washed with saturated brine and dried over anhydrous sodium sulfate. Under reduced pressure, the solvent was evaporated and the obtained residue was purified by silica gel column chromatography [elution solvent: n-hexane/ethyl acetate=1/1-0/1(V/V)] to give the title compound (66 mg, yield: 72%) as a solid.

The structural formulas of the compounds described in the examples and the physicochemical data thereof are summarized below.

**[Table 5]**

| **Example No.** | **structural formula** | **physicochemical data** |
|---|---|---|
| 1a | 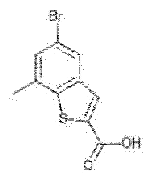 | ¹H-NMR (DMSO-D₆) δ : 2.52 (3H, s), 7.54 (1H, s), 8.10 (2H, s), 13.65 (1H, br s). |
| 1b | 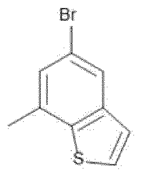 | ¹H-NMR (CDCl₃) δ : 2.55 (3H, s), 7.24-7.30 (2H, m), 7.45 (1H, d, J = 5.4 Hz), 7.81 (1H, d, J = 1.5 Hz). |
| 1c | 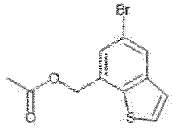 | ¹H-NMR (CDCl₃) δ : 2.16 (3H, s), 5.33 (2H, s), 7.32 (1H, d, J=5.4 Hz), 7.46-7. 48 (1H, m), 7.50 (1H, d, J = 5.4 Hz), 7.94 (1H, d, J = 1.7 Hz). |
| 1d | 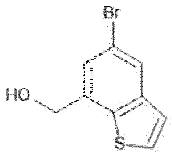 | ¹H-NMR (CDCl₃) δ : 1.90 (1H, t, J = 6.1 Hz), 4.95 (2H, d, J = 6.1 Hz), 7.32 (1H, d, J = 5.4 Hz), 7.46-7.54 (2H, m), 7.91 (1H, d, J = 1.7 Hz). |
| 1e | 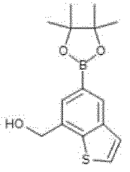 | ¹H-NMR (CDCl₃) δ : 1.38 (12H, s), 4.99 (2H, s), 7.40 (1H, d, J = 5.4 Hz), 7.45 (1H, d, J = 5.4 Hz), 7.75 (1H, s), 8.27 (1H, s). |
| 1f | 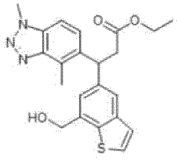 | ¹H-NMR (CDCl₃) δ : 1.10 (3H, t, J = 7. 1 Hz), 1. 86 (1H, t, J = 6.1 Hz), 2.87 (3H, s), 3.06-3.27 (2H, m), 4.02 (2H, q, J = 7.1 Hz), 4.24 (3H, s), 4.90 (2H, d, J = 6.1 Hz), 5.14 (1H, t, J = 8. 0 Hz), 7. 18-7. 23 (1H, m), 7. 28-7. 34 (2H, m), 7.38-7.48 (2H, m), 7.60-7.66 (1H, m). |

**[Table 6]**

| **Example No.** | **structural formula** | **physicochemical data** |
|---|---|---|
| 1g | 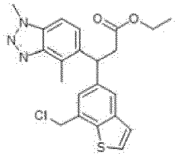 | MS(ESI/APCI) m/z: 428 [M + H]⁺ |
| 1h | 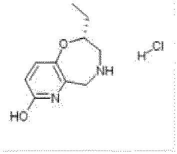 | MS(ESI/APCI) m/z: 195 [M + H]⁺ |
| 1i | 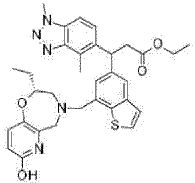 | ¹H-NMR (CDCl₃) δ : 0.93 (1.5H, t, J = 7.3 Hz), 0.98 (1.5H, t, J = 7.3 Hz), 1.10 (3H, t, J = 7.1 Hz), 2.80-3.01 (5H, m), 3.10 (0.5H, d, J = 7.3 Hz), 3.13 (0.5H, d, J = 7.8 Hz), 3.22 (0.5H, d, J = 7.8 Hz), 3.25 (0.5H, d, J = 8.3 Hz), 3.33 (0.5H, d, J = 8.8 Hz), 3.36 (0.5H, d, J = 8.3 Hz), 3.54 (0.5H, d, J = 14.6 Hz), 3.61 (0.5H, d, J = 15.1 Hz), 3.76 (1H, br s), 3.81-4.07 (5H, m), 4.23 (1.5H, s), 4.24 (1.5H, s), 5.06 (0.5H. t, J = 8.1 Hz), 5.11 (0.5H, t, J = 7.8 Hz), 6.34 (1H, t, J = 8.3 Hz), 7.06 (0.5H, s), 7.11 (0.5H, s), 7.23-7.35 (3.5H, m), 7.38-7.43 (1H, m), 7.46 (0.5H, d, J = 8.8 Hz), 7.59 (0.5H, s), 7.63 (0.5H, s). |
| | | MS(ESI/APCI) m/z: 586 [M + H]⁺ |
| 1j=1 | 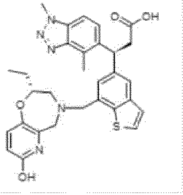 | ¹H-NMR (CD₃OD) δ : 0.92 (3H, t, J = 7.3 Hz), 1.27-1.37 (1H, m), 1.46-1.56 (1H, m), 2.80 (3H. s), 2.83 (1H, d, J = 9.8 Hz), 2.94 (1H, d, J = 13.7 Hz), 3.14 (1H, dd, J = 15.6, 8.3 Hz), 3.25 (1H, dd, J = 15.6, 7.8 Hz), 3.67 (1H, d, J = 15.6 Hz), 3.75-3.82 (1H, m), 3.86-3.93 (3H, m), 4.27 (3H, s), 5.11 (1H, t, J = 7.8 Hz), 6.38 (1H, d, J = 9.3 Hz), 7.13 (1H, s), 7.32 (1H, d, J = 5.4 Hz), 7.41 (1H, d, J = 9.3 Hz), 7.49-7.58 (3H, m), 7.74 (1H, s). |
| | | MS(ESI/APCI) m/z: 556 [M - H]⁻ |
| 2a=2 | 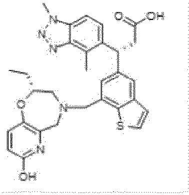 | ¹H-NMR (CD₃OD) δ : 0.90 (3H, t, J = 7.3 Hz), 1.26-1.32 (1H, m), 1.45-1.51 (1H, m), 2.76 (3H, s), 2.83 (1H, dd, J = 13.7, 9.3 Hz), 2.94 (1H, d, J = 13.7 Hz), 3.10 (1H, dd, J = 15.1, 7.8 Hz), 3.20 (1H, dd, J = 14.9, 8.5 Hz), 3.59 (1H, d, J = 15.1 Hz), 3.78 (1H, br s), 3.88-3.90 (3H, m), 4.25 (3H, s), 5.06 (1H, t, J = 7.8 Hz), 6.37 (1H, d, J = 9. 8 Hz), 7. 13 (1H, s), 7.27 (1H, d, J = 5.9 Hz), 7.40 (1H, d, J = 9.8 Hz), 7.48 (1H, d, J = 5.4 Hz), 7.52 (2H, s), 7.65 (1H, s). |
| | | MS(ESI/APCI) m/z: 556 [M - H]⁻ |

**[Table 7]**

| **Example No.** | **structural formula** | **physicochemical data** |
|---|---|---|
| 3a | 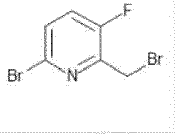 | ¹H-NMR (CDCl₃) δ : 4.54 (2H, d, J = 2.2 Hz), 7.31 (1H, t, J = 8.6 Hz), 7.44 (1H, dd, J = 8.6, 3.4 Hz). |
| 3b | 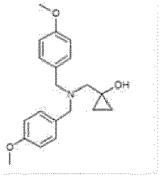 | ¹H-NMR (CDCl₃) δ : 0. 34-0. 40 (2H, m), 0. 75-0. 80 (2H, m), 2.59 (2H, s), 3.43 (1H. br s), 3.63 (4H, s), 3.80 (6H, s), 6.84-6.89 (4H, m), 7.19-7.25 (4H, m). |
| 3c | 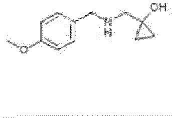 | ¹H-NMR (CDCl₃) δ : 0.38-0.46 (2H, m), 0.75-0.83 (2H, m), 2.52 (2H, br s), 2.72 (2H, s), 3.78-3.84 (5H, m), 6.83-6.92 (2H, m), 7.20-7.30 (2H, m). |
| 3d | 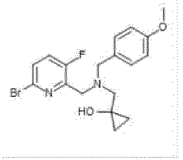 | ¹H-NMR (CDCl₃) δ : 0.38 (2H, dd, J = 6.8, 5.4 Hz), 0.78 (2H, dd, J = 6.8, 5.4 Hz), 2.75 (2H, s), 3.77 (2H, s), 3.78 (3H, s), 3.95 (2H, d, J = 1.7 Hz), 4.47 (1H, br s), 6.78-6.83 (2H, m), 7.15-7.25 (3H, m), 7.33 (1H, dd, J = 8.5, 3.6 Hz). |
| 3e | 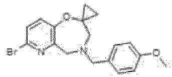 | ¹H-NMR (CDCl₃) δ : 0.48-0.55 (2H, m), 0.85-0.95 (2H, m), 2.98 (2H, s), 3.68 (2H, s), 3.79 (3H, s), 4.16 (2H, s), 6.80-6.87 (2H, m), 6.99 (1H, d, J = 8.3 Hz), 7.16-7.23 (2H, m), 7.26-7.30 (1H, m). |
| 3f | 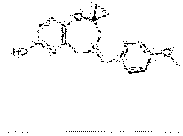 | ¹H-NMR (CDCl₃) δ : 0.45-0.52 (2H, m), 0.87-0.94 (2H, m), 3.00 (2H, s), 3.71 (2H, s), 3.78 (3H, s), 3.99 (2H, s), 6.31 (1H, d, J = 9.5 Hz), 6.79-6.84 (2H, m), 7.10 (1H, d, J = 9.5 Hz), 7.16-7.23 (2H, m), 12.10-12.70 (1H, br s). |

**[Table 8]**

| **Example No.** | **structural formula** | **physicochemical data** |
|---|---|---|
| 3g | 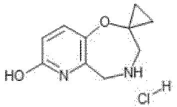 | ¹H-NMR (DMS0-D₆) δ : 0.82-1.00 (4H, m), 3.52 (2H, s), 4.28 (2H, s), 6.50 (1H, d, J = 9.0 Hz), 7.21 (1H, d, J = 9.0 Hz), 9.86 (2H, br s). |
| 3h | 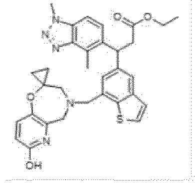 | ¹H-NMR (CDC1₃) δ : 0.40-0.50 (2H, m), 0.85-0.93 (2H, m), 1.10 (3H, t, J = 7.1 Hz), 2.86 (3H, s). 2.98-3.18 (3H, m), 3.26-3.37 (1H, m), 3.82-4.05 (6H, m), 4.24 (3H, s), 5.06 (1H, t, J = 8.0 Hz), 6.32 (1H, d, J = 9.5 Hz), 7.05 (1H, s), 7.09 (1H, d, J = 9.5 Hz), 7.20-7.35 (2H, m), 7.37-7.48 (2H, m), 7.59 (1H, s), 11.20-11.70 (1H, br s). |
| 3i | 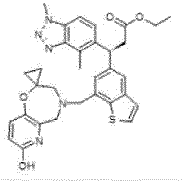 | ¹H-NMR (CDC1₃) δ : 0.39-0.43 (2H, m), 0.85-0.89 (2H, m), 1.10 (3H, t, J = 7.3 Hz), 2.86 (3H, s), 2.97-3.13 (3H, m), 3.29 (1H, dd, J = 15.2, 8.5 Hz), 3.92-4.04 (6H. m), 4.24 (3H, s), 5.07 (1H, t, J = 7.9 Hz), 6.30 (1H, d, J = 9.1 Hz), 7.05 (1H, s), 7.09 (1H, d, J = 9.7 Hz), 7.24 (1H, d, J = 5.5 Hz), 7.31 (1H, d, J = 8.5 Hz), 7.40-7.44 (2H, m), 7.59 (1H, d, J = 1.8 Hz). |
| | | MS(APCI) m/z: 584 [M + H]⁺ |
| 3j=3 | 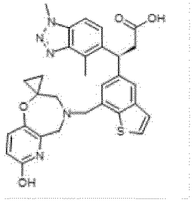 | ¹H-NMR (CDC1₃) δ : 0.62-0.68 (1H, m), 0.74-0.80 (1H, m), 0.93-0.99 (1H, m), 1.04-1.10 (1H, m), 2.57 (3H, s), 2.84-2.91 (2H, m), 3.18 (1H, dd, J = 10.3, 3.0 Hz), 3.25 (1H, d, J = 15.8 Hz), 3.96-4.02 (2H, m), 4.11-4.17 (2H, m), 4.33 (3H, s), 4.82 (1H, dd, J = 12.8, 2.4 Hz), 6.56 (1H, d, J = 9.7 Hz), 7.09 (1H, d, J = 5.5 Hz), 7.13 (1H, s), 7.17 (1H, d, J = 1.8 Hz), 7.32 (1H, d, J = 9.1 Hz), 7.36 (1H, d, J = 5.5 Hz), 7.50 (1H, d, J = 8.5 Hz), 7.63 (1H, d, J = 8.5 Hz). |
| | | MS(APCI) m/z: 556 [M + H]⁺ |
| 4a | 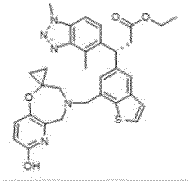 | ¹H-NMR (CDC1₃) δ : 0.40-0.44 (2H, m), 0.86-0.89 (2H, m), 1.10 (3H, t, J = 7.3 Hz), 2.86 (3H, s), 2.99-3.13 (3H, m), 3.30 (1H, dd, J = 15.2, 7.9 Hz), 3. 90-4. 04 (6H, m), 4.24 (3H, s), 5.06 (1H, t, J = 7.9 Hz), 6.31 (1H, d, J = 9.7 Hz), 7.05 (1H, s), 7.09 (1H, d, J = 9.7 Hz), 7.24 (1H, d, J = 5.5 Hz), 7.32 (1H, d, J = 9.1 Hz), 7.40-7.44 (2H, m), 7.59 (1H, d, J = 1.8 Hz). |
| | | MS(APCI) m/z: 584 [M + H]⁺ |

**[Table 9]**

| **Example No.** | **structural formula** | **physicochemical data** |
|---|---|---|
| 4b=4 | 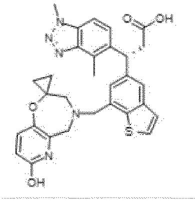 | ¹H-NMR (CDC1₃) δ : 0.64-0.71 (1H, m), 0.75-0.82 (1H, m), 0.94-1.02 (1H, m), 1.05-1.10 (1H, m), 2.59 (3H, s), 2.87-2.96 (2H, m), 3.18 (1H, dd, J = 10.6, 2.7 Hz), 3.28-3.35 (1H, m), 4.00-4.06 (2H, m), 4.08-4.19 (2H, m), 4.32 (3H, s), 4.84 (1H, d, J = 10.3 Hz), 6.56 (1H, d, J = 9.7 Hz), 7.10 (1H, d, J = 5.5 Hz), 7.20 (2H, br s), 7.31 (1H, d, J = 9.7 Hz), 7.37 (1H, d, J = 5.5 Hz), 7.49 (1H, d, J = 8.5 Hz), 7.63 (1H, d, J = 8.5 Hz). |
| | | MS(APCI) m/z: 556 [M + H]⁺ |
| 5a | 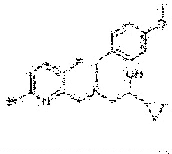 | ¹H-1NMR (CDC1₃) δ : 0.11-0.19 (1H, m), 0.32-0.45 (2H, m), 0.46-0.54 (1H, m). 0.65-0.75 (1H, m), 2.61 (1H, dd, J = 13.0, 10.3 Hz), 2.75 (1H, dd, J = 13.0, 2.7 Hz), 2.94-3.03 (1H, m), 3.55 (1H, d, J = 13.4 Hz), 3.70-3.95 (7H, m), 6.79-6.85 (2H, m), 7.17-7.24 (3H, m), 7.34 (1H, dd, J = 8.5, 3.4 Hz). |
| 5b | 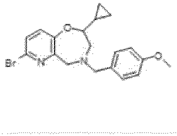 | ¹H-NMR (CDC1₃) δ : 0.15-0.25 (1H, m), 0.40-0.56 (2H, m), 0.57-0.67 (1H, m), 0.95-1.08 (1H, m), 2.96 (1H, dd, J = 13.7, 9.3 Hz), 3.09 (1H, d, J = 13.7 Hz), 3.20-3.29 (1H, m), 3.59 (1H, d, J = 13.2 Hz), 3.68 (1H, d, J = 13.2 Hz), 3.80 (3H, s), 3.97-4.07 (2H, m), 6.82-6.88 (2H, m), 7.16-7.22 (3H, m), 7.28 (1H, d, J = 8.5 Hz). |
| 5c | 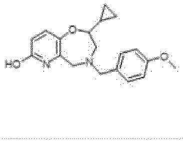 | ¹H-NMR (CDC1₃) δ : 0.13-0.23 (1H, m), 0.40-0.55 (2H, m), 0.56-0. 67 (1H, m), 0.89-1.01 (1H, m), 2.94 (1H, dd, J = 14.2, 9.3 Hz), 3.10 (1H, d, J = 14.2 Hz), 3.13-3.21 (1H, m), 3.57 (1H, d, J = 13.0 Hz), 3.67 (1H, d, J = 13.0 Hz), 3.72 (1H, d, J = 14.9 Hz), 3.79 (3H, s), 3.96 (1H, d, J = 14.9 Hz), 6.32 (1H, d, J = 9.5 Hz), 6.80-6.86 (2H, m), 7.16-7.21 (2H, m), 7.28 (1H, d, J = 9.5 Hz), 12.43 (IH, br s). |
| 5d | 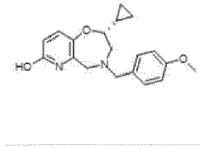 | ¹H-NMR (CDC1₃) δ : 0.13-0.22 (1H, m), 0.41-0.54 (2H, m), 0.57-0.64 (1H, m), 0.89-1. 00 (1H, m), 2.94 (1H, dd, J = 14. 3, 9.4 Hz), 3.10 (1H, d, J = 14.0 Hz), 3.17 (1H, t, J = 8.2 Hz), 3.58 (1H, d, J = 12.8 Hz), 3.65-3.75 (2H, m), 3.80 (3H, s), 3.96 (1H, d, J = 14.6 Hz), 6.32 (1H, d, J = 9.7 Hz), 6.83 (2H, d, J = 8.5 Hz), 7.20 (2H, d, J = 8.5 Hz), 7.29 (1H, d, J = 9.7 Hz), 12.50 (1H, br s). |
| | | MS(APCI) m/z: 327 [M + H]⁺ |

**[Table 10]**

| **Example No.** | **structural formula** | **physicochemical data** |
|---|---|---|
| 5e | 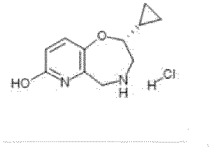 | ¹H-NMR (DMS0-D₆) δ : 0.36-0.40 (1H, m), 0.46-0.50 (1H, m), 0.52-0.61 (2H, m), 1.05-1.11 (1H, m), 3.33-3.45 (2H, m), 3.52-3.58 (1H, m), 4.14 (1H, dd, J = 14.6, 4.9 Hz), 4.26 (1H, dd, J = 14.6, 7.9 Hz), 6.48 (1H, d, J = 9.1 Hz), 7.35 (1H, d, J = 9.1 Hz), 9.65 (1H, br s), 9.97 (1H, br s). |
| | | MS(APCI) m/z: 207 [M + H]⁺ |
| 5f | 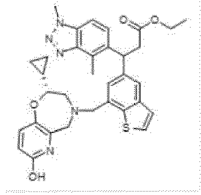 | ¹H-NMR (CDC1₃) δ : 0.35-0.60 (4H, m), 0.84-0.94 (1H, m), 1.10 (3H, t, J = 7.0 Hz), 2.87 (3H, s), 3.01-3.31 (4H, m), 3.57-3.68 (1H, m), 3.80-4.05 (6H, m), 4.23 (3H, s), 4.24 (3H, s), 5.03-5.12 (1H, m), 6.31-6.35 (1H, m), 7.05 and 7.09 (1H, each s), 7.24-7.33 (2H, m), 7.38-7.47 (2H, m), 7.59 and 7.63 (1H, each s). |
| | | MS(APCI) m/z: 598 [M + H]⁺ |
| 5g=5 | 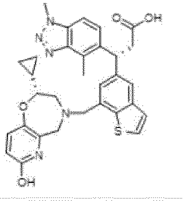 | ¹H-NMR (CDC1₃) δ : 0.36-0.40 (1H, m), 0.53-0.60 (1H, m), 0.65-0.77 (2H, m), 1.01-1.08 (1H, m), 2.56 (3H, s), 2.87 (1H, t, J = 11.5 Hz), 3.11-3.19 (2H, m). 3.29 (1H, td, J = 8.2, 2.2 Hz), 3.50-3.60 (2H, m), 3.80 (1H, d, J = 12.8 Hz), 3.94 (1H, d, J = 12.8 Hz), 4.05 (1H, d, J = 15.2 Hz), 4.33 (3H, s), 4.80 (1H, d, J = 11.5 Hz), 6.56 (1H, d, J = 9.1 Hz), 7.08-7.11 (2H, m), 7.17 (1H, s), 7.37 (1H, d, J = 5.5 Hz), 7.50 (2H, d, J = 9.1 Hz), 7.63 (1H, d, J = 8.5 Hz). |
| | | MS (APCI) m/z: 570 [M + H]⁺ |
| 6a=6 | 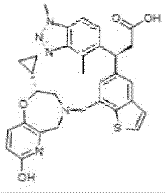 | ¹H-NMR (CDC1₃) δ : 0.35-0.39 (1H, m), 0.48-0.56 (1H, m), 0.62-0.72 (2H, m), 1.04-1.13 (1H, m), 2.69 (3H, s), 3.03-3.19 (2H, m), 3.26 (1H, t, J = 7.0 Hz), 3.43 (1H, dd, J = 14.3, 7.0 Hz), 3.55-3.64 (3H, m), 3.99 (2H, dd, J = 17.6, 13.4 Hz), 4.27 (4H, s), 4.95 (1H, dd, J = 10.0, 5.2 Hz), 6.48 (1H, d, J = 9.1 Hz), 7.14-7.18 (2H, m), 7.34-7.40 (3H, m), 7.44 (1H, d, J = 9.1 Hz), 7.51 (1H, d, J = 8.5 Hz). |
| | | MS(APCI) m/z: 570 [M + H]⁺ |
| 7a | 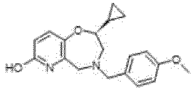 | ¹H-NMR (CDC1₃) δ : 0.15-0.22 (1H, m), 0.42-0.54 (2H, m), 0.58-0.65 (1H, m), 0.90-0.99 (1H, m), 2.95 (1H, dd, J = 14.3, 9.4 Hz), 3.11 (1H, d, J = 14.0 Hz), 3.17 (1H, t, J = 8.2 Hz), 3.58 (1H, d, J = 12.8 Hz), 3.65-3.72 (2H, m), 3.80 (3H, s), 3.95 (1H. d, J = 14.6 Hz), 6.33 (1H, d, J = 9.7 Hz), 6.84 (2H, d, J = 8.5 Hz), 7.20 (2H, d, J = 8.5 Hz), 7.29 (1H, d, J = 9.7 Hz), 12.05 (1H, br s). |
| | | MS(APCI) m/z: 327 [M + H]⁺ |

**[Table 11]**

| **Example No.** | **structural formula** | **physicochemical data** |
|---|---|---|
| 7b | 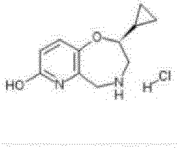 | ¹H-NMR (DMS0-D₆) δ : -0.11--0.07 (1H, m), -0.02-0.03 (1H, m), 0.07-0.13 (2H, m), 0.58-0.64 (1H, m), 2.86-2.99 (2H, m), 3.05-3.11 (1H, m), 3.67 (1H, dd, J = 14.6, 4.9 Hz), 3.79 (1H, dd, J = 14.3, 8.2 Hz), 6.02 (1H, d, J = 8.5 Hz). 6.89 (1H, d, J = 9.1 Hz). |
| | | MS(APCI) m/z: 207 [M + H]⁺ |
| 7c | 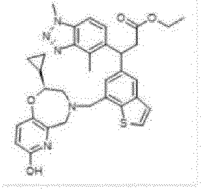 | ¹H-NMR (CDC1₃) δ : 0.35-0.58 (3H, m), 0.85-0.93 (1H, m), 1.10 (3H, t, J = 7.0 Hz), 2.86 (3H, s), 3.00-3.33 (5H, m), 3.56-3.91 (3H, m), 3.97-4.06 (3H, m), 4.23 (0H, s), 4.24 (3H, s), 5.03-5.12 (1H, m), 6.30-6.35 (1H, m), 7.05 and 7.09 (1H, each s), 7.17-7.34 (3H, m), 7.38-7.47 (2H, m), 7.58 and 7.63 (1H, each s). |
| | | MS(APCI) m/z: 598 [M + H]⁺ |
| 7d=7 | 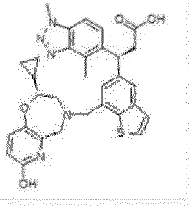 | ¹H-NMR (CDC1₃) δ : 0.34-0.41 (1H, m), 0.53-0. 60 (1H, m), 0.65-0.75 (2H, m), 1.02-1.09 (1H, m), 2.56 (3H, s), 2.86 (1H, dd, J = 12.8, 10.3 Hz), 3.11-3.19 (2H, m), 3.26-3.32 (1H, m), 3.50-3.59 (2H, m). 3.80 (1H, d, J = 12.8 Hz). 3.93 (1H, d, J = 12.8 Hz), 4.05 (1H, d, J = 15.8 Hz), 4.33 (3H, s), 4.80 (1H, dd, J = 12.8, 2.4 Hz), 6.56 (1H, d, J = 9.7 Hz), 7.08-7.11 (2H, m), 7.17 (1H, d, J = 1.2 Hz), 7.37 (1H, d, J = 5.5 Hz), 7.49-7.52 (2H, m), 7.63 (1H, d, J = 8.5 Hz). |
| | | MS(APCI) m/z: 570 [M + H]⁺ |
| 8a=8 | 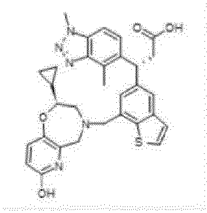 | ¹H-NMR (CDC1₃) δ : 0.35-0.40 (1H, m), 0.50-0.54 (1H, m), 0.66-0.73 (2H, m), 1.04-1.14 (1H, m), 2.68 (3H, s), 3.09-3.18 (3H, m), 3.23-3.29 (1H, m), 3.43 (1H, dd, J= 14.6, 7.3 Hz), 3.57 (1H, d, J = 12.8 Hz), 3.63 (2H, br s), 3.96-4.03 (2H, m). 4.27 (3H, s), 4.95 (1H, dd, J = 10.0, 5.2 Hz), 6.50 (1H, d, J = 9.7 Hz), 7.15-7.21 (2H, m), 7.35-7.41 (3H, m), 7.45 (1H, d, J = 9.7 Hz), 7.51 (1H, d, J = 8.5 Hz). |
| | | MS(APCI) m/z: 570 [M + H]⁺ |
| 9a | 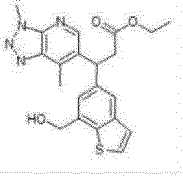 | MS(APCI) m/z: 411 [M + H]⁺ |

**[Table 12]**

| **Example No.** | **structural formula** | **physicochemical data** |
|---|---|---|
| 9b | 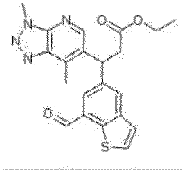 | ¹H-NMR (CDC1₃) δ : 1.14 (3H, t, J = 7.3 Hz), 2.87 (3H, s), 3.23-3.37 (2H, m), 4.07 (2H, q, J = 7.1 Hz), 4.34 (3H, s), 5.20 (1H, t, J = 7.9 Hz), 7.37 (1H, d, J = 5.5 Hz), 7.67 (1H, d, J = 5.5 Hz), 7.72 (1H, s), 7.97 (1H, s), 8.64 (1H, s), 10.16 (1H, s). |
| | | MS(APCI) m/z: 409 [M + H]⁺ |
| 9c | 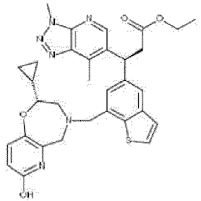 | ¹H-NMR (CDC1₃) δ : -0.02-0.06 (1H, m), 0.38-0.47 (2H, m), 0.53-0.62 (1H, m), 0.86-0.98 (1H, m), 1.13 (3H, t, J = 7.1 Hz), 2.88 (3H, s), 3.06-3.16 (2H, m), 3.16-3.25 (2H, m), 3.36 (1H, dd, J= 15.3, 8.6 Hz), 3.60 (1H, d, J = 15.3 Hz), 3.85 (1H, d, J = 13.5 Hz), 3.91 (1H, d, J = 13.5 Hz), 3.98 (1H, d, J = 15.3 Hz), 4.04 (2H, q, J = 7.2 Hz), 4.31 (3H, s), 5.04 (1H, t, J = 8.3 Hz), 6.34 (1H, d, J = 9.2 Hz), 7.13 (1H, s), 7.24-7.31 (2H, m), 7.43 (1H, d, J = 5.5 Hz), 7.63 (1H, s), 8.68 (1H, s). |
| | | MS(ESI/APCI) m/z: 599 [M + H]⁺ |
| 9d=9 | 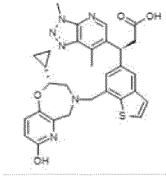 | ¹H-NMR (CDC1₃) δ : 0.33-0.42 (1H, m), 0.48-0.57 (1H, m), 0.65-0.74 (2H, m), 1.03-1.13 (1H, m), 2.70 (3H, s), 3.08-3.31 (3H, m), 3.43 (1H, dd, J = 14.4, 6.4 Hz), 3.52-3.68 (3H, m), 3.93-4.04 (2H, m), 4.34 (3H, s), 4.92 (1H, d, J = 11.0 Hz), 6.47 (1H, br s), 7.10-7.20 (2H, m), 7.33-7.48 (3H, m), 8.72 (1H, s). |
| | | MS (ESI/APCI) m/z: 571 [M + H]⁺ |
| 10a | 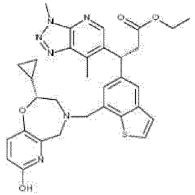 | ¹H-NMR (CDC1₃) δ : 0.15-0.24 (1H, m), 0.46-0.57 (2H, m), 0.61-0.69 (1H, m), 0.96-1.05 (1H, m), 1.11 (3H, t, J = 7.1 Hz), 3.08-3.19 (1H, m), 3.19-3.32 (4H, m), 3.55 (1H, d, J = 14.1 Hz), 3.79-3.87 (2H, m), 3.95-4.07 (3H, m), 4.31 (3H, s), 5.10 (1H, t, J = 8.0 Hz), 6.44 (1H, d, J = 8.6 Hz), 7.24-7.36 (3H, m), 7.42 (1H, d, J = 5.5 Hz), 7.61 (1H, s), 8.81 (1H, s). |
| | | MS(ESI/APCI) m/z: 599 [M + H]⁺ |
| 10b=10 | 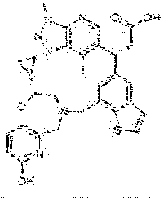 | ¹H-NMR (CDC1₃) δ : 0.34-0.42 (1H, m), 0.52-0.60 (1H, m), 0.65-0.79 (2H, m), 1.00-1.10 (1H, m), 2.58 (3H, s), 2.96 (1H, t, J = 11.3 Hz), 3.12 (1H, d, J = 15.9 Hz), 3.21-3.32 (2H. m), 3.49-3.60 (2H, m), 3.80 (1H, d, J = 12.9 Hz), 3.94 (1H, d, J = 12.9 Hz), 4.06 (1H, d, J = 15.3 Hz), 4.40 (3H, s), 4.79 (1H, d, J = 10.4 Hz), 6.56 (1H, d, J = 9.8 Hz), 7.10 (1H, s), 7.12 (1H, d, J = 5.5 Hz), 7.17 (1H, s), 7.41 (1H, d, J = 5.5 Hz), 7.51 (1H, d, J = 9.8 Hz), 8.77 (1H, s). |
| | | MS(ESI/APCI) m/z: 571 [M + H]⁺ |

**[Table 13]**

| **Example No.** | **structural formula** | **physicochemical data** |
|---|---|---|
| 11a | 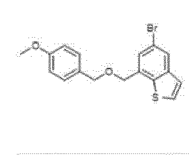 | ¹H-NMR (CDC1₃) δ : 3.82 (3H, s), 4.53 (2H, s), 4.76 (2H, s), 6.90 (2H, dd, J = 6.7, 2.0 Hz), 7.25-7.34 (3H, m), 7.45 (1H, s), 7.49 (1H, d, J = 5.5 Hz), 7.91 (1H, d, J = 1.6 Hz). |
| | | MS(ESI/APCI) m/z: 363 [M + H]⁺ |
| 11b | 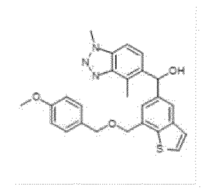 | ¹H-NMR (CDC1₃) δ : 2.85 (3H, s), 3.83 (3H, s), 4.31 (3H, s), 4.51 (2H, s), 4.78 (2H, s), 6.44 (1H, d, J = 3.5 Hz), 6.88 (2H, d, J = 8.6 Hz), 7.26-7.32 (3H, m), 7.34-7.39 (2H, m) , 7.50 (1H, d, J = 5.5 Hz), 7.74 (1H, d, J = 8.6 Hz), 7.79 (1H, s). |
| | | MS (ESI/APCI) m/z: 460 [M + H]⁺ |
| 11c | 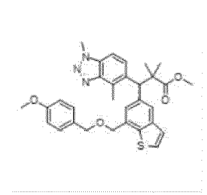 | ¹H-NMR (CDC1₃) δ : 1.35 (3H, s), 1.45 (3H, s), 2.85 (3H, s), 3.48 (3H, s), 3.80 (3H, s), 4.24 (3H, s), 4.42 (2H, s), 4.69 (2H, d, J = 1.2 Hz), 5.04 (1H, s), 6.82 (2H, d, J = 8.6 Hz), 7.10 (1H, s), 7.21 (2H, d, J = 8.6 Hz), 7.27 (1H, d, J = 8.6 Hz), 7.30 (1H, d, J = 5.5 Hz), 7.43 (1H, d, J = 5.1 Hz), 7.62 (1H, d, J = 8.6 Hz), 7.69 (1H, s). |
| | | MS(ESI/APCI) m/z: 544 [M + H]⁺ |
| 11d | 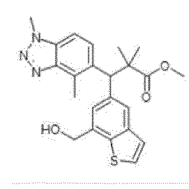 | ¹H-NMR (CDC1₃) δ : 1.35 (3H, s), 1.45 (3H, s), 1.87 (1H, t, J = 6.1 Hz), 2.83 (3H, s), 3.48 (3H, s), 4.25 (3H, s), 4.88 (2H, d, J = 5.9 Hz), 5.03 (1H, s), 7.17 (1H, s), 7.29 (1H, d, J = 9.0 Hz), 7.32 (1H, d, J = 5.5 Hz), 7.44 (1H, d, J = 5.5 Hz), 7.65 (1H, d, J = 8.6 Hz), 7.70 (1H, s). |
| | | MS(ESI/APCI) m/z: 424 [M + H]⁺ |
| 11f | 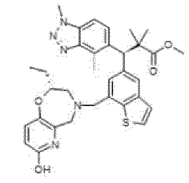 | ¹H-NMR (CDC1₃) δ : 0.88-0.95 (3H, m), 1.36 (3H, s), 1.43 (3H, s), 1.52-1.57 (2H, m), 2.81 (3H, s), 2.86-2.95 (2H, m), 3.56 (3H, s), 3.71 (2H, d, J = 14.2 Hz), 3.84 (1H, d, J = 13.7 Hz), 3.88 (IH, d, J = 13.2 Hz), 4.03 (1H, d, J = 15.1 Hz), 4.24 (3H, s), 4.91 (1H, s), 6.33 (1H, d, J = 9.8 Hz), 7.10 (1H, s), 7.26-7.27 (2H, m), 7.33 (1H, d, J = 4.1 Hz), 7.39 (IH, d, J = 2.7 Hz), 7.67-7.68 (2H, m). |

**[Table 14]**

| **Example No.** | **structural formula** | **physicochemical data** |
|---|---|---|
| 11g=11 | 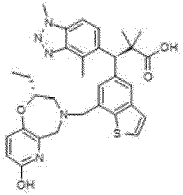 | ¹H-NMR (CDCl₃) δ : 1. 10 (3H, t, J = 7.3 Hz), 1. 26 (3H, s), 1. 31 (3H, s), 1. 47-1. 56 (1H, m), 1. 60-1. 71 (1H, m), 2.78 (3H, s), 3. 20 (1H, d, J = 15. 6 Hz), 3. 25-3. 36 (2H, m), 3. 65 (1H, d, J = 12.7 Hz), 3.70-3.77 (2H, m), 3.95 (1H, d, J = 15.6 Hz), 4. 23 (3H, s), 4.46 (1H, s), 6. 58 (1H, d, J = 9.6 Hz), 6.80 (1H, s), 7.37 (1H, d, J = 5.4 Hz), 7.40 (1H, d, J = 8. 5 Hz), 7. 45 (1H, d, J = 5.4 Hz), 7.48 (1H. d, J = 9.6 Hz), 7.82 (1H, s), 8.32 (1H, d, J = 8.5 Hz). |
| | | MS(ESI/APCI) m/z: 586 [M + H]⁺ |
| 12a | 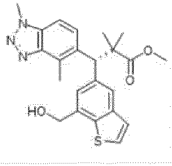 | ¹H-NMR (CDCl₃) δ : 1.35 (3H, s), 1.45 (3H, s), 1.79 (1H, t, J = 6.4 Hz), 2.84 (3H, s), 3.49 (3H, s), 4.25 (3H, s), 4.88 (2H, d, J = 5.5 Hz), 5.03 (1H, s), 7.17 (1H, s), 7.29 (1H, d, J = 8.5 Hz), 7.32 (1H, d, J = 5.5 Hz), 7.44 (1H, d, J = 5.5 Hz), 7.65 (1H, d, J = 8.5 Hz), 7.70 (1H, d, J = 1.2 Hz). |
| | | MS(APCI) m/z: 424 [M + H]⁺ |
| 12b | 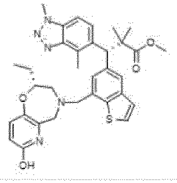 | ¹H-NMR (CDCl₃) δ : 0. 88-0. 93 (3H, m), 1. 34 (3H, s), 1. 44 (3H, s), 1.47-1.63 (2H, m), 2.77-2.88 (2H, m), 2.84 (3H, s), 3.49 (3H, s), 3.64-3.78 (2H, m), 3.85 (1H, d, J = 14.6 Hz), 3. 92 (1H, d, J = 13.7 Hz), 4.00 (1H, d, J = 15.1 Hz), 4.24 (3H, s), 5.01 (1H, s), 6.32 (1H, d, J = 9.8 Hz), 7.06 (1H, s), 7.24-7.27 (2H, m), 7.30 (1H, d, J = 8.8 Hz), 7.38 (1H, d, J = 5.4 Hz), 7.63 (1H, d, J = 8.8 Hz), 7.67 (1H, s). |
| 12c=12 | 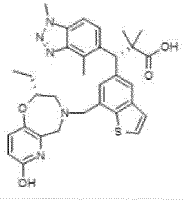 | ¹H-NMR (CDCl₃) δ : 1. 14 (3H, t, J = 7.3 Hz), 1.29 (3H, s), 1. 44 (3H, s), 1. 51-1. 59 (1H, m), 1. 65-1. 73 (1H, m), 2. 77 (3H, s), 3.21 (1H, d, J = 15.6 Hz), 3.38 (2H, d, J = 5.9 Hz), 3. 76-3. 90 (3H, m), 4.06 (1H, d, J = 15.6 Hz), 4. 30 (3H, s), 5. 02 (1H, s), 6. 59 (1H, d, J = 9.3 Hz), 7. 02 (1H, s), 7.21 (1H, d, J = 5.4 Hz), 7.39 (1H, d, J = 5.4 Hz), 7.47 (1H, d, J = 8.3 Hz), 7.51 (1H, d, J = 9.3 Hz), 7.64 (1H, s), 8. 12 (1H, d, J = 8.8 Hz). |
| | | MS(ESI/APCI) m/z: 586 [M + H]⁺ |
| 13a | 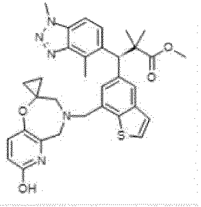 | ¹H-NMR (CDCl₃) δ : 0. 29 (2H, br s), 0.80 (2H, t, J = 6.1 Hz), 1.33 (3H, s), 1. 42 (3H, s), 2. 79 (3H, s), 2. 90 (1H, d, J = 14.9 Hz), 2.96 (1H, d, J = 14.9 Hz), 3.50 (3H, s), 3.94 (2H, s), 4. 09-4. 15 (2H, m), 4.24 (3H, s), 4. 96 (1H, s), 6. 28 (1H, d, J = 9.4 Hz), 7.01 (1H, s), 7.07 (1H, d, J = 9.8 Hz), 7.24 (1H, d, J = 5.5 Hz), 7.29 (1H, d, J = 8.6 Hz), 7.39 (1H, d, J = 5. 5 Hz), 7.60-7.65 (2H, m). |
| | | MS(ESI/APCI) m/z: 598 [M + H]⁺ |

**[Table 15]**

| **Example No.** | **structural formula** | **physicochemical data** |
|---|---|---|
| 13b=13 | 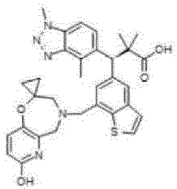 | ¹H-NMR (CD₃OD) δ : 0.48 (2H, br s), 0.86 (2H, t, J = 5. 9 Hz), 1.34 (3H, s), 1.42 (3H, s), 2.74 (3H, s), 3.10 (1H, d, J = 14.5 Hz), 3.17 (1H, d, J = 14.5 Hz), 3.87 (2H, d, J = 3.5 Hz), 3.92 (2H, br s), 4.29 (3H, s), 5.00 (1H, s), 6.41 (1H, d, J = 9.4 Hz), 7.05 (1H, br s), 7.31 (1H, s), 7.32 (1H, d, J = 3.9 Hz), 7.51 (1H, d, J = 5.5 Hz), 7.56 (1H, d, J = 9.0 Hz), 7.78 (1H, br s), 7.92 (1H, d, J = 9.0 Hz). |
| | | MS(ESI/APCI) m/z: 584 [M + H]⁺ |
| 14a | 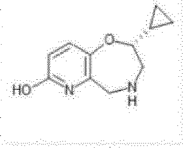 | ¹H-NMR (CDCl₃) δ : 0.27-0.34 (1H, m), 0.44-0.50 (1H, m), 0.57-0.64 (2H, m), 0. 94-1. 02 (1H, m), 2. 98 (1H, td, J = 8. 5, 1.8 Hz), 3. 06 (1H, dd. J = 14.0, 9.1 Hz), 3. 35 (1H, dd, J = 14.0, 1.8 Hz), 3.96 (2H, s), 6.36 (1H, d, J = 9.7 Hz), 7. 32 (1H, d, J = 9. 7 Hz). |
| | | MS (APCI) m/z: 207 [M + H]⁺ |
| 14b | 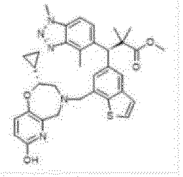 | ¹H-NMR (CDCl₃) δ : 1. 35 (3H, s), 1.42 (3H, s), 2.81 (3H, s), 3.01-3.15 (2H, m), 3.53 (3H, s), 3.73-3.91 (5H, m), 4.24 (3H, s), 4.92 (1H, s), 6.37 (1H. d, J = 9.4 Hz), 6.79-6.92 (1H, m), 7.06 (1H, s), 7. 23-7. 35 (2H, m), 7.39 (1H, d, J = 5.5 Hz), 7.68 (2H, d, J = 9.0 Hz). |
| | | MS(APCI) m/z: 612 [M + H]⁺ |
| 14c=14 | 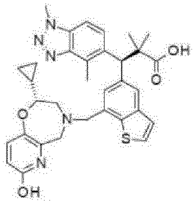 | ¹H-NMR (CDCl₃) δ : 0. 23-0.31 (1H, m), 0.45-0.53 (1H, m), 0.59-0.75 (2H, m), 0.96-1.06 (1H, m), 1.31 (3H, s), 1.44 (3H, s), 2. 78 (3H, s), 3. 13-3. 26 (2H, m), 3. 48 (2H, d, J = 5.9 Hz), 3.61 (1H, d, J = 12.9 Hz), 3.74 (1H, d, J = 12.9 Hz), 3.94 (1H, d, J = 15.3 Hz), 4.23 (3H, s), 4.47 (1H, s), 6.57 (1H, d, J = 9. 4 Hz), 6.79 (1H, s), 7.33-7.41 (2H, m), 7.46 (1H, d, J = 5.5 Hz), 7.48 (1H, d, J = 9.8 Hz), 7.82 (1H, s), 8.30 (1H, d, J = 8.6 Hz). |
| | | MS(APCI) m/z: 598 [M + H]⁺ |
| 15a | 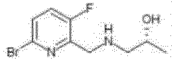 | ¹H-NMR (CDCl₃) δ : 1.15 (3H, J = 6.4 Hz), 1. 72 (1H, br s), 2.45 (1H, dd, J = 12.2, 9.3 Hz), 2.74 (1H, dd, J = 12.2, 2.9 Hz), 2.95-3.30 (1H. br s), 3. 75-3. 84 (1H, m), 3.96 (2H, d, J = 1.9 Hz), 7.26 (1H, t, J = 8.3 Hz), 7.37 (1H, dd, J = 8. 3, 3.4 Hz). |

**[Table 16]**

| **Example No.** | **structural formula** | **physicochemical data** |
|---|---|---|
| 15b | 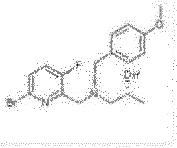 | ¹H-NMR (CDCl₃) δ : 1.09 (3H, J = 6. 1 Hz), 2.42 (1H, dd, J = 12.9, 10.2 Hz), 2.60 (1H, dd, J = 12.9, 2.7 Hz), 3.55 (1H, d, J = 13.4 Hz), 3.70-3.95 (7H. m), 6.78-6.84 (2H, m). 7.16-7.24 (3H, m), 7.34 (1H, dd, J = 8.6, 3.7 Hz). |
| 15c | 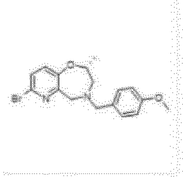 | ¹H-NMR (CDCl₃) δ : 1.27 (3H, d, J = 6.3 Hz), 2.75 (1H, dd, J = 13.9, 9.5 Hz), 2.88-2.95 (1H, m), 3.65 (2H, s), 3.81 (3H, s), 3.97-4.15 (3H, m), 6.83-6.88 (2H, m), 7.15-7.24 (3H, m), 7.26-7.31 (1H, m). |
| 15d | 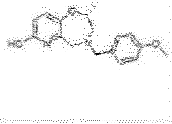 | ¹H-NMR (CDCl₃) δ : 1.23 (3H, d, J = 6. 4 Hz), 2. 73-2. 81 (1H, m), 2.91-2.98 (1H, m), 3.60-3.68 (3H, m), 3.80 (3H, s), 3. 89-4. 07 (2H, m), 6. 33 (1H, d, J = 9. 5 Hz), 6. 81-6. 87 (2H, m), 7. 17-7. 24 (2H, m). 7. 27 (1H, d, J = 9.5 Hz). |
| 15e | 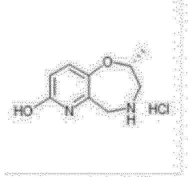 | ¹H-NMR (DMSO-D₆) δ : 1.33 (3H, d, J = 6.4 Hz), 3. 15-3. 25 (1H, m), 3.45-3.55 (1H, m), 4.05-4.15 (2H, m), 4.25-4.34 (1H, m), 6. 49 (1H, d. J = 9.0 Hz), 7.36 (1H, d, J = 9.0 Hz), 9. 47-9. 61 (1H, br m), 9. 85-9. 97 (1H, br m). |
| 15f | 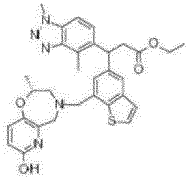 | ¹H-NMR (CDCl₃) δ : 1.07-1. 14 (3H, m), 1.14-1. 21 (3H, m), 2. 77-2. 98 (5H, m), 3.06-3.17 (1H, m), 3. 19-3. 26 (0. 5H, m), 3.27-3.37 (0. 5H, m), 3.57-3.70 (1H, m), 3.85-4.15 (6H, m), 4.22 (1. 5H, s), 4.24 (1. 5H, s), 5.04-5.14 (1H, m), 6. 29-6. 36 (1H, m), 7.05-7. 14 (1H, m), 7.24-7.48 (5H, m), 7.56-7.64 (1H, m). |
| 15g=15 | 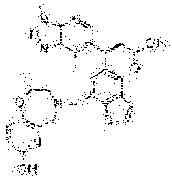 | ¹H-NMR (DMSO-D₆) δ : 1.08 (3H, d, J = 6.6 Hz), 2.68-2.81 (5H, m), 3.02-3.22 (2H, m), 3.66-3.88 (4H, m), 4.00-4. 10 (1H, m), 4.23 (3H, s), 4.91-5.00 (1H, m), 6.23 (1H, d, J = 9.5 Hz), 7.21-7.28 (2H, m), 7.37 (1H, d, J = 5.4 Hz), 7.54 (1H, d, J = 9.0 Hz), 7.57 (1H, d, J = 9.0 Hz), 7.65 (1H, d, J = 5.4 Hz), 7. 71-7. 75 (1H, m), 11. 40-11.80 (2H, br s). |
| | | MS(ESI) m/z: 542 [M - H]⁻, 566 [M + Na]⁺ |

**[Table 17]**

| **Example No.** | **structural formula** | **physicochemical data** |
|---|---|---|
| 16a=16 | 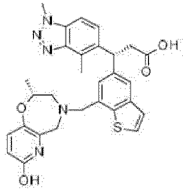 | ¹H-NMR (DMSO-D₆) δ : 1.07 (3H, d, J = 6. 4 Hz), 2. 65-2. 84 (5H, m), 3. 04-3. 23 (2H, m), 3.67 (2H, d, J = 15. 1 Hz), 3. 77-3. 90 (3H, m), 3.99-4.09 (1H, m), 4.23 (3H, s), 4.95 (1H, t, J = 7.6 Hz), 6.23 (1H, d, J = 9.2 Hz), 7. 20-7. 24 (1H, m), 7.26 (1H, d, J = 9.2 Hz), 7.36 (1H, d, J = 5.4 Hz), 7.53 (1H, d, J = 8.8 Hz), 7.57 (1H, d, J = 8.8 Hz), 7.65 (1H, d, J = 5. 4 Hz), 7.69-7.74 (1H, m), 11.40-11.90 (2H, br s). |
| 17b | 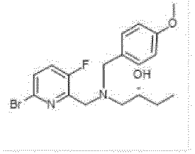 | ¹H-NMR (CDCl₃) δ : 0.94 (3H, t, J = 7.3 Hz), 1. 30-1. 50 (2H, m), 2. 43 (1H, dd, J = 13. 0, 10.5 Hz), 2. 62 (1H, dd, J = 13. 0, 2.7 Hz), 3.52 (1H, d, J = 13.4 Hz), 3.59-3.68 (1H, m), 3.69-3.76 (1H, m), 3.77-3.87 (5H, m), 3.90-3.98 (1H, m), 6.79-6.85 (2H, m), 7.16-7.24 (3H, m), 7.34 (1H, dd, J = 8. 6, 3.4 Hz). |
| 17c | 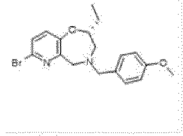 | ¹H-NMR (CDCl₃) δ : 1. 01 (3H, t, J = 7.3 Hz), 1. 40-1. 51 (1H, m), 1. 59-1. 71 (1H, m), 2. 78 (1H, dd, J = 13. 6, 9.5 Hz), 2. 88-2. 96 (1H, m), 3.60-3.69 (2H, m), 3.77-3.84 (4H, m), 4.02 (2H, s), 6.81-6.88 (2H, m), 7.15-7.22 (3H, m), 7.26-7.31 (1H, m). |
| 17d | 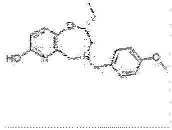 | ¹H-NMR (CDCl₃) δ : 1.01 (3H, t, J = 7.3 Hz), 1. 33-1. 46 (1H, m), 1. 50-1. 58 (1H, m), 2.77 (1H, dd, J = 14. 2, 9.5 Hz), 2.94 (1H, d, J = 14.2 Hz), 3.59-3.84 (7H, m), 3.95 (1H, d, J = 14.6 Hz), 6.33 (1H, d, J = 9.8 Hz), 6.80-6.88 (2H, m), 7. 17-7. 24 (2H, m), 7.28 (1H, d, J = 9.8 Hz), 12.05-12.50 (1H, br s). |
| 17e | 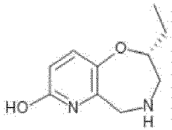 | ¹H-NMR (CDCl₃) δ : 1. 05 (3H, t, J = 7. 3 Hz), 1. 46-1. 69 (2H, m), 2.89 (1H, dd, J = 14.4, 9.5 Hz), 3. 15-3. 24 (1H, m), 3.48-3.58 (1H, m), 3.96 (2H, s), 6.37 (1H, d, J = 9.5 Hz), 7.30 (1H, d, J = 9.5 Hz), 12.30-14.20 (1H, br s). |

**[Table 18]**

| **Example No.** | **structural formula** | **physicochemical data** |
|---|---|---|
| 17f | 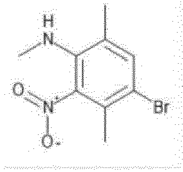 | ¹H-NMR (CDCl₃) δ : 2.23 (3H, s), 2.29 (3H, s), 2.79 (3H, dd, J = 5.8, 0.7 Hz), 3.70 (1H, br s), 7.36 (1H, s). |
| 17g | 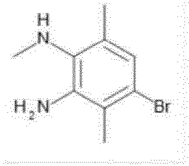 | ¹H-NMR (CDCl₃) δ : 2.21 (3H, s), 2.25 (3H, s), 2.58-2. 72 (4H, m), 4.08 (2H, br s), 6.83 (1H, s). |
| 17h | 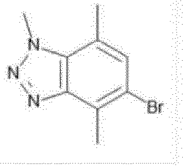 | ¹H-NMR (CDCl₃) δ : 2.69 (3H, s), 2.77 (3H, s), 4.46 (3H, s), 7.32 (1H, s). |
| 17i | 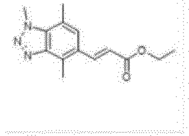 | ¹H-NMR (CDCl₃) δ : 1.36 (3H, t, J = 7.1 Hz), 2.72 (3H, s), 2.85 (3H, s), 4.29 (2H, q, J = 7.1 Hz), 4.47 (3H, s), 6.39 (1H, d. J = 15.9 Hz), 7.39 (1H, s), 8.09 (1H. d, J = 15.9 Hz). |
| 17j | 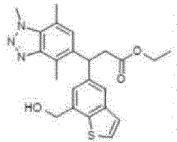 | ¹H-NMR (CDCl₃) δ : 1. 12 (3H, t, J = 7.1 Hz), 1. 79 (1H, t, J = 6. 1 Hz), 2.67 (3H, s), 2. 82 (3H. s), 3. 04-3. 12 (1H, m), 3. 17-3. 24 (1H, m), 4.03 (2H, q, J = 7. 1 Hz), 4. 43 (3H, s), 4.90 (2H, d, J = 6.1 Hz), 5.09 (1H, t, J = 8.0 Hz), 7.04 (1H, s), 7. 19 (1H, s), 7.32 (1H, d, J = 5.6 Hz), 7.45 (1H, d, J = 5.6 Hz), 7.63 (1H, s). |
| 17k | 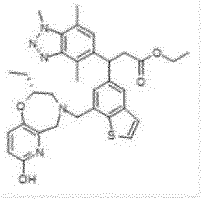 | ¹H-NMR (CDCl₃) δ : 0.92 (1.5H, t, J = 7.3 Hz), 0.98 (1.5H, t, J = 7.3 Hz), 1.11 (3H, t, J = 7. 1 Hz), 1. 29-1. 41 (1H, m), 1.48-1.60 (1H, m), 2.67 (3H. s), 2.78-3.00 (5H. m), 3.04-3.12 (1H, m), 3.22 (0.5H, dd, J = 15.2, 8.0 Hz), 3.31 (0.5H, dd, J = 15. 1, 9.0 Hz), 3.56-3.71 (1H, m), 3.72-3.83 (1H, m), 3.84-3.96 (2H, m), 3.97-4.08 (3H, m), 4.42 (1. 5H, s), 4.43 (1.5H, s), 4.98-5.10 (1H, m), 6.31-6.38 (1H, m), 7.03-7.14 (2H, m), 7.22-7.30 (2H, m), 7.37-7.41 (1H, m), 7.55-7.63 (1H, m), 11.20-12.10 (1H, br s). |

**[Table 19]**

| **Example No.** | **structural formula** | **physicochemical data** |
|---|---|---|
| 171=17 | 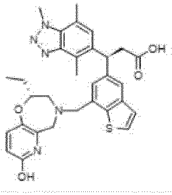 | ¹H-NMR (DMSO-D₆) δ : 0.88 (3H, t, J = 7. 6 Hz), 1. 21-1. 33 (1H, m), 1.37-1.50 (1H, m), 2.64 (3H, s), 2. 68-2. 81 (5H, m), 3.04-3.21 (2H, m), 3.66-3.94 (5H, m), 4.40 (3H, s), 4.90 (1H, t, J = 7.6 Hz), 6.24 (1H, d, J = 9.3 Hz), 7.19-7.24 (2H, m), 7.27 (1H, d, J = 9. 3 Hz), 7.37 (1H, d, J = 5. 6 Hz), 7.66 (1H, d, J = 5.6 Hz), 7. 70-7. 74 (1H, m), 11.62 (1H, br s). |
| | | MS(ESI) m/z: 570 [M - H]⁻, 572 [M + H]⁺ , 594 [M + Na]⁺ |
| 18a=18 | 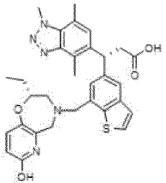 | ¹H-NMR (DMSO-D₆) δ : 0.81 (3H, t, J = 7. 3 Hz), 1. 13-1. 28 (1H, m), 1. 34-1. 48 (1H, m), 2. 64 (3H, s), 2. 66-2. 80 (5H, m), 3. 06 (1H, dd, J = 15.4, 8.3 Hz), 3.16 (1H, dd, J = 15. 4, 7.3 Hz), 3.64-3.77 (2H, m), 3.80-3.93 (3H, m), 4.39 (3H, s), 4.86-4.94 (1H, m), 6.23 (1H, d, J = 9.0 Hz), 7.16-7.22 (2H, m), 7.26 (1H, d, J = 9.0 Hz), 7.38 (1H, d, J = 5.4 Hz), 7.66 (1H, d, J = 5.4 Hz), 7.70-7.74 (1H, m), 11.40-11.90 (1H, br s). |
| 19a | 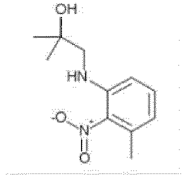 | MS(ESI/APCI) m/z: 225 [M + H]⁺ |
| 19b | 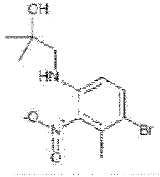 | MS(ESI/APCI) m/z: 303, 305 [M + H]⁺ |
| 19c | 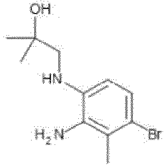 | ¹H-NMR (CDCl₃) δ : 1.35 (6H, s), 1.75 (1H, s), 2.32 (3H, s), 3.03 (2H, s), 3.66 (3H, br s), 6.49 (1H, d, J = 8.5 Hz), 6.99 (1H, d, J = 8.5 Hz). |

**[Table 20]**

| **Example No.** | **structural formula** | **physicochemical data** |
|---|---|---|
| 19d | 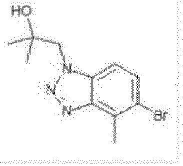 | MS(ESI/APCI) m/z: 284, 286 [M + H]⁺ |
| 19e | 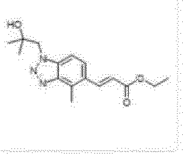 | MS(ESI/APCI) m/z: 304 [M + H]⁺ |
| 19f | 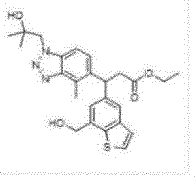 | ¹H-NMR (CDCl₃) δ : 1. 10 (3H, t, J = 7.1 Hz), 1. 26-1. 31 (6H, m), 1.95 (1H, br s), 2.29 (1H, br s), 2.90 (3H, s), 3. 10 (1H, dd, J = 15. 4, 8. 1 Hz), 3.22 (1H, dd, J = 15. 6, 7. 8 Hz), 4.03 (2H, q, J = 7.2 Hz), 4.53 (2H, s), 4.90 (2H, d, J = 5.9 Hz), 5.14 (1H, t, J = 7.8 Hz), 7.23 (1H, s), 7.32 (1H, d, J = 5. 4 Hz), 7. 37-7. 41 (2H, m), 7.45 (1H, d, J = 5.4 Hz), 7.66 (1H, s). |
| 19h | 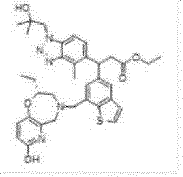 | MS(ESI/APCI) m/z: 644 [M + H]⁺ |
| 19i=19 | 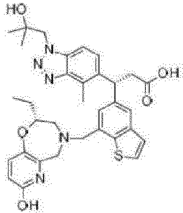 | ¹H-NMR (CDCl₃) δ : 1. 16 (3H, t, J = 7.3 Hz), 1.34 (3H, s). 1.35 (3H, s), 1.56-1.59 (1H, m), 1.67-1.73 (1H, m), 2. 58 (3H, s), 2.86 (1H, dd, J = 12.7, 10.3 Hz), 3. 12-3. 18 (2H, m), 3. 38 (2H, d, J = 5.9 Hz), 3. 84-3. 87 (2H, m), 3.96 (1H, d, J = 12.7 Hz), 4. 05 (1H, d, J = 15.6 Hz), 4.62 (2H, s), 4.81 (1H, t, J = 6. 3 Hz), 6.57 (1H, d, J = 9. 3 Hz), 7. 11-7. 12 (2H, m), 7.22 (1H, s), 7.37 (1H, d, J = 5.4 Hz), 7.50 (1H, d, J = 9.8 Hz), 7.59 (1H, d, J = 8.8 Hz), 7.63 (1H, d, J = 8.8 Hz). |
| | | MS(ESI/APCI) m/z: 616 [M + H]⁺ |

**[Table 21]**

| **Example No.** | **structural formula** | **physicochemical data** |
|---|---|---|
| 20a=20 | 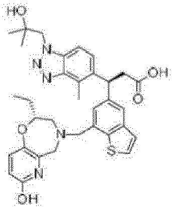 | ¹H-NMR (CDCl₃) δ : 1. 12 (3H, t, J = 7.3 Hz), 1. 30 (6H, s), 1.57-1. 62 (1H, m), 1.69-1.75 (1H, m), 2.75 (3H, s), 3.06 (1H, d, J = 13.2 Hz), 3.17-3.29 (2H, m), 3.40 (1H, d, J = 14.2 Hz), 3.51 (1H, d, J = 14.6 Hz), 3.73 (1H, d, J = 15.1 Hz), 3.87 (1H. s), 3.95 (2H, s), 4.56 (2H, s), 4.99 (1H, d, J = 6.8 Hz), 6.49 (1H, d, J = 9.3 Hz), 7.13 (1H, s), 7.19 (1H, d, J = 4.9 Hz), 7.39-7.49 (5H, m). |
| | | MS(ESI/APCI) m/z: 616 [M + H]⁺ |
| 21a | 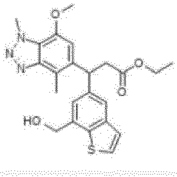 | ¹H-NMR (CDCl₃) δ : 1. 12 (3H, t, J = 7. 1 Hz), 1.85 (1H, t, J = 5.9 Hz), 2.75 (3H, s), 3.08 (1H, dd, J = 15.4, 8.5 Hz). 3.22 (1H, dd, J = 15.4, 7.6 Hz), 3.89 (3H, s), 4.04 (2H, q, J = 7.2 Hz), 4.41 (3H, s), 4.91 (2H, d, J = 5.9 Hz), 5.12 (1H, t, J = 8.1 Hz), 6.65 (1H, s), 7.22 (1H, s), 7.31 (1H, d, J = 5.4 Hz), 7.45 (1H, d, J = 5.4 Hz), 7.63 (1H, s). |
| | | MS(ESI/APCI) m/z: 440 [M + H]⁺ |
| 21c | 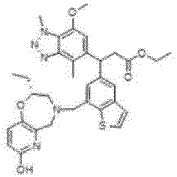 | MS(ESI/APCI) m/z: 616 [M + H]⁺ |
| 21d=21 | 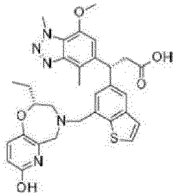 | ¹H-NMR (CDCl₃) δ : 1. 15 (3H, t, J = 7. 3 Hz), 1. 53-1. 61 (1H, m), 1. 66-1. 75 (1H, m), 2.44 (3H, s), 2. 83 (1H, t, J = 11. 2 Hz), 3. 15-3. 17 (2H, m), 3.38 (2H, d, J = 5.9 Hz), 3.83-3.87 (2H, m), 3.95 (1H, d, J = 12. 7 Hz), 4.05 (1H, d, J = 15.1 Hz), 4.09 (3H. s), 4.49 (3H, s), 4.79 (1H, d, J = 10.3 Hz), 6.57 (1H, d, J = 9.3 Hz), 6.89 (1H, s), 7.09 (1H, s), 7.12 (1H, d, J = 5.4 Hz), 7.24 (1H, s), 7.37 (1H, d, J = 5. 4 Hz), 7.50 (1H, d, J = 9.3 Hz). |
| | | MS(ESI/APCI) m/z: 588 [M + H]⁺ |
| 22a=22 | 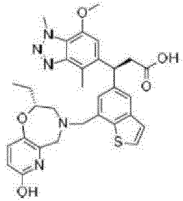 | ¹H-NMR (CDCl₃) δ : 1. 11 (3H, t, J = 7.3 Hz), 1. 55-1. 61 (1H, m), 1. 67-1. 76 (1H, m), 2.58 (3H, s). 3.04 (1H, d, J = 12.7 Hz), 3. 20-3. 30 (2H, m), 3. 37-3. 44 (2H, m), 3.77 (1H, d, J = 15. 6 Hz). 3. 83-3.85 (1H, m), 3.88-3.94 (5H, m), 4.42 (3H, s), 4.97 (1H, br s), 6.48 (1H, d, J = 9.3 Hz), 6.91 (1H, s), 7.01 (1H, s), 7.23 (1H, d, J = 5.4 Hz), 7.40-7.41 (2H, m), 7.53 (1H, s). |
| | | MS(ESI/APCI) m/z: 588 [M + H]⁺ |

**[Table 22]**

| **Example No.** | **structural formula** | **physicochemical data** |
|---|---|---|
| 23b=23 | 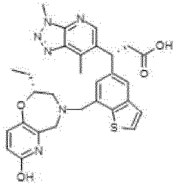 | ¹H-NMR (CDCl₃) δ : 1. 09-1. 18 (3H, br m), 1. 53-1. 59 (1H, m), 1.66-1.72 (1H, m), 2.59 (3H, s), 2.93-2.98 (1H, m), 3.14-3.19 (2H, m), 3.32-3.38 (2H, m), 3.80-4.01 (4H, m), 4.37 (3H, s), 4. 81 (1H, br s), 6. 53 (1H, br s), 7. 10-7. 19 (3H, m), 7.37-7.48 (2H, m), 8.73 (1H, s). |
| | | MS(ESI/APCI) m/z: 559 [M + H]⁺ |
| 24b=24 | 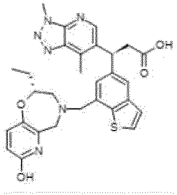 | ¹H-NMR (CDCl₃) δ : 1.07-1.11 (3H, br m), 1.56-1.60 (1H, br m), 1.67-1.71 (1H, br m), 2.73 (3H, s), 2.93-3.02 (1H, br m), 3.16-3.21 (2H, br m), 3.32-3.36 (1H, br m), 3.45-3.50 (1H, br m), 3. 63-3. 68 (1H, br m), 3. 83-3. 93 (3H, br m), 4. 30 (3H, s), 4.93 (1H, br s), 6.25-6.35 (1H, br m), 7.12-7.16 (3H, br m), 7.37-7.41 (2H, br m), 8.63 (1H, br s). |
| | | MS(ESI/APCI) m/z: 559 [M + H]⁺ |
| 25a | 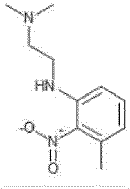 | ¹H-NMR (CDCl₃) δ : 2.27 (6H, s), 2.46 (3H, s), 2.57 (2H, t, J = 6. 1 Hz), 3.23 (2H, q, J = 5.7 Hz), 6.51 (1H, d, J = 7.3 Hz), 6.64 (1H, d, J = 8.3 Hz), 6.79 (1H, br s), 7.21 (1H, t, J = 7.8 Hz). |
| 25b | 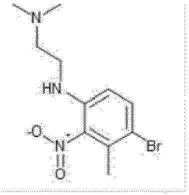 | MS(ESI/APCI) m/z: 302, 304 [M + H]⁺ |
| 25c | 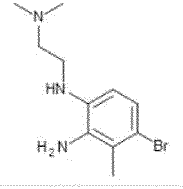 | MS(ESI/APCI) m/z: 272, 274 [M + H]⁺ |

**[Table 23]**

| **Example No.** | **structural formula** | **physicochemical data** |
|---|---|---|
| 25d | 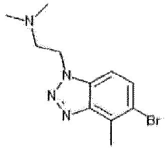 | MS(ESI/APCI) m/z: 283, 285 [M + H]⁺ |
| 26e | 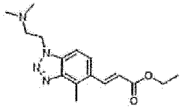 | MS(ESI/APCI) m/z: 303 [M + H]⁺ |
| 26h | 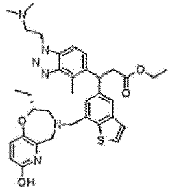 | MS(ESI/APCI) m/z: 643 [M + H]⁺ |
| 25i | 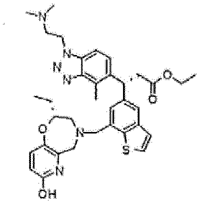 | ¹H-NMR (CDCl₃) δ : 0.92 (3H, t, J = 7.6 Hz), 1. 10 (3H, t, J = 7. 0 Hz), 1. 25-1. 34 and 1. 48-1. 57 (1H, each m), 2.29 (6H, s), 2.81-2.93 (7H, m), 3.10 (1H, dd, J = 15.8, 7.9 Hz), 3.23 (1H, dd, J = 15.8, 7.9 Hz), 3.68 (1H, d, J = 15.2 Hz), 3.74-3.81 (1H, m), 3.87-4.07 (5H, m), 4.67 (2H, t, J = 7.0 Hz), 5. 10 (1H, t, J = 8.2 Hz), 6.32 (1H, d, J = 9.7 Hz), 7.13 (1H, s), 7. 24-7. 27 (3H, m), 7.35 (1H, d, J = 9. 1 Hz), 7.38-7.40 (2H, m), 7.59 (1H, s), 11.66 (1H, br s). |
| | | MS(APCI) m/z: 643 [M + H]⁺ |
| 26j=25 | 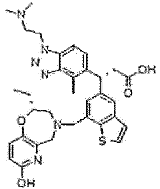 | ¹H-NMR (CDCl₃) δ : 1. 16 (3H, t, J = 7. 3 Hz), 1. 54-1. 59 (1H, m), 1. 66-1.75 (1H, m), 2. 36 (6H, s), 2. 56 (3H, s), 2.86 (1H, t, J = 11.5 Hz), 2.94 (2H, t, J = 6.8 Hz), 3.12-3.18 (2H, m), 3. 38 (2H, d, J = 5.4 Hz), 3. 83-3. 87 (2H, m), 3.95 (1H, d, J = 12. 7 Hz), 4.05 (1H, d, J = 15.6 Hz), 4.75 (2H, t, J = 6.8 Hz), 4.80 (1H, d, J = 10.7 Hz), 6.57 (1H, d, J = 9.3 Hz), 7. 10-7.11 (2H, m), 7.20 (1H, s), 7.37 (1H, d, J = 5.4 Hz), 7.50 (1H, d, J = 9.8 Hz), 7.53 (1H, d, J = 8.8 Hz), 7.61 (1H, d, J = 8.8 Hz). |
| | | MS(ESI/APCI) m/z: 615 [M + H]⁺ |

**[Table 24]**

| **Example No.** | **structural formula** | **physicochemical data** |
|---|---|---|
| 26a | 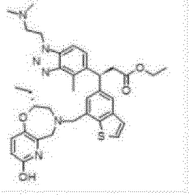 | ¹H-NMR (CDCl₃) δ : 0.95 (3H, t, J = 7.3 Hz), 1. 10 (3H, t, J = 7.0 Hz), 1. 27-1. 36 and 1. 50-1. 57 (1H, each m), 2.28 (6H, s), 2.83-2.95 (7H, m), 3.10 (1H, dd, J = 14.9, 7.6 Hz), 3. 31 (1H, dd, J = 15.2, 8.5 Hz), 3.67 (1H, d, J = 15.2 Hz), 3.74-3.80 (1H, m), 3.89-3.93 (2H, m), 3.98-4.05 (3H, m), 4.66 (2H, t, J = 7.0 Hz), 5.07 (1H, t, J = 7.9 Hz), 6.31 (1H, d, J = 9.7 Hz), 7.09 (1H, s), 7.24-7.27 (5H, m), 7.34 (1H, d, J = 8.5 Hz), 7.39 (1H, d, J = 5.5 Hz), 7.43 (1H, d, J = 8.5 Hz), 7.61 (1H, s), 12.03 (1H, br s). |
| | | MS(APCI) m/z: 643 [M + H]⁺ |
| 26b=26 | 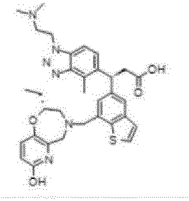 | ¹H-NMR (CDCl₃) δ : 1.09 (3H, t, J = 6.8 Hz), 1. 53-1. 58 (1H, m), 1. 65-1. 70 (1H, m), 2.30 (6H, s), 2. 75 (3H, s), 2.84-2.86 (2H, br m), 2. 95-2. 97 (1H, br m), 3. 12-3. 19 (2H, br m). 3. 32 (1H, d, J = 14.6 Hz), 3. 50 (1H, d, J = 12. 7 Hz), 3.69-3.73 (1H, br m), 3.82-3.94 (3H, m), 4.61-4.67 (2H, m), 4.98 (1H, d, J = 6. 3 Hz), 6.29 (1H, br s), 7. 14-7. 16 (2H, br m), 7. 27-7. 31 (1H, br m), 7.31-7.47 (4H, br m). |
| | | MS(ESI/APCI) m/z: 615 [M + H]⁺ |
| 27a | 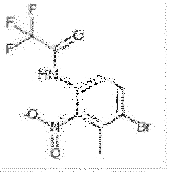 | ¹H-NMR (CDCl₃) δ : 2.50 (3H, s), 7.81 (1H, d, J = 8.8 Hz), 8.01 (1H, d, J = 8.8 Hz), 8.97 (1H, br s). |
| 27c | 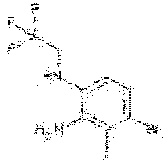 | ¹H-NMR (CDCl₃) δ : 2.32 (3H, s), 3. 43-3. 73 (5H, m), 6.52 (1H, d, J = 8.3 Hz), 7.01 (1H, d, J = 8.3 Hz). |
| 27d | 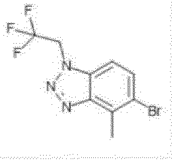 | MS(ESI/APCI) m/z: 294, 296 [M + H]⁺ |

**[Table 25]**

| **Example No.** | **structural formula** | **physicochemical data** |
|---|---|---|
| 27e | 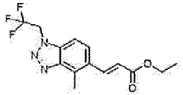 | MS(ESI/APCI) m/z: 314 [M + H]⁺ |
| 27f | 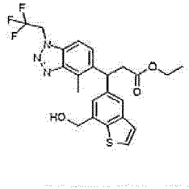 | ¹H-NMR (CDCl₃) δ : 1. 10 (3H, t, J = 7.1 Hz), 2.07 (1H, t, J = 5.6 Hz), 2.90 (3H, s), 3.11 (1H, dd, J = 15. 6, 8.3 Hz), 3.22 (1H, dd, J = 15.6, 7.3 Hz), 4.03 (2H, q, J = 7.0 Hz), 4. 90 (2H, d, J = 4.9 Hz), 5. 12-5.20 (3H, m), 7.22 (1H, s), 7.32 (1H, d, J = 5.4 Hz), 7.34 (1H, d, J = 8.8 Hz), 7.45 (1H, d, J = 5.4 Hz), 7.48 (1H, d, J = 8.8 Hz), 7.63 (1H, s). |
| 27i=27 | 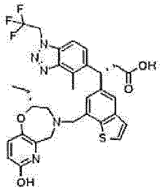 | ¹H-NMR (CDCl₃) δ : 1.16 (3H, t, J = 7. 4 Hz), 1. 54-1. 60 (1H, m), 1. 67-1. 74 (1H, m), 2.59 (3H, s), 2. 86 (1H, dd, J = 12. 7, 10.4 Hz), 3. 13 (1H, d, J = 15.7 Hz), 3.17 (1H, dd, J = 10. 5, 2.5 Hz), 3.39 (2H, d, J = 5.9 Hz), 3.81-3. 88 (2H, m), 3.96 (1H, d, J = 12.5 Hz), 4.05 (1H, d, J = 16. 3 Hz), 4. 81 (1H, dd, J = 12.7, 2,5 Hz), 5.22-5.29 (2H, m), 6.58 (1H, d, J = 9.8 Hz), 7. 11-7. 13 (2H, m), 7.18 (1H, d, J = 1.2 Hz), 7.38 (1H, d, J = 6.5 Hz), 7.51 (1H, d, J = 9.4 Hz), 7. 55 (1H, d, J = 8.6 Hz), 7. 70 (1H, d, J = 8.6 Hz). |
| 28a=28 | 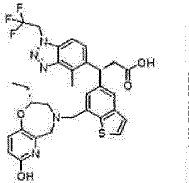 | ¹H-NMR (CDCl₃) δ : 1.12 (3H, t, J = 7.2 Hz), 1. 55-1.64 (1H, m), 1.68-1.77 (1H, m), 2.74 (3H, s), 3.04-3.08 (1H, m), 3. 17-3. 30 (2H, m), 3.41 (1H, d, J = 14.5 Hz), 3. 50 (1H, d, J = 15. 3 Hz), 3. 75 (1H, d, J = 15. 7 Hz), 3. 86-3. 89 (1H, m), 3.96 (2H, s), 4.99 (1H, dd, J = 10.0, 4.5 Hz), 5.16-5. 23 (2H, m), 6.46 (1H, d, J = 9.8 Hz), 7.13 (1H, s), 7. 21 (1H, d, J = 5. 5 Hz), 7. 37 (1H, d, J = 8.6 Hz), 7. 39-7. 42 (2H, m), 7.45 (1H, s), 7.55 (1H, d, J = 9.0 Hz), |
| 29a | 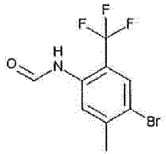 | MS(ESI/APCI) m/z: 282, 284 [M + H]⁺ |

**[Table 26]**

| **Example No.** | **structural formula** | **physicochemical data** |
|---|---|---|
| 29b | 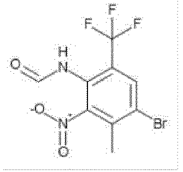 | ¹H-NMR (CDCl₃) *δ* : 2.47 (3H, s), 6.98 (1H, br s), 8.05-8. 11 (1H, m), 8.34 (1H, s). |
| 29d | 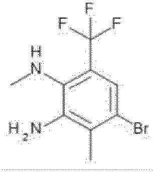 | ¹H-NMR (CDCl₃) *δ* : 2.31 (3H, s), 2.69 (3H, d, J = 5.4 Hz), 3.17 (1H, br s), 4.21 (2H, br s), 7.19 (1H, s). |
| 29e | 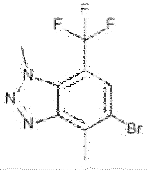 | ¹H-NMR (CDCl₃) *δ* : 2. 90 (3H, s), 4.41 (3H, s), 7.95 (1h, s). |
| 29f | 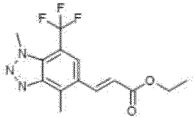 | ¹H-NMR (CDCl₃) *δ* : 1.37 (3H, t, J = 7. 1 Hz), 2.97 (3H, s), 4.31 (2H, q, J = 7.2 Hz), 4.42 (3H, s), 6.48 (1H, d, J = 16.1 Hz), 8.01 (1H, s), 8.10 (1H, d, J = 16.1 Hz). MS(ESI/APCI) m/z: 314 [M + H]' |
| 29g | 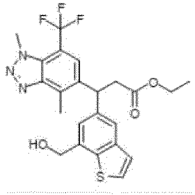 | ¹H-NMR (CDCl₃) *δ* : 1.11 (3H, t, J = 7.1 Hz), 2.91 (3H, s), 3.14 (1H, dd, J = 15.6, 8.3 Hz), 3.23 (1H, dd, J = 15.6, 7.3 Hz), 4.00-4.07 (2H, m), 4.39 (3H, s), 4.90-4.93 (2H, m), 5.15 (1H, t, J = 8.1 Hz), 7.20 (1H, s), 7.32 (1H, d, J = 5.4 Hz), 7.46 (1H, d, J = 5.4 Hz), 7.59 (1H, s), 7.73 (1H, s). |
| | | MS(ESI/APCI) m/z: 478 [M + H]⁺ |

**[Table 27]**

| **Example No.** | **structural formula** | **physicochemical data** |
|---|---|---|
| 29h | 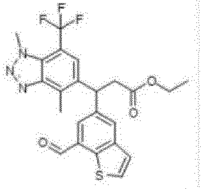 | MS(ESI/APCI) m/z: 476 [M + H]⁺ |
| 29j=29 | 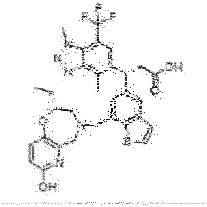 | ¹H-NMR (CDCl₃) δ : 1.16 (3H, t, J = 7. 3 Hz), 1. 55-1.61 (1H, m), 1. 67-1. 76 (1H, m), 2.62 (3H, s), 2.88 (1H, t, J = 11.5 Hz), 3.13 (1H, d, J = 15.6 Hz), 3.20 (1H, dd, J = 10.0, 1.7 Hz), 3.39-3.40 (2H, m), 3.84-3.88 (2H, m), 3.96 (1H, d, J = 12.7 Hz), 4.06 (1H, d, J = 15.6 Hz), 4.47 (3H, s), 4.82 (1H, d, J = 10.7 Hz), 6.58 (1H, d, J = 9.3 Hz), 7.10 (1H, s), 7.12 (1H, s), 7.14 (1H, d, J = 5.9 Hz), 7.40 (1H, d, J = 5.4 Hz), 7.52 (1H, d, J = 9.3 Hz), 7.90 (1H, s). |
| 30a=30 | 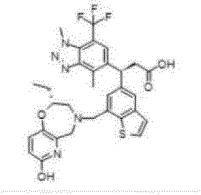 | ¹H-NMR (CDCl₃) *δ* : 1. 13 (3H, t, J = 7.1 Hz), 1. 57-1. 63 (1H, m), 1.70-1.76 (1H, m), 2.77 (3H, s), 3.05-3.09 (1H, br m), 3.20-3.29 (2H, m), 3.41 (1H, d, J = 13. 7 Hz), 3.49-3.53 (1H, m), 3. 72-3. 76 (1H, m), 3.87-3.89 (1H, br m), 3.97 (2H, br s), 4.40 (3H, s), 4.99-5.01 (1H, br m), 6.48 (1H, s), 7. 15 (1H, s), 7. 21 (1H, s), 7. 38-7. 43 (3H, m), 7. 79 (1H, s). |
| 31a | 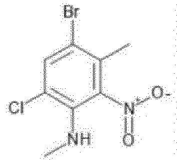 | ¹H-NMR (CDCl₃) δ : 2.29 (3H, s), 2.84 (3H, d, J = 5. 5 Hz), 4.46 (1H, br s), 7.55 (1H, s). |
| | | MS(ESI/APCI) m/z: 279, 281 [M + H]' |
| 31b | 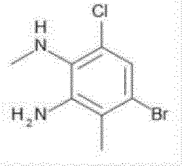 | ¹H-NMR (CDCl₃) δ : 2.24 (3H, s), 2.66 (3H, s), 3.19 (1H, br s), 4.17 (2H, br s), 7.03 (1H, s). |
| | | MS(ESI/APCI) m/z: 249, 251 [M + H]⁺ |

**[Table 28]**

| **Example No.** | **structural formula** | **physicochemical data** |
|---|---|---|
| 31c | 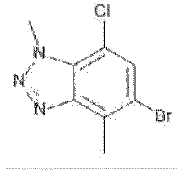 | 'H-NMR (CDCl₃) *δ* : 2.78 (3H, s), 4.53 (3H, s), 7.59 (1H, s). |
| | | MS(ESI/APCI) m/z: 260, 262 [M + H]' |
| 31d | 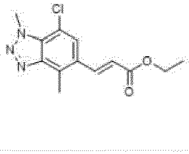 | ¹H-NMR (CDCl₃) *δ* : 1.33 (3H, t, J = 7.2 Hz), 2.83 (3H, s), 4.27 (2H, q, J = 7.2 Hz), 4.51 (3H, s), 6.37 (1H, d, J = 15.9 Hz), 7.61 (1H, s), 8.01 (1H, d, J = 15.9 Hz). |
| | | MS(ESI/APCI) m/z: 280, 282 [M + H]⁺ |
| 31e | 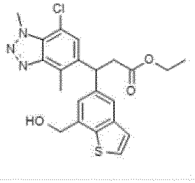 | MS(ESI/APCI) m/z: **444, 446** [M + H]⁺ |
| 31g | 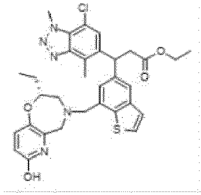 | MS(ESI/APCI) m/z: 620, 622 **[M** + H]⁺ |
| 31h=31 | 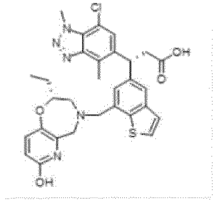 | ¹H-NMR (CDCl₃) *δ* : 1. 16 (3H, t, J = 7. 1 Hz), 1. 56-1. 61 (1H, m), 1. 68-1. 74 (1H, m), 2. 51 (3H, s), 2.81 (1H, dd, J = 12. 7, 10.3 Hz), 3. 11-3. 17 (2H, m), 3.38-3.39 (2H, m), 3.83-3.87 (2H, m), 3.96 (1H, d, J = 12.7 Hz), 4.05 (1H, d, J = 15.6 Hz), 4.58 (3H, s), 4.76 (1H, d, J = 12. 7 Hz), 6. 58 (1H, d, J = 9.3 Hz), 7.09 (1H, s), 7. 14 (1H, d, J = 6.4 Hz), 7. 18 (1H, s), 7.39 (1H, d, J = 5. 4 Hz), 7. 51 (1H, d, J = 9. 3 Hz), 7. 54 (1H, s). |
| | | MS(ESI/APCI) m/z: 592, 594 [M + H]⁺ |

**[Table 29]**

| **Example No.** | **structural formula** | **physicochemical data** |
|---|---|---|
| 32a=32 | 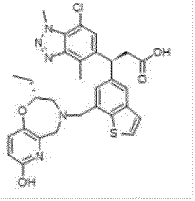 | ¹H-NMR (CDCl₃) *δ* : 1. 12 (3H, t, J = 7. 1 Hz), 1. 58-1. 63 (1H, m), 1. 70-1. 75 (1H, m), 2. 69 (3H, s), 3. 00-3. 04 (1H, br m), 3. 13-3. 17 (1H, br m), 3.26-3.29 (1H, br m), 3.41 (1H, d, J = 14.6 Hz), 3. 50 (1H, d, J = 13. 4 Hz), 3. 72 (IH, d, J = 15. 2 Hz), 3.86-3.88 (1H, br m), 3.96 (2H, br s), 4.52 (3H, s), 4.95 (1H. br s), 6.45 (1H, br s), 7.13 (1H, s), 7.20 (1H, s), 7.36-7.43 (4H, m). |
| | | MS(ESI/APCI) m/z: 592, 594 [M + H)⁺ |
| 33a | 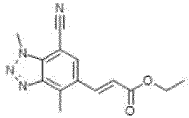 | ¹H-NMR (CDCl₃) *δ* : 1.37 (3H, t, J = 7.3 Hz), 2.97 (3H. s), 4.31 (2H, q, J = 7.0 Hz), 4.55 (3H, s), 6.45 (1H, d, J = 16.1 Hz), 8. 01-8. 07 (2H, m). |
| 33b | 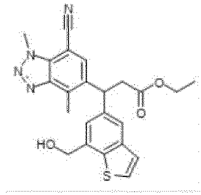 | ¹H-NMR (CDCl₃) δ : 1.15 (3H, t, J = 7.1 Hz), 1.93 (3H, s), 2.94 (3H, s), 3.11 (1H, dd, J = 15.9, 8.5 Hz), 3.22 (1H, dd, J = 15.6, 7.3 Hz), 4.51 (3H, s), 4.92 (2H, d, J = 5.9 Hz), 5.12 (1H, t, J = 7.8 Hz), 7.17 (1H, s), 7.33 (1H, d, J = 5.9 Hz), 7.49 (1H, d, J = 5.4 Hz), 7.59 (1H, s), 7.75 (1H, s). |
| 33e=33 | 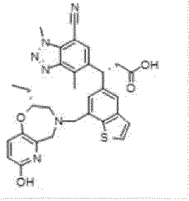 | ¹H-NMR (CDCl₃) *δ* : 1.16 (3H, t, J = 7. 3 Hz), 1.54-1. 62 (1H, m), 1.67-1.76 (1H, m), 2.64 (3H. s), 2.84 (1H, t, J = 11.5 Hz), 3.10-3.18 (2H, m), 3.38-3.40 (2H, m), 3.83-3.88 (2H, m), 3.96 (1H, d, J = 12.7 Hz), 4.06 (1H, d, J = 15.6 Hz), 4.59 (3H, s), 4. 79 (1H, d, J = 11.2 Hz), 6. 59 (1H, d, J = 9.3 Hz), 7.09 (1H, s), 7.11 (1H, s), 7.14 (1H, d, J = 6.4 Hz), 7.42 (1H, d, J = 5.4 Hz), 7.52 (1H, d, J = 9.3 Hz), 7.93 (1H, s). |
| 34a=34 | 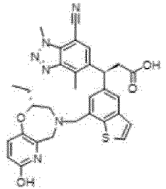 | ¹H-NMR (CDCl₃) *δ* : 1.12 (3H, t, J = 6. 3 Hz), 1. 57-1. 63 (1H, m), 1.69-1.75 (1H, m), 2.86 (3H, s), 2.98-3.01 (1H, br m), 3. 16 (1H, t, J = 13.2 Hz), 3.23-3.28 (1H, m), 3.37 (1H, t, J = 12.9 Hz), 3.46 (1H, d, J = 15. 1 Hz), 3. 76 (1H, d, J = 15.6 Hz), 3.88-3.90 (1H, br m), 3.97 (2H, br s), 4.51 (3H, s), 5.00 (1H, d, J = 8.3 Hz), 6.43 (1H, br s), 7.18 (1h, s), 7.22 (1H, d, J = 6. 0 Hz), 7. 36-7. 42 (2H, m), 7.44 (1H, d, J = 6.0 Hz), 7. 72 (1H, br s). |

**[Table 30]**

| **Example No.** | **structural formula** | **physicochemical data** |
|---|---|---|
| 35a | 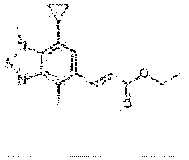 | ¹H-NMR (CDCl₃) *δ* : 0.84-0.92 (2H, m), 1.03-1.13 (2H, m), 1.36 (3H, t, J = 7.1 Hz), 2.27-2.32 (1H, m), 2. 85 (3H, s), 4.29 (2H, q, J = 7.0 Hz), 4.60 (3H, s), 6.39 (1H, d, J = 16.1 Hz), 7.38 (1h, s), 8.09 (1H, d, J = 16.1 Hz). |
| | | MS(ESI/APCI) m/z: 286 [M + H]⁺ |
| 35b | 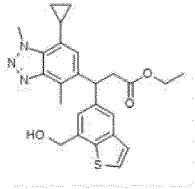 | ¹H-NMR (CDCl₃) *δ* : 0.70-0.76 (1H, m), 0.83-0.90 (1H, m), 1. 00-1.05 (2H, m), 1.10 (3H, t, J = 7. 1 Hz), 2.24-2.33 (1H, m), 2.77 (3H, s), 3.08 (1H, dd, J = 15.6, 8.3 Hz), 3.18 (1H, dd, J = 15. 4, 7.6 Hz), 4.01 (2H, q, J = 7.0 Hz), 4.56 (3H, s), 4.90 (2H, d, J = 5.4 Hz), 5.07 (1H, t, J = 8.1 Hz), 7.12 (1H, s), 7.20 (1H, s), 7.30 (1H, d, J = 5.4 Hz), 7.44 (1H, d, J = 5.4 Hz), 7.59 (1H, s). |
| | | MS(ESI/APCI) m/z: 450 [M + H]⁺ |
| 35g | 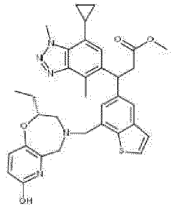 | MS(ESI/APCI) m/z: 612 [M + H]* |
| 35h | 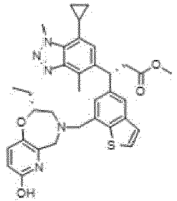 | ¹H-NMR (CDCl₃) *δ* : 0.71-0.75 (1H, m), 0.81-0.85 (1H, m), 0.92 (3H, t, J = 7.3 Hz), 0.99-1.05 (2H, m), 1. 28-1. 34 (1H, m), 1.48-1.55 (1H, m), 2.25-2.32 (1H, m), 2. 77 (3H, s), 2.81-2.92 (2H, m), 3.09 (1H, dd, J = 15.2, 7.9 Hz), 3.22 (1H, dd, J = 15. 5, 8.2 Hz), 3.58 (3H, s), 3. 71 (1H, d, J = 15. 2 Hz), 3. 73-3. 80 (1H, m), 3.86-3.96 (2H, m), 4.01 (1H, d, J = 15.2 Hz), 4. 56 (3H, s), 5.03 (1H, t, J = 7.9 Hz), 6.34 (1H, d, J = 9. 7 Hz), 7.09 (1H, s), 7. 11 (1H, s), 7.24-7.29 (2H, m), 7.39 (1H, d, J = 5.5 Hz), 7.53 (1H, d, J = 1.8 Hz). |
| | | MS(APCI) m/z: 612 [M + H]⁺ |
| 35i=35 | 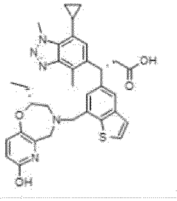 | ¹H-NMR (CDCl₃) *δ* : 0.84-0.93 (3H, m). 1.01-1.05 (1H, m), 1.16 (3H, t, J = 7.3 Hz), 1.53-1.61 (1H, m), 1. 66-1. 74 (1H, m), 2.43-2.48 (1H, m), 2.50 (3H, s), 2.84 (1H, t, J = 11.5 Hz), 3.13-3.16 (2H, m), 3.38-3.39 (2H, m), 3.83-3.87 (2H, m), 3.95 (1H, d, J = 12.7 Hz), 4.05 (1H, d, J = 15.6 Hz), 4.65 (3H, s), 4.76 (1H, d, J = 10.7 Hz), 6.57 (1H, d, J = 9.3 Hz), 7. 10 (1H, s), 7.11 (1H, d, J = 5.4 Hz), 7. 15 (1H, s), 7.30 (1H, s), 7.37 (1H, d, J = 5.4 Hz), 7.50 (1H, d, J = 9. 3 Hz). |

**[Table 31]**

| **Example No.** | **structural formula** | **physicochemical data** |
|---|---|---|
| 36a | 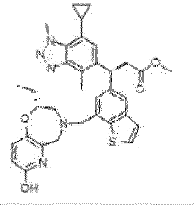 | ¹H-NMR (CDCl₃) δ : 0.70-0. 75 (1H, m), 0.78-0. 84 (1H, m), 0.95 (3H, t, J = 7.3 Hz), 0.98-1.03 (2H, m), 1. 29-1. 36 (1H, m), 1.49-1.57 (1H, m), 2.24-2.31 (1H, m), 2.78 (3H, s), 2. 82-2. 94 (2H, m), 3.09 (1H, dd, J = 14. 6, 7.9 Hz), 3.29 (1H, dd, J = 15.2, 8.5 Hz), 3.56 (3H, s), 3.69 (1H, d, J = 15. 2 Hz), 3. 73-3. 78 (1H, m), 3.88-3.93 (2H, m), 4. 03 (1H, d, J = 14.6 Hz), 4.55 (3H, s), 5.01 (1H, t, J = 8.2 Hz), 6.31 (1H, d, J = 9. 7 Hz), 7.07 (1H, s), 7. 10 (1H, s), 7. 24-7. 28 (2H, m), 7.39 (1H, d, J = 5.5 Hz), 7.57 (1H, d, J = 1.2 Hz). |
| | | MS(APCI) m/z: 612 [M + H]⁺ |
| 36b=36 | 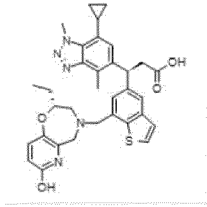 | ¹H-NMR (CDCl₃) *δ* : 0.76-0.80 (1H, m), 0.84-0.91 (2H, m), 1. 04-1. 06 (1H, m), 1. 12 (3H, t, J = 7. 3 Hz), 1.56-1.64 (1H, m), 1. 69-1. 76 (1H, m), 2.30-2.34 (1H, br m), 2.68 (3H, s), 3.03 (1H, d, J = 16. 1 Hz), 3. 19 (1H, t, J = 13. 2 Hz), 3.27 (1H, dd, J = 14.6, 8.3 Hz), 3. 42 (1H, d, J = 14.6 Hz), 3. 52 (1H, d, J = 17. 1 Hz), 3.70-3.73 (1H, m), 3.86-3.88 (1H, br m), 3.95 (2H, s), 4.60 (3H, s), 4.94 (1H, d, J = 7.8 Hz), 6. 51 (1H, d, J = 9. 3 Hz), 7. 10 (1H, s), 7. 18-7.20 (2H, m), 7.39-7.39 (2H, m), 7.42 (1H, d, J = 9.3 Hz). |
| 37a | 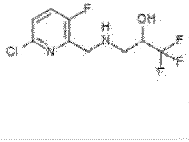 | ¹H-NMR (CDCl₃) *δ* : 2.94-3.00 (2H, m), 3. 98-4. 03 (3H, m), 7.27-7.28 (1H, m), 7.39 (1H, t, J = 8.5 Hz). |
| | | MS(ESI/APCI) m/z: 273 [M + H]⁺ |
| 37b | 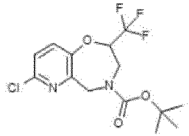 | ¹H-NMR (CDCl₃) *δ* : 1.41 (9H, s), 3.53 (1H, s), 4. 18-4. 20 (1H, m), 4.33 (1H, d, J = 12.3 Hz), 4.41 (1H, d, J = 15.9 Hz), 4.92 (1H, d, J = 15.9 Hz), 7. 19 (1H, d, J = 8.6 Hz), 7. 37 (1H, d, J = 8.6 Hz). |
| | | MS(ESI/APCI) m/z: 297 [M - tBu + H]* |
| 37c | 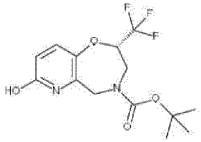 | ¹H-NMR (CDCl₃) *δ* : 1.43 (9H, s), 3.36 (1H, s), 4.03 (1H, t, J = 7.4 Hz), 4.20 (1H, d, J = 15.9 Hz), 4.40 (1H, s), 4.77 (1H, d, J = 15.9 Hz), 6.44 (1H, t, J = 7.1 Hz), 7.32 (1H, d, J = 9.2 Hz). |
| | | MS(ESI/APCI) m/z: 279 [M - tBu + H]⁺ |

**[Table 32]**

| **Example No.** | **structural formula** | **physicochemical data** |
|---|---|---|
| 37d | 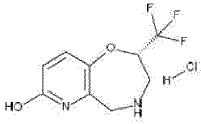 | ¹H-NMR (DMSO-D₆) *δ* : 3.63 (1H, d, J = 11.0 Hz), 3.82 (1H, d, J = 12.9 Hz), 4.22 (1H, d, J = 14.7 Hz), 4.46 (1H, d, J = 14. 7 Hz), 4. 91 (1H, s), 6.57 (1H, d, J = 8.6 Hz), 7.47 (1H, d, J = 9.2 Hz). |
| | | MS(ESI/APCI) m/z: 235 [M + H]⁺ |
| 37e | 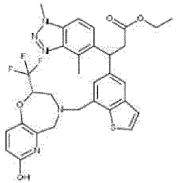 | ¹H-NMR (CDCl₃) δ : 1.07-1. 13 (3H, m), 2. 85 (1. 5H, s), 2.87 (1. 5H. s), 3.07-3.38 (4H. m), 3.64 (0. 5H, d, J = 15. 3 Hz), 3. 74 (0. 5H, d, J = 15. 3 Hz), 3. 87-4. 08 (5H, m), 4. 20-4. 29 (4H, m), 5.01-6.12 (1H, m), 6.32-6.39 (1H, m), 7.08 (0.5H, s), 7. 11 (0. 5H, s), 7. 23-7. 37 (3H, m), 7. 37-7. 47 (2H, m), 7.60 (0.5H, s), 7.65 (0.5H, s). |
| | | MS(ESI/APCI) m/z: 626 [M + H]⁺ |
| 37f=37 | 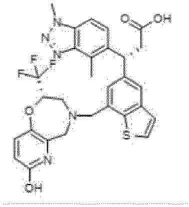 | ¹H-NMR (CDCl₃) *δ* : 2.56 (3H, s), 2.81-2.91 (1H, m), 3. 16-3. 26 (2H, m), 3.61-3.73 (2H, m), 3. 83 (1H, d, J = 12.3 Hz), 4.01 (1H, d, J = 12.9 Hz), 4.07 (1H, d, J = 15.9 Hz), 4. 23-4. 31 (1H, m), 4. 33 (3H, s), 4.80 (1H, d, J = 10.4 Hz), 6.61 (1H, d, J = 9.8 Hz), 7.09 (1H, s), 7. 11 (1H, d, J = 5.5 Hz), 7.20 (1H, s), 7.39 (1H, d, J = 5.5 Hz), 7.51 (1H, d, J = 8.6 Hz), 7.58 (1H, d, J = 9.2 Hz), 7.63 (1H, d, J = 8.6 Hz). |
| | | MS(ESI/APCI) m/z: 598 [M + H]⁺ |
| 38a=38 | 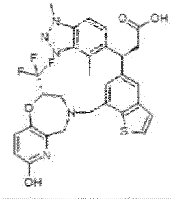 | ¹H-NMR (CDCl₃) *δ* : 2.80 (3H, s), 3.05 (1H, d, J = 16.6 Hz), 3.27 (1H, dd, J = 16.9, 11.3 Hz), 3.52 (2H, dd, J = 14.4, 8.9 Hz), 3.67 (1H, d, J = 13. 5 Hz), 3.86 (1H, d, J = 14. 7 Hz), 3.95 (2H, q, J = 13.3 Hz), 4.21 (1H, dt, J = 13.5, 4.3 Hz), 4.26 (3H, s), 5.03 (1H, dd, J = 11.3, 4.0 Hz), 6.48 (1H, d, J = 9.8 Hz), 7. 15 (1H, s), 7.20 (1H, d, J = 5.5 Hz), 7.33 (1H, d, J = 8.6 Hz), 7.40 (1H, d, J = 5.5 Hz), 7.44-7.52 (3H, m). |
| | | MS(ESI/APCI) m/z: 598 [M + H]* |
| 39a | 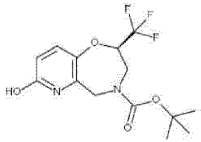 | ¹H-NMR (CDCl₃) δ : 1.44 (9H, s), 3.36 (1H, s), 4.03 (1H, s), 4.20 (1H, d, J = 16.6 Hz), 4.40 (1H, s), 4.75 (1H, d, J = 15.9 Hz), 6.44 (1H, d, J = 9.8 Hz), 7.32 (1H, d, J = 9.2 Hz). |
| | | MS(ESI/APCI) m/z: 279 [M - tBu + H]⁺ |

**[Table 33]**

| **Example No.** | **structural formula** | **physicochemical data** |
|---|---|---|
| 39b | 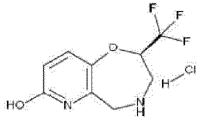 | ¹H-NMR (DMSO-D₆) δ : 3.63 (1H, d, J = 11.7 Hz), 3.82 (1H, d, J = 12. 9 Hz), 4.22 (1H, d, J = 14.7 Hz), 4.46 (1H, d, J = 15.3 Hz), 4.91 (1H, t, J = 7.7 Hz), 6.56 (1H, d, J = 8.6 Hz), 7.47 (1H, d, J = 9.2 Hz). |
| | | MS(ESI/APCI) m/z: 235 [M + H]⁺ |
| 39c | 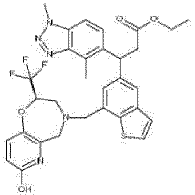 | ¹H-NMR (CDCl₃) δ : 1. 10 (3H, td, J = 7.1, 3. 1 Hz), 2. 86 (3H, d, J = 8.6 Hz), 3. 08-3. 36 (4H, m), 3. 70 (1H, dd, J = 40. 5, 15. 3 Hz), 3. 89-4. 08 (5H, m), 4.23 (4H, d, J = 6.7 Hz), 5.07 (1H, dt, J = 14. 7, 6.3 Hz), 6.35 (1H, t, J = 11.0 Hz), 7. 10 (1H, d, J = 9.8 Hz), 7.32 (3H, ddd, J = 13. 6, 8.4, 5. 1 Hz), 7.42 (2H, dt, J = 13. 5, 6. 1 Hz), 7.63 (1H, d, J = 19.0 Hz). |
| | | MS(ESI/APCI) m/z: 626 [M + H]⁺ |
| 39d=39 | 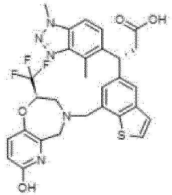 | ¹H-NMR (CDCl₃) *δ* : 2. 56 (3H, s), 2. 80-2. 91 (1H, m), 3. 21 (2H, t, J = 11.0 Hz), 3.61-3.74 (2H, m), 3.83 (1H, d, J = 12.9 Hz), 4.01 (1H, d, J = 12.9 Hz), 4.07 (1H, d, J = 15.9 Hz), 4.26 (1H, dd, J = 11.7, 5.5 Hz), 4.33 (3H, s), 4.80 (1H, d, J = 11.0 Hz), 6.61 (1H, d, J = 9.2 Hz), 7.09 (1H, s), 7.11 (1H, d, J = 5.5 Hz), 7.20 (1H, s), 7.39 (1H, d, J = 5.5 Hz), 7. 51 (1H, d, J = 8.6 Hz), 7.58 (1H, d, J = 9. 2 Hz), 7.63 (1H, d, J = 8.6 Hz). |
| | | MS(ESI/APCI) m/z: 598 [M + H]⁺ |
| 40a=40 | 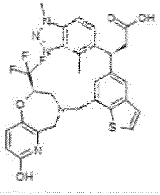 | ¹H-NMR (CDCl₃) δ : 2.80 (3H, s), 3.06 (1H, d, J = 14. 1 Hz), 3.27 (1H, dd, J = 16. 3, 11. 3 Hz), 3.52 (2H, dd, J = 14. 7, 9.2 Hz), 3.67 (1H, d, J = 14.7 Hz), 3.86 (1H, d, J = 15.3 Hz), 3.95 (2H, q, J = 13. 7 Hz), 4. 22 (1H, s), 4.26 (3H, s), 5.03 (1H, dd, J = 11.3, 3.4 Hz), 6.49 (1H, d, J = 9.2 Hz), 7. 15 (1H, s), 7.20 (1H, d, J = 5.5 Hz), 7.33 (1H, d, J = 8.0 Hz), 7.40 (1N, d, J = 5.5 Hz), 7.44-7.52 (3H, m). |
| | | MS(ESI/APCI) m/z: 598 [M + H]* |
| 41a | 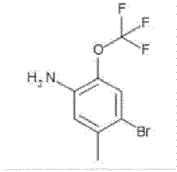 | ¹H-NMR (CDCl₃) *δ* : 2. 29 (3H, s), 3.81 (2H, s), 6.69 (1H, s), 7.29 (1H, s). |
| | | MS(ESI/APCI) m/z: 270 [M + H]' |

**[Table 34]**

| **Example No.** | **structural formula** | **physicochemical data** |
|---|---|---|
| 41b | 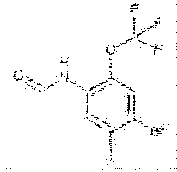 | major rotamer: ¹H-NMR (CDCl₃) δ : 2.41 (3H, s), 7.38 (1H, s), 7.45 (1H, s), 8.39 (1H, s), 8.47 (1H, s). |
| 41c | 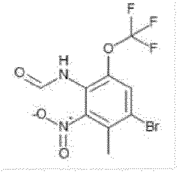 | ¹H-NMR (CDCl₃) *δ* : 2.42 (3H, s), 7.05 (1H, s), 7.74 (1H, s), 8.31 (1H, s). |
| 41d | 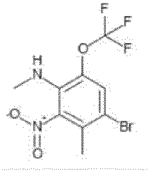 | ¹H-NMR (CDCl₃) *δ* : 2.32 (3H, s), 2.89 (3H, d, J = 5.9 Hz), 4.47 (1H, s), 7.43 (1H, s). |
| | | MS(ESI/APCI) m/z: 329 [M + H]⁺ |
| 41e | 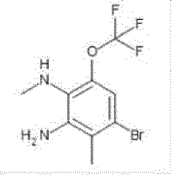 | ¹H-NMR (CDCl₃) *δ* : 2.25 (3H, s), 2.67 (3H, s), 3.13 (1H, s), 4.12 (2H, s), 6.91 (1H, s). |
| | | MS(ESI/APCI) m/z: 299 [M + H]* |
| 41f | 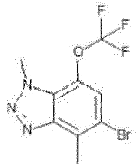 | ¹H-NMR (CDCl₃) *δ* : 2.81 (3H, s), 4.42 (3H, s), 7.50 (1H, s). |
| 41g | 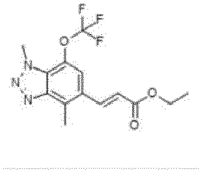 | ¹H-NMR (CDCl₃) δ : 1.37 (3H, t, J = 6.8 Hz), 2.89 (3H, s), 4.30 (2H. q. J = 7.2 Hz), 4. 43 (3H, s), 6.38 (1H, d, J = 16. 1 Hz), 7.53 (1H, s), 8.07 (1H, d, J = 15. 6 Hz). |

**[Table 35]**

| **Example No.** | **structural formula** | **physicochemical data** |
|---|---|---|
| 41h | 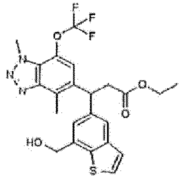 | ¹H-NMR (CDCl₃) 6: 1.11 (3H, t, J = 7.1 Hz), 1.82 (1H, t, J = 6.1 Hz), 2.83 (3H, s), 3.09 (1H, dd, J = 15.6, 8.3 Hz), 3.18 (1H, dd, J = 15.6, 7.8 Hz), 4. 03 (2H, q, J = 7.0 Hz), 4.40 (3H, s), 4.91 (2H, d, J = 6. 3 Hz), 5.11 (1H, t, J = 8.1 Hz), 7. 19 (1H, s), 7.30 (1H, s), 7.31 (1H, d, J = 5.9 Hz), 7.45 (1H, t, J = 7.3 Hz), 7.60 (1H, s). |
| 41i | 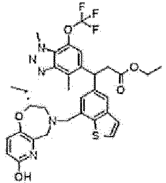 | ¹H-NMR (CDCl₃) *δ* : 0.90-0.95 (3H, m), 1. 11 (3H, t, J = 7. 3 Hz), 1.46-1.65 (2H, m), 2.76-2.87 (6H, m), 3.04-3.09 (1H, m), 3.14-3.24 (1H, m), 3. 64-3. 76 (2H, m), 3.86-3. 94 (2H, m), 3.97-4.03 (3H, m), 4.36 (3H, s), 5.01-5.07 (1b, m), 6. 28-6. 31 (1H, m), 7.04-7.09 (1H, m), 7. 20-7. 30 (3H, m), 7.37-7.40 (1H, m), 7.49-7.66 (1H, m), 12.02 (LH, br s). |
| | | MS(ESI/APCI) m/z: 670 [M + H]⁺ |
| 41j=41 | 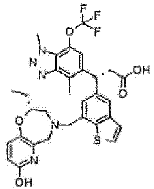 | ¹H-NMR (CDCl₃) *δ* : 1.15 (3H, t, J = 7. 3 Hz), 1.62-1. 61 (1H, m), 1.66-1.75 (1H, m), 2. 54 (3H, s), 2. 80 (1H, t, J = 11. 5 Hz), 3. 09-3. 18 (2H, m), 3.38 (2H, d, J = 5.4 Hz), 3. 81-3.88 (2H, m), 3.95 (1H, d, J = 12.7 Hz), 4.06 (1H, d, J = 15.6 Hz), 4.46 (3H, s), 4. 79 (1H, d, J = 10.7 Hz), 6.57 (1H, d, J = 9.3 Hz), 7.10 (1H, s), 7. 12 (1H, d, J = 5.4 Hz), 7.14 (1H, s), 7.39 (1H, d, J = 5. 4 Hz), 7.47 (1H, s), 7. 51 (1H, d, J = 9. 3 Hz). |
| | | MS(ESI/APCI) m/z: 642 [M + H]⁺ |
| 42a=42 | 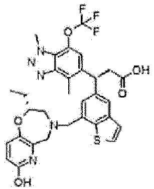 | ¹H-NMR (CDCl₃) *δ* : 1. 12 (3H, t, J = 6.8 Hz), 1. 66-1. 61 (1H, m), 1.69-1.74 (1H, m), 2.73 (3H, s), 2.99-3.06 (1H, m), 3.11-3.16 (1H, m), 3.24-3.28 (1H, m), 3.38-3.41 (1H, m), 3.51 (1H, d, J = 15.8 Hz), 3.71 (1H, d, J = 14.2 Hz), 3. 85-3. 89 (1H, br m), 3. 95 (2H, br s), 4.41 (3H, s), 4. 97 (1H, d, J = 10.0 Hz), 6. 46 (1H, br s), 7. 10 (1H, s), 7. 18-7. 22 (1H, br m), 7. 38-7. 43 (4H, br m). |
| | | MS(ESI/APCI) m/z: 642 [M + H]⁺ |
| 43a | 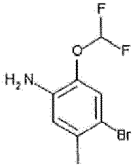 | ¹H-NMR (CDCl₃) *δ* : 2. 28 (3H, s), 3. 80 (2H, br s), 6. 42 (1H, t, J = 74.0 Hz), 6.67 (1H, s), 7.19 (1H, s). |

**[Table 36]**

| **Example No.** | **structural formula** | **physicochemical data** |
|---|---|---|
| 43c | 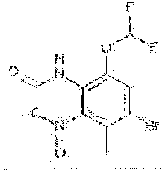 | ¹H-NMR (CDCl₃) *δ* : 2.40 (3H, s), 6. 36-6. 71 (1H, m), 7.06-7.20 (1H, m), 7.68 (1H, s), 8.26-8.37 (1H, m). |
| 43d | 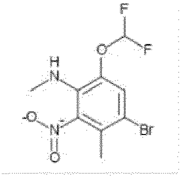 | ¹H-NMR (CDCl₃) *δ* : 2.30 (3H, s), 2.86 (3H, d, J = 5. 4 Hz), 4.45 (1H, br s), 6.45 (1h, t, J = 73.0 Hz), 7.32 (1H, s). |
| | | MS(ESI/APCI) m/z: 311, 313 [M + H]⁺ |
| 43e | 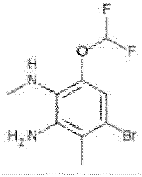 | ¹H-NMR (CDCl₃) *δ* : 2.25 (3H, s), 2.65 (3H, s), 3.20 (1H, br s), 4.13 (2H, br s), 6.46 (1H, t, J = 74.0 Hz), 6.78 (1H, s). |
| | | MS(ESI/APCI) m/z: 281, 283 [M + H]⁺ |
| 43f | 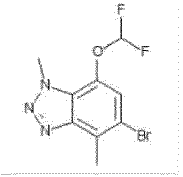 | ¹H-NMR (CDCl₃) *δ* : 2.78 (3H, s), 4.43 (3H, s), 6.67 (1H, t, J = 72.5 Hz), 7.33 (1H, s). |
| 43g | 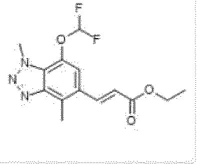 | ¹H-NMR (CDCl₃) *δ* : 1.36 (3H, t, J = 7.1 Hz), 2.86 (3H, s), 4.30 (2H, q, J = 7.2 Hz), 4.44 (3H, s), 6.37 (1H, d, J = 16. 1 Hz), 6.69 (1H, t, J = 72. 5 Hz), 7.35 (1h, s), 8.08 (1H, d, J = 16. 1 Hz). |

**[Table 37]**

| **Example No.** | **structural formula** | **physicochemical data** |
|---|---|---|
| 43h | 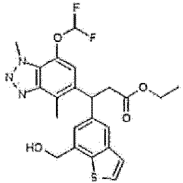 | ¹H-NMR (CDCl₃) δ : 1. 12 (3H, t, J = 7.1 Hz), 1.84 (1H, t, J = 5. 9 Hz), 2.81 (3H, s), 3.08 (1H, dd, J = 15.4, 8.6 Hz), 3. 19 (1H, dd, J = 15. 4, 7.6 Hz), 4.03 (2H, q, J = 7.0 Hz), 4. 40 (3H, s), 4.91 (2H, d, J = 5. 9 llz), 5. 11 (1H, dd, J = 8. 6, 7.6 Hz), 6.61 (1H, t, J = 73.0 Hz), 7. 12 (1H, s), 7.20 (1H, s), 7.32 (1H, d, J = 6.9 Hz), 7. 46 (1H, d, J = 5.4 Hz), 7.61 (1H, s). |
| 43i | 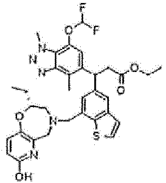 | MS(ESI/APCI) m/z: 652 [M + H]⁺ |
| 43j=43 | 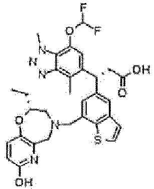 | ¹H-NMR (CDCl₃) δ : 1.16 (3H, t, J = 7.3 Hz), 1. 54-1. 62 (1H, m), 1. 67-1. 75 (1H, m), 2.52 (3H, s), 2.81 (1H, t, J = 11.5 Hz), 3. 11-3. 17 (2H, m), 3.39 (2H, d, J = 5. 9 Hz), 3. 83-3. 88 (2H, m), 3.96 (1H, d, J = 12.7 Hz), 4.05 (1H, d, J = 15.6 Hz), 4.47 (3H, s), 4. 79 (1H, d, J = 11.2 Hz), 6. 58 (1H, d, J = 9.3 Hz), 6.79 (1H, t, J = 72.7 Hz), 7.10 (1H, s), 7.13 (1H, d, J = 5.4 Hz), 7.17 (1H, s), 7.33 (1H, s), 7.39 (1H, d, J = 5.4 Hz), 7.51 (1H, d, J = 9.3 Hz). |
| | | MS(ESI/APCI) m/z: 624 [M + H]⁺ |
| 44a=44 | 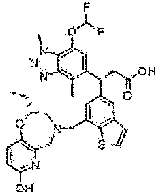 | ¹H-NMR (CDCl₃) δ : 1. 12 (3H, t, J = 6. 6 Hz), 1. 57-1.62 (1H, m), 1. 69-1. 76 (1H, m), 2. 68 (3H, s), 3.04 (1H, d, J = 13.8 Hz), 3. 14-3. 19 (1H, m), 3.26-3.30 (1H, m), 3.39-3.49 (2H, m), 3.74 (1H, d, J = 14.6 Hz), 3.85-3.87 (1H, br m), 3.94 (2H, s), 4. 42 (3H, s), 4.97 (1H, d, J = 8.0 Hz), 6.49 (1H, br s), 6.65 (1H, br s), 7.06 (1H, s), 7.22 (1H, br s), 7.40-7.45 (4H, m). |
| | | MS(ESI/APCI) m/z: 624 [M + H]⁺ |
| 45a | 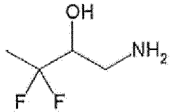 | ¹H-NMR (CDCl₃) δ: 1. 65 (3H, t, J = 19. 3 Hz), 1.90-2.45 (3H, br m), 2.88-3.02 (2H, m), 3.55-3.63 (1H, m). |

**[Table 38]**

| **Example No.** | **structural formula** | **physicochemical data** |
|---|---|---|
| 45b | 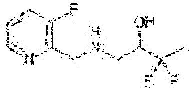 | ¹H-NMR (CDCl₃) δ : 1.40-1. 95 (4H, m), 2. 85-2. 97 (2H, m), 3.70-3.82 (1H, m), 4.00-4.09 (2H, m), 4.40 (1H, br s), 7.21-7.27 (1H, m), 7.35-7.42 (1H, m), 8.36-8.41 (1H, m). |
| 45c | 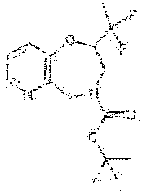 | ¹H-NMR (DMSO-D₆) δ : 1. 31 (9H, s), 1. 79 (3H, t, J = 19. 5 Hz), 3. 69-3. 78 (1H, m), 3. 92-4. 00 (1H, m), 4. 20-4. 31 (1H, m), 4.55 (1H, d, J = 16. 1 Hz), 4.79 (1H, d, J = 16.1 Hz), 7.24 (1H, dd, J = 8. 1, 4. 9 Hz), 7. 39-7. 44 (1H, m), 8. 20-8. 24 (1H, m). |
| | | MS(ESI) m/z: 337 [M + Na]⁺ |
| 45d | 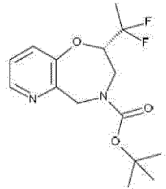 | ¹H-NMR (CDCl₃) δ : 1. 39 (9H, s), 1.81 (3H, t, J = 19. 1 Hz), 3. 48-3. 56 (1H, m), 3. 90-4. 00 (1H, m), 4. 34 (1H, d, J = 12.8 Hz), 4.47 (1H, d, J = 15.2 Hz), 4. 88-5. 06 (1H, m), 7. 18 (1H, dd, J = 7.9, 4.9 Hz), 7.37 (1H, d, J = 7.9 Hz), 8.31 (1H, s). |
| | | MS(APCI) m/z: 315 [M + H]⁺ |
| 45e | 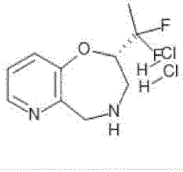 | ¹H-NMR (DMSO-D₆) δ : 1.85 (3H, t, J = 19. 7 Hz), 3.51-3.59 (1H, m), 3. 69 (1H, d, J = 15. 2 Hz), 4. 52 (2H, br s), 4. 57-4. 65 (1H, m), 7.46 (1H, dd, J = 8.2, 4.6 Hz), 7.66 (1H, dd, J = 7.9, 1.2 Hz), 8.38 (1H, dd, J = 4.9, 1.2 Hz), 10.12 (1H, br s), 10.22 (1H, br s). |
| | | MS(APCI) m/z: 215 [M + H]' |
| 45f | 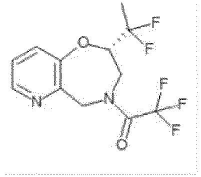 | ¹H-NMR (CDCl₃) δ : 1. 79-1. 91 (3H, m), 3. 70 (0. 5H, dd, J = 14.1, 10.4 Hz), 3. 85 (0.5H, dd, J = 14. 7, 10. 4 Hz), 3. 90-4. 00 (0.5H, m), 4. 01-4. 09 (0.5H, m), 4. 39 (0.5H, d, J = 15. 3 Hz), 4. 55 (0. 5H, d, J = 15. 3 Hz), 4. 67 (0. 5H, d, J = 14. 1 Hz), 4. 80 (0.5H, d, J = 16.6 Hz), 5. 11 (0. 5H, d, J = 16.6 Hz), 5.35 (0. 5H, d, J = 15. 3 Hz), 7.22-7.29 (1H, m), 7.38-7.44 (1H, m), 8.32-8.40 (1H, m). |
| | | MS(ESI/APCI) m/z: 311 [M + H]⁺ |

**[Table 39]**

| **Example No.** | **structural formula** | **physicochemical data** |
|---|---|---|
| 45g | 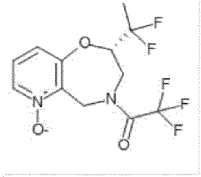 | ¹H-NMR (CDCl₃) δ : 1.83 (3H, t, J = 19.0 Hz), 3.95 (0.5H, dd, J = 14.1, 3.7 Hz), 4.05-4.17 (1H, m), 4.26-4.40 (1H, m), 4.44-4.54 (0. 5H, m), 4. 95 (0.5H, d, J = 15.9 Hz), 5.00 (0.5H, d, J = 14. 7 Hz), 5. 42 (0.5H, d, J = 17. 2 Hz), 5. 52 (0.5H, d, J = 19.0 Hz), 6.99 (1H, dd, J = 13.6, 8.0 Hz), 7.12-7.20 (1H, m), 8.07-8.12 (1H, m). |
| | | MS(ESI/APCI) m/z: 327 [M + H]⁺ |
| 45h | 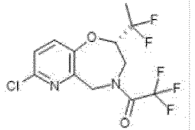 | ¹H-NMR (CDCl₃) δ : 1.77-1. 90 (3H, m), 3. 74 (0. 5H, d, J = 10.4 Hz), 3. 77 (0. 5H, d, J = 10. 4 Hz), 3.85 (0. 5H, d, J = 10.4 Hz), 3. 89 (0.5H, d, 10. 4 Hz), 3.96-4.05 (0.5H, m), 4. 05-4. 12 (0. 5H, m), 4.34 (0.5H, d, J = 15.3 Hz), 4. 58 (1H, d, J = 15. 3 Hz), 4. 77 (0.5H, d, J = 16.6 Hz), 5.04 (0.5H, d, J = 17.2 Hz), 5.23 (0.5H, d, J = 15.3 Hz), 7.23-7.28 (1H, m), 7.36 (0.5H, d, J = 6.7 Hz), 7.38 (0. 5H, d, J = 7.4 Hz). |
| | | MS(ESI/APCI) m/z: 345 [M + H]' |
| 45i | 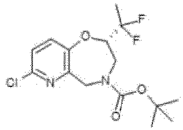 | ¹H-NMR (CDCl₃) δ : 1.40 (9H, s), 1. 77 (3H, t, J = 19.0 Hz), 3.54 (1H, t, J = 12.6 Hz), 3.96 (1H, dt, J = 23. 5, 5.5 Hz), 4.24 (1H, d, J = 14. 1 Hz), 4.44 (1H, d, J = 15.9 Hz), 4.85 (1H, d, J = 15.9 Hz), 7.15 (1H, d, J = 8.0 Hz), 7.30 (1H, d, J = 8.0 Hz). |
| | | MS(ESI/APCI) m/z: 293 [M - tBu + H]⁺ |
| 45j | 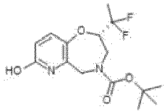 | ¹H-NMR (CDCl₃) δ : 1.43 (9H, s), 1.75 (3H, t, J = 19.0 Hz), 3.31 (1H, t, J = 11.0 Hz), 3.74 (1H, s), 4.18 (1H, d, J = 14.7 Hz), 4.37 (1H, s), 4.65 (1H, d, J = 15.9 Hz), 6.42 (1H, d, J = 9.2 Hz), 7.27 (1H, s). |
| | | MS(ESI/APCI) m/z: 275 [M - tBu + H]⁺ |
| 45k | 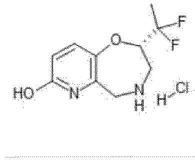 | ¹H-NMR (DMSO-D₆) δ : 1.84 (3H, t, J = 19.9 Hz), 3.49 (1H, s), 3.70 (1H, d. J = 13.5 Hz), 4.21 (1H, d, J = 15.3 Hz), 4.29-4.44 (2H, m), 6.55 (1H, d, J = 8.6 Hz), 7.48 (1H, d, J = 8.6 Hz), 9.67 (1H, s), 9.98 (1H, s). |
| | | MS(ESI/APCI) m/z: 231 [M + H]⁺ |

**[Table 40]**

| **Example No.** | **structural formula** | **physicochemical data** |
|---|---|---|
| 451 | 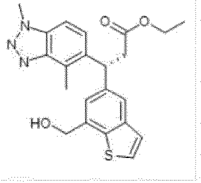 | ¹H-NMR (CDCl₃) δ : 1.10 (3H, t, J = 7. 1 Hz), 1.86 (1H, t, J = 6. 1 Hz), 2.87 (3H, s), 3.06-3.27 (2H, m), 4.02 (2H, q, J = 7. 1 Hz), 4.24 (3H, s), 4.90 (2H, d, J = 6. 1 Hz), 5.14 (1H, t, J = 8.0 Hz), 7. 18-7. 23 (1H, m), 7.28-7.34 (2H, m), 7. 38-7. 48 (2H, m), 7. 60-7. 66 (1H, m). |
| 45m | 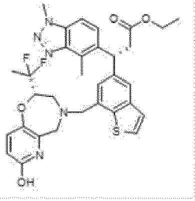 | ¹H-NMR (CDCl₃) δ : 1.10 (3H, t. J = 7. 1 Hz), 1. 70 (3H, t, J = 19. 0 Hz), 2.86 (3H, s), 3. 13 (2H, dd, J = 15. 3, 8.0 Hz), 3.26 (1H, dd, J = 15. 3, 8.0 Hz), 3.37 (1H, d, J = 15. 3 Hz), 3.81 (1H, s), 3.95-4.16 (6H, m), 4.24 (3H, s), 5.10 (1H, t, J = 8.0 Hz). 6.47 (1H, d, J = 9. 2 Hz), 7.27 (8H. d, J = 3.1 Hz), 7.35 (2H, t, J = 8.6 Hz), 7.41 (1H, d, J = 5.5 Hz), 7.48 (1H, d, J = 8.6 Hz), 7.63 (1H, s). |
| | | MS(ESI/APCI) m/z: 622 [M + H]⁺ |
| 45n=45 | 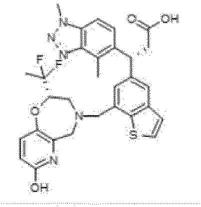 | ¹H-NMR (CDCl₃) δ : 1.82 (3H, t, J = 19.3 Hz), 2.57 (3H, s), 2.87 (1H, dd, J = 12.6, 10.1 Hz), 3.17-3.22 (2H, m), 3.54 (1H, dd, J = 14.7, 10.4 Hz), 3.69 (1H, d, J = 15.3 Hz), 3.83 (1H, d, J = 12.9 Hz), 3.98-4.08 (3H, m), 4.33 (3H, s), 4.80 (1H, dd, J = 12.9, 2.5 Hz), 6.60 (1H, d, J = 9. 2 Hz), 7. 10 (2H, d, J = 5.5 Hz). 7.19 (1H, s), 7.39 (1H, d, J = 5.5 Hz), 7.52 (2H, dd, J = 10.7, 8.9 Hz), 7.63 (1H, d, J = 8.6 Hz). |
| | | MS(ESI/APCI) m/z: 594 [M + H]⁺ |
| 46a | 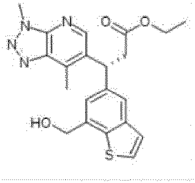 | ¹H-NMR (CDCl₃) δ : 1.12 (3H, t, J = 7.0 Hz), 2.70 (1H, t, J = 6.1 Hz), 2.83 (3H, s), 3.14-3.29 (2H, m), 4.03 (2H, q, J = 7.1 Hz), 4.28 (3H, s), 4.90 (2H, d, J = 5.5 Hz), 5.01 (1H, t, J = 7.6 Hz), 7.22 (1H, s), 7.30 (1H, d, J = 5.5 Hz), 7.45 (1H, d, J = 5.5 Hz), 7.60 (1H, s), 8.56 (1H, s). |
| | | MS(APCI) m/z: 411 [M + H]⁺ |
| 46b | 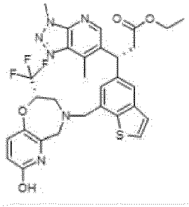 | ¹H-NMR (CDCl₃) δ : 1. 11 (3H, t, J = 7. 1 Hz), 3.02 (3H, s), 3.14-3.36 (3H, m), 3.47 (1H, d, J = 14.1 Hz), 3.56 (1H, d, J = 14. 7 Hz), 3.82 (1H, d, J = 14.7 Hz), 3.93 (1H, d, J = 13.5 Hz), 3.99-4.07 (3H, m), 4.27-4.35 (4H, m), 5. 12 (1H, t, J = 8.0 Hz), 6.55 (1H, d, J = 8.6 Hz), 7.28 (1H, d, J = 5.5 Hz), 7. 40-7. 45 (3H, m), 7.65 (1H, d, J = 1.8 Hz), 8.89 (1H, s). |
| | | MS(ESI/APCI) m/z: 627 [M + H]⁺ |

**[Table 41]**

| **Example No.** | **structural formula** | **physicochemical data** |
|---|---|---|
| 46c=46 | 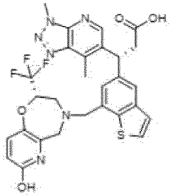 | ¹H-NMR (CDCl₃) δ : 2. 58 (3H, s), 2.96 (1H, dd, J = 12. 6, 10. 1 Hz), 3.20 (1H, d, J = 15.9 Hz), 3.27 (1H, dd, J = 10.1, 2.8 Hz), 3. 63-3. 73 (2H, m), 3.83 (1H, d, J = 12.9 Hz), 4. 02 (1H, d, J = 12.9 Hz), 4.07 (1H, d, J = 15.9 Hz), 4.28 (1H, ddd, J = 15. 2, 7. 8, 4.8 Hz), 4. 40 (3H, s), 4.78 (1H, dd, J = 12.9, 2.5 Hz), 6.62 (1H, d, J = 9.8 Hz), 7.09 (1H, s). 7.14 (1H, d, J = 5.5 Hz), 7.20 (1H, d, J = 1.8 Hz), 7.43 (1H, d, J = 5.5 Hz), 7.59 (1H, d, J = 9.8 Hz), 8.77 (1H, s). |
| | | MS(ESI/APCI) m/z: 599 [M + H]' |
| 47a | 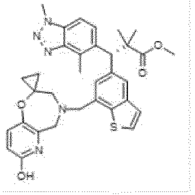 | ¹H-NMR (CDCl₃) δ : 0. 37 (2H, br s), 0.84 (2H, br s), 1. 35 (3H, s), 1.43 (3H, s), 2. 81 (3H, s), 2.95 (1H, d, J = 14.9 Hz), 3.10 (1H, d, J = 14.9 Hz), 3. 54 (3H, s), 3. 92 (2H, s), 3.95-4.05 (2H, m), 4.92 (1H, s), 6.31 (1H, d, J = 9.4 Hz), 7.04 (1H, s), 7.08 (1H, d, J = 9. 4 Hz), 7.25 (1H, s), 7.32 (1H, d, J = 8.6 Hz), 7.40 (1H, d, J = 5.5 Hz), 7.62-7.66 (2H, m). |
| | | MS(APCI) m/z: 598 [M + H]⁺ |
| 47b=47 | 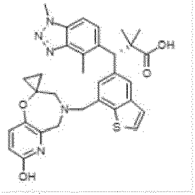 | ¹H-NMR (CD₃OD) δ : 0.47 (2H, br s), 0.86 (2H, br s), 1.33 (3H, s), 1. 42 (3H, s), 2.74 (3H, s), 3. 11 (1H, br s), 3. 13 (1H, br s), 3.88 (2H, br s), 3.92 (2H, br s), 4.29 (3H, s), 5.01 (1H, s), 6.41 (1H, d, J = 9.4 Hz), 7.05 (1H, s), 7.30 (1H, d, J = 1.6 Hz), 7.32 (1H, d, J = 2.3 Hz), 7. 51 (1H, d, J = 5. 5 Hz), 7.56 (1H, d, J = 9. 0 Hz), 7. 78 (1H, s), 7.90 (1H, s). |
| | | MS(APCI) m/z: 584 [M + H]⁺ |
| 48a | 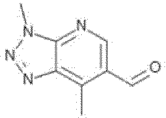 | ¹H-NMR (CDCl₃) δ : 3. 19 (3H, s), 4.39 (3H, s), 9.07 (1H, s), 10.51 (1H, s). |
| | | MS(APCI) m/z: 177 [M + H]⁺ |
| 48b | 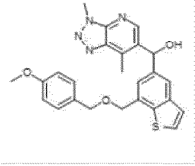 | ¹H-NMR (CDCl₃) δ : 2. 73 (3H, s), 2.87 (1H, br s), 3.80 (3H, s), 4.33 (3H, s), 4.49 (2H, s), 4.73 (2H, s), 6.32 (1H, d, J = 3.1 Hz), 6.86 (2H, d, J = 8.6 Hz). 7.26 (2H, d. J = 6.7 Hz), 7.31 (1H, d, J = 5.5 Hz), 7.48 (1H, d, J = 5.5 Hz), 7. 72 (1H, s), 8.86 (1H, s). |
| | | MS(APCI) m/z: 461 [M + H]⁺ |

**[Table 42]**

| **Example No.** | **structural formula** | **physicochemical data** |
|---|---|---|
| 48c | 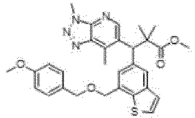 | ¹H-NMR (CDCl₃) δ : 1.38 (3H, s), 1.46 (3H, s), 2.83 (3H, s), 3.52 (3H, s), 3.81 (3H, s), 4.32 (3H, s), 4.46 (2H, s), 4.70 (2H, s), 4.97 (1H, s), 6.86 (2H, d, J = 8.6 Hz), 7.11 (1H, s), 7.24 (2H, d, J = 8.6 Hz), 7.30 (1H, d, J = 5.5 Hz), 7.46 (1H, d, J = 5.5 Hz), 7.67 (1H, s), 8.85 (1H, s). |
| | | MS(APCI) m/z: 545 [M + H]' |
| 48d | 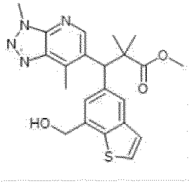 | ¹H-NMR (CDCl₃) δ : 1.37 (3H, s), 1.46 (3H, s), 2.22 (1H, br s), 2.82 (3H, s), 3.53 (3H, s), 4.30 (3H, s), 4.90 (2H, d, J = 3.9 Hz), 4.97 (1H, s), 7.19 (1H, s), 7.31 (1H, d, J = 5.5 Hz), 7.45 (1H, d, J = 5.5 Hz), 7.68 (1H, s), 8.85 (1H, s). |
| | | MS(APCI) m/z: 425 [M + H]⁺ |
| 48f | 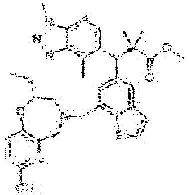 | ¹H-NMR (CDCl₃) δ : 0.97 (3H, t, J = 7. 4 Hz), 1. 37 (4H, s), 1.46 (3H, s), 1.50-1.66 (2H, m), 2.87 (3H, s), 2.91-3.01 (2H, m), 3. 53 (3H, s), 3. 65 (1H, d, J = 15. 3 Hz), 3. 74-3. 82 (1H, m), 3.86 (1H, d, J = 14. 1 Hz), 3.91 (1H, d, J = 13.7 Hz), 3.98 (1H, d, J = 15. 3 Hz), 4. 32 (3H, s), 4.93 (1H, s), 6.37 (1H, d, J = 9. 4 Hz), 7. 21 (1H, s), 7. 24-7. 30 (2H, m), 7.42 (1H, d, J = 5.5 Hz), 7.63 (1H, s), 8.92 (1H, s). |
| | | MS(APCI) m/z: 601 [M + H]⁺ |
| 48g=48 | 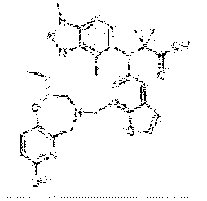 | ¹H-NMR (CDCl₃) δ : 1.12 (3H, t, J = 7.2 Hz), 1.35 (3H, s), 1.47 (3H, s), 1.48-1.58 (1H, m), 1.61-1.71 (1H, m), 2.80 (3H, s), 3.16 (1H, d, J = 15.3 Hz), 3.25-3.39 (2H, m), 3.64-3.78 (3H, m), 3. 96 (1H, d, J = 15. 3 Hz), 4. 32 (3H, s), 4.41 (1H, s), 6.59 (1H, d, J = 9.4 Hz), 6.80 (1H, s), 7.38 (1H, d, J = 5.5 Hz), 7.46-7.50 (2H, m), 7.82 (1H, d, J = 1.6 Hz), 9.55 (1H, s). |
| | | MS(APCI) m/z: 587 [M + H]⁺ |
| 49b | 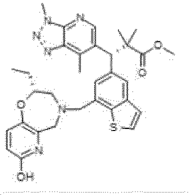 | ¹H-NMR (CDCl₃) δ : 1.07 (3H, t, J = 7.2 Hz), 1.37 (3H, s), 1.40 (3H, s), 1.51-1.70 (2H, m), 2.96 (3H, s), 2.97-3.05 (1H, m), 3.08-3.16 (1H, m), 3.44-3.51 (4H, m), 3.76-3.88 (3H, m), 3.97 (1H, d, J = 13.7 Hz), 4.33 (3H, s), 4.92 (1H, s), 6.48 (1H, d, J = 9.0 Hz), 7.25-7.32 (2H, m), 7.39 (1H, s), 7.42 (1H, d, J = 5.5 Hz), 7.64 (1H, d, J = 6.7 Hz), 9.04 (1H, s). |
| | | MS(APCI) m/z: 601 [M + H]⁺ |

**[Table 43]**

| **Example No.** | **structural formula** | **physicochemical data** |
|---|---|---|
| 49c=49 | 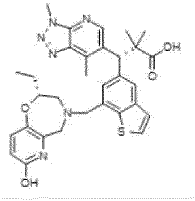 | ¹H-NMR (CDCl₃) δ : 1.15 (3H, t, J = 7. 4 Hz), 1.25 (3H, s), 1.32 (3H, s), 1.51-1.76 (2H, m), 2.62 (3H, s), 3.20 (1H, d, J = 15.7 Hz), 3.39 (2H, d, J = 5.5 Hz), 3.76-3.91 (4H, m), 4.07 (1H, d, J = 15.7 Hz), 4.37 (3H, s), 4.97 (1H, s), 6.60 (1H, d, J = 9.4 Hz), 7.01 (1H, s), 7.23 (1H, d, J = 5.5 Hz), 7.42 (1H, d, J = 5.5 Hz), 7.52 (1H, d, J = 9.4 Hz), 7.57 (1H, s), 9.30 (1H, s). |
| | | MS(APCI) m/z: 587 [M + H]⁺ |
| 50a | 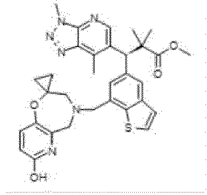 | ¹H-NMR (CDCl₃) δ : 0. 45-0. 54 (2H, m), 0.85-0. 96 (2H, m), 1.37 (3H, s), 1. 43 (3H, s), 2.90 (3H, s), 2. 97 (1H, d, J = 14. 1 Hz), 3. 31 (1H, d, J = 13. 3 Hz), 3. 51 (3H, s), 3. 78-4. 00 (4H, m), 4.32 (3H, s), 4.91 (1H, s), 6.39 (1H, d, J = 9.0 Hz), 7.09 (1H, d, J = 9. 4 Hz), 7. 21-7. 27 (2H, m), 7. 42 (1H, d, J = 5. 5 Hz), 7.63 (1H, d, J = 1.6 Hz), 8.98 (1H, s). |
| | | MS(APCI) m/z: 599 [M + H]⁺ |
| 50b=50 | 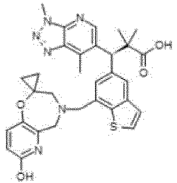 | ¹H-NMR (CD₃OD) δ : 0.48 (2H, br s), 0.87 (2H, t, J = 5.9 Hz), 1.37 (3H, s), 1.43 (3H, s), 2.78 (3H, s), 3.05-3.20 (2H, m), 3.82-3.99 (4H, m), 4.31 (3H, s), 4.98 (1H, s), 6.40 (1H, d, J = 9.4 Hz). 7.07 (1H, s), 7.29-7.37 (2H, m), 7.53 (1H, d, J = 5.5 Hz), 7.80 (1H, s), 9.02 (1H, d, J = 3.9 Hz). |
| | | MS(APCI) m/z: 585 [M + H]⁺ |
| 51a | 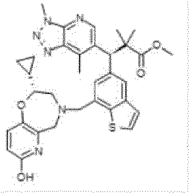 | MS(APCI) m/z: 613 [M + H]⁺ |
| 51c=51 | 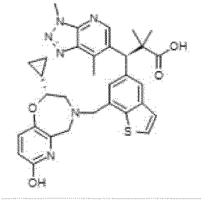 | ¹H-NMR (CDCl₃) δ : 0.27-0.36 (1H, m), 0.49-0.56 (1H, m), 0. 60-0. 75 (2H, m), 0. 97-1. 05 (1H, m), 1. 34 (3H, s), 1. 46 (3H, s), 2.80 (3H, s), 3.14-3.21 (2H, m), 3.48 (2H. d, J = 5.9 Hz), 3. 64 (1H, d, J = 12.9 Hz), 3. 73 (1H, d, J = 13.3 Hz), 3.95 (1H, d, J = 15.3 Hz), 4. 31 (3H, s), 4.41 (1H, s), 6.57 (1H, d, J = 9.4 Hz), 6.78 (1H, s), 7.38 (1H, d, J = 5. 5 Hz), 7.47 (1H, d, J = 1. 2 Hz), 7.49 (1H, d, J = 2. 7 Hz), 7.82 (1H, d, J = 1.2 Hz), 9.52 (1H, s). |
| | | MS(APCI) m/z: 599 [M + H]⁺ |

**[Table 44]**

| **Example No.** | **structural formula** | **physicochemical data** |
|---|---|---|
| 52a | 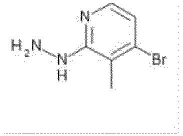 | ¹H-NMR (DMSO-D₆) δ : 2.12-2.14 (3H, m), 4.21 (2H, br s), 6.80-6.84 (1H, m), 7.54 (1H, br s), 7.76-7.82 (1H, m). |
| 52b | 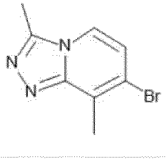 | ¹H-NMR (CDCl₃) δ : 2.70-2.76 (6H, m), 6.97 (1H, d, J = 7.1 Hz), 7.58 (1H, d, J = 7.1 Hz). |
| 52c | 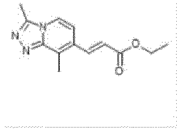 | ¹H-NMR (CDCl₃) δ : 1. 36 (3H, t, J = 7.1 Hz), 2. 76 (3H, s), 2.80 (3H, s), 4.30 (2H, q, J = 7.1 Hz), 6.44 (1H, d, J = 15.9 Hz), 7.00 (1H, d, J = 7.3 Hz), 7.70 (1H, d, J = 7.3 Hz), 8.00 (1H, d, J = 15.9 Hz). |
| 52d | 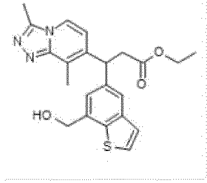 | ¹H-NMR (CDCl₃) δ : 1.15 (3H, t, J = 7.1 Hz), 2.30 (1H, t, J = 6.1 Hz), 2.67 (3H. s), 2. 78 (3H, s), 3. 00-3. 09 (1H, m), 3.16-3.26 (1H, m), 4.06 (2H, q, J = 7.1 Hz), 4.93 (2H, d, J = 6.1 Hz), 5.04-5.11 (1H, m), 6.71 (1H, d, J = 7.3 Hz), 7.19-7.24 (1H, m), 7.33 (1H, d, J = 5.6 Hz), 7.47 (1H, d, J = 5.6 Hz), 7.60-7.67 (2H, m). |
| 52e | 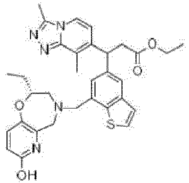 | ¹H-NMR (CDCl₃) δ : 0. 90-1. 02 (3H, m), 1. 14 (3H, t, J = 7. 1 Hz), 1.29-1.43 (1H, m), 1. 48-1. 63 (1H, m), 2. 66 (1. 5H, s), 2. 68 (1. 5H, s), 2. 78 (1. 5H, s), 2. 79 (1. 5H, s), 2.82-3.09 (3H, m), 3.16-3.35 (1H, m), 3.57-3.70 (1H, m), 3.71-4.10 (6H, m), 4.98-5.09 (1H, m), 6.29-6.36 (1H, m), 6.68-6.79 (1H, m), 7.00-7.09 (1H, m), 7.25-7.31 (2H, m), 7.39-7.45 (1H, m), 7.58-7.64 (1H, m), 7.65-7.73 (1H, m). |

**[Table 45]**

| **Example No.** | **structural formula** | **physicochemical data** |
|---|---|---|
| 52f=52 | 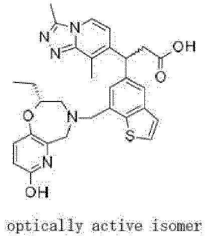 | ¹H-NMR (DMSO-D₆) δ : 0.88 (3H, t, J = 7.6Hz), 1. 21-1. 35 (1H, m). 1. 38-1. 51 (1H, m), 2.61 (3H, s), 2.65 (3H, s), 2. 68-2. 85 (2H, m), 3.07-3.22 (2H, m), 3.65-3.95 (5H, m). 4. 88 (1H, t, J = 8.0 Hz), 6.24 (1H, d, J = 9.5 Hz), 6.95 (1H, d, J = 7. 1 Hz), 7. 20-7. 33 (2H, m), 7. 39 (1H, d, J = 5. 4 Hz), 7.68 (1H, d, J = 5.4 Hz), 7.78-7.85 (1H, m), 8.09 (1H, d, J = 7. 1 Hz). |
| | | MS(ESI) m/z: 556 [M - H]⁻, 558 [M + H]', 580 [M + Na]' |
| 53a=63 | 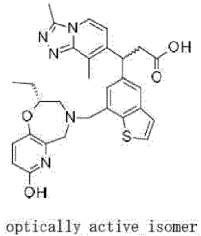 | ¹H-NMR (DMSO-D₆) δ : 0.80 (3H, t, J = 7. 3 Hz), 1.14-1. 32 (1H, m), 1. 34-1. 48 (1H, m), 2.60 (3H, s), 2.66 (3H, s), 2.68-2.84 (2H, m), 3. 04-3. 21 (2H, m), 3.63-3.95 (5H, m), 4.89 (1H, t, J = 8.3 Hz), 6.23 (1H, d, J = 9.3 Hz), 6.92 (1H, d, J = 7. 1 Hz), 7. 19-7. 33 (2H, m), 7.39 (1H, d, J = 5. 6 Hz), 7. 68 (1H, d, J = 5.6 Hz), 7.78-7.85 (1H, m), 8.08 (1H, d, J = 7.3 Hz). |
| | | MS(ESI) m/z: 556 [M - H]⁻, 558 [M + H]', 580 [M + Na]⁺ |
| 54a | 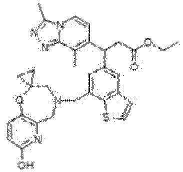 | ¹H-NMR (CDCl₃) δ : 0.40-0. 50 (2H, m), 0.84-0. 95 (2H, m), 1. 14 (3H, t, J = 7.3 Hz), 2.67 (3H, s), 2. 78 (3H, s), 2.95-3.13 (3H, m), 3.20-3.29 (1H, m), 3.86-4.07 (6H, m), 5.01 (1H, t, J = 7.6 Hz), 6. 29 (1H, d, J = 9. 5 Hz), 6.72 (1H, d, J = 7. 1 Hz). 6.98-7.03 (1H, m), 7.09 (1H, d, J = 9.5 Hz), 7. 20-7. 28 (1H, m), 7. 43 (1H, d, J = 5.4 Hz), 7.57-7.62 (1H, m), 7.67 (1H, d, J = 7.1 Hz). |
| 54b=54 | 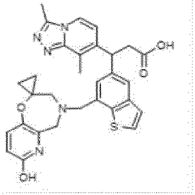 | ¹H-NMR (DMSO-D₆) δ : 0. 38-0. 50 (2H, m), 0. 72-0.83 (2H, m), 2.61 (3H, s), 2.65 (3H, s), 2.83-2.92 (1H, m), 2.97-3.06 (1H, m), 3.07-3.21 (2H, m), 3.81-3.98 (4H, m), 4.88 (1H, t, J = 8.0 Hz), 6.22 (1H, d, J = 9.0 Hz), 6. 93 (1H, d, J = 7.1 Hz), 7. 12 (1H, d, J = 9. 0 Hz), 7.15-7.20 (1H, m), 7.38 (1H, d, J = 5. 4 Hz), 7.68 (1H, d, J = 5.4 Hz), 7.75-7.80 (1H, m), 8.08 (1H, d, J = 7. 1 Hz), 11.27-12.07 (2H, br s). |
| | | MS(ESI) m/z: 554 [M - H]⁻, 556 [M + H]⁺, 578 [M + Na]⁺ |
| 55a | 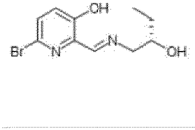 | ¹H-NMR (DMSO-d6) δ : 0.88-0.97 (3H, m), 1.36-1.54 (2H, m), 3.46-3.56 (1H, m), 3.58-3.67 (1H, m), 3.71-3.81 (1H, m), 4.86-4.97 (1H, m), 7.15-7.23 (1H, m), 7.46-7.49 (1H, m), 8.47 (1 H, s), 13.95 (1 H, br s). |

**[Table 46]**

| **Example No.** | **structural formula** | **physicochemical data** |
|---|---|---|
| 55b | 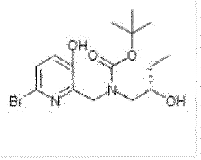 | ¹H-NMR (CDCl₃) δ : 0.99-1. 07 (3H, m), 1. 28-1. 35 (2H, m), 1.49-1.54 (1H, m), 1.58 (9H, s), 3.25-3.40 (1H, m), 3.41-3.52 (1H, m), 3.71-3.93 (1H, m), 4.36-4.59 (2H, m), 4.94-5.27 (1H, m), 7.36-7.51 (2H, m). |
| 55c | 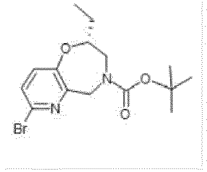 | ¹H-NMR (DMS0-d6) δ : 1. 00 (3H, t, J=6.8 Hz), 1. 23-1. 40 (9H, m), 1. 52-1. 65 (2H, m), 3. 53-3. 70 (2H, m), 3.92-4.12 (1H, m), 4.44-4.69 (2H, m), 7.34 (1H, d, J=8.4 Hz), 7.45 (1H, d, J=8.4 Hz). |
| | | MS(ESI/APCI) m/z: 357 [M + H]⁺ |
| 55d | 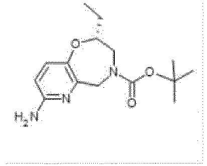 | MS(ESI/APCI) m/z: 294 [M + H]⁺ |
| 55e | 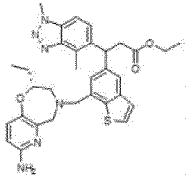 | MS(ESI/APCI) m/z: 585 [M + H]⁺ |
| 55f=55 | 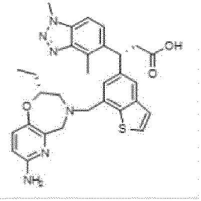 | ¹H-NMR (CDCl₃) δ : 1. 15 (3H, t, J = 7. 2 Hz), 1. 57-1. 60 (1H, m), 1.69-1.73 (1H, m), 2.62 (3H, s), 2.90-2.97 (2H, m), 3.31 (1H, dd, J = 14.7, 9.6 Hz), 3.39 (1H, d, J = 14.1 Hz), 3.58 (1H, d, J = 15.3 Hz), 3. 73-3. 79 (1H, m), 3.86-3.92 (3H, m), 4.33 (3H, s), 4.94 (1H, dd, J = 11.7, 3.1 Hz), 6.48 (1H, d, J = 9. 0 Hz), 7.04 (1H, s), 7.08 (1H, d, J = 5.5 Hz), 7.33 (2H, d, J = 8.6 Hz), 7.37 (1H, d, J = 9.0 Hz), 7.48 (1H, d, J = 8.2 Hz), 7.59 (1H, d, J = 8.6 Hz). |
| | | MS(ESI/APCI) m/z: 557 [M + H]⁺ |

**[Table 47]**

| **Example No.** | **structural formula** | **physicochemical data** |
|---|---|---|
| 56a=56 | 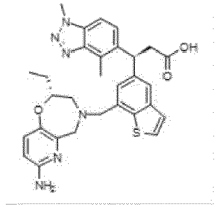 | ¹H-NMR (CDCl₃) δ : 1.05 (3H, t, J = 7.4 Hz), 1.51-1.54 (1H, m), 1.65-1.71 (1H, m), 2.76 (3H, s), 2.97-3.04 (2H, m), 3.12 (1H, d, J = 10.2 Hz), 3.19 (1H, d, J = 13.7 Hz), 3.68 (1H, d, J = 14.1 Hz), 3.75-3.78 (2H, m), 3.90 (1H, d, J = 13.3 Hz), 3.96 (1H, d, J = 14.5 Hz), 4.26 (3H, s), 4.99 (1H, dd, J = 10.4, 5.3 Hz), 6.34 (1H, d, J = 8.6 Hz), 7.22 (2H, dd, J = 7.6, 6.1 Hz), 7.34-7.35 (3H, m), 7.45 (1H, s), 7.56 (1H, d, J = 8. 6 Hz). |
| | | MS(ESI/APCI) m/z: 557 [M + H]⁺ |
| 57a | 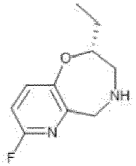 | MS(ESI/APCI) m/z: 197 [M + H]⁺ |
| 57b | 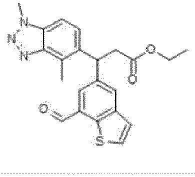 | MS(ESI/APCI) m/z: 408 [M + H]⁺ |
| 57c | 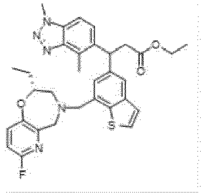 | MS(ESI/APCI) m/z: 588 [M + H]* |
| 57d=57 | 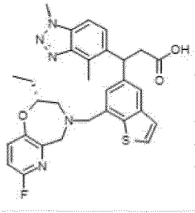 | ¹H-NMR (CDCl₃) δ : 1.00-1.06 (3H, m), 1.46-1.54 (1H, m), 1. 63-1. 71 (1H, m), 2. 69 (1. 5H, s), 2. 79 (1. 5H, s), 2. 95-3. 25 (4H, m), 3.79-3.88 (5H, m), 4.23 (1.5H, s). 4. 26 (1. 5H. s), 4. 97-5. 05 (1H, m), 6. 74 (0. 5H, d, J = 7. 8 Hz), 6. 80 (0. 5H, d, J = 8. 3 Hz), 7. 14 (0. 5H, d, J = 6. 3 Hz), 7. 20 (0. 5H. d, J = 5. 9 Hz), 7. 28-7. 30 (2H, m), 7. 34-7. 40 (2H, m), 7. 46-7. 51 (2H, m). |
| | | MS(ESI/APCI) m/z: 560 [M + H]⁺ |

**[Table 48]**

| **Example No.** | **structural formula** | **physicochemical data** |
|---|---|---|
| 58a | 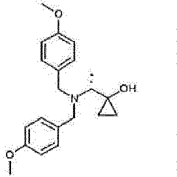 . | ¹H-NMR (CDCl₃) δ : 0. 31-0. 40 (1H, m), 0. 63-0. 73 (3H, m), 0. 82 (3H, d, J = 6. 8 Hz), 3.24-3.31 (3H, m), 3. 79 (6H, s), 3.91 (2H, d, J = 13.2 Hz), 4.28 (1H, br s), 6.82-6.87 (4H, m), 7. 16-7. 21 (4H, m). |
| 58b | 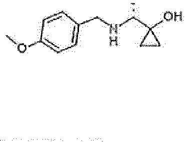 | ¹H-NMR (CDCl₃) δ : 0.29-0.36 (1H, m), 0.45-0.50 (1H, m), 0.71-0. 86 (2H, m), 1.17 (3H, d, J = 6.6 Hz), 2.32 (1H, q, J = 6.6 Hz), 3.76 (1H, d, J = 13.2 Hz), 3.80 (3H, s), 3.87 (1H, d, J = 13.2 Hz), 6.84-6.89 (2H, m), 7. 21-7. 26 (2H, m). |
| 58c | 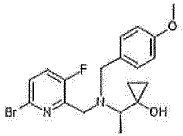 | ¹H-NMR (CDCl₃) δ : 0.36-0.43 (1H, m), 0.61-0.78 (3H, m), 0.90 (3H, d, J = 6.8 Hz), 3.33 (1H, q, J = 6.8 Hz), 3.63 (1H, d, J = 13.2 Hz), 3.69-3.76 (4H, m), 3. 86 (1H, d, J = 13.2 Hz), 4.06-4.12 (1H, m), 5.28 (1H, s), 6.71-6.76 (2H, m), 7.08 (1H, t, J = 8.8 Hz), 7.14-7.19 (2H, m), 7.23-7.28 (1H, m). |
| 68d | 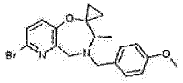 | ¹H-NMR (CDCl₃) δ : 0.46-0. 52 (1H, m), 0.58-0.64 (1H, m), 0.83-0.94 (2H, m), 1.38 (3H, d, J = 6.6 Hz), 2.58 (1H, q, J = 6.6 Hz), 3.74 (2H, s), 3.79 (3H, s), 3.99 (1H, d, J = 15.8 Hz), 4.45 (1H, d, J = 15.8 Hz), 6.81-6.86 (2H, m), 6.98 (1H, d, J = 8.3 Hz), 1.16-7.21 (2H, m), 7.24 (1H, d, J = 8.3 Hz). |
| 58e | 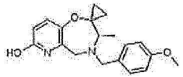 | ¹H-NMR (CDCl₃) δ : 0.32-0.42 (1H, m), 0. 68-0. 67 (1H, m), 0.81-0.99 (2H, m), 1.37 (3H, d, J = 7.1 Hz), 2.55 (IH, q, J = 7.1 Hz), 3. 57-3. 66 (1H, m), 3. 75-3. 84 (5H, m), 4. 39-4. 47 (1H, m), 6. 28 (1H, d, J = 9.5 Hz), 6.77-6.86 (2H, m), 7.09 (1H, d, J = 9. 5 Hz), 7.16-7.23 (2H, m), 12. 03-12. 49 (1H, br s). |

**[Table 49]**

| **Example No.** | **structural formula** | **physicochemical data** |
|---|---|---|
| 58f | 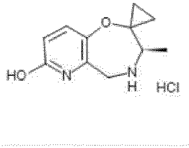 | ¹H-NMR (DMSO-D₆) δ : 0. 84-1. 08 (4H, m), 1. 44 (3H, d, J = 6. 8 Hz), 3.47-3.55 (1H, m), 4.24-4.41 (2H, m), 6.50 (1H, d, J = 9.0 Hz), 7.23 (1H, d, J = 9.0 Hz), 9.50-10.00 (2H, br m). |
| 58g | 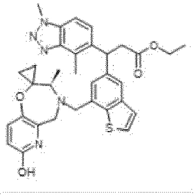 | ¹H-NMR (CDCl₃) δ : 0.24-0.38 (1H, m), 0.50-0.63 (1H, m), 0. 74-0.96 (2H, m), 1. 06-1. 13 (3H, m), 1. 35-1. 44 (3H, m), 2.48-2.58 (1H, m), 2.86 (3H, s), 3.04-3.58 (3H, m), 3. 95-4. 06 (4H, m), 4. 22 (1. 5H, s), 4. 24 (1. 5H, s), 4. 37-4. 45 (1H, m), 4.98-5.12 (1H, m), 6.28-6.35 (1H, m), 6.98-7.02 (0. 5H, m), 7.04-7.08 (1H, m), 7.08-7.11 (0.5H, m), 7.21-7.35 (2H, m), 7.39-7.49 (2H, m), 7.55-7.64 (1H, m). |
| 58h=58 | 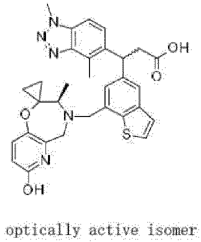 | ¹H-NMR (DMSO-D₆) *δ* : 0. 29-0. 37 (1H, m), 0. 55-0. 65 (1H, m), 0.67-0.75 (1H, m), 0. 80-0. 90 (1H, m), 1.31 (3H, d, J = 6.8 Hz), 2.37 (1H, q, J = 6.8 Hz), 2.76 (3H, s), 3.07-3.20 (2H, m), 3.56 (1H, d, J = 15.6 Hz), 3.86 (1H, d, J = 14.4 Hz), 4.02 (1H. d, J = 14.4 Hz), 4.23 (3H, s), 4.33 (1H, d, J = 15.6 Hz), 4.94 (1H, t, J = 8.1 Hz), 6. 21 (1H, d, J = 9.0 Hz), 7. 10-7. 17 (2H, m), 7. 35 (1H, d, J = 5. 4 Hz), 7. 53-7. 59 (2H, m), 7.66 (1H, d, J = 5. 4 Hz), 7. 70 (1H, s), 11. 64 (2H, br s). |
| | | MS(ESI) m/z: 568 [M - H]⁻, 592 [M + Na]⁺ |
| 59a=59 | 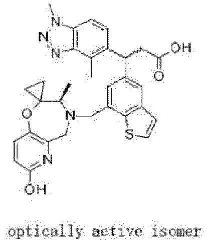 | ¹H-NMR (DMSO-D₆) δ : 0.27-0.37 (1H, m), 0.50-0.60 (1H, m), 0.66-0.74 (1H, m), 0.75-0.89 (1H, m), 1.33 (3H, d, J = 7.0 Hz), 2.36 (1H, q, J = 7.0 Hz), 2.77 (3H, s), 3.04-3.12 (1H, m), 3.13-3.21 (1H. m), 3.53 (1H, d. J = 15.9 Hz), 3.86 (1H, d, J = 14.2 Hz), 4.00 (1H, d, J = 14.2 Hz), 4.23 (3H, s), 4.35 (1H. d, J = 15.9 Hz), 4.95 (1H, t, J = 7.6 Hz), 6.21 (1H, d, J = 9.3 Hz), 7.09-7.15 (2H, m), 7.35 (1H, d, J = 5. 4 Hz), 7. 49 (1H, d, J = 8.6 Hz), 7.56 (1H, d, J = 8. 6 Hz), 7.64-7.70 (2H, m), 11.69 (2H, br s). |
| | | MS(ESI) m/z: 568 [M - H]⁻, 570 [M + H]⁻ , 592 [M + Na]⁺ |

**[Table 50]**

| **Example No.** | **structural formula** | **physicochemical data** |
|---|---|---|
| 60a | 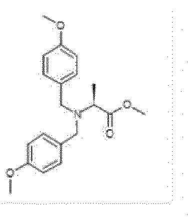 | ¹H-NMR (CDCl₃) δ : 1. 30 (3H, d, J = 7. 1 Hz), 3.45-3.55 (3H, m), 3.71-3.76 (5H, m), 3.79 (6H, s), 6.82-6.86 (4H, m), 7.24-7.29 (4H, m). |
| 60b | 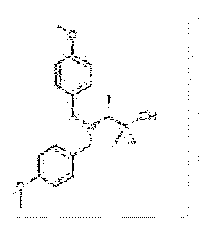 | ¹H-NMR (CDCl₃) δ : 0.31-0.40 (1H, m), 0.63-0.73 (3H, m), 0.82 (3H, d, J = 6. 8 Hz), 3. 24-3. 31 (3H, m), 3.79 (6H, s), 3.91 (2H, d, J = 13.2 Hz), 4.29 (1H, br s), 6.82-6.87 (4H, m), 7. 16-7. 21 (4H, m). |
| 60c | 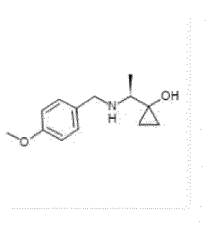 | ¹H-NMR (CDCl₃) δ : 0. 29-0. 36 (1H, m), 0. 45-0. 50 (1H, m), 0.71-0.85 (2H, m), 1.17 (3H, d, J = 6.6 Hz), 2.32 (1H, q, J = 6. 6 Hz), 3. 76 (1H, d, J = 13.2 Hz), 3. 80 (3H, s), 3.87 (1H, d, J = 13. 2 Hz), 6. 84-6. 89 (2H, m), 7. 21-7. 25 (2H, m). |
| 60d | 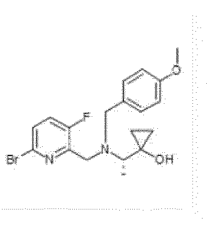 | ¹H-NMR (CDCl₃) δ : 0.36-0.43 (1H, m), 0.61-0.78 (3H, m), 0.90 (3H, d, J = 6.8 Hz), 3.33 (1H, q, J = 6.8 Hz), 3.63 (1H, d, J = 13.2 Hz), 3. 69-3. 76 (4H, m), 3. 85 (1H, d, J = 13.2 Hz), 4.06-4.12 (1H, m), 5.28 (1H, s), 6.71-6.76 (2H, m), 7.08 (1H, t, J = 8.8 Hz), 7. 14-7. 19 (2H, m), 7. 23-7. 28 (1H, m). |
| 60e | 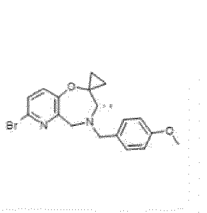 | ¹H-NMR (CDCl₃) δ : 0.46-0.52 (1H, m), 0.58-0. 64 (1H, m), 0.83-0.94 (2H, m), 1.38 (3H, d, J = 6.6 Hz), 2.58 (1H. q, J = 6.6 Hz), 3.74 (2H, s), 3.79 (3H, s), 3.99 (1H, d, J = 15. 8 Hz), 4. 45 (1H, d, J = 15.8 Hz), 6.81-6.86 (2H, m), 6.98 (1H, d, J = 8.3 Hz), 7.16-7.21 (2H, m), 7.24 (1H, d, J = 8.3 Hz). |

**[Table 51]**

| **Example No.** | **structural formula** | **physicochemical data** |
|---|---|---|
| 60f | 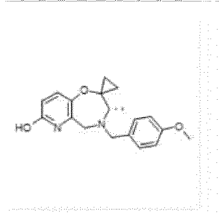 | ¹H-NMR (CDCl₃) δ : 0.34-0.41 (1H, m), 0. 58-0. 66 (1H, m). 0. 81-0. 89 (1H, m), 0. 90-0. 98 (1H, m), 1. 37 (3H, d, J = 6. 8 Hz), 2.54 (1H, q, J = 6.8 Hz), 3.62 (1H, d, J = 15.8 Hz), 3. 75-3.83 (5H, m), 4.43 (1H, d, J = 15. 8 Hz), 6. 28 (1H, d, J = 9.3 Hz), 6. 78-6. 83 (2H, m), 7.09 (1H, d, J = 9.3 Hz), 7.17-7.22 (2H, m), 12.20-12.56 (1H, br s). |
| 60g | 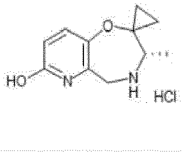 | ¹H-NE (DMSO-D₆) δ : 0. 84-1. 07 (4H, m), 1.43 (3H, d, J = 6. 8 Hz), 3.47-3.55 (1H, m), 4.22-4.42 (2H, m), 6.50 (1H, d, J = 9.0 Hz), 7.23 (1H, d, J = 9. 0 Hz), 9. 44-9.90 (3H, br m). |
| 60h | 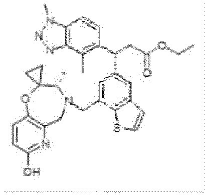 | ¹H-,NM (CDCl₃) δ : 0.28-0.40 (1H, m), 0.54-0.65 (1H. m), 0.80-0.97 (2H. m), 1.07-1.13 (3H, m), 1.37-1.46 (3H, m), 2.53-2.62 (1H, m), 2.84-2.88 (3H, m), 3.06-3.15 (1H, m), 3.22-3.50 (2H. m), 3.96-4.07 (4H, m), 4.22 (1.5H, s), 4.24 (1.5H, s), 4.33-4.42 (1H, m), 4.99-5.11 (1H, m), 6.31-6.36 (1H, m), 7.00-7.10 (2H, m), 7.22-7.52 (4H, m), 7.56-7.65 (1H, m), 10.90-11.30 (1H, br s). |
| 60i=60 | 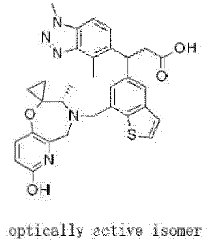 | ¹H-,NMR (DMSO-D₆) δ : 0.29-0. 37 (1H, m), 0. 55-0.65 (1H, m), 0. 67-0. 75 (1H, m), 0.80-0.90 (1H, m), 1.31 (3H, d, J = 6.8 Hz), 2.37 (1H, q, J = 6.8 Hz), 2. 76 (3H, s), 3.07-3.20 (2H, m), 3. 56 (1H. d, J = 15.6 Hz), 3.86 (1H, d, J = 14. 4 Hz), 4.02 (IH, d, J = 14.4 Hz), 4.23 (3H, s), 4.33 (1H, d, J = 15.6 Hz), 4.94 (1H, t, J = 8. 1 Hz), 6.21 (1H, d, J = 9.0 Hz), 7.10-7.17 (2H, m), 1.35 (1H, d, J = 5.4 Hz), 7.53-7.59 (2H, m), 7.66 (1H, d, J = 5. 4 Hz), 7.70 (1H, s), 11. 63 (2H, br s). |
| | | MS(ESI) m/z: 568 [M - H)⁻, 570 [M + H]⁺ , 592 [M + Na]⁺ |

**[Table 52]**

| **Example No.** | **structural formula** | **physicochemical data** |
|---|---|---|
| 61a=61 | 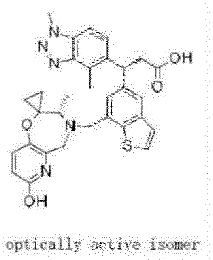 | ¹H-NMR (DMSO-D₆) *δ* : 0.27-0.37 (1H, m), 0.50-0.60 (1H, m), 0.66-0. 74 (1H, m), 0.75-0. 89 (1H, m), 1.33 (3H, d, J = 7. 0 Hz), 2. 36 (1H, q, J = 7.0 Hz), 2. 77 (3H, s). 3. 04-3. 12 (1H, m), 3. 13-3. 21 (1H, m), 3. 53 (1H, d, J = 15.9 Hz), 3. 86 (1H, d, J = 14.2 Hz), 4.00 (1H, d, J = 14.2 Hz), 4.23 (3H, s), 4.35 (1H, d, J = 15.9 Hz), 4.95 (1H, t, J = 7.6 Hz), 6.21 (1H, d, J = 9.3 Hz), 7.09-7.15 (2H, m), 7.35 (1H, d, J = 5.4 Hz), 7.49 (1H, d, J = 8.6 Hz), 7.56 (1H, d. J = 8.6 Hz), 7.64-7.70 (2H, m), 11.69 (2H, br s). |
| | | MS(ESI) m/z: 568 [M - H]⁻, 570 [M + H]⁺ , 592 [M + Na]⁺ |
| 62a | 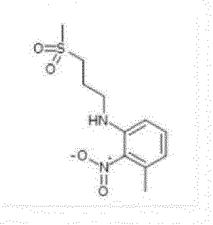 | MS(ESI/APCI) m/z: 273 [M + H]⁺ |
| 62b | 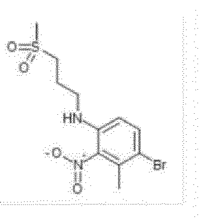 | MS(ESI/APCI) m/z: 351, 353 [M + H]⁺ |
| 62c | 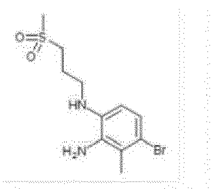 | ¹H-NMR (CDCl₃) δ : 2.18-2.25 (2H, m), 2.31 (3H, s), 2.93 (3H, s), 3.19 (2H, t, J = 7.6 Hz), 3.29 (2H, t, J = 6. 3 Hz), 3.53 (1H, br s), 6.45 (1H, d, J = 8.3 Hz), 7.00 (1H, d, J = 8.3 Hz). |
| 62d | 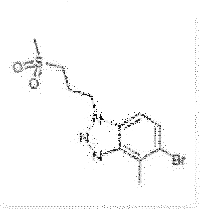 | ¹H-NMR (CDCl₃) δ : 2. 60-2. 65 (2H, m), 2. 85 (3H, s), 2. 92 (3H, s), 3.05 (2H, t, J = 7. 3 Hz), 4.83 (2H, t, J = 6. 6 Hz), 7.30 (1H, d, J = 8.8 Hz), 7.65 (1H, d, J = 8.8 Hz). |
| | | MS(ESI/APCI) m/z: 332, 334 [M + H]⁺ |

**[Table 53]**

| **Example No.** | **structural formula** | **physicochemical data** |
|---|---|---|
| 62e | 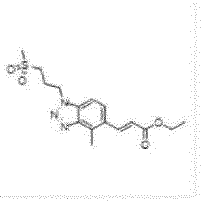 | MS(ESI/APCI) m/z: 352 [M + H]⁺ |
| 62f | 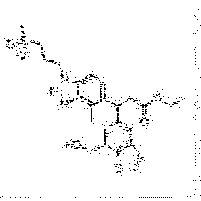 | ¹H-NMR (CDCl₃) δ : 1.12 (3H, t, J = 7.1 Hz), 2.55-2.60 (2H, m), 2.89 (3H, s), 2.89 (3H, s), 3.04 (2H, t, J = 7.1 Hz), 3.12 (1H, dd, J = 15.1, 8.3 Hz), 3.23 (1H. dd, J = 15.6, 7.3Hz) , 4.04 (2H, q, J = 7. 2 Hz), 4.77 (2H, t, J = 6. 1 Hz), 4.91 (2H, d, J = 5.9 Hz), 5.15 (1H, t, J = 8. 1 Hz), 7.23 (1H, s), 1.31-7.36 (2H, m), 7.42-7.47 (2H, m), 7.65 (1H, s). |
| 62h | 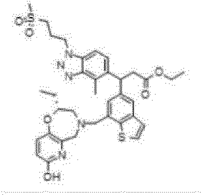 | MS(ESI/APCI) m/z: 692 [M + H]⁺ |
| 62i=62 | 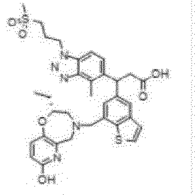 | ¹H-NMR (CDCl₃) δ : 1. 12 (1. 5H, t, J = 7. 1 Hz), 1. 16 (1.5H, t, J = 7. 3 Hz), 1.56-1.62 (1H, m), 1. 68-1. 74 (IH, m), 2. 59 (3H, s), 2.63-2.69 (1H, m), 2.76 (1H, br s). 2.85-2.90 (0. 5H, m), 2.91 (1.5H, s), 2.94 (1.5H, s), 3.01-3.08 (1.5H, m), 3.09-3.18 (2.5H, m), 3. 24-3. 26 (0. 5H, br m), 3. 37-3. 40 (1.5H, m), 3. 48-3. 52 (0. 5H, br m), 3. 69-3. 73 (0. 5H, m), 3. 84-3. 88 (1. 5H, m), 3. 94-3.97 (1.5H, m), 4. 05 (0. 5H, d, J = 15. 6 Hz), 4. 78-4. 83 (1.5H, m), 4.87 (1H, t, J = 6. 3 Hz), 4. 99 (0. 5H, d, J = 9.8 Hz). 6. 37 (0. 5H, br s), 6. 57 (0. 5H, d, J = 9.3 Hz), 7. 12-7. 15 (2.5H, m), 7. 18 (0. 5H, s), 7. 21 (0. 5H, s), 7.34-7.39 (1. 5H, m), 7.43 (0.5H. s), 7.50 (0. 5H, d, J=9.8Hz), 7.55 (0. 5H, d, J = 8. 8 Hz), 7.66 (0.5H, d, J = 8.8 Hz). |
| | | MS(ESI/APCI) m/z: 664 [M + H]⁺ |

**[Table 54]**

| **Example No.** | **structural formula** | **physicochemical data** |
|---|---|---|
| 63a | 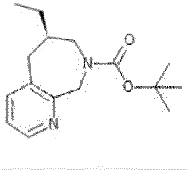 | ¹H-NMR (CDCl₃) *δ* : 0. 92-1. 02 (3H, m), 1. 17-1. 26 (1H, m), 1.30-1.42 (10H, m), 1.81 (1H, br s), 2.64-2.75 (1H, m), 2. 78-2. 93 (1H, m), 3. 19-3. 42 (1H, m), 3.56-3.88 (1H, m), 4.49-4.75 (2H, m), 7.05-7.15 (1H, m), 7.32-7.45 (1H, m), 8.30-8.42 (1H, m). |
| | | MS(ESI/APCI) m/z: 277 [M + H]⁺ |
| 63d=63 | 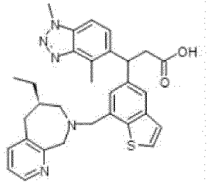 | ¹H-NMR (CDCl₃) δ : 1. 00 (1. 2H, t, J = 7. 6 Hz), 1. 04 (1. 8H, t, J = 7. 6 Hz), 1. 30-1. 45 (2H, m), 1. 80 (1H, br s), 2. 61 (1. 8H, s), 2. 74 (1. 2H, s), 2. 81-2. 89 (2H, m), 2. 92-3. 05 (2H, m), 3. 08-3. 22 (1H, m), 3. 39 (0.4H, d, J = 11.2 Hz), 3. 50 (0.6H, d, J = 13. 7 Hz), 3.65-3.79 (2H, m), 3.82-4.02 (2H, m), 4. 28 (1. 2H, s), 4. 32 (1. 8H, s), 4. 89 (0. 6H, dd, J = 12. 2, 3.4 Hz), 4. 98 (0. 4H, dd, J = 11. 2, 3.9 Hz), 7. 08-7. 24 (2H, m), 7. 25-7. 50 (4H, m), 7. 57-7. 67 (2H, m), 8. 34 (0. 4H, br s), 8. 58 (0.6H, br s). |
| 64a | 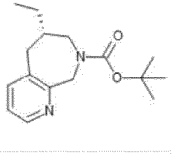 | ¹H-N)(R (CDCl₃) *δ* : 0.92-1.02 (3H, m), 1.17-1.26 (1H, m), 1.30-1.42 (100, m), 1.81 (1H, br s), 2.64-2.75 (1H, m), 2. 78-2. 93 (1H, m), 3.18-3.42 (1H, m), 3. 56-3. 88 (1H, m), 4.49-4.75 (2H, m), 7.05-7.15 (1H, m), 7.36-7.48 (1H, m), 8.30-8.42 (1H, m). |
| | | MS(ESI/APCI) m/z: 277 [M + H]⁺ |
| 64d=64 | 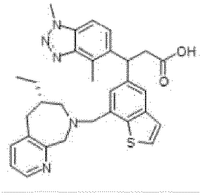 | ¹H-NMR (CDCl₃) δ : 1.00 (1. 2H, t, J = 7. 3 Hz), 1. 04 (1.8H, t, J = 7.3 Hz), 1.30-1. 45 (2H, m), 1. 81 (1H, br s), 2. 61 (1.8H, s), 2. 74 (1. 2H, s), 2.82-2.89 (2H, m), 2.92-3.04 (2H, m), 3.13-3.20 (1H, m), 3.39 (0.4H, d, J = 12.7 Hz), 3. 50 (0.6H, d, J = 12. 2 Hz), 3.65-3.79 (2H, m), 3. 82-4. 02 (2H, m), 4. 28 (1. 2H, s), 4. 32 (1. 8H, s), 4. 89 (0. 6H, dd, J = 12. 0, 4. 1 Hz), 4. 98 (0.4H. dd, J = 11. 7, 4.9 Hz), 7. 08-7. 24 (1H, m), 7. 25-7. 49 (5H, m), 7. 57-7. 66 (2H, n), 8. 34 (0. 4H, br s), 8. 58 (0.6H, br s). |
| 65b | 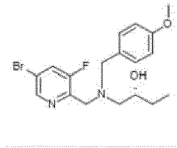 | ¹H-NMR (CDCl₃) δ : 0.94 (3H, t, J = 7.6 Hz), 1.29-1.47 (2H, m), 2.42 (1H, dd, J = 12. 7, 10. 2 Hz), 2.61 (1H, dd, J = 12. 7, 2. 7 Hz), 3. 53 (1H, d, J = 13. 4 Hz), 3. 59-3. 67 (1H, m), 3. 73 (1H, dd, J = 14.4, 1.7 Hz), 3.78 (3H, s), 3.83 (1H, d, J = 13.4 Hz), 3.92 (1H, dd, J = 14.4, 2.0 Hz), 4.09-4. 15 (1H, m), 6.79-6.83 (2H, m), 7.18-7.22 (2H, m), 7.50 (1H, dd, J = 8.8, 1.7 Hz), 8.44-8.47 (1H, m). |

**[Table 55]**

| **Example No.** | **structural formula** | **physicochemical data** |
|---|---|---|
| 65c | 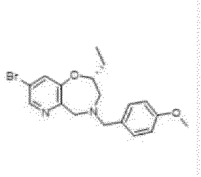 | ¹H-NMR (CDCl₃) δ : 1. 03 (3H, t, J = 7. 3 Hz), 1.42-1. 54 (1H, m). 1.60-1. 73 (1H, m), 2. 85 (1H, dd, J = 13.6, 9.5 Hz), 2.93-2.99 (1H, m), 3. 57-3. 67 (2H, m), 3. 78-3. 88 (4H, m), 3.99 (2H, s), 6.82-6.87 (2H, m), 7.17-7.22 (2H, m), 7.47 (1H, d, J = 2.0 Hz), 8.28 (1H. d, J = 2.0 Hz). |
| 65d | 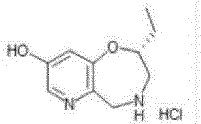 | ¹H-NMR (DMSO-D₆) δ : 1.04 (3H, t, J = 7. 3 Hz), 1.66 (2H. quintet, J = 7. 3 Hz), 3. 22-3. 33 (1H, m), 3. 47-3. 55 (1H, m), 3.94-4.07 (1H, m), 4.31-4.38 (2H, br m), 6.96 (1H, d, J = 2.4 Hz), 7.92 (1H, d, J = 2.4 Hz), 9.47-9.60 (1H, br m), 9.77-9.89 (1H, br m), 10.43-10.74 (1H, br s). |
| 65e | 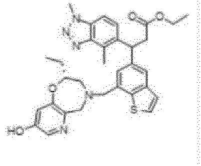 | ¹H-NMR (CDCl₃) δ : 0.92-0.98 (3H, m), 1.08 (3H, t, J = 7. 1 Hz), 1.30-1.41 (1H, m), 1.52-1.65 (1H, m), 2.85-2.97 (5H, m), 3.04-3.15 (1H, m), 3.18-3.28 (1H, m), 3.74-3.90 (3H, m), 3.92-4.08 (4H, m), 4.23 (3H, s), 5.07-5.17 (1H, m), 6.86-6.90 (1H, m), 6.94-7.03 (1H, m), 7.22-7.41 (4H, m), 7.58-7.61 (1H, m), 7.83-7.86 (1H, m). |
| 65f=65 | 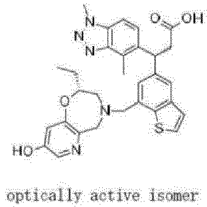 | ¹H-NMR (DMSO-D₆) δ : 0.91 (3H. t, J = 7. 3 Hz), 1. 20-1. 40 (1H, m), 1.44-1. 57 (1H, m), 2.73-2.90 (5H, m), 3.07-3.21 (2H, m), 3.71-3.89 (5H, m), 4.23 (3H. s), 4.94 (1H, t, J = 8. 1 Hz), 6.75-6.78 (1H, m), 7. 18 (1H, s), 7.36 (1H. d, J = 5.6 Hz), 7.52-7. 58 (2H, m), 7.64 (1H, d, J = 5.6 Hz), 7.69-7.71 (1H, m), 7.72-7.76 (1H, m), 9.89 (1H, br s), 11.90-12.40 (1H, br s). |
| | | MS(ESI) m/z: 556 [M - H]⁻, 558 [M + H]', 580 [M + Na]⁺ |
| **66a=66** | 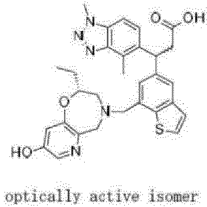 | ¹H-NMR (DMSO-D₆) δ : 0.84-0.90 (3H, m), 1.22-1.37 (1H, m), 1.43-1.55 (1H, m), 2.75-2.90 (5H, m), 3.03-3.12 (1H, m), 3.13-3.22 (1H, m), 3.72-3.90 (5H, m), 4.23 (3H, s), 4.96 (1H, t, J = 8.1 Hz), 6. 76-6. 79 (1H, m), 7. 17 (1H, s), 7.37 (1H, d, J = 5.6 Hz), 7.50 (1H, d, J = 8.8 Hz), 7.55 (1H, d, J = 8.8 Hz). 7.64 (1H, d, J = 5.6 Hz), 7.70-7.74 (2H, m), 9.89 (1H, br s), 11. 90-12. 50 (1H, br s). |
| | | MS(ESI) m/z: 556 [M - H]⁻, 558 [M + H]⁺, 580 [M + Na]⁺ |

**[Table 56]**

| **Example No.** | **structural formula** | **physicochemical data** |
|---|---|---|
| 67a | 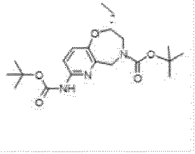 | MS(ESI/APCI) m/z: 394 [M + H]' |
| 67b | 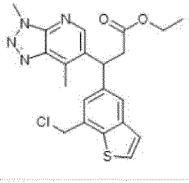 | MS(ESI/APCI) m/z: 429, 431 [M + H]⁺ |
| 67d=67 | 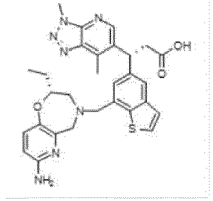 | ¹H-NMR (CDCl₃) δ : 1. 13 (3H, t, J = 7. 3 Hz), 1. 54-1. 58 (1H, m), 1. 67-1. 72 (1H, m), 2.61 (3H, s), 2.95-2.99 (2H, m), 3.29 (1H. dd, J = 14. 6, 9.8 Hz), 3.36 (1H, d, J = 14. 2 Hz), 3.54 (1H, d, J = 16.1 Hz), 3. 72-3. 74 (1H, m), 3. 80-3. 91 (3H, m), 4.37 (3H, s), 4.89 (1H, d, J = 9. 3 Hz), 6. 46 (1H, d, J = 8.8 Hz), 7.00 (1H, s), 7.07 (1H, d, J = 5. 4 Hz), 7.30 (1H, s), 7. 35-7. 36 (2H, m), 8. 70 (1H, s). |
| | | MS(ESI/APCI) m/z: 558 [M + H]⁺ |
| 68a | 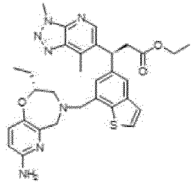 | MS(ESI/APCI) m/z: 586 [M + H]⁺ |
| 68b=68 | 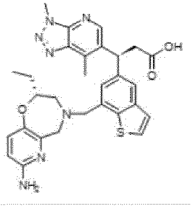 | ¹H-NMR (CDCl₃) δ : 1.05 (3H, t, J = 7. 3 Hz), 1. 53-1. 58 (1H. m), 1.66-1.72 (1H, m), 2.74 (3H, s), 3.00-3.06 (2H, m), 3. 17-3. 24 (2H, m), 3. 60 (1H, d, J = 14. 6 Hz), 3. 75-3. 79 (2H, m), 3.90 (1H, d, J = 13.2 Hz), 3.96 (1H, d, J = 14.2 Hz), 4.31 (3H, s), 4.93 (1H, dd, J = 11.2, 4.4 Hz), 6.33 (1H, d, J = 8.3 Hz), 7. 18 (1H, d, J = 5.4 Hz), 7.22 (1H, d, J = 11.7 Hz), 7.33 (1H, d, J = 5.4 Hz), 7.40 (2H, s), 8.78 (1H, s). |
| | | MS(ESI/APCI) m/z: 558 [M + H]⁺ |

**[Table 57]**

| **Example No.** | **structural formula** | **physicochemical data** |
|---|---|---|
| 69b | 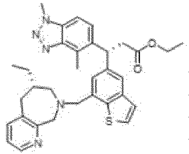 | MS(ESI/APCI) m/z: 568 [M + H]⁺ |
| 69c=69 | 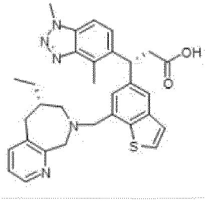 | ¹H-NMR (CDCl₃) δ : 0.94 (3H, t, J = 7. 6 Hz), 1. 24-1. 36 (2H, m), 1. 77 (1H, br s), 2. 70 (3H, s), 2. 77-2. 88 (2H, m), 2.93-3.10 (3H, m), 3. 29 (1H, d, J = 13. 2 Hz), 3. 71-3. 85 (3H, m), 4.03 (1H, d, J = 15. 1 Hz), 4.27 (3H, s), 4.96 (1H. dd, J = 10. 5, 4.6 Hz), 7. 12-7. 16 (2H, m), 7.20 (1H, t, J = 6.3 Hz), 7.30 (1H, s), 7.34 (1H, d, J = 5.4 Hz), 7.37 (1H, d, J = 8.8 Hz), 7.52 (1H, d, J = 8.3 Hz), 7. 56 (1H, d, J = 7.3 Hz), 8.40 (1H, d, J = 4.4 Hz). |
| 70b | 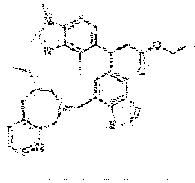 | MS(ESI/APCI) m/z: 568 [M + H]' |
| 70c=70 | 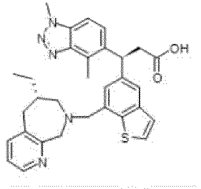 | ¹H-NMR (CDCl₃) δ : 0.92-0.99 (3H, m), 1.23-1.41 (2H, m), 1.81 (1H, br s), 2.74 (3H, s), 2.84 (1H, dd, J = 13.9, 9.5 Hz), 2.92 (1H, d, J = 15. 1 Hz). 2. 99-3. 09 (2H, m), 3. 18 (1H, t, J = 11.5 Hz), 3.33 (1H, t, J = 9. 5 Hz), 3.80 (1H, d, J = 4.4 Hz), 3.83 (1H, d, J = 3.9 Hz), 3. 88 (1H, dd, J = 13.4, 4. 1 Hz), 3.96 (1H, d, J = 14.6 Hz), 4.26 (3H, s), 5.00 (1H, dd, J = 10.5, 4.6 Hz), 7.16-7.22 (2H, m), 7.26-7.29 (1H, m). 7.34 (2H, d, J = 5.4 Hz), 7.41 (1H, s), 7.53-7.60 (2H, m), 8.32 (1H, s). |
| 71a | 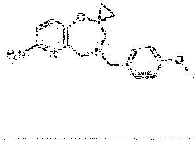 | ¹H-NMR (CDCl₃) δ : 0.42-0. 49 (2H, m), 0.84-0. 91 (2H. m), 2. 99 (2H, s), 3.66 (2H, s), 3.79 (3H, s), 4.03 (2H, s), 4.22 (2H, br s), 6.32 (1H. d, J = 8.3 Hz), 6.79-6.86 (2H, m), 6.94 (1H, d, J = 8.3 Hz), 7.18-7.23 (2H, m). |

**[Table 58]**

| **Example. No.** | **structural formula** | **physicochemical data** |
|---|---|---|
| 71b | 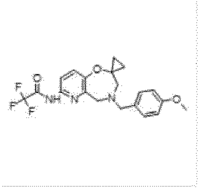 | ¹H-NMR (CDCl₃) δ : 0.50-0.56 (2H, m), 0.90-0.95 (2H, m), 3.04 (2H, s), 3.65 (2H, s), 3.80 (3H, s), 4.06 (2H, s), 6.82-6.88 (2H, m), 7.17-7.24 (3H, m), 7.98 (1H, d, J = 8.6 Hz), 8.42 (1H, br s). |
| 71c | 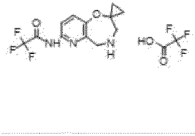 | ¹H-NMR (DMSO-D₆) δ : 0.88-0.93 (2H, m), 1.01-1.06 (2H, m), 3.65 (2H, s), 4.49 (2H, s), 7.52 (1H, d, J = 8.5 Hz), 7.88 (1H, d, J = 8.5 Hz), 9.53 (2H, br s), 12. 11 (IH, s). |
| 71d | 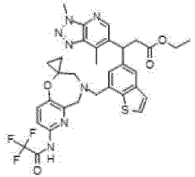 | ¹H-NMR (CDCl₃) δ : 0.55-0. 63 (2H, m), 0. 90-1. 03 (2H, m), 1. 10 (3H, t, J = 7. 3 Hz), 2.88 (3H, s), 3.07 (1H, d, J = 14. 1 Hz), 3.15-3.33 (2H, m), 3. 47 (1H, d, J = 14. 1 Hz), 3. 81 (1H, d, J = 14. 6 Hz), 3. 84-3. 94 (2H, m), 3. 97-4. 10 (3H, m), 4.28 (3H, s), 5.06 (1H, t, J = 7.8 Hz), 7.09-7.12 (1H, m), 7. 26-7. 30 (2H, m), 7. 43 (1H, d, J = 5. 4 Hz), 7. 60-7. 64 (1H, m), 8.08 (1H, d, J = 8.5 Hz), 8.76 (1H, s), 9.93 (IH, br s). |
| 71e=71 | 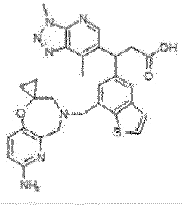 | ¹H-NMR (DMSO-D₆) δ : 0.38-0. 48 (2H, m), 0. 70-0. 78 (2H, m), 2.80 (3H, s), 2. 91 (1H, d, J = 14.2 Hz), 3.01 (1H, d, J = 14.2 Hz), 3. 22-3. 40 (2H, m), 3. 74-3. 90 (4H, m), 4.23 (3H, s), 4.95 (1H, t, J = 7.8 Hz), 5.35-5.86 (2H, br s), 6.25 (1H, d, J = 8.6 Hz), 6.90 (1H, d, J = 8.6 Hz), 7.17-7.21 (1H, m), 7.36 (1H, d, J = 5. 6 Hz), 7.66 (1H, d, J = 5.6 Hz), 7.75-7.78 (1H, m), 8.75 (1H, s). |
| | | MS(ESI) m/z: 554 [M - H]⁻, 556 [M + H]⁺, 578 [M + Na]' |
| 72a | 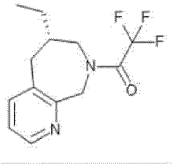 | MS(ESI/APCI) m/z: 273 [M + H]⁺ |

**[Table 59]**

| **Example No.** | **structural formula** | **physicochemical data** |
|---|---|---|
| 72b | 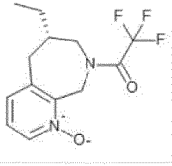 | MS(ESI/APCI) m/z: 289 [M + H]' |
| 72c | 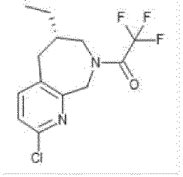 | MS(ESI/APCI) m/z: 307, 309 [M + H]' |
| 72d | 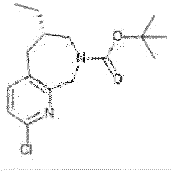 | MS(ESI/APCI) m/z: 311, 313 [M + H]⁺ |
| 72e | 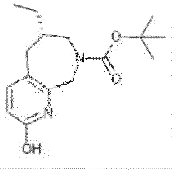 | MS(ESI/APCI) m/z: 293 [M + H]⁺ |
| 72g | 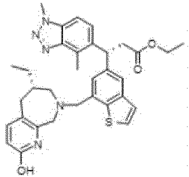 | MS(ESI/APCI) m/z: 584 [M + H]⁺ |

**[Table 60]**

| **Example No.** | **structural formula** | **physicochemical data** |
|---|---|---|
| 72h=72 | 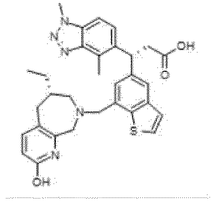 | ¹H-NMR (CDCl₃) δ : 1.04 (3H, t, J = 7. 3 Hz), 1.27-1.37 (2H, m), 1. 72 (1H, br s), 2. 58 (3H, s), 2.66 (2H, d, J = 6. 3 Hz), 2.85 (1H, t, J = 11. 5 Hz), 3. 11-3. 21 (3H, m), 3.54 (1H, d, J = 14.2 Hz), 3.74 (1H, d, J = 13.2 Hz), 3.93 (1H, d, J = 6.3 Hz), 3.96 (1H, br s), 4.33 (3H, s), 4.83 (1H, d. J = 10.7 Hz), 6.66 (1H, d, J = 8.8 Hz), 7.08 (1H, d, J = 5.4 Hz), 7.11 (1H, s), 7. 14 (1H, s), 7.35 (1H, d, J = 5.9 Hz), 7.48-7.53 (2H, m), 7.63 (1H, d, J = 8.3 Hz). |
| 73a | 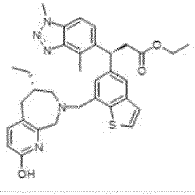 | MS(ESI/APCI) m/z: 584 [M + H]⁺ |
| 73b=73 | 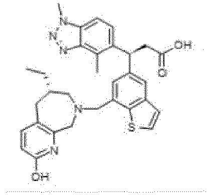 | ¹H-NMR (CDCl₃) δ : 1.02 (3H, t, J = 7. 3 Hz), 1. 33-1. 49 (2H, m), 1. 73 (1H, br s), 2.61-2.71 (4H, m), 2. 99 (2H, br s), 3. 13 (1H, d, J = 11.7 Hz), 3.33 (2H, br s), 3. 52 (1H, d, J = 13. 7 Hz), 3.79 (1H, d, J = 16.1 Hz), 3.91 (1H, d, J = 13. 2 Hz), 4.03 (1H, d, J = 11. 2 Hz), 4. 30 (3H, s). 4.90 (1H, d, J = 11. 7 Hz), 6.50 (1H, d, J = 9.3 Hz), 7.11 (1H, d, J = 4.9 Hz). 7. 19 (1H, s), 7.23-7.30 (1H, m), 7.35 (1H, d, J = 5.4 Hz), 7.45 (2H, br s), 7.61 (1H, d, J = 8.3 Hz). |
| 74a | 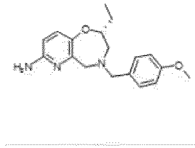 | ¹H-NMR (CDCl₃) δ : 1.01 (3H, t, J = 7. 3 Hz), 1. 34-1. 47 (1H, m), 1. 54-1. 67 (1H, m), 2.75 (1H, dd, J = 13.9, 9.5 Hz), 2.88-2.96 (1H, m), 3.57-3.73 (3H, m), 3.76-3.83 (4H, m), 4.02 (1H, d, J = 14.2 Hz), 4.20 (2H, br s), 6.34 (1H, d, J = 8.6 Hz), 6.81-6.87 (2H, m), 7.13 (1H, d, J = 8.6 Hz), 7.18-7.25 (2H, m). |
| 74b | 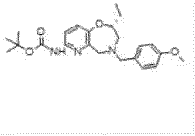 | ¹H-NMR (CDCl₃) δ : 1. 03 (3H, t, J = 7. 6 Hz), 1. 40-1. 55 (10H, m), 1. 60-1. 70 (1H, m), 2.82 (1H, dd, J = 13.9, 9.5 Hz), 2.92-3.00 (1H, m), 3.56 (1H, d, J = 13.2 Hz), 3.63 (1H, d, J = 13.2 Hz), 3. 71-3. 83 (5H, m), 3.99 (1H, d, J = 14.2 Hz), 6. 82-6. 88 (2H, m), 7.09 (1H, br s), 7. 17-7. 23 (2H, m), 7.30 (1H, d, J = 8.8 Hz), 7. 71 (1H, d, J = 8.8 Hz). |

**[Table 61]**

| **Example No.** | **structural formula** | **physicochemical data** |
|---|---|---|
| 74c | 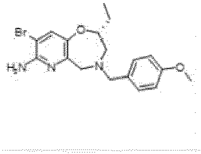 | ¹H-NMR (CDCl₃) δ : 1.01 (3H, t, J = 7.3 Hz), 1. 33-1. 47 (1H, m), 1.55-1.66 (1H, m), 2.78 (1H, dd, J = 13.9, 9.5 Hz), 2.89-2.98 (1H, m), 3.54-3.66 (2H, m), 3.68-3.83 (5H, m), 3. 96 (1H, d, J = 14. 2 Hz), 4. 63 (2H, br s), 6. 81-6. 88 (2H, m), 7. 17-7. 23 (2H, m), 7. 38 (1H, s). |
| 74d | 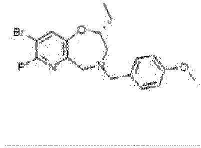 | ¹H-NMR (CDCl₃) δ : 1. 03 (3H, t, J = 7.6 Hz), 1. 40-1. 53 (1H, m), 1.59-1.72 (1H, m), 2.83 (1H, dd, J = 13.7, 9.5 Hz), 2. 92-3. 00 (1H, m), 3.56-3.67 (2H, m), 3. 77-3.96 (6H, m), 6. 81-6. 88 (2H, m), 7.15-7.22 (2H, m), 7.60 (1H, d, J = 7. 3 Hz). |
| 74e | 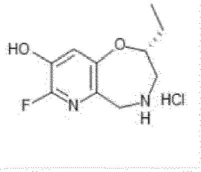 | ¹H-NMR (DMSO-D₆) δ : 1. 03 (3H, t, J = 7. 3 Hz), 1.57-1. 72 (2H, m), 3. 20-3. 32 (1H, m), 3. 51 (1H, d, J = 13. 0 Hz), 3. 91-4. 02 (1H, m), 4. 19 (1H, d, J = 14. 4 Hz), 4.23-4.34 (IH, m), 7. 14 (1H, d, J = 8.5 Hz), 9.25-9.85 (2H, br m), 10.99 (1H, s). |
| 74f | 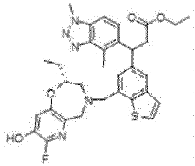 | ¹H-NMR (CDCl₃) δ : 0.90-0.99 (3H, m), 1.09 (3H, t, J = 7. 1 Hz), 1.31-1.41 (1H, m), 1.52-1.65 (1H, m), 2.83-2.96 (5H, m), 3.05-3.16 (1H, m), 3.17-3.28 (1H, m), 3.76-3.89 (4H, m), 3.92-4.05 (3H, m), 4.22-4.27 (3H, m), 5. 06-5. 17 (1H, m), 5. 55-5. 85 (1H, br s), 7.00-7.10 (2H, m), 7. 26-7. 32 (2H, m), 7.37-7.44 (2H, m), 7.55-7.62 (1H, m). |
| 74g=74 | 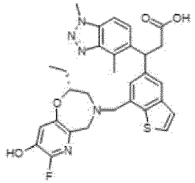 | ¹H-NMR (DMS0-D₆) δ : 0.81-0.95 (3H, m), 1.22-1.39 (1H, m), 1.41-1.58 (1H, m), 2.74-2.91 (5H, m), 3.03-3.23 (2H, m), 3.54-3.66 (1H, m), 3.74-3.92 (4H, m), 4.18-4.27 (3H, m), 4.90-5.00 (1H, m), 6.98-7.05 (1H, m), 7.14-7.22 (1H, m), 7.33-7.40 (1H, m), 7.47-7.57 (2H, m), 7.62-7.67 (IH, m), 7.70-7.76 (1H, m), 10.40 (1H, br s), 11.95-12.40 (1H, br s). |
| | | MS(ESI) m/z: 574 [M - H]⁻, 576 [M + H]⁺, 598 [M + Na]⁺ |

**[Table 62]**

| **Example No.** | **structural formula** | **physicochemical data** |
|---|---|---|
| 75b | 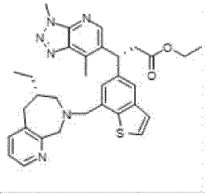 | ¹H-NMR (CDCl₃) δ : 0. 76 (3H, t, J = 7.3 Hz), 1.11 (3H, t, J = 7.3 Hz), 1.13-1. 20 (2H, m), 1. 67-1. 74 (1H, m), 2.71-2.80 (3H, m), 2.84 (3H, s), 2.96 (1H, dd, J = 13.4, 3.0 Hz), 3. 18-3. 30 (2H, m), 3.69 (1H, d, J = 14. 0 Hz), 3.83 (1H, d, J = 14.0 Hz), 3.98-4.06 (3H, m), 4. 17 (1H, d, J = 14.6 Hz), 4.31 (3H, s), 5.03 (1H, dd, J = 8.5, 7.9 Hz), 7.02 (1H, d, J = 1.2 Hz), 7. 10 (1H, dd, J = 7.9. 4.9 Hz), 7.24 (1H, d, J = 5.5 Hz), 7.40 (1H, d, J = 5.5 Hz), 7.44 (1H, dd, J = 7.3, 1.8 Hz), 7.56 (1H, d, J = 1. 2 Hz), 8.30 (1H, dd, J = 4.9, 1.8 Hz), 8.61 (1H, s). |
| | | MS(APCI) m/z: 569 [M + H]⁺ |
| 75c=75 | 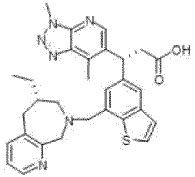 | ¹H-NMR (CDCl₃) δ : 1.00 (3H. t, J = 7. 3 Hz), 1. 30-1. 44 (2H, m), 1.83 (IH, br s), 2.76 (3H, s), 2.85 (1H, dd, J = 14.4, 9.0 Hz), 2.94 (1H, d, J = 14.2 Hz), 3.08 (2H, dd, J = 12.2, 4.4 Hz), 3.28 (1H, t, J = 11.7 Hz), 3.37 (1H, d, J = 12.7 Hz), 3. 75 (1H, d, J = 14.6 Hz), 3.81-3.94 (3H, m), 4.33 (3H, s), 4.96 (1H, dd, J = 11.2, 4.4Hz), 7.17-7.26 (2H, m), 7.30 (1H, s), 7.38 (1H, d, J = 5.9 Hz), 7.42 (1H, s), 7.60 (1H, d, J = 7.8 Hz), 8.33 (1H, d, J = 4.4 Hz), 8.74 (1H, s). |
| | | NS(ESI/APCI) m/z: 541 [M + H]⁺ |
| 76a | 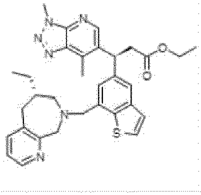 | ¹H-NMR (CDCl₃) δ : 0. 76 (3H, t. J = 7.3 Hz), 1.11 (3H, t, J = 7. 3 Hz), 1. 12-1. 21 (2H, m), 1. 67-1. 75 (1H, m), 2. 72-2. 81 (3H, m), 2.83 (3H, s), 2.97 (1H, dd, J = 13.4, 2.4 Hz), 3. 16-3. 30 (2H, m), 3.69 (1H, d, J = 14. 0 Hz), 3.83 (1H, d, J = 14.0 Hz), 3. 99-4. 06 (3H, m), 4. 18 (1H, d, J = 14.6 Hz), 4. 31 (3H, s). 5. 04 (1H, dd, J = 8. 8, 7. 0 Hz), 7. 01 (1H, s), 7. 10 (1H, dd, J = 7.3, 4.9 Hz), 7.24 (1H, d, J = 5.5 Hz), 7.40 (1H, d, J = 5.5 Hz), 7.45 (1H, d, J = 7.3 Hz), 7. 54 (1H, s), 8. 31 (1H, dd, J = 4.9, 1. 8 Hz), 8. 60 (1H, s). |
| | | MS (APCI) m/z: 569 [M + H]⁺ |
| 76b=76 | 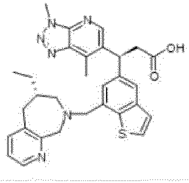 | ¹H-NMR (CDCl₃) δ : 1.03 (3H, t, J = 6. 1 Hz), 1.31-1. 44 (2H, m), 1.79 (1H, br s), 2.62 (3H, s), 2.85 (2H, s), 2.94-3.22 (3H. m), 3.45 (1H, br s), 3.62-3.74 (2H, m), 3.88 (1H, d, J = 13.7 Hz), 3.98 (1H, d, J = 15.1 Hz), 4.37 (3H, s), 4.86 (1H, d, J = 12.2 Hz), 7.08-7.21 (3H, m), 7.26-7.40 (2H, m), 7.62 (1H. br s), 8.53 (1H, br s), 8.72 (1H, br s). |
| | | MS(ESI/APCI) m/z: 541 [M + H]⁺ |

**[Table 63]**

| **Example No.** | **structural formula** | **physicochemical data** |
|---|---|---|
| 77a | 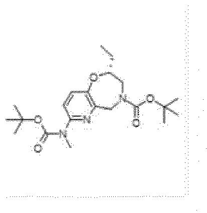 | MS(ESI/APCI) m/z: 408 [M + H]⁺ |
| 77b | 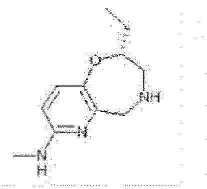 | MS(ESI/APCI) m/z: 208 [M + H]⁺ |
| 77c | 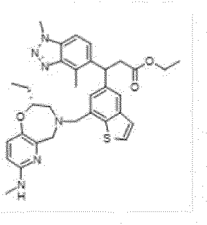 | MS(ESI/APCI) m/z: 599 [M + H]⁺ |
| 77d=77 | 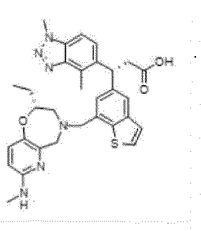 | ¹H-NMR (CDCl₃) δ : 1. 12 (3H, t, J = 7. 3 Hz), 1. 53-1. 58 (1H, m), 1. 66-1. 69 (1H, m), 2. 60 (3H, s), 2. 83-2. 85 (1H, m), 2.89 (3H, s), 2. 95 (1H, d, J = 10. 3 Hz), 3. 27-3. 37 (2H, m), 3.62 (1H, d, J = 15.1 Hz), 3. 71-3. 75 (1H, m), 3. 84-3. 87 (3H, m), 4.29 (3H, s), 4.93 (1H, d, J = 11. 2 Hz), 6.42 (1H, d, J = 9.3 Hz), 7.01 (1H, s), 7.04 (1H, d, J = 5.4 Hz), 7.24 (1H, s), 7.30 (1H, d, J = 4. 9 Hz), 7.43 (1H, d, J = 8. 3 Hz), 7.47 (1H, d, J = 8.8 Hz). 7. 57 (1H, d, J = 8.3 Hz). |
| | | MS(ESI/APCI) m/z: 571 [M + H]⁺ |
| 78a=78 | 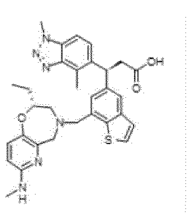 | ¹H-NMR (CDCl₃) δ : 0.97 (3H, t, J = 7.3 Hz), 1. 39-1. 44 (1H, m), 1.57-1.62 (1H, m), 2.72 (3H, s), 2.75 (3H, s), 2.90-2.96 (2H, m), 3.05-3.13 (2H, m), 3.65-3.68 (3H. m), 3.79 (1H, d, J = 13.2 Hz), 3.98 (1H, d, J = 13.6 Hz), 4.21 (3H, s), 4.94 (1H, dd, J = 10.3, 5.4 Hz), 6.25 (1H, d, J = 9.3 Hz), 7.18 (1H, d, J = 5.4 Hz), 7.26-7.31 (4H, m), 7.46 (1H, s), 7.50 (1H, d, J = 8.8 Hz). |
| | | MS(ESI/APCI) m/z: 571 [M + H]⁺ |

**[Table 64]**

| **Example No.** | **structural formula** | **physicochemical data** |
|---|---|---|
| 79a | 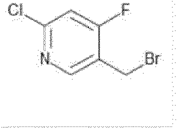 | ¹H-NMR (CDCl₃) δ : 4.45 (2H, s), 7. 12 (1H, d, J = 8.8 Hz), 8. 42 (1H, d, J = 9.8 Hz). |
| 79b | 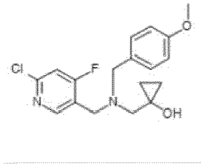 | ¹H-NMR (CDCl₃) δ : 0. 37-0. 43 (2H, m), 0.78-0. 84 (2H, m), 2.63 (2H, s), 3. 17 (1H, s), 3.66 (2H, s), 3.77 (2H, s), 3.80 (3H, s), 6. 82-6. 88 (2H, m), 7. 06 (1H, d, J = 9.0 Hz), 7.18 (2H, m), 8.32 (1H, d, J = 9.8 Hz). |
| 79c | 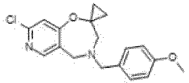 | ¹H-NMR (CDCl₃) δ : 0.57-0.64 (2H, m), 0.95-1.00 (2H, m), 3. 10 (2H, s), 3.63 (2H, s), 3.80 (3H, s), 3.88 (2H, s), 6. 80 (1H, s), 6.82-6.88 (2H, m), 7.14-7.20 (2H, m), 7.95 (1H, s). |
| 79d | 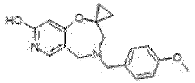 | ¹H-NMR (CDCl₃) δ : 0.54-0. 63 (2H, m), 0. 96-1. 05 (2H, m), 3.06 (2H, s), 3.59-3.70 (4H, m), 3.81 (3H, s), 6.97 (1H, s), 6.81-6.90 (2H, m), 6.98 (1H, s), 7.15-7.22 (2H, m), 11.60-12.05 (1H, br s). |
| 79e | 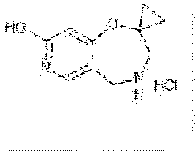 | ¹H-NMR (DMSO-D₆) δ : 0.87-1. 05 (4H, m), 3. 44-3. 52 (2H, m), 4.13-4.20 (2H, m), 5.77 (1H, s), 7.68 (1H, s), 9.71 (2H, br s). |

**[Table 65]**

| **Example No.** | **structural formula** | **physicochemical data** |
|---|---|---|
| 79f | 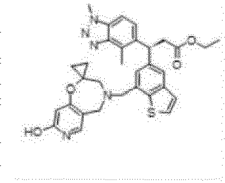 | ¹H-NMR (CDCl₃) δ : 0.50-0.57 (2H, m), 0.94-1. 02 (2H, m), 1. 11 (3H, t, J = 7.1 Hz), 2. 86 (3H, s), 3. 02-3. 16 (3H, m), 3.18-3.27 (1H, m), 3.56-3.68 (2H, m), 3.86 (2H, s), 4.03 (2H, q, J = 7. 1 Hz), 4.26 (3H, s), 5. 10 (1H, t, J = 8.0 Hz), 5.95 (1H, s), 6.76 (1H, s), 6.98 (1H, s), 7.24-7.28 (1H, m), 7.36 (1H, d, J = 8.5 Hz), 7.39-7.44 (2H, m), 7.63 (1H, s), 11.44 (1H, br s). |
| 79g=79 | 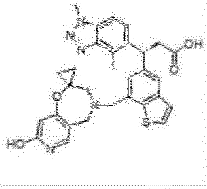 | ¹H-NMR (DMSO-D₆) δ : 0.45-0.55 (2H, m), 0.78-0.88 (2H, m), 2.76 (3H, s), 2.88-3.03 (2H, m), 3.05-3.22 (2H, m), 3.66 (2H, s), 3.86 (2H, s), 4.23 (3H, s), 4.95 (1H, t, J = 7.8 Hz), 5.68 (1H, s), 7.07 (1H, s), 7.16 (1H, s), 7.35 (1H, d, J = 5.4 Hz), 7.52 (1H, d, J = 8.8 Hz), 7.59 (1H, d, J = 8.8 Hz), 7.65 (1H, d, J = 5.4 Hz), 7.68 (1H, s), 11.38 (1H, br s). |
| | | MS(ESI) m/z: 554 [M - H]⁻, 578 [M + Na]⁺ |
| 80b | 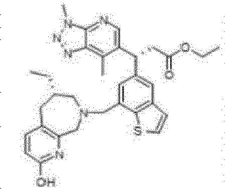 | ¹H-NMR (CDCl₃) δ : 0. 80 (3H, t, J = 7.6 Hz), 1. 12 (3H, t, J = 7. 3 Hz), 1.15- 1.22 (2H, m), 1.66-1. 74 (1H, m), 2. 53-2. 60 (2H, m), 2. 73 (1H, dd, J = 13. 4, 9. 7 Hz), 2.86 (3H, s), 3.02 (1H, d, J = 10. 9 Hz), 3. 21 (1H, dd, J = 15. 8, 8. 5 Hz), 3. 30 (1H, dd, J = 15.8, 7.9 Hz), 3.55 (1H, d, J = 15.2 Hz), 3.80-3.89 (3H, m), 4.04 (2H, q, J = 7.1 Hz), 4. 31 (3H, s), 5.06 (IH, t, J = 8.2 Hz), 6.34 (1H, d, J = 9.1 Hz), 7.11 (1H, s), 7. 23-7. 29 (3H, m), 7.41 (1H, d, J = 5.5 Hz), 7.56 (1H, d, J = 1.2 Hz), 8. 65 (1H, s). |
| | | MS(APCI) m/z: 585 [M + H]⁺ |
| 80c=80 | 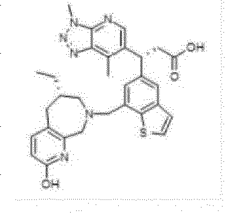 | ¹H-NMR (CDCl₃) δ : 1. 02 (3H, t, J = 7.6 Hz), 1.22-1.39 (3H, m), 2. 56-2. 67 (5H, m), 2.97 (1H, t, J = 11. 5 Hz), 3. 05-3. 25 (3H, m), 3.50 (1H, d, J = 14.2 Hz), 3.74 (1H, d, J = 13.2 Hz), 3.84-3.97 (2H, m), 4.38 (3H, s), 4.84 (1H, d, J = 11. 7 Hz), 6.47 (1H, d, J = 7.8 Hz), 7. 12 (2H, s), 7.21 (1H, s), 7.37 (1H, d, J = 4.9 Hz), 7.44 (1H, d, J = 9.3 Hz), 8. 72 (1H, s). |
| | | MS(ESI/APCI) m/z: 557 [M + H]⁺ |
| 81a | 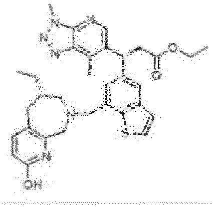 | ¹H-NMR (CDCl₃) δ : 0. 82 (3H, t, J = 7. 3 Hz), 1. 12 (3H, t, J = 7.0 Hz), 1.15-1. 23 (2H, m), 1. 68-1. 72 (1H, m), 2. 54-2. 59 (2H, m), 2.77 (1H, t, J = 11.5 Hz), 2.86 (3H, s), 3. 05 (1H, dd, J = 12.5, 4.6 Hz), 3.20 (1H, dd, J = 14.9, 8.2 Hz), 3. 39-3. 51 (2H, m), 3.81-3.87 (3H. m) , 4.03 (2H, q, J = 7. 1 Hz), 4. 31 (3H, s), 5.01 (1H, t, J = 8.2 Hz), 6.35 (1H, d, J = 9. 1 Hz), 7.07 (1H, s), 7.24-7.28 (3H, m), 7.42 (1H, d, J = 5.5 Hz), 7.61 (1H, d, J = 1.8 Hz), 8.67 (1H, s). |
| | | MS(APCI) m/z: 585 [M + H]⁺ |

**[Table 66]**

| **Example No.** | **structural formula** | **physicochemical data** |
|---|---|---|
| 81b=81 | 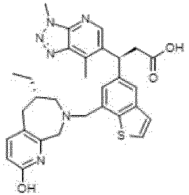 | ¹H-NMR (CDCl₃) δ : 1. 02 (3H, t, J = 7. 3 Hz), 1. 32-1. 51 (3H, m), 2. 59-2. 73 (4H, m), 2.97 (1H, br s), 3.09 (1H, br s), 3. 20 (1H, d, J = 10. 7 Hz), 3. 33 (2H, br s), 3. 53 (1H, d, J = 12.2 Hz), 3.78 (1H, br s), 3.91 (1H, d, J = 13.2 Hz), 4.03 (1H, d, J = 12.2 Hz), 4.37 (3H, s), 4.88 (1H, d, J = 12.7 Hz), 6.51 (1H, d, J = 8.8 Hz), 7. 14 (1H, d, J = 5.4 Hz), 7. 18 (1H, s), 7.24-7.33 (1H, m), 7.38 (1H, d, J = 5.4 Hz), 7.46 (1H, d, J = 8.8 Hz), 8.78 (1H, s). |
| | | MS(ESI/APCI) m/z: 557 [M + H]⁺ |
| 82a | 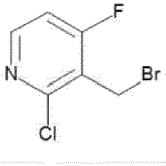 | ¹H-NMR (CDCl₃) δ : 4. 58 (3H, d, J = 1.2 Hz), 7. 05 (1H, dd, J = 8. 3. 6.6 Hz), 8.34 (1H, dd, J = 8.3, 5.6 Hz). |
| 82b | 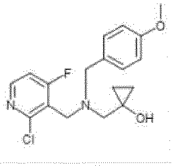 | ¹H-NMR (CDCl₃) δ : 0.38-0.42 (2H, m), 0.79-0.83 (2H, m), 2.61 (2H, s), 3.42 (1H, s), 3.63 (2H, s), 3.78 (3H, s), 3.92 (2H, d, J = 1.2 Hz), 6.79-6.83 (2H, m), 6.98 (1H, dd, J = 8.0, 6.4 Hz), 7. 14-7. 19 (2H, m), 8.28 (1H, dd, J = 8.0, 5.4 Hz). |
| 82c | 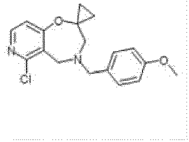 | ¹H-NMR (CDCl₃) δ : 0.57-0. 62 (2H, m), 0.93-0.98 (2H, m), 3.01 (2H, s), 3.72 (2H, s), 3.80 (3H, s), 4.21 (2H, s), 6.69 (1H, d, J = 5.4 Hz), 6.82-6.87 (2H, m), 7.17-7.22 (2H, m), 8.09 (1H, d, J = 5.4 Hz). |
| 82d | 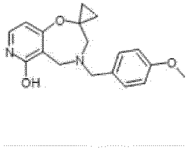 | ¹H-NMR (CDCl₃) δ : 0.57-0.65 (2H, m), 0.94-1.00 (2H, m), 2.89 (2H, s), 3.76 (2H, s), 3.78 (3H, s). 4.06 (2H, s), 5.85 (1H, d, J = 7.1 Hz), 6.80-6.86 (2H, m), 7.07 (1H, d, J = 7.1 Hz), 7.20-7.25 (2H, m), 11.30-11.50 (1H, br s). |

**[Table 67]**

| **Example No.** | **structural formula** | **physicochemical data** |
|---|---|---|
| 82f | 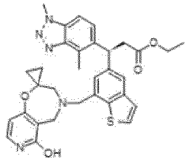 | ¹H-NMR (CDCl₃) δ : 0.54-0.59 (2H, m), 0.93-0.97 (2H, m), 1.08 (3H, t, J = 7. 1 Hz), 2. 86 (3H, s), 2. 87-2. 97 (2H, m), 3.04-3.12 (1H, m), 3.16-3.24 (1H, m), 3.97-4.03 (4H, m), 4.08-4.15 (2H, m), 4.22 (3H, s), 5. 09 (1H, t, J = 8.3 Hz), 5.85 (1H, d, J = 7.3 Hz), 7.04-7.09 (2H, m), 7.23 (1H, d, J = 5. 4 Hz), 7. 25-7. 29 (1H, m), 7. 35-7. 40 (2H, m), 7. 53-7. 57 (1H, m), 11. 07-11. 27 (1H, br s). |
| 82g=82 | 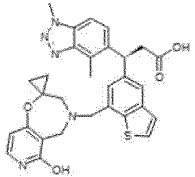 | ¹H-NMR (DMSO-D₆) δ : 0.59-0. 67 (2H, m), 0.82-0. 89 (2H, m), 2.75 (3H, s), 2.85-2.96 (2H, m), 3.03-3.19 (2H, m), 3.79-3.91 (4H, m), 4.23 (3H, s), 4.94 (1H, t, J = 7.6 Hz), 5.76 (1H, d, J = 7.1 Hz), 7. 12-7. 20 (2H, m), 7.35 (1H, d, J = 5.6 Hz), 7. 50 (1H, d, J = 8.8 Hz), 7. 55 (1H, d, J = 8.8 Hz), 7. 64 (1H, d, J = 5.6 Hz), 7. 66-7. 69 (1H, m), 11.24-11. 42 (1H, br s). |
| | | MS (ESI) m/z: 554 [M - H]⁻, 556 [M + H]⁺, 578 [M + Na]⁺ |
| 83e=83 | 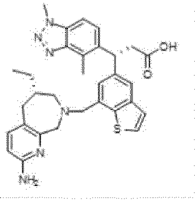 | ¹H-NMR (CDCl₃) δ : 1.03 (3H, t, J = 7.6 Hz), 1.22-1. 40 (3H, m), 2. 60-2. 69 (5H, m), 2. 87 (1H, t, J = 11. 5 Hz), 2.98 (1H, dd, J = 11. 0, 2.2 Hz), 3. 12 (1H, t, J = 12.2 Hz), 3.50 (1H, d, J = 14. 2 Hz), 3.68 (1H, d, J = 15.6 Hz), 3.75 (1H, d, J = 13. 2 Hz), 3. 87 (2H, t, J = 15.6 Hz), 4.32 (3H, s), 4.97 (1H, d, J = 9.8 Hz), 6.45 (1H, d, J = 8.3 Hz), 7.04-7.10 (2H, m), 7. 31 (1H, d, J = 5. 4 Hz), 7.36 (1H, s), 7.42-7.49 (2H, m), 7.62 (1H, d, J = 8.8 Hz). |
| 84b | 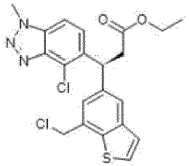 | MS(ESI/APCI) m/z: 448, 450 [M + H]⁺ |
| 84c | 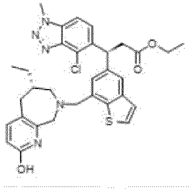 | MS(ESI/APCI) m/z: 604, 606 [M + H]⁺ |

**[Table 68]**

| **Example No.** | **structural formula** | **physicochemical data** |
|---|---|---|
| 84d=84 | 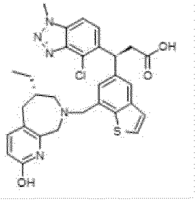 | ¹H-NMR (CDCl₃) δ : 0.99 (3H, t, J = 7.3 Hz), 1.31-1.45 (2H, m), 1.687 (1H, br s), 2.63-2.65 (1H, m), 2.86-2.89 (1H, m), 2.99-3.02 (1H, m), 3.10 (1H, d, J = 9.8 Hz), 3.26 (1H, br s), 3.36 (1H, d, J = 10.7 Hz), 3.48 (1H, d, J = 14.2 Hz), 3.68 (1H, d, J = 15.6 Hz), 3.90 (1H, d, J = 13.2 Hz), 3.98 (1H, d, J = 13.2 Hz), 4.30 (3H, s), 5.20 (1H, d, J = 9.8 Hz), 6.47 (1H, d, J = 9.3 Hz), 7.11 (1H, d, J = 5.4 Hz), 7.20 (1H, s), 7.28 (1H, s), 7.33 (1H, d, J = 5.4 Hz), 7.41 (1H, d, J = 9.3 Hz), 7.49 (1H, d, J = 7.8 Hz), 7.64 (1H, d, J = 8.3 Hz). |
| | | MS(ESI/APCI) m/z: 576 [M + H]⁺ |
| 85b | 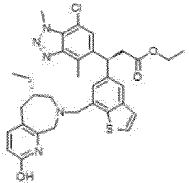 | MS(ESI/APCI) m/z: 618, 620 [M + H]* |
| 85c=85 | 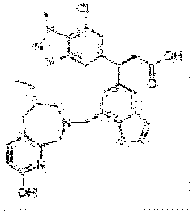 | ¹H-NMR (CDCl₃) δ : 0.98 (3H, t, J = 7. 3 Hz), 1.31-1. 44 (2H, m), 1. 70 (1H, br s), 2.55 (3H, s), 2.63-2.66 (1H, m), 2.91-2.94 (2H, m), 3.07 (1H, d, J = 9.3 Hz), 3.27 (1H, d, J = 11.2 Hz), 3. 33 (1H, d, J = 15.6 Hz), 3.47 (1H, d, J = 13.7 Hz), 3.73 (1H, d, J = 15. 1 Hz), 3.89 (1H, d, J = 13.2 Hz), 3.99 (1H, d, J = 13.7 Hz), 4.52 (3H, s), 4.82 (1H, d, J = 8.3 Hz), 6.46 (1H, d, J = 8.8 Hz), 7.14 (1H, d, J = 5.4 Hz), 7.23 (1H, s), 7.24 (1H, s), 7. 35 (1H, d, J = 5.4 Hz), 7.41 (1H, d, J = 8.8 Hz), 7.45 (1H, s). |
| | | MS(ESI/APCI) m/z: 590, 592 [M + H]' |
| 86b | 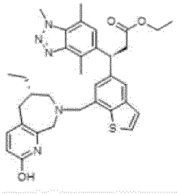 | MS(ESI/APCI) m/z: 598 [M + H]⁺ |

**[Table 69]**

| **Example No.** | **structural formula** | **physicochemical data** |
|---|---|---|
| 86c=86 | 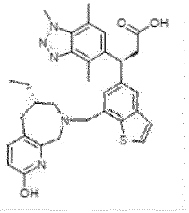 | ¹H-NMR (CDCl₃) δ : 1.05 (3H, t, J = 7.4 Hz), 1. 37-1. 52 (2H, m), 1.75 (1H, br s), 2. 60 (3H, s), 2.69-2.73 (1H, m), 2.85 (3H, s), 2.99 (2H, d, J = 9.8 Hz), 3. 14 (1H, dd, J = 12. 3, 3. 3 Hz), 3. 34-3.37 (2H, m), 3. 56 (1H, dd, J = 13.9, 2.9 Hz), 3.82 (1H, d, J = 14.7 Hz), 3.94 (1H, d, J = 13.3 Hz), 4.07 (1H, d, J = 13. 3 Hz), 4.52 (3H, s), 4.87 (1H, dd, J = 11.9, 2.5Hz), 6.54 (1H, d, J = 9.0 Hz), 7.16 (1H, d, J = 5.5 Hz), 7.21 (1H, s), 7.29 (2H, s), 7.38 (1H, d, J = 5.5 Hz), 7.49 (1H, d, J = 9.4 Hz). |
| | | MS(ESI/APCI) m/z: 570 [M + H]⁺ |
| 87b | 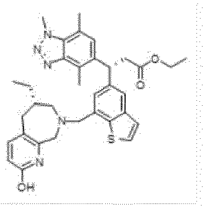 | MS (ESI/APCI) m/z: 598 [M **+** H]⁺ |
| 87c=87 | 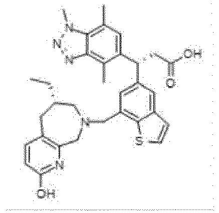 | ¹H-NMR (CDCl₃) δ : 1.01 (3H, t, J = 7.3 Hz), 1. 20-1. 34 (2H, m), 1.69 (1H, br s), 2.48 (3H, s), 2.63 (2H, d, J = 6.3 Hz), 2.78-2.80 (1H, m), 2.84 (3H, s), 3.09-3.17 (3H, m), 3.51 (1H, d, J = 14.2 Hz), 3.69 (1H, dd, J = 13.9, 7.6 Hz), 3.90-3.92 (2H, m), 4.48 (3H, s), 4.75 (1H, d, J = 10.7 Hz), 6. 52 (1H, d, J = 8.8 Hz), 7.07 (1H, *s),* 7.08 (1H, s), 7. 14 (1H, s), 7.24 (1H, d, J = 10.3 Hz), 7.32 (1H, d, J = 5.9 Hz), 7.47 (1H, d, J = 9.3 Hz). |
| | | MS(ESI/APCI) m/z: 570 [M + H]⁺ |
| 88a | 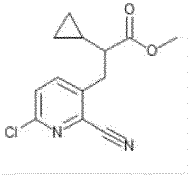 | ¹H-NMR (CDCl₃) δ : 0.10-0.22 (1H, m), 0.37-0.47 (1H, m), 0.50-0.66 (2H, m), 0.95-1.07 (1H, m), 1.90-1.99 (1H, m), 3.17-3.28 (2H, m), 3.66 (3H, s), 7.44 (1H, d, J = 8.6 Hz), 7.67 (1H, d, J = 8.6 Hz). |
| 88b | 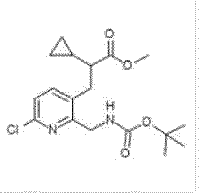 | ¹H-NMR (CDCl₃) δ : 0.06-0. 14 (1H, m), 0.28-0.38 (1H, m), 0.47-0.61 (2H, m), 0.93-1.05 (1H, m), 1.46-1.50 (9H, m), 1.82-1. 95 (1H, m), 2.91-3.11 (2H, m), 3.62 (3H, s), 4.34-4.49 (2H, m), 5.83 (1H, br s), 7. 14 (1H, d, J = 8.1 Hz), 7.43 (1H, d, J = 8. 1 Hz). |

**[Table 70]**

| **Example No.** | **structural formula** | **physicochemical data** |
|---|---|---|
| 88c | 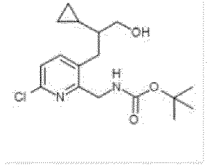 | ¹H-NMR (CDCl₃) δ: -0.23-0. 14 (1H, m), 0.04-0.14 (1H, m), 0.35-0.50 (2H, m), 0.55-0.66 (1H, m), 0.86-0. 96 (1H, m), 1. 46 (9H, s), 2. 34-2. 42 (1H, br m), 2. 67 (1H, dd, J = 13.9, 7.3 Hz), 3.03 (1H, dd, J = 13.9, 6.4 Hz), 3.49-3.58 (1H, m), 3. 60-3. 68 (1H, m), 4.36 (1H, dd, J = 16.1, 4.9 Hz), 4.54 (1H, dd, J = 16. 1, 5.6 Hz), 5.75 (1H, br s), 7. 16 (1H, d, J = 7.8 Hz), 7.49 (1H, d, J = 7.8 Hz). |
| 88d | 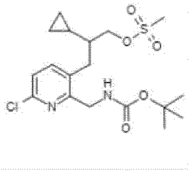 | ¹H-NMR (CDCl₃) δ: -0.18-0.08 (1H, m), 0.09-0.18 (1H, m), 0.40-0.66 (2H, m), 0.62-0.74 (1H, m), 1.12-1.23 (1H, m), 1.46 (9H, s), 2.79 (1H, dd, J = 14.4, 7.8 Hz), 2.94 (1H, dd, J = 14. 4, 6. 6 Hz), 3.06 (3H, s), 4. 08-4. 18 (1H, m), 4.24 (1H, dd, J = 9.8, 4.2 Hz), 4.42 (2H, d, J = 4.9 Hz), 5. 79 (1H, br s), 7. 19 (1H, d, J = 8.0 Hz), 7.49 (1H, d, J = 8.0 Hz). |
| 88e | 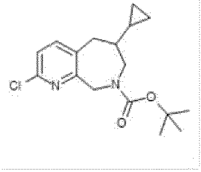 | ¹H-NMR (CDCl₃) δ: 0. 10-0. 22 (1. 3H, m), 0.25-0.35 (0. 7H, m), 0.44-0.60 (3H, m), 0.96-1.12 (1H, m), 1. 27-1. 50 (9H, m), 2.70-3.03 (2H, m), 3.16-3.44 (1H, m), 3. 67-3. 81 (0.3H, m), 3. 92-4. 10 (0.7H, m), 4.35-4.50 (0.7H, m), 4. 54-4. 84 (1.3H, m), 7.07-7.15 (1H, m), 7.35-7.46 (1H, m). |
| 88f | 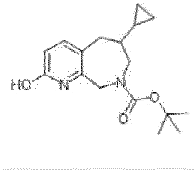 | ¹H-NMR (CDCl₃) δ: 0. 09-0. 20 (1. 3H, m), 0. 24-0. 34 (0.7H, m), 0. 44-0. 57 (3H, m), 0. 84-1. 05 (1H, m), 1. 33-1. 45 (9H, m), 2.57-2.83 (2H, m), 3. 15-3. 43 (1H, m), 3. 72-3. 84 (0.3H, m), 3.96-4.06 (0.7H, m), 4.20-4.37 (1H, m), 4.45-4.58 (1H, m), 6.34-6.43 (1H, m), 7.20-7.31 (1H, m). |
| 88g | 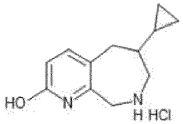 | ¹H-NMR (DMSO-D₆) δ: 0. 13-0. 28 (2H, m), 0.40-0.52 (2H, m), 0.60-0.72 (1H, m), 0.96-1.09 (1H, m), 2.75-2.90 (2H, m), 3.12-3.25 (1H, m), 3.34-3.45 (1H, m), 4.14-4.34 (2H, m), 6.41-6.50 (1H, m), 7.46-7.56 (1H, m), 9. 54 (1H, br s), 9.96 (1H, br s). |

**[Table 71]**

| **Example No.** | **structural formula** | **physicochemical data** |
|---|---|---|
| 88h | 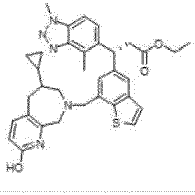 | ¹H-NMR (CDCl₃) *δ*: -0. 03-0. 06 (2H, m), 0.17-0.29 (1H, m), 0. 33-0. 48 (2H, m), 0.79-0.94 (1H, m), 1.08 (3H, t. J = 7. 1 Hz), 2.64-2.95 (6H, m), 3.01-3.15 (2H, m), 3.17-3.37 (1H, m), 3.59-3.96 (4H, m), 3. 97-4. 06 (2H, m), 4.20-4.27 (3H, m), 5.00-5.14 (1H, m), 6.25-6.33 (1H, m), 6.97-7.05 (1H, m), 7.20-7.33 (4H, m), 7.35-7.47 (2H, m), 7.53-7.62 (1H, m), 11. 56 (1H, br s). |
| 88i=88 | 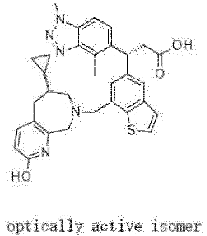 | ¹H-NMR (DMS0-D₆) *δ*: -0.64-0.51 (1H, m), -0.11-0.08 (2H, m), 0.21-0.38 (2H, m), 0.74-0.90 (1H, m), 2.55-2. 80 (7H, m), 3.04-3. 18 (2H, m), 3.65 (1H, d, J = 13.6 Hz), 3. 72-3. 84 (2H, m), 3.99 (1H, d, J = 15.4 Hz), 4.23 (3H, s), 4. 93 (1H, t, J = 8.0 Hz), 6.13 (1H, d, J = 9.0 Hz), 7.14 (1H, s), 7.30-7.40 (2H, m), 7.51-7.60 (2H, m), 7.65 (1H, d, J = 5.6 Hz), 7.72 (1H, s), 11.72 (2H, br s). |
| | | MS(BSI) m/z: 566 [M - H]⁻, 568 [M + H]⁺, 590 [M + Na]⁺ |
| 89a=89 | 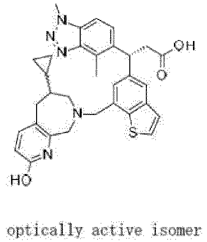 | ¹H-NMR (DMS0-D₆) *δ*: -0.55-0.40 (1H, m), -0.09-0.10 (2H, m), 0.21-0.39 (2H, m), 0.77-0.90 (1H, m), 2. 55-2.82 (7H, m), 3.05-3.19 (2H, m), 3.60-3.84 (3H, m), 3.99 (1N, d, J = 15.1 Hz), 4.22 (3H, s), 4.94 (1H, t, J = 8.1 Hz), 6.13 (1H, d, J = 9.3 Hz), 7. 13 (1H, s), 7.30-7.41 (2H, m), 7.51 (1H, d, J = 8.8 Hz), 7.56 (1H, d, J = 8.8 Hz), 7.66 (1H, d, J = 5.4 Hz), 7.70 (1H, s), 11.30-12.35 (2H, br s). |
| | | MS(ESI) m/z: 566 [M - H]⁻, 568 [M + H]*, 590 [M + Na]⁺ |
| 90a | 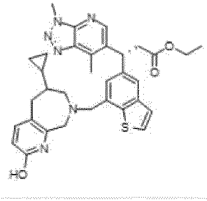 | ¹H-NMR (CDCl₃) *δ* : -0.38-0.29 (0.5H, m), -0. 28--0. 18 (0. 5H, m), -0.02-0.10 (1H, m), 0.17-0.31 (1H, m), 0.34-0.52 (2H, m), 0.81-0.95 (1H, m), 1.08-1.15 (3H, m), 2.66-2.76 (2H, m), 2.81-2.92 (4H, m), 3.05-3.15 (1H, m), 3.15-3.31 (1. 5H, m), 3.36 (0. 5H, dd, J = 15. 4, 8.1 Hz), 3. 58-3. 78 (2H, m), 3.80-3.97 (2H, m), 3. 98-4. 07 (2H, m), 4.28-4.33 (3H, m), 4. 96-5. 09 (1H, m), 6. 30 (1H, d, J = 9. 0 Hz), 7.00-7.08 (1H, m), 7.20-7.29 (2H, m), 7.37-7.42 (1H, m), 7. 53-7. 61 (1H, m), 8.62 (0.5H, s), 8.65 (0.5H. s), 11.15-11.60 (1H, br s). |

**[Table 72]**

| **Example No.** | **structural formula** | **physicochemical data** |
|---|---|---|
| 90b=90 | 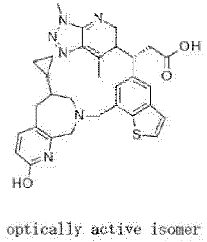 | ¹H-NMR (DMS0-D₆) *δ*: -0.90-0.81 (1H, m), -0. 26--0. 17 (1H, m), -0.16-0.09 (1H, m), 0. 16-0.30 (2H, m), 0.69-0.80 (1H, m), 2.54-2.69 (4H, m), 2.76 (3H, s), 3. 18-3.31 (2H, m), 3.65 (1H, d, J = 13. 4 Hz), 3.72-3.85 (2H, m), 3. 97-4. 06 (1H, m), 4.24 (3H, s), 4.91 (1H, t, J = 8.1 Hz), 6.13 (1H, d, J = 9.0 Hz), 7.18-7.22 (1H, m), 7.33 (1H, d, J = 9.0 Hz), 7.38 (1H, d, J = 5.4 Hz), 7.67 (1H, d, J = 5.4 Hz), 7.78-7.83 (1H, m), 8.81 (1H, s). |
| | | MS(ESI) m/z: 567 [M - H]⁻, 569 [M + H]⁺ , 591 [M + Na]⁺ |
| 91a=91 | 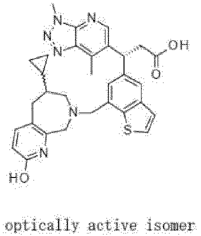 | ¹H-NMR (DMS0-D₆) *δ* : -0. 65-0. 58 (1H, m), -0.11-0.03 (2H, m), 0.20-0. 34 (2H, m), 0. 75-0. 88 (1H, m), 2.55-2.76 (4H, m), 2. 80 (3H, s), 3. 22-3. 28 (2H, m), 3. 62-3. 81 (3H, m), 4.00 (1H, d, J = 15.2 Hz), 4.22 (3H, s), 4.93 (1H, t, J = 7.8 Hz), 6.12 (1H, d, J = 9. 3 Hz), 7. 15-7. 19 (1H, m), 7.33 (1H, d, J = 9.3 Hz), 7.38 (1H, d, J = 5.4 Hz), 7.68 (1H, d, J = 5.4 Hz), 7.77-7.80 (1H, m), 8.72 (1H, s). |
| | | MS(ESI) m/z: 567 [M - H]⁻, 569 [M + H]⁺ , 591 [M + Na]⁺ |
| 92a | 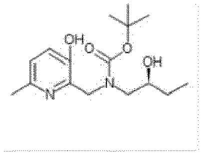 | ¹H-NMR (CDCl₃) *δ* : 0.96 (3H, t, J = 7.6 Hz), 1. 34-1. 52 (11H, m), 2.44 (3H, s), 3.28 (1H, dd, J = 14. 6, 7. 1 Hz), 3. 52-3.60 (1H, m), 3.78 (1H, d, J = 14.6 Hz), 4.31 (1H, d, J = 14.6 Hz), 4. 65 (1H, d, J = 14.6 Hz), 7.01 (1H, d. J = 8.3 Hz), 7.14 (1H, br s), 7.21 (1H, d, J = 8.3 Hz), 9.58 (1H, s). |
| 92b | 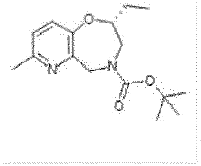 | ¹H-NMR (CDCl₃) *δ* : 1.08 (3H, t, J = 7. 6 Hz), 1. 33-1. 45 (9H, br m), 1.50-1.73 (2H, m), 2.48 (3H, s), 3.30-3.55 (1H, br m), 3. 68-3. 80 (1H, br m), 3. 87-4. 02 (1H, br m), 4. 41-4. 63 (1H, br m), 4. 72-4. 81 (1H, m), 6.93-7.00 (1H, m), 7.13-7.21 (1H, m). |

**[Table 73]**

| **Example No.** | **structural formula** | **physicochemical data** |
|---|---|---|
| 92c | 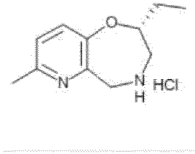 | ¹H-NMR (DMS0-D₆) *δ* : 1.04 (3H, t, J = 7.3 Hz), 1. 66 (2H, quintet, J = 7.3 Hz), 2.48 (3H, s), 3.26-3.37 (1H, br m), 3. 46-3. 55 (1H, m), 3.96-4.04 (1H, m), 4.45 (2H, br s), 7. 34 (1H, d, J = 8.3 Hz), 7.53 (1H, d, J = 8.3 Hz), 9. 72 (1H, br s), 9.99 (1H, br s). |
| 92d | 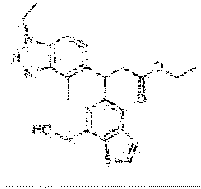 | ¹H-NMR (CDCl₃) δ: 1. 10 (3H, t, J = 7. 0 Hz), 1.56-1.63 (3H, m), 1.91 (1H, t, J = 5.9 Hz), 2.88 (3H, s), 3. 11 (1H, dd, J = 15.4, 8.3 Hz), 3.22 (1H, dd, J = 15.4, 7.6 Hz), 4.03 (2H, q, J = 7.0 Hz), 4.64 (2H, q, J = 7.3 Hz), 4.90 (2H, d, J = 5.9 Hz), 5. 11-5. 17 (1H, m), 7.20-7.23 (1H, m), 7.28-7.33 (2H, m), 7.38 (1H, d, J = 8.8 Hz), 7.44 (1H, d, J = 5.4 Hz), 7.62-7.66 (1H, m). |
| 92e | 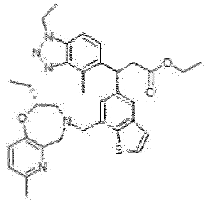 | ¹H-NMR (CDCl₃) *δ* : 0.89-0.97 (3H, m), 1. 06-1. 11 (3H, m), 1.28-1.40 (1H, m), 1. 49-1. 62 (4H, m), 2. 46 (1. 5H, s), 2. 48 (1. 5H, s), 2. 83-2. 88 (5H, m), 3. 06-3. 14 (1H, m), 3. 16-3. 25 (1H, m), 3.75-3.82 (1H, m), 3.83-3.93 (2H, m), 3.97-4.08 (3H, m), 4.15-4.21 (1H, m), 4.63 (2H, q, J = 7.3 Hz), 5.06-5.13 (1H, m), 6.97-7.01 (1H, m), 7.04-7.09 (1H, m), 7.19-7.23 (1H, m), 7.23-7.26 (1H, m), 7.26-7.30 (1H, m), 7.35-7.40 (2H, m), 7.54-7.59 (1H, m). |
| 92f=92 | 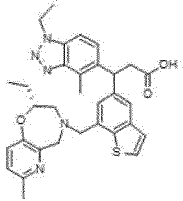 | ¹H-NMR (DMS0-D₆) *δ* : 0.83-0.94 (3H, m), 1. 21-1. 38 (1H, m), 1. 42-1. 57 (4H, m), 2.32 (1. 5H, s), 2. 35 (1. 5H, s), 2. 73-2. 79 (3H, m), 2.80-2.91 (2H, m), 3. 02-3. 21 (2H, m), 3. 75-3. 88 (4H, m), 3.92-3.98 (1H, m), 4.62-4.70 (2H, m), 4.92-5.00 (1H, m), 7.06-7.10 (1H, m), 7.19 (1H, s), 7.25-7.30 (1H, m), 7.36-7.39 (1H, m), 7.48-7.54 (1H, m), 7.56-7.61 (1H, m), 7.64-7.67 (1H, m), 7.71-7.76 (1H, m), 11.40-12.90 (1H, br s). |
| | | MS(ESI) m/z: 568 [M - H]⁻, 570 [M + H]⁺ , 592 [M + Na]⁺ |
| 93a | 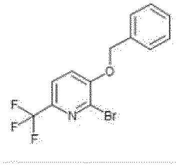 | MS(ESI/APCI) m/z: 332, 334 [M + H]* |

**[Table 74]**

| **Example No.** | **structural formula** | **physicochemical data** |
|---|---|---|
| 93b | 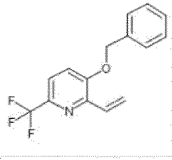 | MS(ESI/APCI) m/z: 280 [M + H]⁺ |
| 93c | 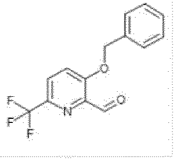 | MS(ESI/APCI) m/z: 282 [M + H]⁺ |
| 93d | 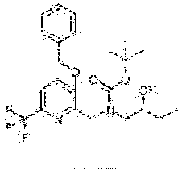 | MS(ESI/APCI) m/z: 355 [M + H - Boc]⁺ |
| 93e | 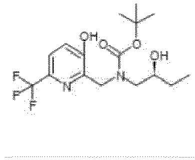 | MS(ESI/APCI) m/z: 265 [M + H - Doc]⁺ |
| 93f | 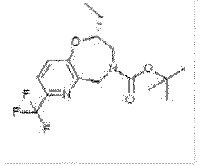 | MS(ESI/APCI) m/z: 291 [M + H - t-Bu]' |

**[Table 75]**

| **Example No.** | **structural formula** | **physicochemical data** |
|---|---|---|
| 93g | 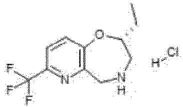 | MS(ESI/APCI) m/z: 247 [M + H]⁺ |
| 93h | 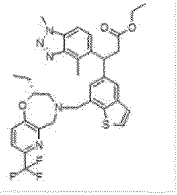 | MS(ESI/APCI) m/z: 638 [M + H]⁺ |
| 93i=93 | 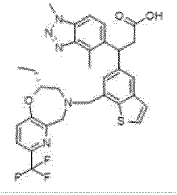 | ¹H-NMR (CDCl₃) *δ* : 1.01 (3H, t, J = 7.4 Hz), 1. 46-1. 53 (1H, m), 1.67-1.71 (1H, m), 2.81 (1. 5H, s), 2.81 (1.5H, s), 2.99-3.03 (2H, m), 3.08-3.12 (1H, m), 3.19-3.26 (1H, m), 3.85-3.88 (1H, m), 3.92-4.01 (2H, m), 4.04-4.19 (2H, m), 4.26 (1.5H, s), 4.27 (1. 5H, s), 5.06 (1H, t. J = 8.0 Hz), 7.12 (1H, s), 7.22 (1H. s), 7.24 (1H, d, J = 5.1 Hz), 7.31-7.34 (1H, m), 7.39 (1H, d, J = 5.5 Hz), 7.44 (1H, d, J = 4.1 Hz), 7.46 (1H, d, J = 6.7 Hz), 7.54-7.56 (1H, m). |
| | | MS(ESI/APCI) m/z: 610 [M + H]⁺ |

Although the present invention has been described in detail and with reference to specific embodiments, it is apparent to those skilled in the art that various changes and modifications can be made without departing from the spirit and scope of the present invention. This application is based on Japanese Patent Application No. 2022-075222 filed on April 28, 2022 in Japan, the contents of which are incorporated in full herein.

## Claims

1. A compound represented by the following formula (1): 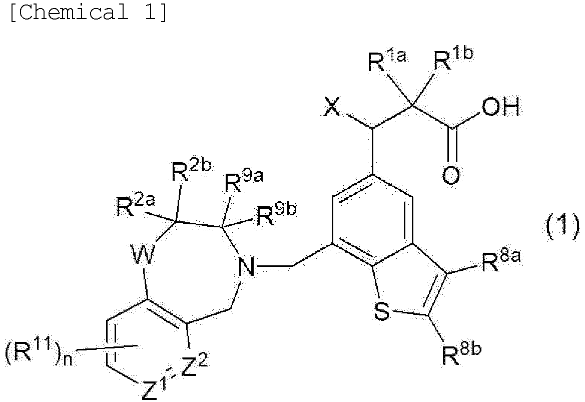 wherein
R^{1a} and R^{1b} are each independently a hydrogen atom or a C₁₋₆ alkyl group,
R^{2a} and R^{2b} are each independently a hydrogen atom, a C₁₋₆ alkyl group optionally substituted by 1 to 3 substituents selected from substituent group a, or a C₃₋₆ cycloalkyl group optionally substituted by 1 to 3 substituents selected from substituent group a, or R^{2a} and R^{2b} are bonded together to form, together with the carbon atom to which R^{2a} and R^{2b} are bonded, a C₃₋₈ cycloalkane optionally substituted by 1 to 3 substituents selected from substituent group b, or a 3- to 8-membered saturated oxygen-containing heterocycle,
Z¹ is -CH-, -CR³- or a nitrogen atom,
R³ is a halogen atom, a hydroxy group, an amino group optionally substituted by one or two C₁₋₆ alkyl groups, a C₁₋₆ alkyl group optionally substituted by 1 to 3 substituents selected from substituent group a, or a C₁₋₆ alkoxy group optionally substituted by 1 to 3 substituents selected from substituent group a,
Z² is -CH-, -CR⁴- or a nitrogen atom,
R⁴ is a halogen atom, a hydroxy group, an amino group optionally substituted by one or two C₁₋₆ alkyl groups, a C₁₋₆ alkyl group optionally substituted by 1 to 3 substituents selected from substituent group a, or a C₁₋₆ alkoxy group optionally substituted by 1 to 3 substituents selected from substituent group a,
R¹¹ in the number of n are each independently a halogen atom, a hydroxy group, an amino group optionally substituted by one or two C₁₋₆ alkyl groups, a C₁₋₆ alkyl group optionally substituted by 1 to 3 substituents selected from substituent group a, or a C₁₋₆ alkoxy group optionally substituted by 1 to 3 substituents selected from substituent group a,
n is an integer of 0 to 2,
R^{8a} and R^{8b} are each independently a hydrogen atom, a halogen atom,
a cyano group, a C₁₋₆ alkyl group or a C₁₋₆ alkoxy group,
R^{9a} and R^{9b} are each independently a hydrogen atom or a C₁₋₆ alkyl group,
W is -CH₂-, -CHR¹⁰- or an oxygen atom,
R¹⁰ is a C₁₋₆ alkyl group, and
X is a group represented by the following formula (A1) or (A2): 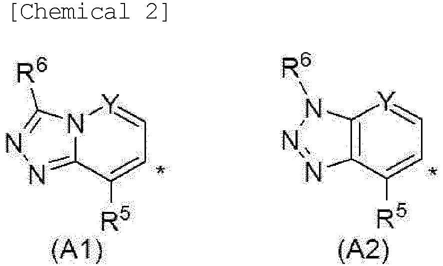
wherein
* is the bonding position to the carbon atom to which X is bonded, R⁵ is a hydrogen atom, a halogen atom, or a C₁₋₆ alkyl group optionally substituted by 1 to 3 substituents selected from substituent group a,
R⁶ is a hydrogen atom, or a C₁₋₆ alkyl group optionally substituted by 1 to 3 substituents selected from substituent group a,
Y is -CH-, -CR⁷- or a nitrogen atom, and
R⁷ is a hydroxy group, a halogen atom, a cyano group, a C₁₋₆ alkyl group optionally substituted by 1 to 3 substituents selected from substituent group a, a C₁₋₆ alkoxy group optionally substituted by 1 to 3 substituents selected from substituent group a, or a C₃₋₆ cycloalkyl group,
provided that when Z¹ is -CH- or -CR³-, then Z² is a nitrogen atom, when Z² is -CH- or -CR⁴-, then Z¹ is a nitrogen atom, and both Z¹ and Z² cannot simultaneously be -CH-, -CR³- or -CR⁴-,
substituent group a:
a hydroxy group,
a halogen atom,
a cyano group,
a C₁₋₆ alkyl group,
a C₁₋₆ alkoxy group,
a C₁₋₆ alkyl group substituted by substituent group b,
a C₁₋₆ alkoxy group substituted by substituent group b,
an amino group optionally substituted by one or two C₁₋₆ alkyl groups,
a C₁₋₆ alkylsulfonyl group
substituent group b:
a halogen atom,
a cyano group,
a C₁₋₆ alkyl group,
a C₁₋₆ alkoxy group,
or a pharmaceutically acceptable salt thereof.

2. The compound according to claim 1, wherein R¹³ and R^{1b} are each independently a hydrogen atom or a methyl group, or a pharmaceutically acceptable salt thereof.

3. The compound according to claim 1 or 2, wherein R^{2a} and R^{2b} are each independently a hydrogen atom, a C₁₋₆ alkyl group, a C₁₋₆ haloalkyl group or a C₃₋₆ cycloalkyl group, or
R^{2a} and R^{2b} are bonded together to form, together with the carbon atom to which R^{2a} and R^{2b} are bonded, a C₃₋₆ cycloalkane, or a pharmaceutically acceptable salt thereof.

4. The compound according to claim 1 or 2, wherein R^{2a} and R^{2b} are each independently a hydrogen atom, a methyl group, an ethyl group, a trifluoromethyl group or a difluoroethyl group, or
R^{2a} and R^{2b} are bonded together to form, together with the carbon atom to which R^{2a} and R^{2b} are bonded, a cyclopropane, or a pharmaceutically acceptable salt thereof.

5. The compound according to any one of claims 1 to 4, wherein Z¹ is -CH- or -CR³-, and
Z² is a nitrogen atom, or a pharmaceutically acceptable salt thereof.

6. The compound according to any one of claims 1 to 4, wherein Z¹ is -CR³-, and
Z² is a nitrogen atom, or a pharmaceutically acceptable salt thereof.

7. The compound according to claim 5 or 6, wherein R³ is a halogen atom, a hydroxy group, an amino group, a C₁₋₆ alkylamino group or a C₁₋₆ alkyl group, or a pharmaceutically acceptable salt thereof.

8. The compound according to claim 5 or 6, wherein R³ is a hydroxy group, or a pharmaceutically acceptable salt thereof.

9. The compound according to any one of claims 1 to 8, wherein n is 1, and
R¹¹ is a hydroxy group, or a pharmaceutically acceptable salt thereof.

10. The compound according to any one of claims 1 to 8, wherein n is 0, or a pharmaceutically acceptable salt thereof.

11. The compound according to any one of claims 1 to 10, wherein R^{8a} and R^{8b} are both hydrogen atoms, or a pharmaceutically acceptable salt thereof.

12. The compound according to any one of claims 1 to 11, wherein R^{9a} and R^{9b} are both hydrogen atoms, or a pharmaceutically acceptable salt thereof.

13. The compound according to any one of claims 1 to 12, wherein W is an oxygen atom, or a pharmaceutically acceptable salt thereof.

14. The compound according to any one of claims 1 to 13, wherein X is a group represented by the following formula (A2): 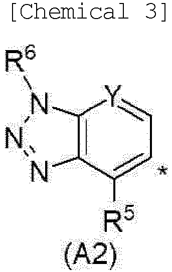
wherein each symbol is as defined above,
or a pharmaceutically acceptable salt thereof.

15. The compound according to claim 14, wherein R⁵ is a halogen atom or a C₁₋₆ alkyl group, or a pharmaceutically acceptable salt thereof.

16. The compound according to claim 14, wherein R⁵ is a methyl group, or a pharmaceutically acceptable salt thereof.

17. The compound according to any one of claims 14 to 16, wherein R⁶ is a methyl group, an ethyl group, a dimethylaminoethyl group, a 2,2,2-trifluoroethyl group, a 2-hydroxy-2-methylpropyl group or a 3-methanesulfonylpropyl group, or a pharmaceutically acceptable salt thereof.

18. The compound according to any one of claims 14 to 16, wherein R⁶ is a methyl group, or a pharmaceutically acceptable salt thereof.

19. The compound according to any one of claims 1 to 18, wherein R⁷ is a chlorine atom, a cyano group, a methyl group, a cyclopropyl group, a trifluoromethyl group, a methoxy group, a difluoromethoxy group or a trifluoromethoxy group, or a pharmaceutically acceptable salt thereof.

20. Any compound selected from the following group or a pharmaceutically acceptable salt thereof:
(3S)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid,
(3R)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)propanoic acid,
(3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoic acid,
(3R)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}propanoic acid,
(3R)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
(3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
(3S)-3-(7-{[(2S)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
(3R)-3-(7-{[(2S)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
(3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid,
(3R)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(3,7-dimethyl-3H-[1,2,3]triazolo[4,5-b]pyridin-6-yl)propanoic acid,
(3S)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoic acid,
(3R)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoic acid,
(3S)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)-2,2-dimethylpropanoic acid, and
(3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoic acid.

21. Any compound selected from the following group or a pharmaceutically acceptable salt thereof:
(3R)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid,
(3S)-3-(1,4-dimethyl-1H-benzotriazol-5-yl)-3-(7-{[(2R)-2-ethyl-7-hydroxy-2,3-dihydropyrido[2,3-f][1,4]oxazepin-4(5H)-yl]methyl}-1-benzothiophen-5-yl)-2,2-dimethylpropanoic acid, and
(3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoic acid.

22. (3R)-3-(7-{[(2R)-2-cyclopropyl-7-hydroxy-2,3-dihydropyrido[2,3-f] [1,4]oxazepin-4(5H)-yl]methyl}-1-benzothiophen-5-yl)-3-(1,4-dimethyl-1H-benzotriazol-5-yl)propanoic acid or a pharmaceutically acceptable salt thereof.

23. (3S)-3-(1,4-dimethyl-1H-benzotriazol-5-yl)-3-(7-{[(2R)-2-ethyl-7-hydroxy-2,3-dihydropyrido[2,3-f] [1,4]oxazepin-4(5H)-yl]methyl}-1-benzothiophen-5-yl)-2,2-dimethylpropanoic acid or a pharmaceutically acceptable salt thereof.

24. (3S)-3-(1,4-dimethyl-1H-benzotriazol-5-yl)-3-{7-[(7'-hydroxy-3'H-spiro[cyclopropane-1,2'-pyrido[2,3-f][1,4]oxazepin]-4'(5'H)-yl)methyl]-1-benzothiophen-5-yl}-2,2-dimethylpropanoic acid or a pharmaceutically acceptable salt thereof.

25. A medicament comprising the compound according to any one of claims 1 to 24 or a pharmaceutically acceptable salt thereof as an active ingredient.

26. The medicament according to claim 25, for activating Nrf2.

27. The medicament according to claim 25, for inhibiting a protein-protein interaction between Keap1 and Nrf2.

28. The medicament according to claim 25, for the prophylaxis and/or treatment of an oxidative stress-related disease.

29. The medicament according to claim 28, wherein the oxidative stress-related disease is selected from the group consisting of renal diseases, liver diseases, respiratory diseases, dermatic diseases, cardiovascular diseases, central nervous system diseases, autoimmune diseases and ophthalmic diseases.

30. The medicament according to claim 25, for the prophylaxis and/or treatment of a disease selected from the group consisting of a renal disease selected from the group consisting of chronic kidney disease, acute nephritis, chronic nephritis, acute renal failure, chronic renal failure, nephrotic syndrome, IgA nephropathy, diabetic nephropathy, gouty kidney, nephrosclerosis, hydronephrosis and tubulointerstitial nephritis; a liver disease selected from the group consisting of alcoholic fatty liver, non-alcoholic steatohepatitis, hepatic fibrosis and cirrhosis; a respiratory disease selected from the group consisting of bronchitis, pneumonia, pleurisy, chronic obstructive pulmonary diseases, acute lung disorder, diffuse panbronchiolitis, interstitial pneumonia and asthma; a dermatic disease selected from the group consisting of UV and radiation skin disorder, radiation mucosal disorder, epidermolysis blister syndrome, psoriasis, atopic dermatitis and scleroderma; a cardiovascular disease selected from the group consisting of cardiac failure, myocardial infarction, arteriosclerosis and pulmonary arterial hypertension; a central nervous system disease selected from the group consisting of Alzheimer's disease, Parkinson's disease, Huntington's disease, amyotrophic lateral sclerosis, cerebral infarction, polyglutamine disease and autism; a mitochondrial disease selected from the group consisting of Friedreich's ataxia and mitochondrial myopathy; an autoimmune disease selected from the group consisting of multiple sclerosis, rheumatoid arthritis, systemic lupus erythematosus, Sjogren syndrome, type 1 diabetes, ulcerative colitis and Crohn's disease; and an ophthalmic disease selected from the group consisting of allergic conjunctival diseases, viral conjunctivitis, pterygium, cornea infectious disease, dry eye, corneal disorders, uveitis, Behcet's disease, diabetic retinopathy, retinal detachment, retinal vein occlusion, central serous chorioretinopathy, age-related macular degeneration, diabetic macular edema, macular disease, retinitis pigmentosa, glaucoma and cataract.

31. The medicament according to claim 25, for the prophylaxis and/or treatment of a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, and age-related macular degeneration

32. The compound according to any one of claims 1 to 24 or a salt thereof, for use in the prophylaxis and/or treatment of a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, and age-related macular degeneration.

33. The medicament according to claim 25, for use in the prophylaxis and/or treatment of a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, and age-related macular degeneration.

34. Use of the compound according to any one of claims 1 to 24 or a pharmaceutically acceptable salt thereof in producing a prophylactic and/or therapeutic agent for a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, and age-related macular degeneration.

35. A method for the prophylaxis and/or treatment of a disease selected from the group consisting of chronic kidney disease, non-alcoholic steatohepatitis, chronic obstructive pulmonary disease, radiation skin disorder, radiation mucosal disorder, cardiac failure, pulmonary arterial hypertension, Parkinson's disease, Friedreich's ataxia, multiple sclerosis, and age-related macular degeneration in a mammal, comprising administering a pharmaceutically effective amount of the compound according to any one of claims 1 to 24 or a pharmaceutically acceptable salt thereof to the mammal.

36. A method for activating Nrf2 in a mammal, comprising administering a pharmaceutically effective amount of the compound according to any one of claims 1 to 24 or a pharmaceutically acceptable salt thereof to the mammal.

37. A method for inhibiting protein-protein interaction between Keap1 and Nrf2 in a mammal, comprising administering a pharmaceutically effective amount of the compound according to any one of claims 1 to 24 or a pharmaceutically acceptable salt thereof to the mammal.

38. An Nrf2 activator comprising the compound according to any one of claims 1 to 24 or a pharmaceutically acceptable salt thereof as an active ingredient.

39. An inhibitor of protein-protein interaction between Keap1 and Nrf2, comprising the compound according to any one of claims 1 to 24 or a pharmaceutically acceptable salt thereof as an active ingredient.
